# EUROPEAN PATENT APPLICATION

(11) **EP 4 775 670 A2**
(43) Date of publication of application: **15.07.2026**
(21) Application number: 26178019.1
(22) Date of filing: 29.11.2019
(51) Int. Cl.: C12N 5/0783

(54) **METHODS FOR TREATMENT USING ADOPTIVE CELL THERAPY**

(30) Priority: 30.11.2018 US 201862774164 P; 06.12.2018 US 201862776415 P; 14.05.2019 US 201962847926 P; 30.05.2019 US 201962854945 P; 22.08.2019 US 201962890600 P; 05.11.2019 US 201962931204 P
(62) Divisional of application: 24174516.5
(71) Applicant: Juno Therapeutics, Inc., Seattle, WA 98109 (US)
(72) Inventor: ALBERTSON, Tina, Seattle, 98109-4703 (US); GARCIA, Jacob Randolph, Seattle, 98109-4703 (US); GILBERT, Mark, Seattle, 98109-4703 (US); LI, He, Seattle, 98109-4703 (US); SUTHERLAND, Claire, Seattle, 98109-4703 (US); TREDE, Nikolaus Sebastian, Seattle, 98109 (US)
(74) Representative: Carpmaels & Ransford LLP

(57) **Abstract**

Provided are adoptive cell therapy involving the administration of doses of cells for treating subjects with disease and conditions such as certain B cell malignancies, and related methods, compositions, uses and articles of manufacture. The cells generally express recombinant receptors such as chimeric antigen receptors (CARs). In some embodiments, the disease or condition is a large B cell lymphoma, such as a diffuse large B-cell lymphoma (DLBCL). Also provided are methods of assessing the risk of developing a toxicity related to a cell therapy, and methods of identifying subjects and methods of treating subjects based on the assessment of risks.

## Description

### Cross-Reference to Related Applications

This application claims priority from U.S. provisional application No. 62/774,164, filed November 30, 2018, entitled "METHODS FOR TREATMENT USING ADOPTIVE CELL THERAPY," U.S. provisional application No. 62/776,415, filed December 6, 2018, entitled "METHODS FOR TREATMENT USING ADOPTIVE CELL THERAPY," U.S. provisional application No. 62/847,926, filed May 14, 2019, entitled "METHODS FOR TREATMENT USING ADOPTIVE CELL THERAPY," U.S. provisional application No. 62/854,945, filed May 30, 2019, entitled "METHODS FOR TREATMENT USING ADOPTIVE CELL THERAPY," U.S. provisional application No. 62/890,600, filed August 22, 2019, entitled "METHODS FOR TREATMENT USING ADOPTIVE CELL THERAPY," and U.S. provisional application No. 62/931,204, filed November 5, 2019, entitled "METHODS FOR TREATMENT USING ADOPTIVE CELL THERAPY," the contents of which are incorporated by reference in their entirety.

### Incorporation by Reference of Sequence Listing

The present application is being filed along with a Sequence Listing in electronic format. The Sequence Listing is provided as a file entitled 735042019640SeqList.txt, created November 28, 2019, which is 34.2 kilobytes in size. The information in the electronic format of the Sequence Listing is incorporated by reference in its entirety.

### Field

The present disclosure relates in some aspects to adoptive cell therapy involving the administration of doses of cells for treating subjects with disease and conditions such as certain B cell malignancies, and related methods, compositions, uses and articles of manufacture. The cells generally express recombinant receptors such as chimeric antigen receptors (CARs). In some embodiments, the disease or condition is a large B cell lymphoma, such as a diffuse large B-cell lymphoma (DLBCL). Also provided are methods of assessing the risk of developing a toxicity related to a cell therapy, and methods of identifying subjects and methods of treating subjects based on the assessment of risks.

### Background

Various immunotherapy and/or cell therapy methods are available for treating diseases and conditions. For example, adoptive cell therapies (including those involving the administration of cells expressing chimeric receptors specific for a disease or disorder of interest, such as chimeric antigen receptors (CARs) and/or other recombinant antigen receptors, as well as other adoptive immune cell and adoptive T cell therapies) can be beneficial in the treatment of cancer or other diseases or disorders. Improved approaches are needed. Provided are methods, uses and articles of manufacture that meet such needs.

### Summary

Provided herein are methods of treating a subject having or suspected of having a disease or condition that is a relapsed or refractory large B cell lymphoma (r/r LBCL). In some of any of the provided embodiments, the method involves administering to the subject a dose of CD4⁺ and CD8⁺ T cells, wherein T cells of each dose comprises a chimeric antigen receptor (CAR) that specifically binds to CD19, wherein: the dose of T cells comprises between at or about 1 x 10⁷ CAR-expressing T cells and at or about 2 x 10⁸ CAR-expressing T cells, inclusive; the dose of T cells comprises a ratio of approximately 1:1 CD4⁺ T cells expressing the CAR to CD8⁺ T cells expressing the CAR; and the administration comprises administering a plurality of separate compositions, wherein the plurality of separate compositions comprises a first composition comprising CD8⁺ T cells and a second composition comprising CD4⁺ T cells.

Also provided herein are methods of treating a subject having or suspected of having a disease or condition, the method containing administering to the subject a dose of CD4⁺ and CD8⁺ T cells, each of the CD4⁺ and the CD8⁺ T cells, individually, containing receptor that specifically binds to an antigen, e.g., target antigen, expressed by the disease or condition or a cell or tissue thereof and/or that is associated with the disease or condition, wherein the administration includes administering a plurality of separate compositions, the plurality of separate compositions containing a first composition containing CD8⁺ T cells and a second composition containing CD4⁺ T cells.

Also provided herein are methods of treating a subject having or suspected of having a disease or condition that is a B cell malignancy, the method comprising administering to the subject a dose of CD4⁺ and CD8⁺ T cells, wherein T cells of each dose comprises a recombinant receptor that specifically binds to an antigen, e.g., target antigen, expressed by the disease or condition or a cell or tissue thereof and/or that is associated with the disease or condition, wherein the administration comprises administering a plurality of separate compositions, the plurality of separate compositions comprising a first composition comprising CD8⁺ T cells and a second composition comprising CD4⁺ T cells.

Also provided herein are methods of treating a subject having or suspected of having a disease or condition that is a large B cell lymphoma, the method comprising administering to the subject a dose of CD4⁺ and CD8⁺ T cells, wherein T cells of each dose comprises a recombinant receptor that specifically binds to an antigen, e.g., target antigen, expressed by the disease or condition or a cell or tissue thereof and/or that is associated with the disease or condition, wherein the administration comprises administering a plurality of separate compositions, the plurality of separate compositions comprising a first composition comprising CD8⁺ T cells and a second composition comprising CD4⁺ T cells.

Provided herein are methods of treating a subject having or suspected of having a disease or condition that is a non-Hodgkin lymphoma (NHL) or a large B cell lymphoma, the method including administering to the subject a dose of T cells containing T cells expressing a chimeric antigen receptor (CAR) that specifically binds to an antigen, e.g., target antigen, expressed by the NHL or large B cell lymphoma, wherein: the dose of T cells contains between at or about 1 x 10⁷ CAR-expressing T cells and at or about 2 x 10⁸ CAR-expressing T cells, inclusive; and the dose of T cells contains CD4⁺ and CD8⁺ T cells, each of the CD4⁺ and the CD8⁺ T cells, individually, containing a receptor that specifically binds to an antigen, e.g., target antigen, expressed by the disease or condition or a cell or tissue thereof and/or that is associated with the disease or condition, wherein the administration includes administering a plurality of separate compositions, the plurality of separate compositions containing a first composition comprising CD8⁺ T cells and a second composition containing CD4⁺ T cells.

In some of any of the provided embodiments, the dose of T cells comprises a defined ratio of CD4⁺ cells expressing the CAR to CD8⁺ cells expressing the CAR and/or of CD4⁺ cells to CD8⁺ cells, which ratio is approximately 1:1 or is between approximately 1:3 and approximately 3:1.

Provided herein are methods of treating a subject having a disease or condition that is a non-Hodgkin lymphoma (NHL) or a large B cell lymphoma, the methods including administering to the subject a dose of T cells comprising T cells expressing a chimeric antigen receptor (CAR) that specifically binds to an antigen, e.g., target antigen, expressed by the NHL or large B cell lymphoma, the dose of T cells containing a defined ratio of CD4⁺ cells expressing the CAR to CD8⁺ cells expressing the CAR and/or of CD4⁺ cells to CD8⁺ cells, which ratio is approximately or is 1:1, wherein the administration includes administering a plurality of separate compositions, the plurality of separate compositions containing a first composition comprising CD8⁺ T cells and a second composition containing CD4⁺ T cells.

Also provided herein are methods of treating a subject having a disease or condition that is a non-Hodgkin lymphoma (NHL) or a large B cell lymphoma, including administering to the subject a dose of T cells comprising T cells expressing a chimeric antigen receptor (CAR) that specifically binds to an antigen, e.g., target antigen, expressed by the NHL or large B cell lymphoma, wherein: the dose of T cells contains between at or about 5 x 10⁷ recombinant receptor-expressing T cells and at or about 1.5 x 10⁸ recombinant receptor-expressing T cells, inclusive, said dose containing a defined ratio of CD4⁺ cells expressing the recombinant receptor to CD8⁺ cells expressing the recombinant receptor and/or of CD4⁺ cells to CD8⁺ cells, which ratio is approximately or is 1:1; and the method results in (1) a complete response (CR) in at least 35%, at least 40% or at least 50% of subjects treated and/or objective response (OR) in at least 50%, at least 60% or at least 70% of subjects treated and (2) results in no more than 50% of subjects exhibiting a cytokine release syndrome (CRS) higher than grade 2 and/or a neurotoxicity higher than grade 2, wherein the administration includes administering a plurality of separate compositions, the plurality of separate compositions containing a first composition containing CD8⁺ T cells and a second containing comprising CD4⁺ T cells.

In some of any of the provided embodiments, the method includes administering, to a subject that has a disease or condition that is a lymphoma, a dose of T cells containing T cells expressing a chimeric antigen receptor (CAR) that specifically binds to an antigen, e.g., target antigen, expressed by the lymphoma, wherein the lymphoma in the subject is associated with or involves central nervous system (CNS) involvement; and the dose of T cells contains CD4⁺ and CD8⁺ T cells, wherein T cells of each dose comprises a receptor that specifically binds to an antigen, e.g., target antigen, expressed by the disease or condition or a cell or tissue thereof and/or that is associated with the disease or condition, wherein the administration includes administering a plurality of separate compositions, the plurality of separate compositions containing a first composition containing CD8⁺ T cells and a second composition containing CD4⁺ T cells. In some of any of the provided embodiments, at or prior to the time of administration of the dose of cells, the subject comprises a brain lesion, such as a temporal lobe brain lesion. In some of any such embodiments, the lymphoma is a B cell malignancy. In some of any such embodiments, the lymphoma is non-Hodgkin lymphoma (NHL) or a large B cell lymphoma.

Provided herein is a method of treatment involving (a) selecting a subject that has a follicular lymphoma (FL) for treatment; (b) administering to the subject a dose of T cells comprising T cells expressing a recombinant receptor that specifically binds to an antigen, e.g., target antigen, expressed by FL or a cell or tissue thereof and/or that is associated with FL.

In some of any of the provided embodiments, the large B cell lymphoma is selected from an aggressive non-Hodgkin lymphoma (NHL), diffuse large B cell lymphoma (DLBCL), optionally DLBCL NOS (de novo or transformed from indolent), high-grade B-cell lymphoma (HGBCL), double/triple hit lymphoma, primary mediastinal large B cell lymphoma (PMBCL), mantle cell lymphoma (MCL), transformed follicular lymphoma (tFL), and/or follicular lymphoma (FL), optionally follicular lymphoma Grade 3B (FL3B). In some of any embodiments, the large B cell lymphoma is a Diffuse Large B-Cell Lymphoma (DLBCL). In some of any embodiments, the DLBCL is a DLBCL NOS, a de novo DLBCL or a DLBCL transformed from indolent lymphoma. In some of any embodiments, the DLBCL is a de novo DLBCL. In some of any embodiments, the DLBCL is a DLBCL transformed from indolent lymphomas other than FL. In some of any embodiments, the DLBCL is a DLBCL transformed from marginal zone lymphoma (tMZL) or a DLBCL transformed from chronic lymphocytic leukemia (tCLL; Richter's). In some of any embodiments, the large B cell lymphoma is a high-grade B cell lymphoma (HGBCL). In some of any embodiments, the HGBCL has MYC and BCL2 and/or BCL6 rearrangements. In some of any embodiments, the HGBCL has a DLBCL histology. In some of any embodiments, the large B cell lymphoma is a double/triple hit lymphoma. In some of any embodiments, the large B cell lymphoma is primary mediastinal B-cell lymphoma (PMBCL). In some of any embodiments, the large B cell lymphoma is mantle cell lymphoma (MCL). In some of any embodiments, the large B cell lymphoma is not a primary central nervous system lymphoma (PCNSL). In some of any embodiments, the large B cell lymphoma is a transformed follicular lymphoma (tFL). In some of any embodiments, the large B cell lymphoma is follicular lymphoma (FL). In some of any embodiments, the FL is associated with co-expression of CD10, BCL6 and BCL2 within the follicles, and/or t(14;18)/(q32;q21) (IGH-BCL2) and/or BCL6 rearrangements. In some of any embodiments, the large B cell lymphoma is follicular lymphoma grade 3B (FL3B).

In some of any embodiments, the dose of T cells is enriched for CD3+ T cells, CD4+ T cells, CD8+ T cells or CD4+ T cells and CD8+ T cells. In some of any embodiments, greater than at or about 70%, 75%, 80%, 85%, 90%, 95% or 98% of the cells in the dose of T cells are CD3+ T cells, CD4+ T cells, CD8+ T cells or CD4+ T cells and CD8+ T cells. In some of any embodiments, the dose of T cells comprises a defined ratio of CD4⁺ cells expressing the receptor to CD8⁺ cells expressing the receptor and/or of CD4⁺ T cells to CD8⁺ T cells, which ratio is approximately 1:1 or is between approximately 1:3 and approximately 3:1. In some of any embodiments, the defined ratio is or is approximately 1:1. In some of any embodiments, the dose of T cells comprises a dose of CD4⁺ and CD8⁺ T cells, wherein T cells of each dose comprises a recombinant receptor that specifically binds to an antigen, e.g., target antigen, expressed by FL or a cell or tissue thereof and/or that is associated with FL, wherein the administration comprises administering a plurality of separate compositions, the plurality of separate compositions comprising a first composition comprising CD8⁺ T cells and a second composition comprising CD4⁺ T cells.

In some of any of the provided embodiments, the initiation of the administration of the first composition is carried out prior to the initiation of the administration of the second composition. In some of any of the provided embodiments, the administration of the first composition and the administration of the second composition are carried out no more than 48 hours apart. In some of any of the provided embodiments, the administration of the first composition and the administration of the second composition are carried out no more than 36 hours apart, no more than 24 hours apart, no more than 12 hours apart, no more than 6 hours apart, no more than 4 hours apart, no more than 2 hours apart, no more than 1 hour apart or no more than 30 minutes apart. In some of any of the provided embodiments, the administration of the first composition and the administration of the second composition are carried out between at or about 0 and at or about 48 hours, between at or about 0 and at or about 36 hours, between at or about 0 and at or about 24 hours, between at or about 0 and at or about 12 hours, between at or about 0 and at or about 6 hours, between at or about 0 and at or about 2 hours, between at or about 0 and at or about 1 hours, between at or about 0 and at or about 30 minutes, between at or about 30 minutes and at or about 48 hours, between at or about 30 minutes and at or about 36 hours, between at or about 30 minutes and at or about 24 hours, between at or about 30 minutes and at or about 12 hours, between at or about 30 minutes and at or about 6 hours, between at or about 30 minutes and at or about 4 hours, between at or about 30 minutes and at or about 2 hours, between at or about 30 minutes and at or about 1 hour, between at or about 1 hours and at or about 48 hours, between at or about 1 hour and at or about 36 hours, between at or about 1 hour and at or about 24 hours, between at or about 1 hour and at or about 12 hours, between at or about 1 hour and at or about 6 hours, between at or about 1 hour and at or about 4 hours, between at or about 1 hour and at or about 2 hours, between at or about 2 hours and at or about 48 hours, between at or about 2 hours and at or about 36 hours, between at or about 2 hours and at or about 24 hours, between at or about 2 hours and at or about 12 hours, between at or about 2 hours and at or about 6 hours, between at or about 2 hours and at or about 4 hours, between at or about 4 hours and at or about 48 hours, between at or about 4 hours and at or about 36 hours, between at or about 4 hours and at or about 24 hours, between at or about 4 hours and at or about 12 hours, between at or about 4 hours and at or about 6 hours, between at or about 6 hours and at or about 48 hours, between at or about 6 hours and at or about 36 hours, between at or about 6 hours and at or about 24 hours, between at or about 6 hours and at or about 12 hours, between at or about 12 hours and at or about 48 hours, between at or about 12 hours and at or about 36 hours, between at or about 12 hours and at or about 24 hours, between at or about 24 hours and at or about 48 hours, between at or about 24 hours and at or about 36 hours or between at or about 36 hours and at or about 48 hours.

In some of any of the provided embodiments, the administration of the first composition and the administration of the second composition are carried out on the same day, are carried out between about 0 and about 12 hours apart, between about 0 and about 6 hours apart or between about 0 to 2 hours apart; or the initiation of administration of the first composition and the initiation of administration of the second composition are carried out between about 1 minute and about 1 hour apart or between about 5 minutes and about 30 minutes apart. In some of any of the provided embodiments, the first composition and second composition are administered no more than 2 hours, no more than 1 hour, no more than 30 minutes, no more than 15 minutes, no more than 10 minutes or no more than 5 minutes apart.

In some of any of the provided embodiments, the receptor contained by the CD4⁺ T cells and/or the receptor contained by the CD8⁺ T cells includes a recombinant receptor that is the same and/or wherein the CD4⁺ T cells and/or the CD8⁺ T cells are genetically engineered to express a recombinant receptor that is the same.

In some of any of the provided embodiments, the dose of CD4⁺ and CD8⁺ T cells contains a defined ratio of CD4⁺ cells expressing a recombinant receptor to CD8⁺ cells expressing a recombinant receptor and/or of CD4⁺ cells to CD8⁺ cells, that is or is approximately 1:1 or is between approximately 1:3 and approximately 3:1; and/or the CD4⁺ T cells containing the receptor in the one of the first and second compositions and the CD8⁺ T cells containing the receptor in the other of the first and second compositions are present at a defined ratio that is or is approximately 1:1 or is between approximately 1:3 and approximately 3:1; and/or the CD4⁺ T cells containing the receptor and the CD8⁺ T cells containing the receptor administered in the first and second compositions are present at a defined ratio, which ratio is or is approximately 1:1 or is between approximately 1:3 and approximately 3:1. In some of any of the provided embodiments, the defined ratio is or is approximately 1:1.

In some of any of the provided embodiments, the dose of CD4⁺ and CD8⁺ T cells contains: between at or about 1 x 10⁷ and at or about 2 x 10⁸ total recombinant receptor-expressing T cells, inclusive; between at or about 2.5 x 10⁷ and at or about 1.5 x 10⁸ total recombinant receptor-expressing T cells, inclusive; between at or about 5 x 10⁷ and at or about 1 x 10⁸ total recombinant receptor-expressing T cells, inclusive; at or about 5 x 10⁷ total recombinant receptor-expressing T cells; at or about 1 x 10⁸ total recombinant receptor-expressing T cells; or at or about 1.5 x 10⁸ total recombinant receptor-expressing T cells. In some of any such embodiments, the dose of CD4⁺ and CD8⁺ T cells comprises at or about 5 x 10⁷ total recombinant receptor-expressing T cells. In some of any such embodiments, the dose of CD4⁺ and CD8⁺ T cells comprises at or about 1 x 10⁸ total recombinant receptor-expressing T cells. In some of any such embodiments, the dose of CD4⁺ and CD8⁺ T cells comprises at or about 1.5 x 10⁸ total recombinant receptor-expressing T cells.

In some of any of the provided embodiments, the dose of CD4⁺ and CD8⁺ T cells contains: between at or about 1 x 10⁷ and at or about 1 x 10⁸ recombinant receptor-expressing CD8⁺ T cells, inclusive; between at or about 1.25 x 10⁷ and at or about 7.5 x 10⁷ recombinant receptor-expressing CD8⁺ T cells, inclusive; between at or about 2.5 x 10⁷ and at or about 5 x 10⁷ recombinant receptor-expressing CD8⁺ T cells, inclusive; at or about 2.5 x 10⁷ recombinant receptor-expressing CD8⁺ T cells; at or about 5 x 10⁷ recombinant receptor-expressing CD8⁺ T cells; or at or about 7.5 x 10⁷ recombinant receptor-expressing CD8⁺ T cells. In some of any such embodiments, the dose of CD4⁺ and CD8⁺ T cells comprises at or about 2.5 x 10⁷ recombinant receptor-expressing CD8⁺ T cells. In some of any such embodiments, the dose of CD4⁺ and CD8⁺ T cells comprises at or about 5 x 10⁷ recombinant receptor-expressing CD8⁺ T cells. In some of any such embodiments, the dose of CD4⁺ and CD8⁺ T cells comprises at or about 7.5 x 10⁷ recombinant receptor-expressing CD8⁺ T cells.

In some of any of the provided embodiments, the recombinant receptor specifically binds to an antigen associated with the disease or condition or expressed in cells of the environment of a lesion associated with the disease or condition.

In some of any of the provided embodiments, the disease or condition is a cancer. In some of any such embodiments, the disease or condition is a B cell malignancy. In some of any such embodiments, the disease or condition is a myeloma, a leukemia or a lymphoma. In some of any of the provided embodiments, the disease or condition is a B cell malignancy and/or is acute lymphoblastic leukemia (ALL), adult ALL, chronic lymphoblastic leukemia (CLL), non-Hodgkin lymphoma (NHL), or a large B cell lymphoma. In some of any of the provided embodiments, the disease or condition is a large B cell lymphoma. In some of any of the provided embodiments, the disease or condition, such as the large B cell lymphoma, is a Diffuse Large B-Cell Lymphoma (DLBCL). In some of any of the provided embodiments, the DLBCL is a DLBCL, not otherwise specified (NOS), a de novo DLBCL or a DLBCL transformed from indolent lymphoma. In some of any of the provided embodiments, the DLBCL is a de novo DLBCL. In some of any of the provided embodiments, the DLBCL is a DLBCL transformed from follicular lymphoma (tFL). In some of any of the provided embodiments, the DLBCL is a DLBCL transformed from marginal zone lymphoma (tMZL) or a DLBCL transformed from chronic lymphocytic leukemia (tCLL; Richter's). In some of any of the provided embodiments, the disease or condition is primary mediastinal B-cell lymphoma (PMBCL) or a follicular lymphoma (FL), such as follicular lymphoma grade 3B (FL3B). In some of any embodiments, the disease or condition is follicular lymphoma (FL). In some of any of the provided embodiments, the NHL or the large B cell lymphoma is selected from the group consisting of aggressive NHL, diffuse large B cell lymphoma (DLBCL), NOS (de novo or transformed from indolent), primary mediastinal large B cell lymphoma (PMBCL), mantle cell lymphoma (MCL), and/or follicular lymphoma (FL), such as follicular lymphoma Grade 3B (FL3B). In some of any embodiments, the disease or condition is follicular lymphoma (FL). In some of any embodiments, wherein the FL is associated with co-expression of CD10, BCL6 and BCL2 within the follicles, and/or t(14;18)/(q32;q21) (IGH-BCL2) and/or BCL6 rearrangements. In some of any embodiments, the disease or condition is mantle cell lymphoma (MCL).

In some of any such embodiments, the target antigen is a B cell antigen. In some of any of the provided embodiments, the antigen is CD19.

In some of any embodiments, at or immediately prior to the time of the administration of the dose of cells, the subject has relapsed following remission after treatment with, or become refractory to, two, three, four or more prior therapy for the disease or condition other than another dose of cells expressing the CAR.

In some of any embodiments, the prior therapy comprises an anthracycline and a CD20-targeted agent. In some of any embodiments, the one or more CD20-targeted agent comprises rituximab. In some of any embodiments, the one or more CD20-targeted agent comprises R-CHOP (rituximab, cyclophosphamide, doxorubicin hydrochloride (hydroxydaunomycin), vincristine sulfate (oncovin) and prednisone).

In some of any embodiments, the prior therapy comprises an allogeneic or an autologous hematopoietic stem cell transplantation (HSCT). In some of any embodiments, the subject has relapsed within 1 year or less than 1 year after receiving the HSCT.

In some of any embodiments, the subject has not achieved complete remission (CR) in response to the prior therapy.

In some of any embodiments, at or prior to the administration of the dose of cells, the subject has been identified as having an aggressive disease or high-risk disease or as having poor prognosis.

In some of any embodiments, at or prior to the administration of the dose of cells, the subject has been identified as having a chemorefractory disease or having a persistent or relapsed disease following chemotherapy. In some of any embodiments, at or prior to the administration of the dose of cells, the subject has been identified as having a chemorefractory lymphoma, optionally a chemorefractory DLBCL.

In some of any embodiments, at or prior to the administration of the dose of cells, the subject has been identified as having a lymphoma associated with or involving central nervous system (CNS) involvement or a secondary CNS lymphoma. In some of any embodiments: at or prior to administration of the dose of cells, the subject is or has been identified as having a lymphoma associated with or involving central nervous system (CNS) involvement or a secondary CNS lymphoma; and/or at least 70%, at least 80%, at least 90% or at least 95% of subjects treated according to the method who, at or prior to the administration of the dose of cells exhibited or were identified to exhibit a lymphoma with CNS involvement or a secondary CNS lymphoma, achieved a resolution of the CNS disease.

In some of any embodiments, the subject is age 65 years or older. In some of any embodiments, among the subjects treated, greater than at or about 35%, greater than at or about 40%, greater than at or about 45% or greater than at or about 50%, or any value between any of the foregoing, are aged 65 years or older. In some of any embodiments the subject is age 70 years or older.

In some of any embodiments, at or prior to the administration of the dose of cells, the subject is or has been identified as having an impaired cardiac function, optionally with a left ventricular ejection fraction (LVEF) of less than at or about 50%. In some of any embodiments, at or prior to the administration of the dose of cells, the subject is or has been identified as having an impaired renal function, optionally with a calculated creatinine clearance of less than at or about 60 mL/min. In some of any embodiments, at or prior to the administration of the dose of cells, the subject is or has been identified as having an impaired pulmonary function, optionally with a diffusing capacity of the lungs for carbon monoxide (DLCO) of at or about 60% or less. In some of any embodiments, at or prior to the administration of the dose of cells, the subject is or has been identified as having an impaired hepatic function, optionally with an aspartate aminotransferase (AST) and alanine aminotransferase (ALT) of more than at or about twice the upper limit of normal (ULN).

In some of any embodiments, at or prior to the administration of the dose of cells, the subject has received a bridging chemotherapy between the time of leukapheresis to produce the dose of CD4⁺ and CD8⁺ T cells and the administration of the dose of CD4⁺ and CD8⁺ T cells. In some of any embodiments, at or prior to the administration of the dose of cells, the subject has received a bridging chemotherapy for disease control after a prior therapy. In some of any embodiments, the bridging chemotherapy is selected from among one or more of: Rituximab-gemcitabine plus oxaliplatin, Dexamethasone, radiotherapy, Rituximab, Prednisone, BR, Lenalidomide, gemcitabine plus oxaliplatin, Brentuximab vedotin, Ibrutinib, Bendamustine, and/or Gemcitabine + rituximab.

In some of any embodiments, the subject is or has been identified as having an Eastern Cooperative Oncology Group Performance Status (ECOG PS) of 0, 1 or 2. In some of any embodiments, the subject is or has been identified as having an Eastern Cooperative Oncology Group Performance Status (ECOG PS) of 0 or 1. In some of any embodiments, the subject is or has been identified as having an Eastern Cooperative Oncology Group Performance Status (ECOG PS) of 2.

In some of any embodiments, prior to the administration of the dose of cells, the subject is or has been identified as having a sum of product dimensions (SPD) of a tumor in the subject that is at or about 50 cm² or greater.

In some of any of the provided embodiments, the recombinant receptor is a chimeric antigen receptor (CAR). In some of any of the provided embodiments, the CAR contains an extracellular antigen-recognition domain that specifically binds to the antigen and an intracellular signaling domain containing a CD3-zeta (CD3ζ) chain and a costimulatory signaling region that is a signaling domain of CD28 or 4-1BB.

In some of any of the provided embodiments, the T cells are primary T cells obtained from a subject. In some of any of the provided embodiments, the T cells are autologous to the subject.

In some of any of the provided embodiments, at least 40%, at least 50%, at least 60%, at least 70% of the subjects who, at or prior to the administration of the dose of cells had or were identified to have a double/triple hit lymphoma or relapse following administration of an autologous stem cell transplant (ASCT), achieved an OR or an OR that is durable for at or greater than 3 months or at or greater than 6 months.

In some of any of the provided embodiments, at least 35%, at least 40% or at least 50% of subjects treated according to the method achieve a complete response (CR): at least 60%, 70%, 80%, 90%, or 95% of subjects achieving a CR exhibit a CR that is durable for at or greater than 3 months or at or greater than 6 months; and/or least 60%, 70%, 80%, 90%, or 95% of subjects achieving a CR by one month and/or by 3 months remain in response, remain in CR, and/or survive or survive without progression, for at or greater than 3 months and/or at or greater than 6 months and/or at greater than 9 months after achieving the CR; and/or at least 50%, at least 60% or at least 70% of the subjects treated according to the method achieve objective response (OR); at least 60%, 70%, 80%, 90%, or 95% of subjects achieving an OR exhibit an OR that is durable for at or greater than 3 months or at or greater than 6 months; and/or at least 35%, at least 40%, or at least 50% of subjects achieving an OR remain in response or survive for at or greater than 3 months and/or at or greater than 6 months after achieving the OR; and/or at least 40%, at least 50%, at least 60%, at least 70% of the subjects who, at or prior to the administration of the dose of cells had or were identified to have a double/triple hit lymphoma or relapse, following administration of an autologous stem cell transplant (ASCT), achieved an OR, or an OR that is durable for at or greater than 3 months or at or greater than 6 months.

In some of any of the provided embodiments, the cells are autologous to the subject, and no minimum absolute lymphocyte count (ALC) for apheresis is required and/or specified for production of the therapy; and/or the cells are produced by a process which, for at least 90%, 91%, 92%, 93%, 94%, 95%, 96%, 97%, 98%, 99%, or 100% of subjects having the disease or condition or of the selected population of subjects, is capable of generating a cell product for administration according to the method.

In some of any of the provided embodiments, greater than or greater than about 50%, about 60%, about 70%, or about 80% of the subjects treated according to the method do not exhibit a grade 3 or greater cytokine release syndrome (CRS) and/or do not exhibit a grade 3 or greater neurotoxicity and/or greater than 40% or 50% or 55% do not exhibit any neurotoxicity or CRS.

In some of any of the provided embodiments, the CR or the OR is durable for greater than 3 months or greater than 6 months; and/or at least 20%, at least 25%, at least 35%, at least 40% or at least 50% of subjects treated according to the method achieve a CR that is durable for greater than 3 months or greater than 6 months; and/or at least 60%, 70%, 80%, 90%, or 95% of subjects treated with the method and who achieve a CR, remain in CR or remain in response or remain surviving for at or greater than 3 months or at or greater than 6 months or at or greater than 9 months; and/or at least 60%, 70%, 80%, 90%, or 95% of subjects treated with the method who achieve a CR by one month and/or by 3 months remain in response, remain in CR, and/or survive or survive without progression, for greater at or greater than 3 months and/or at or greater than 6 months and/or at greater than 9 months; and/or at least 50%, at least 60% or at least 70% of the subjects treated according to the method achieve objective response (OR); at least 60%, 70%, 80%, 90%, or 95% of subjects achieve an OR that is durable for at or greater than 3 months or at or greater than 6 months; and/or at least at least 35%, at least 40%, or at least 50% of subjects treated with the method and achieving an OR remain in response or survive for at or greater than 3 months and/or at or greater than 6 months.

In some of any of the provided embodiments, at or prior to administration of the dose of cells, the subject is or has been identified as having a lymphoma associated with or involving central nervous system (CNS) involvement; and/or at least 70%, at least 80%, at least 90% or at least 95% of subjects treated according to the method who, at or prior to the administration of the dose of cells exhibited or were identified to exhibit a lymphoma with CNS involvement, achieved a resolution of the CNS disease.

In some of any of the provided embodiments, the administration of the first composition and the administration of the second composition are carried out no more than 48 hours apart. In some of any of the provided embodiments, the administration of the first composition and the administration of the second composition are carried out no more than 36 hours apart, no more than 24 hours apart, no more than 12 hours apart, no more than 6 hours apart, no more than 4 hours apart, no more than 2 hours apart, no more than 1 hour apart or no more than 30 minutes apart.

In some of any of the provided embodiments, the administration of the first composition and the administration of the second composition are carried out between at or about 0 and at or about 48 hours, between at or about 0 and at or about 36 hours, between at or about 0 and at or about 24 hours, between at or about 0 and at or about 12 hours, between at or about 0 and at or about 6 hours, between at or about 0 and at or about 2 hours, between at or about 0 and at or about 1 hours, between at or about 0 and at or about 30 minutes, between at or about 30 minutes and at or about 48 hours, between at or about 30 minutes and at or about 36 hours, between at or about 30 minutes and at or about 24 hours, between at or about 30 minutes and at or about 12 hours, between at or about 30 minutes and at or about 6 hours, between at or about 30 minutes and at or about 4 hours, between at or about 30 minutes and at or about 2 hours, between at or about 30 minutes and at or about 1 hour, between at or about 1 hours and at or about 48 hours, between at or about 1 hour and at or about 36 hours, between at or about 1 hour and at or about 24 hours, between at or about 1 hour and at or about 12 hours, between at or about 1 hour and at or about 6 hours, between at or about 1 hour and at or about 4 hours, between at or about 1 hour and at or about 2 hours, between at or about 2 hours and at or about 48 hours, between at or about 2 hours and at or about 36 hours, between at or about 2 hours and at or about 24 hours, between at or about 2 hours and at or about 12 hours, between at or about 2 hours and at or about 6 hours, between at or about 2 hours and at or about 4 hours, between at or about 4 hours and at or about 48 hours, between at or about 4 hours and at or about 36 hours, between at or about 4 hours and at or about 24 hours, between at or about 4 hours and at or about 12 hours, between at or about 4 hours and at or about 6 hours, between at or about 6 hours and at or about 48 hours, between at or about 6 hours and at or about 36 hours, between at or about 6 hours and at or about 24 hours, between at or about 6 hours and at or about 12 hours, between at or about 12 hours and at or about 48 hours, between at or about 12 hours and at or about 36 hours, between at or about 12 hours and at or about 24 hours, between at or about 24 hours and at or about 48 hours, between at or about 24 hours and at or about 36 hours or between at or about 36 hours and at or about 48 hours.

In some of any of the provided embodiments, the administration of the first composition and the administration of the second composition are carried out on the same day, are carried out between about 0 and about 12 hours apart, between about 0 and about 6 hours apart or between about 0 to 2 hours apart; or the initiation of administration of the first composition and the initiation of administration of the second composition are carried out between about 1 minute and about 1 hour apart or between about 5 minutes and about 30 minutes apart.

In some of any of the provided embodiments, the first composition and second composition are administered no more than 2 hours, no more than 1 hour, no more than 30 minutes, no more than 15 minutes, no more than 10 minutes or no more than 5 minutes apart.

In some of any of the provided embodiments, the receptor comprised by the CD4⁺ T cells and/or the receptor comprised by the CD8⁺ T cells comprises a recombinant receptor that is the same and/or wherein the CD4⁺ T cells and/or the CD8⁺ T cells are genetically engineered to express a recombinant receptor that is the same.

In some of any of the provided embodiments, the lymphoma is a B cell malignancy. In some of any of the provided embodiments, the lymphoma is non-Hodgkin lymphoma (NHL).

In some of any of the provided embodiments, at least 35%, at least 40% or at least 50% of subjects treated according to the method achieve a complete response (CR) or remission of CNS disease; at least 60%, 70%, 80%, 90%, or 95% of subjects who achieve a CR remain in CR for at or greater than 3 months or at or greater than 6 months; and/or at least 60%, 70%, 80%, 90%, or 95% of subjects achieving a CR or remission of CNS disease by one month and/or by 3 months remain in response, remain in CR, and/or survive or survive without progression, for greater at or greater than 3 months and/or at or greater than 6 months and/or at greater than 9 months; and/or at least 50%, at least 60% or at least 70% of the subjects treated according to the method achieve objective response (OR) or remission of CNS disease; at least 60%, 70%, 80%, 90%, or 95% of subjects achieving the OR, for at or greater than 3 months or at or greater than 6 months; and/or at least 60%, 70%, 80%, 90%, or 95% of subjects achieving OR or remission of CNS disease remain in response or survive for at or greater than 3 months and/or at or greater than 6 months; and/or the brain lesion is reduced in size or volume by greater than or greater than about 25%, 50%, 75% or more; and/or reduction or remission or clearance of CNS disease is achieved in at least 35%, at least 40% or at least 50% of subjects treated according to the method.

In some of any of the provided embodiments, greater than or greater than about 30%, 35%, 40%, or 50% of the subjects treated according to the method do not exhibit any grade of cytokine release syndrome (CRS) or neurotoxicity; and/or at least at or about 45%, 50%, 60%, 65%, 70%, 75%, 80%, 85%, 90%, or 95% of subjects treated according to the method do not exhibit onset of CRS earlier than 3 days following initiation of the administration and/or do not exhibit onset of neurotoxicity earlier than 5 days following initiation of the administration; and/or the median onset of neurotoxicity among subjects treated according to the method is at or after the median peak of, or median time to resolution of, CRS in subjects treated according to the method and/or the median onset of neurotoxicity among subjects treated according to the method is greater than at or about 8, 9, 10, or 11 days.

In some of any embodiments, greater than or greater than about 25%, 30%, 35%, 40%, 45%, 50%, 55% or 60% of the subjects treated according to the method exhibit an improvement of 10 points or greater in European Organization for Research and Treatment Core Quality of Life Questionnaire version 3.0 (EORTC QLQ-C30) in global health status at months 6 or month 12 after administration compared to the score prior to treatment or at month 1 after treatment. In some of any embodiments, greater than or greater than about 25%, 30%, 35%, 40%, 45%, 50%, 55% or 60% of the subjects treated according to the method exhibit an improvement of 10 points or greater in EORTC QLQ-C30 in physical functioning at months 6 or month 12 after administration compared to the score prior to treatment or at month 1 after treatment. In some of any embodiments, greater than or greater than about 25%, 30%, 35%, 40%, 45%, 50%, 55% or 60% of the subjects treated according to the method exhibit an improvement of 10 points or greater in EORTC QLQ-C30 in fatigue at months 6 or month 12 after administration compared to the score prior to treatment or at month 1 after treatment. In some of any embodiments, greater than or greater than about 25%, 30%, 35%, 40%, 45%, 50%, 55% or 60% of the subjects treated according to the method exhibit an improvement of 10 points or greater in EORTC QLQ-C30 in pain at months 6 or month 12 after administration compared to the score prior to treatment or at month 1 after treatment. In some of any embodiments, greater than or greater than about 25%, 30%, 35%, 40%, 45%, 50%, 55% or 60% of the subjects treated according to the method exhibit an improvement of 10 points or greater in EORTC QLQ-C30 in pain at months 6 or month 12 after administration compared to the score prior to treatment or at month 1 after treatment.

In some of any embodiments, the mean 5-level EuroQol-5D (EQ-5D-5L) score among subjects treated according to the method is the same or greater at months 6 or month 12 after administration compared to the score prior to treatment or at month 1 after treatment. In some of any embodiments, the mean EuroQol global visual analog scale (EQ-VAS) score among subjects treated according to the method is the same or greater at months 6 or month 12 after administration compared to the score prior to treatment or at month 1 after treatment.

In some of any of the provided embodiments, prior to initiation of administration of the dose of cells, the subject has not been administered an agent or treatment capable of treating, preventing, delaying, reducing or attenuating the development or risk of development of a toxicity following administration of the dose of cells.

In some of any of the provided embodiments, the agent is or comprises an anti-IL-6 antibody, anti-IL-6 receptor antibody or a steroid. In some of any of the provided embodiments, the agent is or comprises tocilizumab, siltuximab, dexamethasone or methylprednisolone.

In some of any of the provided embodiments, the administration and any follow up is carried out on an outpatient basis and/or without requiring admission to or an overnight stay at a hospital; and if the subject exhibits a sustained fever or a fever that is or has not been reduced or not reduced by more than 1°C after treatment with an antipyretic, the subject is admitted to the hospital or to an overnight stay at a hospital and/or is administered an agent or treatment for the treatment or prevention or reduction or attenuation of a neurotoxicity and/or a cytokine release syndrome or risk thereof.

In some of any of the provided embodiments, the NHL is selected from the group consisting of aggressive NHL, diffuse large B cell lymphoma (DLBCL), NOS (de novo or transformed from indolent), primary mediastinal large B cell lymphoma (PMBCL), mantle cell lymphoma (MCL), and/or follicular lymphoma (FL), such as follicular lymphoma Grade 3B (FL3B). In some of any embodiments, the subject has follicular lymphoma (FL). In some of any of the provided embodiments, the NHL is a DLBCL. In some of any of the provided embodiments, the DLBCL is a DLBCL, not otherwise specified (NOS), a de novo DLBCL or a DLBCL transformed from indolent lymphoma. In some of any of the provided embodiments, the DLBCL is a de novo DLBCL. In some of any of the provided embodiments, the DLBCL is a DLBCL transformed from follicular lymphoma (tFL). In some of any of the provided embodiments, the DLBCL is a DLBCL transformed from marginal zone lymphoma (tMZL) or a DLBCL transformed from chronic lymphocytic leukemia (tCLL; Richter's). In some of any of the provided embodiments, the NHL is primary mediastinal B-cell lymphoma (PMBCL) or a follicular lymphoma (FL), such as follicular lymphoma grade 3B (FL3B). In some of any embodiments, the subject has follicular lymphoma (FL).

In some of any of the provided embodiments, the subject is or has been identified as having an Eastern Cooperative Oncology Group Performance Status (ECOG) status of 0, 1 or 2. In some of any embodiments, the subject is or has been identified as having an Eastern Cooperative Oncology Group Performance Status (ECOG PS) of 0 or 1.

In some of any of the provided embodiments, at or immediately prior to the time of the administration of the dose of cells the subject has relapsed following remission after treatment with, or become refractory to, one or more prior therapies for the disease or condition, such as a large B cell lymphoma or an NHL. In some embodiments, the one, two or three prior therapies are other than another dose of cells expressing the CAR.

In some of any embodiments, at or immediately prior to the time of the administration of the dose of cells, the subject has relapsed following remission after treatment with, or become refractory to, one prior therapy for the disease or condition other than another dose of cells expressing the CAR. In some of any embodiments, at or immediately prior to the time of the administration of the dose of cells, the subject has relapsed following remission after treatment with, or become refractory to, two or more prior therapy for the disease or condition other than another dose of cells expressing the CAR. In some of any embodiments, at or immediately prior to the time of the administration of the dose of cells, the subject is or has been identified as being ineligible for a high-dose chemotherapy. In some of any embodiments, at or immediately prior to the time of the administration of the dose of cells, the subject is or has been identified as being ineligible for a hematopoietic stem cell transplantation (HSCT). In some of any embodiments, at or immediately prior to the time of the administration of the dose of cells, the subject is or has been identified as being ineligible for both a high-dose chemotherapy and a hematopoietic stem cell transplantation (HSCT).

In some of any embodiments, the subject has a relapsed/refractory NHL, and at or immediately prior to the time of the administration of the dose of cells, the subject is or has been identified as being ineligible for both a high-dose chemotherapy and a hematopoietic stem cell transplantation (HSCT), and the subject has relapsed following remission after treatment with, or become refractory to, one prior therapy for the disease or condition other than another dose of cells expressing the CAR.In some of any embodiments, at or prior to the administration of the dose of cells: the subject has or has been identified as having a relapsed or refractory large B cell lymphoma; and/or the subject is or has been treated with an anthracycline and one or more CD20-targeted agent; and/or the subject is or has relapsed or refractory disease after two or more lines of therapy or after autologous HSCT; and/or the subject is or has been identified as having an ECOG performance status of 1 or 2; and/or if the subject has received a prior CD19-targeted therapy, a biological sample obtained from the subject after the prior CD19-targeted therapy comprises a cell expressing CD19; and the administration of the cell dose is carried out via outpatient delivery.

In some of any of the provided embodiments, at or prior to the administration of the dose of cells: the subject is or has been identified as having a double/triple hit lymphoma; and/or the subject is or has been identified as having a chemorefractory lymphoma, such as a chemorefractory DLBCL; and/or the subject has not achieved complete remission (CR) in response to a prior therapy; and/or the subject has relapsed within 1 year or less than 1 year after receiving an autologous stem cell transplant (ASCT).

In some of any embodiments, at or prior to the administration of the dose of cells, the subject has a positron emission tomography (PET)-positive disease.

In some of any embodiments, at or prior to the administration of the dose of cells, the subject is or has been treated with an anthracycline and one or more CD20-targeted agent. In some of any embodiments, the one or more CD20-targeted agent comprises rituximab. In some of any embodiments, the one or more CD20-targeted agent comprises R-CHOP (rituximab, cyclophosphamide, doxorubicin hydrochloride (hydroxydaunomycin), vincristine sulfate (oncovin) and prednisone).

In some of any of the provided embodiment, prior to administration of the dose of cells, identifying or selecting a subject for the administration of the dose of cells that has: a double/triple hit lymphoma; a chemorefractory lymphoma, such as a chemorefractory DLBCL; not achieved complete remission (CR) in response to a prior therapy for treating the disease or disorder, such as the malignancy, such as the NHL or large B cell lymphoma; and/or has relapsed within 1 year or less than 1 year after receiving an autologous stem cell transplant (ASCT); and/or has a lymphoma associated with or involving central nervous system (CNS) involvement.

In some of any embodiments, prior to administration of the dose of cells, the provided embodiments involve identifying or selecting for the administration of the dose of cells a subject that has a follicular lymphoma (FL), such a FL that is associated with co-expression of CD10, BCL6 and BCL2 within the follicles, and/or t(14;18)/(q32;q21) (IGH-BCL2) and/or BCL6 rearrangements.

In some of any embodiments, at or prior to the administration of the dose of cells, if the subject has received a prior CD19-targeted therapy, a biological sample obtained from the subject after the prior CD19-targeted therapy comprises a cell expressing CD19.

In some of any embodiments, at or prior to the administration of the dose of cells: the subject has or has been identified as having a relapsed or refractory large B cell lymphoma; and/or the subject is or has been treated with an anthracycline and one or more CD20-targeted agent; and/or the subject is or has relapsed or refractory disease after two or more lines of therapy or after autologous HSCT; and/or the subject is or has been identified as having an ECOG performance status of 1 or 2; and/or if the subject has received a prior CD19-targeted therapy, a biological sample obtained from the subject after the prior CD19-targeted therapy comprises a cell expressing CD19.

In some of any embodiments, the administration of the cell dose is carried out via outpatient delivery.

In some of any embodiments, at or prior to the administration of the dose of cells: the subject has or has been identified as having a relapsed or refractory large B cell lymphoma; and/or the subject is or has been treated with an anthracycline and one or more CD20-targeted agent; and/or the subject is or has relapsed or refractory disease after two or more lines of therapy or after autologous HSCT; and/or the subject is or has been identified as having an ECOG performance status of 1 or 2; and/or if the subject has received a prior CD19-targeted therapy, a biological sample obtained from the subject after the prior CD19-targeted therapy comprises a cell expressing CD19; and the administration of the cell dose is carried out via outpatient delivery.

In some of any embodiments, prior to administration of the dose of cells, identifying or selecting for the administration of the dose of cells a subject that has a relapsed/refractory NHL; that is or has been identified as being ineligible for both a high-dose chemotherapy and a hematopoietic stem cell transplantation (HSCT); and that has relapsed following remission after treatment with, or become refractory to, one prior therapy for the disease or condition other than another dose of cells expressing the CAR.

In some of any embodiments, prior to administration of the dose of cells, identifying or selecting for the administration of the dose of cells a subject that is or has: age 70 years or older; and/or an ECOG performance status of 2; and/or an impaired pulmonary function, optionally with a diffusing capacity of the lungs for carbon monoxide (DLCO) of at or about 60% or less; and/or an impaired cardiac function, optionally with a left ventricular ejection fraction (LVEF) of less than at or about 50%; and/or an impaired renal function, optionally with a calculated creatinine clearance of less than at or about 60 mL/min; and/or an impaired hepatic function, optionally with an aspartate aminotransferase (AST) and alanine aminotransferase (ALT) of more than at or about twice the upper limit of normal (ULN).

In some of any embodiments, prior to administration of the dose of cells that is carried out via carried out via outpatient delivery, identifying or selecting for the administration of the dose of cells a subject that is or has: a relapsed or refractory large B cell lymphoma; and/or an anthracycline and one or more CD20-targeted agent; and/or relapsed or refractory disease after two or more lines of therapy or after autologous HSCT; and/or an ECOG performance status of 1 or 2; and/or if the subject has received a prior CD19-targeted therapy, a biological sample obtained from the subject after the prior CD19-targeted therapy comprises a cell expressing CD19.

In some of any embodiments, prior to the administration, the subject has been preconditioned with a lymphodepleting therapy comprising the administration of fludarabine and/or cyclophosphamide. In some of any embodiments, the methods also involveimmediately prior to the administration of a dose of the cells, administering a lymphodepleting therapy to the subject comprising the administration of fludarabine and/or cyclophosphamide.

In some of any embodiments, the administration of the cell dose and/or the lymphodepleting therapy is carried out via outpatient delivery. In some of any embodiments, the administration of the cell dose and/or the lymphodepleting therapy is carried out in a non-tertiary care center. In some of any embodiments, after the administration of a dose of the cells, the subject is monitored in an outpatient setting, optionally via contacting by telephone and/or a visit by a healthcare professional.

In some of any of the provided embodiments, the methods further include administering to the subject an additional therapeutic agent or therapy. In some embodiments, the additional agent or therapy is a therapy other than a cell therapy, such as other than CAR⁺ T cell therapy.

In some of any of the provided embodiments, the CAR comprises an extracellular antigen-binding domain specific for the antigen, a transmembrane domain, a cytoplasmic signaling domain derived from a costimulatory molecule, which, in some cases, is a 4-1BB, and a cytoplasmic signaling domain derived from a primary signaling ITAM-containing molecule, which, in some cases, is a CD3zeta; the CAR comprises, in order, an extracellular antigen-binding domain specific for the antigen, a transmembrane domain, a cytoplasmic signaling domain derived from a costimulatory molecule, and a cytoplasmic signaling domain derived from a primary signaling ITAM-containing molecule; or the CAR comprises an extracellular antigen-recognition domain that specifically binds to the antigen and an intracellular signaling domain comprising a CD3-zeta (CD3ζ) chain and a costimulatory signaling region that is a signaling domain of CD28 or 4-1BB.

In some of any of the provided embodiments, the antigen-binding domain is an scFv. In some of any such embodiments, the scFv comprises an amino acid sequence of RASQDISKYLN (SEQ ID NO: 35), an amino acid sequence of SRLHSGV (SEQ ID NO: 36), and/or an amino acid sequence of GNTLPYTFG (SEQ ID NO: 37) and/or an amino acid sequence of DYGVS (SEQ ID NO: 38), an amino acid sequence of VIWGSETTYYNSALKS (SEQ ID NO: 39), and/or an amino acid sequence of YAMDYWG (SEQ ID NO: 40) or wherein the scFv comprises a variable heavy chain region of FMC63 and a variable light chain region of FMC63 and/or a CDRL1 sequence of FMC63, a CDRL2 sequence of FMC63, a CDRL3 sequence of FMC63, a CDRH1 sequence of FMC63, a CDRH2 sequence of FMC63, and a CDRH3 sequence of FMC63 or binds to the same epitope as or competes for binding with any of the foregoing, and, in some cases, wherein the scFv comprises, in order, a V_{H}, a linker, which in some cases, comprising SEQ ID NO: 24, and a V_{L}, and/or the scFv comprises a flexible linker and/or comprises the amino acid sequence set forth as SEQ ID NO: 43.

In some of any of the provided embodiments, the costimulatory signaling region is a signaling domain of CD28 or 4-1BB. In some of any such embodiments, the costimulatory signaling region is a signaling domain of 4-1BB. In some of any such embodiments, the costimulatory domain comprises SEQ ID NO: 12 or a variant thereof having at least 85%, 86%, 87%, 88%, 89%, 90%, 91%, 92%, 93%, 94%, 95%, 96%, 97%, 98%, 99% or more sequence identity thereto.

In some of any of the provided embodiments, the primary signaling domain is a CD3zeta signaling domain. In some of any such embodiments, the primary signaling domain comprises SEQ ID NO: 13, 14 or 15 having at least 85%, 86%, 87%, 88%, 89%, 90%, 91%, 92%, 93%, 94%, 95%, 96%, 97%, 98%, 99% or more sequence identity thereto.

In some of any of the provided embodiments, the CAR further comprises a spacer between the transmembrane domain and the scFv. In some of any such embodiments, the spacer is a polypeptide spacer that comprises or consists of all or a portion of an immunoglobulin hinge or a modified version thereof, in some cases, an IgG4 hinge, or a modified version thereof.

In some of any such embodiments, the spacer is about 15 amino acids or less, and does not comprise a CD28 extracellular region or a CD8 extracellular region. In some of any such embodiments, the spacer is at or about 12 amino acids in length. In some of any such embodiments, the spacer has or consists of the sequence of SEQ ID NO: 1, a sequence encoded by SEQ ID NO: 2, SEQ ID NO: 30, SEQ ID NO: 31, SEQ ID NO: 32, SEQ ID NO: 33, SEQ ID NO: 34, or a variant of any of the foregoing having at least 85%, 86%, 87%, 88%, 89%, 90%, 91%, 92%, 93%, 94%, 95%, 96%, 97%, 98%, 99% or more sequence identity thereto; and/or the spacer comprises or consists of the formula X₁PPX₂P, where X₁ is glycine, cysteine or arginine and X₂ is cysteine or threonine.

In some of any of the provided embodiments, the CAR contains an scFv specific for the antigen, a transmembrane domain, a cytoplasmic signaling domain derived from a costimulatory molecule, which, in some cases, is or contains a 4-1BB, and a cytoplasmic signaling domain derived from a primary signaling ITAM-containing molecule, which, in some cases, is or contains a CD3zeta signaling domain and, in some cases, further contains a spacer between the transmembrane domain and the scFv; the CAR contains, in order, an scFv specific for the antigen, a transmembrane domain, a cytoplasmic signaling domain derived from a costimulatory molecule, which, in some cases, is or comprises a 4-1BB signaling domain, and a cytoplasmic signaling domain derived from a primary signaling ITAM-containing molecule, which, in some cases, is a CD3zeta signaling domain; or the CAR contains, in order, an scFv specific for the antigen, a spacer, a transmembrane domain, a cytoplasmic signaling domain derived from a costimulatory molecule, which, in some cases, is a 4-1BB signaling domain, and a cytoplasmic signaling domain derived from a primary signaling ITAM-containing molecule, which, in some cases, is or contains a CD3zeta signaling domain; and wherein: the spacer is a polypeptide spacer that (a) contains or consists of all or a portion of an immunoglobulin hinge or a modified version thereof or contains about 15 amino acids or less, and does not contain a CD28 extracellular region or a CD8 extracellular region, (b) contains or consists of all or a portion of an immunoglobulin hinge, which, in some cases, is an IgG4 hinge, or a modified version thereof and/or contains about 15 amino acids or less, and does not contain a CD28 extracellular region or a CD8 extracellular region, or (c) is at or about 12 amino acids in length and/or contains or consists of all or a portion of an immunoglobulin hinge, which, in some cases, is an IgG4, or a modified version thereof; or (d) has or consists of the sequence of SEQ ID NO: 1, a sequence encoded by SEQ ID NO: 2, SEQ ID NO: 30, SEQ ID NO: 31, SEQ ID NO: 32, SEQ ID NO: 33, SEQ ID NO: 34, or a variant of any of the foregoing having at least 85%, 86%, 87%, 88%, 89%, 90%, 91%, 92%, 93%, 94%, 95%, 96%, 97%, 98%, 99% or more sequence identity thereto, or (e) contains or consists of the formula X₁PPX₂P, where X₁ is glycine, cysteine or arginine and X₂ is cysteine or threonine; and/or the costimulatory domain contains SEQ ID NO: 12 or a variant thereof having at least 85%, 86%, 87%, 88%, 89%, 90%, 91%, 92%, 93%, 94%, 95%, 96%, 97%, 98%, 99% or more sequence identity thereto; and/or the primary signaling domain includes SEQ ID NO: 13, 14 or 15 having at least 85%, 86%, 87%, 88%, 89%, 90%, 91%, 92%, 93%, 94%, 95%, 96%, 97%, 98%, 99% or more sequence identity thereto; and/or the scFv contains an amino acid sequence of RASQDISKYLN (SEQ ID NO: 35), an amino acid sequence of SRLHSGV (SEQ ID NO: 36), and/or an amino acid sequence of GNTLPYTFG (SEQ ID NO: 37) and/or an amino acid sequence of DYGVS (SEQ ID NO: 38), an amino acid sequence of VIWGSETTYYNSALKS (SEQ ID NO: 39), and/or an amino acid sequence of YAMDYWG (SEQ ID NO: 40) or wherein the scFv comprises a variable heavy chain region of FMC63 and a variable light chain region of FMC63 and/or a CDRL1 sequence of FMC63, a CDRL2 sequence of FMC63, a CDRL3 sequence of FMC63, a CDRH1 sequence of FMC63, a CDRH2 sequence of FMC63, and a CDRH3 sequence of FMC63 or binds to the same epitope as or competes for binding with any of the foregoing, and in some cases wherein the scFv contains, in order, a V_{H}, a linker, which, in some cases, includes SEQ ID NO: 24, and a V_{L}, and/or the scFv contains a flexible linker and/or contains the amino acid sequence set forth as SEQ ID NO: 24.

In some of any of the provided embodiments, the antigen is a B cell antigen. In some of any such embodiments, the antigen is CD19. In some of any of the provided embodiments, prior to the administration, the subject has been preconditioned with a lymphodepleting therapy including the administration of fludarabine and/or cyclophosphamide. In some of any of the provided embodiments, immediately prior to the administration, administering a lymphodepleting therapy to the subject including the administration of fludarabine and/or cyclophosphamide. In some of any of the provided embodiments, the administration of the cell dose and/or the lymphodepleting therapy is carried out via outpatient delivery; and if the subject exhibits a sustained fever or a fever that is or has not been reduced or not reduced by more than 1°C after treatment with an antipyretic, the subject is admitted to the hospital or to an overnight stay at a hospital and/or is administered an agent or treatment for the treatment or prevention or reduction or attenuation of a neurotoxicity and/or a cytokine release syndrome or risk thereof.

In some of any of the provided embodiments, the dose of cells is administered parenterally. In some of any such embodiments, the dose of cells is administered intravenously. In some of any of the provided embodiments, the T cells are primary T cells obtained from a subject. In some of any of the provided embodiments, the T cells are autologous to the subject. In some of any of the provided embodiments, the subject is a human subject.

Also provided herein are articles of manufacture containing a composition containing genetically engineered cells expressing a recombinant receptor. In some of any such embodiments, the articles of manufacture also contains instructions for administering a dose of the cells in accord with any of the methods provided herein.

Provided herein are methods of assessing the risk of developing a toxicity after administration of a cell therapy containing: (a) assessing one or more parameters in a subject, wherein the one or more parameters is selected from the level, amount or concentration of LDH, ferritin or C-reactive protein (CRP) in a biological sample from the subject, or is a sum of product dimensions (SPD) of a tumor in the subject; wherein the subject is a candidate for treatment with the cell therapy, said cell therapy containing a dose of genetically engineered cells expressing a recombinant receptor; and wherein the assessing is carried out prior to administering the cell therapy and/or said biological sample or tumor does not contain the recombinant receptor and/or said engineered cells; and (b) identifying if the subject is as at risk of toxicity wherein the subject is at risk of toxicity if the one or more parameters is above a threshold level and the subject is not at risk of toxicity if the one or more parameters is at or below a threshold level, wherein: (i) the parameter is a sum of product dimensions (SPD), and the threshold level is above at or about 30 cm², 40 cm², 50 cm², 60 cm² or 70 cm²; (ii) the parameter is LDH, and the threshold level is above at or about 300 units per liter, 400 units per liter, 500 units per liter or 600 units per liter; (iii) the parameter is ferritin, and the threshold level is above at or about 1000 nanograms per milliliter, 2000 nanograms per milliliter, 3000 nanograms per milliliter, 4000 nanograms per milliliter, 5000 nanograms per milliliter, 6000 nanograms per milliliter, 7000 nanograms per milliliter or 8000 nanograms per milliliter; and/or (iv) the parameter is CRP, and the threshold level is above at or about 5 milligrams per liter, 10 milligrams per liter, 15 milligrams per liter, 20 milligrams per liter, 25 milligrams per liter, 30 milligrams per liter, 40 milligrams per liter or 50 milligrams per liter.

Also provided are methods of identifying a subject, the method containing: (a) assessing one or more parameters in a subject, wherein the one or more parameters is selected from the level, amount or concentration of LDH, ferritin or C-reactive protein (CRP) in a biological sample from the subject, or is a sum of product dimensions (SPD) of a tumor in the subject; wherein the subject is a candidate for treatment with the cell therapy, said cell therapy containing a dose of genetically engineered cells expressing a recombinant receptor; and wherein the assessing is carried out prior to administering the cell therapy and/or said biological sample or tumor does not contain the recombinant receptor and/or said engineered cells; and (b) identifying a subject who has a risk of developing a toxicity after administration of a cell therapy, wherein the subject is at risk of toxicity if the one or more parameters is above a threshold level and the subject is not at risk of toxicity if the one or more parameters is at or below a threshold level, wherein:(i) the parameter is a sum of product dimensions (SPD), and the threshold level is above at or about 30 cm², 40 cm², 50 cm², 60 cm² or 70 cm²; (ii) the parameter is LDH, and the threshold level is above at or about 300 units per liter, 400 units per liter, 500 units per liter or 600 units per liter; (iii) the parameter is ferritin, and the threshold level is above at or about 1000 nanograms per milliliter, 2000 nanograms per milliliter, 3000 nanograms per milliliter, 4000 nanograms per milliliter, 5000 nanograms per milliliter, 6000 nanograms per milliliter, 7000 nanograms per milliliter or 8000 nanograms per milliliter; and/or (iv) the parameter is CRP, and the threshold level is above at or about 5 milligrams per liter, 10 milligrams per liter, 15 milligrams per liter, 20 milligrams per liter, 25 milligrams per liter, 30 milligrams per liter, 40 milligrams per liter or 50 milligrams per liter.

Also provided herein are methods of treatment, containing: (a) assessing one or more parameters in a subject, wherein the one or more parameters is selected from the level, amount or concentration of LDH, ferritin or C-reactive protein (CRP) in a biological sample from the subject, or is a sum of product dimensions (SPD) of a tumor in the subject; wherein the subject is a candidate for treatment with the cell therapy, said cell therapy containing a dose of genetically engineered cells expressing a recombinant receptor; and wherein the assessing is carried out prior to administering the cell therapy and/or said biological sample or tumor does not contain the recombinant receptor and/or said engineered cells; and (b) identifying if the subject is as at risk of toxicity wherein the subject is at risk of toxicity if the one or more parameters is above a threshold level and the subject is not at risk of toxicity if the one or more parameters is at or below a threshold level, wherein: (i) the parameter is a sum of product dimensions (SPD), and the threshold level is above at or about 30 cm², 40 cm², 50 cm², 60 cm² or 70 cm²; (ii) the parameter is LDH, and the threshold level is above at or about 300 units per liter, 400 units per liter, 500 units per liter or 600 units per liter; (iii) the parameter is ferritin, and the threshold level is above at or about 1000 nanograms per milliliter, 2000 nanograms per milliliter, 3000 nanograms per milliliter, 4000 nanograms per milliliter, 5000 nanograms per milliliter, 6000 nanograms per milliliter, 7000 nanograms per milliliter or 8000 nanograms per milliliter; and/or (iv) the parameter is CRP, and the threshold level is above at or about 5 milligrams per liter, 10 milligrams per liter, 15 milligrams per liter, 20 milligrams per liter, 25 milligrams per liter, 30 milligrams per liter, 40 milligrams per liter or 50 milligrams per liter; and (c) following or based on the results of the assessment, administering to the subject the cell therapy. In some of any such embodiments, the methods also involve administering an agent or other treatment capable of treating, preventing, delaying, reducing or attenuating the development or risk of development of a toxicity.

In some of any of the provided embodiments, the parameter in the provided methods is SPD, and the threshold level is 50 cm². In some of any of the provided embodiments, the parameter in the provided methods is LDH, and the threshold level is 500 units per liter. In some of any of the provided embodiments, the one or more parameter in the provided methods is SPD and LDH, and the threshold level for SPD is 50 cm² and the threshold level for LDH is 500 units per liter. In some of any of the provided embodiments, the parameter in the provided methods is ferritin, and the threshold level is 5000 nanograms per milliliter. In some of any of the provided embodiments, the parameter in the provided methods is CRP, and the threshold level is 10 milligrams per liter. In some of any of the provided embodiments, the one or more parameter in the methods is ferritin and CRP, and the threshold level for ferritin is 5000 nanograms per milliliter and the threshold level for CRP is 10 milligrams per liter.

In some of any of the provided embodiments, the biologic sample in the provided methods is a blood or plasma sample. In some of any of the provided embodiments, the SPD in the provided methods is measured based on CT and/or MRI imaging or other imaging of the body. In some of any of the provided embodiments, the level, amount or concentration of one or more analyte and/or the SPD is measured prior to treatment, prior to apheresis, or prior to cell product manufacturing.

Also provided herein are methods of assessing the risk of developing a toxicity after administration of a cell therapy, the method containing: (a) assessing the peak concentration of genetically engineered cells expressing a recombinant receptor in a biological sample from a subject that has been previously administered a cell therapy containing the genetically engineered cells; and (b) identifying if the subject is as at risk of toxicity wherein the subject is at risk of toxicity if the one or more parameters, such as the peak concentration of genetically engineered cells, is above a threshold level and the subject is not at risk of toxicity if the peak concentration of genetically engineered cells is at or below a threshold level, wherein: the threshold level is above at or about 300 cells per microliter, 400 cells per microliter, 500 cells per microliter, 600 cells per microliter, 700 cells per microliter, 800 cells per microliter, 900 cells per microliter or 1000 cells per microliter.

Also provided herein are methods of identifying a subject, the method containing: (a) assessing the peak concentration of genetically engineered cells expressing a recombinant receptor in a biological sample from a subject that has been previously administered a cell therapy containing the genetically engineered cells; and (b) identifying a subject who has a risk of developing a toxicity after administration of a cell therapy, wherein the subject is at risk of toxicity if the one or more parameters, such as the peak concentration of genetically engineered cells, is above a threshold level and the subject is not at risk of toxicity if the peak concentration of genetically engineered cells is at or below a threshold level, wherein: the threshold level is above at or about 300 cells per microliter, 400 cells per microliter, 500 cells per microliter, 600 cells per microliter, 700 cells per microliter, 800 cells per microliter, 900 cells per microliter or 1000 cells per microliter.

Also provided herein are methods of treatment, containing: (a) assessing the peak concentration of genetically engineered cells expressing a recombinant receptor in a biological sample from a subject that has been previously administered a cell therapy containing the genetically engineered cells; and (b) identifying if the subject is as at risk of toxicity wherein the subject is at risk of toxicity if the one or more parameters, such as the peak concentration of genetically engineered cells, is above a threshold level and the subject is not at risk of toxicity if the peak concentration of genetically engineered cells is at or below a threshold level, wherein: the threshold level is above at or about 300 cells per microliter, 400 cells per microliter, 500 cells per microliter, 600 cells per microliter, 700 cells per microliter, 800 cells per microliter, 900 cells per microliter or 1000 cells per microliter; and (c) following or based on the results of the assessment, administering to the subject the cell therapy. In some of any such embodiments, the methods also involve administering an agent or other treatment capable of treating, preventing, delaying, reducing or attenuating the development or risk of development of a toxicity. In some of any of the provided embodiments, the threshold level in the provided methods is 500 cells per microliter.

Also provided herein are methods further containing monitoring the subject for symptoms of toxicity if the subject is administered a cell therapy and is identified as having a risk of developing a toxicity.

In some of any of the provided embodiments, the toxicity is neurotoxicity or CRS. In some of any of the provided embodiments, the toxicity is a grade 1 or higher neurotoxicity or CRS. In some of any of the provided embodiments, the toxicity is a severe neurotoxicity or is a grade 2 or higher neurotoxicity, a grade 3 or higher neurotoxicity, or a grade 4 or higher neurotoxicity. In some of any of the provided embodiments, the toxicity is a grade 1 or higher neurotoxicity. In some of any of the provided embodiments, the toxicity is a severe neurotoxicity or a grade 3 or higher neurotoxicity. In some of any of the provided embodiments, the toxicity is a severe CRS or is a grade 2 or higher CRS, a grade 3 or higher CRS, or a grade 4 or higher CRS. In some of any of the provided embodiments, the toxicity is a grade 1 or higher CRS. In some of any of the provided embodiments, the toxicity is a severe CRS or a grade 3 or higher CRS.

Also provided herein are methods of treatment, wherein if the subject is identified as having a risk of developing a toxicity, administering to the subject: (a) (1) an agent or other treatment capable of treating, preventing, delaying, reducing or attenuating the development or risk of development of a toxicity and (2) the cell therapy, wherein administration of the agent is to be administered (i) prior to, (ii) within one, two, or three days of, (iii) concurrently with and/or (iv) at first fever following, the initiation of administration of the cell therapy to the subject; and/or (b) a cell therapy at a reduced dose or at a dose that is not associated with risk of developing toxicity or severe toxicity, or is not associated with a risk of developing a toxicity or severe toxicity in a majority of subjects, and/or a majority of subjects having a disease or condition that the subject has or is suspected of having, following administration of the cell therapy; and/or (c) administering to the subject a cell therapy in an inpatient setting and/or with admission to the hospital for one or more days. In some of any such embodiments, the cell therapy is otherwise to be administered to subjects on an outpatient basis or without admission to the hospital for one or more days.

In some of any of the provided embodiments the agent or other treatment is an anti-IL-6 antibody or an anti-IL-6 receptor antibody. In some of any of the provided embodiments, the agent or other treatment is or contains an agent selected from among tocilizumab, siltuximab, clazakizumab, sarilumab, olokizumab (CDP6038), elsilimomab, ALD518/BMS-945429, sirukumab (CNTO 136), CPSI-2634, ARGX-109, FE301 and FM101. In some of any of the provided embodiments, the agent or other treatment is tocilizumab. In some of any of the provided embodiments, the agent or other treatment is or contains one or more steroids. In some of any of the provided embodiments, the steroid is dexamethasone or methylprednisolone. In some of any of the provided embodiments, the steroid is dexamethasone. In some of any of the provided embodiments, the agent or other treatment contains administration of a vasopressor. In some of any of the provided embodiments, the agent or other treatment includes intubation. In some of any of the provided embodiments, the agent or other treatment includes dialysis.

In some of any of the provided embodiments, the recombinant receptor specifically binds to an antigen (e.g., target antigen) associated with the disease or condition or expressed in cells of the environment of a lesion associated with the disease or condition. In some of any such embodiments, the antigen is a B cell antigen. In some of any such embodiments, the antigen is CD19. In some of any of the provided embodiments, the disease or condition is a cancer. In some of any of the provided embodiments, the disease or condition is a myeloma, a leukemia or a lymphoma. In some of any of the provided embodiments, the disease or condition is a B cell malignancy and/or is acute lymphoblastic leukemia (ALL), adult ALL, chronic lymphoblastic leukemia (CLL), non-Hodgkin lymphoma (NHL), or a large B cell lymphoma. In some of any such embodiments, the disease or condition is a large B cell lymphoma.In some of any of the provided embodiments, the disease or condition, such as the large B cell lymphoma, is a Diffuse Large B-Cell Lymphoma (DLBCL). In some of any of the provided embodiments, the DLBCL is a DLBCL, not otherwise specified (NOS), a de novo DLBCL or a DLBCL transformed from indolent lymphoma. In some of any of the provided embodiments, the DLBCL is a de novo DLBCL. In some of any of the provided embodiments, the DLBCL is a DLBCL transformed from follicular lymphoma (tFL). In some of any of the provided embodiments, the DLBCL is a DLBCL transformed from marginal zone lymphoma (tMZL) or a DLBCL transformed from chronic lymphocytic leukemia (tCLL; Richter's). In some of any of the provided embodiments, the disease or condition is primary mediastinal B-cell lymphoma (PMBCL) or a follicular lymphoma (FL), such as follicular lymphoma grade 3B (FL3B). In some of any embodiments, the disease or condition is follicular lymphoma (FL). In some of any embodiments, the disease or condition is follicular lymphoma (FL). In some of any embodiments, the FL is associated with co-expression of CD10, BCL6 and BCL2 within the follicles, and/or t(14;18)/(q32;q21) (IGH-BCL2) and/or BCL6 rearrangements. In some of any embodiments, the disease or condition is mantle cell lymphoma (MCL).

In some of any of the provided embodiments, the recombinant receptor is a chimeric antigen receptor (CAR). In some of any of the provided embodiments, the engineered cells comprise T cells, such as CD4⁺ T cells and/or CD8⁺ T cells. In some of any of the provided embodiments, the cell therapy includes administering a dose of CD4⁺ and CD8⁺ T cells to a subject, wherein T cells of each dose comprises a receptor that specifically binds to an antigen, e.g., target antigen, expressed by the disease or condition or a cell or tissue thereof and/or that is associated with the disease or condition, wherein the administration includes administering a plurality of separate compositions, the plurality of separate compositions containing a first composition containing CD8⁺ T cells and a second composition containing CD4⁺ T cells.

In some of any of the provided embodiments, the initiation of the administration of the first composition is carried out prior to the initiation of the administration of the second composition. In some of any of the provided embodiments, the administration of the first composition and the administration of the second composition are carried out no more than 48 hours apart. In some of any of the provided embodiments, the administration of the first composition and the administration of the second composition are carried out no more than 36 hours apart, no more than 24 hours apart, no more than 12 hours apart, no more than 6 hours apart, no more than 4 hours apart, no more than 2 hours apart, no more than 1 hour apart or no more than 30 minutes apart.

In some of any of the provided embodiments, the receptor contained by the CD4⁺ T cells and/or the receptor contained by the CD8⁺ T cells includes a recombinant receptor that is the same and/or wherein the CD4⁺ T cells and/or the CD8⁺ T cells are genetically engineered to express a recombinant receptor that is the same.

In some of any of the provided embodiments, the dose of CD4⁺ and CD8⁺ T cells contains a defined ratio of CD4⁺ cells expressing a recombinant receptor to CD8⁺ cells expressing a recombinant receptor and/or of CD4⁺ cells to CD8⁺ cells, that is or is approximately 1:1 or is between approximately 1:3 and approximately 3:1; and/or the CD4⁺ T cells containing the receptor in the one of the first and second compositions and the CD8⁺ T cells containing the receptor in the other of the first and second compositions are present at a defined ratio that is or is approximately 1:1 or is between approximately 1:3 and approximately 3:1; and/or the CD4⁺ T cells containing the receptor and the CD8⁺ T cells containing the receptor administered in the first and second compositions are present at a defined ratio, which ratio is or is approximately 1:1 or is between approximately 1:3 and approximately 3:1. In some of any of the provided embodiments, the defined ratio is or is approximately 1:1.

In some of any of the provided embodiments, the dose of CD4⁺ and CD8⁺ T cells contains: between at or about 1 x 10⁷ and at or about 2 x 10⁸ total recombinant receptor-expressing T cells, inclusive; between at or about 2.5 x 10⁷ and at or about 1.5 x 10⁸ total recombinant receptor-expressing T cells, inclusive; between at or about 5 x 10⁷ and at or about 1 x 10⁸ total recombinant receptor-expressing T cells, inclusive; at or about 5 x 10⁷ total recombinant receptor-expressing T cells; at or about 1 x 10⁸ total recombinant receptor-expressing T cells; or at or about 1.5 x 10⁸ total recombinant receptor-expressing T cells. In some of any such embodiments, the dose of CD4⁺ and CD8⁺ T cells comprises at or about 5 x 10⁷ total recombinant receptor-expressing T cells. In some of any such embodiments, the dose of CD4⁺ and CD8⁺ T cells comprises at or about 1 x 10⁸ total recombinant receptor-expressing T cells. In some of any such embodiments, the dose of CD4⁺ and CD8⁺ T cells comprises at or about 1.5 x 10⁸ total recombinant receptor-expressing T cells.

In some of any of the provided embodiments, the dose of CD4⁺ and CD8⁺ T cells contains: between at or about 1 x 10⁷ and at or about 1 x 10⁸ recombinant receptor-expressing CD8⁺ T cells, inclusive; between at or about 1.25 x 10⁷ and at or about 7.5 x 10⁷ recombinant receptor-expressing CD8⁺ T cells, inclusive; between at or about 2.5 x 10⁷ and at or about 5 x 10⁷ recombinant receptor-expressing CD8⁺ T cells, inclusive; at or about 2.5 x 10⁷ recombinant receptor-expressing CD8⁺ T cells; at or about 5 x 10⁷ recombinant receptor-expressing CD8⁺ T cells; or at or about 7.5 x 10⁷ recombinant receptor-expressing CD8⁺ T cells. In some of any such embodiments, the dose of CD4⁺ and CD8⁺ T cells comprises at or about 2.5 x 10⁷ recombinant receptor-expressing CD8⁺ T cells. In some of any such embodiments, the dose of CD4⁺ and CD8⁺ T cells comprises at or about 5 x 10⁷ recombinant receptor-expressing CD8⁺ T cells. In some of any such embodiments, the dose of CD4⁺ and CD8⁺ T cells comprises at or about 7.5 x 10⁷ recombinant receptor-expressing CD8⁺ T cells.

In some of any of the provided embodiments, the T cells are primary T cells obtained from a subject or are autologous to the subject.

Provided herein are articles of manufacture containing a composition including genetically engineered cells expressing a recombinant receptor; and instructions for assessing the risk of developing a toxicity, identifying a subject or treating a subject in accord with any of the methods provided herein. In some of any such embodiments, the articles of manufacture also contain an agent or other treatment capable of treating, preventing, delaying, reducing or attenuating the development or risk of development of a toxicity

### Brief Description of the Drawings

**FIG. 1** shows the percentage of subjects who experienced laboratory abnormalities and treatment-emergent adverse events (TEAEs) that occurred in ≥20% of subjects. *: One Grade 5 AE of multi-organ failure unrelated to study treatment and due to progression of lymphoma; †: One Grade 5 AE of diffuse alveolar damage, investigator assessed as related to fludarabine, cyclophosphamide, and CAR T cell therapy, occurred on day 23 in a subject who refused mechanical ventilation for progressive respiratory failure while neutropenic on growth factors and broad spectrum antibiotics and antifungals

**FIG. 2** is a Kaplan meier curve depicting observed time to onset of CRS and neurotoxicity.

**FIG. 3A** and **FIG. 3B** depict 3 month objective response rates (ORR) among subgroups of treated subjects.

**FIG. 4A** and **FIG. 4B** show the duration of response (CR/PR, CR or PR) and overall survival in the full and core cohort of subjects.

**FIG. 5A** shows the pharmacokinetics of the CAR⁺ T cells in peripheral blood at various time points post-treatment at different dose levels. **FIG. 5B** shows the pharmacokinetics of the CAR⁺ T cells in peripheral blood at various time points post-treatment between responders and nonresponders. **FIG. 5C** shows the pharmacokinetics of the CAR⁺ T cells in peripheral blood at various time points post-treatment in subjects that did or did not develop any neurotoxicity.

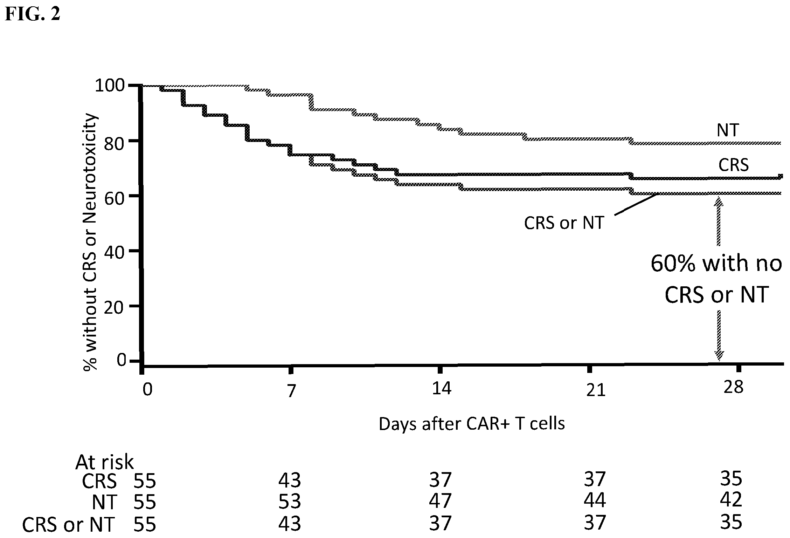

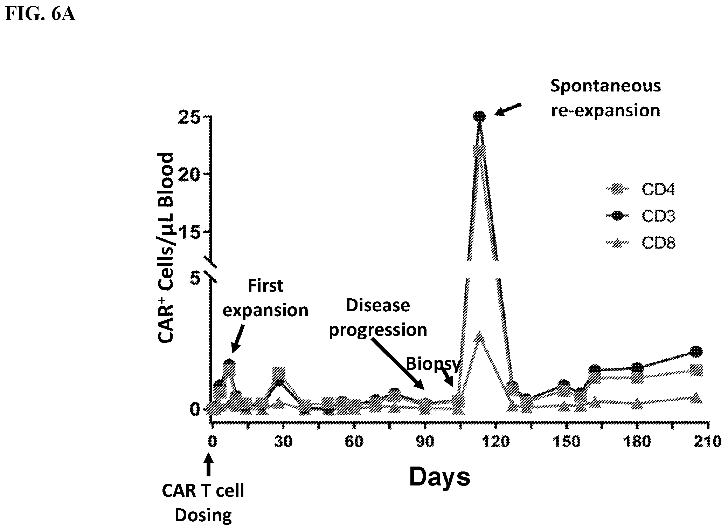

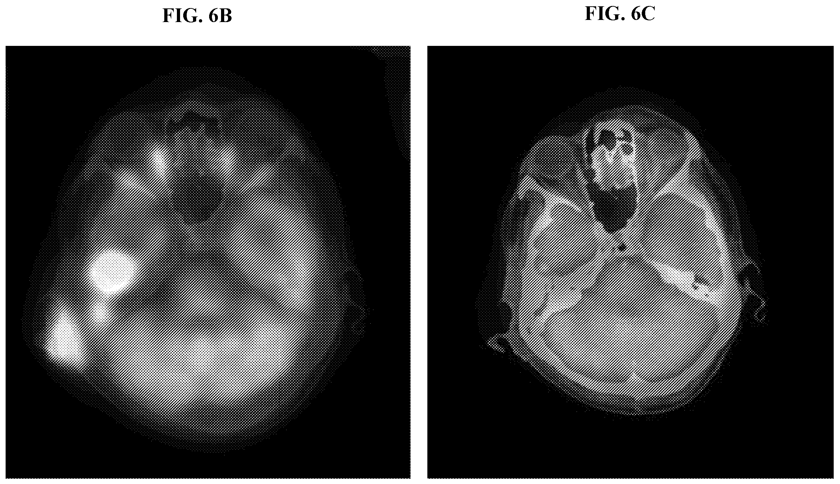

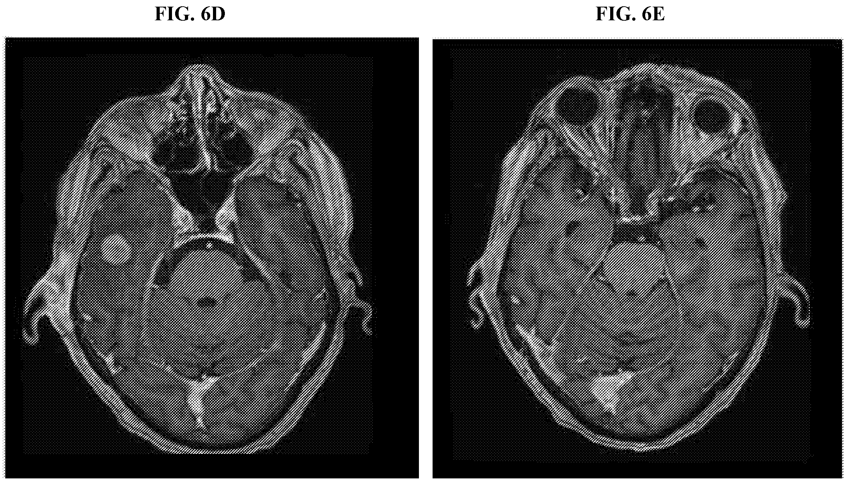

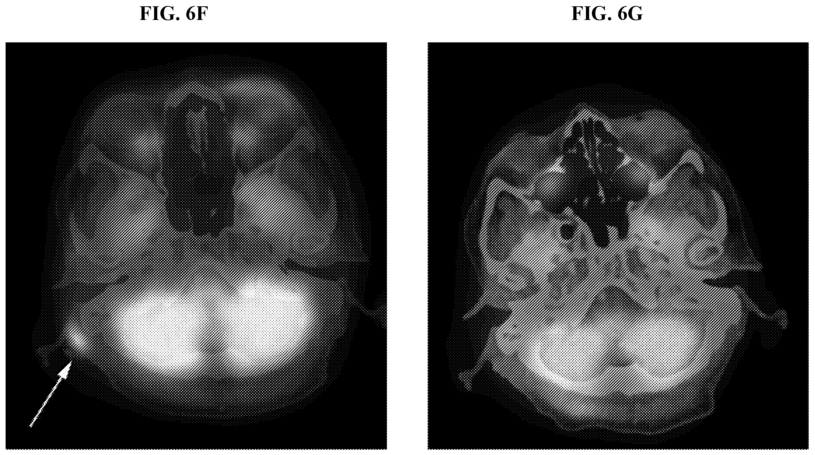

**FIG. 6A** shows the number of CD3⁺/CAR⁺, CD4⁺/CAR⁺, CD8⁺/CAR⁺ T cells in peripheral blood of a subject with chemorefractory transformed DLBCL measured at certain time points. **FIG. 6B** depicts a pretreatment axial PET-CT image showing an intracranial abnormality in the right middle cranial foss and extensive abnormality in subcutaneous tissues in the right posterior auricular region. **FIG. 6C** is a post-treatment PET-CT image depicting resolution of the abnormality in FIG. 2B after treatment with anti-CD19 CAR⁺ T cells. **FIG. 6D** is a pretreatment brain MRI (high-resolution T₁-weighted image with the use of contrast material; axial view) showing a homogeneously enhancing mass in the right middle cranial fossa. **FIG. 6E** is a post-treatment MRI image showing near-complete resolution of the enhancing mass. **FIG. 6F** is an axial PET-CT image at relapse showing right posterior auricular tumor recurrence associated with intense uptake of ¹⁸F-flurodeoxyglycose (arrow). **FIG. 6G** is a PET-CT imaging showing resolution of the posterior auricular tumor after incisional biopsy and re-expansion of CAR⁺ T cells.

**FIG. 7** shows levels of analytes measured in the serum of subjects prior to administration of the CAR⁺ T cells and correlation to the development of neurotoxicity.

**FIG. 8** shows a graph plotting progression-free time (months) and indicating best overall response and response durability, and individual clinical outcomes observed over time in individual subjects within a Full cohort and a Core cohort of NHL subjects treated with an anti-CD19 cell therapy containing CAR-T-expressing CD4⁺ and CD8⁺ T cells. ^{a} : Patients achieved BOR at month 1 except where otherwise noted; ^{b} :Complete resolution of CNS involvement by lymphoma observed in 2 patients; ^{c} : One patient re-expanded after biopsy upon disease progression

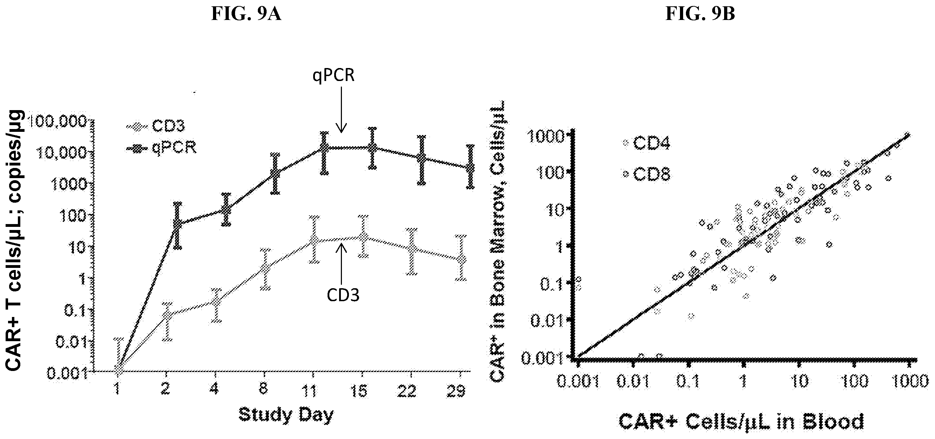

**FIG. 9A** depicts the median (±quartiles) number of CAR-expressing CD3⁺ cells/µL blood, assessed by flow cytometry using an antibody specific for a truncated receptor (CD3, circle; N=87); or median (±quartiles) number of copies integrated CAR transgene/µg genomic DNA, assessed by quantitative polymerase chain reaction (qPCR) using primers specific for a woodchuck hepatitis virus post-transcriptional regulatory element (WPRE) present in the vector encoding the CAR (qPCR, square; N=85) in blood samples from 87 subjects that have been administered anti-CD19 CAR-expressing cells. The cutoff for CAR⁺ cell detection in flow cytometry was set at ≥ 25 events in the CAR⁺ gate, and limit of detection for qPCR was ≥ 12.5 copies of CAR transgene per µg of genomic DNA. **FIG. 9B** depicts the relative numbers of CD4⁺and CD8⁺CAR-expressing cells/µL in blood and bone marrow samples from 67 subjects that have been administered anti-CD19 CAR-expressing cells, on day 11 ± 3 days. The line represents the line of unity and is not a regression line.

**FIGS. 10A** and **10B** depict the median (±quartiles) area under the curve between days 0 and 28 (AUC₀₋₂₈; **FIG. 10A**) and maximum serum concentration (Cₘₐₓ; CAR⁺ cells/µL blood; **FIG. 10B**) of CD4⁺ and CD8⁺ CAR⁺ cells in subject subgroups with diffuse large B-cell lymphoma de novo or transformed from indolent lymphoma (DLBCL, NOS; N=27), transformed follicular lymphoma (tFL; N=10), DLBCL transformed from marginal zone lymphoma or chronic lymphocytic leukemia (tMZL/tCLL; N=4), or mantle cell lymphoma (MCL; N-5), who have received CAR-expressing T cells at DL1.

**FIGS. 11A** and **11B** depict the median (±quartiles) area under the curve between days 0 and 28 (AUC₀₋₂₈; **FIG. 11A**) and maximum serum concentration (Cₘₐₓ; CAR⁺ cells/µL blood; **FIG. 11B**) of CD3⁺, CD4⁺ and CD8⁺ CAR⁺ cells in subjects who have received CAR⁺ cells at DL1 or DL2.

**FIGS. 12A-12D** depict the median (±quartiles) number of CAR-expressing CD4⁺ and CD8⁺ CAR⁺ cells/µL blood over time, in subjects that developed cytokine release syndrome (any CRS) compared to subjects that have not developed CRS (no CRS) (CD4⁺: **FIG. 12A****;** CD8⁺: **FIG. 12B**) or in subjects that developed neurotoxicity (any NT) compared to subjects that have not developed NT (no NT) (CD4⁺: **FIG. 12C****;** CD8⁺: **FIG. 12D**).

**FIGS. 13A** and **13B** depict the number of peak CD3⁺ CAR⁺ cells/µL (CD3⁺ Cₘₐₓ) in subjects grouped by subjects who had the best overall response (BOR) of CR, PR or PD, or a 3-month (M3) durable response of CR, PR or PD.

**FIG. 14A** depicts pre-lymphodepletion blood analyte levels in serum samples from subjects that exhibited high CAR⁺ cell expansion (CD3⁺ Cₘₐₓ > 500) and subjects that exhibited low CAR⁺ cell expansion (CD3⁺ Cₘₐₓ < 500). **FIG. 14B** depicts the peak blood analyte levels in serum samples from subjects that exhibited high CAR⁺ cell expansion (CD3⁺ Cₘₐₓ > 500) and subjects that exhibited low CAR⁺ cell expansion (CD3⁺ Cₘₐₓ < 500).

**FIG. 15** depicts a plot depicting pre-lymphodepletion SPD (cm²) against AUC₀₋₂₈ (cells*day/µL) of CD3⁺ CAR⁺ cells, for individual subjects administered DL1 or DL2 of CAR⁺ cells.

**FIGS. 16A** and **16B** depict pre-lymphodepletion blood analyte levels in serum samples from subjects that developed cytokine release syndrome (CRS grade 1-4) compared to subjects that have not developed CRS (CRS grade 0) (**FIG. 16A**) or in subjects that developed neurotoxicity (NT grade 0) compared to subjects that have not developed NT (NT grade 1-4) (**FIG. 16B**). The units were: Ferritin and D-dimer (µg/L); CRP (mg/L) and cytokines (pg/mL).

**FIG. 17** depicts the assessment of pre-lymphodepletion patient parameter sum of product dimensions (SPD; cm²), indicative of tumor burden, and lactate dehydrogenase (LDH; U/L) level, in subjects that developed cytokine release syndrome (any CRS) compared to subjects that have not developed CRS (no CRS) or in subjects that developed neurotoxicity (any NT) compared to subjects that have not developed NT (no NT).

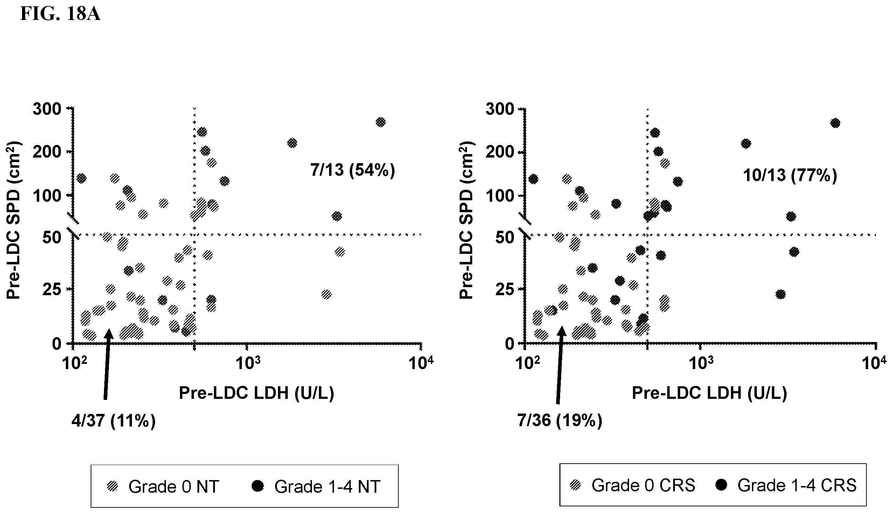

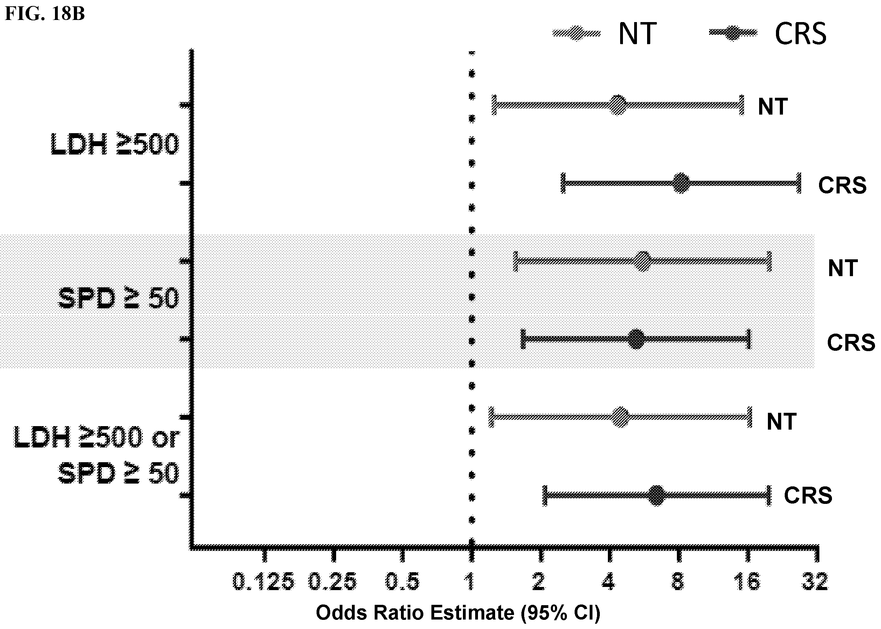

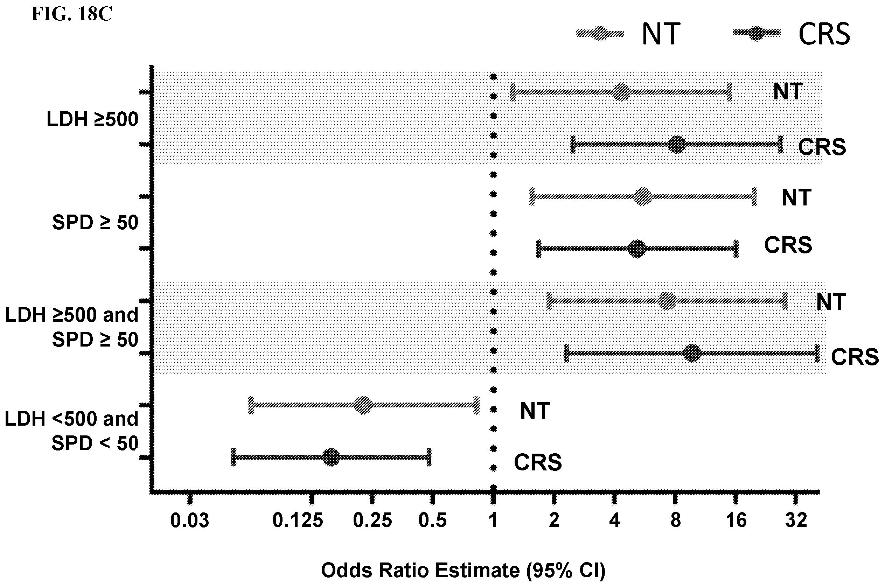

**FIG. 18A** is a plot depicting pre-lymphodepletion SPD (cm²) against pre-lymphodepletion LDH (U/L) levels, in individuals that have developed neurotoxicity (Grade 1-4 NT) or subjects that have not developed NT (Grade 0 NT) (left panel), and in individuals that have developed CRS (Grade 1-4 CRS) or subjects that have not developed CRS (Grade 0 CRS) (right panel). Dotted lines represent levels of SPD (50 cm² or higher) or LDH (500 U/L or higher) that is associated with higher rates of CRS or NT. **FIG. 18B** depicts the odds ratio estimates for developing CRS or NT based on the levels of SPD (50 cm² or higher) or LDH (500 U/L or higher), with 95% confidence intervals (CI). **FIG. 18C** depicts the odds ratio estimates for developing CRS or NT based on the levels of SPD or LDH, including the odds ratio estimates for values lower than the threshold, with 95% confidence intervals (CI).

**FIG. 19** depicts pre-lymphodepletion tumor burden parameter (SPD) and blood analyte levels in for subjects that had a durable response at 3 months versus for subjects that did not have a response at 3 months. The units were: Ferritin and D-dimer (µg/L); CRP and SAA-1 (mg/L) and cytokines (pg/mL).

**FIGS. 20A** and **20B** depict peak blood analyte levels in serum samples from subjects that developed cytokine release syndrome (any CRS) compared to subjects that have not developed CRS (no CRS) (**FIG. 20A**) or in subjects that developed neurotoxicity (any NT) compared to subjects that have not developed NT (no NT) (**FIG. 20B**). The units were: CRP (mg/L), SAA-1 (mg/L) and cytokines (pg/mL).

**FIG. 21A** depicts peak blood analyte levels in serum samples from subjects that had a best overall response (BOR) of complete response (CR) or partial response (PR) (N=57) compared to levels in subjects that had stable disease (SD) or progressive disease (PD) (N=17). **FIG. 21B** depicts peak blood analyte levels in serum samples from subjects that had a 3-month response of SD/PD (N=31), compared to subjects who had a 3-month response CR/PR (N=35). The units were: CRP (mg/L), SAA-1 (mg/L) and cytokines (pg/mL).

**FIG. 22** depicts month 3 objective response rates (ORR) among subgroups of treated subjects, with the 95% confidence interval.

**FIGS. 23A** and **23B** depict the duration of response (DOR) for the full cohort (**FIG. 23A**) and the core cohort (**FIG. 23B**), and **FIGS. 23C** and **23D** depict the overall survival for the full cohort (**FIG. 23C**) and the core cohort (**FIG. 23D**), for subjects who achieved CR, PR, all subjects that showed a response, non-responders, and all treated subjects. Median F/U was 6.3 months for duration of response.

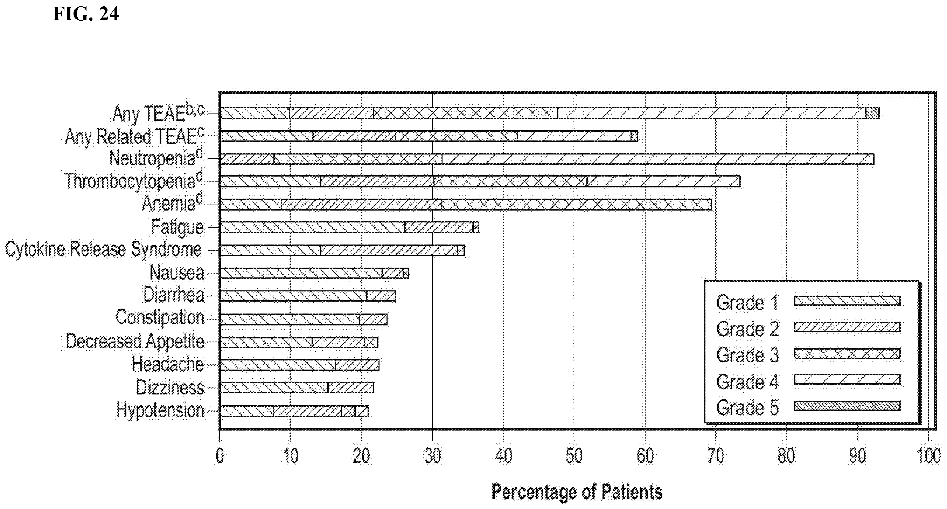

**FIG. 24** shows the percentage of subjects who experienced treatment-emergent adverse events (TEAEs) in the FULL DLBCL cohort occurring in ≥20% of patients. Data for 5 patients with MCL treated with conforming product at DL1 with at least 28 days of follow-up were not included. ^{b} : One grade 5 AE of septic shock unrelated to CAR⁺ T cell administration. ^{c} : One grade 5 AE of diffuse alveolar damage, investigator assessed as related to fludarabine, cyclophosphamide, and CAR⁺ T cells, occurred on day 23 in a patient who refused mechanical ventilation for progressive respiratory failure while neutropenic on growth factors and broad-spectrum antibiotics and antifungals. ^{d} : Laboratory anomalies.

**FIG. 25** shows the percentage of subjects who developed CRS or neurotoxicity over time, in the full cohort.

**FIG. 26** shows box plots displaying the T cell purity of T cell compositions enriched for CD4⁺ and CD8⁺ cells at different stages of the process for generating engineered cell compositions containing CAR T cells that is described in Example 8. The frequency (% of total leukocytes) of CD4⁺ and CD8⁺ cells in the compositions are shown.

**FIGS. 27A-27C** show box plots displaying the concentration (**FIG. 27A**), viability (**FIG. 27B**), and frequency of caspase-3 negative (**FIG. 27C**) CD4⁺ and CD8⁺ CAR⁺ T cells in therapeutic cell compositions of a high or low formulation volume.

**FIG. 28A** shows the number of CD3⁺ CAR⁺ T cells present in CAR T cell compositions for administration at DL1 and DL2. **FIG. 28B** shows the number of CD4⁺CAR⁺ and CD8⁺CAR⁺ cells, and CD4⁺CAR⁺TNF-α⁺ cells and CD8⁺CAR⁺TNF-α⁺ cells present in CAR T cell compositions for administration at DL1 and DL2.

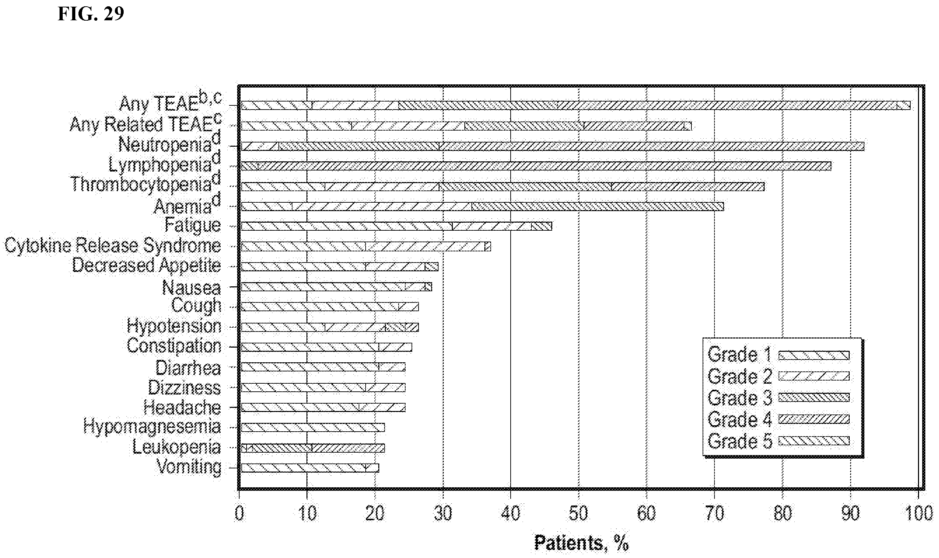

**FIG. 29** shows the percentage of subjects who experienced treatment-emergent adverse events (TEAEs) in the FULL DLBCL cohort occurring in ≥20% of the subject at a study time point described in Example 6. Data for 6 subjects with MCL treated with conforming product at DL1 with at least 28 days of follow-up were not included. ^{b} : One grade 5 AE of septic shock unrelated to CAR⁺ T cell administration, occurred in the setting of disease progression. ^{c} : One grade 5 AE of diffuse alveolar damage, investigator assessed as related to fludarabine, cyclophosphamide, and CAR⁺ T cells, occurred on day 23 in a patient who refused mechanical ventilation for progressive respiratory failure while neutropenic on growth factors and broad-spectrum antibiotics and antifungals. ^{d} : Laboratory anomalies.

**FIG. 30** depict the six (6) month objective response rates (ORR) among subgroups of treated subjects, with the 95% confidence interval. ^{a} Includes all DLBCL subjects treated at all dose levels in the CORE cohort.

**FIGS. 31A** and **31B** depict the duration of response (DOR) for the full cohort (**FIG. 31A**) and the core cohort (**FIG. 31B**), and **FIGS. 31C** and **31D** depict the overall survival for the full cohort (**FIG. 31C**) and the core cohort (**FIG. 31D**), for subjects who achieved CR, PR, all subjects that showed a response, non-responders, and all treated subjects. NE, not estimable.

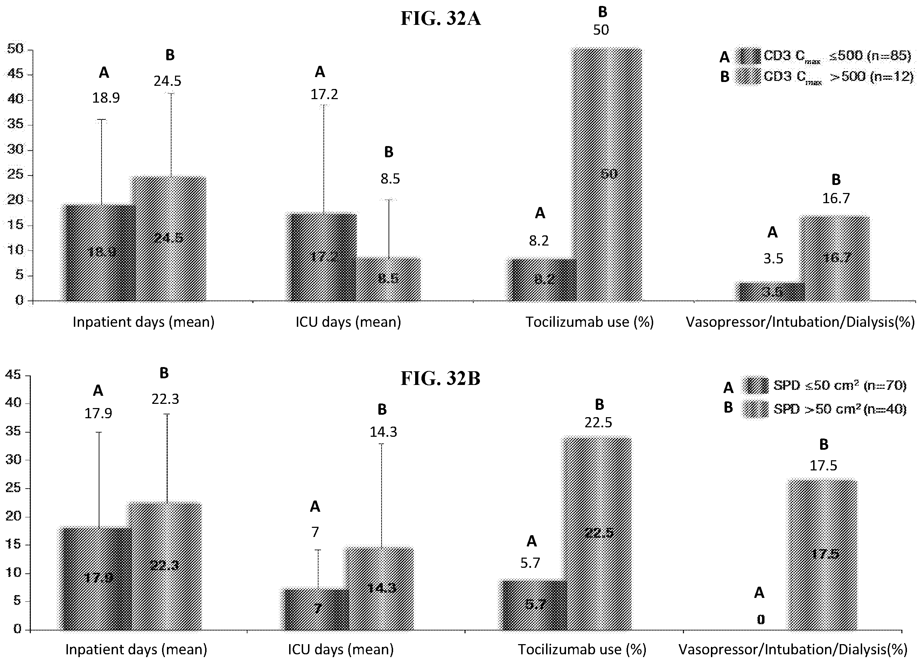

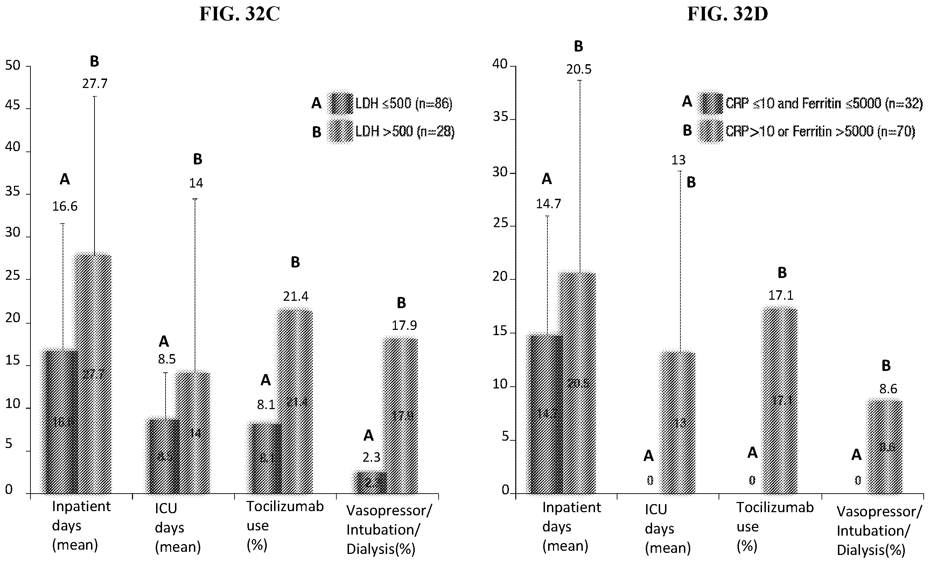

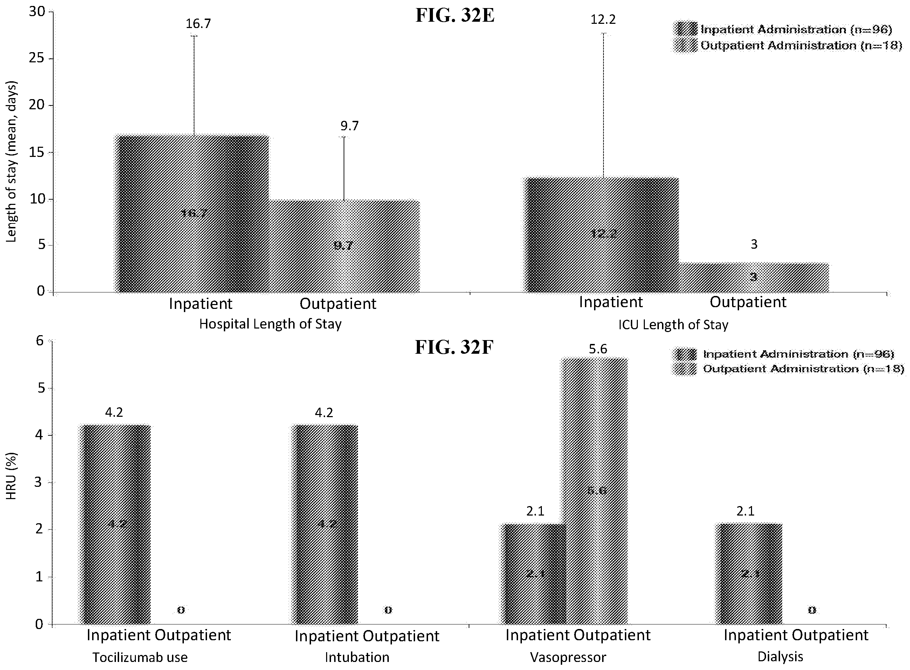

**FIGS. 32A-32F** show the use of various healthcare resource parameters (mean inpatient days, mean days in the intensive care unit (ICU), % tocilizumab use and % vasopressor, intubation or dialysis use, mean length of hospital stay) in subjects grouped by: Cₘₐₓ of at or below 500 cells/µL or above 500 cells/µL CAR⁺ cells/µL (**FIG. 32A**); SPD at or below 50 cm² or above 50 cm² (**FIG. 32B**); LDH at or below 500 units/L or above 500 units/L (**FIG. 32C**); CRP at or below 10 mg/L and ferritin at or below 5000 ng/mL or CRP above 10 mg/L and ferritin above 5000 ng/mL (**FIG. 32D**), and inpatient setting or outpatient setting (**FIGS. 32E-32F**).

**FIG. 33** depicts mean preference-weighted health status score for subjects from baseline through 6 months post-infusion of CAR⁺ T cell compositions.

**FIG. 34** depicts mean change from baseline for subjects from baseline through 6 months post-infusion of CAR⁺ T cell compositions.

**FIG. 35** depicts proportion of subjects with clinically meaningful changes in health state utility scores evaluated from baseline through 6 months post-infusion of CAR⁺ T cell compositions.

**FIGS. 36A-36E** show the percentage of patients reporting moderate, severe, or extreme problems with the mobility (**FIG. 36A**), self-care (**FIG. 36B**), usual activities (**FIG. 36C**), pain/discomfort (**FIG. 36D**) and anxiety/depression (**FIG. 36E**) dimensions from baseline to 6 months post-infusion of CAR⁺ T cell compositions.

**FIG. 37** depicts mean global health rating scores (EQ-VAS) for subjects from baseline through 6 months post-infusion of CAR⁺ T cell compositions.

**FIG. 38** shows mean change from baseline in EQ-VAS scores for subjects from baseline through 6 months post-infusion of CAR⁺ T cell compositions.

**FIG. 39** shows CAR T cell expansion by dose level for dose level 1 (DL1) and dose level 2 (DL2).

**FIG. 40** shows methods for identifying Healthcare Resource Utilization (HRU) within the dates of onset and resolution of CRS and NEs.

**FIG. 41** shows facility, drug and diagnostic costs for subjects with relapsed and refractory Non-Hodgkin Lymphoma after administration of anti-CD 19 CAR-expressing cells.

**FIG. 42** shows differential gene expression profiles in pre-treatment tumor biopsies in subjects showing complete response (CR) or progressive disease (PD) at 3 months post-treatment.

**FIG. 43** shows various enriched gene sets associated with PD at 3 months post-treatment, including genes expressed more highly in diffuse large B-cell lymphoma (DLBCL) cell line samples compared to follicular lymphoma cell line samples (FL; FL_DLBCL_DN).

**FIG. 44** shows differential gene expression between FL tumor biopsies and DLBCL tumor biopsies.

**FIGS. 45A-45B** show differential gene expression of exemplary genes EZH2 (**FIG. 45A**) and CD3ε (**FIG. 45B**) between FL and DLBCL tumors.

**FIGS. 46A** and **46B** show the single-sample Gene Set Enrichment Analysis (ssGSEA) scores between genes found to be elevated in DLBCL (designated "DLBCL gene set"; **FIG. 46A**) versus in FL (designated "FL gene set"; **FIG. 46B**) and subjects who went onto exhibit a CR or subjects who went onto exhibit PD, and illustrates that subjects having tumor gene expression profiles more similar to those seen in FL, as compared to those seen in DLBCL, were more likely to show CR at 3 months post-treatment.

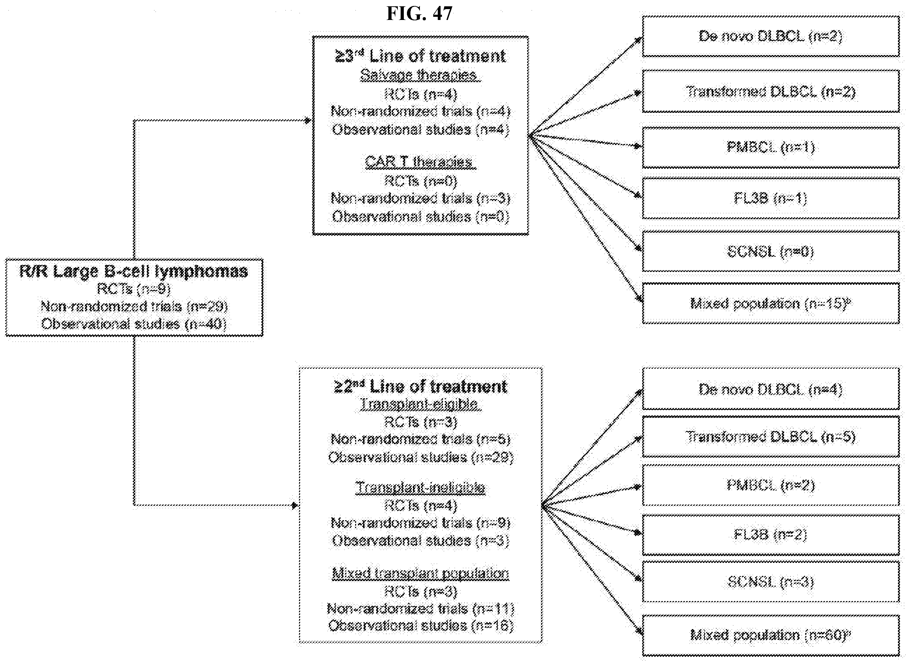

**FIG. 47** shows an overview of studies by lines of treatment, disease subtypes and subpopulations of relapsed/refractory large B cell lymphoma (R/R LBCL). DLBCL, diffuse large B-cell lymphoma; FL3B, follicular lymphoma grade 3B; PMBCL, primary mediastinal large B-cell lymphoma; RCT, randomized controlled trial; R/R, relapsed/refractory; SCNSL, secondary central nervous system lymphoma; ^{a} study count listed in figure exceeds a total of 78 due to reporting of multiple subgroups within some publications; ^{b} the "mixed transplant population" includes studies wherein both transplant eligible and transplant noneligible patients were enrolled. "Mixed population" includes studies wherein the population was unspecified.

**FIG. 48** depicts an overview of the subjects that were leukapheresed and consequently treated with CAR+ T cell therapy, and the treatment regimen used to assess outcomes across all dose levels DL1, DL1D, DL2 and DL3.

**FIGS. 49A-49C** depict efficacy and response outcomes among subjects evaluable for response, including the median DOR (**FIG. 49A**), the median PFS among subjects with CR (**FIG. 49B**), and the median OS among subjects achieving CR (**FIG. 49C**).

**FIGS. 50A-50B** depict responses observed across subgroups for all evaluable subjects and subjects with response including objective response rate (**FIG. 50A**) and complete response rate (**FIG. 50B**).

**FIGS. 51A-51F** depict duration of response across subgroups relative to progression free survival (**FIG 51A**), by histologic subgroup (**FIG. 51B**), by bridging therapy (**FIG. 51C**), by chemotherapy-sensitive versus chemotherapy refractory status (**FIG. 51D**), by pre-LDC SPD status (**FIG. 51E**), by age group (**FIG. 51F**) and duration of response in subjects with versus without comorbidities (**FIG. 51G**).

**FIGS. 52A-52B** depict responses evaluated as mean (SE) change from baseline in global health status (**FIG. 52A**) and physical functioning (**FIG. 52B**).

**FIGS. 53A-53B** depict responses evaluated as mean (SE) change from baseline for fatigue (**FIG. 53A**) and pain (**FIG. 53B**).

**FIGS. 54A-54D** depict results of subjects evaluable for the EORTC QLQ-C30 questionnaire who experienced clinically meaningful improvement, no change in status or deterioration, in areas including global health status (**FIG. 54A**), physical functioning (**FIG. 54B**; with the exception at Month 1), fatigue (**FIG. 54C**) and pain (**FIG. 54D**).

**FIGS. 55A-55B** depict responses of subjects evaluable for the EORTC QLQ-C30 questionnaire evaluated as mean (SE) change from baseline for health state index score (**FIG. 55A**) and EQ-VAS (**FIG. 55B**).

**FIG. 56** depicts results of individual subjects monitored as inpatients or outpatients evaluated for efficacy and objective response.

**FIG. 57** depicts the percentage of subjects with CRS, NEs or both CRS and NEs that had received tocilizumab, tocilizumab and steroids, vasopressors or steroids only.

**FIG. 58** depicts the probability of continued response and duration of response among subjects evaluable for response for complete responders (CR) and partial responders (PR) with a median follow up (95% CI) of 12 months (11.2-16.7 range).

**FIG. 59** depicts overall response rate (ORR) and complete response (CR), with 95% confidence interval, among subjects with varying clinical characteristics including age, LBCL subtype,use of bridging therapy, presence of high disease burden, prior HSCT and secondary CNS lymphoma.

**FIG. 60** depicts probability of progression free survival (PFS) for subjects evaluated by objective response, including complete responders (CR), partial responders (PR), non-responders, and a total average.

**FIG. 61** depicts probability of overall survival (OS) for subjects evaluated by objective response, including complete responders (CR), partial responders (PR), non-responders, and a total average.

**FIG. 62** depicts progression free survival (PFS) by histologic subtype including HGL, tFL, PMBCL, tiNHL and DLBCL, NOS.

**FIG. 63** depicts cellular kinetic parameters for CAR+ T cells (CD19+ B cell, CD3+ truncated receptor and transgene) analyzed by qPCR and flow cytometry vs. CD19+ B cells for 261 subjects by study day.

**FIG. 64** depicts cellular kinetic parameters for CAR+ T cells (CD3+, CD4+ and CD8+ truncated receptor +) analyzed by study day through flow cytometry.

### Detailed Description

### I. METHODS AND USES OF CELL THERAPY WITH GENETICALLY ENGINEERED CELLS

Provided are methods and uses of engineered cells (e.g., T cells) and/or compositions thereof, for the treatment of subjects having a disease or condition, which generally is or includes a cancer or a tumor, such as a leukemia or a lymphoma, most particularly a B cell malignancy or a non-Hodgkin lymphoma (NHL). In particular embodiments of any of the provided methods, the T cells are engineered with a chimeric antigen receptor (CAR) that is directed against CD19. In some aspects, the disease or condition is a B cell lymphoma. In some aspects, the disease or condition is a large B cell lymphoma. In some aspects, the disease or condition is a diffuse large B-cell lymphoma (DLBCL) or a subtype thereof. In some aspects, the methods and uses provide for or achieve improved response and/or more durable responses or efficacy and/or a reduced risk of toxicity or other side effects, e.g., in particular groups of subjects treated, as compared to certain alternative methods. In some embodiments, the methods are advantageous by virtue of the administration of specified numbers or relative numbers of the engineered cells, the administration of defined ratios of particular types of the cells, treatment of particular patient populations, such as those having a particular risk profile, staging, and/or prior treatment history, and/or combinations thereof.

Also provided are methods that include assessing particular parameters, e.g., expression of specific biomarkers or analytes, that can be correlated with development of toxicity, and methods for treatment, e.g., intervention therapy, to prevent and/or ameliorate toxicities. Also provided are methods that involve assessing particular parameters, e.g., expression of specific biomarkers or analytes, that can be correlated with an outcome, such as a therapeutic outcome, including a response, such as a complete response (CR) or a partial response (PR), which in some cases is a durable response, such as a response that is durable for at least 3 months, 6 months or more; or a safety outcome, such as a development of a toxicity, for example, neurotoxicity or CRS, after administration of an immunotherapy and/or cell therapy. Also provided are methods to assess the likelihood of response and/or likelihood of risk of toxicity, based on assessment of the parameters, such as expression of biomarkers or analytes. Also provided are compositions for use in cell therapy. Also provided are articles of manufacture and kits, e.g., for use in the methods provided herein. In some embodiments, the articles of manufacture and kits also contain instructions for using, according to the methods provided herein.

In some embodiments, the methods and uses include administering to the subject cells expressing genetically engineered (recombinant) cell surface receptors in adoptive cell therapy, which generally are chimeric receptors such as chimeric antigen receptors (CARs), recognizing an antigen expressed by, associated with and/or specific to the leukemia or lymphoma and/or cell type from which it is derived. The cells are generally administered in a composition formulated for administration; the methods generally involve administering one or more doses of the cells to the subject, which dose(s) may include a particular number or relative number of cells or of the engineered cells, and/or a defined ratio or compositions of two or more sub-types within the composition, such as CD4 vs.CD8 T cells.

In some embodiments, the cells, populations, and compositions are administered to a subject having the particular disease or condition to be treated, e.g., via adoptive cell therapy, such as adoptive T cell therapy. In some embodiments, the methods involve treating a subject having a lymphoma or a leukemia, or a B cell malignancy, such as a large B cell lymphoma or a non-Hodgkin lymphoma (NHL) with a dose of antigen receptor-expressing cells (e.g. CAR-expressing cells).

In some embodiments, the provided methods involve treating a specific group or subset of subjects, e.g., subjects identified as having high-risk disease, e.g., high-risk NHL or a high-risk large B cell lymphoma. In some aspects, the methods treat subjects having a form of aggressive and/or poor prognosis B-cell non-Hodgkin lymphoma (NHL), such as NHL that has relapsed or is refractory (R/R) to standard therapy and/or has a poor prognosis. In some aspects, the methods treat subjects having a large B cell lymphoma that has relapsed or is refractory (R/R) to standard therapy. In some cases, the overall response rate (ORR; also known in some cases as objective response rate) to available therapies, to a standard of care (SOC), or to a reference therapy for the disease and/or patient population for which the therapy is indicated, is less than 40% and/or the complete response (CR; also known in some cases as complete remission) is less than 20%. In some embodiments, in chemorefractory DLBCL, the ORR with a reference or available treatment or standard-of-care therapy is about 26% and the CR rate is about 8% (Crump et al. Outcomes in refractory aggressive diffuse large B-cell lymphoma (DLBCL): Results from the international SCHOLAR study. ASCO 2016 [Abstract 7516]). In some aspects, the provided methods, compositions, uses and articles of manufacture achieve improved and superior responses to available therapies. In some embodiments, the improved or superior responses are to current standard of care (SOC). In some embodiments, the current SOC for treatment of B cell malignancies, such as a B-cell NHL, non-Hodgkin lymphoma (NHL), include up to 3 cycles of either rituximab, dexamethasone, cytarabine (AraC), and cisplatin (R-DHAP), rituximab, ifosfamide, carboplatin and etoposide (R-ICE), or rituximab, gemcitabine, dexamethasone, and cisplatin (R-GDP) followed by carmustine, etoposide, cytarabine, and melphalan (BEAM) high-dose chemotherapy and hematopoietic stem cell transplant (HSCT) in responding subjects (see, e.g., Crump et al., J Clin Oncol. 2014; 32(31):3490-6; Gisselbrecht, et al., J Clin Oncol. 2010;28(27):4184-90; van Imhoff et al., J Clin Oncol. 2017;35(5):544-51).

Large B-cell lymphoma (LBCL) is the most common subtype of non-Hodgkin lymphoma (NHL). Frontline treatment is curative in approximately 60% of subjects; however, approximately 30% of subjects relapse and approximately 10% are refractory to frontline treatment. Treatment options for subjects with relapsed/refractory (R/R) disease, especially as third-line or greater (3L+) therapy, primarily include salvage chemotherapies (CTs). Two chimeric antigen receptor (CAR) T cell products and an antibody-drug conjugate have been approved as a third-line therapy. Unmet medical needs within the second-line and greater (2L+) or 3L+ therapy for R/R LBCL were identified based on a systematic literature review (SLR) of evidence on clinical outcomes in LBCL subjects, including the new therapies listed above.

Based on an exemplary SLR, conducted in accordance with the Cochrane Handbook for Systematic Reviews of Interventions and European Union Health Technology Assessment requirements of screening 8683 database records and additional sources, 103 publications covering 78 unique studies were identified. The review identified randomized and nonrandomized/observational studies within R/R LBCL, including diffuse large B-cell lymphoma (DLBCL), follicular lymphoma grade 3B (FL3B), primary mediastinal large B-cell lymphoma (PMBCL), DLBCL transformed from indolent lymphomas, and R/R DLBCL with secondary central nervous system (SCNS) involvement. Sources reviewed included EMBASE, MEDLINE, The Cochrane Library, and clinical conferences (ASCO, ESMO, EHA, ASH, ICML, AACR, and EORTC). An exemplary schematic representing the SLR is depicted in **FIG. 47****.** Studies identified were characterized by line of treatment and R/R LBCL subtype. OS, PFS, DOR, OR, and safety observed from the identified studies were described. Disease subtypes, subject eligibility criteria, and length of follow-up varied notably across studies.

Based on the exemplary SLR, in the 3L+ population, 11 salvage CT and 2 CART cell therapy studies reported survival outcomes. With salvage CT, the reported ORR across studies ranged from 0% to 54%, while CR ranged from 5.6%-31%. Median OS (mOS) ranged between 3-9 months, with one outlying study reporting mOS at 20 months. Median PFS (mPFS) reported within the salvage CT studies ranged from 2-6 months. Among CAR T cell therapies, subjects treated with an anti-CD19 CAR T cell therapy (n=101) reported a CR rate of 58% and median DOR (mDOR) was 11.1 months after a median follow-up of 27.1 months. mPFS was 5.9 months and mOS was not reached. At a median follow-up of 19.3 months, subjects treated with another anti-CD19 CAR T cell therapy (n=115) had a CR of 40% but the mDOR was not reached. mOS was 11.1 months for all infused patients.

In the 2L+ transplant-eligible population (36 studies), subjects who received high-dose CT + HSCT achieved mOS between 9 months to 5 years. In the transplant noneligible population, 16 studies reported mOS between 3-20 months. Studies involving mixed transplant-eligible and noneligible populations (30 studies) reported mOS of 1-17 months.

A few studies with limited sample sizes were found to report outcomes in LBCL subtypes (e.g., PMBCL, SCNS lymphoma, DLBCL transformed from non-FL indolent lymphoma, FL3B). In the 3L+ setting, 1 study reported that mOS was not reached after a median of 6.6 months. In the 2L+ setting, 4 studies reported mPFS and mOS outcomes ranging between 2-9 months and 10-16 months, respectively.

Among studies assessing safety of salvage chemotherapies in R/R LBCL, neutropenia, leukocytopenia, thrombocytopenia, and infections were the most commonly reported adverse events (AEs), with neutropenia being the most reported. Among the 3 studies reporting safety outcomes of CAR T cell therapy, data indicated that hematologic AEs (possibly related to lymphodepleting CT), cytokine release syndrome, and neurotoxicity are the most reported.

Based on exemplary studies, fewer than 50% of patients with relapsed/refractory large B-cell lymphoma (LBCL) achieve responses to third-line or subsequent treatments (Van Den Neste et al. Bone Marrow Transplant. 2016;51:51-7; Gonzalez-Barca E et al. Bone Marrow Transplant 2019). High-dose chemotherapy with autologous hematopoietic stem cell transplantation (HSCT) remains the standard treatment at first relapse in transplant-eligible patients with chemotherapy-sensitive disease (National Comprehensive Cancer Network Clinical Practice Guidelines in Oncology. March 6, 2019), but most patients will not be cured with this approach (Van Den Neste et al. Bone Marrow Transplant. 2016;51:51-7; Gonzalez-Barca E et al. Bone Marrow Transplant 2019, National Comprehensive Cancer Network Clinical Practice Guidelines in Oncology. March 6, 2019, Gisselbrecht C et al. J Clin Oncol. 2010; 28:4184-90). In some studies, outcomes are worse in subjects with chemotherapy-refractory disease, with a complete response (CR) rate to conventional treatment of 7% and overall survival (OS) of 6 months. (Crump et al. Blood. 2017;130:1800-8). Adverse outcomes were associated with older age, central nervous system (CNS) involvement (Thanarajasingam et al. Br J Haematol. 2018;183:149-52; Nabhan et al. J Clin Oncol. 2018; 36:7545) and comorbidities (Pfreundschuh Blood. 2010;116:5103-10).

Certain CD19-directed CAR-T cell therapies are available for treatment of B cell lymphoma, including axicabtagene ciloleucel (axi-cel) and tisagenlecleucel. In one exemplary study, axi-cel-treated subjects achieved CR rates (per investigator) of 54%, with 40% in durable remission (median follow-up, 15.4 months) (Neelapu et al. N Engl Med. 2017. 377;2531-44). Most subjects developed CRS (93%) and NEs (64%), with median time to onset of 2 and 5 days, respectively, and grade ≥3 CRS (Lee grading criteria (Lee et al. Blood. 2014;124:188-95)) and NEs occurred in 13% and 28%, respectively, and 43% received tocilizumab (27% received corticosteroids). In another exemplary study, approximately one-third of patients who received tisagenlecleucel maintained durable remission at 1 year (Schuster et al. N Engl J Med. 2019. 380:45-56). Most subjects (58%) developed CRS, while 21% had NEs. Grade ≥3 CRS (Penn grading criteria, Porter et al. J Hematol Oncol. 2018;11:35) and NEs were reported in 22% and 12% of patients, respectively (Schuster et al. N Engl J Med. 2019. 380:45-56). Further, these therapies do not include treatment of certain high-risk patients, including patients with PMBCL, DLBCL transformed from indolent lymphoma other than FL, FL3B, and patients with certain high-risk features, such as secondary CNS lymphoma, moderate renal/cardiac comorbidities, and requirement for bridging therapy.

The SLR and examination of the current evidence demonstrated an important and high unmet need for additional therapeutic options that provide favorable benefit/risk and durable response, which is not met with available therapies for subjects with 2L+ and 3L+ LBCL. Furthermore, limited data were available for the rarer subtypes of LBCL. These findings revealed important treatment gaps for R/R LBCL that must be addressed, and a need for improvement of the existing treatments. Provided herein are embodiments that can meet such needs.

In some embodiments, the methods, uses and articles of manufacture involve, or are used for treatment of subjects involving, selecting or identifying a particular group or subset of subjects, e.g., based on specific types of disease, diagnostic criteria, prior treatments and/or response to prior treatments. In some embodiments, the methods involve treating a subject having relapsed following remission after treatment with, or become refractory to, one or more prior therapies; or a subject that has relapsed or is refractory (R/R) to one or more prior therapies, e.g., one or more lines of standard therapy.

In some embodiments, the subject has a B cell malignancy, such as a B cell lymphoma and/or a non-Hodgkin lymphoma (NHL). In some embodiments, the subject has a B cell malignancy, such as a large B cell lymphoma, e.g., a relapsed/refractory (R/R) large B cell lymphoma. In some embodiments, the subject has a large B cell lymphoma, such as a diffuse large B-cell lymphoma (DLBCL) (e.g., a DLBCL not otherwise specified (NOS; de novo or transformed from indolent) or other DLBCL). In some embodiments, the subject has a primary mediastinal B-cell lymphoma (PMBCL) or a follicular lymphoma, such as a follicular lymphoma grade 3B (FL3B). In some aspects, the B cell lymphoma is or includes diffuse large B-cell lymphoma (DLBCL), follicular lymphoma or PBMCL. In some aspects, the subject has a DLBCL that is a DLBCL, not otherwise specified (NOS). In some embodiments, the lymphoma, such as the DLBCL, is de novo. In some embodiments, the lymphoma, such as the DLBCL, is transformed from another indolent lymphoma. In some embodiments the lymphoma, such as the DLBCL, is transformed from a follicular lymphoma (tFL). In some of any embodiments, the subject has follicular lymphoma (FL).

In particular embodiments, the methods provided herein are based on administration of a CD19-directed CAR T cell therapy in which the CAR contains a CD19-directed scFv antigen binding domain (e.g. from FMC63). The CAR further contains an intracellular signaling domain containing a signaling domain from CD3zeta, and also incorporates a 4-1BB costimulatory domain, which has been associated with lower incidence of CRS and NE compared with CD28-containing constructs (Lu et al. J Clin Oncol. 2018;36:3041). In some embodiments, the methods provided herein include CD8+ and CD4+ T-cell subsets that are transduced and expanded separately in vitro, and administered at equal (about 1:1) target doses. In some embodiments, there is low variability in the administered total and CD8+ CAR+ T-cell doses, two parameters associated with increased toxicity in previous studies (Neelapu et al. N Engl Med. 2017. 377;2531-44; Turtle et al. Sci Transl Med. 2016;8:355ra116; Hay et al. Blood. 2017;130:2295-306).

In particular embodiments, the provided methods can be used to treat particular LBCL subtypes or high-risk groups, such as elderly patients and those with comorbidities or CNS involvement, in which available treatment options remain limited. For example, existing CAR T cell therapies are associated with severe CAR T-cell-related toxicities, including cytokine release syndrome (CRS) and neurological events (NEs), that may limit administration to specialized treatment center (Yescarta Risk Evaluation and Mitigation Strategy (REMS). Gilead Pharma September 10, 2019; Kymriah Risk Evaluation and Mitigation Strategy (REMS) Novartis September 10, 2019) and impact use in difficult-to-treat patients. CAR T-cell therapies with a favorable benefit/risk, specifically those with high efficacy and low incidences of severe CRS and NEs, may allow for broader inclusion of subject subgroups and outpatient administration/monitoring.

In particular embodiments, provided methods result in favorable outcomes in subjects with LBCL, including in certain subjects that have been previously excluded from treatment with other therapies, including other anti-CD19 CAR-T cell therapies. Treatment with the CD19-directed CAR T cells in subjects with LBCL in the group of subjects shown herein resulted in durable responses,, including responses associated with increased CAR T-cell expansion in vivo, and CAR T cells persisted long-term after infusion. In some embodiments, the provided methods demonstrated favorable outcomes in heavily pretreated patients with aggressive, high-risk disease, including patients that were chemotherapy refractory or required immediate treatment for disease control with bridging therapy. The observations herein support treating subjects with aggressive, high-risk disease with a CD19-directed CAR T cell therapy in accord with the provided methods. For example, subjects with PMBCL, DLBCL transformed from indolent lymphoma other than FL, FL3B, and patients with certain high-risk features, such as secondary CNS lymphoma, moderate renal/cardiac comorbidities, and requirement for bridging therapy can be treated in accord with the provided methods. In some embodiments, the provided methods can be used to treat subjects that have been heavily pretreated (e.g. with two, three or more prior therapies for treating the disease). Among the subgroups that can be treated by the provided methods also are older subgroups of greater than or equal to 65 years of age, including those >75 years. Observations herein demonstrate that ORR and CR, including durable responses, were observed across all subgroups with low incidence of severe CRS and NEs.

In some embodiments, fewer than one-half of all subjects treated by the methods provided herein develop CRS or NEs. In some embodiments, the incidence of grade ≥3 CRS and NEs is low (2% and 10%, respectively), and no fatal CRS or NE occurred in the group of subjects treated as described in the Examples. In some embodiments, low overall incidence and severity of CRS and NEs, along with their late onset (median, 5 and 9 days, respectively), support outpatient administration/monitoring in select subjects. In some embodiments, safety and efficacy outcomes in subjects who receive CAR T cell compositions in the outpatient setting are similar to the entire treated population. In some embodiments, chimeric antigen receptor (CAR) T cell therapy has generally been limited to inpatient treatment at university medical centers; however, most patients in the US with relapsed/refractory (R/R) diffuse large B-cell lymphoma (DLBCL) receive therapy at nonuniversity medical centers where outpatient delivery of cancer therapy is common. In some embodiments of any of the methods provided herein, infusion and management of CAR T cell therapies in the outpatient setting leads to wider utilization in community/nonuniversity centers and improved access.

In some embodiments, treatment with any of the methods provided herein results in a high rate of durable CR and low incidence of severe CRS and NEs among subjects with high-risk, aggressive relapsed/refractory LBCL in this study. In some embodiments, clinically meaningful activity is observed across subject subgroups with unmet medical need, including uncommon LBCL histologic subtypes and those with poor prognostic characteristics. In some embodiments, low incidence of severe CRS and NEs and later time to onset allows for outpatient administration/monitoring. In some embodiments, the unique risk/benefit profile of any of the methods provided herein may allow for greater inclusion of patients and potential sites of care.

In some embodiments, the methods involve treating a subject that has an Eastern Cooperative Oncology Group Performance Status (ECOG) of 0-1 or 0-2. In some embodiments, the methods treat a poor-prognosis population of DLBCL patients or subject thereof that generally responds poorly to therapies or particular reference therapies, such as one having one or more, such as two or three, chromosomal translocations (such as so-called "double-hit" or "triple-hit" lymphoma; having translocations MYC/8q24 loci, usually in combination with the t(14; 18) (q32; q21) bcl-2 gene or/and BCL6/3q27 chromosomal translocation; see, e.g., Xu et al. (2013) Int J Clin Exp Pathol. 6(4): 788-794), and/or one having relapsed, such as relapsed within 12 months, following administration of an autologous stem cell transplant (ASCT), and/or one having been deemed chemorefractory.

In some aspects, the provided embodiments are based on observations that the provided methods can be used to achieve a high response rate with high durability, compared to certain available methods for cell therapy, without an increased risk of toxicity. In some embodiments, the provided methods permit prolonged persistence of adoptively transferred cells for cell therapy, and/or low rate of developing toxicity in the subject. In some embodiments, the methods can be used to select subjects for treatment with cell therapy that are likely or more likely to respond to the therapy and/or to determine appropriate doses or dosing regimen for higher response rate and/or more durable response, while minimizing the risk of toxicity. In some aspects, the provided embodiments are based on observations that subjects that exhibit improved response, such as a complete response (CR) exhibited gene expression patterns that were associated with gene expression patterns that were more similar to those of follicular lymphoma (FL) compared to diffuse large B-cell lymphoma (DLBCL). The provided embodiments and such methods can inform rational strategies to facilitate the safe and effective clinical application of adoptive cell therapy, such as CAR-T cell therapy.

In some aspects, the subject has a transplant non-eligible (TNE) lymphoma, for example, the subject is ineligible for high-dose chemotherapy and hematopoietic stem cell transplantation (HSCT). In some embodiments, the subject has a TNE relapsed/refractory (R/R) aggressive large B cell NHL. In some aspects, subjects with R/R aggressive large B cell NHL that have failed first-line therapy with immunochemotherapy and are ineligible for high-dose chemotherapy and hematopoietic stem cell transplantation (HSCT) have a poor prognosis. In some aspects, available treatment options for these subjects include platinum/gemcitabine-based or bendamustine-based regimens in combination with rituximab, with or without radiotherapy. However, in some aspects, long-term outcomes of the available therapies remain poor due to lack of a curative option. The provided methods offer an improved treatment for such subjects.

In some embodiments, the provided embodiments offer an advantage of improvements in health-related quality of life (HRQoL) as reported by the subjects receiving the provided CAR-expressing T cell therapy. In some aspects, early during the course of treatment, e.g., within 1 month of administration, the subject may still be recovering from the administration and adverse events (e.g., toxicities) related to the administration of the cell therapy. It has been observed that the provided methods and uses of cell therapy result in a notable improvement in patient-reported HRQoL, particularly over long term (e.g., at 12 months or after administration of the cell therapy). The provided methods offer an improved treatment outcome, including improved HRQoL, for various subjects.

In some embodiments, the antigen receptor (e.g. CAR) specifically binds to a target antigen associated with the disease or condition, such as associated with a B cell malignancy, such as a large B cell lymphoma or an NHL. In some embodiments, the antigen associated with the disease or disorder is selected from CD20, CD19, CD22, ROR1, CD45, CD21, CD5, CD33, Igkappa, Iglambda, CD79a, CD79b or CD30. In some embodiments, the antigen associated with the disease or disorder, such as a B cell malignancy, such as a large B cell lymphoma, is CD19.

In some embodiments, the methods include an agent or other treatment capable of treating, preventing, delaying, reducing or attenuating the development or risk of development of a toxicity for use in a method of reducing toxicity after administration of a cell therapy, wherein the method comprises (a) assessing one or more parameters in a subject, wherein the one or more parameters is selected from the level, amount or concentration of ferritin or C-reactive protein (CRP) in a biological sample from the subject; wherein the subject is a candidate for treatment with a cell therapy, said cell therapy comprising a dose of genetically engineered cells expressing a recombinant receptor; and wherein the assessing is carried out prior to administering the cell therapy and/or said biological sample or tumor does not comprise the recombinant receptor and/or said engineered cells; (b) identifying if the subject is as at risk of toxicity wherein the subject is at risk of toxicity if the one or more parameters is above a threshold level and the subject is not at risk of toxicity if the one or more parameters is at or below a threshold level, (c) following or based on the results of the assessment, administering to the subject the cell therapy.

In some embodiments, the methods include a cell therapy for use in a method of treatment, wherein the method comprises administering to a subject the cell therapy, wherein said subject has been identified as at risk of developing a toxicity, wherein identifying the subject comprises: (a) assessing one or more parameters in a subject, wherein the one or more parameters is selected from the level, amount or concentration of LDH, ferritin or C-reactive protein (CRP) in a biological sample from the subject, of a tumor in the subject; wherein the subject is a candidate for treatment with a cell therapy, said cell therapy comprising a dose of genetically engineered cells expressing a recombinant receptor; and wherein the assessing is carried out prior to administering the cell therapy and/or said biological sample or tumor does not comprise the recombinant receptor and/or said engineered cells; and (b) identifying if the subject is as at risk of toxicity wherein the subject is at risk of toxicity if the one or more parameters is above a threshold level and the subject is not at risk of toxicity if the one or more parameters is at or below a threshold level, wherein: (i) the parameter is ferritin, and the threshold level is above at or about 1000 nanograms per milliliter, 2000 nanograms per milliliter, 3000 nanograms per milliliter, 4000 nanograms per milliliter, 5000 nanograms per milliliter, 6000 nanograms per milliliter, 7000 nanograms per milliliter or 8000 nanograms per milliliter; and/or (ii) the parameter is CRP, and the threshold level is above at or about 5 milligrams per liter, 10 milligrams per liter, 15 milligrams per liter, 20 milligrams per liter, 25 milligrams per liter, 30 milligrams per liter, 40 milligrams per liter or 50 milligrams per liter; and wherein (c) following or based on the results of the assessment, administering to the subject the cell therapy, and, optionally, an agent or other treatment capable of treating, preventing, delaying, reducing or attenuating the development or risk of development of a toxicity.

An agent or other treatment capable of treating, preventing, delaying, reducing or attenuating the development or risk of development of a toxicity for use in a method of treatment, wherein the method comprises administering to a subject the agent or other treatment, wherein said subject has been identified as at risk of developing a toxicity, wherein identifying the subject comprises: (a) assessing one or more parameters in a subject, wherein the one or more parameters is selected from the level, amount or concentration of ferritin or C-reactive protein (CRP) in a biological sample from the subject; wherein the subject is a candidate for treatment with a cell therapy, said cell therapy comprising a dose of genetically engineered cells expressing a recombinant receptor; and wherein the assessing is carried out prior to administering the cell therapy and/or said biological sample or tumor does not comprise the recombinant receptor and/or said engineered cells; and (b) identifying if the subject is as at risk of toxicity wherein the subject is at risk of toxicity if the one or more parameters is above a threshold level and the subject is not at risk of toxicity if the one or more parameters is at or below a threshold level, wherein: (i) the parameter is ferritin, and the threshold level is above at or about 1000 nanograms per milliliter, 2000 nanograms per milliliter, 3000 nanograms per milliliter, 4000 nanograms per milliliter, 5000 nanograms per milliliter, 6000 nanograms per milliliter, 7000 nanograms per milliliter or 8000 nanograms per milliliter; and/or (ii) the parameter is CRP, and the threshold level is above at or about 5 milligrams per liter, 10 milligrams per liter, 15 milligrams per liter, 20 milligrams per liter, 25 milligrams per liter, 30 milligrams per liter, 40 milligrams per liter or 50 milligrams per liter; and wherein (c) following or based on the results of the assessment, administering to the subject the cell therapy and the agent or other treatment capable of treating, preventing, delaying, reducing or attenuating the development or risk of development of a toxicity.

An agent or other treatment capable of treating, preventing, delaying, reducing or attenuating the development or risk of development of a toxicity for use in a method of reducing toxicity after administration of a cell therapy, wherein the method comprises: (a) assessing one or more parameters in a subject, wherein the one or more parameters is selected from the level, amount or concentration of ferritin or C-reactive protein (CRP) in a biological sample from the subject; wherein the subject is a candidate for treatment with a cell therapy, said cell therapy comprising a dose of genetically engineered cells expressing a recombinant receptor; and wherein the assessing is carried out prior to administering the cell therapy and/or said biological sample or tumor does not comprise the recombinant receptor and/or said engineered cells; and (b) identifying if the subject is as at risk of toxicity wherein the subject is at risk of toxicity if the one or more parameters is above a threshold level and the subject is not at risk of toxicity if the one or more parameters is at or below a threshold level, wherein: (i) the parameter is ferritin, and the threshold level is above at or about 1000 nanograms per milliliter, 2000 nanograms per milliliter, 3000 nanograms per milliliter, 4000 nanograms per milliliter, 5000 nanograms per milliliter, 6000 nanograms per milliliter, 7000 nanograms per milliliter or 8000 nanograms per milliliter; and/or (ii) the parameter is CRP, and the threshold level is above at or about 5 milligrams per liter, 10 milligrams per liter, 15 milligrams per liter, 20 milligrams per liter, 25 milligrams per liter, 30 milligrams per liter, 40 milligrams per liter or 50 milligrams per liter; and wherein (c) following or based on the results of the assessment, administering to the subject the cell therapy and the agent or other treatment capable of treating, preventing, delaying, reducing or attenuating the development or risk of development of a toxicity.

In some embodiments, the methods include administration of the cells or a composition containing the cells to a subject, tissue, or cell, such as one having, at risk for, or suspected of having the disease, condition or disorder. In some embodiments, the subject is the subject is an adult. In some embodiments, the subject is over at or about 30, 40, 50, 60, or 70 years of age.

In some embodiments, the methods include administration of cells to a subject selected or identified as having a certain prognosis or risk of a B cell malignancy, such as a large B cell lymphoma (e.g., DLBCL). In some embodiments, the methods include administration of cells to subjects selected or identified has having a non-Hodgkin lymphoma (NHL). Lymphomas, such as non-Hodgkin lymphoma (NHL) or large B cell lymphomas can be a variable disease. Some subjects with NHL may survive without treatment while others may require immediate intervention. In some cases, subjects with NHL may be classified into groups that may inform disease prognosis and/or recommended treatment strategy. In some cases, these groups may be "low risk," "intermediate risk," "high risk," and/or "very high risk" and patients may be classified as such depending on a number of factors including, but not limited to, genetic abnormalities and/or morphological or physical characteristics. In some embodiments, subjects treated in accord with the methods, and/or with the articles of manufacture or compositions, are classified or identified based on the risk of NHL. In some embodiments, the subject is one that has high risk NHL.

In some embodiments, the subject has been previously treated with a therapy or a therapeutic agent targeting the disease or condition, e.g., a large B cell lymphoma or an NHL, prior to administration of the cells expressing the recombinant receptor. In some embodiments, the subject has been previously treated with a hematopoietic stem cell transplantation (HSCT), e.g., allogeneic HSCT or autologous HSCT. In some embodiments, the subject has had poor prognosis after treatment with standard therapy and/or has failed one or more lines of previous therapy. In some embodiments, the subject has been treated or has previously received at least or at least about or about 1, 2, 3, or 4 other therapies for treating the disease or disorder, such as a large B cell lymphoma or NHL, other than a lymphodepleting therapy and/or the dose of cells expressing the antigen receptor. In some embodiments, the subject has been treated or has previously received a therapy that includes anthracycline, a CD20 targeted agent, and/or ibrutinib.

In some embodiments, the subject has been previously treated with chemotherapy or radiation therapy. In some aspects, the subject is refractory or non-responsive to the other therapy or therapeutic agent. In some embodiments, the subject has persistent or relapsed disease, e.g., following treatment with another therapy or therapeutic intervention, including chemotherapy or radiation.

In some embodiments, the subject is one that is eligible for a transplant, such as is eligible for a hematopoietic stem cell transplantation (HSCT), *e.g.,* allogeneic HSCT. In some embodiments, the subject is one that is eligible for a transplant, such as is eligible for a hematopoietic stem cell transplantation (HSCT), *e.g.,* autologous HSCT. In some such embodiments, the subject has not previously received a transplant, despite being eligible, prior to administration of the engineered cells (e.g. CAR-T cells) or a composition containing the cells to the subject as provided herein.

In some embodiments, the subject is one that is not eligible for a transplant (also known as transplant non-eligible, TNE), such as is not eligible for a hematopoietic stem cell transplantation (HSCT), *e.g.,* allogeneic HSCT. In some embodiments, such a subject is administered the engineered cells (*e.g.* CAR-T cells) or a composition containing the cells according to the provided embodiments herein.

In some embodiments, the subject is one that is not eligible for hematopoietic stem cell transplant because the subject met at least one of the following criteria: ≥70 years of age, ECOG PS of 2, and/or impaired pulmonary (DLCO ≤60%, but SaO₂≥92% on room air and CTCAE ≤1 dyspnea), cardiac (LVEF >40% and <50%), renal (creatinine clearance >30 and <60 mL/min), or hepatic function (AST/ALT >2 and ≤5 x ULN).

In some of any embodiments, at or immediately prior to the time of the administration of the dose of cells, the subject is or has been identified as being ineligible for a high-dose chemotherapy. In some of any embodiments, at or immediately prior to the time of the administration of the dose of cells, the subject is or has been identified as being ineligible for a hematopoietic stem cell transplantation (HSCT). In some of any embodiments, at or immediately prior to the time of the administration of the dose of cells, the subject is or has been identified as being ineligible for both a high-dose chemotherapy and a hematopoietic stem cell transplantation (HSCT).

In some of any embodiments, the subject has a relapsed/refractory NHL, and at or immediately prior to the time of the administration of the dose of cells, the subject is or has been identified as being ineligible for both a high-dose chemotherapy and a hematopoietic stem cell transplantation (HSCT), and the subject has relapsed following remission after treatment with, or become refractory to, one prior therapy for the disease or condition other than another dose of cells expressing the CAR.

In some of any embodiments, at or prior to the administration of the dose of cells the subject is or has been identified as age 70 years or older. In some of any embodiments, the subject is or has been identified as having an ECOG performance status of 2. In some of any embodiments, the subject is or has been identified as having an impaired pulmonary function, optionally with a diffusing capacity of the lungs for carbon monoxide (DLCO) of at or about 60% or less. In some of any embodiments, the subject is or has been identified as having an impaired cardiac function, optionally with a left ventricular ejection fraction (LVEF) of less than at or about 50%. In some of any embodiments, the subject is or has been identified as having an impaired renal function, optionally with a calculated creatinine clearance of less than at or about 60 mL/min. In some of any embodiments, the subject is or has been identified as having an impaired hepatic function, optionally with an aspartate aminotransferase (AST) and alanine aminotransferase (ALT) of more than at or about twice the upper limit of normal (ULN).

In some embodiments, the subject has a lymphoma that is associated with or involves central nervous system (CNS) involvement, and the subject has been previously treated with an anticonvulsant, such as levetiracetam.

In some embodiments, the methods include administration of cells to a subject selected or identified as having a high-risk large B cell lymphoma or a high-risk NHL. In some embodiments, the subject exhibits one or more cytogenetic abnormalities, such as associated with the B cell malignancy, such as a high-risk B cell lymphoma or a high-risk NHL. In some embodiments, the subject is selected or identified based on having a disease or condition characterized or determined to be aggressive NHL, diffuse large B cell lymphoma (DLBCL), primary mediastinal large B cell lymphoma (PMBCL), T cell/histocyte-rich large B cell lymphoma (TCHRBCL), Burkitt's lymphoma (BL), mantle cell lymphoma (MCL), and/or follicular lymphoma (FL). In particular embodiments, the subject to be treated using the methods provided herein include subjects with an aggressive large B cell lymphoma or an aggressive NHL, in particular, with diffuse large B-cell lymphoma (DLBCL), not otherwise specified (NOS; de novo or transformed from indolent), primary mediastinal B-cell lymphoma (PMBCL) or follicular lymphoma grade 3B (FL3B). In some of any embodiments, the subject has follicular lymphoma (FL). In particular embodiments, the subject to be treated using the methods provided herein include subjects with DLBCL that is transformed from a follicular lymphoma (FL), or another indolent lymphoma. In particular embodiments, the subject to be treated using the methods provided herein include subjects with DLBCL that is transformed from indolent histology (tDLBCL). In some embodiments, the subject has DLBCL transformed from marginal zone lymphoma (MZL) or chronic lymphocytic leukemia (CLL) (e.g., Richter's). In some embodiments, a subject with transformation from CLL can exhibit Richter's syndrome (RS), defined as the transformation of CLL into an aggressive lymphoma, most commonly diffuse large B-cell lymphoma (DLBCL) (see, e.g., Parikh et al. Blood 2014 123:1647-1657).

In some embodiments, the subject has mantle cell lymphoma (MCL). In some embodiments, the MCL is characterized by the chromosomal translocation t(11:14)(q13;132) (Vose JM, et al. Am J Hematol. 2017.; 92:806-813). In some embodiments, the subject has poor risk factors including TP53 mutations and/or a high proliferation index (Ki67>30%). In some embodiments, the subject has poor risk factors including prior bone marrow involvement, prior pleural effusions and/or CNS disease. In some embodiments, the subject has poor risk factors including MCL variants. In some embodiments, the subject has a blastoid variant of MCL. In some embodiments, the subject has a pleiomorphic variant of MCL. In some embodiments, the subjects has mantle cell lymphoma (MCL) that has failed (relapsed/refractory, R/R) after ≥ 1 prior lines of therapy. In some embodiments, the subjects has mantle cell lymphoma (MCL) that has failed (relapsed/refractory, R/R) after 1, 2, 3, 4, 5, 6 or 7 prior lines of therapy. In some embodiments, the subject had received prior ibrutinib and/or venetoclax. In some embodiments, the subject has MCL that has relapsed after receiving ibrutinib and/or venetoclax. In some embodiments, the subject had received 1 or more prior lines of immunochemotherapy containing an anthracycline and a CD20-targeted agent (e.g., R-CHOP). In some embodiments, the subject had received prior hematopoietic stem cell therapy (HSCT), e.g., allogeneic HSCT or autologous HSCT. In some embodiments, the subject has confirmed cyclin D1 expressing MCL with R/R disease.

In some of any embodiments, at or prior to the administration of the dose of cells, the subject is or has been treated with an anthracycline and one or more CD20-targeted agent. In some of any embodiments, the one or more CD20-targeted agent comprises rituximab. In some of any embodiments, the one or more CD20-targeted agent comprises R-CHOP (rituximab, cyclophosphamide, doxorubicin hydrochloride (hydroxydaunomycin), vincristine sulfate (oncovin) and prednisone).

In some embodiments, the subject has poor performance status. In some aspects, the population to be treated includes subjects having an Eastern Cooperative Oncology Group Performance Status (ECOG) that is anywhere from 0-2. In other aspects of any of the embodiments, the subjects to be treated included ECOG 0-1 or do not include ECOG 2 subjects. In some aspects of any of the embodiments, the subjects to be treated have failed two or more prior therapies. In some embodiments, the subject does not have DLBCL transformed from marginal zone lymphoma (MZL) or chronic lymphocytic leukemia (CLL) (e.g., Richter's). In some embodiments, the subject has features that correlate with poor overall survival. In some embodiments, the subject has never achieved a complete response (CR), never received autologous stem cell transplant (ASCT), is refractory to 1 or more second line therapy, has primary refractory disease, and/or has an ECOG performance score of 2 or an ECOG score of between 0 and 1. In some embodiments, the subject is or has been identified as having ECOG performance status of 0 or 1.

In some embodiments, the subject to be treated includes a group of subjects with diffuse large B-cell lymphoma (DLBCL), de novo or transformed from indolent lymphoma (not otherwise specified, NOS), primary mediastinal large b-cell lymphoma (PMBCL), and follicular lymphoma grade 3b (FL3B) after failure of 2 lines of therapy, and ECOG score of 0-2, and the subject may optionally have previously been treated with allogeneic stem cell transplantation (SCT). In some of any embodiments, the subject to be treated has follicular lymphoma (FL). In some embodiments, such subject group can be referred to as the "full cohort." In some embodiments, the subject is selected for treatment with adoptive cell therapy, if the subject meets said criteria. In some embodiments, within said group ("full cohort"), the subject is not selected for treatment or excluded from treatment, if the subject has a poor performance status (e.g. ECOG 2) and/or has DLBCL transformed from marginal zone lymphomas (MZL) or chronic lymphocytic leukemia (CLL, Richter's). Thus, in some embodiments, the subject is selected for treatment if the subject has subjects with diffuse large B-cell lymphoma (DLBCL), de novo or transformed from indolent lymphoma (NOS), primary mediastinal large b-cell lymphoma (PMBCL), and follicular lymphoma grade 3b (FL3B) after failure of 2 lines of therapy, and ECOG score of 0 or 1, and the subject may optionally have previously been treated with allogeneic stem cell transplantation (SCT) but does not have DLBCL transformed from marginal zone lymphomas (MZL) or chronic lymphocytic leukemia (CLL, Richter's). In some embodiments, the subject is selected for treatment if the subject has follicular lymphoma (FL).

In some of any embodiments, at or prior to the administration of the dose of cells the subject is or has been identified as having a double/triple hit lymphoma. In some of any embodiments, the subject is or has been identified as having a chemorefractory lymphoma, optionally a chemorefractory DLBCL. In some of any embodiments, the subject has not achieved complete remission (CR) in response to a prior therapy. In some of any embodiments, the subject has relapsed within 1 year or less than 1 year after receiving an autologous stem cell transplant (ASCT).

In some embodiments, such subject group can be referred to as the "core cohort." In some embodiments, the subject to be treated is subjects in the "core cohort." In some aspects, the provided embodiments are based on observations that certain subject population, for example, the "core cohort" subjects who have been administered a certain dose of the cell therapy, show an overall response rate (ORR) of more than 80%, with a complete response (CR) rate of more than 55%, with high durability, e.g., response that is maintained over a longer period of time, e.g., more than 3 months, with a 3-month ORR of over 65%, and a 3-month CR rate of approximately 50%. In particular, the provided observations indicated that the 3-month ORR was high in subjects with two or three chromosomal translocations ("double-hit" or "triple-hit" lymphoma; having translocations MYC/8q24 loci, usually in combination with the t(14; 18) (q32; q21) bcl-2 gene or/and BCL6/3q27 chromosomal translocation; see, e.g., Xu et al. (2013) Int J Clin Exp Pathol. 6(4): 788-794), primary-refractory lymphomas, chemorefractory DLBCL, and subjects who have never previously achieved CR.

In some aspects, provided are compositions, methods and uses for administration of a defined composition of the cell therapy, at particular doses, that are associated with a high response rate and/or high durability of response, and low levels and/or incidence of toxicity. In some embodiments, the composition or dose administered is a flat and/or fixed dose, such as a precise flat dose, of cells and/or of one or more cells having a particular phenotype, such as a particular number of such cells or a number that is within a particular range and/or degree of variability or variance as compared to a target number. In some embodiments, the composition or dose administered contains a defined ratio of CD4⁺ and CD8⁺ cells (e.g., 1:1 ratio of CD4⁺:CD8⁺ CAR⁺ T cells) and/or contains a ratio that is within a certain degree of variability from such ratio, such as no more than ± 10%, such as no more than ± 8%, such as a degree of variability or variance of no more than ± 10%, such as no more than ± 8%. In some embodiments, the CD4⁺ and CD8⁺ cells are individually formulated and administered. In some embodiments, the administered cells exhibit consistent activity and/or function, e.g., cytokine production, apoptosis and/or expansion. In some embodiments, the provided compositions exhibit highly consistent and defined activity, and low variability between cells, e.g., in terms of cell number, cell function and/or cell activity, in the composition or between preparations. In some embodiments, the consistency in activity and/or function, e.g., low variability between preparations of compositions, allows improved efficacy and/or safety. In some embodiments, administration of the defined compositions resulted in low product variability and low toxicity, e.g., CRS or neurotoxicity, compared to administration of cell compositions with high heterogeneity. In some embodiments, the defined, consistent composition also exhibits consistent cell expansion. Such consistency can facilitate the identification of dose, therapeutic window, evaluation of dose response and identification of factors of the subject that may correlate with safety or toxicity outcomes.

In some embodiments, in a certain cohort of subjects receiving a single infusion of a particular dose level, a durable response rate after 6 months of greater than 60% can be achieved. In some embodiments, the subjects in some cohorts can achieve an overall response rate (ORR, in some cases also known as objective response rate) of more than 80%, a complete response (CR) rate of more than 60% and/or a high durable CR rate at 6 months. In some embodiments, subjects receiving a defined dose show improved safety outcomes, e.g., more than two-thirds of the subjects that do not exhibit any CRS or NT. In some aspects, the rate of severe CRS or severe NT is low. In some embodiments, a higher exposure (e.g., Cₘₐₓ and AUC₀₋₂₈) observed with a particular defined dose, does not associate with increased toxicity, e.g., CRS or NT. In some embodiments, particular factors of the subject, e.g., certain biomarkers, can be used to predict the risk of toxicity. In some embodiments, the provided embodiments can be used to achieve high response rate with low risk of toxicity.

In some embodiments, no more than 25%, no more than 20%, no more than 15%, no more than 10% or no more than 5% of subjects treated using the provided compositions, articles of manufacture, kits, methods and uses are administered an agent (e.g. tocilizumab and/or dexamethasone) to ameliorate, treat or prevent a toxicity, either prior to or subsequent to administration of the cell therapy. In some embodiments, the subject is not administered any prophylaxis treatment prior to receiving the engineered cells (e.g. CAR-T cells).

In some embodiments, the provided embodiments provide an advantage, e.g., permits administration of the cell therapy on an outpatient basis. CAR T cell therapy has generally been administered in inpatient settings, such as at university medical centers. However, many subjects with R/R diffuse large B cell lymphoma receive therapy at medical centers where outpatient delivery of cancer therapy is carried out. In some aspects, infusion and management of CAR T cell therapies in the outpatient setting may improve access to such therapies, including wider utilization of outpatient treatment in community/non-university centers. In some embodiments, the administration of the cell dose and/or the lymphodepleting therapy is carried out in a non-tertiary care center. In some embodiments, the administration of the cell therapy, e.g. dose of T cells in accord with the provided embodiments, can be performed on an outpatient basis or does not require admission to the subject to the hospital, such as admission to the hospital requiring an overnight stay. In some embodiments, such outpatient administration can allow increased access and decreased costs, while maintaining a high, durable response rate with low toxicity. In some aspects, outpatient treatment can be advantageous for patients who already are otherwise immunocompromised by prior treatments, e.g. post-lympodepletion, and are at a greater risk for exposures at a hospital stay or in an inpatient setting. In some aspects, outpatient treatments also increases options for treatment for subjects who may not have access to inpatient, hospital settings, or transplant centers, thereby expanding access to the treatment. In some embodiments, after the administration of a dose of the cells, the subject is monitored in an outpatient setting, optionally via contacting by telephone and/or a visit by a healthcare professional.

In some embodiments, subjects treated on an outpatient basis using the provided compositions, articles of manufacture, kits, methods and uses remain in outpatient for at least 3 days or a certain percentage of subjects, e.g. at least 60%, at least 70%, at least 80%, at least 85%, at least 90% or at least 95%, of subjects so treated remain in outpatient for at least 3 days. In some aspects, the subjects remain in outpatient for at least 4 days, 5 days, 6 days, 7 days, 8 days or more. In some embodiments, subjects treated using the provided compositions, articles of manufacture, kits, methods and uses show a reduction in the duration of hospital stay, e.g., of at least 10%, at least 15%, at least 20%, at least 25%, at least 30%, at least 35% or at least 40%, compared to subjects treated with other compositions, articles of manufacture, kits, methods and uses.

In some embodiments, the methods, cells and compositions can provide high rate of durable response to subjects across a range of patient characteristics and/or tumor burden. In some embodiments, the methods, cells and compositions can provide high rate of durable response to high risk patients with poor prognosis, with a reduced risk of adverse effects or toxicities. In some embodiments, the methods and uses provide for or achieve a higher response rate and/or more durable responses or efficacy and/or a reduced risk of toxicity or other side effects that can be associated with cell therapy, such as neurotoxicity (NT) or cytokine release syndrome (CRS). In some aspects, the provided observations indicated a low rate of severe NT (sNT) or severe CRS (sCRS), and a high rate of patients without any toxicities, e.g., NT or CRS.

In some embodiments, at least 35%, at least 40%, at least 50%, at least 55%, at least 60%, at least 65%, at least 70%, or at least 75% or more of the subjects treated according to the provided methods, and/or with the provided articles of manufacture or compositions, achieve a complete response (CR). In some embodiments, at least 50%, at least 60%, at least 70%, at least 80%, or at least 90% of the subjects treated according to the provided methods, and/or with the provided articles of manufacture or compositions, achieve an objective response (OR). In some embodiments, at least 60%, at least 70%, at least 80%, at least 90%, at least 95% or more of the subjects treated according to the provided methods, and/or with the provided articles of manufacture or compositions, achieve a CR or OR by one month, by two months or by 3 months.

In some embodiments, by 3 months, four months, five months, six months or more after initiation of administration of the cell therapy, at least 60%, at least 70%, at least 80%, at least 90%, at least 95% or more of the subjects treated according to the provided methods, and/or with the provided articles of manufacture or compositions, remain in response, such as remain in CR or OR. In some embodiments, such response, such as CR or OR, is durable for at least 3 months, four months, five months, six months, seven months, eight months or 9 months, such as in at least or at least about 60%, at least 70%, at least 80%, at least 90%, at least 95% or more of the subjects treated according to the provided methods or in such subjects who achieve a CR by one month or by 3 months. In some embodiments, at least 60%, at least 70%, at least 80%, at least 90%, at least 95% or more of the subjects treated according to the provided methods, and/or with the provided articles of manufacture or compositions, or such subjects who achieve a CR by one month or by 3 months, survive or survive without progression for greater than or greater than about 3 months, four months, five months, six months, seven months, eight months or 9 months.

In some embodiments, the resulting response observed in such subjects by the treatment in accord with the provided methods, and/or with the provided articles of manufacture or compositions, is associated with or results in a low risk of any toxicity or a low risk of severe toxicity in a majority of the subjects treated. In some embodiments, greater than or greater than about 30%, 35%, 40%, 50%, 55%, 60% or more of the subjects treated according to the provided methods and/or with the provided articles of manufacture or compositions do not exhibit any grade of CRS or any grade of neurotoxicity (NT). In some embodiments, greater than or greater than about 50%, 60%, 70%, 80% or more of the subjects treated according to the provided methods and/or with the provided articles of manufacture or compositions do not exhibit severe CRS or grade 3 or higher CRS. In some embodiments, greater than or greater than about 50%, 60%, 70%, 80% or more of the subjects treated according to the provided methods, and/or with the provided articles of manufacture or compositions, do not exhibit severe neurotoxicity or grade 3 or higher neurotoxicity, such as grade 4 or 5 neurotoxicity.

In some embodiments, at least at or about 45%, 50%, 60%, 65%, 70%, 75%, 80%, 85%, 90%, or 95% of subjects treated according to the method and/or with the provided articles of manufacture or compositions do not exhibit early onset CRS or neurotoxicity and/or do not exhibit onset of CRS earlier than 1 day, 2 days, 3 days or 4 days following initiation of the administration. In some embodiments, at least at or about 45%, 50%, 60%, 65%, 70%, 75%, 80%, 85%, 90%, or 95% of subjects treated according to the methods, and/or with the provided articles of manufacture or compositions, do not exhibit onset of neurotoxicity earlier than 3 days, 4 days, 5 days, six days or 7 days following initiation of the administration. In some aspects, the median onset of neurotoxicity among subjects treated according to the methods, and/or with the provided articles of manufacture or compositions, is at or after the median peak of, or median time to resolution of, CRS in subjects treated according to the method. In some cases, the median onset of neurotoxicity among subjects treated according to the method is greater than at or about 8, 9, 10, or 11 days.

In some embodiments, such results are observed following administration of from or from about 5 x 10⁷ to or to about 1.5 x 10⁸, such as from or from about 5 x 10⁷ to or to about 1 x 10⁸ total recombinant receptor-expressing T cells (e.g. CAR+ T cells), such as a dose of T cells including CD4⁺ and CD8⁺ T cells administered at a defined ratio as described herein, e.g. at or about a 1:1 ratio, and/or at a precise or flat or fixed number of CAR⁺ T cells, or precise or flat or fixed number of a particular type of CAR⁺ T cells such as CD4⁺CAR⁺ T cells and/or CD8⁺CAR⁺ T cells, and/or a number of any of such cells that is within a specified degree of variance, such as no more than, + or - (plus or minus, in some cases indicated as ±), 5, 6, 7, 8, 9, 10, 11, 12, 13, 14, or 15% as compared to such precise or flat or fixed number. In some embodiments, such flat or fixed number of cells is at or about 2.5 x 10⁷, 5×10⁷, 10 x 10⁷, 15 x 10⁷ or 20 x 10⁷, e.g., of total CAR⁺ T cells or of CD8⁺ and/or CD4⁺ CAR⁺ T cells. In some embodiments, the number of cells in the dose includes or consists of or consists essentially of 5 x 10⁷ CD4⁺CAR⁺ T cells (in some cases 2.5 x 10⁷ CD4⁺CAR⁺ T cells and 2.5 x 10⁷ CD8⁺CAR⁺ T cells); in some embodiments, it includes or consists of or consists essentially of 10 × 10⁷ CAR⁺ T cells (in some cases 5 x 10⁷ CD4⁺CAR⁺ T cells and 5 x 10⁷ CD8⁺CAR⁺ T cells). In some aspects, the number of cells administered, is within a certain degree of variance of such numbers in the aforementioned embodiments, such as within plus or minus (±) 5, 6, 7, 8, 9, or 10%, such as within plus or minus 8%, as compared to such number(s) of cells. In some aspects, the dose is within a range in which a correlation is observed (optionally a linear relationship) between the number of such cells (e.g., of total CAR⁺ T cells or of CD8⁺ and/or CD4⁺ CAR⁺ T cells) and one or more outcomes indicative of therapeutic response, or duration thereof (e.g., likelihood of achieving a remission, a complete remission, and/or a particular duration of remission) and/or duration of any of the foregoing. In some aspects, it is found that the higher dose of cells administered can result in greater response without or without substantially impacting or affecting the incidence or risk of toxicity (e.g. CRS or neurotoxicity), or degree of incidence or risk of toxicity, in the subject e.g. severe CRS or severe neurotoxicity.

In some aspects, the provided methods can achieve a high or a particular rate of response (such as a rate of response among a population as assessed after a certain period post-administration, such as 3 months or six months), e.g., ORR (such as a 6-month or 3-month ORR) of 40% or more, 45% or more, 50% or more, 55% or more, 60% or more, 65% or more, 70% or more, 75% or 80% or 81%, 82%, 83%, 84% or 85% or more and CR rate (such as a 6-month or 3-month CR rate) of 30% or more, 35% or more, 40% or more, 45% or more, 50% or more, 55% or more, 60% or more, 65% or more, 70% or more, 71%, 72%, 73% or more or approximately 75% or more, which also is durable such as for a particular period of time or at least a particular period of time, e.g., is sustained for more than 1, 3 or 6 months or more or 9 months or more after initiation of therapy. In some embodiments, such rates of response and durability are received following only a single administration or dose of such therapy. Treatment of such subjects by the provided methods, and/or with the provided articles of manufacture or compositions, in some embodiments, also result in the subjects achieving the high rate of response, yet not exhibiting higher incidence of developing toxicities, such as neurotoxicity or CRS, even at a higher cell dosage. In some embodiments, about or greater than 50%, 55% or 60% of subjects achieving such responses do not develop any grade of toxicity, such as any grade of CRS and/or neurotoxicity.

Thus, in some embodiments, the provided methods, articles of manufacture and/or compositions, can offer advantages over other available methods or solutions or approaches for treatment such as for adoptive cell therapy. In particular, among the provided embodiments are those that offer an advantage for subjects with high-risk NHL, by achieving a durable response at a high rate, with reduced incidence of toxicities or side effects.

### A. Method of Treatment

Provided herein are methods of treatment that involve administering engineered cells or compositions containing engineered cells, such as engineered T cells. Also provided are methods and uses of engineered cells (e.g., T cells) and/or compositions thereof, including methods for the treatment of subjects having a disease or condition such as a leukemia or a lymphoma or a B-cell malignancy, e.g., a diffuse large B-cell lymphoma (DLBCL) or a subtype thereof, a large B cell lymphoma and/or a non-Hodgkin lymphoma (NHL), that involves administration of the engineered cells and/or compositions thereof. In some embodiments, the provided methods and uses can achieve improved response and/or more durable responses or efficacy and/or a reduced risk of toxicity or other side effects, e.g., in particular groups of subjects treated, as compared to certain alternative methods. In some aspects, also provided are methods of administering engineered cells or compositions containing engineered cells, such as engineered T cells, to a subject, such as a subject that has a disease or disorder. In some aspects, also provided are uses of engineered cells or compositions containing engineered cells, such as engineered T cells for treatment of a disease or disorder. In some aspects, also provided are uses of engineered cells or compositions containing engineered cells, such as engineered T cells for the manufacture of a medicament for the treatment of a disease or disorder. In some aspects, also provided are methods of administering engineered cells or compositions containing engineered cells, such as engineered T cells, for use in treatment of a disease or disorder, or for administration to a subject having a disease or disorder. In some aspects, the uses of the engineered cells or compositions containing engineered cells, such as engineered T cells are in accord with any of the methods described herein.

Also provided are methods of treatment involving selecting a subject that a disease or condition, such as any disease or condition described herein; and administering to the subject a dose of T cells comprising T cells expressing a recombinant receptor that specifically binds to a target antigen expressed by the disease or condition or a cell or tissue thereof and/or that is associated with the disease or condition.

The engineered cells expressing a recombinant receptor, such as a chimeric antigen receptor (CAR), or compositions comprising the same are useful in a variety of therapeutic, diagnostic and prophylactic indications. For example, the engineered cells or compositions comprising the engineered cells are useful in treating a variety of diseases and disorders in a subject. Such methods and uses include therapeutic methods and uses, for example, involving administration of the engineered cells, or compositions containing the same, to a subject having a disease, condition, or disorder, such as a B cell malignancy, e.g., a large B cell lymphoma. In some embodiments, the engineered cells or compositions comprising the same are administered in an effective amount to effect treatment of the disease or disorder. Uses include uses of the engineered cells or compositions in such methods and treatments, and in the preparation of a medicament in order to carry out such therapeutic methods. In some embodiments, the methods are carried out by administering the engineered cells, or compositions comprising the same, to the subject having or suspected of having the disease or condition. In some embodiments, the methods thereby treat the disease or condition or disorder in the subject.

General methods for administration of cells for adoptive cell therapy are known and may be used in connection with the provided methods and compositions. For example, adoptive T cell therapy methods are described, e.g., in US Patent Application Publication No. 2003/0170238 to Gruenberg et al; US Patent No. 4,690,915 to Rosenberg; Rosenberg (2011) Nat Rev Clin Oncol. 8(10):577-85). See, e.g., Themeli et al. (2013) Nat Biotechnol. 31(10): 928-933; Tsukahara et al. (2013) Biochem Biophys Res Commun 438(1): 84-9; Davila et al. (2013) PLoS ONE 8(4): e61338.

The disease or condition that is treated can be any in which expression of an antigen is associated with and/or involved in the etiology of a disease condition or disorder, e.g. causes, exacerbates or otherwise is involved in such disease, condition, or disorder. Exemplary diseases and conditions can include diseases or conditions associated with malignancy or transformation of cells (e.g. cancer), autoimmune or inflammatory disease, or an infectious disease, e.g. caused by a bacterial, viral or other pathogen. Exemplary antigens, which include antigens associated with various diseases and conditions that can be treated, are described above. In particular embodiments, the chimeric antigen receptor (CAR) or transgenic TCR specifically binds to an antigen associated with the disease or condition.

Among the diseases, conditions, and disorders are tumors, including solid tumors, hematologic malignancies, and melanomas, and including localized and metastatic tumors, infectious diseases, such as infection with a virus or other pathogen, e.g., HIV, HCV, HBV, CMV, HPV, and parasitic disease, and autoimmune and inflammatory diseases.

In some embodiments, the disease, disorder or condition to be treated is a tumor, cancer, malignancy, neoplasm, or other proliferative disease or disorder. Such diseases include but are not limited to leukemia, lymphoma, e.g., acute myeloid (or myelogenous) leukemia (AML), chronic myeloid (or myelogenous) leukemia (CML), acute lymphocytic (or lymphoblastic) leukemia (ALL), chronic lymphocytic leukemia (CLL), hairy cell leukemia (HCL), small lymphocytic lymphoma (SLL), Mantle cell lymphoma (MCL), Marginal zone lymphoma (MZL), Burkitt lymphoma (BL), Hodgkin lymphoma (HL), non-Hodgkin lymphoma (NHL), Anaplastic large cell lymphoma (ALCL), follicular lymphoma (FL), refractory follicular lymphoma, diffuse large B-cell lymphoma (DLBCL) and multiple myeloma (MM).

In some embodiments, the disease or condition to be treated is a B cell malignancy. In some embodiments, disease or condition is a B cell malignancy selected from among acute lymphoblastic leukemia (ALL), adult ALL, chronic lymphoblastic leukemia (CLL), non-Hodgkin lymphoma (NHL), and Diffuse Large B-Cell Lymphoma (DLBCL). In some embodiments, the disease or condition is an NHL. In some embodiments, the disease or condition is a large B cell lymphoma. In some embodiments, the disease or condition is a DLBCL. In some embodiments, the disease or condition is a DLBCL, not otherwise specified (DLBCL, NOS). In some embodiments, the disease or condition is NHL and the NHL is selected from the group consisting of aggressive NHL, diffuse large B cell lymphoma (DLBCL), NOS (de novo or transformed from indolent), primary mediastinal large B cell lymphoma (PMBCL), T cell/histocyte-rich large B cell lymphoma (TCHRBCL), Burkitt's lymphoma (BL), mantle cell lymphoma (MCL), and/or follicular lymphoma (FL). In some embodiments, the disease or condition is a follicular lymphoma (FL). In some embodiments, the follicular lymphoma is a follicular lymphoma Grade 3B (FL3B). In some embodiments, the disease or condition is mantle cell lymphoma (MCL).

In some embodiments, the disease or condition to be treated according to the provided methods, uses or articles of manufacture, is DLBCL. In some aspects, DLBCL is a DLBCL, not otherwise specified (NOS), which in some cases, can be characterized as de novo or transformed from an indolent disease.

In some embodiments, a subject having a DLBCL, NOS selected for treatment and/or that is treated in accord with any of the provided methods, has a DLBCL that is not a DLBCL with predominant extranodal location, a DLBCL that is not a large-cell lymphoma of terminally differentiated B cells, or a DLBCL that is not a B cell neoplasm with features intermediated between DLBCL and other lymphoid tumors.

In some embodiments, a subject having a DLBCL, NOS selected for treatment and/or that is treated in accord with any of the provided methods, has a DLBCL that is not a T cell/histocyte-rich large B cell lymphoma (TCHRBCL), that is not a primary DLBCL of the central nervous system (CNS), that is not a primary cutaneous DLBCL, leg type, or a Epstein-Barr virus (EBV)-positive DLBCL (e.g., an EBV-positive DLBCL of the elderly), and in some cases a DLBCL that is not a DLBCL associated with chronic inflammation.

In some embodiments, a subject having a DLBCL, NOS selected for treatment and/or that is treated in accord with any of the provided methods, has a high-grade B cell lymphoma that is not a B-lymphoblastic leukemia/lymphoma (B-LBL), a high-grade B cell lymphoma that is not a Burkitt lymphoma, or a high-grade B cell lymphoma that is not a high-grade B cell lymphoma with MYC and BCL2 and/or BCL6 rearrangements. In some aspects, DLBCL is be a DLBCL, NOS, which in some cases, can be characterized as a high-grade B cell lymphoma that is not a B-lymphoblastic leukemia/lymphoma (B-LBL), a high-grade B cell lymphoma that is not a Burkitt lymphoma, or a high-grade B cell lymphoma that is not a high-grade B cell lymphoma with MYC and BCL2 and/or BCL6 rearrangements.

In some embodiments, a subject having a DLBCL, NOS selected for treatment and/or that is treated in accord with any of the provided methods, has a DLBCL that is germinal center B-cell-like (GCB) and activated B-cell-like (ABC) based on the molecular and/or cytogenetic features of the cells of origin.

In some embodiments, the DLBCL is a de novo or a primary DLBCL. In some embodiments, the disease or condition (such as the lymphoma such as the DLBCL) is transformed from a different subtype of disease or condition, such as transformed from an indolent lymphoma, such as a follicular lymphoma (FL). In some embodiments, such other indolent lymphomas can include, for example, marginal zone B-cell lymphoma (MZL) and chronic lymphocytic leukemia/small-cell lymphocytic lymphoma (CLL/SLL). In some embodiments, the disease or condition is DLBCL transformed from follicular lymphoma (tFL); in some aspects, it is a DLBCL transformed from another indolent lymphoma. In some embodiments, the subject is suspected or characterized as having transformed follicular lymphoma (tFL). In some embodiments, the disease or condition is a DLBCL transformed from FL. In some aspects, the disease or condition is a DLBCL transformed from an indolent lymphoma other than a FL.

In some embodiments, the disease or condition to be treated according to the provided methods, uses or articles of manufacture, is DLBCL that is transformed from another indolent lymphoma, such as DLBCL transformed from marginal zone lymphoma (tMZL) or DLBCL transformed from chronic lymphocytic leukemia (tCLL; Richter's). In some cases, the disease or condition is DLBCL tMZL or DLBCL tCLL. In some embodiments, it is a disease or condition transformed from an indolent lymphoma other than FL. In some embodiments, it is a DLBCL or a large B cell lymphoma, such as a DLBCL or a large B cell lymphoma transformed from an FL or other indolent lymphoma. In some embodiments, the subject is characterized as having DLBCL transformed from another indolent lymphoma, such as DLBCL tMZL or DLBCL tCLL.

In some embodiments, the disease or condition is a follicular lymphoma (FL). In some embodiments, the subject is selected for treatment if the subject has a follicular lymphoma (FL). In some embodiments, the FL exhibits or is associated with neoplastic follicles that show attenuated mantle zones, loss of polarization, and/or absence of tangible body macrophages. In some embodiments, the FL is associated with a mixture of centrocytes and centroblasts. In some embodiments, the FL is not associated with centrocytes. In some embodiments, the FL is a Grade 3 FL. In some embodiments, the Grade 3 FL exhibits or is associated with more than 15 centroblasts per high-powered field (HPF). In some embodiments, the FL is associated with co-expression of CD10, BCL6 and BCL2 within the follicles. In some embodiments, the FL is associated with or characterized by t(14;18)/IGH-BCL2 and/or BCL6 rearrangements. In some embodiments, the FL is associated with a t(14;18)(q32;q21) translocation. In some aspects, the t(14;18)(q32;q21) translocation places BCL2 expression under the control of the immunoglobulin (Ig) heavy locus (IGH) enhancer. In some aspects, t(14;18) is detected in approximately 90% of grades 1 and 2 FLs, 60 to 70% of grade 3A and 15 to 30% of grade 3B FL cases. In some embodiments, the FL is associated with BCL2 translocations t(2;18) and t(18;22). In some embodiments, the FL associated with translocations t(2;18) and t(18;22) is also associated with BCL6 rearrangements. In some of any embodiments, the FL is associated with co-expression of CD10, BCL6 and BCL2 within the follicles, and/or t(14;18)/(q32;q21) (IGH-BCL2) and/or BCL6 rearrangements.

In some embodiments, the FL involves lymph nodes and/or spleen, bone marrow, peripheral blood, and other extranodal sites. In some embodiments, the FL involves lymph nodes. In some aspects, exemplary features associated with FL include those described in Choi et al. (2018) Arch Pathol Lab Med 142:1330-1340; Luminari et al., (2012) Rev. Brad. Hematol. Hemoter., 34:54-59 and Salles (2007) ASH Education Book, 2007:216-25. In some aspects, in the case of FL, exemplary parameters used to assess the extent of disease burden include such parameters as hemoglobin levels (e.g., <12 g/dL or <10 g/dL), erythrocyte sedimentation rate (ESR), lactic dehydrogenase (LDH) level, and β2-microglubilin (B2M) value, gene expression, single nucleotide polymorphisms (SNPs; e.g. in IL-8, IL-2, Il-12B, and IL1RN), miRNA expression, and protein expression (e.g., CD68, STAT1, FOXP3, CD57). (Salles (2007) ASH Education Book, 2007:216-25). In the case of FL, the extent or burden of disease may be assessed by the Ann Arbor staging system, tumor burden, bulky disease, number of nodal or extranodal sites of disease, and/or bone marrow involvement.

In some aspects, survival rates in subjects, such as subjects with FL, are based on scoring systems developed by the Italian Lymphoma Intergroup (ILI) and/or the International Follicular Lymphoma Prognostic Factor Project (IFLPFP). (Luminari et al., (2012) Rev. Brad. Hematol. Hemoter., 34:54-59). In some aspects, ILI score is based on the independent prognostic roles of age, gender, B symptoms, number of extranodal sites, erythrocyte sedimentation rate (ESR) and lactic dehydrogenase (LDH). In some aspects, the IFLPFP score is based on the risk factors of age, Ann Arbor stage, hemoglobin level, number of nodal site areas, and serum LDH levels. In some cases, IFLPFP scores may be used to characterize or predict overall survival rates of subjects with FL.

In some embodiments, the provided methods involve selecting a subject that has a follicular lymphoma (FL) for treatment; and administering to the subject a dose of T cells comprising T cells expressing a recombinant receptor that specifically binds to a target antigen expressed by FL or a cell or tissue thereof and/or that is associated with FL.

In some of any embodiments, the dose of T cells comprises a dose of CD4⁺ and CD8⁺ T cells, wherein T cells of each dose comprises a recombinant receptor that specifically binds to a target antigen expressed by FL or a cell or tissue thereof and/or that is associated with FL, wherein the administration comprises administering a plurality of separate compositions, the plurality of separate compositions comprising a first composition comprising CD8⁺ T cells and a second composition comprising CD4⁺ T cells.

In some embodiments, the disease or condition is an extranodal high-grade non-Hodgkin B-cell lymphoma. In some embodiments, the extranodal high-grade non-Hodgkin B-cell lymphoma is primary CNS lymphoma (PCNSL). In some embodiments, the PCNSL involves the central nervous system (CNS) without systemic lymphoma presence. In some embodiments, the PCNSL is confined to the brain, spine, cerebrospinal fluid (CSF), and eyes. In some embodiments, the PCNSL is a diffuse large B-cell lymphoma (DLBCL). In some embodiments, the PCNSL is a Burkitt, low-grade or T-cell lymphoma. In some embodiments, the PCNSL includes neurological signs. In some embodiments, the neurological signs include focal neurologic deficits, mental status and behavioral changes, symptoms of increased intracranial pressure, and/or seizures. In some embodiments, exemplary features associated with the disease or condition include those described in Grommes et al. (J. Clin Oncol 2017; 35(21):2410-18).

In some embodiments, the subject for treatment in accordance with the methods provided herein do not have a primary central nervous system lymphoma (PCNSL).

In some embodiments, the disease or condition is a secondary central nervous system lymphoma (SCNSL). In some embodiments, the SCNSL is in patients with systemic lymphoma. In some embodiments, the SCNSL is referred to as metastatic lymphoma. In some embodiments, the SCNSL is a DLBCL. In some embodiments, the SCNSL is an aggressive lymphoma that may involve the brain, meninges, spinal cord, and eyes. In some embodiments, the SCNSL includes leptomeningeal spread. In some embodiments, the SCNSL includes brain parenchymal disease. In some embodiments, exemplary features associated with the disease or condition include those described in Malikova et al.

### (Neurophychiatric Disease and Treatment 2018; 14:733-40.)

In some embodiments, the disease or condition is a high-grade B cell lymphoma with MYC and BCL2 and/or BCL6 rearrangements, optionally with DLBCL histology. In some embodiments, the disease or condition is a DLBCL NOS (de novo or transformed from indolent). In some embodiments, the disease or condition is primary mediastinal B-cell lymphoma (PMBCL) or follicular lymphoma grade 3B (FL3B). In some embodiments, the disease or condition is follicular lymphoma (FL). In some embodiments, it is a DLBCL with CNS involvement. In some embodiments, the subject has a relapse of DLBCL in the central nervous system (secondary CNS lymphoma). In some embodiments, the secondary CNS lymphoma involves the brain parenchyma and/or leptomeninges. In some embodiments, the subject has been treated or has previously received at least or at least about or about 1, 2, 3, 4 or 5 other therapies for treating the disease or disorder. In some embodiments, the subject had received prior methrotrexate, thiotepa and/or cytarabine. In some embodiments, the subject has MCL that has relapsed after receiving methrotrexate, thiotepa and/or cytarabine. In some embodiments, the subject had received prior hematopoietic stem cell therapy (HSCT), e.g., allogeneic HSCT or autologous HSCT.

In some embodiments, the subject has or has been identified as having as having a double/triple hit lymphoma or a lymphoma of the double/triple hit molecular subtypes. In some embodiments, the lymphoma is a double hit lymphoma characterized by the presence of MYC (myelocytomatosis oncogene), BCL2 (B-cell lymphoma 2), and/or BCL6 (B-cell lymphoma 6) gene rearrangements (e.g., translocations). In some embodiments, the gene rearrangement affects the MYC/8q24 locus in combination with another gene rearrangement. For example, the other gene rearrangement includes t(14;18)(q32;q21) involving BCL2. In some embodiments, the gene rearrangements affect the MYC/8q24 locus in combination with BCL6/3q27. In some embodiments, the lymphoma is a triple hit lymphoma characterized by the presence of MYC, BCL2, and BCL6 gene rearrangements; see, e.g., Aukema et al., (2011) Blood 117:2319-2331. In some aspects of such embodiments the subject is ECOG 0-1 or does not have or is not suspected or characterized as having DLBCL transformed from MZL or CLL. In aspects, the therapy is indicated for such subjects and/or the instructions indicate administration to a subject within such population. In some embodiments, based on the 2016 WHO criteria (Swerdlow et al., (2016) Blood 127(20):2375-2390), double/triple hit lymphoma can be considered high-grade B-cell lymphoma, with MYC and BCL2 and/or BCL6 rearrangements with DLBCL histology (double/triple hit).

In some embodiments, NHL can be staged based on the Lugano classification (see, e.g., Cheson et al., (2014) JCO 32(27):3059-3067; Cheson, B.D. (2015) Chin Clin Oncol 4(1):5). In some cases, the stages are described by Roman numerals I through IV (1-4), and limited stage (I or II) lymphomas that affect an organ outside the lymph system (an extranodal organ) are indicated by an E. Stage I represents involvement in one node or a group of adjacent nodes, or a single extranodal lesions without nodal involvement (IE). Stage 2 represents involvement in two or more nodal groups on the same side of the diaphragm or stage I or II by nodal extent with limited contiguous extranodal involvement (IIE). Stage III represents involvement in nodes on both sides of the diaphragm or nodes above the diaphragm with spleen involvement. Stage IV represents involvement in additional non-contiguous extra-lymphatic involvement. In addition, "bulky disease" can be used to describe large tumors in the chest, in particular for stage II. The extent of disease is determined by positron emission tomography (PET)-computed tomography (CT) for avid lymphomas, and CT for non-avid histologies. In some of any embodiments, at or prior to the administration of the dose of cells, the subject to be treated according to the provided embodiments has a positron emission tomography (PET)-positive disease.

In some of any embodiments, at or prior to the administration of the dose of cells, if the subject has received a prior CD19-targeted therapy, a biological sample obtained from the subject after the prior CD19-targeted therapy comprises a cell expressing CD19.

In some embodiments, the Eastern Cooperative Oncology Group (ECOG) performance status indicator can be used to assess or select subjects for treatment, e.g., subjects who have had poor performance from prior therapies (see, e.g., Oken et al. (1982) Am J Clin Oncol. 5:649-655). The ECOG Scale of Performance Status describes a patient's level of functioning in terms of their ability to care for themselves, daily activity, and physical ability (e.g., walking, working, etc.). In some embodiments, an ECOG performance status of 0 indicates that a subject can perform normal activity. In some aspects, subjects with an ECOG performance status of 1 exhibit some restriction in physical activity but the subject is fully ambulatory. In some aspects, patients with an ECOG performance status of 2 is more than 50% ambulatory. In some cases, the subject with an ECOG performance status of 2 may also be capable of self-care; see e.g., Sørensen et al., (1993) Br J Cancer 67(4) 773-775. The criteria reflective of the ECOG performance status are described in **Table 1** below:

| **Table 1. ECOG Performance Status Criteria** | |
|---|---|
| **Grade** | **ECOG performance status** |
| 0 | Fully active, able to carry on all pre-disease performance without restriction |
| 1 | Restricted in physically strenuous activity but ambulatory and able to carry out work of a light or sedentary nature, e.g., light house work, office work |
| 2 | Ambulatory and capable of all self-care but unable to carry out any work activities; up and about more than 50% of waking hours |
| 3 | Capable of only limited self-care; confined to bed or chair more than 50% of waking hours |
| 4 | Completely disabled; cannot carry on any self-care; totally confined to bed or chair |
| 5 | Dead |

In some of any embodiments, at or immediately prior to the time of the administration of the dose of cells, the subject has relapsed following remission after treatment with, or become refractory to, one or more prior therapies for the disease or conditions other than another dose of cells expressing the CAR. In some embodiments, the subject has relapsed following remission after treatment to, or become refractory to, one, two or three or more prior therapies (other than another dose of cells expressing the CAR). In some embodiments, the subject has relapsed following remission after treatment to, or become refractory to, one prior therapies (other than another dose of cells expressing the CAR), for example, such that the dose of cells is a second-line therapy. In some embodiments, the subject has relapsed following remission after treatment to, or become refractory to, two or more prior therapies (other than another dose of cells expressing the CAR), for example, such that the dose of cells is a third-line or later therapy, such as a fourth-line therapy.

In some aspects, subjects to be treated in accordance with the provided embodiments include adult subjects with relapsed or refractory aggressive large B-cell lymphoma (R/R LBCL). Eligible subjects had diffuse large B cell lymphoma (DLBCL, not otherwise specified [NOS]; including transformed DLBCL from indolent histology [tDLBCL]), high-grade B-cell lymphoma with *MYC* and *BCL2* and/or *BCL6* rearrangements with DLBCL histology, primary mediastinal B-cell lymphoma (PMBCL), or follicular lymphoma Grade 3B after treatment with 2 or more prior therapies. In some embodiments, subjects with secondary CNS lymphoma can be treated in accordance with the provided embodiments. In some aspects, subjects who achieved a complete response after infusion of an anti-CD19 CAR but who relapsed can be treated in accordance with the provided embodiments. In some embodiments, subjects who have previously been administered a CAR-expressing T cell therapy, e.g., engineered T cells that express the same CAR+ T cell, that had achieved stable disease (SD) as their best response after the first infusion can be treated in accordance with the provided embodiments, e.g., as a second infusion or cycle of the CAR-expressing T cell therapy.

In some embodiments, at or prior to the administration of the dose of cells: the subject has or has been identified as having a relapsed or refractory large B cell lymphoma; and/or the subject is or has been treated with an anthracycline and one or more CD20-targeted agent; and/or the subject is or has relapsed or refractory disease after two or more lines of therapy or after autologous HSCT; and/or the subject is or has been identified as having an ECOG performance status of 1 or 2; and/or if the subject has received a prior CD19-targeted therapy, a biological sample obtained from the subject after the prior CD19-targeted therapy comprises a cell expressing CD19. In some embodiments, the administration of the cell dose is carried out via outpatient delivery.

In some aspects, subjects to be treated in accordance with the provided embodiments, such as in an outpatient setting, e.g., in non-tertiary centers, include adult patients with relapsed/refractory B-cell NHL. In some aspects, subject to be treated in accordance with the provided embodiments, for example in an outpatient setting, include subjects with diffuse large B cell lymphoma (DLBCL), transformed DLBCL arising from follicular lymphoma (tDLBCL), and high-grade B-cell lymphoma with MYC and BCL2 and/or BCL6 rearrangements with DLBCL histology. In some aspects, subject to be treated in accordance with the provided embodiments, for example in an outpatient setting, include subjects that have been treated with an anthracycline and rituximab and have relapsed/refractory disease after 2 or more systemic lines of therapy for DLBCL or after autologous HSCT.

In some embodiments, prior to administration of the dose of cells that is carried out via carried out via outpatient delivery, the provided embodiments involve identifying or selecting for the administration of the dose of cells a subject that is or has: a relapsed or refractory large B cell lymphoma; and/or an anthracycline and one or more CD20-targeted agent; and/or relapsed or refractory disease after two or more lines of therapy or after autologous HSCT; and/or an ECOG performance status of 1 or 2; and/or if the subject has received a prior CD19-targeted therapy, a biological sample obtained from the subject after the prior CD19-targeted therapy comprises a cell expressing CD19.

In some embodiments, prior to administration of the dose of cells, the provided embodiments involve identifying or selecting for the administration of the dose of cells a subject that has: a double/triple hit lymphoma; a chemorefractory lymphoma, optionally a chemorefractory DLBCL; not achieved complete remission (CR) in response to a prior therapy for treating the malignancy, optionally the NHL; and/or has relapsed within 1 year or less than 1 year after receiving an autologous stem cell transplant (ASCT); and/or has a lymphoma associated with or involving central nervous system (CNS) involvement.

In some aspects, subjects to be treated in accordance with the provided embodiments include adult subjects who have relapsed from, or are refractory to, a single line of immunochemotherapy for aggressive B-cell NHL and are ineligible for HSCT. In some aspects, subjects to be treated in accordance with the provided embodiments include subjects have diffuse large B-cell lymphoma (DLBCL), not otherwise specified (NOS; de novo or transformed follicular lymphoma [tFL]), high-grade B-cell lymphoma with MYC and BCL2 and/or BCL6 rearrangements with DLBCL histology (double/triple hit lymphoma [DHL/THL]), and follicular lymphoma Grade 3B after 1 prior line of immunochemotherapy containing an anthracycline and a CD20-targeted agent. In some aspects, subject to be treated in accordance with the provided embodiments include subjects with secondary CNS involvement. In some aspects, subject to be treated in accordance with the provided embodiments include subjects that are deemed ineligible for both high-dose chemotherapy and HSCT and be transplant ineligible (TNE), while remaining eligible for CAR T cell therapy. In some aspects, subjects that are TNE include subjects that meet at least one of the following TNE criteria: (a) Age ≥70 years; (b) ECOG performance status of 2; and/or (c) impaired pulmonary (diffusing capacity of the lung for carbon monoxide [DLCO] ≤60%, but SaO₂ ≥92% on room air and CTCAE ≤1 dyspnea), cardiac (left ventricular ejection fraction [LVEF] ≥40% and <50%), renal (creatinine clearance >30 and,60 mL/min), or hepatic function (AST/ALT >2 and ≤5 x ULN). In some aspects, subject to be treated in accordance with the provided embodiments include subjects who have previously been administered a CAR-expressing T cell therapy, e.g., engineered T cells that express the same CAR+ T cell, that had achieved a complete response (CR) after the first infusion but who relapsed, e.g., as a second infusion of the CAR-expressing T cell therapy.

In some embodiments, prior to administration of the dose of cells, the provided embodiments involve identifying or selecting for the administration of the dose of cells a subject that has a relapsed/refractory NHL; that is or has been identified as being ineligible for both a high-dose chemotherapy and a hematopoietic stem cell transplantation (HSCT); and that has relapsed following remission after treatment with, or become refractory to, one prior therapy for the disease or condition other than another dose of cells expressing the CAR.

In some embodiments, prior to administration of the dose of cells, the provided embodiments involve identifying or selecting for the administration of the dose of cells a subject that is or has: age 70 years or older; and/or an ECOG performance status of 2; and/or an impaired pulmonary function, optionally with a diffusing capacity of the lungs for carbon monoxide (DLCO) of at or about 60% or less; and/or an impaired cardiac function, optionally with a left ventricular ejection fraction (LVEF) of less than at or about 50%; and/or an impaired renal function, optionally with a calculated creatinine clearance of less than at or about 60 mL/min; and/or an impaired hepatic function, optionally with an aspartate aminotransferase (AST) and alanine aminotransferase (ALT) of more than at or about twice the upper limit of normal (ULN).

In some aspects, the subject for treatment in accordance with the provided embodiments include subjects that have adequate organ function. In some aspects, exemplary criteria for determining adequate organ function include: saturating O₂ concentration SaO₂ ≥ 92% on room air and) Common Terminology Criteria for Adverse Events (CTCAE) ≤1 dyspnea; LVEF ≥ 40%; calculated creatinine clearance (Cockcroft and Gault) > 30 mL/min; AST/ALT ≤ 5 × ULN; adequate bone marrow function to receive lymphodepleting chemotherapy; and/or total bilirubin < 2.0 mg/dL (or < 3.0 mg/dL for subjects with Gilbert's syndrome or lymphomatous infiltration of the liver).

In some embodiments, the disease or condition is or is associated with an infectious disease or condition, such as, but not limited to, viral, retroviral, bacterial, and protozoal infections, immunodeficiency, Cytomegalovirus (CMV), Epstein-Barr virus (EBV), adenovirus, BK polyomavirus. In some embodiments, the disease or condition is an autoimmune or inflammatory disease or condition, such as arthritis, e.g., rheumatoid arthritis (RA), Type I diabetes, systemic lupus erythematosus (SLE), inflammatory bowel disease, psoriasis, scleroderma, autoimmune thyroid disease, Grave's disease, Crohn's disease, multiple sclerosis, asthma, and/or a disease or condition associated with transplant.

In some embodiments, the antigen associated with the disease or disorder is selected from among αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha (IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some embodiments include antigens associated with a B cell malignancy, such as any of a number of known B cell marker. In some embodiments, the antigen is or includes CD20, CD19, CD22, ROR1, CD45, CD21, CD5, CD33, Igkappa, Iglambda, CD79a, CD79b or CD30. In some embodiments, the disease or condition is a B cell malignancy, such as a large B cell lymphoma (e.g., DLBCL) and the antigen is CD19.

In some embodiments, the antigen is or includes a pathogen-specific or pathogen-expressed antigen. In some embodiments, the antigen is a viral antigen (such as a viral antigen from HIV, HCV, HBV, etc.), bacterial antigens, and/or parasitic antigens. In some embodiments, the cell therapy, e.g., adoptive T cell therapy, is carried out by autologous transfer, in which the cells are isolated and/or otherwise prepared from the subject who is to receive the cell therapy, or from a sample derived from such a subject. Thus, in some aspects, the cells are derived from a subject, e.g., patient, in need of a treatment and the cells, following isolation and processing are administered to the same subject.

In some embodiments, the cell therapy, e.g., adoptive T cell therapy, is carried out by allogeneic transfer, in which the cells are isolated and/or otherwise prepared from a subject other than a subject who is to receive or who ultimately receives the cell therapy, e.g., a first subject. In such embodiments, the cells then are administered to a different subject, e.g., a second subject, of the same species. In some embodiments, the first and second subjects are genetically identical. In some embodiments, the first and second subjects are genetically similar. In some embodiments, the second subject expresses the same HLA class or supertype as the first subject.

The cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some embodiments, they are administered by parenteral, intrapulmonary, and intranasal, and, if desired for local treatment, intralesional administration. Parenteral infusions include intramuscular, intravenous, intraarterial, intraperitoneal, or subcutaneous administration. In some embodiments, a given dose is administered by a single bolus administration of the cells. In some embodiments, it is administered by multiple bolus administrations of the cells, for example, over a period of no more than 3 days, or by continuous infusion administration of the cells. In some embodiments, administration of the cell dose or any additional therapies, e.g., the lymphodepleting therapy, intervention therapy and/or combination therapy, is carried out via outpatient delivery.

In some embodiments, administration of the cell dose or any additional therapies, e.g., the lymphodepleting therapy, intervention therapy and/or combination therapy, is carried out via inpatient delivery. In some aspects, administration of the cell dose or any additional therapies, e.g., the lymphodepleting therapy, intervention therapy and/or combination therapy, is performed in an inpatient setting, e.g., at university medical centers. In some aspects, the therapy is received in an outpatient setting, e.g., at non-university medical centers. In some aspects, administration and management of the cell therapy or any additional therapies, e.g., the lymphodepleting therapy, intervention therapy and/or combination therapy, in an outpatient setting can result in wider utilization in community/non-university centers and improved access.

For the prevention or treatment of disease, the appropriate dosage may depend on the type of disease to be treated, the type of cells or recombinant receptors, the severity and course of the disease, whether the cells are administered for preventive or therapeutic purposes, previous therapy, the subject's clinical history and response to the cells, and the discretion of the attending physician. The compositions and cells are in some embodiments suitably administered to the subject at one time or over a series of treatments.

In some embodiments, the cells are administered as part of a combination treatment, such as simultaneously with or sequentially with, in any order, another or additional therapeutic intervention, such as an antibody or engineered cell or receptor or agent, such as a cytotoxic or therapeutic agent. The cells in some embodiments are co-administered with one or more additional therapeutic agents or in connection with another therapeutic intervention, either simultaneously or sequentially in any order. In some embodiments, the additional therapeutic agent is any interventions or agents described herein, such as any interventions or agents descried that can ameliorate symptoms of toxicity described herein, for example, in Section II. In some contexts, the cells are co-administered with another therapy sufficiently close in time such that the cell populations enhance the effect of one or more additional therapeutic agents, or vice versa. In some embodiments, the cells are administered prior to the one or more additional therapeutic agents. In some embodiments, the cells are administered after the one or more additional therapeutic agents. In some embodiments, the one or more additional agents include a cytokine, such as IL-2, for example, to enhance persistence. In some embodiments, the methods comprise administration of a chemotherapeutic agent.

In some embodiments, the methods comprise administration of a chemotherapeutic agent, e.g., a conditioning chemotherapeutic agent, for example, to reduce tumor burden prior to the administration.

Preconditioning subjects with immunodepleting (e.g., lymphodepleting) therapies in some aspects can improve the effects of adoptive cell therapy (ACT).

Thus, in some embodiments, the methods include administering a preconditioning agent, such as a lymphodepleting or chemotherapeutic agent, such as cyclophosphamide, fludarabine, or combinations thereof, to a subject prior to the initiation of the cell therapy. For example, the subject may be administered a preconditioning agent at least 2 days prior, such as at least 3, 4, 5, 6, or 7 days prior, to the initiation of the cell therapy. In some embodiments, the subject is administered a preconditioning agent no more than 7 days prior, such as no more than 6, 5, 4, 3, or 2 days prior, to the initiation of the cell therapy.

In some embodiments, the subject is preconditioned with cyclophosphamide at a dose between or between about 20 mg/kg and 100 mg/kg body weight of the subject, such as between or between about 40 mg/kg and 80 mg/kg. In some aspects, the subject is preconditioned or administered with or with about 60 mg/kg of cyclophosphamide. In some embodiments, the cyclophosphamide can be administered in a single dose or can be administered in a plurality of doses, such as given daily, every other day or every three days. In some embodiments, the cyclophosphamide is administered once daily for one or two days. In some embodiments, where the lymphodepleting agent comprises cyclophosphamide, the subject is administered cyclophosphamide at a dose between or between about 100 mg/m² and 500 mg/m² body surface area of the subject, such as between or between about 200 mg/m² and 400 mg/m², or 250 mg/m² and 350 mg/m², inclusive. In some instances, the subject is administered about 100 mg/m² of cyclophosphamide. In some instances, the subject is administered about 150 mg/m² of cyclophosphamide. In some instances, the subject is administered about 200 mg/m² of cyclophosphamide. In some instances, the subject is administered about 250 mg/m² of cyclophosphamide. In some instances, the subject is administered about 300 mg/m² of cyclophosphamide. In some embodiments, the cyclophosphamide can be administered in a single dose or can be administered in a plurality of doses, such as given daily, every other day or every three days. In some embodiments, cyclophosphamide is administered daily, such as for 1-5 days, for example, for 3 to 5 days. In some instances, the subject is administered about 300 mg/m² body surface area of the subject, of cyclophosphamide, daily for 3 days, prior to initiation of the cell therapy. In some embodiments, the subject is administered a total of at or about 300 mg/m², 400 mg/m², 500 mg/m², 600 mg/m², 700 mg/m², 800 mg/m², 900 mg/m², 1000 mg/m², 1200 mg/m², 1500 mg/m², 1800 mg/m², 2000 mg/m², 2500 mg/m², 2700 mg/m², 3000 mg/m², 3300 mg/m², 3600 mg/m², 4000 mg/m² or 5000 mg/m² cyclophosphamide, or a range defined by any of the foregoing, prior to initiation of the cell therapy.

In some embodiments, where the lymphodepleting agent comprises fludarabine, the subject is administered fludarabine at a dose between at or about 1 mg/m² and at or 100 mg/m², such as between at or about 10 mg/m² and at or about 75 mg/m², at or about 15 mg/m² and at or about 50 mg/m², at or about 20 mg/m² and at or about 40 mg/m², at or about or 24 mg/m² and at or about 35 mg/m², inclusive. In some instances, the subject is administered at or at or about 10 mg/m² of fludarabine. In some instances, the subject is administered at or about 15 mg/m² of fludarabine. In some instances, the subject is administered at or about 20 mg/m² of fludarabine. In some instances, the subject is administered at or about 25 mg/m² of fludarabine. In some instances, the subject is administered at or about 30 mg/m² of fludarabine. In some embodiments, the fludarabine can be administered in a single dose or can be administered in a plurality of doses, such as given daily, every other day or every three days. In some embodiments, fludarabine is administered daily, such as for 1-5 days, for example, for 3 to 5 days. In some instances, the subject is administered at or about 30 mg/m² body surface area of the subject, of fludarabine, daily for 3 days, prior to initiation of the cell therapy. In some embodiments, the subject is administered a total of at or about 10 mg/m², 20 mg/m², 25 mg/m², 30 mg/m², 40 mg/m², 50 mg/m², 60 mg/m², 70 mg/m², 80 mg/m², 90 mg/m², 100 mg/m², 120 mg/m², 150 mg/m², 180 mg/m², 200 mg/m², 250 mg/m², 270 mg/m², 300 mg/m², 330 mg/m², 360 mg/m², 400 mg/m² or 500 mg/m² cyclophosphamide, or a range defined by any of the foregoing, prior to initiation of the cell therapy.

In some embodiments, the lymphodepleting agent comprises a single agent, such as cyclophosphamide or fludarabine. In some embodiments, the subject is administered cyclophosphamide only, without fludarabine or other lymphodepleting agents. In some embodiments, prior to the administration, the subject has received a lymphodepleting therapy comprising the administration of cyclophosphamide at or about 200-400 mg/m² body surface area of the subject, optionally at or about 300 mg/m², daily, for 2-4 days. In some embodiments, the subject is administered fludarabine only, for example, without cyclophosphamide or other lymphodepleting agents. In some embodiments, prior to the administration, the subject has received a lymphodepleting therapy comprising the administration of fludarabine at or about 20-40 mg/m² body surface area of the subject, optionally at or about 30 mg/m², daily, for 2-4 days.

In some embodiments, the lymphodepleting agent comprises a combination of agents, such as a combination of cyclophosphamide and fludarabine. Thus, the combination of agents may include cyclophosphamide at any dose or administration schedule, such as those described above, and fludarabine at any dose or administration schedule, such as those described above. For example, in some aspects, the subject is administered at or about 60 mg/kg (~2 g/m²) of cyclophosphamide and 3 to 5 doses of 25 mg/m² fludarabine prior to the first or subsequent dose. In some the subject is administered fludarabine (30 mg/m²/day for 3 days) and cyclophosphamide (300 mg/m²/day for 3 days) (flu/cy) concurrently, intravenously, prior to administration of the cells. In some embodiments, the subject is administered a reduced, delayued or eliminated dose of one or more doses of the lymphodepleting agent(s).

In some embodiments, after collecting the cells from a subject (e.g. by leukapheresis) for engineering the cells of the cell therapy with a recombinant receptor (e.g. CAR) as described in Section III.E and prior to the lymphodepleting therapy, the subject can receive a bridging therapy. In some embodiments, the bridging therapy is a chemotherapy. The bridging therapy can be any anticancer therapy for control of the disease prior to receiving the dose of engineered (e.g. CAR+) T cells. Any of a variety of therapies can be administered as a bridging therapy based on the judgment of a skilled practitioner for treating the particular disease or condition, including based on factors such as the age of the patient, severity or extent of the disease, potential for side effects, timing of the administration prior to the lymphodepleting therapy, previous therapies and other factors. A bridging therapy can include radiotherapy or a systemic therapy. Exemplary therapies that can be given as a bridge prior to the lymphodepleting therapy include, but are not limited to, rituximab, dexamethasone, prednisone, lenalidomide, gemcitabine, oxaliplatin, Brentuximab vedotin, ibrutininb, or bendamustine, or any combination of any of the foregoing. In some case, the bridging therapy is gemcitabine and oxaliplatin. In some cases, the bridging therapy is gemcitabine and rituximab. In some embodiments, the bridging therapy is rituximab and gemcitabine and oxaliplatin. Prior to receiving the lympodepleting therapy the subject is assessed for disease status, such as by positron emission tomography (PET). In some embodiments, only subjects that exhibit PET-positive disease after bridging therapy are given the lymphodepleting therapy and administered the dose of engineered (e.g. CAR+) T cells. In other embodiments, if the subject achieves a CR after bridging therapy, the subject is not given the lymphodepleting therapy or the dose of engineered (e.g. CAR+) T cells.

Following administration of the cells, the biological activity of the engineered cell populations in some embodiments is measured, e.g., by any of a number of known methods. Parameters to assess include specific binding of an engineered or natural T cell or other immune cell to antigen, in vivo, e.g., by imaging, or ex vivo, e.g., by ELISA or flow cytometry. In certain embodiments, the ability of the engineered cells to destroy target cells can be measured using any suitable known methods, such as cytotoxicity assays described in, for example, Kochenderfer et al., J. Immunotherapy, 32(7): 689-702 (2009), and Herman et al. J. Immunological Methods, 285(1): 25-40 (2004). In certain embodiments, the biological activity of the cells is measured by assaying expression and/or secretion of one or more cytokines, such as CD107a, IFNγ, IL-2, and TNF. In some aspects the biological activity is measured by assessing clinical outcome, such as reduction in tumor burden or load.

In certain embodiments, the engineered cells are further modified in any number of ways, such that their therapeutic or prophylactic efficacy is increased. For example, the engineered CAR or TCR expressed by the population can be conjugated either directly or indirectly through a linker to a targeting moiety. The practice of conjugating compounds, e.g., the CAR or TCR, to targeting moieties is known. See, for instance, Wadwa et al., J. Drug Targeting 3: 1 1 1 (1995), and U.S. Patent 5,087,616. In some embodiments, the cells are administered as part of a combination treatment, such as simultaneously with or sequentially with, in any order, another therapeutic intervention, such as an antibody or engineered cell or receptor or agent, such as a cytotoxic or therapeutic agent. The cells in some embodiments are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some embodiments, the cells are administered prior to the one or more additional therapeutic agents. In some embodiments, the cells are administered after the one or more additional therapeutic agents. In some embodiments, the one or more additional agent includes a cytokine, such as IL-2, for example, to enhance persistence.

In some embodiments, the subjects are premedicated, e.g., to minimize the risk of infusion reaction. In some aspects, the premedication includes administering pain reliever and/or an antihistamine. In some embodiments, the premedication includes administering an acetaminophen and/or a diphenhydramine, or another H1-antihistamine. In some embodiments, the patient with acetaminophen (e.g., 650 mg orally) and diphenhydramine (e.g., 25-50 mg, IV or orally), or another H1-antihistamine, at or about 30 to 60 minutes prior to treatment with the cell therapy.

### B. Dosing

In some embodiments, a dose of cells is administered to subjects in accord with the provided methods, and/or with the provided articles of manufacture or compositions. In some embodiments, the size or timing of the doses is determined as a function of the particular disease or condition in the subject. In some cases, the size or timing of the doses for a particular disease in view of the provided description may be empirically determined.

In some of any of the provided embodiments, the dose of T cells, such as engineered T cells expressing a recombinant receptor, includes is enriched for, or comprises a cell composition or a cell population that is enriched for, CD3+ T cells, CD4+ T cells, CD8+ T cells or CD4+ T cells and CD8+ T cells. In some of any such embodiments, greater than at or about 70%, 75%, 80%, 85%, 90%, 95% or 98% of the cells in the dose of T cells are CD3+ T cells, CD4+ T cells, CD8+ T cells or CD4+ T cells and CD8+ T cells. In some of any embodiments, the dose of T cells comprises a defined ratio of CD4⁺ cells expressing the receptor to CD8⁺ cells expressing the receptor and/or of CD4⁺ T cells to CD8⁺ T cells, which ratio is approximately 1:1 or is between approximately 1:3 and approximately 3:1. In some of any embodiments, the defined ratio is or is approximately 1:1.

In some of any provided embodiments, the dose of T cells comprises a dose of CD4⁺ and CD8⁺ T cells, wherein T cells of each dose comprises a recombinant receptor that specifically binds to a target antigen expressed by the disease or disorder, such as any described herein, or a cell or tissue thereof and/or that is associated with the disease or disorder. In some aspects, the administration comprises administering a plurality of separate compositions, the plurality of separate compositions comprising a first composition comprising CD8⁺ T cells and a second composition comprising CD4⁺ T cells.

In some embodiments, the dose of cells comprises between at or about 2 x 10⁵ of the cells/kg and at or about 2 x 10⁶ of the cells/kg, such as between at or about 4 x 10⁵ of the cells/kg and at or about 1 x 10⁶ of the cells/kg or between at or about 6 x 10⁵ of the cells/kg and at or about 8 x 10⁵ of the cells/kg. In some embodiments, the dose of cells comprises no more than 2 x 10⁵ of the cells (e.g. antigen-expressing, such as CAR-expressing cells) per kilogram body weight of the subject (cells/kg), such as no more than at or about 3 x 10⁵ cells/kg, no more than at or about 4 x 10⁵ cells/kg, no more than at or about 5 x 10⁵ cells/kg, no more than at or about 6 x 10⁵ cells/kg, no more than at or about 7 x 10⁵ cells/kg, no more than at or about 8 x 10⁵ cells/kg, no more than at or about 9 x 10⁵ cells/kg, no more than at or about 1 x 10⁶ cells/kg, or no more than at or about 2 x 10⁶ cells/kg. In some embodiments, the dose of cells comprises at least or at least about or at or about 2 x 10⁵ of the cells (e.g. antigen-expressing, such as CAR-expressing cells) per kilogram body weight of the subject (cells/kg), such as at least or at least about or at or about 3 x 10⁵ cells/kg, at least or at least about or at or about 4 x 10⁵ cells/kg, at least or at least about or at or about 5 x 10⁵ cells/kg, at least or at least about or at or about 6 x 10⁵ cells/kg, at least or at least about or at or about 7 x 10⁵ cells/kg, at least or at least about or at or about 8 x 10⁵ cells/kg, at least or at least about or at or about 9 x 10⁵ cells/kg, at least or at least about or at or about 1 x 10⁶ cells/kg, or at least or at least about or at or about 2 x 10⁶ cells/kg. In some embodiments, the number of cells is the number of such cells that are viable cells, e.g., viable T cells.

In certain embodiments, the cells, or individual populations of sub-types of cells, are administered to the subject at a range of at or about 0.1 million to at or about 100 billion cells and/or that amount of cells per kilogram of body weight of the subject, such as, *e.g.,* at or about 0.1 million to at or about 50 billion cells (*e.g.,* at or about 5 million cells, at or about 25 million cells, at or about 500 million cells, at or about 1 billion cells, at or about 5 billion cells, at or about 20 billion cells, at or about 30 billion cells, at or about 40 billion cells, or a range defined by any two of the foregoing values), at or about 1 million to at or about 50 billion cells (*e.g.,* at or about 5 million cells, at or about 25 million cells, at or about 500 million cells, at or about 1 billion cells, at or about 5 billion cells, at or about 20 billion cells, at or about 30 billion cells, at or about 40 billion cells, or a range defined by any two of the foregoing values), such as at or about 10 million to at or about 100 billion cells (*e.g.,* at or about 20 million cells, at or about 30 million cells, at or about 40 million cells, at or about 60 million cells, at or about 70 million cells, at or about 80 million cells, at or about 90 million cells, at or about 10 billion cells, at or about 25 billion cells, at or about 50 billion cells, at or about 75 billion cells, at or about 90 billion cells, or a range defined by any two of the foregoing values), and in some cases at or about 100 million cells to at or about 50 billion cells (*e.g.,* at or about 120 million cells, at or about 250 million cells, at or about 350 million cells, at or about 650 million cells, at or about 800 million cells, at or about 900 million cells, at or about 3 billion cells, at or about 30 billion cells, at or about 45 billion cells) or any value in between these ranges and/or per kilogram of body weight of the subject. Dosages may vary depending on attributes particular to the disease or disorder and/or patient and/or other treatments. In some embodiments, such values refer to numbers of recombinant receptor-expressing cells; in other embodiments, they refer to number of T cells or PBMCs or total cells administered. In some embodiments, the number of cells is the number of such cells that are viable cells.

In some embodiments, the dose of cells is a flat dose of cells or fixed dose of cells such that the dose of cells is not tied to or based on the body surface area or weight of a subject.

In some embodiments, the dose of genetically engineered cells comprises from at or about 1 x 10⁵ to at or about 5 x 10⁸ total CAR-expressing T cells, from at or about 1 x 10⁵ to at or about 2.5 x 10⁸ total CAR-expressing T cells, from at or about 1 x 10⁵ to at or about 1 x 10⁸ total CAR-expressing T cells, from at or about 1 x 10⁵ to at or about 5 x 10⁷ total CAR-expressing T cells, from at or about 1 x 10⁵ to at or about 2.5 x 10⁷ total CAR-expressing T cells, from at or about 1 x 10⁵ to at or about 1 x 10⁷ total CAR-expressing T cells, from at or about 1 x 10⁵ to at or about 5 x 10⁶ total CAR-expressing T cells, from at or about 1 x 10⁵ to at or about 2.5 x 10⁶ total CAR-expressing T cells, from at or about 1 x 10⁵ to at or about 1 x 10⁶ total CAR-expressing T cells, from at or about 1 x 10⁶ to at or about 5 x 10⁸ total CAR-expressing T cells, from at or about 1 x 10⁶ to at or about 2.5 x 10⁸ total CAR-expressing T cells, from at or about 1 x 10⁶ to at or about 1 x 10⁸ total CAR-expressing T cells, from at or about 1 x 10⁶ to at or about 5 x 10⁷ total CAR-expressing T cells, from at or about 1 x 10⁶ to at or about 2.5 x 10⁷ total CAR-expressing T cells, from at or about 1 x 10⁶ to at or about 1 x 10⁷ total CAR-expressing T cells, from at or about 1 x 10⁶ to at or about 5 x 10⁶ total CAR-expressing T cells, from at or about 1 x 10⁶ to at or about 2.5 x 10⁶ total CAR-expressing T cells, from at or about 2.5 x 10⁶ to at or about 5 x 10⁸ total CAR-expressing T cells, from at or about 2.5 x 10⁶ to at or about 2.5 x 10⁸ total CAR-expressing T cells, from at or about 2.5 x 10⁶ to at or about 1 x 10⁸ total CAR-expressing T cells, from at or about 2.5 x 10⁶ to at or about 5 x 10⁷ total CAR-expressing T cells, from at or about 2.5 x 10⁶ to at or about 2.5 x 10⁷ total CAR-expressing T cells, from at or about 2.5 x 10⁶ to at or about 1 x 10⁷ total CAR-expressing T cells, from at or about 2.5 x 10⁶ to at or about 5 x 10⁶ total CAR-expressing T cells, from at or about 5 x 10⁶ to at or about 5 x 10⁸ total CAR-expressing T cells, from at or about 5 x 10⁶ to at or about 2.5 x 10⁸ total CAR-expressing T cells, from at or about 5 x 10⁶ to at or about 1 x 10⁸ total CAR-expressing T cells, from at or about 5 x 10⁶ to at or about 5 x 10⁷ total CAR-expressing T cells, from at or about 5 x 10⁶ to at or about 2.5 x 10⁷ total CAR-expressing T cells, from at or about 5 x 10⁶ to at or about 1 x 10⁷ total CAR-expressing T cells, from at or about 1 x 10⁷ to at or about 5 x 10⁸ total CAR-expressing T cells, from at or about 1 x 10⁷ to at or about 2.5 x 10⁸ total CAR-expressing T cells, from at or about 1 x 10⁷ to at or about 1 x 10⁸ total CAR-expressing T cells, from at or about 1 x 10⁷ to at or about 5 x 10⁷ total CAR-expressing T cells, from at or about 1 x 10⁷ to at or about 2.5 x 10⁷ total CAR-expressing T cells, from at or about 2.5 x 10⁷ to at or about 5 x 10⁸ total CAR-expressing T cells, from at or about 2.5 x 10⁷ to at or about 2.5 x 10⁸ total CAR-expressing T cells, from at or about 2.5 x 10⁷ to at or about 1 x 10⁸ total CAR-expressing T cells, from at or about 2.5 x 10⁷ to at or about 5 x 10⁷ total CAR-expressing T cells, from at or about 5 x 10⁷ to at or about 5 x 10⁸ total CAR-expressing T cells, from at or about 5 x 10⁷ to at or about 2.5 x 10⁸ total CAR-expressing T cells, from at or about 5 x 10⁷ to at or about 1 x 10⁸ total CAR-expressing T cells, from at or about 1 x 10⁸ to at or about 5 x 10⁸ total CAR-expressing T cells, from at or about 1 x 10⁸ to at or about 2.5 x 10⁸ total CAR-expressing T cells, from at or about or 2.5 x 10⁸ to at or about 5 x 10⁸ total CAR-expressing T cells. In some embodiments, the dose of genetically engineered cells comprises from or from about 2.5 x 10⁷ to at or about 1.5 x 10⁸ total CAR-expressing T cells, such as from or from about 5 x 10⁷ to or to about 1 x 10⁸ total CAR-expressing T cells. In some embodiments, the number of cells is the number of such cells that are viable cells, such as viable T cells.

In some embodiments, the dose of genetically engineered cells comprises at least or at least about 1 x 10⁵ CAR-expressing cells, at least or at least about 2.5 x 10⁵ CAR-expressing cells, at least or at least about 5 x 10⁵ CAR-expressing cells, at least or at least about 1 x 10⁶ CAR-expressing cells, at least or at least about 2.5 x 10⁶ CAR-expressing cells, at least or at least about 5 x 10⁶ CAR-expressing cells, at least or at least about 1 x 10⁷ CAR-expressing cells, at least or at least about 2.5 x 10⁷ CAR-expressing cells, at least or at least about 5 x 10⁷ CAR-expressing cells, at least or at least about 1 x 10⁸ CAR-expressing cells, at least or at least about 1.5 x 10⁸ CAR-expressing cells, at least or at least about 2.5 x 10⁸ CAR-expressing cells, or at least or at least about 5 x 10⁸ CAR-expressing cells. In some embodiments, the number of cells is the number of such cells that are viable cells, such as viable T cells.

In some embodiments, the cell therapy comprises administration of a dose comprising a number of cell from or from about 1 x 10⁵ to or to about 5 x 10⁸ total recombinant receptor-expressing cells, total T cells, or total peripheral blood mononuclear cells (PBMCs), from or from about 5 x 10⁵ to or to about 1 x 10⁷ total recombinant receptor-expressing cells, total T cells, or total peripheral blood mononuclear cells (PBMCs) or from or from about 1 x 10⁶ to or to about 1 x 10⁷ total recombinant receptor-expressing cells, total T cells, or total peripheral blood mononuclear cells (PBMCs), each inclusive. In some embodiments, the cell therapy comprises administration of a dose of cells comprising a number of cells at least or at least about 1 x 10⁵ total recombinant receptor-expressing cells, total T cells, or total peripheral blood mononuclear cells (PBMCs), such at least or at least 1 x 10⁶, at least or at least about 1 x 10⁷, at least or at least about 1 x 10⁸ of such cells. In some embodiments, the number of cells is the number of such cells that are viable cells, such as viable T cells.

In some embodiments, the number is with reference to the total number of CD3⁺, CD8⁺, or CD4+ and CD8+, in some cases also recombinant receptor-expressing (e.g. CAR⁺) cells. In some embodiments, the number of cells is the number of such cells that are viable cells.

In some embodiments, the cell therapy comprises administration of a dose comprising a number of cell from or from about 1 x 10⁵ to or to about 5 x 10⁸ CD3⁺, CD8⁺ or CD4⁺ and CD8⁺ total T cells or CD3⁺, CD8⁺ or CD4⁺ and CD8⁺ recombinant receptor (e.g. CAR)-expressing cells, from or from about 5 x 10⁵ to or to about 1 x 10⁷ CD3⁺, CD8⁺ or CD4⁺ and CD8⁺ total T cells or CD3⁺, CD8⁺ or CD4⁺ and CD8⁺ recombinant receptor (e.g. CAR)-expressing cells, or from or from about 1 x 10⁶ to or to about 1 x 10⁷ CD3⁺, CD8⁺ or CD4⁺ and CD8⁺ total T cells or CD3⁺, CD8⁺ or CD4⁺ and CD8⁺ recombinant receptor (e.g. CAR)-expressing cells, each inclusive. In some embodiments, the cell therapy comprises administration of a dose comprising a number of cell from or from about 1 x 10⁵ to or to about 5 x 10⁸ total CD3⁺/CAR⁺, CD8⁺/CAR⁺ or CD4⁺/CD8⁺/CAR⁺ cells, from or from about 5 x 10⁵ to or to about 1 x 10⁷ total CD3⁺/CAR⁺, CD8⁺/CAR⁺ or CD4⁺/CD8⁺/CAR⁺ cells, or from or from about 1 x 10⁶ to or to about 1 x 10⁷ total CD3⁺/CAR⁺, CD8⁺/CAR⁺ or CD4⁺/CD8⁺/CAR⁺ cells, each inclusive. In some embodiments, the number of cells is the number of such cells that are viable cells.

In some embodiments, the dose of genetically engineered cells comprises at least or at least about 2.5 x 10⁷ CD3⁺/CAR⁺, CD8⁺/CAR⁺, or CD4⁺/CD8⁺/CAR⁺ T cells, at least or at least about 5 x 10⁷ CD3⁺/CAR⁺, CD8⁺/CAR⁺, or CD4⁺/CD8⁺/CAR⁺ T cells, or at least or at least about 1 x 10⁸ CD3⁺/CAR⁺, CD8⁺/CAR⁺, or CD4⁺/CD8⁺/CAR⁺ T cells. In some embodiments, the dose of genetically engineered cells comprises at or about 2.5 x 10⁷ CD3⁺/CAR⁺, CD8⁺/CAR⁺, or CD4⁺/CD8⁺/CAR⁺ T cells, at or about 5 x 10⁷ CD3⁺/CAR⁺, CD8⁺/CAR⁺, or CD4⁺/CD8⁺/CAR⁺ T cells, or at or about 1 x 10⁸ CD3⁺/CAR⁺, CD8⁺/CAR⁺, or CD4⁺/CD8⁺/CAR⁺ T cells. In some embodiments, the number of cells is the number of such cells that are viable cells.

In some embodiments, the dose of T cells comprises: at or about 5 x 10⁷ recombinant receptor (e.g. CAR)-expressing T cells or at or about 2.5 x 10⁷ recombinant receptor (e.g. CAR)-expressing CD8⁺ T cells. In some embodiments, the dose of T cells comprises: at or about 1 x 10⁸ recombinant receptor (e.g. CAR)-expressing T cells or at or about 5 x 10⁷ recombinant receptor (e.g. CAR)-expressing CD8⁺ T cells. In some embodiments, the dose of T cells comprises: at or about 1.5 x 10⁸ recombinant receptor (e.g. CAR)-expressing T cells or at or about 0.75 x 10⁸ recombinant receptor (e.g. CAR) -expressing CD8⁺ T cells. In some embodiments, the number of cells is the number of such cells that are viable cells.

In some embodiments, the T cells of the dose include CD4⁺ T cells, CD8⁺ T cells or CD4⁺ and CD8⁺ T cells.

In some embodiments, for example, where the subject is human, the CD8+ T cells of the dose, including in a dose including CD4+ and CD8+ T cells, includes between at or about 1 x 10⁶ and at or about 5 x 10⁸ total recombinant receptor (e.g., CAR)-expressing CD8+cells, e.g., in the range of from at or about 5 x 10⁶ to at or about 1 x 10⁸ such cells, such as 1 x 10⁷, 2.5 x 10⁷, 5 x 10⁷, 7.5 x 10⁷, 1 x 10⁸, 1.5 x 10⁸, or 5 x 10⁸ total such cells, or the range between any two of the foregoing values. In some embodiments, the patient is administered multiple doses, and each of the doses or the total dose can be within any of the foregoing values. In some embodiments, the dose of cells comprises the administration of from or from about 1 x 10⁷ to or to about0.75 x 10⁸ total recombinant receptor-expressing CD8+ T cells, from or from about 1 x 10⁷ to or to about 5 x 10⁷ total recombinant receptor-expressing CD8+ T cells, from or from about 1 x 10⁷ to or to about 0.25 x 10⁸ total recombinant receptor-expressing CD8+ T cells, each inclusive. In some embodiments, the dose of cells comprises the administration of at or about 1 x 10⁷, 2.5 x 10⁷, 5 x 10⁷, 7.5 x 10⁷, 1 x 10⁸, 1.5 x 10⁸, 2.5 x 10⁸, or 5 x 10⁸ total recombinant receptor-expressing CD8+ T cells. In some embodiments, the number of cells is the number of such cells that are viable cells.

In some embodiments, for example, where the subject is a human, the dose includes fewer than about 5 x 10⁸ total recombinant receptor (e.g., CAR)-expressing cells, T cells, or peripheral blood mononuclear cells (PBMCs), e.g., in the range of at or about 1 x 10⁶ to at or about 5 x 10⁸ such cells, such as at or about 2 x 10⁶, 5 x 10⁶, 1 x 10⁷, 5 x 10⁷, 1 x 10⁸, 1.5 x 10⁸, or 5 x 10⁸ total such cells, or the range between any two of the foregoing values. In some embodiments, the number of cells is the number of such cells that are viable cells.

In some embodiments, the patient is administered multiple doses, and each of the doses or the total dose can be within any of the foregoing values. In some embodiments, the dose of cells comprises the administration of from or from about 1 x 10⁵ to or to about 5 x 10⁸ total recombinant receptor (e.g. CAR)-expressing T cells or total T cells, from or from about 1 x 10⁵ to or to about 1.5 x 10⁸ total recombinant receptor (e.g. CAR)-expressing T cells or total T cells, from or from about 1 x 10⁵ to or to about 1 x 10⁸ total recombinant receptor (e.g. CAR)-expressing T cells or total T cells, from or from about 5 x 10⁵ to or to about 1 x 10⁷ total recombinant receptor (e.g. CAR)-expressing T cells or total T cells, or from or from about 1 x 10⁶ to or to about 1 x 10⁷ total recombinant receptor (e.g. CAR)-expressing T cells or total T cells, each inclusive.

In some embodiments, the T cells of the dose include CD4⁺ T cells, CD8⁺ T cells or CD4⁺ and CD8⁺ T cells.

In some embodiments, the dose of cells, e.g., recombinant receptor-expressing T cells, is administered to the subject as a single dose or is administered only one time within a period of two weeks, one month, 3 months, six months, 1 year or more.

In the context of adoptive cell therapy, administration of a given "dose" encompasses administration of the given amount or number of cells as a single composition and/or single uninterrupted administration, e.g., as a single injection or continuous infusion, and also encompasses administration of the given amount or number of cells as a split dose or as a plurality of compositions, provided in multiple individual compositions or infusions, over a specified period of time, such as over no more than 3 days. Thus, in some contexts, the dose is a single or continuous administration of the specified number of cells, given or initiated at a single point in time. In some contexts, however, the dose is administered in multiple injections or infusions over a period of no more than three days, such as once a day for three days or for two days or by multiple infusions over a single day period.

Thus, in some aspects, the cells of the dose are administered in a single pharmaceutical composition. In some embodiments, the cells of the dose are administered in a plurality of compositions, collectively containing the cells of the dose.

In some embodiments, the term "split dose" refers to a dose that is split so that it is administered over more than one day. This type of dosing is encompassed by the present methods and is considered to be a single dose.

Thus, the dose of cells may be administered as a split dose, e.g., a split dose administered over time. For example, in some embodiments, the dose may be administered to the subject over 2 days or over 3 days. Exemplary methods for split dosing include administering 25% of the dose on the first day and administering the remaining 75% of the dose on the second day. In other embodiments, 33% of the dose may be administered on the first day and the remaining 67% administered on the second day. In some aspects, 10% of the dose is administered on the first day, 30% of the dose is administered on the second day, and 60% of the dose is administered on the third day. In some embodiments, the split dose is not spread over more than 3 days.

In some embodiments, cells of the dose may be administered by administration of a plurality of compositions or solutions, such as a first and a second, optionally more, each containing some cells of the dose. In some aspects, the plurality of compositions, each containing a different population and/or sub-types of cells, are administered separately or independently, optionally within a certain period of time. For example, the populations or sub-types of cells can include CD8⁺ and CD4⁺ T cells, respectively, and/or CD8⁺- and CD4⁺-enriched populations, respectively, e.g., CD4⁺ and/or CD8⁺ T cells each individually including cells genetically engineered to express the recombinant receptor. In some embodiments, the administration of the dose comprises administration of a first composition comprising a dose of CD8⁺ T cells or a dose of CD4⁺ T cells and administration of a second composition comprising the other of the dose of CD4⁺ T cells and the CD8⁺ T cells.

In some embodiments, the administration of the composition or dose, e.g., administration of the plurality of cell compositions, involves administration of the cell compositions separately. In some aspects, the separate administrations are carried out simultaneously, or sequentially, in any order. In particular embodiments, the separate administrations are carried out sequentially by administering, in any order, a first composition comprising a dose of CD8⁺ T cells or a dose of CD4⁺ T cells and a second composition comprising the other of the dose of CD4⁺ T cells and the CD8⁺ T cells. In some embodiments, the dose comprises a first composition and a second composition, and the first composition and second composition are administered within 48 hours of each other, such as no more than 36 hours of each other or not more than 24 hours of each other. In some embodiments, the first composition and second composition are administered 0 to 12 hours apart, 0 to 6 hours apart or 0 to 2 hours apart. In some embodiments, the initiation of administration of the first composition and the initiation of administration of the second composition are carried out no more than 2 hours, no more than 1 hour, or no more than 30 minutes apart, no more than 15 minutes, no more than 10 minutes or no more than 5 minutes apart. In some embodiments, the initiation and/or completion of administration of the first composition and the completion and/or initiation of administration of the second composition are carried out no more than 2 hours, no more than 1 hour, or no more than 30 minutes apart, no more than 15 minutes, no more than 10 minutes or no more than 5 minutes apart.

In some composition, the first composition, e.g., first composition of the dose, comprises CD4⁺ T cells. In some composition, the first composition, e.g., first composition of the dose, comprises CD8⁺ T cells. In some embodiments, the first composition is administered prior to the second composition. In particular embodiments, the CD8+ T cells are administered prior to the CD4+ T cells.

In some embodiments, the dose or composition of cells includes a defined or target ratio of CD4⁺ cells expressing a recombinant receptor (e.g. CAR) to CD8⁺ cells expressing a recombinant receptor (e.g. CAR) and/or of CD4⁺ cells to CD8⁺ cells, which ratio optionally is approximately 1:1 or is between approximately 1:3 and approximately 3:1, such as approximately 1:1. In some aspects, the administration of a composition or dose with the target or desired ratio of different cell populations (such as CD4⁺:CD8⁺ ratio or CAR⁺CD4⁺:CAR⁺CD8⁺ ratio, e.g., 1:1) involves the administration of a cell composition containing one of the populations and then administration of a separate cell composition comprising the other of the populations, where the administration is at or approximately at the target or desired ratio. In some aspects, administration of a dose or composition of cells at a defined ratio leads to improved expansion, persistence and/or antitumor activity of the T cell therapy.

In some embodiments, the dose of genetically engineered cells is or is about 5 x 10⁷ CD3+ CAR+ viable cells, that includes a separate dose of at or about 2.5 x 10⁷ CD4+ CAR+ viable cells and at or about 2.5 x 10⁷ CD8+CAR+ viable cells. In some embodiments, the dose of genetically engineered cells is or is about 1 x 10⁸ CD3+CAR+ viable cells, that includes a separate dose of at or about 5 x 10⁷ CD4+CAR+ viable cells and at or about 5 x10⁷ CD8+CAR+ viable cells. In some embodiments, the dose of genetically engineered cells is or is about 1.5 x 10⁸ CD3+CAR+ viable cells, that includes a separate dose of at or about 0.75 x 10⁸ CD4+CAR+ viable cells and at or about 0.75 x10⁸ CD8+CAR+ viable cells.

In some embodiments, the subject receives multiple doses, e.g., two or more doses or multiple consecutive doses, of the cells. In some embodiments, two doses are administered to a subject. In some embodiments, the subject receives the consecutive dose, e.g., second dose, is administered approximately 4, 5, 6, 7, 8, 9, 10, 11, 12, 13, 14, 15, 16, 17, 18, 19, 20 or 21 days after the first dose. In some embodiments, multiple consecutive doses are administered following the first dose, such that an additional dose or doses are administered following administration of the consecutive dose. In some aspects, the number of cells administered to the subject in the additional dose is the same as or similar to the first dose and/or consecutive dose. In some embodiments, the additional dose or doses are larger than prior doses.

In some aspects, the size of the first and/or consecutive dose is determined based on one or more criteria such as response of the subject to prior treatment, e.g. chemotherapy, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

In some aspects, the time between the administration of the first dose and the administration of the consecutive dose is about 9 to about 35 days, about 14 to about 28 days, or 15 to 27 days. In some embodiments, the administration of the consecutive dose is at a time point more than about 14 days after and less than about 28 days after the administration of the first dose. In some aspects, the time between the first and consecutive dose is about 21 days. In some embodiments, an additional dose or doses, e.g. consecutive doses, are administered following administration of the consecutive dose. In some aspects, the additional consecutive dose or doses are administered at least about 14 and less than about 28 days following administration of a prior dose. In some embodiments, the additional dose is administered less than about 14 days following the prior dose, for example, 4, 5, 6, 7, 8, 9, 10, 11, 12, or 13 days after the prior dose. In some embodiments, no dose is administered less than about 14 days following the prior dose and/or no dose is administered more than about 28 days after the prior dose.

In some embodiments, the dose of cells, e.g., recombinant receptor-expressing cells, comprises two doses (e.g., a double dose), comprising a first dose of the T cells and a consecutive dose of the T cells, wherein one or both of the first dose and the second dose comprises administration of the split dose of T cells.

In some embodiments, the dose of cells is generally large enough to be effective in reducing disease burden.

In some embodiments, the cells are administered at a desired dosage, which in some aspects includes a desired dose or number of cells or cell type(s) and/or a desired ratio of cell types. Thus, the dosage of cells in some embodiments is based on a total number of cells (or number per kg body weight) and a desired ratio of the individual populations or sub-types, such as the CD4⁺ to CD8⁺ ratio. In some embodiments, the dosage of cells is based on a desired total number (or number per kg of body weight) of cells in the individual populations or of individual cell types. In some embodiments, the dosage is based on a combination of such features, such as a desired number of total cells, desired ratio, and desired total number of cells in the individual populations.

In some embodiments, the populations or sub-types of cells, such as CD8⁺ and CD4⁺ T cells, are administered at or within a tolerated difference of a desired dose of total cells, such as a desired dose of T cells. In some aspects, the desired dose is a desired number of cells or a desired number of cells per unit of body weight of the subject to whom the cells are administered, e.g., cells/kg. In some aspects, the desired dose is at or above a minimum number of cells or minimum number of cells per unit of body weight. In some aspects, among the total cells, administered at the desired dose, the individual populations or sub-types are present at or near a desired output ratio (such as CD4⁺ to CD8⁺ ratio), e.g., within a certain tolerated difference or error of such a ratio.

In some embodiments, the cells are administered at or within a tolerated difference of a desired dose of one or more of the individual populations or sub-types of cells, such as a desired dose of CD4⁺ cells and/or a desired dose of CD8⁺ cells. In some aspects, the desired dose is a desired number of cells of the sub-type or population, or a desired number of such cells per unit of body weight of the subject to whom the cells are administered, e.g., cells/kg. In some aspects, the desired dose is at or above a minimum number of cells of the population or sub-type, or minimum number of cells of the population or sub-type per unit of body weight.

Thus, in some embodiments, the dosage is based on a desired fixed dose of total cells and a desired ratio, and/or based on a desired fixed dose of one or more, e.g., each, of the individual sub-types or sub-populations. Thus, in some embodiments, the dosage is based on a desired fixed or minimum dose of T cells and a desired ratio of CD4⁺ to CD8⁺ cells, and/or is based on a desired fixed or minimum dose of CD4⁺ and/or CD8⁺ cells.

In some embodiments, the cells are administered at or within a tolerated range of a desired output ratio of multiple cell populations or sub-types, such as CD4⁺ and CD8⁺ cells or sub-types. In some aspects, the desired ratio can be a specific ratio or can be a range of ratios. for example, in some embodiments, the desired ratio (e.g., ratio of CD4⁺ to CD8⁺ cells) is between at or about 5:1 and at or about 5:1 (or greater than at or about 1:5 and less than at or about 5:1), or between at or about 1:3 and at or about 3:1 (or greater than at or about 1:3 and less than at or about 3:1), such as between at or about 2:1 and at or about 1:5 (or greater than at or about 1:5 and less than at or about 2:1), such as at or about 5:1, 4.5:1, 4:1, 3.5:1, 3:1, 2.5:1, 2:1, 1.9:1, 1.8:1, 1.7:1, 1.6:1, 1.5:1, 1.4:1, 1.3:1, 1.2:1, 1.1:1, 1:1, 1:1.1, 1:1.2, 1:1.3, 1:1.4, 1:1.5, 1:1.6, 1:1.7, 1:1.8, 1:1.9: 1:2, 1:2.5, 1:3, 1:3.5, 1:4, 1:4.5, or 1:5. In some aspects, the tolerated difference is within about 1%, about 2%, about 3%, about 4% about 5%, about 10%, about 15%, about 20%, about 25%, about 30%, about 35%, about 40%, about 45%, about 50% of the desired ratio, including any value in between these ranges.

In particular embodiments, the numbers and/or concentrations of cells refer to the number of recombinant receptor (e.g., CAR)-expressing cells. In other embodiments, the numbers and/or concentrations of cells refer to the number or concentration of all cells, T cells, or peripheral blood mononuclear cells (PBMCs) administered.

In some aspects, the size of the dose is determined based on one or more criteria such as response of the subject to prior treatment, e.g. chemotherapy, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

In some embodiments, the methods also include administering one or more additional doses of cells expressing a chimeric antigen receptor (CAR) and/or lymphodepleting therapy, and/or one or more steps of the methods are repeated. In some embodiments, the one or more additional dose is the same as the initial dose. In some embodiments, the one or more additional dose is different from the initial dose, e.g., higher, such as at or about 2-fold, 3-fold, 4-fold, 5-fold, 6-fold, 7-fold, 8-fold, 9-fold or 10-fold or more higher than the initial dose, or lower, such as e.g., higher, such as 2-fold, 3-fold, 4-fold, 5-fold, 6-fold, 7-fold, 8-fold, 9-fold or 10-fold or more lower than the initial dose. In some embodiments, administration of one or more additional doses is determined based on response of the subject to the initial treatment or any prior treatment, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

### C. Response, Efficacy and Survival

In some embodiments, the administration effectively treats the subject despite the subject having become resistant to another therapy. In some embodiments, at least 30%, at least 35%, at least 40% or at least 50% of subjects treated according to the method achieve complete remission (CR); and/or at least about 40%, at least about 50%, at least about 60% or at least about 70% of the subjects treated according to the method achieve an objective response (OR). In some embodiments, at least or at least about 50% of subjects, at least or at least about 60% of the subjects, at least or at least about 70% of the subjects, at least or at least about 80% of the subjects or at least or at least about 90% of the subjects treated according to the method achieve CR and/or achieve an objective response (OR). In some embodiments, criteria assessed for effective treatment includes overall response rate (ORR; also known in some cases as objective response rate), complete response (CR; also known in some cases as complete remission), duration of response (DOR), progression-free survival (PFS), and/or overall survival (OS).

In some embodiments, at least 40% or at least 50% of subjects treated according to the methods provided herein achieve complete remission (CR; also known in some cases as complete response), exhibit progression-free survival (PFS) and/or overall survival (OS) of greater than at or about 3 months, 6 months or 12 months or greater than 13 months or approximately 14 months; on average, subjects treated according to the method exhibit a median PFS or OS of greater than at or about 6 months, 12 months, or 18 months; and/or the subject exhibits PFS or OS following therapy for at least at or about 6, 12, 18 or more months or longer.

In some aspects, response rates in subjects, such as subjects with NHL, are based on the Lugano criteria. (Cheson et al., (2014) JCO 32(27):3059-3067; Johnson et al., (2015) Radiology 2:323-338; Cheson, B.D. (2015) Chin Clin Oncol 4(1):5). In some aspects, response assessment utilizes any of clinical, hematologic, and/or molecular methods. In some aspects, response assessed using the Lugano criteria involves the use of positron emission tomography (PET)-computed tomography (CT) and/or CT as appropriate. PET-CT evaluations may further comprise the use of fluorodeoxyglucose (FDG) for FDG-avid lymphomas. In some aspects, where PET-CT will be used to assess response in FDG-avid histologies, a 5-point scale may be used. In some respects, the 5-point scale comprises the following criteria: 1, no uptake above background; 2, uptake ≤ mediastinum; 3, uptake > mediastinum but ≤ liver; 4, uptake moderately > liver; 5, uptake markedly higher than liver and/or new lesions; X, new areas of uptake unlikely to be related to lymphoma.

In some aspects, a complete response as described using the Lugano criteria involves a complete metabolic response and a complete radiologic response at various measureable sites. In some aspects, these sites include lymph nodes and extralymphatic sites, wherein a CR is described as a score of 1, 2, or 3 with or without a residual mass on the 5-point scale, when PET-CT is used. In some aspects, in Waldeyer's ring or extranodal sites with high physiologic uptake or with activation within spleen or marrow (e.g., with chemotherapy or myeloid colony-stimulating factors), uptake may be greater than normal mediastinum and/or liver. In this circumstance, complete metabolic response may be inferred if uptake at sites of initial involvement is no greater than surrounding normal tissue even if the tissue has high physiologic uptake. In some aspects, response is assessed in the lymph nodes using CT, wherein a CR is described as no extralymphatic sites of disease and target nodes/nodal masses must regress to ≤ 1.5 cm in longest transverse diameter of a lesion (LDi). Further sites of assessment include the bone marrow wherein PET-CT-based assessment should indicate a lack of evidence of FDG-avid disease in marrow and a CT-based assessment should indicate a normal morphology, which if indeterminate should be IHC negative. Further sites may include assessment of organ enlargement, which should regress to normal. In some aspects, non-measured lesions and new lesions are assessed, which in the case of CR should be absent (Cheson et al., (2014) JCO 32(27):3059-3067; Johnson et al., (2015) Radiology 2:323-338; Cheson, B.D. (2015) Chin Clin Oncol 4(1):5).

In some aspects, a partial response (PR; also known in some cases as partial remission) as described using the Lugano criteria involves a partial metabolic and/or radiological response at various measureable sites. In some aspects, these sites include lymph nodes and extralymphatic sites, wherein a PR is described as a score of 4 or 5 with reduced uptake compared with baseline and residual mass(es) of any size, when PET-CT is used. At interim, such findings can indicate responding disease. At the end of treatment, such findings can indicate residual disease. In some aspects, response is assessed in the lymph nodes using CT, wherein a PR is described as ≥50% decrease in SPD of up to 6 target measureable nodes and extranodal sites. If a lesion is too small to measure on CT, 5 mm × 5 mm is assigned as the default value; if the lesion is no longer visible, the value is 0 mm × 0 mm; for a node >5 mm × 5 mm, but smaller than normal, actual measurements are used for calculation. Further sites of assessment include the bone marrow wherein PET-CT-based assessment should indicate residual uptake higher than uptake in normal marrow but reduced compared with baseline (diffuse uptake compatible with reactive changes from chemotherapy allowed). In some aspects, if there are persistent focal changes in the marrow in the context of a nodal response, consideration should be given to further evaluation with MRI or biopsy, or an interval scan. In some aspects, further sites may include assessment of organ enlargement, where the spleen must have regressed by >50% in length beyond normal. In some aspects, non-measured lesions and new lesions are assessed, which in the case of PR should be absent/normal, regressed, but no increase. No response/stable disease (SD) or progressive disease (PD) can also be measured using PET-CT and/or CT based assessments. (Cheson et al., (2014) JCO 32(27):3059-3067; Johnson et al., (2015) Radiology 2:323-338; Cheson, B.D. (2015) Chin Clin Oncol 4(1):5).

The study was conducted in accordance with the Declaration of Helsinki, International Conference on Harmonisation Good Clinical Practice guidelines, and applicable regulatory requirements.

In some respects, progression-free survival (PFS) is described as the length of time during and after the treatment of a disease, such as cancer, that a subject lives with the disease but it does not get worse. In some aspects, objective response (OR) is described as a measurable response. In some aspects, objective response rate (ORR; also known in some cases as overall response rate) is described as the proportion of patients who achieved CR or PR. In some aspects, overall survival (OS) is described as the length of time from either the date of diagnosis or the start of treatment for a disease, such as cancer, that subjects diagnosed with the disease are still alive. In some aspects, event-free survival (EFS) is described as the length of time after treatment for a cancer ends that the subject remains free of certain complications or events that the treatment was intended to prevent or delay. These events may include the return of the cancer or the onset of certain symptoms, such as bone pain from cancer that has spread to the bone, or death.

In some embodiments, the measure of duration of response (DOR) includes the time from documentation of tumor response to disease progression. In some embodiments, the parameter for assessing response can include durable response, e.g., response that persists after a period of time from initiation of therapy. In some embodiments, durable response is indicated by the response rate at approximately 1, 2, 3, 4, 5, 6, 7, 8, 9, 10, 11, 12, 18 or 24 months after initiation of therapy. In some embodiments, the response is durable for greater than 3 months or greater than 6 months.

In some aspects, the RECIST criteria is used to determine objective tumor response; in some aspects, in solid tumors. (Eisenhauer et al., European Journal of Cancer 45 (2009) 228-247.) In some aspects, the RECIST criteria is used to determine objective tumor response for target lesions. In some respects, a complete response as determined using RECIST criteria is described as the disappearance of all target lesions and any pathological lymph nodes (whether target or non-target) must have reduction in short axis to <10 mm. In other aspects, a partial response as determined using RECIST criteria is described as at least a 30% decrease in the sum of diameters of target lesions, taking as reference the baseline sum diameters. In other aspects, progressive disease (PD) is described as at least a 20% increase in the sum of diameters of target lesions, taking as reference the smallest sum on study (this includes the baseline sum if that is the smallest on study). In addition to the relative increase of 20%, the sum must also demonstrate an absolute increase of at least 5 mm (in some aspects the appearance of one or more new lesions is also considered progression). In other aspects, stable disease (SD) is described as neither sufficient shrinkage to qualify for PR nor sufficient increase to qualify for PD, taking as reference the smallest sum diameters while on study.

In some embodiments survival rates in subjects with follicular lymphoma (FL) are based on scoring systems developed by the Italian Lymphoma Intergroup (ILI) and/or the International Follicular Lymphoma Prognostic Factor Project (IFLPFP), generally as described above (Luminari et al., (2012) Rev. Brad. Hematol. Hemoter., 34:54-59). In some embodiments, the extent of disease such as a FL may be assessed by the Ann Arbor staging system, tumor burden, bulky disease, number of nodal or extranodal sites of disease, and/or bone marrow involvement, generally as described above.

In some aspects, the administration in accord with the provided methods, and/or with the provided articles of manufacture or compositions, generally reduces or prevents the expansion or burden of the disease or condition in the subject. For example, where the disease or condition is a tumor, the methods generally reduce tumor size, bulk, metastasis, percentage of blasts in the bone marrow or molecularly detectable cancer and/or improve prognosis or survival or other symptom associated with tumor burden.

Disease burden can encompass a total number of cells of the disease in the subject or in an organ, tissue, or bodily fluid of the subject, such as the organ or tissue of the tumor or another location, e.g., which would indicate metastasis. For example, tumor cells may be detected and/or quantified in the blood or bone marrow in the context of certain hematological malignancies. Disease burden can include, in some embodiments, the mass of a tumor, the number or extent of metastases and/or the percentage of blast cells present in the bone marrow.

In some embodiments, a subject has leukemia. The extent of disease burden can be determined by assessment of residual leukemia in blood or bone marrow.

In some aspects, response rates in subjects, such as subjects with CLL, are based on the International Workshop on Chronic Lymphocytic Leukemia (IWCLL) response criteria (Hallek, et al., Blood 2008, Jun 15; 111(12): 5446-5456). In some aspects, these criteria are described as follows: complete remission (CR; also known in some cases as complete response), which in some aspects requires the absence of peripheral blood clonal lymphocytes by immunophenotyping, absence of lymphadenopathy, absence of hepatomegaly or splenomegaly, absence of constitutional symptoms and satisfactory blood counts; complete remission with incomplete marrow recovery (CRi), which in some aspects is described as CR above, but without normal blood counts; partial remission (PR; also known in some cases as partial response), which in some aspects is described as ≥ 50% fall in lymphocyte count, ≥ 50% reduction in lymphadenopathy or ≥ 50% reduction in liver or spleen, together with improvement in peripheral blood counts; progressive disease (PD), which in some aspects is described as ≥ 50% rise in lymphocyte count to > 5 x10⁹/L, ≥ 50% increase in lymphadenopathy, ≥ 50% increase in liver or spleen size, Richter's transformation, or new cytopenias due to CLL; and stable disease, which in some aspects is described as not meeting criteria for CR, CRi, PR or PD.

In some embodiments, the subjects exhibits a CR or an OR if, within 1 month of the administration of the dose of cells, lymph nodes in the subject are less than at or about 20 mm in size, less than at or about 10 mm in size or less than at or about 10 mm in size.

In some embodiments, an index clone of the CLL is not detected in the bone marrow of the subject (or in the bone marrow of greater than 50%, 60%, 70%, 80%, 90% or more of the subjects treated according to the methods. In some embodiments, an index clone of the CLL is assessed by IgH deep sequencing. In some embodiments, the index clone is not detected at a time that is at or about or at least at or about 1, 2, 3, 4, 5, 6, 12, 18 or 24 months following the administration of the cells.

In some embodiments, a subject exhibits morphologic disease if there are greater than or equal to 5% blasts in the bone marrow, for example, as detected by light microscopy, such as greater than or equal to 10% blasts in the bone marrow, greater than or equal to 20% blasts in the bone marrow, greater than or equal to 30% blasts in the bone marrow, greater than or equal to 40% blasts in the bone marrow or greater than or equal to 50% blasts in the bone marrow. In some embodiments, a subject exhibits complete or clinical remission if there are less than 5% blasts in the bone marrow.

In some embodiments, a subject has leukemia. The extent of disease burden can be determined by assessment of residual leukemia in blood or bone marrow.

In some embodiments, a subject exhibits morphologic disease if there are greater than or equal to 5% blasts in the bone marrow, for example, as detected by light microscopy, such as greater than or equal to 10% blasts in the bone marrow, greater than or equal to 20% blasts in the bone marrow, greater than or equal to 30% blasts in the bone marrow, greater than or equal to 40% blasts in the bone marrow or greater than or equal to 50% blasts in the bone marrow. In some embodiments, a subject exhibits complete or clinical remission if there are less than 5% blasts in the bone marrow.

In some embodiments, a subject may exhibit complete remission, but a small proportion of morphologically undetectable (by light microscopy techniques) residual leukemic cells are present. A subject is said to exhibit minimum residual disease (MRD) if the subject exhibits less than 5% blasts in the bone marrow and exhibits molecularly detectable cancer. In some embodiments, molecularly detectable cancer can be assessed using any of a variety of molecular techniques that permit sensitive detection of a small number of cells. In some aspects, such techniques include PCR assays, which can determine unique Ig/T-cell receptor gene rearrangements or fusion transcripts produced by chromosome translocations. In some embodiments, flow cytometry can be used to identify cancer cell based on leukemia-specific immunophenotypes. In some embodiments, molecular detection of cancer can detect as few as 1 leukemia cell in 100,000 normal cells. In some embodiments, a subject exhibits MRD that is molecularly detectable if at least or greater than 1 leukemia cell in 100,000 cells is detected, such as by PCR or flow cytometry. In some embodiments, the disease burden of a subject is molecularly undetectable or MRD⁻, such that, in some cases, no leukemia cells are able to be detected in the subject using PCR or flow cytometry techniques.

In some embodiments, an index clone of the leukemia, e.g. CLL, is not detected in the bone marrow of the subject (or in the bone marrow of greater than 50%, 60%, 70%, 80%, 90% or more of the subjects treated according to the methods. In some embodiments, an index clone of the leukemia, e.g. CLL, is assessed by IGH deep sequencing. In some embodiments, the index clone is not detected at a time that is at or about or at least at or about 1, 2, 3, 4, 5, 6, 12, 18 or 24 months following the administration of the cells.

In some aspects MRD is detected by flow cytometry. Flow cytometry can be used to monitor bone marrow and peripheral blood samples for cancer cells. In particular aspects, flow cytometry is used to detect or monitor the presence of cancer cells in bone marrow. In some aspects, multiparameter immunological detection by flow cytometry is used to detect cancer cells (see for example, Coustan-Smith et al., (1998) Lancet 351:550-554). In some aspects, multiparameter immunological detection by mass cytometry is used to detect cancer cells. In some examples, 1, 2, 3, 4, 5, 6, 7, 8, 9, 10, 11, 12, 13, 14, 15, 20, 25, 30, 35, 40, 45 or 50 parameters can be used to detect cancer cells. The antigens used for detection are selected based on the cancer being detected (Foon and Todd (1986) Blood 68:1-31).

In some examples, bone marrow is harvested by bone marrow aspirates or bone marrow biopsies, and lymphocytes are isolated for analysis. Monoclonal and/or polyclonal antibodies conjugated to a fluorochrome (*e.g*., fluorescein isothiocyanate (FITC), phycoerythrin, peridinin chlorophyll protein, or biotin) can be used to detect epitopes, such as terminal deoxynucleotidyl transferase (TdT), CD3, CD10, CD11c, CD13, CD14, CD33, CD19, CD20, CD21, CD22, CD23, CD34, CD45, CD56, CD79b, IgM, and/or KORSA3544, on isolated lymphocytes. Labeled cells can then be detected using flow cytometry, such as multiparameter flow cytometry, or mass cytometry, to detect multiple epitopes.

Lymphoid cells can be identified and gated based on a light-scatter dot plot and then secondarily gated to identify cell populations expressing the immunophenotypic features of interest. Exemplary epitopes are set forth in **Table 2** below. Other immunologic classification of leukemias and lymphomas are provided by Foon and Todd (Blood (1986) 68(1): 1-31). In some aspects, flow cytometric assessment of MRD can be achieved by quantifying live lymphocytes bearing one or more CLL immunophenotypes (e.g., low forward/side scatter; CD3^{neg}; CD5⁺; CD14^{neg}; CD19⁺; CD23⁺; CD45⁺; CD56^{neg}).

| **Table 2. Exemplary Immnunophenotype and Cytogentics Characteristics** | | |
|---|---|---|
| **Disease** | **Immunophenotype** | **Cytogenetics** |
| Chronic Lymphocytic Leukemia (CLL) | Pan-B+; CD5+; CD23+; CD79b/CD22 weak; FMC7-; sIg weak | Trisomy12 |
| | | del(13)(q14.3) |
| | | del 11q22-q23 |
| | | del 17p13 (p53) |
| | | t(11;14)(q13;q32) BCL1/IgH rearrangement |
| | | t(14;19)(q32;q13) |
| | | IgH deletion (14q32) |
| | | del(6q) |
| | | +8q24 |
| | | +3 |
| | | +18 |
| | | del 6q21 |
| Small lymphocytic lymphoma (SLL) | Pan-B+; CD5+; CD23+; CD10-; sIgM+ faint | del(6)(q21-23) |
| Lymphoplasmacytic lymphoma | Pan-B+; CD5-; CD10-; cyIgM+ | t(9;14)(p13;q32) PAX5/IgH |
| Follicle centre cell lymphoma | Pan-B+; CD10+/-; CD5-; sIg+ | t(14;18)(q32;q21) / BCL2 Rearr |
| Diffuse large cell lymphoma | CD19+; CD22+; CD10-/+; SIg+ | t(14;18) and p53 mutations t(3;V)(q27;V)/ BCL6 Rearr variants c-MYC Rearr |
| Burkitt's lymphoma | Pan-B+; TdT-; CD10+; CD5-; sIgM+ | t(8;14)(q24;q32) or variants / c-MYC Rearr |
| Burkitt-like lymphoma | Pan-B+; TdT-; CD10-/+ CD5-; sIg+ | t(8;14) or variants |
| | | t(8;14)+ t(14;18) |
| Mantle cell lymphoma | Pan-B +; CD5+; CD23-; CD10-/+; sIgM+ bright | t(11;14)(q13;q32) / BCL1 Rearr |
| Marginal zone B-cell lymphoma (MZBCL) | pan-B+; CD5-/+; CD10-; CD23-; CD11c+/-; cyIg + (40% of the cells), sIgM+ bright; sIgD- | t(11;18)(q21;q21) / PI2 / MLT fusion: Extra-nodal low-grade MALT lymphoma; indolent disease |
| | | t(1;14)(p21;q32): Extra-nodal MALT |
| | | lymphoma |
| | | del(7)(q22-31): Splenic MZBCL |
| | | /+3q: Nodal, extra-nodal and splenic MZBCL |
| +: positive in >90% of the cases | | |
| +/-: positive in more than 50% of the cases | | |
| -/+: positive in less than 50% of cases | | |
| -: positive in <10% of the cases | | |
| Pan-B markers: e.g., CD19, CD20, CD79a | | |
| sIG: surface immunoglobulins | | |
| cyIg: cytoplasmic immunoglobulins | | |

In some aspects, deep sequencing of the immunoglobulin heavy chain (IGH) locus of harvested B cells can be used to detect minimal residual disease (MRD). Clonal presence of a particular IgG rearrangement can provide a marker to detect the presence of B cell malignancies, such as CLL or NHL and/or residual presence of malignant cells thereof. In some aspects cells such as a population containing or suspected of containing B cells are harvested and isolated from blood. In some aspects, cells are harvested and isolated from bone marrow, e.g., from bone marrow aspirates or bone marrow biopsies and/or from other biological samples. In some aspects, polymerase chain reaction (PCR) amplification of the complementarity determining region 3 (CDR3) is achieved using primers to highly conserved sequences within the V and J regions of the gene locus, which may be used to identify clonal populations of cells for purposes of assessing minimal residual disease. Other methods for detecting clonal populations, such as single cell sequencing approaches, including those providing information regarding number of cells of a particular lineage and/or expressing a particular variable chain such as variable heavy chain or binding site thereof, such as a clonal population, may be used. In some aspects, the IGH DNA is amplified using a degenerate primers or primers recognizing regions of variable chains shared among different cell clones, such as those recognizing consensus V and degenerate consensus J region of the IGH sequence. An exemplary sequence of the V region is ACACGGCCTCGTGTATTACTGT (SEQ ID NO: 57). An exemplary degenerate consensus sequence of the J region is ACCTGAGGAGACGGTGACC (SEQ ID NO: 58).

The PCR product or sequencing result in some aspects is specific to the rearranged allele and serves as a clonal marker for MRD detection. Following PCR amplification of the CDR3 region, PCR products can be sequenced to yield patient-specific oligonucleotides constructed as probes for allele-specific PCR for sensitive detection of MRD following treatment of B-cell malignancies with CAR-T cell therapy, e.g. CD19 CAR- T cell therapy. In examples where a PCR product is not generated using the consensus primers, V region family-specific primers for the framework region 1 can be used instead.

In some aspects, persistence of PCR-detectable tumor cells such as cells of the B cell malignancy such as the NHL or CLL, such as detectable IGH sequences corresponding to the malignant or clonal IGH sequences, after treatment is associated with increased risk of relapse. In some aspects, patients who are negative for malignant IGH sequences following treatment (in some aspects, even in the context of other criteria indicating progressive disease or only a partial response, such as persistence of enlarged lymph nodes or other criteria that may in some contexts be associated with disease or lack of complete response) may be deemed to have increased likelihood of PFS or to enter into CR or durable CR or prolonged survival, compared to patients with persistent malignant IGH sequences. In some embodiments, such prognostic and staging determinations are particularly relevant for treatments in which clearance of malignant cells is observed within a short period of time following administration of the therapy, e.g., in comparison to resolution of other clinical symptoms such as lymph node size or other staging criteria. For example, in some such aspects, absence of detectable IGH or minimal residual disease in a sample such as the bone marrow may be a preferred readout for response or likelihood of response or durability thereof, as compared to other available staging or prognostic approaches. In some aspects, results from MRD, e.g., IGH deep sequencing information, may inform further intervention or lack thereof. For example, the methods and other provided embodiments in some contexts provide that a subject deemed negative for malignant IGH may in some aspects be not further treated or not be further administered a dose of the therapy provided, or that the subject be administered a lower or reduced dose. Conversely, it may be provided or specified that a subject exhibiting MRD via IGH deep sequencing be further treated, e.g., with the therapy initially administered at a similar or higher dose or with a further treatment. In some aspects, the disease or condition persists following administration of the first dose and/or administration of the first dose is not sufficient to eradicate the disease or condition in the subject.

In some embodiments, the method reduces the burden of the disease or condition, e.g., number of tumor cells, size of tumor, duration of patient survival or event-free survival, to a greater degree and/or for a greater period of time as compared to the reduction that would be observed with a comparable method using an alternative dosing regimen, such as one in which the subject receives one or more alternative therapeutic agents and/or one in which the subject does not receive a dose of cells and/or a lymphodepleting agent in accord with the provided methods, and/or with the provided articles of manufacture or compositions. In some embodiments, the burden of a disease or condition in the subject is detected, assessed, or measured. Disease burden may be detected in some aspects by detecting the total number of disease or disease-associated cells, e.g., tumor cells, in the subject, or in an organ, tissue, or bodily fluid of the subject, such as blood or serum. In some aspects, survival of the subject, survival within a certain time period, extent of survival, presence or duration of event-free or symptom-free survival, or relapse-free survival, is assessed. In some embodiments, any symptom of the disease or condition is assessed. In some embodiments, the measure of disease or condition burden is specified.

In some embodiments, the event-free survival rate or overall survival rate of the subject is improved by the methods, as compared with other methods, for example, methods in which the subject receives one or more alternative therapeutic agents and/or one in which the subject does not receive a dose of cells and/or a lymphodepleting agent in accord with the provided methods, and/or with the provided articles of manufacture or compositions. For example, in some embodiments, event-free survival rate or probability for subjects treated by the methods at 6 months following the dose is greater than about 40%, greater than about 50%, greater than about 60%, greater than about 70%, greater than about 80%, greater than about 90%, or greater than about 95%. In some aspects, overall survival rate is greater than about 40%, greater than about 50%, greater than about 60%, greater than about 70%, greater than about 80%, greater than about 90%, or greater than about 95%. In some embodiments, the subject treated with the methods exhibits event-free survival, relapse-free survival, or survival to at least 6 months, or at least 1, 2, 3, 4, 5, 6, 7, 8, 9, or 10 years. In some embodiments, the time to progression is improved, such as a time to progression of greater than at or about 6 months, or at least 1, 2, 3, 4, 5, 6, 7, 8, 9, or 10 years.

In some embodiments, following treatment by the method, the probability of relapse is reduced as compared to other methods, for example, methods in which the subject receives one or more alternative therapeutic agents and/or one in which the subject does not receive a dose of cells and/or a lymphodepleting agent in accord with the provided methods, and/or with the provided articles of manufacture or compositions. For example, in some embodiments, the probability of relapse at 6 months following the first dose is less than about 80%, less than about 70%, less than about 60%, less than about 50%, less than about 40%, less than about 30%, less than about 20%, or less than about 10%.

In some cases, the pharmacokinetics of administered cells, e.g., adoptively transferred cells are determined to assess the availability, e.g., bioavailability of the administered cells. Methods for determining the pharmacokinetics of adoptively transferred cells may include drawing peripheral blood from subjects that have been administered engineered cells, and determining the number or ratio of the engineered cells in the peripheral blood. Approaches for selecting and/or isolating cells may include use of chimeric antigen receptor (CAR)-specific antibodies (e.g., Brentjens et al., Sci. Transl. Med. 2013 Mar; 5(177): 177ra38) Protein L (Zheng et al., J. Transl. Med. 2012 Feb; 10:29), epitope tags, such as Strep-Tag sequences, introduced directly into specific sites in the CAR, whereby binding reagents for Strep-Tag are used to directly assess the CAR (Liu et al. (2016) Nature Biotechnology, 34:430; international patent application Pub. No. WO2015095895) and monoclonal antibodies that specifically bind to a CAR polypeptide (see international patent application Pub. No. WO2014190273). Extrinsic marker genes may in some cases be utilized in connection with engineered cell therapies to permit detection or selection of cells and, in some cases, also to promote cell suicide. A truncated epidermal growth factor receptor (EGFRt) in some cases can be co-expressed with a transgene of interest (a CAR or TCR) in transduced cells (see e.g. U.S. Patent No. 8,802,374). EGFRt may contain an epitope recognized by the antibody cetuximab (Erbitux^{®}) or other therapeutic anti-EGFR antibody or binding molecule, which can be used to identify or select cells that have been engineered with the EGFRt construct and another recombinant receptor, such as a chimeric antigen receptor (CAR), and/or to eliminate or separate cells expressing the receptor. See U.S. Patent No. 8,802,374 and Liu et al., Nature Biotech. 2016 April; 34(4): 430-434).

In some embodiments, the number of CAR⁺ T cells in a biological sample obtained from the patient, e.g., blood, can be determined at a period of time after administration of the cell therapy, e.g., to determine the pharmacokinetics of the cells. In some embodiments, number of CAR⁺ T cells, optionally CAR⁺ CD8⁺ T cells and/or CAR⁺ CD4⁺ T cells, detectable in the blood of the subject, or in a majority of subjects so treated by the method, is greater than 1 cells per µL, greater than 5 cells per µL or greater than per 10 cells per µL.

### D. Toxicity

In some embodiments, the provided methods are designed to or include features that result in a lower rate and/or lower degree of toxicity, toxic outcome or symptom, toxicity-promoting profile, factor, or property, such as a symptom or outcome associated with or indicative of cytokine release syndrome (CRS) or neurotoxicity (NT), for example, compared to administration of an alternative cell therapy, such as an alternative CAR⁺ T cell composition and/or an alternative dosing of cells, e.g. a dosing of cells that is not administered at a defined ratio.

In some embodiments, the provided methods do not result in a high rate or likelihood of toxicity or toxic outcomes, or reduces the rate or likelihood of toxicity or toxic outcomes, such as neurotoxicity (NT), cytokine release syndrome (CRS), such as compared to certain other cell therapies. In some embodiments, the methods do not result in, or do not increase the risk of, severe NT (sNT), severe CRS (sCRS), macrophage activation syndrome, tumor lysis syndrome, fever of at least at or about 38 degrees Celsius for three or more days and a plasma level of CRP of at least at or about 20 mg/dL. In some embodiments, greater than or greater than about 30%, 35%, 40%, 50%, 55%, 60% or more of the subjects treated according to the provided methods do not exhibit any grade of CRS or any grade of neurotoxcity. In some embodiments, no more than 50% of subjects treated (e.g. at least 60%, at least 70%, at least 80%, at least 90% or more of the subjects treated) exhibit a cytokine release syndrome (CRS) higher than grade 2 and/or a neurotoxicity higher than grade 2. In some embodiments, at least 50% of subjects treated according to the method (e.g. at least 60%, at least 70%, at least 80%, at least 90% or more of the subjects treated) do not exhibit a severe toxic outcome (e.g. severe CRS or severe neurotoxicity), such as do not exhibit grade 3 or higher neurotoxicity and/or does not exhibit severe CRS, or does not do so within a certain period of time following the treatment, such as within a week, two weeks, or one month of the administration of the cells. In some embodiments, parameters assessed to determine certain toxicities include adverse events (AEs), dose-limiting toxicities (DLTs), CRS and NT.

Administration of adoptive T cell therapy, such as treatment with T cells expressing chimeric antigen receptors, can induce toxic effects or outcomes such as cytokine release syndrome and neurotoxicity. In some examples, such effects or outcomes parallel high levels of circulating cytokines, which may underlie the observed toxicity.

In some aspects, the toxic outcome is or is associated with or indicative of cytokine release syndrome (CRS) or severe CRS (sCRS). CRS, e.g., sCRS, can occur in some cases following adoptive T cell therapy and administration to subjects of other biological products. See Davila et al., Sci Transl Med 6, 224ra25 (2014); Brentjens et al., Sci. Transl. Med. 5, 177ra38 (2013); Grupp et al., N. Engl. J. Med. 368, 1509-1518 (2013); and Kochenderfer et al., Blood 119, 2709-2720 (2012); Xu et al., Cancer Letters 343 (2014) 172-78.

Typically, CRS is caused by an exaggerated systemic immune response mediated by, for example, T cells, B cells, NK cells, monocytes, and/or macrophages. Such cells may release a large amount of inflammatory mediators such as cytokines and chemokines. Cytokines may trigger an acute inflammatory response and/or induce endothelial organ damage, which may result in microvascular leakage, heart failure, or death. Severe, life-threatening CRS can lead to pulmonary infiltration and lung injury, renal failure, or disseminated intravascular coagulation. Other severe, life-threatening toxicities can include cardiac toxicity, respiratory distress, neurologic toxicity and/or hepatic failure. In some aspects, fever, especially high fever (≥ 38.5°C or ≥ 101.3°F), is associated with CRS or risk thereof. In some cases, features or symptoms of CRS mimic infection. In some embodiments, infection is also considered in subjects presenting with CRS symptoms, and monitoring by cultures and empiric antibiotic therapy can be administered. Other symptoms associated with CRS can include cardiac dysfunction, adult respiratory distress syndrome, renal and/or hepatic failure, coagulopathies, disseminated intravascular coagulation, and capillary leak syndrome.

CRS may be treated using anti-inflammatory therapy such as an anti-IL-6 therapy, e.g., anti-IL-6 antibody, e.g., tocilizumab, or antibiotics or other agents as described. Outcomes, signs and symptoms of CRS are known and include those described herein. In some embodiments, where a particular dosage regimen or administration effects or does not effect a given CRS-associated outcome, sign, or symptom, particular outcomes, signs, and symptoms and/or quantities or degrees thereof may be specified.

In the context of administering CAR-expressing cells, CRS typically occurs 6-20 days after infusion of cells that express a CAR. See Xu et al., Cancer Letters 343 (2014) 172-78. In some cases, CRS occurs less than 6 days or more than 20 days after CAR T cell infusion. The incidence and timing of CRS may be related to baseline cytokine levels or tumor burden at the time of infusion. Commonly, CRS involves elevated serum levels of interferon (IFN)-γ, tumor necrosis factor (TNF)-α, and/or interleukin (IL)-2. Other cytokines that may be rapidly induced in CRS are IL-1β, IL-6, IL-8, and IL-10.

Exemplary outcomes associated with CRS include fever, rigors, chills, hypotension, dyspnea, acute respiratory distress syndrome (ARDS), encephalopathy, ALT/AST elevation, renal failure, cardiac disorders, hypoxia, neurologic disturbances, and death. Neurological complications include delirium, seizure-like activity, confusion, word-finding difficulty, aphasia, and/or becoming obtunded. Other CRS-related outcomes include fatigue, nausea, headache, seizure, tachycardia, myalgias, rash, acute vascular leak syndrome, liver function impairment, and renal failure. In some aspects, CRS is associated with an increase in one or more factors such as serum-ferritin, d-dimer, aminotransferases, lactate dehydrogenase and triglycerides, or with hypofibrinogenemia or hepatosplenomegaly. Other exemplary signs or symptoms associated with CRS include hemodynamic instability, febrile neutropenia, increase in serum C-reactive protein (CRP), changes in coagulation parameters (for example, international normalized ratio (INR), prothrombin time (PTI) and/or fibrinogen), changes in cardiac and other organ function, and/or absolute neutrophil count (ANC).

In some embodiments, outcomes associated with CRS include one or more of: persistent fever, e.g., fever of a specified temperature, e.g., greater than at or about 38 degrees Celsius, for two or more, e.g., three or more, e.g., four or more days or for at least three consecutive days; fever greater than at or about 38 degrees Celsius; elevation of cytokines, such as a max fold change, e.g., of at least at or about 75, compared to pre-treatment levels of at least two cytokines (e.g., at least two of the group consisting of interferon gamma (IFNγ), GM-CSF, IL-6, IL-10, Flt-3L, fracktalkine, and IL-5, and/or tumor necrosis factor alpha (TNFα)), or a max fold change, e.g., of at least at or about 250 of at least one of such cytokines; and/or at least one clinical sign of toxicity, such as hypotension (e.g., as measured by at least one intravenous vasoactive pressor); hypoxia (e.g., plasma oxygen (PO₂) levels of less than at or about 90%); and/or one or more neurologic disorders (including mental status changes, obtundation, and seizures). In some embodiments, neurotoxicity (NT) can be observed concurrently with CRS.

Exemplary CRS-related outcomes include increased or high serum levels of one or more factors, including cytokines and chemokines and other factors associated with CRS. Exemplary outcomes further include increases in synthesis or secretion of one or more of such factors. Such synthesis or secretion can be by the T cell or a cell that interacts with the T cell, such as an innate immune cell or B cell.

In some embodiments, the CRS-associated serum factors or CRS-related outcomes include inflammatory cytokines and/or chemokines, including interferon gamma (IFN-γ), TNF-a, IL-1β, IL-2, IL-6, IL-7, IL-8, IL-10, IL-12, sIL-2Ra, granulocyte macrophage colony stimulating factor (GM-CSF), macrophage inflammatory protein (MIP)-1, tumor necrosis factor alpha (TNFα), IL-6, and IL-10, IL-1β, IL-8, IL-2, MIP-1, Flt-3L, fracktalkine, and/or IL-5. In some embodiments, the factor or outcome includes C reactive protein (CRP). In addition to being an early and easily measurable risk factor for CRS, CRP also is a marker for cell expansion. In some embodiments, subjects that are measured to have high levels of CRP, such as ≥ 15 mg/dL, have CRS. In some embodiments, subjects that are measured to have high levels of CRP do not have CRS. In some embodiments, a measure of CRS includes a measure of CRP and another factor indicative of CRS.

In some embodiments, one or more inflammatory cytokines or chemokines are monitored before, during, or after CAR treatment. In some aspects, the one or more cytokines or chemokines include IFN-γ, TNF-α, IL-2, IL-1β, IL-6, IL-7, IL-8, IL-10, IL-12, sIL-2Rα, granulocyte macrophage colony stimulating factor (GM-CSF), or macrophage inflammatory protein (MIP). In some embodiments, IFN-γ, TNF-α, and IL-6 are monitored.

CRS criteria that appear to correlate with the onset of CRS to predict which patients are more likely to be at risk for developing sCRS have been developed (see Davilla et al. Science translational medicine. 2014;6(224):224ra25). Factors include fevers, hypoxia, hypotension, neurologic changes, elevated serum levels of inflammatory cytokines, such as a set of seven cytokines (IFNγ, IL-5, IL-6, IL-10, Flt-3L, fractalkine, and GM-CSF) whose treatment-induced elevation can correlate well with both pretreatment tumor burden and sCRS symptoms. Other guidelines on the diagnosis and management of CRS are known (see e.g., Lee et al, Blood. 2014;124(2):188-95). In some embodiments, the criteria reflective of CRS grade are those detailed in **Table 3** below.

| **Table 3: Exemplary Grading Criteria for CRS** | |
|---|---|
| **Grade** | **Description of Symptoms** |
| 1 Mild | Not life-threatening, require only symptomatic treatment such as antipyretics and anti-emetics (e.g., fever, nausea, fatigue, headache, myalgias, malaise) |
| 2 Moderate | Require and respond to moderate intervention: |
| | • Oxygen requirement < 40%, or |
| | • Hypotension responsive to fluids or low dose of a single vasopressor, or |
| | • Grade 2 organ toxicity (by CTCAE v4.0) |
| 3 Severe | Require and respond to aggressive intervention: |
| | • Oxygen requirement ≥ 40%, or |
| | • Hypotension requiring high dose of a single vasopressor (e.g., norepinephrine ≥ 20 µg/kg/min, dopamine ≥ 10 µg/kg/min, phenylephrine ≥ 200 µg/kg/min, or epinephrine ≥ 10 µg/kg/min), or |
| | • Hypotension requiring multiple vasopressors (e.g., vasopressin + one of the above agents, or combination vasopressors equivalent to ≥ 20 µg/kg/min norepinephrine), or |
| | • Grade 3 organ toxicity or Grade 4 transaminitis (by CTCAE v4.0) |
| 4 Life-threatening | Life-threatening: |
| | • Requirement for ventilator support, or |
| | • Grade 4 organ toxicity (excluding transaminitis) |
| 5 Fatal | Death |

In some embodiments, a criteria reflective of CRS grade are those detailed in **Table 4** below.

| **Table 4.** Exemplary Grading Criteria for CRS | | | | |
|---|---|---|---|---|
| **Symptoms/Signs** | **Grade 1 (mild)** | **Grade 2 (moderate)** | **Grade 3 (severe)** | **Grade 4 (life-threatening)** |
| | | CRS grade is defined by the most severe symptom (excluding fever) | | |
| Temperature ≥ 38.5°C/101.3°F | Any | Any | Any | Any |
| Systolic blood pressure ≤ 90 mm Hg | N/A | Responds to fluid or single low-dose vasopressor | Needs high-dose or multiple vasopressors | Life-threatening |
| Need for oxygen to reach SaO₂ > 90% | N/A | FiO2 < 40% | FiO₂ ≥ 40% | Needs ventilator support |
| Organ toxicity | N/A | Grade 2 | Grade 3 or transaminitis | Grade 4 (excluding transaminitis) |

In some embodiments, high-dose vasopressor therapy include those described in Table 5 below.

| **Table 5.** High dose vasopressors (all doses required for ≥ 3 hours) | |
|---|---|
| **Vasopressor** | **Dose** |
| Norepinephrine monotherapy | ≥ 20 µg/min |
| Dopamine monotherapy | ≥ 10 µg/kg/min |
| Phenylephrine monotherapy | ≥ 200 µg/min |
| Epinephrine monotherapy | ≥ 10 µg/min |
| If on vasopressin | Vasopressin + norepinephrine equivalent (NE) of ≥ 10 µg/min^{a} |
| If on combination vasopressors (not vasopressin) | Norepinephrine equivalent of ≥ 20 µg/min^{a} |
| ^{a} VASST Trial Vasopressor Equivalent Equation: Norepinephrine equivalent dose = [norepinephrine (µg/min)] + [dopamine (µg/kg/min) ÷ 2] + [epinephrine (µg/min)] + [phenylephrine (µg/min) ÷ 10] | |

In some embodiments, the toxic outcome is a severe CRS. In some embodiments, the toxic outcome is the absence of severe CRS (e.g. moderate or mild CRS). In some embodiments, a subject is deemed to develop "severe CRS" ("sCRS") in response to or secondary to administration of a cell therapy or dose of cells thereof, if, following administration, the subject displays: (1) fever of at least 38 degrees Celsius for at least three days; (2) cytokine elevation that includes either (a) a max fold change of at least 75 for at least two of the following group of seven cytokines compared to the level immediately following the administration: interferon gamma (IFNγ), GM-CSF, IL-6, IL-10, Flt-3L, fracktalkine, and IL-5 and/or (b) a max fold change of at least 250 for at least one of the following group of seven cytokines compared to the level immediately following the administration: interferon gamma (IFNγ), GM-CSF, IL-6, IL-10, Flt-3L, fracktalkine, and IL-5; and (c) at least one clinical sign of toxicity such as hypotension (requiring at least one intravenous vasoactive pressor) or hypoxia (PO₂ < 90%) or one or more neurologic disorder(s) (including mental status changes, obtundation, and/or seizures). In some embodiments, severe CRS includes CRS with a grade of 3 or greater, such as set forth in **Table** 3 and **Table 4.**

In some embodiments, the level of the toxic outcome, e.g. the CRS-related outcome, e.g. the serum level of an indicator of CRS, is measured by ELISA. In some embodiments, fever and/or levels of C-reactive protein (CRP) can be measured. In some embodiments, subjects with a fever and a CRP ≥ 15 mg/dL may be considered high-risk for developing severe CRS. In some embodiments, the CRS-associated serum factors or CRS-related outcomes include an increase in the level and/or concentration of inflammatory cytokines and/or chemokines, including Flt-3L, fracktalkine, granulocyte macrophage colony stimulating factor (GM-CSF), interleukin-1 beta (IL-1β), IL-2, IL-5, IL-6, IL-7, IL-8, IL-10, IL-12, interferon gamma (IFN-γ), macrophage inflammatory protein (MIP)-1, MIP-1, sIL-2Rα, or tumor necrosis factor alpha (TNFα). In some embodiments, the factor or outcome includes C reactive protein (CRP). In addition to being an early and easily measurable risk factor for CRS, CRP also is a marker for cell expansion. In some embodiments, subjects that are measured to have high levels of CRP, such as ≥ 15 mg/dL, have CRS. In some embodiments, subjects that are measured to have high levels of CRP do not have CRS. In some embodiments, a measure of CRS includes a measure of CRP and another factor indicative of CRS.

In some embodiments, outcomes associated with severe CRS or grade 3 CRS or greater, such as grade 4 or greater, include one or more of: persistent fever, e.g., fever of a specified temperature, e.g., greater than at or about 38 degrees Celsius, for two or more, e.g., three or more, e.g., four or more days or for at least three consecutive days; fever greater than at or about 38 degrees Celsius; elevation of cytokines, such as a max fold change, e.g., of at least at or about 75, compared to pre-treatment levels of at least two cytokines (e.g., at least two of the group consisting of interferon gamma (IFNγ), GM-CSF, IL-6, IL-10, Flt-3L, fracktalkine, and IL-5, and/or tumor necrosis factor alpha (TNFα)), or a max fold change, e.g., of at least at or about 250 of at least one of such cytokines; and/or at least one clinical sign of toxicity, such as hypotension (e.g., as measured by at least one intravenous vasoactive pressor); hypoxia (e.g., plasma oxygen (PO₂) levels of less than at or about 90%); and/or one or more neurologic disorders (including mental status changes, obtundation, and seizures). In some embodiments, severe CRS includes CRS that requires management or care in the intensive care unit (ICU).

In some embodiments, the CRS, such as severe CRS, encompasses a combination of (1) persistent fever (fever of at least 38 degrees Celsius for at least three days) and (2) a serum level of CRP of at least at or about 20 mg/dL. In some embodiments, the CRS encompasses hypotension requiring the use of two or more vasopressors or respiratory failure requiring mechanical ventilation. In some embodiments, the dosage of vasopressors is increased in a second or subsequent administration.

In some embodiments, severe CRS or grade 3 CRS encompasses an increase in alanine aminotransferase, an increase in aspartate aminotransferase, chills, febrile neutropenia, headache, left ventricular dysfunction, encephalopathy, hydrocephalus, and/or tremor.

The method of measuring or detecting the various outcomes may be specified.

In some aspects, the toxic outcome is or is associated with neurotoxicity. In some embodiments, symptoms associated with a clinical risk of neurotoxicity include confusion, delirium, aphasia, expressive aphasia, obtundation, myoclonus, lethargy, altered mental status, convulsions, seizure-like activity, seizures (optionally as confirmed by electroencephalogram (EEG)), elevated levels of beta amyloid (Aβ), elevated levels of glutamate, and elevated levels of oxygen radicals. In some embodiments, neurotoxicity is graded based on severity (e.g., using a Grade 1-5 scale (see, e.g., Guido Cavaletti & Paola Marmiroli Nature Reviews Neurology 6, 657-666 (December 2010); National Cancer Institute-Common Toxicity Criteria version 4.03 (NCI-CTCAE v4.03).

In some instances, neurologic symptoms may be the earliest symptoms of sCRS. In some embodiments, neurologic symptoms are seen to begin 5 to 7 days after cell therapy infusion. In some embodiments, duration of neurologic changes may range from 3 to 19 days. In some cases, recovery of neurologic changes occurs after other symptoms of sCRS have resolved. In some embodiments, time or degree of resolution of neurologic changes is not hastened by treatment with anti-IL-6 and/or steroid(s).

In some embodiments, a subject is deemed to develop "severe neurotoxicity" in response to or secondary to administration of a cell therapy or dose of cells thereof, if, following administration, the subject displays symptoms that limit self-care (e.g. bathing, dressing and undressing, feeding, using the toilet, taking medications) from among: 1) symptoms of peripheral motor neuropathy, including inflammation or degeneration of the peripheral motor nerves; 2) symptoms of peripheral sensory neuropathy, including inflammation or degeneration of the peripheral sensory nerves, dysesthesia, such as distortion of sensory perception, resulting in an abnormal and unpleasant sensation, neuralgia, such as intense painful sensation along a nerve or a group of nerves, and/or paresthesia, such as functional disturbances of sensory neurons resulting in abnormal cutaneous sensations of tingling, numbness, pressure, cold and warmth in the absence of stimulus. In some embodiments, severe neurotoxicity includes neurotoxicity with a grade of 3 or greater, such as set forth in **Table 6.**

| **Table 6: Exemplary Grading Criteria for neurotoxicity** | |
|---|---|
| **Grade** | **Description of Symptoms** |
| 1 Asymptomatic or Mild | Mild or asymptomatic symptoms |
| 2 Moderate | Presence of symptoms that limit instrumental activities of daily living (ADL), such as preparing meals, shopping for groceries or clothes, using the telephone, managing money |
| 3 Severe | Presence of symptoms that limit self-care ADL, such as bathing, dressing and undressing, feeding self, using the toilet, taking medications |
| 4 Life-threatening | Symptoms that are life-threatening, requiring urgent intervention |
| 5 Fatal | Death |

In some embodiments, the methods reduce symptoms associated with CRS or neurotoxicity compared to other methods. In some aspects, the provided methods reduce symptoms, outcomes or factors associated with CRS, including symptoms, outcomes or factors associated with severe CRS or grade 3 or higher CRS, compared to other methods. For example, subjects treated according to the present methods may lack detectable and/or have reduced symptoms, outcomes or factors of CRS, e.g. severe CRS or grade 3 or higher CRS, such as any described, e.g. set forth in **Table 3** and **Table 4.** In some embodiments, subjects treated according to the present methods may have reduced symptoms of neurotoxicity, such as limb weakness or numbness, loss of memory, vision, and/or intellect, uncontrollable obsessive and/or compulsive behaviors, delusions, headache, cognitive and behavioral problems including loss of motor control, cognitive deterioration, and autonomic nervous system dysfunction, and sexual dysfunction, compared to subjects treated by other methods. In some embodiments, subjects treated according to the present methods may have reduced symptoms associated with peripheral motor neuropathy, peripheral sensory neuropathy, dysethesia, neuralgia or paresthesia.

In some embodiments, the methods reduce outcomes associated with neurotoxicity including damages to the nervous system and/or brain, such as the death of neurons. In some aspects, the methods reduce the level of factors associated with neurotoxicity such as beta amyloid (Aβ), glutamate, and oxygen radicals.

In some embodiments, the toxicity outcome is a dose-limiting toxicity (DLT). In some embodiments, the toxic outcome is a dose-limiting toxicity. In some embodiments, the toxic outcome is the absence of a dose-limiting toxicity. In some embodiments, a dose-limiting toxicity (DLT) is defined as any grade 3 or higher toxicity as assessed by any known or published guidelines for assessing the particular toxicity, such as any described above and including the National Cancer Institute (NCI) Common Terminology Criteria for Adverse Events (CTCAE) version 4.0.

In some embodiments, the low rate, risk or likelihood of developing a toxicity, e.g. CRS or neurotoxicity or severe CRS or neurotoxicity, e.g. grade 3 or higher CRS or neurotoxicity, observed with administering a dose of T cells in accord with the provided methods, and/or with the provided articles of manufacture or compositions, permits administration of the cell therapy on an outpatient basis. In some embodiments, the administration of the cell therapy, e.g. dose of T cells (e.g. CAR⁺ T cells) in accord with the provided methods, and/or with the provided articles of manufacture or compositions, is performed on an outpatient basis or does not require admission to the subject to the hospital, such as admission to the hospital requiring an overnight stay.

In some aspects, subjects administered the cell therapy, e.g. dose of T cells (e.g. CAR⁺ T cells) in accord with the provided methods, and/or with the provided articles of manufacture or compositions, including subjects treated on an outpatient basis, are not administered an intervention for treating any toxicity prior to or with administration of the cell dose, unless or until the subject exhibits a sign or symptom of a toxicity, such as of a neurotoxicity or CRS. Exemplary agents for treating, delaying, attenuating or ameliorating a toxicity are described in Section II.

In some embodiments, if a subject administered the cell therapy, e.g. dose of T cells (e.g. CAR⁺ T cells), including subjects treated on an outpatient basis, exhibits a fever the subject is given or is instructed to receive or administer a treatment to reduce the fever. In some embodiments, the fever in the subject is characterized as a body temperature of the subject that is (or is measured at) at or above a certain threshold temperature or level. In some aspects, the threshold temperature is that associated with at least a low-grade fever, with at least a moderate fever, and/or with at least a high-grade fever. In some embodiments, the threshold temperature is a particular temperature or range. For example, the threshold temperature may be at or about or at least at or about 38, 39, 40, 41, or 42 degrees Celsius, and/or may be a range of at or about 38 degrees Celsius to at or about 39 degrees Celsius, a range of at or about 39 degrees Celsius to at or about 40 degrees Celsius, a range of at or about 40 degrees Celsius to at or about 41 degrees, or a range of at or about 41 degrees Celsius to at or about 42 degrees Celsius.

In some embodiments, the treatment designed to reduce fever includes treatment with an antipyretic. An antipyretic may include any agent, e.g., compound, composition, or ingredient, that reduces fever, such as one of any number of agents known to have antipyretic effects, such as NSAIDs (such as ibuprofen, naproxen, ketoprofen, and nimesulide), salicylates, such as aspirin, choline salicylate, magnesium salicylate, and sodium salicylate, paracetamol, acetaminophen, Metamizole, Nabumetone, Phenaxone, antipyrine, febrifuges. In some embodiments, the antipyretic is acetaminophen. In some embodiments, acetaminophen can be administered at a dose of 12.5 mg/kg orally or intravenously up to every four hours. In some embodiments, it is or comprises ibuprofen or aspirin.

In some embodiments, if the fever is a sustained fever, the subject is administered an alternative treatment for treating the toxicity, such as any described in Section II below. For subjects treated on an outpatient basis, the subject is instructed to return to the hospital if the subject has and/or is determined to or to have a sustained fever. In some embodiments, the subject has, and/or is determined to or considered to have, a sustained fever if he or she exhibits a fever at or above the relevant threshold temperature, and where the fever or body temperature of the subject is not reduced, or is not reduced by or by more than a specified amount (e.g., by more than 1 °C, and generally does not fluctuate by about, or by more than about, 0.5 °C, 0.4 °C, 0.3 °C, or 0.2 °C), following a specified treatment, such as a treatment designed to reduce fever such as treatment with an antipyreticm, e.g. NSAID or salicylates, e.g. ibuprofen, acetaminophen or aspirin. For example, a subject is considered to have a sustained fever if he or she exhibits or is determined to exhibit a fever of at least at or about 38 or 39 degrees Celsius, which is not reduced by or is not reduced by more than at or about 0.5 °C, 0.4 °C, 0.3 °C, or 0.2 °C, or by at or about 1%, 2%, 3%, 4%, or 5%, over a period of 6 hours, over a period of 8 hours, or over a period of 12 hours, or over a period of 24 hours, even following treatment with the antipyretic such as acetaminophen. In some embodiments, the dosage of the antipyretic is a dosage ordinarily effective in such as subject to reduce fever or fever of a particular type such as fever associated with a bacterial or viral infection, e.g., a localized or systemic infection.

In some embodiments, the subject has, and/or is determined to or considered to have, a sustained fever if he or she exhibits a fever at or above the relevant threshold temperature, and where the fever or body temperature of the subject does not fluctuate by about, or by more than about, 1 °C, and generally does not fluctuate by about, or by more than about, 0.5 °C, 0.4 °C, 0.3 °C, or 0.2 °C. Such absence of fluctuation above or at a certain amount generally is measured over a given period of time (such as over a 24-hour, 12-hour, 8-hour, 6-hour, 3-hour, or 1-hour period of time, which may be measured from the first sign of fever or the first temperature above the indicated threshold). For example, in some embodiments, a subject is considered to or is determined to exhibit sustained fever if he or she exhibits a fever of at least at or about or at least at or about 38 or 39 degrees Celsius, which does not fluctuate in temperature by more than at or about 0.5°C, 0.4 °C, 0.3 °C, or 0.2 °C, over a period of 6 hours, over a period of 8 hours, or over a period of 12 hours, or over a period of 24 hours.

In some embodiments, the fever is a sustained fever; in some aspects, the subject is treated at a time at which a subject has been determined to have a sustained fever, such as within one, two, three, four, five six, or fewer hours of such determination or of the first such determination following the initial therapy having the potential to induce the toxicity, such as the cell therapy, such as dose of T cells, e.g. CAR⁺ T cells.

In some embodiments, one or more interventions or agents for treating the toxicity, such as a toxicity-targeting therapies, is administered at a time at which or immediately after which the subject is determined to or confirmed to (such as is first determined or confirmed to) exhibit sustained fever, for example, as measured according to any of the aforementioned embodiments. In some embodiments, the one or more toxicity-targeting therapies is administered within a certain period of time of such confirmation or determination, such as within 30 minutes, 1 hour, 2 hours, 3 hours, 4 hours, 6 hours, or 8 hours thereof.

### E. Health Status and Health Related Quality of Life

In some embodiments, the provided methods are designed to or include features that result in, an increase in subject reported Health Related Quality of Life (HQRL) and effect on symptom impact after administration of the dose of CAR+ T cells compared to prior to the administration. In some embodiments, such features can be monitored or assessed by administration of the European Organization for Research and Treatment Core Quality of Life Questionnaire version 3.0 (EORTC QLQ-C30) prior to administration of the dose of CAR-expressing cells (e.g., at baseline (BL)) and at one or more various times post-administration of the CAR-expressing cells, e.g. every two weeks, every month, every two months, every 3 months or more. In some embodiments, the assessments can include a measure of the strength of a person's preference for a specific health state in relation to alternative health states. In some aspects, the EORTC QLQ-C30 is a quality of life measure applicable to subjects with any cancer diagnosis. In some aspects, it includes 30 items that address general physical symptoms, physical functioning, fatigue and malaise, and social and emotional functioning. In some aspects, subscale scores are transformed to a 0 to 100 scale, with higher scores on functional scales indicating better function and higher scores on symptom scales indicating worse symptoms. In some embodiments, the utility scale assigns numerical values on a scale from 0 (death) to 1 (optimal or "perfect" health). In some embodiments, time spent in different health states is aggregated into quality-adjusted life-years.

In some embodiments, the provided methods are designed to, or include features that result in, increases in well-being and perceived health status in subjects, such as determined by the EuroQol-5D (EQ-5D), a standardized measure of health status developed by the EuroQoL Group as a simple, generic measure of health for clinical and economic appraisal. In some embodiments, the provided methods are designed to, or include features that result in, increases in well-being and perceived health status in subjects, such as determined by the health utility index score and visual analog scale [VAS] (EQ-5D-5L). In some embodiments, the EQ-5D-5L (a generic, preference-based measure), allows for estimation of health state utility (Brooks R. Health Policy (1996); 37(1):53-72; The EuroQoL Group. Health Policy (1990); 16(3):199-208; Pickard AS et al. health Qual Life Outcomes (2007); 5:70) In some embodiments, the EQ-5D-5L allows for estimation of health state utility in a wide range of health conditions. In some embodiments, the EQ-5D-5L includes a single summary index (i.e., health state index score) and a global visual analog scale (EQ-VAS). In some embodiments, the EQ-5D-5L health status evaluation is composed of 5 dimensions of health including mobility, self-care, usual activities, pain/discomfort and anxiety/depression. In some embodiments, the EQ-5D-5L is scored as a single summary index using a US value set on a -0.109 to 1 scale, with a score of 0 indicating death, 1 indicating "full health," and negative scores reflecting states perceived to be worse than death. In some embodiments, a change from baseline of ≥0.07 in the EQ-5D-5L single summary index score is an indicator of clinically meaningful change (Pickard et al. Health Qual Life Outcomes (2007); 5:70).

In some embodiments, the EQ-VAS is rated on a 0-100 scale, with 0 representing the worst imaginable health state and 100 representing the best imaginable health state. In some embodiments, subjects assess their health on the day of assessment by placing an X on a scale numbered from 0-100 (EQ-5D-5L User Guide. https://euroqol.org/wp-content/uploads/2016/09/ EQ-5D-5L_UserGuide_2015.pdf. Accessed April 9, 2019).

In some embodiments, subjects determine their score. In some aspects, the descriptive system comprises dimensions (mobility, self-care, usual activities, pain/discomfort, anxiety/depression). In some aspects, each dimension has 5 levels (no problems, slight problems, moderate problems, severe problems, extreme problems). The VAS rating scale is a vertical 20 cm visual analogue scale with the end points labeled best imaginable health state at the top and worst imaginable health state at the bottom having numeric values of 100 and 0 respectively. In some cases, the participant is asked to indicate his/her health state by ticking (or placing a cross) in the box against the most appropriate statement in each of the 5 dimensions.

In some aspects, patient-reported outcomes (PROs) can be used to measure a patient's experience with their treatment. In some aspects, PROs can be provided directly by the patient and without interpretation from a healthcare personnel. In some aspects, exemplar tools for examining HRQoL in patients receiving CAR T cell therapy include PROMIS, SF-36, and EQ-5D-5L. In some of any embodiments, EORTC QLQ-C30 and EQ-5D-5L are used to assess health-related quality of life. In some aspects, both short-term and long-term effects of the treatment on a patient's HRQoL is examined.

In some embodiments, greater than or greater than about 25%, 30%, 35%, 40%, 45%, 50%, 55% or 60% of the subjects treated according to the provided embodiments exhibit an improvement of 10 points or greater in European Organization for Research and Treatment Core Quality of Life Questionnaire version 3.0 (EORTC QLQ-C30) in global health status at months 6 or month 12 after administration compared to the score prior to treatment or at month 1 after treatment. In some embodiments, greater than or greater than about 25%, 30%, 35%, 40%, 45%, 50%, 55% or 60% of the subjects treated according to the provided embodiments exhibit an improvement of 10 points or greater in EORTC QLQ-C30 in physical functioning at months 6 or month 12 after administration compared to the score prior to treatment or at month 1 after treatment. In some embodiments, greater than or greater than about 25%, 30%, 35%, 40%, 45%, 50%, 55% or 60% of the subjects treated according to the provided embodiments an improvement of 10 points or greater in EORTC QLQ-C30 in fatigue at months 6 or month 12 after administration compared to the score prior to treatment or at month 1 after treatment. In some embodiments, greater than or greater than about 25%, 30%, 35%, 40%, 45%, 50%, 55% or 60% of the subjects treated according to the provided embodiments exhibit an improvement of 10 points or greater in EORTC QLQ-C30 in pain at months 6 or month 12 after administration compared to the score prior to treatment or at month 1 after treatment. In some embodiments, greater than or greater than about 25%, 30%, 35%, 40%, 45%, 50%, 55% or 60% of the subjects treated according to the provided embodiments exhibit an improvement of 10 points or greater in EORTC QLQ-C30 in pain at months 6 or month 12 after administration compared to the score prior to treatment or at month 1 after treatment.

In some embodiments, the mean 5-level EuroQol-5D (EQ-5D-5L) score among subjects treated according to the provided embodiments is the same or greater at months 6 or month 12 after administration compared to the score prior to treatment or at month 1 after treatment. In some embodiments, the mean EuroQol global visual analog scale (EQ-VAS) score among subjects treated according to the provided embodiments is the same or greater at months 6 or month 12 after administration compared to the score prior to treatment or at month 1 after treatment.

### F. Biomarkers, Analytes or Parameters

Among the provided methods are methods of assessing a risk for developing toxicity associated with cell therapy in a subject that involves assessing or detecting biomarkers (*e.g.,* analytes) or parameters that are associated with the toxicity, *e.g.,* neurotoxicity, such as severe neurotoxicity, and/or CRS, such as severe CRS. Also among the provided methods are methods of assessing the likelihood of response to a cell therapy in a subject that involves assessing or detecting biomarkers (*e.g.,* analytes) or parameters that are associated with a response outcome, such as objective response (OR), including complete response (CR) and partial response (PR). In some embodiments, the associate response outcome includes durable response, such as a response that is durable for 3 months, 6 months, 9 months 12 months or more, after the initial response.

In some embodiments, the methods involve assessing or detecting the presence or absence of one or a panel of biomarkers (e.g. analytes) and/or parameters (e.g. concentration, amount, level or activity) associated with one or a panel of biomarkers (e.g. analytes). In some cases, the methods can include comparing the one or more parameters to a particular reference value, such as a threshold level (also called "threshold value" herein), *e.g.,* those associated with a risk for developing toxicity or those associated with a particular response, such as OR, CR or PR, or durable response, such as a response that is durable for 3 months, 6 months, 9 months 12 months or more, after the initial response. In some embodiments, the methods also involve selecting subjects for treatment with a cell therapy based on the assessment of the presence or absence of the biomarker and/or comparison of the biomarkers to a reference value or threshold level of the biomarker. In some embodiments, the methods also involve administering an agent or a therapy that can treat, prevent, delay and/or attenuate development of the toxicity, *e.g.,* based on the assessment of the presence or absence of the biomarker and/or comparison of the biomarkers to a reference value or threshold level of the biomarker.

In some embodiments, the methods involve assessing the likelihood of response of the subject or the risk of development of a toxicity, after administration of a cell therapy. In some embodiments, the methods involve assessing the level, amount or concentration of one or more analyte in a biological sample, wherein the biological sample is from a subject that is a candidate for treatment with the cell therapy, said cell therapy optionally comprising a dose or composition of genetically engineered cells expressing a recombinant receptor; and the biological sample is obtained from the subject prior to administering the cell therapy and/or said biological sample does not comprise the recombinant receptor and/or said engineered cells. In some aspects, the methods involve comparing, individually, the level, amount or concentration of the analyte in the sample to a threshold level, thereby determining a risk of developing a toxicity after administration of the cell therapy. In some aspects, the comparisons can be used to determine the likelihood of response of the subject or the risk of development of a toxicity, after administration of a cell therapy.

In some embodiments, the methods also involve selecting subjects for treatment with an a cell therapy, such as a particular dose of cell therapy, including administration of a particular dose of cell therapy such as those described herein, e.g., in Section I.A and I.B, based on the assessment of the presence or absence of the biomarker and/or comparison of the biomarkers to a reference value or threshold level of the biomarker. In some embodiments, the methods also involve selecting subjects for treatment with an additional agent, such as an agent or other treatment capable of treating, preventing, delaying, reducing or attenuating the development or risk of development of a toxicity, based on the assessment of the presence or absence of the biomarker and/or comparison of the biomarkers to a reference value or threshold level of the biomarker.

In some embodiments, the parameter is or includes attributes, factors, characteristic of the patient and/or the disease or condition. In some embodiments, the parameter is a parameter related to tumor burden, e.g., a measurement of tumor burden. In some aspects, the methods also involve further monitoring the subject for possible symptoms of toxicity based on the risk of toxicity determined by assessment of the presence or absence of the biomarker and/or comparison of the biomarkers to a reference value or threshold level of the biomarker. In some aspects, a biological sample, e.g., blood sample or tissue sample from the subject, can be obtained for detecting the presence or absence of a biomarker (e.g. analyte), such as for detecting or measuring a parameter (*e.g.* concentration, amount, level or activity) of the biomarker and/or assessing the presence of a biomarker, for analysis, correlation and/or detection of particular outcomes and/or toxicities. In some embodiments, certain physiological or biological parameters associated with a biomarker, including expression of biomarkers and/or clinical and laboratory parameters, can be assessed, from a biological sample, e.g., blood, from subjects before or after administration of the cell therapy. In some embodiments, expression biomarkers or analytes and/or clinical and laboratory parameters, can be assessed from a biological sample, e.g., blood, from subjects before administration of the cell therapy (pre-treatment). In some embodiments, expression biomarkers or analytes and/or clinical and laboratory parameters, can be assessed from a biological sample, e.g., blood, from subjects after administration of the cell therapy (post-treatment). In some embodiments, the concentration, amount, level or activity of biomarkers (e.g., analytes) and/or clinical and laboratory parameters can be assessed at one or more time points before or after administration of the cell therapy. In some embodiments, the peak concentration, amount, level or activity of biomarkers (e.g., analytes) and/or clinical and laboratory parameters during a specified period of time can also be determined.

In some embodiments, a biomarker or an analyte is an objectively measurable characteristic or a molecule expressed by or in a biological sample, including cells, that can be indicative of or associated with a particular state or phenomenon, such as a biological process, a therapeutic outcome, a cell phenotype or a diseased state. In some aspects, a biomarker or an analyte or parameters associated with a biomarker or an analyte can be measured or detected. For example, the presence or absence of expression of a biomarker or analyte, can be detected. In some aspects, the parameters such as concentration, amount, level or activity of the biomarker or analyte can be measured or detected. In some embodiments, the presence, absence, expression, concentration, amount, level and/or activity of the biomarker can be associated with, correlated to, indicative of and/or predictive of particular states, such as particular therapeutic outcomes or state of the subject. In some aspects, the presence, absence, expression, concentration, amount, level and/or activity of the biomarker or analyte, such as any described herein, can be used to assess the likelihood of a particular outcome or state, such as a particular therapeutic outcome, including response outcome or toxicity outcome. In some embodiments, exemplary biomarkers include cytokines, cell surface molecules, chemokines, receptors, soluble receptors, soluble serum proteins and/or degradation products. In some embodiments, biomarkers or analytes can also include particular attributes, factors, characteristic of the patient and/or the disease or condition or factors indicative of the state of the patient and/or the disease or condition of the patient (including disease burden), and/or clinical or laboratory parameters.

In some embodiments, the biomarkers can be used singly or in combination with other biomarkers, such as in a panel of biomarkers. In some embodiments, expression of particular biomarkers can be correlated to particular outcomes or toxicities, e.g., development of neurotoxicity. In some embodiments, biomarkers (e.g. analytes), including parameters thereof, that can be assessed include Lactate dehydrogenase (LDH), ferritin, C-reactive protein (CRP), Interleukin-6 (IL-6), IL-7, IL-8, IL-10, IL-15, IL-16, tumor necrosis factor alpha (TNF-α), interferon alpha 2 (IFN-α2), monocyte chemoattractant protein-1 (MCP-1), macrophage inflammatory protein 1 alpha (MIP-1α), macrophage inflammatory protein 1 beta (MIP-1β), Eotaxin, Granulocyte-colony stimulating factor (G-CSF), IL-1 receptor alpha (IL-1Rα), IL-1β, IFN-γ-Inducible Protein 10 (IP-10), perforin, and D-dimer (fibrin degradation product). In some embodiments, the biomarkers (e.g. analytes), including parameters thereof, include LDH, ferritin, CRP, IL-6, IL-8, IL-10, TNF-α, IFN-α2, MCP-1 and MIP-1β. In some embodiments, the biomarkers (e.g. analytes), including parameters thereof, include ferritin, CRP, D-dimer, IL-6, IL-15, TNF-α and MIP-1α. In some embodiments, the biomarkers (e.g. analytes), including parameters thereof, include ferritin, CRP, IL-10, IL-15, IL-16, TNF-α, or MIP-1β.

In some embodiments, the methods include detecting the presence or absence of one or more biomarkers, such as a parameter (*e.g.* concentration, amount, level or activity) associated with one or more biomarkers, in which the one or more biomarkers are selected from among Lactate dehydrogenase (LDH), ferritin, C-reactive protein (CRP), Interleukin-6 (IL-6), IL-7, IL-8, IL-10, IL-15, IL-16, tumor necrosis factor alpha (TNF-α), interferon alpha 2 (IFN-α2), monocyte chemoattractant protein-1 (MCP-1), macrophage inflammatory protein 1 alpha (MIP-1α), macrophage inflammatory protein 1 beta (MIP-1β), Eotaxin, Granulocyte-colony stimulating factor (G-CSF), IL-1 receptor alpha (IL-1Rα), IL-1β, IFN-γ-Inducible Protein 10 (IP-10), perforin, and D-dimer (fibrin degradation product).

In some embodiments, the parameter that is assessed is or includes attributes, factors, characteristic of the patient and/or the disease or condition, and/or expression of biomarkers. In some embodiments, the parameter is or includes one or more factors indicative of the state of the patient and/or the disease or condition of the patient. In some embodiments, the parameter is indicative of tumor burden. In some embodiments, the factor indicative of tumor burden is a volumetric measure of tumor(s). In some embodiments, the volumetric measure is a measure of the lesion(s), such as the tumor size, tumor diameter, tumor volume, tumor mass, tumor load or bulk, tumor-related edema, tumor-related necrosis, and/or number or extent of metastases. In some embodiments, the volumetric measure of tumor is a bidimensional measure. For example, in some embodiments, the area of lesion(s) are calculated as the product of the longest diameter and the longest perpendicular diameter of all measurable tumors. In some cases, the volumetric measure of tumor is a unidimensional measure. In some cases, the size of measurable lesions is assessed as the longest diameter. In some embodiments, the sum of the products of diameters (SPD), longest tumor diameters (LD), sum of longest tumor diameters (SLD), necrosis, tumor volume, necrosis volume, necrosis-tumor ratio (NTR), peritumoral edema (PTE), and edema-tumor ratio (ETR) is measured.

Exemplary methods for measuring and assessing tumor burden include those described in, e.g., Carceller et al., Pediatr Blood Cancer. (2016) 63(8):1400-1406 and Eisenhauer et al., Eur J Cancer. (2009) 45(2):228-247. In some embodiments, the volumetric is a sum of the products of diameters (SPD) measured by determining the sum of the products of the largest perpendicular diameters of all measurable tumors. In some aspects, the tumor or lesion are measured in one dimension with the longest diameter (LD) and/or by determining the sum of longest tumor diameters (SLD) of all measurable lesions. In some embodiments, the volumetric measure of tumor is a volumetric quantification of tumor necrosis, such as necrosis volume and/or necrosis-tumor ratio (NTR), see Monsky et al., Anticancer Res. (2012) 32(11): 4951-4961. In some aspects, the volumetric measure of tumor is a volumetric quantification of tumor-related edema, such as peritumoral edema (PTE) and/or edema-tumor ratio (ETR). In some embodiments, measuring can be performed using imaging techniques such as computed tomography (CT), positron emission tomography (PET), and/or magnetic resonance imaging (MRI) of the subject.

In some embodiments, the volumetric measure of tumor is determined at a screening session, such as a routine assessment or blood draw to confirm and/or identify the condition or disease in the subject.

In some embodiments, the presence or absence and/or a parameter of one or more biomarkers (e.g. analytes) is assessed from a biological sample. In some aspects, the biological sample is a bodily fluid or a tissue. In some such embodiments, the biological sample, *e.g.,* bodily fluid, is or contains whole blood, serum or plasma.

In some embodiments, the presence or absence and/or a parameter of one or more biomarkers (e.g. analytes) is assessed prior to administration of the cell therapy (e.g., pre-infusion), e.g., obtained up to 2 days, up to 7 days, up to 14 days, up to 21 days, up to 28 days, up to 35 days or up to 40 days prior to initiation of the administration of the engineered cells. In some embodiments, the biological sample is obtained from the subject prior to administration of the cell therapy (e.g., pre-infusion), e.g., obtained up to 2 days, up to 7 days, up to 14 days, up to 21 days, up to 28 days, up to 35 days or up to 40 days prior to initiation of the administration of the engineered cells.

In some embodiments, the biological sample is an apheresis or leukaphresis sample. In some embodiments, the or absence and/or a parameter of one or more biomarkers (e.g. analytes) is assessed or the biological sample is obtained after administration of the cell therapy. In some embodiments, the reagents can be used prior to the administration of the cell therapy or after the administration of cell therapy, for diagnostic purposes, to identify subjects and/or to assess treatment outcomes and/or toxicities.

In some embodiments, measuring the value of the one or more biomarkers comprises performing an *in vitro* assay. In some aspects, the *in vitro* assay is an immunoassay, an aptamer-based assay, a histological or cytological assay, or an mRNA expression level assay. In some embodiments, the values of the one or more biomarkers are measured by an enzyme-linked immunosorbent assay (ELISA), immunoblotting, immunoprecipitation, radioimmunoassay (RIA), immunostaining, a flow cytometry assay, surface plasmon resonance (SPR), a chemiluminescence assay, a lateral flow immunoassay, an inhibition assay or an avidity assay. In some cases, the value of at least one of the one or more biomarkers is determined using a binding reagent that specifically binds to at least one biomarker. In some aspects, the binding reagent is an antibody or antigen-binding fragment thereof, an aptamer or a nucleic acid probe.

In some embodiments, measuring the value of the one or more biomarkers (*e.g.,* analytes) comprises contacting a reagent capable of directly or indirectly detecting the analyte with the biological sample and determining the presence or absence, level, amount or concentration of the analyte in the biological sample. In some embodiments, the one or more biomarker (e.g. analyte) is lactate dehydrogenase (LDH), ferritin, CRP, IL-6, IL-7, IL-8, IL-10, IL-15, IL-16, TNF-alpha, IFN-gamma, MCP-1, MIP-1beta, eotaxin, G-CSF, IL-1Ralpha, IL-1Rbeta, IP-10, perforin, and D-dimer (fibrin degradation product). In some embodiments, the one or more biomarker (e.g. analyte) is LDH, ferritin, CRP, IL-6, IL-8, IL-10, TNF-alpha, IFN-alpha2, MCP-1 and MIP-1beta. In some embodiments, the one or more biomarker (e.g. analyte) is or includes LDH.

In some aspects, the reagent is a binding molecule that specifically binds to the analyte. For example, in some embodiments, the reagent is an antibody or an antigen-binding fragment thereof. In some embodiments, the reagent is or includes a substrate or binding partner of the analyte.

In some embodiments, the presence, absence or parameter (e.g. level, amount, concentration and/or other measure) of LDH is detected or determined in a sample. Various methods of detecting or determining LDH are known. For example, an assay which measures LDH conversion of lactate to pyruvate through NAD⁺ reduction to NADH can be used to detect LDH in the sample. In some embodiments, the sample is contacted with lactate in the presence of coenzyme NAD which, as a measure of LDH in the sample, results in NADH that is then oxidized in the presence of an electron transfer agent. In some embodiments, the NADH interacts with a probe or dye precursor that is detectable by measuring absorption in a visible light range. In some examples, diaphorase uses the NADH to reduce tetrazolium salt (INT) to a red formazan product and the product is measured. Therefore, in some embodiments, the amount of colored product formed is directly proportional to the LDH activity in the sample.

In some embodiments, the methods involve comparing, individually, the level, amount or concentration of the analyte in the sample to a threshold level, thereby determining a risk of developing a toxicity after administration of the cell therapy, or thereby determining a likelihood that a subject will achieve a response to the cell therapy. In some aspects, the exemplary threshold levels can be determined based on the mean or median values and values within a range or standard deviation of the mean or median values of the level, amount or concentration of the analyte in a biological sample obtained from a group of subjects prior to receiving a cell therapy, wherein each of the subjects of the group went on to exhibit a particular outcome, such as a particular therapeutic outcome, including either exhibiting a response or not exhibiting a response; or either developing a toxicity or not developing a toxicity. In some embodiments, particular aspects of determining threshold values include those described below in Sections I.E.1 and I.E.2.

### 1. Exemplary Biomarkers, Analytes or Parameters Associated with Response Outcomes

In some embodiments, the analyte or biomarker is associated with, correlated to, indicative of and/or predictive of a particular outcome, such as a particular response outcome, such as an objective response (OR) a complete response (CR) or a partial response (PR), or durable response, such as an OR a CR or a PR that is durable at 3, 6, 9 months or more. In some embodiments, lower or reduced levels or increased levels of one or more of such biomarkers (e.g., analytes), such as compared to a reference value or threshold level, can be associated with the a response, such as an OR, a CR or a PR, or any response outcomes described herein, e.g., in Section I.C, optionally a durable response, such as a response that is durable for at least 3 months, 6 months or more.

In some embodiments, the analyte or biomarker is associated with, correlated to, indicative of and/or predictive of a particular outcome, such as a particular response or durable response outcome, in a subject that has been administered a cell therapy, such as with a composition containing genetically engineered cells. In some embodiments, the presence, expression, level, amount or concentration of one or more analyte in a biological sample obtained from a subject prior to the administration of cell therapy, can be associated with, correlated to, indicative of and/or predictive of a particular outcome, such as a particular response or durable response outcome. In some embodiments, presence, expression, level, amount or concentration of particular biomarkers can be correlated to a particular response or durable response outcome. In some embodiments, the response outcome can be any response outcomes described herein, e.g., in Section I.C.

In some embodiments, the methods include comparing, individually, the level, amount or concentration of the analyte in the sample to a threshold level, thereby determining a likelihood that a subject will achieve a response to the cell therapy. In some embodiments, the methods include selecting a subject who is likely to respond to treatment based on the results of determining a likelihood that a subject will achieve a response to the cell therapy by comparing, individually, the level, amount or concentration of the analyte in the sample to a threshold level. In some embodiments, the methods also include administering the cell therapy to the subject selected for treatment. In some embodiments, if the subject is determined as not likely to achieve a response or a durable response, further comprising administering an additional therapeutic agent to the subject.

In some embodiments, the biomarkers (e.g., analytes) include those associated with a response outcome, and/or a durable response. In some embodiments, the biomarkers (e.g. analytes), including parameters thereof, include LDH, ferritin, CRP, D-dimer, Serum Amyloid A1 (SAA-1), IL-6, IL-10, IL-15, IL-16, TNF-α, IFN-γ, MIP-1α and C-X-C motif chemokine 10 (CXCL10).

In some aspects, exemplary analytes or biomarkers that can be assessed or analyzed with respect to assessment of likelihood of response after administration of a cell therapy include one or more analyte selected from ferritin, LDH, CXCL10, G-CSF, and IL-10. In some embodiments, for any of the foregoing analytes or biomarkers, the subject is likely to achieve a response if the level, amount or concentration of the one or more of the analyte is below a threshold level and the subject is not likely to achieve a response if the level, amount or concentration of the one or more of the analyte is above a threshold level. In some embodiments, the response is or comprises objective response. In some embodiments, the objective response is or comprises a complete response (CR) or a partial response (PR). In some aspects, reduced levels of ferritin, LDH, CXCL10, G-CSF, and IL-10, in a biological sample from a subject obtained prior to administration of a cell therapy (pre-treatment), can be associated with achieving objective response, including a complete response (CR) or a partial response (PR).

In some embodiments, the threshold level is within 25%, within 20%, within 15%, within 10% or within 5% and/or is within a standard deviation below the median or mean level, amount or concentration of ferritin, LDH, CXCL10, G-CSF, or IL-10 in a biological sample obtained from a group of subjects prior to receiving a cell therapy, wherein each of the subjects of the group went on to achieve a response after administration of a recombinant-receptor-expressing therapeutic cell composition for treating the same disease or condition. In some embodiments, the threshold level is within 25%, within 20%, within 15%, within 10% or within 5% and/or is within a standard deviation above the median or mean level, amount or concentration of ferritin, LDH, CXCL10, G-CSF, or IL-10 in a biological sample obtained from a group of subjects prior to receiving a cell therapy, wherein each of the subjects of the group went on to exhibit stable disease (SD) and/or progressive disease (PD) after administration of a recombinant-receptor-expressing therapeutic cell composition for treating the same disease or condition.

In some aspects, exemplary analytes or biomarkers that can be assessed or analyzed with respect to assessment of likelihood of durable response after administration of a cell therapy include one or more analyte selected from LDH, ferritin, CRP, D-dimer, SAA-1, IL-6, IL-10, IL-15, IL-16, TNF-α, IFN-γ, MIP-1α, CXCL-10, IL-8, MCP-1 and MIP-1β. In some embodiments, for any of the foregoing analytes or biomarkers, the subject is likely to achieve a durable response if the level, amount or concentration of the one or more of the analyte is below a threshold level and the subject is not likely to achieve a durable response if the level, amount or concentration of the one or more of the analyte is above a threshold level. In some embodiments, the durable response is or comprises a complete response (CR) or a partial response (PR) that is durable for at or greater than 3 months, 4 months, 5 months, or 6 months. In some embodiments, the durable response is or comprises a CR or a PR that is durable for at least 3 months. In some aspects, reduced levels of LDH, ferritin, CRP, D-dimer, SAA-1, IL-6, IL-10, IL-15, IL-16, TNF-α, IFN-γ, MIP-1α, CXCL-10, IL-8, MCP-1 and MIP-1β, in a biological sample from a subject obtained prior to administration of a cell therapy (pre-treatment), can be associated with achieving durable response, such as a CR or a PR that is durable for at least 3 months.

In some embodiments, the threshold level is within 25%, within 20%, within 15%, within 10% or within 5% and/or is within a standard deviation below the median or mean level, amount or concentration of LDH, ferritin, CRP, D-dimer, SAA-1, IL-6, IL-10, IL-15, IL-16, TNF-α, IFN-γ, MIP-1α, CXCL-10, IL-8, MCP-1 or MIP-1β in a biological sample obtained from a group of subjects prior to receiving a cell therapy, wherein each of the subjects of the group went on to achieve a durable response after administration of a recombinant-receptor-expressing therapeutic cell composition for treating the same disease or condition.

In some embodiments, the threshold level is within 25%, within 20%, within 15%, within 10% or within 5% and/or is within a standard deviation above the median or mean level, amount or concentration of LDH, ferritin, CRP, D-dimer, SAA-1, IL-6, IL-10, IL-15, IL-16, TNF-α, IFN-γ, MIP-1α, CXCL-10, IL-8, MCP-1 or MIP-1β in a biological sample obtained from a group of subjects prior to receiving a cell therapy, wherein each of the subjects of the group did not achieve a durable response after administration of a recombinant-receptor-expressing therapeutic cell composition for treating the same disease or condition.

In some embodiments, the response is durable response, such as a CR or a PR that is durable for at least 3 months.

In some embodiments, the threshold level for LDH is at or at about or below or below about 600 U/L, 500 U/L, 400 U/L, 300 U/L or 200 U/L.

In some embodiments, exemplary threshold level for ferritin is at or at about or below or below about 1000 µg/L, 900 µg/L, 800 µg/L, 700 µg/L, 600 µg/L, 500 µg/L, 400 µg/L, 300 µg/L or 200 µg/L.

In some embodiments, exemplary threshold level for CRP is at or at about or below or below about 20 mg/L, 19 mg/L, 18 mg/L, 17 mg/L, 16 mg/L, 15 mg/L, 14 mg/L, 13 mg/L, 12 mg/L, 11 mg/L, 10 mg/L, 9 mg/L, 8 mg/L, 7 mg/L, 6 mg/L or 5 mg/L.

In some embodiments, exemplary threshold level for D-dimer is at or at about or below or below about 1000 µg/L, 900 µg/L, 800 µg/L, 700 µg/L, 600 µg/L, 500 µg/L, 400 µg/L, 300 µg/L or 200 µg/L.

In some embodiments, exemplary threshold level for SAA-1 is at or at about or below or below about 100 mg/L, 90 mg/L, 80 mg/L, 70 mg/L, 60 mg/L, 50 mg/L, 40 mg/L, 30 mg/L or 20 mg/L.

In some embodiments, exemplary threshold level for IL-6 is at or at about or below or below about 6 pg/mL. 5 pg/mL, 4 pg/mL, 3 pg/mL or 2 pg/mL.

In some embodiments, exemplary threshold level for IL-10 is at or at about or below or below about 2 pg/mL, 1 pg/mL, 0.9 pg/mL, 0.8 pg/mL, 0.7 pg/mL. 0.6 pg/mL or 0.5 pg/mL.

In some embodiments, exemplary threshold level for IL-15 is at or at about or below or below about 7 pg/mL, 6 pg/mL, 5 pg/mL, 4 pg/mL or 3 pg/mL.

In some embodiments, exemplary threshold level for IL-16 is at or at about or below or below about 1000 pg/mL, 900 pg/mL, 800 pg/mL, 700 pg/mL or 600 pg/mL.

In some embodiments, exemplary threshold level for TNF-α is at or at about or below or below about 10 pg/mL, 9 pg/mL, 8 pg/mL, 7 pg/mL or 6 pg/mL.

In some embodiments, exemplary threshold level for IFN-γ is at or at about or below or below about 30 pg/mL, 20 pg/mL, 10 pg/mL, 9 pg/mL, 8 pg/mL, or 7 pg/mL;

In some embodiments, exemplary threshold level for MIP-1α is at or at about or below or below about 40 pg/mL, 30 pg/mL or 20 pg/mL; and/or

In some embodiments, exemplary threshold level for CXCL-10 is at or at about or below or below about 1500 pg/mL, 1000 pg/mL, 900 pg/mL, 800 pg/mL, 700 pg/mL, 600 pg/mL, or 500 pg/mL.

In some aspects, exemplary analytes or biomarkers that can be assessed or analyzed with respect to assessment of likelihood of durable response after administration of a cell therapy include one or more analyte selected from ferritin, CRP, LDH, CXCL10, IL-8, IL-10, IL-15, MCP-1, MIP-1β and TNF-α. In some embodiments, for any of the foregoing analytes or biomarkers, the subject is likely to achieve a durable response if the level, amount or concentration of the one or more of the analyte is below a threshold level and the subject is not likely to achieve a durable response if the level, amount or concentration of the one or more of the analyte is above a threshold level. In some embodiments, the durable response is or comprises a complete response (CR) or a partial response (PR) that is durable for at or greater than 3 months, 4 months, 5 months, or 6 months. In some embodiments, the durable response is or comprises a CR or a PR that is durable for at least 3 months. In some aspects, reduced levels of ferritin, CRP, LDH, CXCL10, IL-8, IL-10, IL-15, MCP-1, MIP-1β and TNF-α, in a biological sample from a subject obtained prior to administration of a cell therapy (pre-treatment), can be associated with achieving durable response, such as a CR or a PR that is durable for at least 3 months.

In some embodiments, the threshold level is within 25%, within 20%, within 15%, within 10% or within 5% and/or is within a standard deviation below the median or mean level, amount or concentration of ferritin, CRP, LDH, CXCL10, IL-8, IL-10, IL-15, MCP-1, MIP-1β or TNF-α in a biological sample obtained from a group of subjects prior to receiving a cell therapy, wherein each of the subjects of the group went on to achieve a durable response after administration of a recombinant-receptor-expressing therapeutic cell composition for treating the same disease or condition.

In some embodiments, the threshold level is within 25%, within 20%, within 15%, within 10% or within 5% and/or is within a standard deviation above the median or mean level, amount or concentration of ferritin, CRP, LDH, CXCL10, IL-8, IL-10, IL-15, MCP-1, MIP-1β or TNF-α in a biological sample obtained from a group of subjects prior to receiving a cell therapy, wherein each of the subjects of the group did not achieve a durable response after administration of a recombinant-receptor-expressing therapeutic cell composition for treating the same disease or condition.

In some aspects, exemplary analytes or biomarkers that can be assessed or analyzed with respect to assessment of likelihood of durable response after administration of a cell therapy include one or more analyte selected from hemoglobin and albumin. In some embodiments, for any of the foregoing analytes or biomarkers, the subject is likely to achieve a durable response if the level, amount or concentration of the one or more of the analyte is above a threshold level and the subject is not likely to achieve a durable response if the level, amount or concentration of the one or more of the analyte is below a threshold level. In some embodiments, the durable response is or comprises a complete response (CR) or a partial response (PR) that is durable for at or greater than 3 months, 4 months, 5 months, or 6 months. In some embodiments, the durable response is or comprises a CR or a PR that is durable for at least 3 months. In some aspects, elevated levels of hemoglobin and albumin, in a biological sample from a subject obtained prior to administration of a cell therapy (pre-treatment), can be associated with achieving durable response, such as a CR or a PR that is durable for at least 3 months.

In some embodiments, the threshold level is within 25%, within 20%, within 15%, within 10% or within 5% and/or is within a standard deviation above the median or mean level, amount or concentration of hemoglobin or albumin in a biological sample obtained from a group of subjects prior to receiving a cell therapy, wherein each of the subjects of the group went on to achieve a durable response after administration of a recombinant-receptor-expressing therapeutic cell composition for treating the same disease or condition.

In some embodiments, the threshold level is within 25%, within 20%, within 15%, within 10% or within 5% and/or is within a standard deviation below the median or mean level, amount or concentration of hemoglobin or albumin in a biological sample obtained from a group of subjects prior to receiving a cell therapy, wherein each of the subjects of the group did not achieve a durable response after administration of a recombinant-receptor-expressing therapeutic cell composition for treating the same disease or condition.

### 2. Exemplary Biomarkers, Analytes or Parameters Associated with Toxicity Outcomes

In some embodiments, the analyte or biomarker is associated with, correlated to, indicative of and/or predictive of a particular outcome, such as development of a toxicity, in a subject that has been administered a cell therapy, such as with a composition containing genetically engineered cells. In some embodiments, the presence, expression, level, amount or concentration of one or more analyte in a biological sample obtained from a subject prior to the administration of cell therapy, can be associated with, correlated to, indicative of and/or predictive of a particular outcome, such as development of a toxicity, such as any toxicity outcomes described herein, e.g., in Section I.D. In some embodiments, presence, expression, level, amount or concentration of particular biomarkers can be correlated to particular outcomes or toxicities, e.g., development of NT or CRS. In some embodiments, the toxicity is a toxicity potentially associated with cell therapy, such as any described herein, for example, in Section I.D. In some embodiments, the toxicity is neurotoxicity (NT) or cytokine release syndrome (CRS). In some embodiments, the toxicity is a severe NT or severe CRS. In some embodiments, the toxicity is a grade 2 or higher NT or grade 2 or higher CRS. In some embodiments, the toxicity is a grade 3 or higher NT or a grade 3 or higher CRS.

In some embodiments, the methods include comparing, individually, the level, amount or concentration of the analyte in the sample to a threshold level, thereby determining a risk of developing a toxicity after administration of the cell therapy. In some embodiments, the methods include identifying a subject who has a risk of developing a toxicity after administration of a cell therapy by comparing, individually, the level, amount or concentration of the analyte in the sample to a threshold level. In some embodiments, the methods also include following or based on the results of the assessment, administering to the subject the cell therapy, and, optionally, an agent or other treatment capable of treating, preventing, delaying, reducing or attenuating the development or risk of development of a toxicity. In some embodiments, the methods also involve monitoring the subject for symptoms of toxicity if the subject is administered a cell therapy and is identified as having a risk of developing a toxicity.

In some embodiments, if the subject is identified as having a risk of developing a toxicity, one or more of the following steps can be performed can be administered to the subject: (a) (1) an agent or other treatment capable of treating, preventing, delaying, reducing or attenuating the development or risk of development of a toxicity and (2) the cell therapy, wherein administration of the agent is to be administered (i) prior to, (ii) within one, two, or three days of, (iii) concurrently with and/or (iv) at first fever following, the initiation of administration of the cell therapy to the subject; and/or (b) administering to the subject a cell therapy at a reduced dose or at a dose that is not associated with risk of developing toxicity or severe toxicity, or is not associated with a risk of developing a toxicity or severe toxicity in a majority of subjects, and/or a majority of subjects having a disease or condition that the subject has or is suspected of having, following administration of the cell therapy; and/or (c) administering to the subject a cell therapy in an inpatient setting and/or with admission to the hospital for one or more days, optionally wherein the cell therapy is otherwise to be administered to subjects on an outpatient basis or without admission to the hospital for one or more days.

In some embodiments, biomarkers or analytes, including parameters thereof, that can be assessed include Lactate dehydrogenase (LDH), ferritin, C-reactive protein (CRP), Interleukin-6 (IL-6), IL-7, IL-8, IL-10, IL-15, IL-16, tumor necrosis factor alpha (TNF-α), interferon alpha 2 (IFN-α2), monocyte chemoattractant protein-1 (MCP-1), macrophage inflammatory protein 1 alpha (MIP-1α), macrophage inflammatory protein 1 beta (MIP-1β), Eotaxin, Granulocyte-colony stimulating factor (G-CSF), IL-1 receptor alpha (IL-1Rα), IL-1β, IFN-γ-Inducible Protein 10 (IP-10), perforin, and D-dimer (fibrin degradation product). In some embodiments, the biomarkers (e.g. analytes), including parameters thereof, include LDH, ferritin, CRP, IL-6, IL-8, IL-10, TNF-α, IFN-α2, MCP-1 and MIP-1β. In some embodiments, the biomarkers (e.g. analytes), including parameters thereof, include ferritin, CRP, D-dimer, IL-6, IL-15, TNF-α and MIP-1α. In some embodiments, the biomarkers (e.g. analytes), including parameters thereof, include ferritin, CRP, IL-10, IL-15, IL-16, TNF-α, or MIP-1β. In some embodiments, elevated levels or increased levels of one or more of such biomarkers (e.g., biomarkers), such as compared to a reference value or threshold level, can be associated with the development of neurotoxcity, e.g. severe neurotoxicity or grade 3 or higher or grade 4 or 5 neurotoxicity. In some embodiments, elevated levels or increased levels of one or more of such biomarkers (e.g., analytes), such as compared to a reference value or threshold level, can be associated with the development of neurotoxcity, e.g. severe neurotoxicity or grade 3 or higher or grade 4 or 5 neurotoxicity.

In some aspects, exemplary analytes or biomarkers that can be assessed or analyzed with respect to assessment of the risk of developing a toxicity after administration of a cell therapy include one or more analyte selected from LDH, Ferritin, C-reactive protein (CRP), IL-6, IL-8, IL-10, TNF-α, IFN-α2, MCP-1 and MIP-1β. In some embodiments, for any of the foregoing analytes or biomarkers, the subject has a risk of developing a toxicity if the level, amount or concentration of the one or more of the analyte is above a threshold level and the subject has a low risk of developing a toxicity if the level, amount or concentration of the one or more of the analyte is below a threshold level. In some embodiments, the toxicity is neurotoxicity. In some aspects, elevated levels of LDH, Ferritin, C-reactive protein (CRP), IL-6, IL-8, IL-10, TNF-α, IFN-α2, MCP-1 and MIP-1β, in a biological sample from a subject obtained prior to administration of a cell therapy (pre-treatment), can be associated with a higher risk of developing a neurotoxicity.

In some embodiments, the threshold level is within 25%, within 20%, within 15%, within 30% or within 5% and/or is within a standard deviation above the median or mean level, amount or concentration of LDH, Ferritin, C-reactive protein (CRP), IL-6, IL-8, IL-10, TNF-α, IFN-α2, MCP-1 or MIP-1β in a biological sample obtained from a group of subjects prior to receiving a cell therapy, wherein each of the subjects of the group went on not develop any toxicity after receiving a recombinant-receptor-expressing therapeutic cell composition for treating the same disease or condition.

In some embodiments, the threshold level is within 25%, within 20%, within 15%, within 30% or within 5% and/or is within a standard deviation below the median or mean level, amount or concentration of LDH, Ferritin, C-reactive protein (CRP), IL-6, IL-8, IL-10, TNF-α, IFN-α2, MCP-1 or MIP-1β in a biological sample obtained from a group of subjects prior to receiving a cell therapy, wherein each of the subjects of the group went on to develop a toxicity after receiving a recombinant-receptor-expressing therapeutic cell composition for treating the same disease or condition.

In some embodiments, the toxicity is NT. In some embodiments, the toxicity is a grade 2 or higher NT. In some embodiments, the toxicity is a grade 3 or higher NT. In some embodiment, the toxicity is a severe NT. In some embodiments, the toxicity is CRS. In some embodiments, the toxicity is a grade 2 or higher CRS. In some embodiments, the toxicity is a grade 3 or higher CRS. In some embodiment, the toxicity is a severe CRS.

In some embodiments, exemplary threshold level for LDH is at or at about or above or above about 300 U/L, 400 U/L, 500 U/L, 600 U/L or 700 U/L.

In some embodiments, exemplary threshold level for Ferritin is at or at about or above or above about 500 ng/mL, 600 ng/mL, 700 ng/mL, 800 ng/mL, 900 ng/mL, 1000 ng/mL, or 1500 ng/mL.

In some embodiments, exemplary threshold level for CRP is at or at about or above or above about 20 mg/L, 30 mg/L, 40 mg/L, 50 mg/L, 60 mg/L, 70 mg/L or 80 mg/L.

In some embodiments, exemplary threshold level for IL-6 is at or at about or above or above about 5 pg/mL, 6 pg/mL, 7 pg/mL, 8 pg/mL, 9 pg/mL, 10 pg/mL, 20 pg/mL, or 30 pg/mL.

In some embodiments, exemplary threshold level for IL-8 is at or at about or above or above about 8 pg/mL, 9 pg/mL, 10 pg/mL, 20 pg/mL or 30 pg/mL.

In some embodiments, exemplary threshold level for IL-10 is at or at about or above or above about 20 pg/mL, 30 pg/mL, 40 pg/mL, 50 pg/mL, 60 pg/mL or 70 pg/mL.

In some embodiments, exemplary threshold level for TNF-α is at or at about or above or above about 20 pg/mL or 30 pg/mL.

In some embodiments, exemplary threshold level for IFN-α2 is at or at about or above or above about 40 pg/mL, 50 pg/mL, 60 pg/mL, 70 pg/mL or 80 pg/mL.

In some embodiments, exemplary threshold level for MCP-1; and/or is at or at about or above or above about 200 pg/mL or 300 pg/mL.

In some embodiments, exemplary threshold level for MIP-1β is at or at about or above or above about 40 pg/mL, 50 pg/mL, 60 pg/mL, 70 pg/mL or 80 pg/mL.

In some embodiments, exemplary threshold level for SPD is at or about or above at or about 30 cm², 40 cm², 50 cm², 60 cm² or 70 cm².

In some embodiments, exemplary threshold level for LDH is at or about or above at or about 300 units per liter, 400 units per liter, 500 units per liter or 600 units per liter.

In some embodiments, exemplary threshold level for ferritin is at or about or above at or about 1000 ng/mL, 2000 ng/mL, 3000 ng/mL, 4000 ng/mL, 5000 ng/mL, 6000 ng/mL, 7000 ng/mL or 8000 ng/mL.

In some embodiments, exemplary threshold level for CRP is at or about or above at or about 5 mg/L, 10 mg/L, 15 mg/L, 20 mg/L, 25 mg/L, 30 mg/L, 40 mg/L or 50 mg/L.

In some embodiments, the parameter is SPD, and the threshold level is 50 cm². In some embodiments, the parameter is LDH, and the threshold level is 500 units per liter. In some embodiments, the one or more parameter is SPD and LDH, and the threshold level for SPD is 50 cm² and the threshold level for LDH is 500 units per liter.

In some embodiments, the parameter is ferritin, and the threshold level is 5000 ng/mL. In some embodiments, the parameter is CRP, and the threshold level is 10 mg/L. In some embodiments, the one or more parameter is ferritin and CRP, and the threshold level for ferritin is 5000 ng/mL and the threshold level for CRP is 10 mg/L.

In some aspects, exemplary analytes or biomarkers that can be assessed or analyzed with respect to assessment of the risk of developing a toxicity after administration of a cell therapy include one or more analyte selected from IL-8, IL-10 and CXCL10. In some embodiments, for any of the foregoing analytes or biomarkers, the subject has a risk of developing a toxicity if the level, amount or concentration of the one or more of the analyte is above a threshold level and the subject has a low risk of developing a toxicity if the level, amount or concentration of the one or more of the analyte is below a threshold level. In some embodiments, the toxicity is NT. In some embodiments, the toxicity is a severe NT or a grade 3 or higher NT. In some aspects, elevated levels of IL-8, IL-10 and CXCL10, in a biological sample from a subject obtained prior to administration of a cell therapy (pre-treatment), can be associated with a higher risk of developing a NT, or a severe NT or a grade 3 or higher NT.

In some embodiments, the threshold level is within 25%, within 20%, within 15%, within 30% or within 5% and/or is within a standard deviation above the median or mean level, amount or concentration of IL-8, IL-10 or CXCL10 in a biological sample obtained from a group of subjects prior to receiving a cell therapy, wherein each of the subjects of the group went on not develop any toxicity after receiving a recombinant-receptor-expressing therapeutic cell composition for treating the same disease or condition.

In some embodiments, the threshold level is within 25%, within 20%, within 15%, within 30% or within 5% and/or is within a standard deviation below the median or mean level, amount or concentration of IL-8, IL-10 or CXCL10 in a biological sample obtained from a group of subjects prior to receiving a cell therapy, wherein each of the subjects of the group went on to develop a toxicity after receiving a recombinant-receptor-expressing therapeutic cell composition for treating the same disease or condition.

In some aspects, exemplary analytes or biomarkers or a volumetric measure of tumor burden that can be assessed or analyzed with respect to assessment of the risk of developing a toxicity after administration of a cell therapy include one or more analyte or volumetric measure of tumor burden selected from a sum of the products of diameters (SPD), LDH, Ferritin, C-reactive protein (CRP), D-dimer (fibrin degradation product), IL-6, IL-10, IL-15, IL-16 TNF-α, MIP-1α and MIP-1β. In some embodiments, for any of the foregoing analytes or biomarkers or volumetric measure of tumor burden, the subject has a risk of developing a toxicity if the level, amount or concentration of the one or more of the analyte or the volumetric measure of tumor burden is above a threshold level and the subject has a low risk of developing a toxicity if the level, amount or concentration of the one or more of the analyte or the volumetric measure of tumor burden is below a threshold level. In some embodiments, the toxicity is NT. In some aspects, elevated levels or measure of a sum of the products of diameters (SPD), LDH, Ferritin, C-reactive protein (CRP), D-dimer (fibrin degradation product), IL-6, IL-10, IL-15, IL-16 TNF-α, MIP-1α and MIP-1β, in a biological sample from a subject obtained prior to administration of a cell therapy (pre-treatment), can be associated with a higher risk of developing a NT (NT) or a cytokine release syndrome (CRS).

In some embodiments, the one or more analyte or volumetric measure of tumor burden selected from LDH, SPD, IL-10, IL-15, IL-16, TNF-α and MIP-1β, and the toxicity is NT In some embodiments, the one or more analyte or volumetric measure of tumor burden selected from LDH, SPD, CRP, d-dimer, IL-6, IL-15, TNF-α and MIP-1α, and the toxicity is CRS. In some aspects, elevated levels or measure of LDH, SPD, IL-10, IL-15, IL-16, TNF-α and MIP-1β, in a biological sample from a subject obtained prior to administration of a cell therapy (pre-treatment), can be associated with a higher risk of developing a NT (NT). In some aspects, elevated levels or measure of LDH, SPD, CRP, d-dimer, IL-6, IL-15, TNF-α and MIP-1α, in a biological sample from a subject obtained prior to administration of a cell therapy (pre-treatment), can be associated with a higher risk of developing a cytokine release syndrome (CRS).

In some embodiments, the threshold level is within 25%, within 20%, within 15%, within 32% or within 5% and/or is within a standard deviation above the median or mean level, amount or concentration of LDH, Ferritin, C-reactive protein (CRP), D-dimer (fibrin degradation product), IL-6, IL-10, IL-15, IL-16 TNF-α, MIP-1α or MIP-1β, or the median or mean volumetric measure of tumor burden of a sum of the products of diameters (SPD), in a biological sample obtained from a group of subjects prior to receiving a cell therapy, wherein each of the subjects of the group went on not develop any toxicity after receiving a recombinant-receptor-expressing therapeutic cell composition for treating the same disease or condition.

In some embodiments, the threshold level is within 25%, within 20%, within 15%, within 32% or within 5% and/or is within a standard deviation below the median or mean level, amount or concentration of LDH, Ferritin, C-reactive protein (CRP), D-dimer (fibrin degradation product), IL-6, IL-10, IL-15, IL-16 TNF-α, MIP-1α or MIP-1β, or the median or mean volumetric measure of tumor burden of a sum of the products of diameters (SPD), in a biological sample obtained from a group of subjects prior to receiving a cell therapy, wherein each of the subjects of the group went on to develop a toxicity after receiving a recombinant-receptor-expressing therapeutic cell composition for treating the same disease or condition.

In some embodiments, the toxicity is NT and exemplary threshold level for LDH is at or at about or above or above about 300 U/L, 400 U/L, 500 U/L or 600 U/L.

In some embodiments, the toxicity is NT and exemplary threshold level for SPD is at or at about or above or above about 30 cm², 40 cm², 50 cm², 60 cm², 70 cm², 80 cm² or 90cm².

In some embodiments, the toxicity is NT and exemplary threshold level for IL-10 is at or at about or above or above about 0.8 pg/mL, 0.9 pg/mL, 1 pg/mL, 2 pg/mL, 3 pg/mL or 4 pg/mL.

In some embodiments, the toxicity is NT and exemplary threshold level for IL-15 is at or at about or above or above about 3 pg/mL, 4 pg/mL, 5 pg/mL, 6 pg/mL or 7 pg/mL.

In some embodiments, the toxicity is NT and exemplary threshold level for IL-16 is at or at about or above or above about 600 pg/mL, 700 pg/mL, 800 pg/mL, 900 pg/mL or 1000 pg/mL.

In some embodiments, the toxicity is NT and exemplary threshold level for TNF-α is at or at about or above or above about 6 pg/mL, 7 pg/mL, 8 pg/mL, 9 pg/mL or 10 pg/mL.

In some embodiments, the toxicity is NT and exemplary threshold level for MIP-1β is at or at about or above or above about 70 pg/mL, 80 pg/mL, 90 pg/mL or 100 pg/mL.

In some embodiments, the toxicity is CRS and exemplary threshold level for LDH is at or at about or above or above about 300 U/L, 400 U/L, 500 U/L or 600 U/L.

In some embodiments, the toxicity is CRS the and threshold level for SPD is at or at about or above or above about 20 cm², 30 cm², 40 cm² or 50 cm².

In some embodiments, the toxicity is CRS and exemplary threshold level for ferritin is at or at about or above or above about 300 ng/mL, 400 ng/mL, 500 ng/mL, 600 ng/mL, 700 ng/mL, 800 ng/mL, 900 ng/mL, or 1000 ng/mL.

In some embodiments, the toxicity is CRS and exemplary threshold level for CRP is at or at about or above or above about 20 mg/L, 30 mg/L or 40 mg/L.

In some embodiments, the toxicity is CRS and exemplary threshold level for d-dimer is at or at about or above or above about 300 pg/mL, 400 pg/mL, 500 pg/mL, 600 pg/mL, 700 pg/mL, 800 pg/mL, 900 pg/mL or 1000 pg/mL.

In some embodiments, the toxicity is CRS and exemplary threshold level for IL-6 is at or at about or above or above about 2 pg/mL, 3 pg/mL, 4 pg/mL, 5 pg/mL, 6 pg/mL, 7 pg/mL, 8 pg/mL or 9 pg/mL.

In some embodiments, the toxicity is CRS and exemplary threshold level for IL-15 is at or at about or above or above about 3 pg/mL, 4 pg/mL, 5 pg/mL, 6 pg/mL, 7 pg/mL, 8 pg/mL, 9 pg/mL or 10 pg/mL.

In some embodiments, the toxicity is CRS and exemplary threshold level for TNF-α is at or at about or above or above about 7 pg/mL, 8 pg/mL, 9 pg/mL, 10 pg/mL or 15 pg/mL.

In some embodiments, the toxicity is CRS and exemplary threshold level for MIP-1α is at or at about or above or above about 20 pg/mL, 30 pg/mL or 40 pg/mL.

In some embodiments, the biomarker is LDH and in some cases, development of toxicity, e.g., CRS or NT, is correlated with the LDH value that is above a threshold value. In some embodiments, the inflammatory marker is LDH and the threshold value is or is about 300 units per liter, is or is about 400 units per liter, is or is about 500 units per liter or is or is about 600 units per liter.

In some embodiments, the toxicity is NT or CRS and exemplary threshold level for SPD is at or about or above at or about 30 cm², 40 cm², 50 cm², 60 cm² or 70 cm².

In some embodiments, the toxicity is NT or CRS and exemplary threshold level for LDH is at or about or above at or about 300 units per liter, 400 units per liter, 500 units per liter or 600 units per liter.

In some embodiments, the toxicity is NT or CRS and exemplary threshold level for ferritin is at or about or above at or about 1000 ng/mL, 2000 ng/mL, 3000 ng/mL, 4000 ng/mL, 5000 ng/mL, 6000 ng/mL, 7000 ng/mL, or 8000 ng/mL.

In some embodiments, the toxicity is NT or CRS and exemplary threshold level for CRP is at or about or above at or about 5 mg/L, 10 mg/L, 15 mg/L, 20 mg/L, 25 mg/L, 30 mg/L, 40 mg/L or 50 mg/L.

In some embodiments, the parameter is SPD, and the threshold level is 50 cm². In some embodiments, the parameter is LDH, and the threshold level is 500 units per liter. In some embodiments, the one or more parameter is SPD and LDH, and the threshold level for SPD is 50 cm² and the threshold level for LDH is 500 units per liter.

In some embodiments, if the level, amount or concentration of the biomarker (*e.g.,* analyte) in the sample is at or above a threshold level of the analyte, an agent or other treatment capable of treating, preventing, delaying, reducing or attenuating the development or risk of development of a toxicity is administered to the subject prior to, within one, two, or three days of, concurrently with and/or at first fever following, the initiation of administration of the cell therapy to the subject. Exemplary agents or interventions for use in connection with the provided methods to treat, prevent, delay, reduce or attenuate the risk of developing toxicity are described in Section II.

In some cases, if the level, amount of concentration of the biomarker in the sample is at or above a threshold level, the cell therapy is administered to the subject at a reduced dose or at a dose that is not associated with risk of developing toxicity or severe toxicity, or is not associated with a risk of developing a toxicity or severe toxicity in a majority of subjects, and/or a majority of subjects having a disease or condition that the subject has or is suspected of having, following administration of the cell therapy. In some cases, if the level, amount of concentration of the biomarker in the sample is at or above a threshold level, the cell therapy is administered to the subject in an inpatient setting and/or with admission to the hospital for one or more days, optionally wherein the cell therapy is otherwise to be administered to subjects on an outpatient basis or without admission to the hospital for one or more days.

In some embodiments, if the level, amount or concentration of the biomarker (*e.g.,* analyte) is below a threshold level for the analyte, the cell therapy is administered to the subject, optionally at a non-reduced dose. In some cases, the cells therapy is optionally administered on an outpatient basis or without admission to the hospital for one or more days. In some embodiments, if the level, amount or concentration of the analyte, is below a threshold level, the administration of the cell therapy does not include administering, prior to or concurrently with administering the cell therapy and/or prior to the development of a sign of symptom of a toxicity other than fever, an agent or treatment capable of treating, preventing, delaying, or attenuating the development of the toxicity; and/or the administration of the cell therapy is to be or may be administered to the subject on an outpatient setting and/or without admission of the subject to the hospital overnight or for one or more consecutive days and/or is without admission of the subject to the hospital for one or more days.

In some aspects of the provided methods, a subject is determined to be at risk of developing toxicity (*e*.g. neurotoxcity, such as severe NT or grade 3 or higher neurotoxcity, e.g. grade 4 or 5 NT and/or CRS, such as severe CRS or grade 3 or higher CRS) by a comparison of the parameter (*e.g.* concentration, amount, level or activity) of the biomarker (e.g. analyte) or, individually, each of the biomarkers (e.g. analytes) to a reference value, such as threshold level, of the corresponding parameter for the biomarker or each biomarker. In some embodiments, the comparison indicates whether the subject is or is not at risk for developing toxicity, *e.g.,* NT such as severe NT or grade 3 or higher neurotoxcity, e.g. grade 4 or 5 NT and/or CRS, such as severe CRS or grade 3 or higher CRS, and/or indicates a degree of risk for developing said toxicity. In some embodiments, the reference value is one that is a threshold level or cut-off at which there is a good predictive value (*e.g.* accuracy, sensitivity and/or specificity) that such toxicity will occur or is likely to occur either alone or in combination with one or more biomarkers in the panel. In some cases, such reference value, e.g. threshold level, can be or is predetermined or known prior to performing the method, such as from a plurality of subjects previously treated with a cell therapy and assessed for the correlation of the parameter of the biomarker or, individually, each of the biomarkers in a panel to the presence of a toxic outcome (*e.g.* the presence of NT such as severe NT or grade 3 or higher neurotoxcity, e.g. grade 4 or 5 NT and/or CRS, such as severe CRS or grade 3 or higher CRS).

In some embodiments, a parameter of a biomarker (*e.g.* LDH, ferritin, CRP, IL-6, IL-8, IL-10, TNF-α, IFN-α2, MCP-1 and MIP-1β) that is higher or greater than the reference value, e.g. threshold level, of the corresponding parameter is associated with a positive prediction of a risk of toxicity (alone or in conjunction with assessment of the other biomarkers in the panel). In some embodiments, a parameter of a biomarker that is equal to or lower than the reference value, e.g. threshold level, of the corresponding parameter is associated with a negative prediction of a risk of toxicity (alone or in conjunction with assessment of the other biomarkers in the panel).

In some embodiments, the threshold level is determined based on the level, amount, concentration or other measure of the biomarker (e.g. analyte) in the sample positive for the biomarker. In some aspects, the threshold level is within 25%, within 20%, within 15%, within 10% or within 5% of the average level, amount or concentration or measure, and/or is within a standard deviation of the average level, amount or concentration or measure, of the analyte or parameter in a biological sample obtained from a group of subjects prior to receiving a recombinant receptor-expressing therapeutic cell composition, wherein each of the subjects of the group went on to develop a toxicity, e.g. NT such as severe NT or grade 3 or higher neurotoxcity, e.g. grade 4 or 5 NT and/or CRS, such as severe CRS or grade 3 or higher CRS, after receiving a recombinant-receptor-expressing therapeutic cell composition for treating the same disease or condition.

In some embodiments of any of the provided methods, the biomarker (e.g. analyte) correlates to and/or is predictive of the risk of developing severe NT, such as severe NT or grade 3 or higher neurotoxcity, e.g. grade 4 or 5 NT and/or severe CRS or grade 3 or higher CRS. In some embodiments, the threshold level is within 25%, within 20%, within 15%, within 10% or within 5% of the average level, amount or concentration or measure, and/or is within a standard deviation of the average level, amount or concentration or measure, of the analyte or parameter in a biological sample obtained from a group of subjects prior to receiving a recombinant receptor-expressing therapeutic cell composition, wherein each of the subjects of the group went on to develop severe NT or grade 3 or higher neurotoxcity, e.g. grade 4 or 5 NT and/or severe CRS or grade 3 or higher CRS, after receiving a recombinant-receptor-expressing therapeutic cell composition for treating the same disease or condition.

In some embodiments, the volumetric measure is SPD and in some cases, development of toxicity, e.g., CRS or NT, is correlated with the SPD value that is above a threshold value. In some embodiments, the volumetric measure is SPD, and the threshold value is or is about 30 cm², is or is about 40 cm², is or is about 50 cm², is or is about 60 cm², or is or is about 70 cm². In some embodiments, the volumetric measure is SPD and the threshold value is or is about 30 cm²,is or is about 40 cm², is or is about 50 cm², is or is about 60 cm², or is or is about 70 cm².

In some embodiments, the parameter, including volumetric tumor measurements or is associated with response to the cell therapy, and/or a risk for developing toxicity, e.g., CRS or NT (NT).

In some embodiments, the volumetric measure is SPD and the threshold level is or is about 30 cm², is or is about 40 cm², is or is about 50 cm², is or is about 60 cm², or is or is about 70 cm². In some embodiments, the volumetric measure is SPD and the threshold level is or is about 50 cm².

In some embodiments, the analyte is LDH and the threshold level is or is about 300 units per liter (U/L), is or is about 400 U/L, is or is about 500 U/L or is or is about 600 U/L. In some embodiments, the analyte is LDH and the threshold level is or is about 500 U/L.

In some embodiments, the parameter or biomarker is LDH. In some embodiments, the biomarker is LDH and the threshold value is or is about 500 U/L or higher. In some embodiments, the parameter or biomarker is SPD. In some embodiments, the parameter is SPD, and the threshold value is or is about 50 cm² or higher. In some embodiments, biomarker or parameters are SPD and LDH, and the threshold values are SPD of at or about 50 cm² or higher and LDH of at or about 500 U/L or higher. In some embodiments, the biomarkers or parameters are associated with increased risk of developing CRS or NT.

In some embodiments, a measurement of the parameter or marker that is above the threshold value, e.g., SPD of at or about 50 cm² or higher and LDH of at or about 500 U/L or higher, are associated with an approximately 2-, 3-, 4-, 5-, 6-, 7-, 8-, 9-, 10-fold or more increased risk of developing CRS or NT, such as any grade CRS or NT. In some embodiments, a measurement of the parameter or marker that is below the threshold value, e.g., SPD of lower than at or about 500 cm² and LDH of lower than at or about 500 U/L, are associated with an approximately 2-, 3-, 4-, 5-, 6-, 7-, 8-, 9-, 10-fold or more decreased risk of developing CRS or NT, such as any grade CRS or NT.

In some embodiments, the biomarkers (e.g., analytes), include those associated with increased pharmacokinetic (PK) parameters of the cell, e.g., increased maximum serum concentration of cell (Cₘₐₓ) or increased exposure (e.g., area under the curve (AUC)). In some embodiments, the biomarkers (e.g. analytes), including parameters thereof, include IL-7, IL-15, MIP-1α and TNF-α.

In some embodiments, the parameter is a parameter related to tumor burden, e.g., a measurement of tumor burden. In some aspects, the methods also involve further monitoring the subject for possible symptoms of toxicity based on the risk of toxicity determined by assessment of the presence or absence of the biomarker and/or comparison of the biomarkers to a reference value or threshold level of the biomarker.\

In some embodiments, subjects with NHL who have high pre-treatment tumor burden (measured by the sum of product of the perpendicular diameters (SPD; ≥ 500 cm²) or high serum lactate dehydrogenase (LDH; ≥ 500 U/L) prior to the start of lymphodepletion may also have a higher risk for developing CRS and/or NT. In some embodiments, high pre-administration levels of inflammatory markers, such as ferritin and C-reactive protein (CRP) can be also associated with CRS. In some embodiments, peak levels of IL-6, IFN-γ, ferritin, and CRP can be associated with any grade or Grade 3 or higher NT. In some embodiments, subjects with B-cell acute lymphoblastic leukemia (ALL) and high burden of disease may be at an elevated risk of developing CRS. In some embodiments, severe NT can be associated with higher disease burden at the time of adoptive cell therapy. In some embodiments, protein levels in the cerebrospinal fluid (CSF) can be elevated in patients with NT, compared with baseline measurements In some aspects, other organ dysfunction (hepatic and renal), as well as hypoxemia, and infection, might also contribute to the encephalopathy. In some aspects, cytokine-mediated endothelial activation can be associated with coagulopathy, capillary leak, and blood-brain barrier disruption allowing transit of high concentrations of systemic cytokines into the CSF.

### 3. Reagents for Measuring

In some embodiments, the parameter, e.g., patient factor, biomarker, inflammatory marker and/or cytokine, is detected using one or more reagent(s) capable of detecting or that is specific for the parameter. In some embodiments, also provided are kits and articles of manufacture, for detection or assessment of the parameters and/or for modulating the therapy, e.g., cell therapy.

In some embodiments, instructions are also provided for using the reagent to assay a biological sample from a subject that is a candidate for treatment, optionally with a cell therapy, said cell therapy optionally including a dose or composition of genetically engineered cells expressing a recombinant receptor. In some embodiments of using the articles of manufacture, the level or presence of C-C Motif Chemokine Ligand 13 (CCL13), C-reactive protein (CRP), C-X-C motif chemokine 10 (CXCL10), D-dimer (fibrin degradation product), ferritin, IFN-α2, interleukin-2 (IL-2), IL-10, IL-15, IL-16, IL-6, IL-7, IL-8, interferon gamma (IFN-γ), lactate dehydrogenase (LDH), macrophage inflammatory protein (MIP-1α), MIP-1β, Monocyte chemoattractant protein-1 (MCP-1), SAA-1, Serum Amyloid A1 (SAA-1), tumor necrosis factor alpha (TNF-α), is detected and assessed. In some embodiments of using the articles of manufacture, the level or presence of C-reactive protein (CRP), erythrocyte sedimentation rate (ESR), albumin, ferritin, β2 microglobulin (β2-M), or lactate dehydrogenase (LDH) is detected and assessed. Also provided are methods of detecting and assessing one or more patient attributes, factors and/or biomarkers indicative of tumor burden.

**In** some embodiments, measuring the value of the one or more parameters, e.g., biomarkers, comprises contacting a reagent capable of directly or indirectly detecting the analyte with the biological sample and determining the presence or absence, level, amount or concentration of the analyte in the biological sample. In some embodiments, the one or more parameters, e.g., biomarkers, is C-C Motif Chemokine Ligand 13 (CCL13), C-reactive protein (CRP), C-X-C motif chemokine 10 (CXCL10), D-dimer (fibrin degradation product), ferritin, IFN-α2, interleukin-2 (IL-2), IL-10, IL-15, IL-16, IL-6, IL-7, IL-8, interferon gamma (IFN-γ), lactate dehydrogenase (LDH), macrophage inflammatory protein (MIP-1α), MIP-1β, Monocyte chemoattractant protein-1 (MCP-1), SAA-1, Serum Amyloid A1 (SAA-1), tumor necrosis factor alpha (TNF-α), is detected and assessed. In some embodiments of using the articles of manufacture, the level or presence of C-reactive protein (CRP), erythrocyte sedimentation rate (ESR), albumin, ferritin, β2 microglobulin (β2-M), or lactate dehydrogenase (LDH). In some embodiments, the one or more parameters, e.g., biomarkers, is or includes LDH.

In some aspects, the reagent is a binding molecule that specifically binds to the biomarker. For example, in some embodiments, the reagent is an antibody or an antigen-binding fragment thereof. In some embodiments, the reagent is or includes a substrate or binding partner of the biomarker.

In some embodiments, the presence, absence or level, amount, concentration and/or other measure of LDH is detected or determined in a sample. Various methods of detecting or determining LDH are known. For example, an assay which measures LDH conversion of lactate to pyruvate through NAD+ reduction to NADH can be used to detect LDH in the sample. In some embodiments, the sample is contacted with lactate in the presence of coenzyme NAD which, as a measure of LDH in the sample, results in NADH that is then oxidized in the presence of an electron transfer agent. In some embodiments, the NADH interacts with a probe or dye precursor that is detectable by measuring absorption in a visible light range. In some examples, diaphorase uses the NADH to reduce tetrazolium salt (INT) to a red formazan product and the product is measured. Therefore, in some embodiments, the amount of colored product formed is directly proportional to the LDH activity in the sample.

In some embodiments, the patient attributes, factors and/or biomarkers is assessed using an immune assay. For example, an enzyme-linked immunosorbent assay (ELISA), enzyme immunoassay (EIA), radioimmunoassay (RIA), surface plasmon resonance (SPR), Western Blot, Lateral flow assay, immunohistochemistry, protein array or immuno-PCR (iPCR) can be used to detect the patient attributes, factors and/or biomarkers. In some embodiments, using the articles of manufacture include detecting patient attributes, factors and/or biomarkers indicative of tumor burden. In some cases, the assaying or assessing of an patient attributes, factors and/or biomarkers is using flow cytometry. In some cases, the reagent is a soluble protein that binds the patient attributes, factors and/or biomarkers. In some example, the reagent is a protein that binds C-reactive protein (CRP), erythrocyte sedimentation rate (ESR), albumin, ferritin, β2 microglobulin (β2-M), or lactate dehydrogenase (LDH).

In some embodiments, C-reactive protein (CRP) is assessed using an in vitro enzyme-linked immunosorbent assay to obtain a quantitative measurement of human CRP from a sample such as serum, plasma, or blood. In some examples, CRP is detected using a human Enzyme- Linked Immunosorbent Assay (ELISA). In some embodiments, erythrocyte sedimentation rate (ESR) is assessed by measuring the distance (in millimeters per hour) that red cells have fallen after separating from the plasma in a vertical pipette or tube. In some aspects, albumin is assessed using a colorimetric test or an in vitro enzyme-linked immunosorbent assay. In some examples, albumin is detected using a human Enzyme-Linked Immunosorbent Assay (ELISA). In some embodiments, ferritin or β2 microglobulin is assessed using an immunoassay or detected using an ELISA. In some aspects, lactate dehydrogenase (LDH) is assessed using a colorimetric test or an in vitro enzyme-linked immunosorbent assay.

Among the provided antibodies are monoclonal antibodies, including monoclonal antibody fragments. The term "monoclonal antibody" as used herein refers to an antibody obtained from or within a population of substantially homogeneous antibodies, i.e., the individual antibodies comprising the population are identical, except for possible variants containing naturally occurring mutations or arising during production of a monoclonal antibody preparation, such variants generally being present in minor amounts. In contrast to polyclonal antibody preparations, which typically include different antibodies directed against different epitopes, each monoclonal antibody of a monoclonal antibody preparation is directed against a single epitope on an antigen. The term is not to be construed as requiring production of the antibody by any particular method. A monoclonal antibody may be made by a variety of techniques, including but not limited to generation from a hybridoma, recombinant DNA methods, phage-display and other antibody display methods.

Also provided are antibody immunoconjugates comprising an antibody against biomarker attached to a label, which can generate a detectable signal, indirectly or directly. These antibody immunoconjugates can be used for research or diagnostic applications. The label is preferably capable of producing, either directly or indirectly, a detectable signal. For example, the label may be radio-opaque or a radioisotope, such as ³H, ¹⁴C, ³²P, ³⁵S, ¹²³I, ¹²⁵I, ¹³¹I; a fluorescent (fluorophore) or chemiluminescent (chromophore) compound, such as fluorescein isothiocyanate, rhodamine or luciferin; an enzyme, such as alkaline phosphatase,β-galactosidase or horseradish peroxidase; an imaging agent; or a metal ion. In some embodiments, the label is a radioactive atom for scintigraphic studies, for example ⁹⁹Tc or ¹²³I, or a spin label for nuclear magnetic resonance (NMR) imaging (also known as magnetic resonance imaging, MRI), such as zirconium-89, iodine-123, iodine-131, indium-111, fluorine-19, carbon-13, nitrogen-15, oxygen-17, gadolinium, manganese or iron. Zirconium-89 may be complexed to various metal chelating agents and conjugated to antibodies, *e.g*., for PET imaging (WO 2011/056983).

In some embodiments, the antibody immunoconjugate is detectable indirectly. For example, a secondary antibody that is specific for the antibody against the marker expressed on a population of myeloid cells immunoconjugate and contains a detectable label can be used to detect the antibody immunoconjugate.

In some embodiments, antibodies capable of detecting or that is specific the patient attributes, factors and/or biomarkers provided herein may be identified, screened for, or characterized for their physical/chemical properties and/or biological activities by various known assays. In one aspect, the antibody is tested for its antigen binding activity, e.g., by known methods such as an immunoassay, ELISA, Western blotting, and/or flow cytometric assays, including cell-based binding assays.

### II. INTERVENTIONS OR AGENTS THAT TREAT OR AMELIORATE SYMPTOMS OF TOXICITY

In some embodiments, the provided methods and articles of manufacture can be used in connection with, or involve or include, one or more agents or treatments for treating, preventing, delaying, or attenuating the development of a toxicity. In some examples, the agent or other treatment capable of treating, preventing, delaying, or attenuating the development of a toxicity is administered prior to and/or concurrently with administration of a therapeutic cell composition comprising the genetically engineered cells.

In some embodiments, the agent, e.g., a toxicity-targeting agent, or treatment capable of treating, preventing, delaying, or attenuating the development of a toxicity is a steroid, is an antagonist or inhibitor of a cytokine receptor, such as IL-6 receptor, CD122 receptor (IL-2Rbeta receptor), or CCR2, or is an inhibitor of a cytokine, such as IL-6, MCP-1, IL-10, IFN-γ, IL-8, or IL-18. In some embodiments, the agent is an agonist of a cytokine receptor and/or cytokine, such as TGF-β. In some embodiments, the agent, e.g., agonist, antagonist or inhibitor, is an antibody or antigen-binding fragment, a small molecule, a protein or peptide, or a nucleic acid.

In some embodiments, a fluid bolus can be employed as an intervention, such as to treat hypotension associated with CRS. In some embodiments, the target hematocrit levels are >24%. In some embodiments, the intervention includes the use of absorbent resin technology with blood or plasma filtration. In some cases, the intervention includes dialysis, plasmapheresis, or similar technologies. In some embodiments, vassopressors or acetaminophen can be employed.

In some embodiments, the agent can be administered sequentially, intermittently, or at the same time as or in the same composition as the therapy, such as cells for adoptive cell therapy. For example, the agent can be administered before, during, simultaneously with, or after administration of the immunotherapy and/or cell therapy.

In some embodiments, the agent is administered at a time as described herein and in accord with the provided methods, and/or with the provided articles of manufacture or compositions. In some embodiments, the toxicity-targeting agent is administered at a time that is within, such as less than or no more than, 3, 4, 5, 6, 7, 8, 9 or 10 days after initiation of the immunotherapy and/or cell therapy. In some embodiments, the toxicity-targeting agent is administered within or within about 1 day, 2 days or 3 days after initiation of administration of the immunotherapy and/or cell therapy.

In some embodiments, the agent, e.g., toxicity-targeting agent, is administered to a subject after initiation of administration of the immunotherapy and/or cell therapy at a time at which the subject does not exhibit grade 2 or higher CRS or grade 2 or higher neurotoxicity. In some aspects, the toxicity-targeting agent is administered after initiation of administration of the immunotherapy and/or cell therapy at a time at which the subject does not exhibit severe CRS or severe neurotoxicity. Thus, between initiation of administration of the immunotherapy and/or cell therapy and the toxicity-targeting agent, the subject is one that does not exhibit grade 2 or higher CRS, such as severe CRS, and/or does not exhibit grade 2 or higher neurotoxicity, such as severe neurotoxicity.

In some embodiments, the agent can be administered based on or according certain procedures, guidelines, interventions or regimens, e.g., based on assessment and monitoring of outcomes, such as toxicity and/or response outcomes, and/or monitoring of parameters or biomarkers, e.g., pharmacokinetic parameters, patient attributes or factors and/or expression of biomarkers. In some embodiments, exemplary procedures, guidelines, intervention or regimen includes, but are not limited to, intervention or regimens described in WO 2019/109035.

Non-limiting examples of interventions for treating or ameliorating a toxicity, such as a severe CRS (sCRS), or a severe neurotoxicity, are described in **Table 7.** In some embodiments, the intervention includes tocilizumab or other toxicity-targeting agent as described, which can be at a time in which there is a sustained or persistent fever of greater than or about 38 °C or greater than or greater than about 39 °C in the subject. In some embodiments, the fever is sustained in the subject for more than 10 hours, more than 12 hours, more than 16 hours, or more than 24 hours before intervention.

| **Table 7. Exemplary Interventions.** | |
|---|---|
| **Symptoms related to CRS** | **Suggested Intervention** |
| Fever of ≥ 38.3°C | Acetaminophen (12.5 mg/kg) PO/IV up to every four hours |
| Persistent fever of ≥ 39°C for 10 hours that is unresponsive to acetaminophen | Tocilizumab (8-12 mg/kg) IV |
| Persistent fever of ≥ 39°C after tocilizumab | Dexamethasone 5-10 mg IV/PO up to every 6-12 hours with continued fevers |
| Recurrence of symptoms 48 hours after initial dose of tocilizumab | Tocilizumab (8-12 mg/kg) IV |
| Hypotension | Fluid bolus, target hematocrit >24% |
| Persistent/recurrent hypotension after initial fluid bolus (within 6 hours) | Tocilizumab (8-12 mg/kg) IV |
| Use of low dose pressors for hypotension for longer than 12 hours | Dexamethasone 5-10 mg IV/PO up to every 6 hours with continued use of pressors |
| Initiation of higher dose pressors or addition of a second pressor for hypotension | Dexamethasone 5-10 mg IV/PO up to every 6 hours with continued use of pressors |
| Initiation of oxygen supplementation | Tocilizumab (8-12 mg/kg) IV |
| Increasing respiratory support with concern for impending intubation | Dexamethasone 5-10 mg IV/PO up to every 6 hours with continued use of pressors |
| Recurrence/Persistence of symptoms for which tocilizumab was given ≥ 48 hours after initial dose was administered | Tocilizumab (8-12 mg/kg) IV |

Other non-limiting examples of administering the agent or therapy or intervention, are described in **Table 8** (CRS) and **Table 9** (NT) below. In some aspects, cytokine release syndrome (CRS) is identified based on clinical presentation. In some aspects, the subject is evaluated for and treated for other causes of fever, hypoxia, and hypotension. In some embodiments, if the subject is suspected of having CRS, the CRS is managed according to the guidelines in **Table 8.** In some embodiments, if the subject is suspected of having a concurrent neurologic toxicity, during the CRS, the neurologic toxicity is managed according to the guidelines in **Table 9.**

**Table 8. CRS Grading and Management Guidance**

| **CRS Grade^{a}** | **Tocilizumab** | **Corticosteroids** |
|---|---|---|
| **Grade 1** | If 72 hours or more after infusion, treat symptomatically. | If 72 hours or more after infusion, treat symptomatically. |
| Fever | | |
| | If less than 72 hours after infusion, administer tocilizumab 8 mg/kg IV over 1 hour (not to exceed 800 mg). | If less than 72 hours after infusion, administer dexamethasone 10 mg IV every 24 hours. |
| **Grade 2** | Administer tocilizumab 8 mg/kg IV over 1 hour (not to exceed 800 mg). | If 72 hours or more after infusion, consider dexamethasone 10 mg IV every 12-24 hours. |
| Symptoms require and respond to moderate intervention. | | |
| Fever, oxygen requirement less than 40% FiO₂, or hypotension responsive to fluids or low dose of one vasopressor, or Grade 2 organ toxicity. | | If less than 72 hours after infusion, administer dexamethasone 10 mg IV every 12-24 hours. |
| | If no improvement within 24 hours or rapid progression, repeat tocilizumab and escalate dose and frequency of dexamethasone (10-20 mg IV every 6 to 12 hours). | |
| | If no improvement or continued rapid progression, maximize dexamethasone, switch to high-dose methylprednisolone 2 mg/kg if needed. After 2 doses of tocilizumab, consider alternative immunosuppressants. Do not exceed 3 doses tocilizumab in 24 hours, or 4 doses in total. | |
| **Grade 3** | Per Grade 2. | Administer dexamethasone 10 mg IV every 12 hours. |
| Symptoms require and respond to aggressive intervention. | | |
| | If no improvement within 24 hours or rapid progression of CRS, escalate tocilizumab and steroid use as per Grade 2. | |
| Fever, oxygen requirement greater than or equal to 40% FiO₂, or hypotension requiring high-dose or multiple vasopressors, or Grade 3 organ toxicity, or Grade 4 transaminitis. | | |
| **Grade 4** | Per Grade 2. | Administer dexamethasone 20 mg IV every 6 hours. |
| Life-threatening symptoms | | |
| Requirements for ventilator support or continuous veno-venous hemodialysis (CVVHD) or Grade 4 organ toxicity (excluding transaminitis). | If no improvement within 24 hours or rapid progression of CRS, escalate tocilizumab and steroid use as per Grade 2. | |

| | | |
|---|---|---|
| If steroids are initiated, continue steroids for at least 3 doses or until complete resolution of symptoms, and consider steroid taper. ^{a} Lee criteria for grading CRS (Lee et al, 2014). | | |

In some aspects, for neurological events (NE) or neurotoxicity (NT), the subject is monitored for signs and symptoms of neurologic toxicities, e.g., as described in **Table 9.** In some aspects, other causes of neurologic symptoms are ruled out. In some aspects, intensive care supportive therapy for severe or life-threatening neurologic toxicities are provided. In some embodiments, if NT is suspected, the NT is managed according to the guidelines in **Table 9.** In some aspects, if the subject is suspected to have a concurrent CRS during the neurologic toxicity event, CRS is managed according to the recommendations in Table 8.

**Table 9. Neurologic Toxicity (NT) Grading and Management Guidance**

| **NT Grade^{a}** | **Corticosteroids and Antiseizure Medication** |
|---|---|
| **Grade 1** | Start nonsedating, antiseizure medicines (e.g., levetiracetam) for seizure prophylaxis. |
| | If 72 hours or more after infusion, observe. |
| | If less than 72 hours after infusion, consider dexamethasone 10 mg IV every 12 to 24 hours for 2 to 3 days. |
| **Grade 2** | Start nonsedating, antiseizure medicines (e.g., levetiracetam) for seizure prophylaxis. |
| | Dexamethasone 10 mg IV every 12 hours for 2-3 days, or longer for persistent symptoms. Consider taper for a total steroid exposure of greater than 3 days. |
| | If no improvement after 24 hours or worsening of neurologic toxicity, increase the dose and/or frequency of dexamethasone up to a maximum of 20 mg IV every 6 hours. |
| | If no improvement after another 24 hours, rapidly progressing symptoms, or life-threatening complications arise, give methylprednisolone (2 mg/kg loading dose, followed by 2 mg/kg divided 4 times a day; taper within 7 days). |
| **Grade 3** | Start nonsedating, antiseizure medicines (e.g., levetiracetam) for seizure prophylaxis. |
| | Dexamethasone 10 to 20 mg IV every 8 to 12 hours. Steroids are not recommended for isolated Grade 3 headaches. |
| | If no improvement after 24 hours or worsening of neurologic toxicity, escalate to methylprednisolone (dose and frequency as per Grade 2). |
| | If cerebral edema is suspected, consider hyperventilation and hyperosmolar therapy. Give high-dose methylprednisolone (1-2 g, repeat every 24 hours if needed; taper as clinically indicated) and cyclophosphamide 1.5 mg/m². |
| **Grade 4** | Start nonsedating, antiseizure medicines (e.g., levetiracetam) for seizure |
| | prophylaxis. |
| | Dexamethasone 20 mg IV every 6 hours. |
| | If no improvement after 24 hours or worsening of neurologic toxicity, escalate to methylprednisolone (dose and frequency as per Grade 2). |
| | If cerebral edema is suspected, consider hyperventilation and hyperosmolar therapy. Give high-dose methylprednisolone (1-2 g, repeat every 24 hours if needed; taper as clinically indicated), and cyclophosphamide 1.5 mg/m². |

| | |
|---|---|
| ^{a} NCI CTCAE criteria for grading neurologic toxicities. | |

In some cases, the agent or therapy or intervention, e.g., toxicity-targeting agent, is administered alone or is administered as part of a composition or formulation, such as a pharmaceutical composition or formulation, as described herein. Thus, the agent alone or as part of a pharmaceutical composition can be administered intravenously or orally, or by any other acceptable known route of administration or as described herein.

In some embodiments, the dosage of agent or the frequency of administration of the agent in a dosage regimen is reduced compared to the dosage of the agent or its frequency in a method in which a subject is treated with the agent after a grade 2 or higher CRS or neurotoxicity, such as after a severe, e.g., grade 3 or higher, CRS or after severe, e.g., grade 3 or higher neurotoxicity, has developed or been diagnosed (e.g. after physical signs or symptoms of grade 3 or higher CRS or neurotoxicity has manifested). In some embodiments, the dosage of agent or the frequency of administration of the agent in a dosage regimen is reduced compared to the dosage of the agent or its frequency in a method in which a subject is treated for CRS or neurotoxicity greater than 3 days, 4 days, 5 days, 6 days, 1 week, 2 weeks, three weeks, or more after administration of the immunotherapy and/or cell therapy. In some embodiments, the dosage is reduced by greater than or greater than about 1.2-fold, 1.5-fold, 2-fold, 3-fold, 4-fold, 5-fold, 6-fold, 7-fold, 8-fold, 9-fold, 10-fold or more. In some embodiments, the dosage is reduced by greater than or about 10%, 20%, 30%, 40%, 50%, 60%, 70%, 80%, 90%, or more. In some embodiments, the frequency of dosing is reduced, such as the number of daily doses is reduced or the number of days of dosing is reduced.

### A. Steroid

In some embodiments, the agent, e.g., toxicity-targeting agent, that treats and/or that prevents, delays, or attenuates the development of or risk for developing a toxicity to an immunotherapy and/or a cell therapy, is a steroid, e.g., corticosteroid. Corticosteroids typically include glucocorticoids and mineralocorticoids.

Any corticosteroid, e.g., glucocorticoid, can be used in the methods provided herein. In some embodiments, glucocorticoids include synthetic and non-synthetic glucocorticoids. Exemplary glucocorticoids include, but are not limited to: alclomethasones, algestones, beclomethasones (e.g. beclomethasone dipropionate), betamethasones (e.g. betamethasone 17-valerate, betamethasone sodium acetate, betamethasone sodium phosphate, betamethasone valerate), budesonides, clobetasols (e.g. clobetasol propionate), clobetasones, clocortolones (e.g. clocortolone pivalate), cloprednols, corticosterones, cortisones and hydrocortisones (e.g. hydrocortisone acetate), cortivazols, deflazacorts, desonides, desoximethasones, dexamethasones (e.g. dexamethasone 21-phosphate, dexamethasone acetate, dexamethasone sodium phosphate), diflorasones (e.g. diflorasone diacetate), diflucortolones, difluprednates, enoxolones, fluazacorts, flucloronides, fludrocortisones (e.g., fludrocortisone acetate), flumethasones (e.g. flumethasone pivalate), flunisolides, fluocinolones (e.g. fluocinolone acetonide), fluocinonides, fluocortins, fluocortolones, fluorometholones (e.g. fluorometholone acetate), fluperolones (e.g., fluperolone acetate), fluprednidenes, fluprednisolones, flurandrenolides, fluticasones (e.g. fluticasone propionate), formocortals, halcinonides, halobetasols, halometasones, halopredones, hydrocortamates, hydrocortisones (e.g. hydrocortisone 21-butyrate, hydrocortisone aceponate, hydrocortisone acetate, hydrocortisone buteprate, hydrocortisone butyrate, hydrocortisone cypionate, hydrocortisone hemisuccinate, hydrocortisone probutate, hydrocortisone sodium phosphate, hydrocortisone sodium succinate, hydrocortisone valerate), loteprednol etabonate, mazipredones, medrysones, meprednisones, methylprednisolones (methylprednisolone aceponate, methylprednisolone acetate, methylprednisolone hemisuccinate, methylprednisolone sodium succinate), mometasones (e.g., mometasone furoate), paramethasones (e.g., paramethasone acetate), prednicarbates, prednisolones (e.g. prednisolone 25-diethylaminoacetate, prednisolone sodium phosphate, prednisolone 21-hemisuccinate, prednisolone acetate; prednisolone farnesylate, prednisolone hemisuccinate, prednisolone-21 (beta-D-glucuronide), prednisolone metasulphobenzoate, prednisolone steaglate, prednisolone tebutate, prednisolone tetrahydrophthalate), prednisones, prednivals, prednylidenes, rimexolones, tixocortols, triamcinolones (e.g. triamcinolone acetonide, triamcinolone benetonide, triamcinolone hexacetonide, triamcinolone acetonide 21-palmitate, triamcinolone diacetate). These glucocorticoids and the salts thereof are discussed in detail, for example, in Remington's Pharmaceutical Sciences, A. Osol, ed., Mack Pub. Co., Easton, Pa. (16th ed. 1980).

In some examples, the glucocorticoid is selected from among cortisones, dexamethasones, hydrocortisones, methylprednisolones, prednisolones and prednisones. In a particular example, the glucocorticoid is dexamethasone.

In some embodiments, the agent is a corticosteroid and is administered in an amount that is therapeutically effective to treat, ameliorate or reduce one or more symptoms of a toxicity to an immunotherapy and/or a cell therapy, such as CRS or neurotoxicity. In some embodiments, indicators of improvement or successful treatment include determination of the failure to manifest a relevant score on toxicity grading scale (e.g. CRS or neurotoxicity grading scale), such as a score of less than 3, or a change in grading or severity on the grading scale as discussed herein, such as a change from a score of 4 to a score of 3, or a change from a score of 4 to a score of 2, 1 or 0.

In some aspects, the corticosteroid is provided in a therapeutically effective dose. Therapeutically effective concentration can be determined empirically by testing in known in vitro or in vivo (e.g. animal model) systems. For example, the amount of a selected corticosteroid to be administered to ameliorate symptoms or adverse effects of a toxicity to an immunotherapy and/or a cell therapy, such as CRS or neurotoxicity, can be determined by standard clinical techniques. In addition, animal models can be employed to help identify optimal dosage ranges. The precise dosage, which can be determined empirically, can depend on the particular therapeutic preparation, the regimen and dosing schedule, the route of administration and the seriousness of the disease.

The corticosteroid can be administered in any amount that is effective to ameliorate one or more symptoms associated with the toxicity, such as with the CRS or neurotoxicity. The corticosteroid, e.g., glucocorticoid, can be administered, for example, at an amount between at or about 0.1 and at or about 100 mg, per dose, at or about 0.1 and at or about 80 mg, at or about 0.1 and at or about 60 mg, at or about 0.1 and at or about 40 mg, at or about 0.1 and at or about 30 mg, at or about 0.1 and at or about 20 mg, at or about 0.1 and at or about 15 mg, at or about 0.1 and at or about 10 mg, at or about 0.1 and at or about 5 mg, at or about 0.2 and at or about 40 mg, at or about 0.2 and at or about 30 mg, at or about 0.2 and at or about 20 mg, at or about 0.2 and at or about 15 mg, at or about 0.2 and at or about 10 mg, at or about 0.2 and at or about 5 mg, at or about 0.4 and at or about 40 mg, at or about 0.4 and at or about 30 mg, at or about 0.4 and at or about 20 mg, at or about 0.4 and at or about 15 mg, at or about 0.4 and at or about 10 mg, at or about 0.4 and at or about 5 mg, at or about 0.4 and at or about 4 mg, at or about 1 and at or about 20 mg, at or about 1 and at or about 15 mg or 1 and at or about 10 mg, to a 70 kg adult human subject. Typically, the corticosteroid, such as a glucocorticoid is administered at an amount between at or about 0.4 and at or about 20 mg, for example, at or about 0.4 mg, 0.5 mg, 0.6 mg, 0.7 mg, 0.75 mg, 0.8 mg, 0.9 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg or 20 mg per dose, to an average adult human subject.

In some embodiments, the corticosteroid can be administered, for example, at a dosage of at or about 0.001 mg/kg (of the subject), 0.002 mg/kg, 0.003 mg/kg, 0.004 mg/kg, 0.005 mg/kg, 0.006 mg/kg, 0.007 mg/kg, 0.008 mg/kg, 0.009 mg/kg, 0.01 mg/kg, 0.015 mg/kg, 0.02 mg/kg, 0.025 mg/kg, 0.03 mg/kg, 0.035 mg/kg, 0.04 mg/kg, 0.045 mg/kg, 0.05 mg/kg, 0.055 mg/kg, 0.06 mg/kg, 0.065 mg/kg, 0.07 mg/kg, 0.075 mg/kg, 0.08 mg/kg, 0.085 mg/kg, 0.09 mg/kg, 0.095 mg/kg, 0.1 mg/kg, 0.15 mg/kg, 0.2 mg/kg, 0.25 mg/kg, 0.30 mg/kg, 0.35 mg/kg, 0.40 mg/kg, 0.45 mg/kg, 0.50 mg/kg, 0.55 mg/kg, 0.60 mg/kg, 0.65 mg/kg, 0.70 mg/kg, 0.75 mg/kg, 0.80 mg/kg, 0.85 mg/kg, 0.90 mg/kg, 0.95 mg/kg, 1 mg/kg, 1.05 mg/kg, 1.1 mg/kg, 1.15 mg/kg, 1.20 mg/kg, 1.25 mg/kg, 1.3 mg/kg, 1.35 mg/kg or 1.4 mg/kg, to an average adult human subject, typically weighing about 70 kg to 75 kg.

The corticosteroid, or glucocorticoid, for example dexamethasone, can be administered orally (tablets, liquid or liquid concentrate), PO, intravenously (IV), intramuscularly or by any other known route or route described herein (e.g., with respect to pharmaceutical formulations). In some aspects, the corticosteroid is administered as a bolus, and in other aspects it may be administered over a period of time.

In some aspects, the glucocorticoid can be administered over a period of more than one day, such as over two days, over 3 days, or over 4 or more days. In some embodiments, the corticosteroid can be administered one per day, twice per day, or three times or more per day. For example, the corticosteroid, e.g., dexamethasone, may in some examples be administered at 10 mg (or equivalent) IV twice a day for three days.

In some embodiments, the dosage of corticosteroid, e.g., glucocorticoid, is administered in successively lower dosages per treatment. Hence, in some such treatment regimes, the dose of corticosteroid is tapered. For example, the corticosteroid may be administered at an initial dose (or equivalent dose, such as with reference to dexamethasone) of 4 mg, and upon each successive administration the dose may be lowered, such that the dose is 3 mg for the next administration, 2 mg for the next administration, and 1 mg for the next administration

Generally, the dose of corticosteroid administered is dependent upon the specific corticosteroid, as a difference in potency exists between different corticosteroids. It is typically understood that drugs vary in potency, and that doses can therefore vary, in order to obtain equivalent effects. **Table 8** shows equivalence in terms of potency for various glucocorticoids and routes of administration. Equivalent potency in clinical dosing is well known. Information relating to equivalent steroid dosing (in a non-chronotherapeutic manner) may be found in the British National Formulary (BNF) 37, March 1999.

| **Table 8: Glucocorticoid administration** | |
|---|---|
| **Glucocorticoid (Route)** | **Equivalency Potency** |
| Hydrocortisone (IV or PO) | 20 |
| Prednisone | 5 |
| Prednisolone (IV or PO) | 5 |
| Methylprednisolone sodium succinate (IV) | 4 |
| Dexamethasone (IV or PO) | 0.5-0.75 |

Thus, in some embodiments, the steroid is administered in an equivalent dosage amount of from or from about 1.0 mg to or to about 20 mg dexamethasone per day, such as from or from about 1.0 mg to or to about 15 mg dexamethasone per day, from or from about 1.0 mg to or to about 10 mg dexamethasone per day, from or from about 2.0 mg to or to about 8 mg dexamethasone per day, or from or from about 2.0 mg to or to about 6.0 mg dexamethasone per day, each inclusive. In some cases, the steroid is administered in an equivalent dose of at or about 4 mg or at or about 8 mg dexamethasone per day.

In some embodiments, the steroid is administered if fever persists after treatment with tocilizumab. For example, in some embodiments, dexamethasone is administered orally or intravenously at a dosage of 5-10 mg up to every 6-12 hours with continued fevers. In some embodiments, tocilizumab is administered concurrently with or subsequent to oxygen supplementation.

### B. Microglial Cell Inhibitor

In some embodiments, the inhibitor in the combination therapy is an inhibitor of a microglial cell activity. In some embodiments, the administration of the inhibitor modulates the activity of microglia. In some embodiments, the inhibitor is an antagonist that inhibits the activity of a signaling pathway in microglia. In some embodiments, the microglia inhibitor affects microglial homeostasis, survival, and/or proliferation. In some embodiments, the inhibitor targets the CSF1R signaling pathway. In some embodiments, the inhibitor is an inhibitor of CSF1R. In some embodiments, the inhibitor is a small molecule. In some cases, the inhibitor is an antibody.

In some aspects, administration of the inhibitor results in one or more effects selected from an alteration in microglial homeostasis and viability, a decrease or blockade of microglial cell proliferation, a reduction or elimination of microglial cells, a reduction in microglial activation, a reduction in nitric oxide production from microglia, a reduction in nitric oxide synthase activity in microglia, or protection of motor neurons affected by microglial activation. In some embodiments, the agent alters the level of a serum or blood biomarker of CSF1R inhibition, or a decrease in the level of urinary collagen type 1 cross-linked N-telopeptide (NTX) compared to at a time just prior to initiation of the administration of the inhibitor. In some embodiments, the administration of the agent transiently inhibits the activity of microglia activity and/or wherein the inhibition of microglia activity is not permanent. In some embodiments, the administration of the agent transiently inhibits the activity of CSF1R and/or wherein the inhibition of CSF1R activity is not permanent.

In some embodiments, the agent that reduces microglial cell activity is a small molecule, peptide, protein, antibody or antigen-binding fragment thereof, an antibody mimetic, an aptamer, or a nucleic acid molecule. In some embodiments, the method involves administration of an inhibitor of microglia activity. In some embodiments, the agent is an antagonist that inhibits the activity of a signaling pathway in microglia. In some embodiments, the agent that reduces microglial cell activity affects microglial homeostasis, survival, and/or proliferation.

In some embodiments, the agent that reduces microglial cell activation is selected from an anti-inflammatory agent, an inhibitor of NADPH oxidase (NOX2), a calcium channel blocker, a sodium channel blocker, inhibits GM-CSF, inhibits CSF1R, specifically binds CSF-1, specifically binds IL-34, inhibits the activation of nuclear factor kappa B (NF-κB), activates a CB₂ receptor and/or is a CB₂ agonist, a phosphodiesterase inhibitor, inhibits microRNA-155 (miR-155), upregulates microRNA-124 (miR-124), inhibits nitric oxide production in microglia, inhibits nitric oxide synthase, or activates the transcription factor NRF2 (also called nuclear factor (erythroid-derived 2)-like 2, or NFE2L2).

In some embodiments, the agent that reduces microglial cell activity targets CSF1 (also called macrophage colony-stimulating factor MCSF). In some embodiments, the agent that reduces microglial cell activity affects MCSF-stimulated phosphorylation of the M-CSF receptor (Pryer et al. Proc Am Assoc Cancer Res, AACR Abstract nr DDT02-2 (2009)). In some cases, the agent that reduces microglial cell activity is MCS110 (international patent application publication number WO2014001802; Clinical Trial Study Record Nos.:A1 NCT00757757; NCT02807844; NCT02435680; NCT01643850).

In some embodiments, the agent that reduces microglial cell activity is a small molecule that targets the CSF1 pathway. In some embodiments, the agent is a small molecule that binds CSF1R. In some embodiments, the agent is a small molecule which inhibits CSF1R kinase activity by competing with ATP binding to CSF1R kinase. In some embodiments, the agent is a small molecule which inhibits the activation of the CFS1R receptor. In some cases, the binding of the CSF-1 ligand to the CSF1R is inhibited. In some embodiments, the agent that reduces microglial cell activity is any of the inhibitors described in US Patent Application Publication Number US20160032248.

In some embodiments, the agent is a small molecule inhibitor selected from PLX-3397, PLX7486, JNJ-40346527, JNJ28312141, ARRY-382, PLX73086 (AC-708), DCC-3014, AZD6495, GW2580, Ki20227, BLZ945, PLX647, PLX5622. In some embodiments, the agent is any of the inhibitors described in Conway et al., Proc Natl Acad Sci U S A, 102(44):16078-83 (2005); Dagher et al., Journal of Neuroinflammation, 12:139 (2015); Ohno et al., Mol Cancer Ther. 5(11):2634-43 (2006); von Tresckow et al. Clin Cancer Res.,21(8) (2015); Manthey et al. Mol Cancer Ther. (8(11):3151-61 (2009); Pyonteck et al., Nat Med. 19(10): 1264-1272 (2013); Haegel et al., Cancer Res AACR Abstract nr 288

(2015); Smith et al., Cancer Res AACR Abstract nr 4889 (2016); Clinical Trial Study Record Nos.: NCT01525602; NCT02734433; NCT02777710; NCT01804530; NCT01597739; NCT01572519; NCT01054014; NCT01316822; NCT02880371; NCT02673736; international patent application publication numbers WO2008063888A2, WO2006009755A2, US patent application publication numbers US20110044998, US 2014/0065141, and US 2015/0119267.

In some embodiments, the agent that reduces microglial cell activity is 4-((2-(((1R,2R)-2-hydroxycyclohexyl)amino)benzo[d]thiazol-6-yl)oxy)-N-methylpicolinamide (BLZ945) or a pharmaceutically acceptable salt thereof or derivatives thereof. In some embodiments, the agent is the following compound: 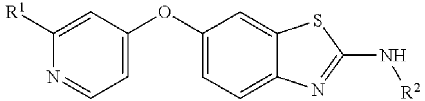 wherein R1 is an alkyl pyrazole or an alkyl carboxamide, and R2 is a hydroxycycloalkyl or a pharmaceutically acceptable salt thereof.

**In** some embodiments, the agent that reduces microglial cell activity is 5-((5-chloro-1H-pyrrolo[2,3-b]pyridin-3-yl)methyl)-N-((6-(trifluoromethyl)pyridin-3-yl)methyl)pyridin-2-amine, N-[5-[(5-Chloro-1H-pyrrolo[2,3-b]pyridin-3-yl)methyl]-2-pyridinyl]-6-(trifluoromethyl)-3-pyridinemethanamine) (PLX 3397) or a pharmaceutically acceptable salt thereof or derivatives thereof. In some embodiments, the agent is 5-(1H-Pyrrolo[2,3-b]pyridin-3-ylmethyl)-N-[[4-(trifluoromethyl)phenyl]methyl]-2-pyridinamine dihydrochloride (PLX647) or a pharmaceutically acceptable salt thereof or derivatives thereof. In some embodiments, the agent that reduces microglial cell activity is the following compound: 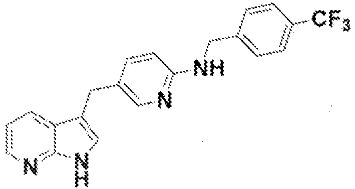 or a pharmaceutically acceptable salt thereof. In some embodiments, the agent that reduces microglial cell activity is the following compound: 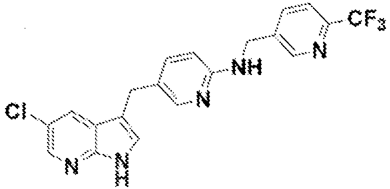 or a pharmaceutically acceptable salt thereof. In some embodiments, the agent is any of the inhibitors described in US patent number US7893075.

In some embodiments, the agent that reduces microglial cell activity is 4-cyano-N-[2-(1-cyclohexen-1-yl)-4-[1-[(dimethylamino)acetyl]-4-piperidinyl]phenyl]-1H-imidazole-2-carboxamide monohydrochloride (JNJ28312141) or a pharmaceutically acceptable salt thereof or derivatives thereof. In some embodiments, the agent is the following compound: 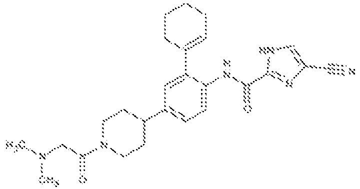 or a pharmaceutically acceptable salt thereof. In some embodiments, the agent is any of the inhibitors described in US patent number US7645755.

In some embodiments, the agent that reduces microglial cell activity is 1H-Imidazole-2-carboxamide, 5-cyano-N-(2-(4,4-dimethyl-1-cyclohexen-1-yl)-6-(tetrahydro-2,2,6,6-tetramethyl-2H-pyran-4-yl)-3-pyridinyl)-, 4-Cyano-1H-imidazole-2-carboxylic acid N-(2-(4,4-dimethylcyclohex-1-enyl)-6-(2,2,6,6-tetramethyltetrahydropyran-4-yl)pyridin-3-yl)amide, 4-Cyano-N-(2-(4,4-dimethylcyclohex-1-en-1-yl)-6-(2,2,6,6-tetramethyl-tetrahydro-2H-pyran-4-yl)pyridin-3-yl)-1H-imidazole-2-carboxamide (JNJ-40346527) or a pharmaceutically acceptable salt thereof or derivatives thereof. In some embodiments, the agent is the following compound: 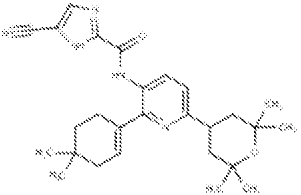 or a pharmaceutically acceptable salt thereof.

In another embodiment, the agent that reduces microglial cell activity is 5-(3- Methoxy-4-((4-methoxybenzyl)oxy)benzyl)pyrimidine-2,4-diamine (GW2580) or a pharmaceutically acceptable salt thereof or derivatives thereof. In some embodiments, the agent is the following compound: 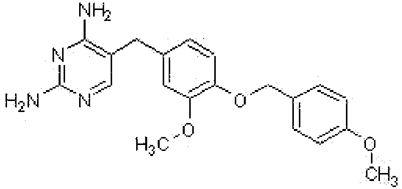 or a pharmaceutically acceptable salt thereof (international patent application publication number WO2009099553).

In some embodiments, the agent that reduces microglial cell activity is 4-(2,4-difluoroanilino)-7-ethoxy-6-(4-methylpiperazin-1-yl)quinoline-3-carboxamide (AZD6495) or a pharmaceutically acceptable salt thereof or derivatives thereof. In some embodiments, the agent is the following compound: 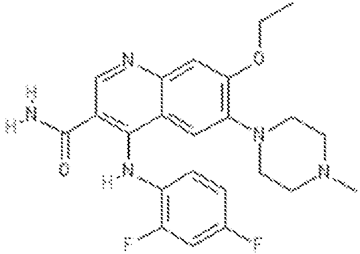 or a pharmaceutically acceptable salt thereof.

In some embodiments, the agent that reduces microglial cell activity is N-{4-[(6,7-dimethoxy-4-quinolyl)oxy]-2-methoxyphenyl}-N0-[1-(1,3-thiazole-2-yl)ethyl]urea (Ki20227) or a pharmaceutically acceptable salt thereof or derivatives thereof. In some embodiments, the agent is the following compound: 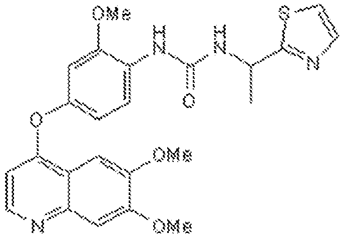 or a pharmaceutically acceptable salt thereof.

In some embodiments, the agent that reduces microglial cell activation is an antibody that targets the CSF1 pathway. In some embodiments, the agent is an antibody that binds CSF1R. In some embodiments, the anti-CSF1R antibody blocks CSF1R dimerization. In some embodiments, the anti-CSF1R antibody blocks the CSF1R dimerization interface that is formed by domains D4 and D5 (Ries et al. Cancer Cell 25(6):846-59 (2014)). In some cases, the agent is selected from emactuzumab (RG7155; RO5509554), Cabiralizumab (FPA-008), LY-3022855 (IMC-CS4), AMG-820, TG-3003, MCS110, H27K15, 12-2D6, 2-4A5 (Rovida and Sbarba, J Clin Cell Immunol.6:6 (2015); Clinical Trial Study Record Nos.: NCT02760797; NCT01494688; NCT02323191; NCT01962337; NCT02471716; NCT02526017; NCT01346358; NCT02265536; NCT01444404; NCT02713529, NCT00757757; NCT02807844; NCT02435680; NCT01643850).

In some embodiments, the agent that reduces microglial cell activation is a tetracycline antibiotic. For example, the agent affects IL-1b, IL-6, TNF-α, or iNOS concentration in microglia cells (Yrjänheikki et al. PNAS 95(26): 15769-15774 (1998); Clinical Trial Study Record No: NCT01120899). In some embodiments, the agent is an opioid antagonist (Younger et al. Pain Med. 10(4):663-672 (2009.) In some embodiments, the agent reduces glutamatergic neurotransmission (US Patent Number 5,527,814). In some embodiments, the agent modulates NFkB signaling (Valera et al J. Neuroinflammation 12:93 (2015); Clinical Trial Study Record No: NCT00231140). In some embodiments, the agent targets cannabinoid receptors (Ramírez et al. J. Neurosci 25(8):1904-13(2005)). In some embodiments, the agent is selected from minocycline, naloxone, riluzole, lenalidomide, and a cannabinoid (optionally WIN55 or 212-2).

Nitric oxide production from microglia is believed, in some cases, to result in or increase neurotoxicity. In some embodiments, the agent modulates or inhibitis nitric oxide production from microglia. In some embodiments, the agent inhibits nitric oxide synthase (NOS). In some embodiments, the NOS inhibitor is Ronopterin (VAS-203), also known as 4-amino-tetrahydrobiopterin (4-ABH4). In some embodiments, the NOS inhibitor is cindunistat, A-84643, ONO-1714, L-NOARG, NCX-456, VAS-2381, GW-273629, NXN-462, CKD-712, KD-7040, or guanidinoethyldisulfide. In some embodiments, the agent is any of the inhibitors described in Höing et al., Cell Stem Cell. 2012 Nov 2;11(5):620-32.

In some embodiments, the agent blocks T cell trafficking, such as to the central nervous system. In some embodiments, blocking T cell trafficking can reduce or prevent immune cells from crossing blood vessel walls into the central nervous system, including crossing the blood-brain barrier. In some cases, activated antigen-specific T cells produce proinflammatory cytokines, including IFN-γ and TNF, upon reactivation in the CNS, leading to activation of resident cells such as microglia and astrocytes. See Kivisäkk et al., Neurology. 2009 Jun 2; 72(22): 1922-1930. Thus, in some embodiments, sequestering activated T cells from microglial cells, such as by blocking trafficking and/or inhibiting the ability of such cells to cross the blood-brain barrier, can reduce or eliminate microglial activation. In some embodiments, the agent inhibits adhesion molecules on immune cells, including T cells. In some embodiments, the agent inhibits an integrin. In some embodiments, the integrin is alpha-4 integrin. In some embodiments, the agent is natalizumab (Tysabri^{®}). In some embodiments, the agent modulates a cell surface receptor. In some embodiments, the agent modulates the sphingosine-1-phosphate (S1P) receptor, such as S1PR1 or S1PR5. In some embodiments, the agent causes the internalization of a cellular receptor, such as a sphingosine-1-phosphate (S1P) receptor, such as S1PR1 or S1PR5. In some embodiments, the agent is fingolimod (Gilenya^{®}) or ozanimod (RPC-1063).

The transcription factor NRF2 is believed to regulate the anti-oxidant response, for example, by turning on genes that contain a cis-acting element in their promoter region. An example of such an element includes an antioxidant response element (ARE). In some embodiments, the agent activates NRF2. In some embodiments, activating NRF2 in microglial cells reduces the microglial cells' responsiveness to IFN and LPS. In some embodiments, activating NRF2 inhibits, slows, or reduces demyelination, axonal loss, neuronal death, and/or oligodendrocyte death. In some embodiments, the agent upregulates the cellular cytoprotective pathway regulated by NRF2. In some embodiments, the agent that activates NRF2 is dimethyl fumarate (Tecfidera^{®}). In some embodiments, the agent is any of the inhibitors described in US patent number 8,399,514. In some embodiments, the agent is any of the inhibitors described in Höing et al., Cell Stem Cell. 2012 Nov 2;11(5):620-32.

In some embodiments, the agent that reduces microglial cell activation is (4S,4aS,5aR,12aS)-4,7-bis(dimethylamino)-3,10,12,12a-tetrahydroxy-1,11-dioxo-1.,4,4a,5,5a,6,11,12a-octahydrotetracene-2-carboxamide (Minocycline) or a pharmaceutically acceptable salt thereof or derivatives thereof. In some embodiments, the agent is any of the compounds described in US patent application publication number US20100190755. In some embodiments, the agent is the following compound: 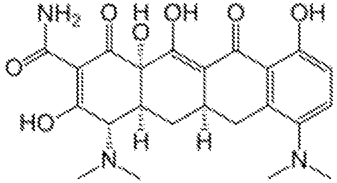 or a pharmaceutically acceptable salt thereof.

In some embodiments, the agent that reduces microglial cell activation is 3-(7-amino-3-oxo-1H-isoindol-2-yl)piperidine-2,6-dione (lenalidomide) or a pharmaceutically acceptable salt thereof or derivatives thereof. In some embodiments, the agent is the following compound: 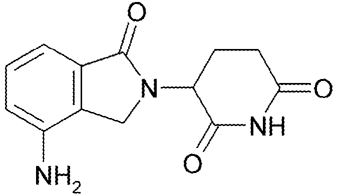 or a pharmaceutically acceptable salt thereof.

In some embodiments, the agent that reduces microglial cell activation is 4R,4aS,7aR,12bS)-4a,9-dihydroxy-3-prop-2-enyl-2,4,5,6,7a,13-hexahydro-1H-4,12-methanobenzofuro[3,2-e]isoquinoline-7-one (naloxone) or a pharmaceutically acceptable salt thereof or derivatives thereof. In some embodiments, the agent is any of the compounds described in US patent number US8247425. In some embodiments, the agent is the following compound: 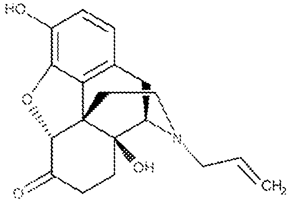 or a pharmaceutically acceptable salt thereof.

In some embodiments, the agent that reduces microglial cell activation is 2-amino-6-(trifluoromethoxy)benzothiazole, 6-(trifluoromethoxy)benzo[d]thiazol-2-amine, or 6-(trifluoromethoxy)-1,3-benzothiazol-2-amine (riluzole) or a pharmaceutically acceptable salt thereof or derivatives thereof as described in US patent number US5527814. In some embodiments, the agent is the following compound: 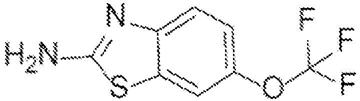 or a pharmaceutically acceptable salt thereof.

In some embodiments, the agent that reduces microglial cell activation is a modulator of a signaling pathway in microglia. In some cases, the agent reduces microglia singling. In some embodiments, the agent is a GM-CSF (CSF2) inhibitor. In other embodiments, the agent that reduces microglial cell activation is an ion channel blocker. In some specific embodiments, the agent is a calcium channel blocker. For example, in some specific examples, the agent is a dihydropyridine calcium channel blocker. In some embodiments, the agent is a microRNA inhibitor. For example, the agent targets miR-155. In some embodiments, the agent that reduces microglial cell activation is selected from MOR103, Nimodipine, IVIg, and LNA-anti-miR-155 (Butoxsky et al. Ann Neurol., 77(1):75-99 (2015) and Sanz et al., Br J Pharmacol. 167(8): 1702-1711 (2012); Winter et al., Ann Clin and Transl Neurol. 2328-9503 (2016); Clinical Trial Study Record Nos.: NCT01517282, NCT00750867).

In some embodiments, the agent that reduces microglial cell activation is 3-(2-methoxyethyl) 5-propan-2-yl 2,6-dimethyl-4-(3-nitrophenyl)-1,4-dihydropyridine-3,5-dicarboxylate (nimodipine) or a pharmaceutically acceptable salt thereof or derivatives thereof. In some embodiments, the agent is any of the inhibitors described in US patent number US3799934. In some embodiments, the agent is the following compound: 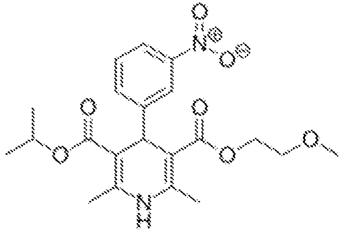 or a pharmaceutically acceptable salt thereof.

In some cases, the agent that reduces microglial cell activation is administered in a form that only affects to central nervous system and/or does not affect tumor-associated macrophages. In some embodiments, the agent promotes microglia quiescence but does not eliminate or reduce the number of microglia. In some embodiments, the method involves inhibiting microglia activity specifically in the brain such as described in Ponomarev et al., Nature Medicine, (1):64-70 (2011)

Exemplary agents that reduce microglial cell activation, and exemplary dosing regimens for administering such agents, are set forth in **Table 9** below.

| **Table 9. Exemplary microglia inhibitors and dosage regimens** | | | |
|---|---|---|---|
| **Exemplary Inhibitor** | **Type of Molecule** | **Molecular Target(s)** | **Exemplary Dosing Regimen(s)** |
| Pexidartinib (PLX3397) | small molecule | CSF1R; c-Kit; FLT3 | 200 mg tablets, twice daily for 28 days; Administer daily as split dose regimen, five dose-levels possible in dose escalation part: 400mg 5 days on 2 days off (intermittent schedule), 400 mg, 600 mg, 800 mg or 1000 mg; 1000 mg/day for 2 weeks then 800 mg/day for 22 weeks |
| Emactuzumab (RG1755; RO5509554) | monoclonal antibody | CSF1R | 100-3000 mg once every 2 weeks |
| Cabiralizumab (FPA-008) | antibody | CSF1R | Intravenous infusion over 30 minutes every 2 weeks |
| LY-3022855 (IMC-CS4) | monoclonal antibody | CSF1R | 1.25 mg/kg intravenous delivery every 2 weeks for 6 weeks |
| JNJ-40346527 | small molecule | CSF1R | 100 mg twice daily for 12 weeks; 100-1000 mg capsule daily |
| MCS110 | antibody | MCSF (CSF1) | Up to 4 doses of 10 mg/kg MCS110 administered intravenously once every 4 weeks starting at Day 1 |
| MOR103 | antibody | GM-CSF | 6 doses of 0.5-2.0 mg/kg over 70 days |
| IVIg | immunoglobulin | Unknown | Intravenous infusion of 0.4g/kg each month for 6 months |
| Minocyline | small molecule | broad spectrum antibiotic: IL-1b; IL-6, TNF-a; iNOS | Oral dose of 100 mg of minocycline twice daily for 24 months |
| Naloxone | small molecule | Opioid receptors | 4.5 mg naltrexone hydrochloride capsules once/day for 8 weeks |
| Lenalidomide/thali domide | small molecule | NFkB signaling | 100-400 mg daily |
| Riluzole | small molecule | Glutamate release by microglia | 50 mg twice daily |
| Cannabinoids/cann abidiol (*e.g.* WIN55,212-2) | small molecule | cannabinoid receptors | Orally 10 mg/kg/day for 6 weeks (average of 700 mg/day) |
| Dimethyl fumarate (Tecfidera^{®}). | small molecule | Nrf2 signaling | Starting dose of 120 mg taken orally twice/day for 7 days. Dose increased to 240 mg taken orally twice/day thereafter |
| natalizumab (Tysabri^{®}) | antibody | alpha-4 integrin | 300 mg infused intravenously over one hour, every four weeks |
| fingolimod (Gilenya^{®}) | small molecule | S1P receptors, including S1PR1 | 0.5 mg orally once-daily |
| ozanimod (RPC-1063) | small molecule | S1PR1 and S1PR5 | 0.25 mg, 0.5 mg, or 1 mg once daily |

### C. Other Agents (e.g. cytokine targeting agents)

In some embodiments, the agent, e.g. toxicity-targeting agent, that treats or ameliorates symptoms of a toxicity of immunotherapy and/or a cell therapy, such as CRS or neurotoxicity, is one that targets a cytokine, e.g., is an antagonist or inhibitor of a cytokine, such as transforming growth factor beta (TGF-beta), interleukin 6 (IL-6), interleukin 10 (IL-10), IL-2, MIP1β (CCL4), TNF alpha, IL-1, interferon gamma (IFN-gamma), or monocyte chemoattractant protein-1 (MCP-1). In some embodiments, the agent that treats or ameliorates symptoms of a toxicity of an immunotherapy and/or a cell therapy, such as CRS or neurotoxicity, is one that targets (e.g. inhibits or is an antagonist of) a cytokine receptor, such as IL-6 receptor (IL-6R), IL-2 receptor (IL-2R/CD25), MCP-1 (CCL2) receptor (CCR2 or CCR4), a TGF-beta receptor (TGF-beta I, II, or III), IFN-gamma receptor (IFNGR), MIP1β receptor (e.g., CCR5), TNF alpha receptor (e.g., TNFR1), IL-1 receptor (IL1-Rα/IL-1Rβ), or IL-10 receptor (IL-10R).

The amount of a selected agent that treats or ameliorates symptoms of a toxicity of an immunotherapy and/or a cell therapy, such as CRS or neurotoxicity to be administered to ameliorate symptoms or adverse effects of a toxicity to an immunotherapy and/or a cell therapy, such as CRS or neurotoxicity, can be determined by standard clinical techniques. Exemplary adverse events include, but are not limited to, an increase in alanine aminotransferase, an increase in aspartate aminotransferase, chills, febrile neutropenia, headache, hypotension, left ventricular dysfunction, encephalopathy, hydrocephalus, seizure, and/or tremor.

In some embodiments, the agent is administered in a dosage amount of from or from about 30 mg to or to about 5000 mg, such as from or from about 50 mg to or to about 1000 mg, from or from about 50 mg to or to about 500 mg, from or from about 50 mg to or to about 200 mg, from or from about 50 mg to or to about 100 mg, from or from about 100 mg to or to about 1000 mg, from or from about 100 mg to or to about 500 mg, from or from about 100 mg to or to about 200 mg, from or from about 200 mg to or to about 1000 mg, from or from about 200 mg to or to about 500 mg or 500 mg to or to about 1000 mg.

In some embodiments, the agent is administered from or from about 0.5 mg/kg to or to about 100 mg/kg, such as from or from about 1 mg/kg to or to about 50 mg/kg, from or from about 1 mg/kg to or to about 25 mg/kg, from or from about 1 mg/kg to or to about 10 mg/kg, from or from about 1 mg/kg to or to about 5 mg/kg, from or from about 5 mg/kg to or to about 100 mg/kg, from or from about 5 mg/kg to or to about 50 mg/kg, from or from about 5 mg/kg to or to about 25 mg/kg, from or from about 5 mg/kg to or to about 10 mg/kg, from or from about 10 mg/kg to or to about 100 mg/kg, from or from about 10 mg/kg to or to about 50 mg/kg, from or from about 10 mg/kg to or to about 25 mg/kg, from or from about 25 mg/kg to or to about 100 mg/kg, from or from about 25 mg/kg to or to about 50 mg/kg or from or from about 50 mg/kg to or to about 100 mg/kg. In some embodiments, the agent is administered in a dosage amount of from or from about 1 mg/kg to or to about 10 mg/kg, from or from about 2 mg/kg to or to about 8 mg/kg, from or from about 2 mg/kg to or to about 6 mg/kg, from or from about 2 mg/kg to or to about 4 mg/kg or 6 mg/kg to or to about 8 mg/kg, each inclusive. In some aspects, the agent is administered in a dosage amount of at least or at least about or about 1 mg/kg, 2 mg/kg, 4 mg/kg, 6 mg/kg, 8 mg/kg, 10 mg/kg or more. In some embodiments, the agent is administered at a dose of 4 mg/kg or 8 mg/kg.

In some embodiments, the agent is administered by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some embodiments, they are administered by parenteral, intrapulmonary, and intranasal, and, if desired for local treatment, intralesional administration. Parenteral infusions include intramuscular, intravenous, intraarterial, intraperitoneal, or subcutaneous administration.

In some embodiments, the amount of the agent is administered about or approximately twice daily, daily, every other day, three times a week, weekly, every other week or once a month.

In some embodiments, the agent is administered as part of a composition or formulation, such as a pharmaceutical composition or formulation as described below. Thus, in some cases, the composition comprising the agent is administered as described below. In other aspects, the agent is administered alone and may be administered by any known acceptable route of administration or by one described herein, such as with respect to compositions and pharmaceutical formulations.

In some embodiments, the agent that treats or ameliorates symptoms of a toxicity of the immunotherapy and/or cell therapy, such as CRS or neurotoxicity, is an antibody or antigen binding fragment. In some embodiments, the agent is tocilizumab, siltuximab, sarilumab, olokizumab (CDP6038), elsilimomab, ALD518/BMS-945429, sirukumab (CNTO 136), CPSI-2634, ARGX-109, FE301, or FM101.

In some embodiments, the agent is an antagonist or inhibitor of IL-6 or the IL-6 receptor (IL-6R). In some aspects, the agent is an antibody that neutralizes IL-6 activity, such as an antibody or antigen-binding fragment that binds to IL-6 or IL-6R. For example, in some embodiments, the agent is or comprises tocilizumab (atlizumab) or sarilumab, anti-IL-6R antibodies. In some embodiments, the agent is an anti-IL-6R antibody described in U.S. Patent No: 8,562,991. In some cases, the agent that targets IL-6 is an anti-IL-6 antibody, such as siltuximab, elsilimomab, ALD518/BMS-945429, sirukumab (CNTO 136), CPSI-2634, ARGX-109, FE301, FM101, or olokizumab (CDP6038). In some aspects, the agent may neutralize IL-6 activity by inhibiting the ligand-receptor interactions. The feasibility of this general type of approach has been demonstrated with a natural occurring receptor antagonist for interleukin-1. *See* Harmurn, C. H. et al., Nature (1990) 343:336-340. In some aspects, the IL-6/IL-6R antagonist or inhibitor is an IL-6 mutein, such as one described in U.S. Patent No. 5591827. In some embodiments, the agent that is an antagonist or inhibitor of IL-6/IL-6R is a small molecule, a protein or peptide, or a nucleic acid.

In some embodiments, the agent is tocilizumab. In some embodiments, tocilizumab is administered as an early intervention in accord with the provided methods, and/or with the provided articles of manufacture or compositions, at a dosage of from or from about 1 mg/kg to 12 mg/kg, such as at or about 4 mg/kg, 8 mg/kg, or 10 mg/kg. In some embodiments, tocilizumab is administered by intravenous infusion. In some embodiments, tocilizumab is administered for a persistent fever of greater than 39°C lasting 10 hours that is unresponsive to acetaminophen. In some embodiments, a second administration of tocilizumab is provided if symptoms recur after 48 hours of the initial dose.

In some embodiments, the agent is an agonist or stimulator of TGF-β or a TGF-β receptor (e.g., TGF-β receptor I, II, or III). In some aspects, the agent is an antibody that increases TGF-β activity, such as an antibody or antigen-binding fragment that binds to TGF-β or one of its receptors. In some embodiments, the agent that is an agonist or stimulator of TGF-β and/or its receptor is a small molecule, a protein or peptide, or a nucleic acid.

In some embodiments, the agent is an antagonist or inhibitor of MCP-1 (CCL2) or a MCP-1 receptor (e.g., MCP-1 receptor CCR2 or CCR4). In some aspects, the agent is an antibody that neutralizes MCP-1 activity, such as an antibody or antigen-binding fragment that binds to MCP-1 or one of its receptors (CCR2 or CCR4). In some embodiments, the MCP-1 antagonist or inhibitor is any described in Gong et al. J Exp Med. 1997 Jul 7; 186(1): 131-137 or Shahrara et al. J Immunol 2008; 180:3447-3456. In some embodiments, the agent that is an antagonist or inhibitor of MCP-1 and/or its receptor (CCR2 or CCR4) is a small molecule, a protein or peptide, or a nucleic acid.

In some embodiments, the agent is an antagonist or inhibitor of IFN-γ or an IFN-γ receptor (IFNGR). In some aspects, the agent is an antibody that neutralizes IFN-γ activity, such as an antibody or antigen-binding fragment that binds to IFN-γ or its receptor (IFNGR). In some aspects, the IFN-gamma neutralizing antibody is any described in Dobber et al. Cell Immunol. 1995 Feb;160(2):185-92 or Ozmen et al. J Immunol. 1993 Apr 1;150(7):2698-705. In some embodiments, the agent that is an antagonist or inhibitor of IFN-γ/IFNGR is a small molecule, a protein or peptide, or a nucleic acid.

In some embodiments, the agent is an antagonist or inhibitor of IL-10 or the IL-10 receptor (IL-10R). In some aspects, the agent is an antibody that neutralizes IL-10 activity, such as an antibody or antigen-binding fragment that binds to IL-10 or IL-10R. In some aspects, the IL-10 neutralizing antibody is any described in Dobber et al. Cell Immunol. 1995 Feb;160(2):185-92 or Hunter et al. J Immunol. 2005 Jun 1;174(11):7368-75. In some embodiments, the agent that is an antagonist or inhibitor of IL-10/IL-10R is a small molecule, a protein or peptide, or a nucleic acid.

In some embodiments, the agent is an antagonist or inhibitor of IL-1 or the IL-1 receptor (IL-1R). In some aspects, the agent is an IL-1 receptor antagonist, which is a modified form of IL-1R, such as anakinra (see, e.g., Fleischmann et al., (2006) Annals of the rheumatic diseases. 65(8):1006-12). In some aspects, the agent is an antibody that neutralizes IL-1 activity, such as an antibody or antigen-binding fragment that binds to IL-1 or IL-1R, such as canakinumab (see also EP 2277543). In some embodiments, the agent that is an antagonist or inhibitor of IL-1/IL-1R is a small molecule, a protein or peptide, or a nucleic acid.

In some embodiments, the agent is an antagonist or inhibitor of a tumor necrosis factor (TNF) or a tumor necrosis factor receptor (TNFR). In some aspects, the agent is an antibody that blocks TNF activity, such as an antibody or antigen-binding fragment that binds to a TNF, such as TNFα, or its receptor (TNFR, e.g., TNFRp55 or TNFRp75). In some aspects, the agent is selected from among infliximab, adalimumab, certolizumab pegol, golimumab and etanercept. In some embodiments, the agent that is an antagonist or inhibitor of TNF/TNFR is a small molecule, a protein or peptide, or a nucleic acid. In some embodiments, the agent is a small molecule that affects TNF, such as lenalidomide (see, e.g., Muller et al. (1999) Bioorganic & Medicinal Chemistry Letters. 9 (11):1625).

In some embodiments, the agent is an antagonist or inhibitor of signaling through the Janus kinase (JAK) and two Signal Transducer and Activator of Transcription (STAT) signaling cascade. JAK/STAT proteins are common components of cytokine and cytokine receptor signaling. In some embodiments, the agent that is an antagonist or inhibitor of JAK/STAT, such as ruxolitinib (see, e.g., Mesa et al. (2012) Nature Reviews Drug Discovery. 11(2):103-104), tofacitinib (also known as Xeljanz, Jakvinus tasocitinib and CP-690550), Baricitinib (also known as LY-3009104, INCB-28050), Filgotinib (G-146034, GLPG-0634), Gandotinib (LY-2784544), Lestaurtinib (CEP-701), Momelotinib (GS-0387, CYT-387), Pacritinib (SB1518), and Upadacitinib (ABT-494). In some embodiments, the agent is a small molecule, a protein or peptide, or a nucleic acid.

In some embodiments, the agent is a kinase inhibitor. In some embodiments, the agent is an inhibitor of Bruton's tyrosine kinase (BTK). In some embodiments, the inhibitor is or comprises ibrutinib or acalabrutinib (see, e.g., Barrett et al., ASH 58th Annual Meeting San Diego, CA December 3-6, 2016, Abstract 654; Ruella et al., ASH 58th Annual Meeting San Diego, CA December 3-6, 2016, Abstract 2159). In some embodiments, the agent is an inhibitor as described in U.S. Patent No. 7,514,444; 8,008,309; 8,476,284; 8,497,277; 8,697,711; 8,703,780; 8,735,403; 8,754,090; 8,754,091; 8.957,079; 8,999,999; 9,125,889; 9,181,257; or 9,296,753.

In some embodiments, a device, such as absorbent resin technology with blood or plasma filtration, can be used to reduce cytokine levels. In some embodiments, the device used to reduce cytokine levels is a physical cytokine absorber, such as an extracorporeal cytokine absorber. In some embodiments, a physical cytokine absorber can be used to eliminate cytokines from the bloodstream in an *ex vivo,* extracorporeal manner. In some embodiments, the agent is a porous polymer. In some embodiments, the agent is CytoSorb (see, e.g., Basu et al. Indian J Crit Care Med. (2014) 18(12): 822-824).

### III. RECOMBINANT ANTIGEN RECEPTORS EXPRESSED BY THE CELLS

**In** some embodiments, the cells for use in or administered in connection with the provided methods contain or are engineered to contain an engineered receptor, e.g., an engineered antigen receptor, such as a chimeric antigen receptor (CAR), or a T cell receptor (TCR). Also provided are populations of such cells, compositions containing such cells and/or enriched for such cells, such as in which cells of a certain type such as T cells or CD8⁺ or CD4⁺ cells are enriched or selected. Among the compositions are pharmaceutical compositions and formulations for administration, such as for adoptive cell therapy. Also provided are therapeutic methods for administering the cells and compositions to subjects, e.g., patients, in accord with the provided methods, and/or with the provided articles of manufacture or compositions.

**In** some embodiments, the cells include one or more nucleic acids introduced via genetic engineering, and thereby express recombinant or genetically engineered products of such nucleic acids. In some embodiments, gene transfer is accomplished by first stimulating the cells, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications.

The cells generally express recombinant receptors, such as antigen receptors including functional non-TCR antigen receptors, e.g., chimeric antigen receptors (CARs), and other antigen-binding receptors such as transgenic T cell receptors (TCRs). Also among the receptors are other chimeric receptors.

### A. Chimeric Antigen Receptors (CARs)

In some embodiments of the provided methods and uses, chimeric receptors, such as a chimeric antigen receptors, contain one or more domains that combine a ligand-binding domain (e.g. antibody or antibody fragment) that provides specificity for a desired antigen (e.g., tumor antigen) with intracellular signaling domains. In some embodiments, the intracellular signaling domain is a stimulating or an activating intracellular domain portion, such as a T cell stimulating or activating domain, providing a primary activation signal or a primary signal. In some embodiments, the intracellular signaling domain contains or additionally contains a costimulatory signaling domain to facilitate effector functions. In some embodiments, chimeric receptors when genetically engineered into immune cells can modulate T cell activity, and, in some cases, can modulate T cell differentiation or homeostasis, thereby resulting in genetically engineered cells with improved longevity, survival and/or persistence *in vivo,* such as for use in adoptive cell therapy methods.

Exemplary antigen receptors, including CARs, and methods for engineering and introducing such receptors into cells, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, 8,339,645, 8,398,282, 7,446,179, 6,410,319, 7,070,995, 7,265,209, 7,354,762, 7,446,191, 8,324,353, and 8,479,118, and European patent application number EP2537416,and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some aspects, the antigen receptors include a CAR as described in U.S. Patent No.: 7,446,190, and those described in International Patent Application Publication No.: WO/2014055668 A1. Examples of the CARs include CARs as disclosed in any of the aforementioned publications, such as WO2014031687, US 8,339,645, US 7,446,179, US 2013/0149337, U.S. Patent No.: 7,446,190, US Patent No.: 8,389,282, Kochenderfer et al., 2013, Nature Reviews Clinical Oncology, 10, 267-276 (2013); Wang et al. (2012) J. Immunother. 35(9): 689-701; and Brentjens et al., Sci Transl Med. 2013 5(177). See also WO2014031687, US 8,339,645, US 7,446,179, US 2013/0149337, U.S. Patent No.: 7,446,190, and US Patent No.: 8,389,282.

The chimeric receptors, such as CARs, generally include an extracellular antigen binding domain, such as a portion of an antibody molecule, generally a variable heavy (V_{H}) chain region and/or variable light (V_{L}) chain region of the antibody, e.g., an scFv antibody fragment.

In some embodiments, the antigen targeted by the receptor is a polypeptide. In some embodiments, it is a carbohydrate or other molecule. In some embodiments, the antigen is selectively expressed or overexpressed on cells of the disease or condition, e.g., the tumor or pathogenic cells, as compared to normal or non-targeted cells or tissues. In other embodiments, the antigen is expressed on normal cells and/or is expressed on the engineered cells.

In some embodiments, the CAR is constructed with a specificity for a particular antigen (or marker or ligand), such as an antigen expressed in a particular cell type to be targeted by adoptive therapy, *e.g.,* a cancer marker, and/or an antigen intended to induce a dampening response, such as an antigen expressed on a normal or non-diseased cell type. Thus, the CAR typically includes in its extracellular portion one or more antigen binding molecules, such as one or more antigen-binding fragment, domain, or portion, or one or more antibody variable domains, and/or antibody molecules. In some embodiments, the CAR includes an antigen-binding portion or portions of an antibody molecule, such as a single-chain antibody fragment (scFv) derived from the variable heavy (V_{H}) and variable light (V_{L}) chains of a monoclonal antibody (mAb).

In some embodiments, the antibody or antigen-binding portion thereof is expressed on cells as part of a recombinant receptor, such as an antigen receptor. Among the antigen receptors are functional non-TCR antigen receptors, such as chimeric antigen receptors (CARs). Generally, a CAR containing an antibody or antigen-binding fragment that exhibits TCR-like specificity directed against peptide-MHC complexes also may be referred to as a TCR-like CAR. In some embodiments, the extracellular antigen binding domain specific for an MHC-peptide complex of a TCR-like CAR is linked to one or more intracellular signaling components, in some aspects via linkers and/or transmembrane domain(s). In some embodiments, such molecules can typically mimic or approximate a signal through a natural antigen receptor, such as a TCR, and, optionally, a signal through such a receptor in combination with a costimulatory receptor.

In some embodiments, the recombinant receptor, such as a chimeric receptor (e.g. CAR), includes a ligand-binding domain that binds, such as specifically binds, to an antigen (or a ligand). Among the antigens targeted by the chimeric receptors are those expressed in the context of a disease, condition, or cell type to be targeted via the adoptive cell therapy. Among the diseases and conditions are proliferative, neoplastic, and malignant diseases and disorders, including cancers and tumors, including hematologic cancers, cancers of the immune system, such as lymphomas, leukemias, and/or myelomas, such as B, T, and myeloid leukemias, lymphomas, and multiple myelomas.

In some embodiments, the antigen (or a ligand) is a polypeptide. In some embodiments, it is a carbohydrate or other molecule. In some embodiments, the antigen (or a ligand) is selectively expressed or overexpressed on cells of the disease or condition, *e.g.,* the tumor or pathogenic cells, as compared to normal or non-targeted cells or tissues.

In some embodiments, the CAR contains an antibody or an antigen-binding fragment (e.g. scFv) that specifically recognizes an antigen, such as an intact antigen, expressed on the surface of a cell.

In some embodiments, the antigen targeted by the receptor is or comprises selected from among αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha (IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some embodiments include antigens associated with a B cell malignancy, such as any of a number of known B cell marker. In some embodiments, the antigen targeted by the receptor is or includes CD20, CD19, CD22, ROR1, CD45, CD21, CD5, CD33, Igkappa, Iglambda, CD79a, CD79b or CD30. In some embodiments, the disease or condition is a B cell malignancy, such as a large B cell lymphoma (e.g., DLBCL) and the antigen is CD19.

In some embodiments, the antigen is or includes a pathogen-specific or pathogen-expressed antigen. In some embodiments, the antigen is a viral antigen (such as a viral antigen from HIV, HCV, HBV, etc.), bacterial antigens, and/or parasitic antigens.

Reference to "Major histocompatibility complex" (MHC) refers to a protein, generally a glycoprotein, that contains a polymorphic peptide binding site or binding groove that can, in some cases, complex with peptide antigens of polypeptides, including peptide antigens processed by the cell machinery. In some cases, MHC molecules can be displayed or expressed on the cell surface, including as a complex with peptide, *i.e.* MHC-peptide complex, for presentation of an antigen in a conformation recognizable by an antigen receptor on T cells, such as a TCRs or TCR-like antibody. Generally, MHC class I molecules are heterodimers having a membrane spanning α chain, in some cases with three α domains, and a non-covalently associated β2 microglobulin. Generally, MHC class II molecules are composed of two transmembrane glycoproteins, α and β, both of which typically span the membrane. An MHC molecule can include an effective portion of an MHC that contains an antigen binding site or sites for binding a peptide and the sequences necessary for recognition by the appropriate antigen receptor. In some embodiments, MHC class I molecules deliver peptides originating in the cytosol to the cell surface, where a MHC-peptide complex is recognized by T cells, such as generally CD8⁺ T cells, but in some cases CD4⁺ T cells. In some embodiments, MHC class II molecules deliver peptides originating in the vesicular system to the cell surface, where they are typically recognized by CD4⁺ T cells. Generally, MHC molecules are encoded by a group of linked loci, which are collectively termed H-2 in the mouse and human leukocyte antigen (HLA) in humans. Hence, typically human MHC can also be referred to as human leukocyte antigen (HLA).

The term "MHC-peptide complex" or "peptide-MHC complex" or variations thereof, refers to a complex or association of a peptide antigen and an MHC molecule, such as, generally, by noncovalent interactions of the peptide in the binding groove or cleft of the MHC molecule. In some embodiments, the MHC-peptide complex is present or displayed on the surface of cells. In some embodiments, the MHC-peptide complex can be specifically recognized by an antigen receptor, such as a TCR, TCR-like CAR or antigen-binding portions thereof.

In some embodiments, a peptide, such as a peptide antigen or epitope, of a polypeptide can associate with an MHC molecule, such as for recognition by an antigen receptor. Generally, the peptide is derived from or based on a fragment of a longer biological molecule, such as a polypeptide or protein. In some embodiments, the peptide typically is about 8 to about 24 amino acids in length. In some embodiments, a peptide has a length of from or from about 9 to 22 amino acids for recognition in the MHC Class II complex. In some embodiments, a peptide has a length of from or from about 8 to 13 amino acids for recognition in the MHC Class I complex. In some embodiments, upon recognition of the peptide in the context of an MHC molecule, such as MHC-peptide complex, the antigen receptor, such as TCR or TCR-like CAR, produces or triggers an activation signal to the T cell that induces a T cell response, such as T cell proliferation, cytokine production, a cytotoxic T cell response or other response.

In some embodiments, a TCR-like antibody or antigen-binding portion, are known or can be produced by known methods (see e.g. US Published Application Nos. US 2002/0150914; US 2003/0223994; US 2004/0191260; US 2006/0034850; US 2007/00992530; US20090226474; US20090304679; and International PCT Publication No. WO 03/068201).

In some embodiments, an antibody or antigen-binding portion thereof that specifically binds to a MHC-peptide complex, can be produced by immunizing a host with an effective amount of an immunogen containing a specific MHC-peptide complex. In some cases, the peptide of the MHC-peptide complex is an epitope of antigen capable of binding to the MHC, such as a tumor antigen, for example a universal tumor antigen, myeloma antigen or other antigen as described below. In some embodiments, an effective amount of the immunogen is then administered to a host for eliciting an immune response, wherein the immunogen retains a three-dimensional form thereof for a period of time sufficient to elicit an immune response against the three-dimensional presentation of the peptide in the binding groove of the MHC molecule. Serum collected from the host is then assayed to determine if desired antibodies that recognize a three-dimensional presentation of the peptide in the binding groove of the MHC molecule is being produced. In some embodiments, the produced antibodies can be assessed to confirm that the antibody can differentiate the MHC-peptide complex from the MHC molecule alone, the peptide of interest alone, and a complex of MHC and irrelevant peptide. The desired antibodies can then be isolated.

In some embodiments, an antibody or antigen-binding portion thereof that specifically binds to an MHC-peptide complex can be produced by employing antibody library display methods, such as phage antibody libraries. In some embodiments, phage display libraries of mutant Fab, scFv or other antibody forms can be generated, for example, in which members of the library are mutated at one or more residues of a CDR or CDRs. See *e.g.* US published application No. US20020150914, US2014/0294841; and Cohen CJ. et al. (2003) J Mol. Recogn. 16:324-332.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, variable heavy chain (V_{H}) regions capable of specifically binding the antigen, single chain antibody fragments, including single chain variable fragments (scFv), and single domain antibodies (*e.g*., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, *e.g.*, bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD.

In some embodiments, the antigen-binding proteins, antibodies and antigen binding fragments thereof specifically recognize an antigen of a full-length antibody. In some embodiments, the heavy and light chains of an antibody can be full-length or can be an antigen-binding portion (a Fab, F(ab')2, Fv or a single chain Fv fragment (scFv)). In other embodiments, the antibody heavy chain constant region is chosen from, *e.g.,* IgG1, IgG2, IgG3, IgG4, IgM, IgA1, IgA2, IgD, and IgE, particularly chosen from, *e.g.,* IgG1, IgG2, IgG3, and IgG4, more particularly, IgG1 (*e.g.,* human IgG1). In another embodiment, the antibody light chain constant region is chosen from, *e.g.*, kappa or lambda, particularly kappa.

Among the provided antibodies are antibody fragments. An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; variable heavy chain (V_{H}) regions, single-chain antibody molecules such as scFvs and single-domain V_{H} single antibodies; and multispecific antibodies formed from antibody fragments. In particular embodiments, the antibodies are single-chain antibody fragments comprising a variable heavy chain region and/or a variable light chain region, such as scFvs.

The terms "complementarity determining region," and "CDR," synonymous with "hypervariable region" or "HVR," are known, in some cases, to refer to non-contiguous sequences of amino acids within antibody variable regions, which confer antigen specificity and/or binding affinity. In general, there are three CDRs in each heavy chain variable region (CDR-H1, CDR-H2, CDR-H3) and three CDRs in each light chain variable region (CDR-L1, CDR-L2, CDR-L3). "Framework regions" and "FR" are known, in some cases, to refer to the non-CDR portions of the variable regions of the heavy and light chains. In general, there are four FRs in each full-length heavy chain variable region (FR-H1, FR-H2, FR-H3, and FR-H4), and four FRs in each full-length light chain variable region (FR-L1, FR-L2, FR-L3, and FR-L4).

The precise amino acid sequence boundaries of a given CDR or FR can be readily determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme); Al-Lazikani et al., (1997) JMB 273,927-948 ("Chothia" numbering scheme); MacCallum et al., J. Mol. Biol. 262:732-745 (1996), "Antibody-antigen interactions: Contact analysis and binding site topography," J. Mol. Biol. 262, 732-745." ("Contact" numbering scheme); Lefranc MP et al., "IMGT unique numbering for immunoglobulin and T cell receptor variable domains and Ig superfamily V-like domains," Dev Comp Immunol, 2003 Jan;27(1):55-77 ("IMGT" numbering scheme); Honegger A and Plückthun A, "Yet another numbering scheme for immunoglobulin variable domains: an automatic modeling and analysis tool," J Mol Biol, 2001 Jun 8;309(3):657-70, ("Aho" numbering scheme); and Martin et al., "Modeling antibody hypervariable loops: a combined algorithm," PNAS, 1989, 86(23):9268-9272, ("AbM" numbering scheme).

The boundaries of a given CDR or FR may vary depending on the scheme used for identification. For example, the Kabat scheme is based on structural alignments, while the Chothia scheme is based on structural information. Numbering for both the Kabat and Chothia schemes is based upon the most common antibody region sequence lengths, with insertions accommodated by insertion letters, for example, "30a," and deletions appearing in some antibodies. The two schemes place certain insertions and deletions ("indels") at different positions, resulting in differential numbering. The Contact scheme is based on analysis of complex crystal structures and is similar in many respects to the Chothia numbering scheme. The AbM scheme is a compromise between Kabat and Chothia definitions based on that used by Oxford Molecular's AbM antibody modeling software.

**Table 10,** below, lists exemplary position boundaries of CDR-L1, CDR-L2, CDR-L3 and CDR-H1, CDR-H2, CDR-H3 as identified by Kabat, Chothia, AbM, and Contact schemes, respectively. For CDR-H1, residue numbering is listed using both the Kabat and Chothia numbering schemes. FRs are located between CDRs, for example, with FR-L1 located before CDR-L1, FR-L2 located between CDR-L1 and CDR-L2, FR-L3 located between CDR-L2 and CDR-L3 and so forth. It is noted that because the shown Kabat numbering scheme places insertions at H35A and H35B, the end of the Chothia CDR-H1 loop when numbered using the shown Kabat numbering convention varies between H32 and H34, depending on the length of the loop.

| **Table 10.** Boundaries of CDRs according to various numbering schemes. | | | | |
|---|---|---|---|---|
| **CDR** | **Kabat** | **Chothia** | **AbM** | **Contact** |
| CDR-L1 | L24--L34 | L24--L34 | L24--L34 | L30--L36 |
| CDR-L2 | L50--L56 | L50--L56 | L50--L56 | L46--L55 |
| CDR-L3 | L89--L97 | L89--L97 | L89--L97 | L89--L96 |
| CDR-H1 (Kabat Numbering¹) | H31--H35B | H26--H32.34 | H26--H35B | H30--H35B |
| CDR-H1 (Chothia Numbering²) | H31--H35 | H26--H32 | H26--H35 | H30--H35 |
| CDR-H2 | H50--H65 | H52--H56 | H50--H58 | H47--H58 |
| CDR-H3 | H95--H102 | H95--H102 | H95--H102 | H93--H101 |

1 - Kabat et al. (1991), "Sequences of Proteins of Immunological Interest," 5th Ed. Public Health Service, National Institutes of Health, Bethesda, MD
2 - Al-Lazikani et al., (1997) JMB 273,927-948

Thus, unless otherwise specified, a "CDR" or "complementary determining region," or individual specified CDRs (*e.g.,* CDR-H1, CDR-H2, CDR-H3), of a given antibody or region thereof, such as a variable region thereof, should be understood to encompass a (or the specific) complementary determining region as defined by any of the aforementioned schemes, or other known schemes. For example, where it is stated that a particular CDR (*e.g.,* a CDR-H3) contains the amino acid sequence of a corresponding CDR in a given V_{H} or V_{L} region amino acid sequence, it is understood that such a CDR has a sequence of the corresponding CDR (*e.g.,* CDR-H3) within the variable region, as defined by any of the aforementioned schemes, or other known schemes. In some embodiments, specific CDR sequences are specified. Exemplary CDR sequences of provided antibodies are described using various numbering schemes, although it is understood that a provided antibody can include CDRs as described according to any of the other aforementioned numbering schemes or other numbering schemes known to a skilled artisan.

Likewise, unless otherwise specified, a FR or individual specified FR(s) (*e.g.,* FR-H1, FR-H2, FR-H3, FR-H4), of a given antibody or region thereof, such as a variable region thereof, should be understood to encompass a (or the specific) framework region as defined by any of the known schemes. In some instances, the scheme for identification of a particular CDR, FR, or FRs or CDRs is specified, such as the CDR as defined by the Kabat, Chothia, AbM or Contact method, or other known schemes. In other cases, the particular amino acid sequence of a CDR or FR is given.

The term "variable region" or "variable domain" refers to the domain of an antibody heavy or light chain that is involved in binding the antibody to antigen. The variable domains of the heavy chain and light chain (V_{H} and V_{L}, respectively) of a native antibody generally have similar structures, with each domain comprising four conserved framework regions (FRs) and three CDRs. (See, *e.g.,* Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, *e.g.,* Portolano et al., J. Immunol. 150:880-887 (1993); Clarkson et al., Nature 352:624-628 (1991).

Single-domain antibodies are antibody fragments comprising all or a portion of the heavy chain variable domain or all or a portion of the light chain variable domain of an antibody. In certain embodiments, a single-domain antibody is a human single-domain antibody. In some embodiments, the CAR comprises an antibody heavy chain domain that specifically binds the antigen, such as a cancer marker or cell surface antigen of a cell or disease to be targeted, such as a tumor cell or a cancer cell, such as any of the target antigens described herein or known.

Antibody fragments can be made by various techniques, including but not limited to proteolytic digestion of an intact antibody as well as production by recombinant host cells. In some embodiments, the antibodies are recombinantly-produced fragments, such as fragments comprising arrangements that do not occur naturally, such as those with two or more antibody regions or chains joined by synthetic linkers, e.g., peptide linkers, and/or that are may not be produced by enzyme digestion of a naturally-occurring intact antibody. In some embodiments, the antibody fragments are scFvs.

A "humanized" antibody is an antibody in which all or substantially all CDR amino acid residues are derived from non-human CDRs and all or substantially all FR amino acid residues are derived from human FRs. A humanized antibody optionally may include at least a portion of an antibody constant region derived from a human antibody. A "humanized form" of a non-human antibody, refers to a variant of the non-human antibody that has undergone humanization, typically to reduce immunogenicity to humans, while retaining the specificity and affinity of the parental non-human antibody. In some embodiments, some FR residues in a humanized antibody are substituted with corresponding residues from a non-human antibody (*e.g*., the antibody from which the CDR residues are derived), *e.g.,* to restore or improve antibody specificity or affinity.

In some embodiments, the antigen or antigen binding domain is CD19. In some embodiments, the scFv contains a V_{H} and a V_{L} derived from an antibody or an antibody fragment specific to CD19. In some embodiments, the antibody or antibody fragment that binds CD19 is a mouse derived antibody such as FMC63 and SJ25C1. In some embodiments, the antibody or antibody fragment is a human antibody, e.g., as described in U.S. Patent Publication No. US 2016/0152723.

In some embodiments, the scFv is derived from FMC63. FMC63 generally refers to a mouse monoclonal IgG1 antibody raised against Nalm-1 and -16 cells expressing CD19 of human origin (Ling, N. R., et al. (1987). Leucocyte typing III. 302). In some embodiments, the FMC63 antibody comprises CDR-H1 and CDR-H2 set forth in SEQ ID NOS: 38 and 39, respectively, and CDR-H3 set forth in SEQ ID NO: 40 or 54; and CDR-L1 set forth in SEQ ID NO: 35 and CDR-L2 set forth in SEQ ID NO: 36 or 55 and CDR-L3 set forth in SEQ ID NO: 37 or 34. In some embodiments, the FMC63 antibody comprises the heavy chain variable region (V_{H}) comprising the amino acid sequence of SEQ ID NO: 41 and the light chain variable region (V_{L}) comprising the amino acid sequence of SEQ ID NO: 42.

In some embodiments, the scFv comprises a variable light chain containing the CDR-L1 sequence of SEQ ID NO:35, a CDR-L2 sequence of SEQ ID NO:36, and a CDR-L3 sequence of SEQ ID NO:37 and/or a variable heavy chain containing a CDR-H1 sequence of SEQ ID NO:38, a CDR-H2 sequence of SEQ ID NO:39, and a CDR-H3 sequence of SEQ ID NO:40. In some embodiments, the scFv comprises a variable heavy chain region set forth in SEQ ID NO:41 and a variable light chain region set forth in SEQ ID NO:42. In some embodiments, the variable heavy and variable light chains are connected by a linker. In some embodiments, the linker is set forth in SEQ ID NO:56. In some embodiments, the scFv comprises, in order, a V_{H}, a linker, and a V_{L}. In some embodiments, the scFv comprises, in order, a V_{L}, a linker, and a V_{H}. In some embodiments, the scFv is encoded by a sequence of nucleotides set forth in SEQ ID NO:57 or a sequence that exhibits at least 85%, 86%, 87%, 88%, 89%, 90%, 91%, 92%, 93%, 94%, 95%, 96%, 97%, 98%, or 99% sequence identity to SEQ ID NO:57. In some embodiments, the scFv comprises the sequence of amino acids set forth in SEQ ID NO:43 or a sequence that exhibits at least 85%, 86%, 87%, 88%, 89%, 90%, 91%, 92%, 93%, 94%, 95%, 96%, 97%, 98%, or 99% sequence identity to SEQ ID NO:43.

In some embodiments the scFv is derived from SJ25C1. SJ25C1 is a mouse monoclonal IgG1 antibody raised against Nalm-1 and -16 cells expressing CD19 of human origin (Ling, N. R., et al. (1987). Leucocyte typing III. 302). In some embodiments, the SJ25C1 antibody comprises CDR-H1, CDR-H2 and CDR-H3 set forth in SEQ ID NOS: 47-49, respectively, and CDR-L1, CDR-L2 and CDR-L3 sequences set forth in SEQ ID NOS: 44-46, respectively. In some embodiments, the SJ25C1 antibody comprises the heavy chain variable region (V_{H}) comprising the amino acid sequence of SEQ ID NO: 50 and the light chain variable region (V_{L}) comprising the amino acid sequence of SEQ ID NO: 51.

In some embodiments, the scFv comprises a variable light chain containing a CDR-L1 sequence of SEQ ID NO:44, a CDR-L2 sequence of SEQ ID NO: 45, and a CDR-L3 sequence of SEQ ID NO:46 and/or a variable heavy chain containing a CDR-H1 sequence of SEQ ID NO:47, a CDR-H2 sequence of SEQ ID NO:48, and a CDR-H3 sequence of SEQ ID NO:49. In some embodiments, the scFv comprises a variable heavy chain region set forth in SEQ ID NO:50 and a variable light chain region set forth in SEQ ID NO:51. In some embodiments, the variable heavy and variable light chain are connected by a linker. In some embodiments, the linker is set forth in SEQ ID NO:52. In some embodiments, the scFv comprises, in order, a V_{H}, a linker, and a V_{L}. In some embodiments, the scFv comprises, in order, a V_{L}, a linker, and a V_{H}. In some embodiments, the scFv comprises the sequence of amino acids set forth in SEQ ID NO:53 or a sequence that exhibits at least 85%, 86%, 87%, 88%, 89%, 90%, 91%, 92%, 93%, 94%, 95%, 96%, 97%, 98%, or 99% sequence identity to SEQ ID NO:53.

In some embodiments, the antigen is CD20. In some embodiments, the scFv contains a V_{H} and a V_{L} derived from an antibody or an antibody fragment specific to CD20. In some embodiments, the antibody or antibody fragment that binds CD20 is an antibody that is or is derived from Rituximab, such as is Rituximab scFv.

In some embodiments, the antigen is CD22. In some embodiments, the scFv contains a V_{H} and a V_{L} derived from an antibody or an antibody fragment specific to CD22. In some embodiments, the antibody or antibody fragment that binds CD22 is an antibody that is or is derived from m971, such as is m971 scFv.

In some embodiments, the antigen or antigen binding domain is BCMA. In some embodiments, the scFv contains a V_{H} and a V_{L} derived from an antibody or an antibody fragment specific to BCMA. In some embodiments, the antibody or antibody fragment that binds BCMA is or contains a V_{H} and a V_{L} from an antibody or antibody fragment set forth in International Patent Applications, Publication Number WO 2016/090327 and WO 2016/090320.

In some embodiments, the antigen or antigen binding domain is GPRC5D. In some embodiments, the scFv contains a V_{H} and a V_{L} derived from an antibody or an antibody fragment specific to GPRC5D. In some embodiments, the antibody or antibody fragment that binds GPRC5D is or contains a V_{H} and a V_{L} from an antibody or antibody fragment set forth in International Patent Applications, Publication Number WO 2016/090329 and WO 2016/090312.

In some embodiments, the chimeric antigen receptor includes an extracellular portion containing an antibody or antibody fragment. In some aspects, the chimeric antigen receptor includes an extracellular portion containing the antibody or fragment and an intracellular signaling domain. In some embodiments, the antibody or fragment includes an scFv. In some aspects, the chimeric antigen receptor includes an extracellular portion containing the antibody or fragment and an intracellular signaling region. In some embodiments, the intracellular signaling region comprises an intracellular signaling domain. In some embodiments, the intracellular signaling domain is or comprises a primary signaling domain, a signaling domain that is capable of inducing a primary activation signal in a T cell, a signaling domain of a T cell receptor (TCR) component, and/or a signaling domain comprising an immunoreceptor tyrosine-based activation motif (ITAM).

In some embodiments, the antibody portion of the recombinant receptor, e.g., CAR, further includes at least a portion of an immunoglobulin constant region, such as a hinge region, e.g., an IgG4 hinge region, and/or a C_{H}1/C_{L} and/or Fc region. In some embodiments, the constant region or portion is of a human IgG, such as IgG4 or IgG1. In some aspects, the portion of the constant region serves as a spacer region between the antigen-recognition component, e.g., scFv, and transmembrane domain. The spacer can be of a length that provides for increased responsiveness of the cell following antigen binding, as compared to in the absence of the spacer. Exemplary spacers include, but are not limited to, those described in Hudecek et al. (2013) Clin. Cancer Res., 19:3153, international patent application publication number WO2014031687, U.S. Patent No. 8,822,647 or published app. No. US2014/0271635.

In some embodiments, the constant region or portion is of a human IgG, such as IgG4 or IgG1. In some embodiments, the spacer has the sequence ESKYGPPCPPCP (set forth in SEQ ID NO: 1), and is encoded by the sequence set forth in SEQ ID NO: 2. In some embodiments, the spacer has the sequence set forth in SEQ ID NO: 3. In some embodiments, the spacer has the sequence set forth in SEQ ID NO: 4. In some embodiments, the constant region or portion is of IgD. In some embodiments, the spacer has the sequence set forth in SEQ ID NO: 5. In some embodiments, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 1, 3, 4 or 5. In some embodiments, the spacer has the sequence set forth in SEQ ID NOS: 26-34. In some embodiments, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 26-34.

In some embodiments, the antigen receptor comprises an intracellular domain linked directly or indirectly to the extracellular domain. In some embodiments, the chimeric antigen receptor includes a transmembrane domain linking the extracellular domain and the intracellular signaling domain. In some embodiments, the intracellular signaling domain comprises an ITAM. For example, in some aspects, the antigen recognition domain (e.g. extracellular domain) generally is linked to one or more intracellular signaling components, such as signaling components that mimic activation through an antigen receptor complex, such as a TCR complex, in the case of a CAR, and/or signal via another cell surface receptor. In some embodiments, the chimeric receptor comprises a transmembrane domain linked or fused between the extracellular domain (e.g. scFv) and intracellular signaling domain. Thus, in some embodiments, the antigen-binding component (e.g., antibody) is linked to one or more transmembrane and intracellular signaling domains.

In one embodiment, a transmembrane domain that naturally is associated with one of the domains in the receptor, e.g., CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some embodiments is derived either from a natural or from a synthetic source. Where the source is natural, the domain in some aspects is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154. Alternatively the transmembrane domain in some embodiments is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain. In some embodiments, the linkage is by linkers, spacers, and/or transmembrane domain(s). In some aspects, the transmembrane domain contains a transmembrane portion of CD28.

In some embodiments, the extracellular domain and transmembrane domain can be linked directly or indirectly. In some embodiments, the extracellular domain and transmembrane are linked by a spacer, such as any described herein. In some embodiments, the receptor contains extracellular portion of the molecule from which the transmembrane domain is derived, such as a CD28 extracellular portion.

Among the intracellular signaling domains are those that mimic or approximate a signal through a natural antigen receptor, a signal through such a receptor in combination with a costimulatory receptor, and/or a signal through a costimulatory receptor alone. In some embodiments, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

T cell activation is in some aspects described as being mediated by two classes of cytoplasmic signaling sequences: those that initiate antigen-dependent primary activation through the TCR (primary cytoplasmic signaling sequences), and those that act in an antigen-independent manner to provide a secondary or co-stimulatory signal (secondary cytoplasmic signaling sequences). In some aspects, the CAR includes one or both of such signaling components.

The receptor, e.g., the CAR, generally includes at least one intracellular signaling component or components. In some aspects, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from CD3 zeta chain, FcR gamma, CD3 gamma, CD3 delta and CD3 epsilon. In some embodiments, cytoplasmic signaling molecule(s) in the CAR contain(s) a cytoplasmic signaling domain, portion thereof, or sequence derived from CD3 zeta.

In some embodiments, the receptor includes an intracellular component of a TCR complex, such as a TCR CD3 chain that mediates T-cell activation and cytotoxicity, e.g., CD3 zeta chain. Thus, in some aspects, the antigen-binding portion is linked to one or more cell signaling modules. In some embodiments, cell signaling modules include CD3 transmembrane domain, CD3 intracellular signaling domains, and/or other CD transmembrane domains. In some embodiments, the receptor, e.g., CAR, further includes a portion of one or more additional molecules such as Fc receptor γ, CD8, CD4, CD25, or CD16. For example, in some aspects, the CAR or other chimeric receptor includes a chimeric molecule between CD3-zeta (CD3-ζ) or Fc receptor γ and CD8, CD4, CD25 or CD16.

In some embodiments, upon ligation of the CAR or other chimeric receptor, the cytoplasmic domain or intracellular signaling domain of the receptor activates at least one of the normal effector functions or responses of the immune cell, e.g., T cell engineered to express the CAR. For example, in some contexts, the CAR induces a function of a T cell such as cytolytic activity or T-helper activity, such as secretion of cytokines or other factors. In some embodiments, a truncated portion of an intracellular signaling domain of an antigen receptor component or costimulatory molecule is used in place of an intact immunostimulatory chain, for example, if it transduces the effector function signal. In some embodiments, the intracellular signaling domain or domains include the cytoplasmic sequences of the T cell receptor (TCR), and in some aspects also those of co-receptors that in the natural context act in concert with such receptors to initiate signal transduction following antigen receptor engagement.

In the context of a natural TCR, full activation generally requires not only signaling through the TCR, but also a costimulatory signal. Thus, in some embodiments, to promote full activation, a component for generating secondary or co-stimulatory signal is also included in the CAR. In other embodiments, the CAR does not include a component for generating a costimulatory signal. In some aspects, an additional CAR is expressed in the same cell and provides the component for generating the secondary or costimulatory signal.

In some embodiments, the chimeric antigen receptor contains an intracellular domain of a T cell costimulatory molecule. In some embodiments, the CAR includes a signaling domain and/or transmembrane portion of a costimulatory receptor, such as CD28, 4-1BB, OX40, DAP10, and ICOS. In some aspects, the same CAR includes both the activating and costimulatory components. In some embodiments, the chimeric antigen receptor contains an intracellular domain derived from a T cell costimulatory molecule or a functional variant thereof, such as between the transmembrane domain and intracellular signaling domain. In some aspects, the T cell costimulatory molecule is CD28 or 41BB.

In some embodiments, the activating domain is included within one CAR, whereas the costimulatory component is provided by another CAR recognizing another antigen. In some embodiments, the CARs include activating or stimulatory CARs, costimulatory CARs, both expressed on the same cell (see WO2014/055668). In some aspects, the cells include one or more stimulatory or activating CAR and/or a costimulatory CAR. In some embodiments, the cells further include inhibitory CARs (iCARs, see Fedorov et al., Sci. Transl. Medicine, 5(215) (December, 2013), such as a CAR recognizing an antigen other than the one associated with and/or specific for the disease or condition whereby an activating signal delivered through the disease-targeting CAR is diminished or inhibited by binding of the inhibitory CAR to its ligand, e.g., to reduce off-target effects.

In some embodiments, the two receptors induce, respectively, an activating and an inhibitory signal to the cell, such that ligation of one of the receptor to its antigen activates the cell or induces a response, but ligation of the second inhibitory receptor to its antigen induces a signal that suppresses or dampens that response. Examples are combinations of activating CARs and inhibitory CARs (iCARs). Such a strategy may be used, for example, to reduce the likelihood of off-target effects in the context in which the activating CAR binds an antigen expressed in a disease or condition but which is also expressed on normal cells, and the inhibitory receptor binds to a separate antigen which is expressed on the normal cells but not cells of the disease or condition.

In some aspects, the chimeric receptor is or includes an inhibitory CAR (e.g. iCAR) and includes intracellular components that dampen or suppress an immune response, such as an ITAM- and/or co stimulatory-promoted response in the cell. Exemplary of such intracellular signaling components are those found on immune checkpoint molecules, including PD-1, CTLA4, LAG3, BTLA, OX2R, TIM-3, TIGIT, LAIR-1, PGE2 receptors, EP2/4 Adenosine receptors including A2AR. In some aspects, the engineered cell includes an inhibitory CAR including a signaling domain of or derived from such an inhibitory molecule, such that it serves to dampen the response of the cell, for example, that induced by an activating and/or costimulatory CAR.

In certain embodiments, the intracellular signaling domain comprises a CD28 transmembrane and signaling domain linked to a CD3 (e.g., CD3-zeta) intracellular domain. In some embodiments, the intracellular signaling domain comprises a chimeric CD28 and CD137 (4-1BB, TNFRSF9) co-stimulatory domains, linked to a CD3 zeta intracellular domain.

In some embodiments, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some embodiments, the antigen receptor further includes a marker and/or cells expressing the CAR or other antigen receptor further includes a surrogate marker, such as a cell surface marker, which may be used to confirm transduction or engineering of the cell to express the receptor. In some aspects, the marker includes all or part (e.g., truncated form) of CD34, a NGFR, or epidermal growth factor receptor, such as truncated version of such a cell surface receptor (e.g., tEGFR). In some embodiments, the nucleic acid encoding the marker is operably linked to a polynucleotide encoding for a linker sequence, such as a cleavable linker sequence, e.g., T2A. For example, a marker, and optionally a linker sequence, can be any as disclosed in published patent application No. WO2014031687. For example, the marker can be a truncated EGFR (tEGFR) that is, optionally, linked to a linker sequence, such as a T2A cleavable linker sequence.

An exemplary polypeptide for a truncated EGFR (e.g. tEGFR) comprises the sequence of amino acids set forth in SEQ ID NO: 7 or 16 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 16. An exemplary T2A linker sequence comprises the sequence of amino acids set forth in SEQ ID NO: 6 or 17 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 6 or 17.

In some embodiments, the marker is a molecule, e.g., cell surface protein, not naturally found on T cells or not naturally found on the surface of T cells, or a portion thereof. In some embodiments, the molecule is a non-self molecule, e.g., non-self protein, i.e., one that is not recognized as "self" by the immune system of the host into which the cells will be adoptively transferred.

In some embodiments, the marker serves no therapeutic function and/or produces no effect other than to be used as a marker for genetic engineering, e.g., for selecting cells successfully engineered. In other embodiments, the marker may be a therapeutic molecule or molecule otherwise exerting some desired effect, such as a ligand for a cell to be encountered in vivo, such as a costimulatory or immune checkpoint molecule to enhance and/or dampen responses of the cells upon adoptive transfer and encounter with ligand.

In some cases, CARs are referred to as first, second, and/or third generation CARs. In some aspects, a first generation CAR is one that solely provides a CD3-chain induced signal upon antigen binding; in some aspects, a second-generation CARs is one that provides such a signal and costimulatory signal, such as one including an intracellular signaling domain from a costimulatory receptor such as CD28 or CD137; in some aspects, a third generation CAR is one that includes multiple costimulatory domains of different costimulatory receptors.

For example, in some embodiments, the CAR contains an antibody, e.g., an antibody fragment, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some embodiments, the CAR contains an antibody, e.g., antibody fragment, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such embodiments, the receptor further includes a spacer containing a portion of an Ig molecule, such as a human Ig molecule, such as an Ig hinge, e.g. an IgG4 hinge, such as a hinge-only spacer.

In some embodiments, the transmembrane domain of the recombinant receptor, e.g., the CAR, is or includes a transmembrane domain of human CD28 (e.g. Accession No. P01747.1) or variant thereof, such as a transmembrane domain that comprises the sequence of amino acids set forth in SEQ ID NO: 8 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 8; in some embodiments, the transmembrane-domain containing portion of the recombinant receptor comprises the sequence of amino acids set forth in SEQ ID NO: 9 or a sequence of amino acids having at least at or about 85%, 86%, 87%, 88%, 89%, 90%, 91%, 92%, 93%, 94%, 95%, 96%, 97%, 98%, 99% or more sequence identity thereto.

In some embodiments, the intracellular signaling component(s) of the recombinant receptor, *e.g.* the CAR, contains an intracellular costimulatory signaling domain of human CD28 or a functional variant or portion thereof, such as a domain with an LL to GG substitution at positions 186-187 of a native CD28 protein. For example, the intracellular signaling domain can comprise the sequence of amino acids set forth in SEQ ID NO: 10 or 11 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 10 or 11. In some embodiments, the intracellular domain comprises an intracellular costimulatory signaling domain of 4-1BB (e.g. (Accession No. Q07011.1) or functional variant or portion thereof, such as the sequence of amino acids set forth in SEQ ID NO: 12 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 12.

In some embodiments, the intracellular signaling domain of the recombinant receptor, *e.g.* the CAR, comprises a human CD3 zeta stimulatory signaling domain or functional variant thereof, such as an 112 AA cytoplasmic domain of isoform 3 of human CD3ζ (Accession No.: P20963.2) or a CD3 zeta signaling domain as described in U.S. Patent No.: 7,446,190 or U.S. Patent No. 8,911,993. For example, in some embodiments, the intracellular signaling domain comprises the sequence of amino acids as set forth in SEQ ID NO: 13, 14 or 15 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 13, 14 or 15.

In some aspects, the spacer contains only a hinge region of an IgG, such as only a hinge of IgG4 or IgG1, such as the hinge only spacer set forth in SEQ ID NO: 1. In other embodiments, the spacer is or contains an Ig hinge, e.g., an IgG4-derived hinge, optionally linked to a C_{H}2 and/or C_{H}3 domains. In some embodiments, the spacer is an Ig hinge, e.g., an IgG4 hinge, linked to C_{H}2 and C_{H}3 domains, such as set forth in SEQ ID NO: 4. In some embodiments, the spacer is an Ig hinge, e.g., an IgG4 hinge, linked to a C_{H}3 domain only, such as set forth in SEQ ID NO: 3. In some embodiments, the spacer is or comprises a glycine-serine rich sequence or other flexible linker such as known flexible linkers.

For example, in some embodiments, the CAR includes an antibody such as an antibody fragment, including scFvs, a spacer, such as a spacer containing a portion of an immunoglobulin molecule, such as a hinge region and/or one or more constant regions of a heavy chain molecule, such as an Ig-hinge containing spacer, a transmembrane domain containing all or a portion of a CD28-derived transmembrane domain, a CD28-derived intracellular signaling domain, and a CD3 zeta signaling domain. In some embodiments, the CAR includes an antibody or fragment, such as scFv, a spacer such as any of the Ig-hinge containing spacers, a CD28-derived transmembrane domain, a 4-1BB-derived intracellular signaling domain, and a CD3 zeta-derived signaling domain.

In particular embodiments, the CAR is a CD19-directed CAR containing an scFv antigen-binding domain from FMC63; a immunoglobulin hinge spacer, a transmembrane domain, and an intracellular signaling domain containing a costimulatory signaling region that is a signaling domain of 4-1BB and a signaling domain of a CD3-zeta (CD3ζ) chain. In some embodiments, the scFv contains the sequence set forth in SEQ ID NO::43. In some embodiments, the scFv ha a VL having CDRs having an amino acid sequences RASQDISKYLN (SEQ ID NO: 35), an amino acid sequence of SRLHSGV (SEQ ID NO: 36), and an amino acid sequence of GNTLPYTFG (SEQ ID NO: 37); and a VH with CDRs having an amino acid sequence of DYGVS (SEQ ID NO: 38), an amino acid sequence of VIWGSETTYYNSALKS (SEQ ID NO: 39) and YAMDYWG (SEQ ID NO: 40)). In some embodiments, the transmembrane domain has the sequence set forth in SQ ID NO:8. In some embodiments, the transmembrane domain has a sequence that has at least 85%, 86%, 87%, 88%, 89%, 90%, 91%, 92%, 93%, 94%, 95%, 96%, 97%, 98%, 99% or more sequence identity to SEQ ID NO:8. In som embodiments, the 4-1BB costimulatory signaling domain has the sequence set forth in SEQ ID NO:12. In some embodiments, the 4-1BB costimulatory signaling domain has a sequence at least 85%, 86%, 87%, 88%, 89%, 90%, 91%, 92%, 93%, 94%, 95%, 96%, 97%, 98%, 99% or more sequence identity to SEQ ID NO:12. In some embodiments, the CD3-zeta domain has the sequence set forth in SEQ ID NO: 13. In some embodiments, the CD3zeta signaling domain has a sequence having at least 85%, 86%, 87%, 88%, 89%, 90%, 91%, 92%, 93%, 94%, 95%, 96%, 97%, 98%, 99% or more sequence identity thereto. In some embodiments, the CD19-directed CAR binds to CD19 and mediates cytokine production and/or cytotoxic activity against CD19+ target cells when expressed in a T cell and stimulated via the CAR, such as by binding to CD19.

In some embodiments, nucleic acid molecules encoding such CAR constructs further includes a sequence encoding a T2A ribosomal skip element and/or a tEGFR sequence, e.g., downstream of the sequence encoding the CAR. In some embodiments, the sequence encodes a T2A ribosomal skip element set forth in SEQ ID NO: 6 or 17, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 6 or 17. In some embodiments, T cells expressing an antigen receptor (e.g. CAR) can also be generated to express a truncated EGFR (EGFRt) as a non-immunogenic selection epitope (e.g. by introduction of a construct encoding the CAR and EGFRt separated by a T2A ribosome switch to express two proteins from the same construct), which then can be used as a marker to detect such cells (see e.g. U.S. Patent No. 8,802,374). In some embodiments, the sequence encodes an tEGFR sequence set forth in SEQ ID NO: 7 or 16, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 16. In some cases, the peptide, such as T2A, can cause the ribosome to skip (ribosome skipping) synthesis of a peptide bond at the C-terminus of a 2A element, leading to separation between the end of the 2A sequence and the next peptide downstream (*see,* for example, de Felipe. Genetic Vaccines and Ther. 2:13 (2004) and deFelipe et al. Traffic 5:616-626 (2004)). Many 2A elements are known. Examples of 2A sequences that can be used in the methods and nucleic acids disclosed herein, without limitation, 2A sequences from the foot-and-mouth disease virus (F2A, *e.g.,* SEQ ID NO: 21), equine rhinitis A virus (E2A, *e.g.,* SEQ ID NO: 20), Thosea asigna virus (T2A, *e.g.,* SEQ ID NO: 6 or 17), and porcine teschovirus-1 (P2A, *e.g.,* SEQ ID NO: 18 or 19) as described in U.S. Patent Publication No. 20070116690.

The recombinant receptors, such as CARs, expressed by the cells administered to the subject generally recognize or specifically bind to a molecule that is expressed in, associated with, and/or specific for the disease or condition or cells thereof being treated. Upon specific binding to the molecule, e.g., antigen, the receptor generally delivers an immunostimulatory signal, such as an ITAM-transduced signal, into the cell, thereby promoting an immune response targeted to the disease or condition. For example, in some embodiments, the cells express a CAR that specifically binds to an antigen expressed by a cell or tissue of the disease or condition or associated with the disease or condition.

### B. T Cell Receptors (TCRs)

In some embodiments, engineered cells, such as T cells, used in connection with the provided methods, uses, articles of manufacture or compositions are cells that express a T cell receptor (TCR) or antigen-binding portion thereof that recognizes an peptide epitope or T cell epitope of a target polypeptide, such as an antigen of a tumor, viral or autoimmune protein.

In some embodiments, a "T cell receptor" or "TCR" is a molecule that contains a variable α and β chains (also known as TCRα and TCRβ, respectively) or a variable γ and δ chains (also known as TCRα and TCRβ, respectively), or antigen-binding portions thereof, and which is capable of specifically binding to a peptide bound to an MHC molecule. In some embodiments, the TCR is in the αβ form. Typically, TCRs that exist in αβ and γδ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules.

Unless otherwise stated, the term "TCR" should be understood to encompass full TCRs as well as antigen-binding portions or antigen-binding fragments thereof. In some embodiments, the TCR is an intact or full-length TCR, including TCRs in the αβ form or γδ form. In some embodiments, the TCR is an antigen-binding portion that is less than a full-length TCR but that binds to a specific peptide bound in an MHC molecule, such as binds to an MHC-peptide complex. In some cases, an antigen-binding portion or fragment of a TCR can contain only a portion of the structural domains of a full-length or intact TCR, but yet is able to bind the peptide epitope, such as MHC-peptide complex, to which the full TCR binds. In some cases, an antigen-binding portion contains the variable domains of a TCR, such as variable α chain and variable β chain of a TCR, sufficient to form a binding site for binding to a specific MHC-peptide complex. Generally, the variable chains of a TCR contain complementarity determining regions involved in recognition of the peptide, MHC and/or MHC-peptide complex.

In some embodiments, the variable domains of the TCR contain hypervariable loops, or complementarity determining regions (CDRs), which generally are the primary contributors to antigen recognition and binding capabilities and specificity. In some embodiments, a CDR of a TCR or combination thereof forms all or substantially all of the antigen-binding site of a given TCR molecule. The various CDRs within a variable region of a TCR chain generally are separated by framework regions (FRs), which generally display less variability among TCR molecules as compared to the CDRs (see, e.g., Jores et al., Proc. Nat'l Acad. Sci. U.S.A. 87:9138, 1990; Chothia et al., EMBO J. 7:3745, 1988; see also Lefranc et al., Dev. Comp. Immunol. 27:55, 2003). In some embodiments, CDR3 is the main CDR responsible for antigen binding or specificity, or is the most important among the three CDRs on a given TCR variable region for antigen recognition, and/or for interaction with the processed peptide portion of the peptide-MHC complex. In some contexts, the CDR1 of the alpha chain can interact with the N-terminal part of certain antigenic peptides. In some contexts, CDR1 of the beta chain can interact with the C-terminal part of the peptide. In some contexts, CDR2 contributes most strongly to or is the primary CDR responsible for the interaction with or recognition of the MHC portion of the MHC-peptide complex. In some embodiments, the variable region of the β-chain can contain a further hypervariable region (CDR4 or HVR4), which generally is involved in superantigen binding and not antigen recognition (Kotb (1995) Clinical Microbiology Reviews, 8:411-426).

In some embodiments, a TCR also can contain a constant domain, a transmembrane domain and/or a short cytoplasmic tail (see, e.g., Janeway et al., Immunobiology: The Immune System in Health and Disease, 3rd Ed., Current Biology Publications, p. 4:33, 1997). In some aspects, each chain of the TCR can possess one N-terminal immunoglobulin variable domain, one immunoglobulin constant domain, a transmembrane region, and a short cytoplasmic tail at the C-terminal end. In some embodiments, a TCR is associated with invariant proteins of the CD3 complex involved in mediating signal transduction.

In some embodiments, a TCR chain contains one or more constant domain. For example, the extracellular portion of a given TCR chain (e.g., α-chain or β-chain) can contain two immunoglobulin-like domains, such as a variable domain (e.g., Vα or Vβ; typically amino acids 1 to 116 based on Kabat numbering Kabat et al., "Sequences of Proteins of Immunological Interest, US Dept. Health and Human Services, Public Health Service National Institutes of Health, 1991, 5th ed.) and a constant domain (e.g., α-chain constant domain or Cα, typically positions 117 to 259 of the chain based on Kabat numbering or β chain constant domain or C_{β}, typically positions 117 to 295 of the chain based on Kabat) adjacent to the cell membrane. For example, in some cases, the extracellular portion of the TCR formed by the two chains contains two membrane-proximal constant domains, and two membrane-distal variable domains, which variable domains each contain CDRs. The constant domain of the TCR may contain short connecting sequences in which a cysteine residue forms a disulfide bond, thereby linking the two chains of the TCR. In some embodiments, a TCR may have an additional cysteine residue in each of the α and β chains, such that the TCR contains two disulfide bonds in the constant domains.

In some embodiments, the TCR chains contain a transmembrane domain. In some embodiments, the transmembrane domain is positively charged. In some cases, the TCR chain contains a cytoplasmic tail. In some cases, the structure allows the TCR to associate with other molecules like CD3 and subunits thereof. For example, a TCR containing constant domains with a transmembrane region may anchor the protein in the cell membrane and associate with invariant subunits of the CD3 signaling apparatus or complex. The intracellular tails of CD3 signaling subunits (e.g. CD3γ, CD3δ, CD3ε and CD3ζ chains) contain one or more immunoreceptor tyrosine-based activation motif or ITAM that are involved in the signaling capacity of the TCR complex.

In some embodiments, the TCR may be a heterodimer of two chains α and β (or optionally γ and δ) or it may be a single chain TCR construct. In some embodiments, the TCR is a heterodimer containing two separate chains (α and β chains or γ and δ chains) that are linked, such as by a disulfide bond or disulfide bonds.

In some embodiments, the TCR can be generated from a known TCR sequence(s), such as sequences of Vα,β chains, for which a substantially full-length coding sequence is readily available. Methods for obtaining full-length TCR sequences, including V chain sequences, from cell sources are well known. In some embodiments, nucleic acids encoding the TCR can be obtained from a variety of sources, such as by polymerase chain reaction (PCR) amplification of TCR-encoding nucleic acids within or isolated from a given cell or cells, or synthesis of publicly available TCR DNA sequences.

In some embodiments, the TCR is obtained from a biological source, such as from cells such as from a T cell (e.g. cytotoxic T cell), T-cell hybridomas or other publicly available source. In some embodiments, the T-cells can be obtained from *in vivo* isolated cells. In some embodiments, the TCR is a thymically selected TCR. In some embodiments, the TCR is a neoepitope-restricted TCR. In some embodiments, the T- cells can be a cultured T-cell hybridoma or clone. In some embodiments, the TCR or antigen-binding portion thereof or antigen-binding fragment thereof can be synthetically generated from knowledge of the sequence of the TCR.

In some embodiments, the TCR is generated from a TCR identified or selected from screening a library of candidate TCRs against a target polypeptide antigen, or target T cell epitope thereof. TCR libraries can be generated by amplification of the repertoire of Vα and Vβ from T cells isolated from a subject, including cells present in PBMCs, spleen or other lymphoid organ. In some cases, T cells can be amplified from tumor-infiltrating lymphocytes (TILs). In some embodiments, TCR libraries can be generated from CD4⁺ or CD8⁺ cells. In some embodiments, the TCRs can be amplified from a T cell source of a normal of healthy subject, i.e. normal TCR libraries. In some embodiments, the TCRs can be amplified from a T cell source of a diseased subject, i.e. diseased TCR libraries. In some embodiments, degenerate primers are used to amplify the gene repertoire of Vα and Vβ, such as by RT-PCR in samples, such as T cells, obtained from humans. In some embodiments, scTv libraries can be assembled from naive Vα and Vβ libraries in which the amplified products are cloned or assembled to be separated by a linker. Depending on the source of the subject and cells, the libraries can be HLA allele-specific. Alternatively, in some embodiments, TCR libraries can be generated by mutagenesis or diversification of a parent or scaffold TCR molecule. In some aspects, the TCRs are subjected to directed evolution, such as by mutagenesis, e.g., of the α or β chain. In some aspects, particular residues within CDRs of the TCR are altered. In some embodiments, selected TCRs can be modified by affinity maturation. In some embodiments, antigen-specific T cells may be selected, such as by screening to assess CTL activity against the peptide. In some aspects, TCRs, e.g. present on the antigen-specific T cells, may be selected, such as by binding activity, e.g., particular affinity or avidity for the antigen.

In some embodiments, the TCR or antigen-binding portion thereof is one that has been modified or engineered. In some embodiments, directed evolution methods are used to generate TCRs with altered properties, such as with higher affinity for a specific MHC-peptide complex. In some embodiments, directed evolution is achieved by display methods including, but not limited to, yeast display (Holler et al. (2003) Nat Immunol, 4, 55-62; Holler et al. (2000) Proc Natl Acad Sci U S A, 97, 5387-92), phage display (Li et al. (2005) Nat Biotechnol, 23, 349-54), or T cell display (Chervin et al. (2008) J Immunol Methods, 339, 175-84). In some embodiments, display approaches involve engineering, or modifying, a known, parent or reference TCR. For example, in some cases, a wild-type TCR can be used as a template for producing mutagenized TCRs in which in one or more residues of the CDRs are mutated, and mutants with an desired altered property, such as higher affinity for a desired target antigen, are selected.

In some embodiments, peptides of a target polypeptide for use in producing or generating a TCR of interest are known or can be readily identified. In some embodiments, peptides suitable for use in generating TCRs or antigen-binding portions can be determined based on the presence of an HLA-restricted motif in a target polypeptide of interest, such as a target polypeptide described below. In some embodiments, peptides are identified using available computer prediction models. In some embodiments, for predicting MHC class I binding sites, such models include, but are not limited to, ProPred1 (Singh and Raghava (2001) Bioinformatics 17(12):1236-1237, and SYFPEITHI (see Schuler et al. (2007) Immunoinformatics Methods in Molecular Biology, 409(1): 75-93 2007). In some embodiments, the MHC-restricted epitope is HLA-A0201, which is expressed in approximately 39-46% of all Caucasians and therefore, represents a suitable choice of MHC antigen for use preparing a TCR or other MHC-peptide binding molecule.

HLA-A0201-binding motifs and the cleavage sites for proteasomes and immune-proteasomes using computer prediction models are known. For predicting MHC class I binding sites, such models include, but are not limited to, ProPred1 (described in more detail in Singh and Raghava, ProPred: prediction of HLA-DR binding sites. BIOINFORMATICS 17(12):1236-1237 2001), and SYFPEITHI (see Schuler et al. SYFPEITHI, Database for Searching and T-Cell Epitope Prediction. in Immunoinformatics Methods in Molecular Biology, vol 409(1): 75-93 2007)

In some embodiments, the TCR or antigen binding portion thereof may be a recombinantly produced natural protein or mutated form thereof in which one or more property, such as binding characteristic, has been altered. In some embodiments, a TCR may be derived from one of various animal species, such as human, mouse, rat, or other mammal. A TCR may be cell-bound or in soluble form. In some embodiments, for purposes of the provided methods, the TCR is in cell-bound form expressed on the surface of a cell.

In some embodiments, the TCR is a full-length TCR. In some embodiments, the TCR is an antigen-binding portion. In some embodiments, the TCR is a dimeric TCR (dTCR). In some embodiments, the TCR is a single-chain TCR (sc-TCR). In some embodiments, a dTCR or scTCR have the structures as described in WO 03/020763, WO 04/033685, WO2011/044186.

In some embodiments, the TCR contains a sequence corresponding to the transmembrane sequence. In some embodiments, the TCR does contain a sequence corresponding to cytoplasmic sequences. In some embodiments, the TCR is capable of forming a TCR complex with CD3. In some embodiments, any of the TCRs, including a dTCR or scTCR, can be linked to signaling domains that yield an active TCR on the surface of a T cell. In some embodiments, the TCR is expressed on the surface of cells.

In some embodiments a dTCR contains a first polypeptide wherein a sequence corresponding to a TCR α chain variable region sequence is fused to the N terminus of a sequence corresponding to a TCR α chain constant region extracellular sequence, and a second polypeptide wherein a sequence corresponding to a TCR β chain variable region sequence is fused to the N terminus a sequence corresponding to a TCR β chain constant region extracellular sequence, the first and second polypeptides being linked by a disulfide bond. In some embodiments, the bond can correspond to the native interchain disulfide bond present in native dimeric αβ TCRs. In some embodiments, the interchain disulfide bonds are not present in a native TCR. For example, in some embodiments, one or more cysteines can be incorporated into the constant region extracellular sequences of dTCR polypeptide pair. In some cases, both a native and a non-native disulfide bond may be desirable. In some embodiments, the TCR contains a transmembrane sequence to anchor to the membrane.

In some embodiments, a dTCR contains a TCR α chain containing a variable α domain, a constant α domain and a first dimerization motif attached to the C-terminus of the constant α domain, and a TCR β chain comprising a variable β domain, a constant β domain and a first dimerization motif attached to the C-terminus of the constant β domain, wherein the first and second dimerization motifs easily interact to form a covalent bond between an amino acid in the first dimerization motif and an amino acid in the second dimerization motif linking the TCR α chain and TCR β chain together.

In some embodiments, the TCR is a scTCR. Typically, a scTCR can be generated using methods known, See e.g., Soo Hoo, W. F. et al. PNAS (USA) 89, 4759 (1992); Wülfing, C. and Plückthun, A., J. Mol. Biol. 242, 655 (1994); Kurucz, I. et al. PNAS (USA) 90 3830 (1993); International published PCT Nos. WO 96/13593, WO 96/18105, WO99/60120, WO99/18129, WO 03/020763, WO2011/044186; and Schlueter, C. J. et al. J. Mol. Biol. 256, 859 (1996). In some embodiments, a scTCR contains an introduced non-native disulfide interchain bond to facilitate the association of the TCR chains (see e.g. International published PCT No. WO 03/020763). In some embodiments, a scTCR is a non-disulfide linked truncated TCR in which heterologous leucine zippers fused to the C-termini thereof facilitate chain association (see e.g. International published PCT No. WO99/60120). In some embodiments, a scTCR contain a TCRα variable domain covalently linked to a TCRβ variable domain via a peptide linker (see e.g., International published PCT No. WO99/18129).

In some embodiments, a scTCR contains a first segment constituted by an amino acid sequence corresponding to a TCR α chain variable region, a second segment constituted by an amino acid sequence corresponding to a TCR β chain variable region sequence fused to the N terminus of an amino acid sequence corresponding to a TCR β chain constant domain extracellular sequence, and a linker sequence linking the C terminus of the first segment to the N terminus of the second segment.

In some embodiments, a scTCR contains a first segment constituted by an α chain variable region sequence fused to the N terminus of an α chain extracellular constant domain sequence, and a second segment constituted by a β chain variable region sequence fused to the N terminus of a sequence β chain extracellular constant and transmembrane sequence, and, optionally, a linker sequence linking the C terminus of the first segment to the N terminus of the second segment.

In some embodiments, a scTCR contains a first segment constituted by a TCR β chain variable region sequence fused to the N terminus of a β chain extracellular constant domain sequence, and a second segment constituted by an α chain variable region sequence fused to the N terminus of a sequence α chain extracellular constant and transmembrane sequence, and, optionally, a linker sequence linking the C terminus of the first segment to the N terminus of the second segment.

In some embodiments, the linker of a scTCRs that links the first and second TCR segments can be any linker capable of forming a single polypeptide strand, while retaining TCR binding specificity. In some embodiments, the linker sequence may, for example, have the formula -P-AA-P-wherein P is proline and AA represents an amino acid sequence wherein the amino acids are glycine and serine. In some embodiments, the first and second segments are paired so that the variable region sequences thereof are orientated for such binding. Hence, in some cases, the linker has a sufficient length to span the distance between the C terminus of the first segment and the N terminus of the second segment, or vice versa, but is not too long to block or reduces bonding of the scTCR to the target ligand. In some embodiments, the linker can contain from or from about 10 to 45 amino acids, such as 10 to 30 amino acids or 26 to 41 amino acids residues, for example 29, 30, 31 or 32 amino acids. In some embodiments, the linker has the formula -PGGG-(SGGGG)₅-P- wherein P is proline, G is glycine and S is serine (SEQ ID NO:28). In some embodiments, the linker has the sequence

### GSADDAKKDAAKKDGKS (SEQ ID NO:29)

In some embodiments, the scTCR contains a covalent disulfide bond linking a residue of the immunoglobulin region of the constant domain of the α chain to a residue of the immunoglobulin region of the constant domain of the β chain. In some embodiments, the interchain disulfide bond in a native TCR is not present. For example, in some embodiments, one or more cysteines can be incorporated into the constant region extracellular sequences of the first and second segments of the scTCR polypeptide. In some cases, both a native and a non-native disulfide bond may be desirable.

In some embodiments of a dTCR or scTCR containing introduced interchain disulfide bonds, the native disulfide bonds are not present. In some embodiments, the one or more of the native cysteines forming a native interchain disulfide bonds are substituted to another residue, such as to a serine or alanine. In some embodiments, an introduced disulfide bond can be formed by mutating non-cysteine residues on the first and second segments to cysteine. Exemplary non-native disulfide bonds of a TCR are described in published International PCT No. WO2006/000830.

In some embodiments, the TCR or antigen-binding fragment thereof exhibits an affinity with an equilibrium binding constant for a target antigen of between or between about 10-5 and 10-12 M and all individual values and ranges therein. In some embodiments, the target antigen is an MHC-peptide complex or ligand.

In some embodiments, nucleic acid or nucleic acids encoding a TCR, such as α and β chains, can be amplified by PCR, cloning or other suitable means and cloned into a suitable expression vector or vectors. The expression vector can be any suitable recombinant expression vector, and can be used to transform or transfect any suitable host. Suitable vectors include those designed for propagation and expansion or for expression or both, such as plasmids and viruses.

In some embodiments, the vector can a vector of the pUC series (Fermentas Life Sciences), the pBluescript series (Stratagene, LaJolla, Calif.), the pET series (Novagen, Madison, Wis.), the pGEX series (Pharmacia Biotech, Uppsala, Sweden), or the pEX series (Clontech, Palo Alto, Calif.). In some cases, bacteriophage vectors, such as λG10, λGT11, λZapII (Stratagene), λEMBL4, and λNM1149, also can be used. In some embodiments, plant expression vectors can be used and include pBI01, pBI101.2, pBI101.3, pBI121 and pBIN19 (Clontech). In some embodiments, animal expression vectors include pEUK-Cl, pMAM and pMAMneo (Clontech). In some embodiments, a viral vector is used, such as a retroviral vector.

In some embodiments, the recombinant expression vectors can be prepared using standard recombinant DNA techniques. In some embodiments, vectors can contain regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based. In some embodiments, the vector can contain a nonnative promoter operably linked to the nucleotide sequence encoding the TCR or antigen-binding portion (or other MHC-peptide binding molecule). In some embodiments, the promoter can be a non-viral promoter or a viral promoter, such as a cytomegalovirus (CMV) promoter, an SV40 promoter, an RSV promoter, and a promoter found in the long-terminal repeat of the murine stem cell virus. Other known promoters also are contemplated.

In some embodiments, to generate a vector encoding a TCR, the α and β chains are PCR amplified from total cDNA isolated from a T cell clone expressing the TCR of interest and cloned into an expression vector. In some embodiments, the α and β chains are cloned into the same vector. In some embodiments, the α and β chains are cloned into different vectors. In some embodiments, the generated α and β chains are incorporated into a retroviral, e.g. lentiviral, vector. Genetically Engineered Cells and Methods of Producing Cells

In some embodiments, the provided methods involve administering to a subject having a disease or condition cells expressing a recombinant antigen receptor. Various methods for the introduction of genetically engineered components, e.g., recombinant receptors, e.g., CARs or TCRs, are well known and may be used with the provided methods and compositions. Exemplary methods include those for transfer of nucleic acids encoding the receptors, including via viral, e.g., retroviral or lentiviral, transduction, transposons, and electroporation.

Among the cells expressing the receptors and administered by the provided methods are engineered cells. The genetic engineering generally involves introduction of a nucleic acid encoding the recombinant or engineered component into a composition containing the cells, such as by retroviral transduction, transfection, or transformation.

### C. Chimeric Auto-Antibody Receptors (CAARs)

In some embodiments, among the recombinant receptor expressed by the engineered cells used in connection with the provided methods, uses, articles of manufacture and compositions is a chimeric autoantibody receptor (CAAR). In some embodiments, the CAAR is specific for an autoantibody. In some embodiments, a cell expressing the CAAR, such as a T cell engineered to express a CAAR, can be used to specifically bind to and kill autoantibody-expressing cells, but not normal antibody expressing cells. In some embodiments, CAAR-expressing cells can be used to treat an autoimmune disease associated with expression of self-antigens, such as autoimmune diseases. In some embodiments, CAAR-expressing cells can target B cells that ultimately produce the autoantibodies and display the autoantibodies on their cell surfaces, mark these B cells as disease-specific targets for therapeutic intervention. In some embodiments, CAAR-expressing cells can be used to efficiently targeting and killing the pathogenic B cells in autoimmune diseases by targeting the disease-causing B cells using an antigen-specific chimeric autoantibody receptor. In some embodiments, the recombinant receptor is a CAAR, such as any described in U.S. Patent Application Pub. No. US 2017/0051035.

In some embodiments, the CAAR comprises an autoantibody binding domain, a transmembrane domain, and an intracellular signaling region. In some embodiments, the intracellular signaling region comprises an intracellular signaling domain. In some embodiments, the intracellular signaling domain is or comprises a primary signaling domain, a signaling domain that is capable of inducing a primary activation signal in a T cell, a signaling domain of a T cell receptor (TCR) component, and/or a signaling domain comprising an immunoreceptor tyrosine-based activation motif (ITAM). In some embodiments, the intracellular signaling region comprises a secondary or costimulatory signaling region (secondary intracellular signaling regions).

In some embodiments, the autoantibody binding domain comprises an autoantigen or a fragment thereof. The choice of autoantigen can depend upon the type of autoantibody being targeted. For example, the autoantigen may be chosen because it recognizes an autoantibody on a target cell, such as a B cell, associated with a particular disease state, e.g. an autoimmune disease, such as an autoantibody-mediated autoimmune disease. In some embodiments, the autoimmune disease includes pemphigus vulgaris (PV). Exemplary autoantigens include desmoglein 1 (Dsg1) and Dsg3.

### D. Multi-targeting

In some embodiments, the cells used in connection with the provided methods, uses, articles of manufacture and compositions include cells employing multi-targeting strategies, such as expression of two or more genetically engineered receptors on the cell, each recognizing the same of a different antigen and typically each including a different intracellular signaling component. Such multi-targeting strategies are described, for example, in International Patent Application, Publication No.: WO 2014055668 A1 (describing combinations of activating and costimulatory CARs, e.g., targeting two different antigens present individually on off-target, e.g., normal cells, but present together only on cells of the disease or condition to be treated) and Fedorov et al., Sci. Transl. Medicine, 5(215) (2013) (describing cells expressing an activating and an inhibitory CAR, such as those in which the activating CAR binds to one antigen expressed on both normal or non-diseased cells and cells of the disease or condition to be treated, and the inhibitory CAR binds to another antigen expressed only on the normal cells or cells which it is not desired to treat).

For example, in some embodiments, the cells include a receptor expressing a first genetically engineered antigen receptor (e.g., CAR or TCR) which is capable of inducing an activating or stimulatory signal to the cell, generally upon specific binding to the antigen recognized by the first receptor, e.g., the first antigen. In some embodiments, the cell further includes a second genetically engineered antigen receptor (e.g., CAR or TCR), e.g., a chimeric costimulatory receptor, which is capable of inducing a costimulatory signal to the immune cell, generally upon specific binding to a second antigen recognized by the second receptor. In some embodiments, the first antigen and second antigen are the same. In some embodiments, the first antigen and second antigen are different.

In some embodiments, the first and/or second genetically engineered antigen receptor (e.g. CAR or TCR) is capable of inducing an activating signal to the cell. In some embodiments, the receptor includes an intracellular signaling component containing ITAM or ITAM-like motifs. In some embodiments, the activation induced by the first receptor involves a signal transduction or change in protein expression in the cell resulting in initiation of an immune response, such as ITAM phosphorylation and/or initiation of ITAM-mediated signal transduction cascade, formation of an immunological synapse and/or clustering of molecules near the bound receptor (e.g. CD4 or CD8, etc.), activation of one or more transcription factors, such as NF-κB and/or AP-1, and/or induction of gene expression of factors such as cytokines, proliferation, and/or survival.

In some embodiments, the first and/or second receptor includes intracellular signaling domains or regions of costimulatory receptors such as CD28, CD137 (4-1BB), OX40, and/or ICOS. In some embodiments, the first and second receptor include an intracellular signaling domain of a costimulatory receptor that are different. In one embodiment, the first receptor contains a CD28 costimulatory signaling region and the second receptor contain a 4-1BB co-stimulatory signaling region or vice versa.

In some embodiments, the first and/or second receptor includes both an intracellular signaling domain containing ITAM or ITAM-like motifs and an intracellular signaling domain of a costimulatory receptor.

In some embodiments, the first receptor contains an intracellular signaling domain containing ITAM or ITAM-like motifs and the second receptor contains an intracellular signaling domain of a costimulatory receptor. The costimulatory signal in combination with the activating signal induced in the same cell is one that results in an immune response, such as a robust and sustained immune response, such as increased gene expression, secretion of cytokines and other factors, and T cell mediated effector functions such as cell killing.

In some embodiments, neither ligation of the first receptor alone nor ligation of the second receptor alone induces a robust immune response. In some aspects, if only one receptor is ligated, the cell becomes tolerized or unresponsive to antigen, or inhibited, and/or is not induced to proliferate or secrete factors or carry out effector functions. In some such embodiments, however, when the plurality of receptors are ligated, such as upon encounter of a cell expressing the first and second antigens, a desired response is achieved, such as full immune activation or stimulation, e.g., as indicated by secretion of one or more cytokine, proliferation, persistence, and/or carrying out an immune effector function such as cytotoxic killing of a target cell.

In some embodiments, the two receptors induce, respectively, an activating and an inhibitory signal to the cell, such that binding by one of the receptor to its antigen activates the cell or induces a response, but binding by the second inhibitory receptor to its antigen induces a signal that suppresses or dampens that response. Examples are combinations of activating CARs and inhibitory CARs or iCARs. Such a strategy may be used, for example, in which the activating CAR binds an antigen expressed in a disease or condition but which is also expressed on normal cells, and the inhibitory receptor binds to a separate antigen which is expressed on the normal cells but not cells of the disease or condition.

In some embodiments, the multi-targeting strategy is employed in a case where an antigen associated with a particular disease or condition is expressed on a non-diseased cell and/or is expressed on the engineered cell itself, either transiently (e.g., upon stimulation in association with genetic engineering) or permanently. In such cases, by requiring ligation of two separate and individually specific antigen receptors, specificity, selectivity, and/or efficacy may be improved.

In some embodiments, the plurality of antigens, e.g., the first and second antigens, are expressed on the cell, tissue, or disease or condition being targeted, such as on the cancer cell. In some aspects, the cell, tissue, disease or condition is multiple myeloma or a multiple myeloma cell. In some embodiments, one or more of the plurality of antigens generally also is expressed on a cell which it is not desired to target with the cell therapy, such as a normal or non-diseased cell or tissue, and/or the engineered cells themselves. In such embodiments, by requiring ligation of multiple receptors to achieve a response of the cell, specificity and/or efficacy is achieved.

### E. Methods of Engineering Cells

In particular embodiments, the engineered cells are produced by a process that generates an output composition of enriched T cells from one or more input compositions and/or from a single biological sample. In certain embodiments, the output composition contains cells that express a recombinant receptor, e.g., a CAR, such as an anti-CD19 CAR. In particular embodiments, the cells of the output compositions are suitable for administration to a subject as a therapy, e.g., an autologous cell therapy. In some embodiments, the output composition is a composition of enriched CD4+ or CD8+ T cells.

In some embodiments, the process for generating or producing engineered cells is by a process that includes some or all of the steps of: collecting or obtaining a biological sample; isolating, selecting, or enriching input cells from the biological sample; cryopreserving and storing the input cells; thawing and/or incubating the input cells under stimulating conditions; engineering the stimulated cells to express or contain a recombinant polynucleotide, e.g., a polynucleotide encoding a recombinant receptor such as a CAR; cultivating the engineered cells, e.g. to a threshold amount, density, or expansion; formulating the cultivated cells in an output composition; and/or cryopreserving and storing the formulated output cells until the cells are released for infusion and/or are suitable to be administered to a subject. In certain embodiments, the process is performed with two or more input compositions of enriched T cells, such as a separate CD4+ composition and a separate CD8+ composition, that are separately processed and engineered from the same starting or initial biological sample and re-infused back into the subject at a defined ratio, e.g. 1:1 ratio of CD4+ to CD8+ T cells. In some embodiments, the enriched T cells are or include engineered T cells, e.g., T cells transduced to express a recombinant receptor.

In particular embodiments, an output composition of engineered cells expressing a recombinant receptor (e.g. anti-CD19 CAR) is produced from an initial and/or input composition of cells. In some embodiments, the input composition is a composition of enriched T cells, enriched CD4+ T cells, and/or enriched CD8+ T cells (herein after also referred to as compositions of enriched T cells, compositions of enriched CD4+ T cells, and compositions of enriched CD8+ T cells, respectively). In some embodiments, a composition enriched in CD4+ T cells contains at least 60%, 65%, 70%, 75%, 80%, 85%, 90%, 95%, 98%, 99%, or 99.9% CD4+ T cells. In particular embodiments, the composition of enriched CD4+ T cells contains 100% CD4+ T cells contains about 100% CD4+ T cells. In certain embodiments, the composition of enriched T cells includes or contains less than 20%, less than 10%, less than 5%, less than 1%, less than 0.1%, or less than 0.01% CD8+ T cells, and/or contains no CD8+ T cells, and/or is free or substantially free of CD8+ T cells. In some embodiments, the populations of cells consist essentially of CD4+ T cells. In some embodiments, a composition enriched in CD8+ T cells contains at least 75%, 80%, 85%, 90%, 95%, 98%, 99%, or 99.9% CD8+ T cells, or contains or contains about 100% CD8+ T cells. In certain embodiments, the composition of enriched CD8+ T cells includes or contains less than 20%, less than 10%, less than 5%, less than 1%, less than 0.1%, or less than 0.01% CD4+ T cells, and/or contains no CD4+ T cells, and/or is free or substantially free of CD4+ T cells. In some embodiments, the populations of cells consist essentially of CD8+ T cells.

In certain embodiments, the process for producing engineered cells further can include one or more of: activating and/or stimulating a cells, e.g., cells of an input composition; genetically engineering the activated and/or stimulated cells, e.g., to introduce a polynucleotide encoding a recombinant protein by transduction or transfection; and/or cultivating the engineered cells, e.g., under conditions that promote proliferation and/or expansion. In particular embodiments, the provided methods may be used in connection with harvesting, collecting, and/or formulating output compositions produced after the cells have been incubated, activated, stimulated, engineered, transduced, transfected, and/or cultivated.

In some embodiments, engineered cells, such as those that express an anti-CD19 CAR as described, used in accord with the provided methods and uses are produced or generated by a process for selecting, isolating, activating, stimulating, expanding, cultivating, and/or formulating cells. In some embodiments, such methods include any as described.

In some embodiments, engineered cells, such as those that express an anti-CD19 CAR as described, used in accord with the provided methods and uses are produced or generated by exemplary processes as described in, for example, WO 2019/089855 and WO 2015/164675.

In some of any embodiments, exemplary processes for generating, producing or manufacturing the engineered cells, such as those that express an anti-CD19 CAR as described, or a composition comprising such cells, such as a composition comprising engineered CD4+ T cells and engineered CD8+ T cells each expressing the same anti-CD19 chimeric antigen receptor (CAR), involve subjecting enriched CD4+ and enriched CD8+ cell populations, separately, to process steps. In some aspects of the exemplary process for generating or manufacturing engineered cells, CD4+ and CD8+ cells are separately selected from human peripheral blood mononuclear cells (PBMCs), for example, that are obtained by leukapheresis, generating separate enriched CD4+ and enriched CD8+ cell compositions. In some aspects, such cells can be cryopreserved. In some aspects, the CD4+ and CD8+ compositions can be subsequently thawed and separately subject to steps for stimulation, transduction, and expansion.

In some aspects of the exemplary process for generating or manufacturing engineered cells, thawed CD4+ and CD8+ cells are separately stimulated, for example, in the presence of paramagnetic polystyrene-coated beads coupled to anti-CD3 and anti-CD28 antibodies (such as at a 1:1 bead to cell ratio). In some aspects, the stimulation is carried out in media containing human recombinant IL-2, human recombinant IL-15, and N-Acetyl Cysteine (NAC). In some aspects, the cell culture media for CD4+ cells also can include human recombinant IL-7.

In some aspects of the exemplary process for generating or manufacturing engineered cells, following the introduction of the beads, CD4+ and CD8+ cells are separately transduced with a lentiviral vector encoding the same CAR, such as the same anti-CD19 CAR. In some aspects, the CAR can contain an anti-CD19 scFv derived from a murine antibody, an immunoglobulin spacer, a transmembrane domain derived from CD28, a costimulatory region derived from 4-1BB, and a CD3-zeta intracellular signaling domain. In some aspects, the vector can encode a truncated receptor that serves as a surrogate marker for CAR expression that is connected to the CAR construct by a T2A sequence. In some aspects of the exemplary process, the cells are transduced in the presence of 10 µg/ml protamine sulfate.

In some aspects of the exemplary process for generating or manufacturing engineered cells, following transduction, the beads are removed from the cell compositions by exposure to a magnetic field. In some aspects, the CD4+ and CD8+ cell compositions are separately cultivated for expansion with continual mixing and oxygen transfer by a bioreactor (for example, a Xuri W25 Bioreactor). In some cases, poloxamer is added to the media. In some aspects, both the CD4+ and the CD8+ cell compositions are cultivated in the presence of IL-2 and IL-15. In some aspects, the CD4+ cell media also included IL-7. In some cases, the CD4+ and CD8+ cells are each cultivated, prior to harvest, to 4-fold expansion. In some aspects, one day after reaching the threshold, cells from each composition can be separately harvested, formulated, and cryopreserved. In some aspects, the exemplary processes for generating, producing or manufacturing the engineered cells, such as those that express an anti-CD19 CAR as described, or a composition comprising such cells, such as a composition comprising engineered CD4+ T cells and engineered CD8+ T cells each expressing the same anti-CD19 chimeric antigen receptor (CAR), include those described in **Table 11** below.

**Table 11: Exemplary process for generating CD4+ and CD8+ CAR-T cells**

| **Stage** | **CD4+ cells** | | **CD8+ cells** | |
|---|---|---|---|---|
| **Stimulation** (day 1-2) | | • anti-CD3/CD28 antibody conjugated beads | | • anti-CD3/CD28 antibody conjugated beads |
| | | • 1:1 bead to cell ratio | | • 1:1 bead to cell ratio |
| | | • media: IL-2, IL-7, IL-15, and NAC | | • media: IL-2, IL-15, and NAC |
| **Transduction** (day 2-5) | | • transduction adjuvant (e.g. 10 µg/ml protamine sulfate) | | • transduction adjuvant (e.g. 10 µg/ml protamine sulfate) |
| **Bead removal** (day 5*) | | • magnetic bead removal | | • magnetic bead removal |
| **Expansion (day 5* - Harvest)** | | • rocking motion bioreactor and/or continuous mixing | | • rocking motion bioreactor and/or continuous mixing |
| | | • media: IL-2, IL-7, IL15, and poloxamer | | • media: IL-2, IL15, and poloxamer |

| | | | | |
|---|---|---|---|---|
| *Approximate | | | | |

In other aspects, a different exemplary process for generating, producing or manufacturing the engineered cells or a composition comprising such cells include a process that differs from the exemplary process above in that: NAC is not added to the media during stimulation; CD4+ cell media does not contain IL-2; cells are stimulated at a bead to cell ratio of 3:1; cells are transduced with a higher concentration of protamine sulfate; bead removal occurs at about day 7; and expansion is performed at a static setting, i.e., without continual mixing or perfusion (e.g., semi-continuous and/or stepwise perfusion), and without poloxamer.

In some embodiments, at least one separate composition of enriched CD4+ T cells and at least one separate composition of enriched CD8+ T cells are isolated, selected, enriched, or obtained from a single biological sample, e.g., a sample of PBMCs or other white blood cells from the same donor such as a patient or healthy individual. In some embodiments, a separate composition of enriched CD4+ T cells and a separate composition of enriched CD8+ T cells originated, e.g., were initially isolated, selected, and/or enriched, from the same biological sample, such as a single biological sample obtained, collected, and/or taken from a single subject. In some embodiments, a biological sample is first subjected to selection of CD4+ T cells, where both the negative and positive fractions are retained, and the negative fraction is further subjected to selection of CD8+ T cells. In other embodiments, a biological sample is first subjected to selection of CD8+ T cells, where both the negative and positive fractions are retained, and the negative fraction is further subjected to selection of CD4+ T cells. In some embodiments, methods of selection are carried out as described in International PCT publication No. WO2015/164675. In some embodiments, methods of selection are carried out as described in International PCT publication No. WO 2019/089855. In some aspects, a biological sample is first positively selected for CD8+ T cells to generate at least one composition of enriched CD8+ T cells, and the negative fraction is then positively selected for CD4+ T cells to generate at least one composition of enriched CD4+ T cells, such that the at least one composition of enriched CD8+ T cells and the at least one composition of enriched CD4+ T cells are separate compositions from the same biological sample, e.g., from the same donor patient or healthy individual. In some aspects, two or more separate compositions of enriched T cells, e.g., at least one being a composition of enriched CD4+ T cells and at least one being a separate composition of enriched CD8+ T cells from the same donor, are separately frozen, e.g., cryoprotected or cryopreserved in a cryopreservation media.

In some aspects, two or more separate compositions of enriched T cells, e.g., at least one being a composition of enriched CD4+ T cells and at least one being a separate composition of enriched CD8+ T cells from the same biological sample, are activated and/or stimulated by contacting with a stimulatory reagent (e.g., by incubation with CD3/CD28 conjugated magnetic beads for T cell activation). In some aspects, each of the activated/stimulated cell composition is engineered, transduced, and/or transfected, e.g., using a viral vector encoding a recombinant protein (e.g. CAR), to express the same recombinant protein in the CD4+ T cells and CD8+ T cells of each cell composition. In some aspects, the method comprises removing the stimulatory reagent, e.g., magnetic beads, from the cell composition. In some aspects, a cell composition containing engineered CD4+ T cells and a cell composition containing engineered CD8+ T cells are separately cultivated, e.g., for separate expansion of the CD4+ T cell and CD8+ T cell populations therein. In certain embodiments, a cell composition from the cultivation is harvested and/or collected and/or formulated, e.g., by washing the cell composition in a formulation buffer. In certain embodiments, a formulated cell composition comprising CD4+ T cells and a formulated cell composition comprising CD8+ T cells is frozen, e.g., cryoprotected or cryopreserved in a cryopreservation media. In some aspects, engineered CD4+ T cells and CD8+ T cells in each formulation originate from the same donor or biological sample and express the same recombination protein (e.g., CAR, such as anti-CD19 CAR). In some aspects, a separate engineered CD4+ formulation and a separate engineered CD8+ formulation are administered at a defined ratio, e.g. 1:1, to a subject in need thereof such as the same donor.

### 1. Cells and Preparation of Cells for Genetic Engineering

In some embodiments, cells, such as T cells, used in connection with the provided methods, uses, articles of manufacture or compositions are cells have been genetically engineered to express a recombinant receptor, e.g., a CAR or a TCR described herein. In some embodiments, the engineered cells are used in the context of cell therapy, e.g., adoptive cell therapy. In some embodiments, the engineered cells are immune cells. In some embodiments, the engineered cells are T cells, such as CD4+ or CD8+ T cells.

In some embodiments, the nucleic acids, such as nucleic acids encoding a recombinant receptor, are heterologous, i.e., normally not present in a cell or sample obtained from the cell, such as one obtained from another organism or cell, which for example, is not ordinarily found in the cell being engineered and/or an organism from which such cell is derived. In some embodiments, the nucleic acids are not naturally occurring, such as a nucleic acid not found in nature, including one comprising chimeric combinations of nucleic acids encoding various domains from multiple different cell types.

The cells generally are eukaryotic cells, such as mammalian cells, and typically are human cells. In some embodiments, the cells are derived from the blood, bone marrow, lymph, or lymphoid organs, are cells of the immune system, such as cells of the innate or adaptive immunity, e.g., myeloid or lymphoid cells, including lymphocytes, typically T cells and/or NK cells. Other exemplary cells include stem cells, such as multipotent and pluripotent stem cells, including induced pluripotent stem cells (iPSCs). The cells typically are primary cells, such as those isolated directly from a subject and/or isolated from a subject and frozen. In some embodiments, the cells include one or more subsets of T cells or other cell types, such as whole T cell populations, CD4⁺ cells, CD8⁺ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. With reference to the subject to be treated, the cells may be allogeneic and/or autologous. Among the methods include off-the-shelf methods. In some aspects, such as for off-the-shelf technologies, the cells are pluripotent and/or multipotent, such as stem cells, such as induced pluripotent stem cells (iPSCs). In some embodiments, the methods include isolating cells from the subject, preparing, processing, culturing, and/or engineering them, and re-introducing them into the same subject, before or after cryopreservation.

Among the sub-types and subpopulations of T cells and/or of CD4⁺ and/or of CD8⁺ T cells are naïve T (T_{N}) cells, effector T cells (T_{EFF}), memory T cells and sub-types thereof, such as stem cell memory T (T_{SCM}), central memory T (T_{CM}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells.

In some embodiments, the cells are natural killer (NK) cells. In some embodiments, the cells are monocytes or granulocytes, e.g., myeloid cells, macrophages, neutrophils, dendritic cells, mast cells, eosinophils, and/or basophils.

In some embodiments, the cells include one or more nucleic acids introduced via genetic engineering, and thereby express recombinant or genetically engineered products of such nucleic acids. In some embodiments, the nucleic acids are heterologous, i.e., normally not present in a cell or sample obtained from the cell, such as one obtained from another organism or cell, which for example, is not ordinarily found in the cell being engineered and/or an organism from which such cell is derived. In some embodiments, the nucleic acids are not naturally occurring, such as a nucleic acid not found in nature, including one comprising chimeric combinations of nucleic acids encoding various domains from multiple different cell types.

In some embodiments, preparation of the engineered cells includes one or more culture and/or preparation steps. The cells for introduction of the nucleic acid encoding the transgenic receptor such as the CAR, may be isolated from a sample, such as a biological sample, e.g., one obtained from or derived from a subject. In some embodiments, the subject from which the cell is isolated is one having the disease or condition or in need of a cell therapy or to which cell therapy will be administered. The subject in some embodiments is a human in need of a particular therapeutic intervention, such as the adoptive cell therapy for which cells are being isolated, processed, and/or engineered.

Accordingly, the cells in some embodiments are primary cells, e.g., primary human cells. The samples include tissue, fluid, and other samples taken directly from the subject, as well as samples resulting from one or more processing steps, such as separation, centrifugation, genetic engineering (e.g. transduction with viral vector), washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample from which the cells are derived or isolated is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some embodiments, the cells are derived from cell lines, e.g., T cell lines. The cells in some embodiments are obtained from a xenogeneic source, for example, from mouse, rat, non-human primate, and pig.

In some embodiments, isolation of the cells includes one or more preparation and/or non-affinity based cell separation steps. In some examples, cells are washed, centrifuged, and/or incubated in the presence of one or more reagents, for example, to remove unwanted components, enrich for desired components, lyse or remove cells sensitive to particular reagents. In some examples, cells are separated based on one or more property, such as density, adherent properties, size, sensitivity and/or resistance to particular components.

In some examples, cells from the circulating blood of a subject are obtained, e.g., by apheresis or leukapheresis. The samples, in some aspects, contain lymphocytes, including T cells, monocytes, granulocytes, B cells, other nucleated white blood cells, red blood cells, and/or platelets, and in some aspects contains cells other than red blood cells and platelets.

In some embodiments, the blood cells collected from the subject are washed, e.g., to remove the plasma fraction and to place the cells in an appropriate buffer or media for subsequent processing steps. In some embodiments, the cells are washed with phosphate buffered saline (PBS). In some embodiments, the wash solution lacks calcium and/or magnesium and/or many or all divalent cations. In some aspects, a washing step is accomplished a semi-automated "flow-through" centrifuge (for example, the Cobe 2991 cell processor, Baxter) according to the manufacturer's instructions. In some aspects, a washing step is accomplished by tangential flow filtration (TFF) according to the manufacturer's instructions. In some embodiments, the cells are resuspended in a variety of biocompatible buffers after washing, such as, for example, Ca⁺⁺/Mg⁺⁺ free PBS. In certain embodiments, components of a blood cell sample are removed and the cells directly resuspended in culture media.

In some embodiments, the methods include density-based cell separation methods, such as the preparation of white blood cells from peripheral blood by lysing the red blood cells and centrifugation through a Percoll or Ficoll gradient.

In some embodiments, at least a portion of the selection step includes incubation of cells with a selection reagent. The incubation with a selection reagent or reagents, e.g., as part of selection methods which may be performed using one or more selection reagents for selection of one or more different cell types based on the expression or presence in or on the cell of one or more specific molecules, such as surface markers, e.g., surface proteins, intracellular markers, or nucleic acid. In some embodiments, any known method using a selection reagent or reagents for separation based on such markers may be used. In some embodiments, the selection reagent or reagents result in a separation that is affinity- or immunoaffinity-based separation. For example, the selection in some aspects includes incubation with a reagent or reagents for separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

In some aspects of such processes, a volume of cells is mixed with an amount of a desired affinity-based selection reagent. The immunoaffinity-based selection can be carried out using any system or method that results in a favorable energetic interaction between the cells being separated and the molecule specifically binding to the marker on the cell, e.g., the antibody or other binding partner on the solid surface, e.g., particle. In some embodiments, methods are carried out using particles such as beads, e.g. magnetic beads, that are coated with a selection agent (e.g. antibody) specific to the marker of the cells. The particles (e.g. beads) can be incubated or mixed with cells in a container, such as a tube or bag, while shaking or mixing, with a constant cell density-to-particle (e.g., bead) ratio to aid in promoting energetically favored interactions. In other cases, the methods include selection of cells in which all or a portion of the selection is carried out in the internal cavity of a centrifugal chamber, for example, under centrifugal rotation. In some embodiments, incubation of cells with selection reagents, such as immunoaffinity-based selection reagents, is performed in a centrifugal chamber. In certain embodiments, the isolation or separation is carried out using a system, device, or apparatus described in International Patent Application, Publication Number WO2009/072003, or US 20110003380 A1. In one example, the system is a system as described in International Publication Number WO2016/073602.

In some embodiments, by conducting such selection steps or portions thereof (e.g., incubation with antibody-coated particles, e.g., magnetic beads) in the cavity of a centrifugal chamber, the user is able to control certain parameters, such as volume of various solutions, addition of solution during processing and timing thereof, which can provide advantages compared to other available methods. For example, the ability to decrease the liquid volume in the cavity during the incubation can increase the concentration of the particles (e.g. bead reagent) used in the selection, and thus the chemical potential of the solution, without affecting the total number of cells in the cavity. This in turn can enhance the pairwise interactions between the cells being processed and the particles used for selection. In some embodiments, carrying out the incubation step in the chamber, e.g., when associated with the systems, circuitry, and control as described herein, permits the user to effect agitation of the solution at desired time(s) during the incubation, which also can improve the interaction.

In some embodiments, at least a portion of the selection step is performed in a centrifugal chamber, which includes incubation of cells with a selection reagent. In some aspects of such processes, a volume of cells is mixed with an amount of a desired affinity-based selection reagent that is far less than is normally employed when performing similar selections in a tube or container for selection of the same number of cells and/or volume of cells according to manufacturer's instructions. In some embodiments, an amount of selection reagent or reagents that is/are no more than 5%, no more than 10%, no more than 15%, no more than 20%, no more than 25%, no more than 50%, no more than 60%, no more than 70% or no more than 80% of the amount of the same selection reagent(s) employed for selection of cells in a tube or container-based incubation for the same number of cells and/or the same volume of cells according to manufacturer's instructions is employed.

In some embodiments, for selection, e.g., immunoaffinity-based selection of the cells, the cells are incubated in the cavity of the chamber in a composition that also contains the selection buffer with a selection reagent, such as a molecule that specifically binds to a surface marker on a cell that it desired to enrich and/or deplete, but not on other cells in the composition, such as an antibody, which optionally is coupled to a scaffold such as a polymer or surface, e.g., bead, e.g., magnetic bead, such as magnetic beads coupled to monoclonal antibodies specific for CD4 and CD8. In some embodiments, as described, the selection reagent is added to cells in the cavity of the chamber in an amount that is substantially less than (e.g. is no more than 5%, 10%, 20%, 30%, 40%, 50%, 60%, 70% or 80% of the amount) as compared to the amount of the selection reagent that is typically used or would be necessary to achieve about the same or similar efficiency of selection of the same number of cells or the same volume of cells when selection is performed in a tube with shaking or rotation. In some embodiments, the incubation is performed with the addition of a selection buffer to the cells and selection reagent to achieve a target volume with incubation of the reagent of, for example, 10 mL to 200 mL, such as at least or at least about 10 mL, 20 mL, 30 mL, 40 mL, 50 mL, 60 mL, 70 mL, 80 mL, 90 mL, 100 mL, 150 mL or 200 mL. In some embodiments, the selection buffer and selection reagent are pre-mixed before addition to the cells. In some embodiments, the selection buffer and selection reagent are separately added to the cells. In some embodiments, the selection incubation is carried out with periodic gentle mixing condition, which can aid in promoting energetically favored interactions and thereby permit the use of less overall selection reagent while achieving a high selection efficiency.

In some embodiments, the total duration of the incubation with the selection reagent is from or from about 5 minutes to 6 hours, such as 30 minutes to 3 hours, for example, at least or at least about 30 minutes, 60 minutes, 120 minutes or 180 minutes.

In some embodiments, the incubation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some embodiments, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In some embodiments, such process is carried out within the entirely closed system to which the chamber is integral. In some embodiments, this process (and in some aspects also one or more additional step, such as a previous wash step washing a sample containing the cells, such as an apheresis sample) is carried out in an automated fashion, such that the cells, reagent, and other components are drawn into and pushed out of the chamber at appropriate times and centrifugation effected, so as to complete the wash and binding step in a single closed system using an automated program.

In some embodiments, after the incubation and/or mixing of the cells and selection reagent and/or reagents, the incubated cells are subjected to a separation to select for cells based on the presence or absence of the particular reagent or reagents. In some embodiments, the separation is performed in the same closed system in which the incubation of cells with the selection reagent was performed. In some embodiments, after incubation with the selection reagents, incubated cells, including cells in which the selection reagent has bound are transferred into a system for immunoaffinity-based separation of the cells. In some embodiments, the system for immunoaffinity-based separation is or contains a magnetic separation column.

In some embodiments, the isolation methods include the separation of different cell types based on the expression or presence in the cell of one or more specific molecules, such as surface markers, e.g., surface proteins, intracellular markers, or nucleic acid. In some embodiments, any known method for separation based on such markers may be used. In some embodiments, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Such separation steps can be based on positive selection, in which the cells having bound the reagents are retained for further use, and/or negative selection, in which the cells having not bound to the antibody or binding partner are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

The separation need not result in 100% enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples, multiple rounds of separation steps are carried out, where the positively or negatively selected fraction from one step is subjected to another separation step, such as a subsequent positive or negative selection. In some examples, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some aspects, specific subpopulations of T cells, such as cells positive or expressing high levels of one or more surface markers, e.g., CD28⁺, CD62L⁺, CCR7⁺, CD27⁺, CD127⁺, CD4⁺, CD8⁺, CD45RA⁺, and/or CD45RO⁺ T cells, are isolated by positive or negative selection techniques.

For example, CD3⁺, CD28⁺ T cells can be positively selected using anti-CD3/anti-CD28 conjugated magnetic beads (e.g., DYNABEADS^{®} M-450 CD3/CD28 T Cell Expander).

In some embodiments, isolation is carried out by enrichment for a particular cell population by positive selection, or depletion of a particular cell population, by negative selection. In some embodiments, positive or negative selection is accomplished by incubating cells with one or more antibodies or other binding agent that specifically bind to one or more surface markers expressed or expressed (marker⁺) at a relatively higher level (marker^{high}) on the positively or negatively selected cells, respectively.

In particular embodiments, a biological sample, e.g., a sample of PBMCs or other white blood cells, are subjected to selection of CD4+ T cells, where both the negative and positive fractions are retained. In certain embodiments, CD8+ T cells are selected from the negative fraction. In some embodiments, a biological sample is subjected to selection of CD8+ T cells, where both the negative and positive fractions are retained. In certain embodiments, CD4+ T cells are selected from the negative fraction.

In some embodiments, T cells are separated from a PBMC sample by negative selection of markers expressed on non-T cells, such as B cells, monocytes, or other white blood cells, such as CD14. In some aspects, a CD4⁺ or CD8⁺ selection step is used to separate CD4⁺ helper and CD8⁺ cytotoxic T cells. Such CD4⁺ and CD8⁺ populations can be further sorted into sub-populations by positive or negative selection for markers expressed or expressed to a relatively higher degree on one or more naive, memory, and/or effector T cell subpopulations.

In some embodiments, CD8⁺ cells are further enriched for or depleted of naive, central memory, effector memory, and/or central memory stem cells, such as by positive or negative selection based on surface antigens associated with the respective subpopulation. In some embodiments, enrichment for central memory T (T_{CM}) cells is carried out to increase efficacy, such as to improve long-term survival, expansion, and/or engraftment following administration, which in some aspects is particularly robust in such sub-populations. See Terakura et al. (2012) Blood.1:72-82; Wang et al. (2012) J Immunother. 35(9):689-701. In some embodiments, combining T_{CM}-enriched CD8⁺ T cells and CD4⁺ T cells further enhances efficacy.

In embodiments, memory T cells are present in both CD62L⁺ and CD62L⁻ subsets of CD8⁺ peripheral blood lymphocytes. PBMC can be enriched for or depleted of CD62L⁻CD8⁺ and/or CD62L⁺CD8⁺ fractions, such as using anti-CD8 and anti-CD62L antibodies.

In some embodiments, the enrichment for central memory T (T_{CM}) cells is based on positive or high surface expression of CD45RO, CD62L, CCR7, CD28, CD3, and/or CD127; in some aspects, it is based on negative selection for cells expressing or highly expressing CD45RA and/or granzyme B. In some aspects, isolation of a CD8⁺ population enriched for T_{CM} cells is carried out by depletion of cells expressing CD4, CD14, CD45RA, and positive selection or enrichment for cells expressing CD62L. In one aspect, enrichment for central memory T (T_{CM}) cells is carried out starting with a negative fraction of cells selected based on CD4 expression, which is subjected to a negative selection based on expression of CD14 and CD45RA, and a positive selection based on CD62L. Such selections in some aspects are carried out simultaneously and in other aspects are carried out sequentially, in either order. In some aspects, the same CD4 expression-based selection step used in preparing the CD8⁺ cell population or subpopulation, also is used to generate the CD4⁺ cell population or sub-population, such that both the positive and negative fractions from the CD4-based separation are retained and used in subsequent steps of the methods, optionally following one or more further positive or negative selection steps.

In a particular example, a sample of PBMCs or other white blood cell sample is subjected to selection of CD4⁺ cells, where both the negative and positive fractions are retained. The negative fraction then is subjected to negative selection based on expression of CD14 and CD45RA or CD19, and positive selection based on a marker characteristic of central memory T cells, such as CD62L or CCR7, where the positive and negative selections are carried out in either order.

CD4⁺ T helper cells are sorted into naive, central memory, and effector cells by identifying cell populations that have cell surface antigens. CD4⁺ lymphocytes can be obtained by standard methods. In some embodiments, naive CD4⁺ T lymphocytes are CD45RO⁻, CD45RA⁺, CD62L⁺, CD4⁺ T cells. In some embodiments, central memory CD4⁺ cells are CD62L⁺ and CD45RO⁺. In some embodiments, effector CD4⁺ cells are CD62L⁻ and CD45RO⁻.

In one example, to enrich for CD4⁺ cells by negative selection, a monoclonal antibody cocktail typically includes antibodies to CD14, CD20, CD11b, CD16, HLA-DR, and CD8. In some embodiments, the antibody or binding partner is bound to a solid support or matrix, such as a magnetic bead or paramagnetic bead, to allow for separation of cells for positive and/or negative selection. For example, in some embodiments, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In Vitro and In Vivo, p 17-25 Edited by: S. A. Brooks and U. Schumacher © Humana Press Inc., Totowa, NJ).

In some aspects, the sample or composition of cells to be separated is incubated with small, magnetizable or magnetically responsive material, such as magnetically responsive particles or microparticles, such as paramagnetic beads (e.g., such as Dynalbeads or MACS beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select.

In some embodiments, the magnetic particle or bead comprises a magnetically responsive material bound to a specific binding member, such as an antibody or other binding partner. There are many well-known magnetically responsive materials used in magnetic separation methods. Suitable magnetic particles include those described in Molday, U.S. Pat. No. 4,452,773, and in European Patent Specification EP 452342 B, which are hereby incorporated by reference. Colloidal sized particles, such as those described in Owen U.S. Pat. No. 4,795,698, and Liberti et al., U.S. Pat. No. 5,200,084 are other examples.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In some aspects, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some aspects, a combination of positive and negative selection is performed during the same selection step, where the positive and negative fractions are retained and further processed or subject to further separation steps.

In certain embodiments, the magnetically responsive particles are coated in primary antibodies or other binding partners, secondary antibodies, lectins, enzymes, or streptavidin. In certain embodiments, the magnetic particles are attached to cells via a coating of primary antibodies specific for one or more markers. In certain embodiments, the cells, rather than the beads, are labeled with a primary antibody or binding partner, and then cell-type specific secondary antibody- or other binding partner (e.g., streptavidin)-coated magnetic particles, are added. In certain embodiments, streptavidin-coated magnetic particles are used in conjunction with biotinylated primary or secondary antibodies.

In some embodiments, the magnetically responsive particles are left attached to the cells that are to be subsequently incubated, cultured and/or engineered; in some aspects, the particles are left attached to the cells for administration to a patient. In some embodiments, the magnetizable or magnetically responsive particles are removed from the cells. Methods for removing magnetizable particles from cells are known and include, e.g., the use of competing non-labeled antibodies, and magnetizable particles or antibodies conjugated to cleavable linkers. In some embodiments, the magnetizable particles are biodegradable.

In some embodiments, the affinity-based selection is via magnetic-activated cell sorting (MACS) (Miltenyi Biotec, Auburn, CA). Magnetic Activated Cell Sorting (MACS) systems are capable of high-purity selection of cells having magnetized particles attached thereto. In certain embodiments, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain embodiments, the non-target cells are labelled and depleted from the heterogeneous population of cells.

In certain embodiments, the isolation or separation is carried out using a system, device, or apparatus that carries out one or more of the isolation, cell preparation, separation, processing, incubation, culture, and/or formulation steps of the methods. In some aspects, the system is used to carry out each of these steps in a closed or sterile environment, for example, to minimize error, user handling and/or contamination. In one example, the system is a system as described in International Patent Application, Publication Number WO2009/072003, or US 20110003380 A1.

In some embodiments, the system or apparatus carries out one or more, e.g., all, of the isolation, processing, engineering, and formulation steps in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some aspects, the separation and/or other steps is carried out using CliniMACS system (Miltenyi Biotec), for example, for automated separation of cells on a clinical-scale level in a closed and sterile system. Components can include an integrated microcomputer, magnetic separation unit, peristaltic pump, and various pinch valves. The integrated computer in some aspects controls all components of the instrument and directs the system to perform repeated procedures in a standardized sequence. The magnetic separation unit in some aspects includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some aspects uses antibody-coupled magnetizable particles that are supplied in a sterile, non-pyrogenic solution. In some embodiments, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some embodiments, the cell populations for use with the methods described herein are unlabeled and are not retained in the column. In some embodiments, the cell populations for use with the methods described herein are labeled and are retained in the column. In some embodiments, the cell populations for use with the methods described herein are eluted from the column after removal of the magnetic field, and are collected within the cell collection bag.

In certain embodiments, separation and/or other steps are carried out using the CliniMACS Prodigy system (Miltenyi Biotec). The CliniMACS Prodigy system in some aspects is equipped with a cell processing unity that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood is automatically separated into erythrocytes, white blood cells and plasma layers. The CliniMACS Prodigy system can also include an integrated cell cultivation chamber which accomplishes cell culture protocols such as, e.g., cell differentiation and expansion, antigen loading, and long-term cell culture. Input ports can allow for the sterile removal and replenishment of media and cells can be monitored using an integrated microscope. See, e.g., Klebanoff et al. (2012) J Immunother. 35(9): 651-660, Terakura et al. (2012) Blood.1:72-82, and Wang et al. (2012) J Immunother. 35(9):689-701.

In some embodiments, a cell population described herein is collected and enriched (or depleted) via flow cytometry, in which cells stained for multiple cell surface markers are carried in a fluidic stream. In some embodiments, a cell population described herein is collected and enriched (or depleted) via preparative scale (FACS)-sorting. In certain embodiments, a cell population described herein is collected and enriched (or depleted) by use of microelectromechanical systems (MEMS) chips in combination with a FACS-based detection system (see, e.g., WO 2010/033140, Cho et al. (2010) Lab Chip 10, 1567-1573; and Godin et al. (2008) J Biophoton. 1(5):355-376. In both cases, cells can be labeled with multiple markers, allowing for the isolation of well-defined T cell subsets at high purity.

In some embodiments, the antibodies or binding partners are labeled with one or more detectable marker, to facilitate separation for positive and/or negative selection. For example, separation may be based on binding to fluorescently labeled antibodies. In some examples, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, e.g., in combination with a flow-cytometric detection system. Such methods allow for positive and negative selection based on multiple markers simultaneously.

In some embodiments, the preparation methods include steps for freezing, e.g., cryopreserving, the cells, either before or after isolation, incubation, and/or engineering. In some embodiments, the freeze and subsequent thaw step removes granulocytes and, to some extent, monocytes in the cell population. In some embodiments, the cells are suspended in a freezing solution, e.g., following a washing step to remove plasma and platelets. Any of a variety of known freezing solutions and parameters in some aspects may be used. One example involves using PBS containing 20% DMSO and 8% human serum albumin (HSA), or other suitable cell freezing media. This is then diluted 1:1 with media so that the final concentration of DMSO and HSA are 10% and 4%, respectively. In some embodiments, the cell compositions are stored in a formulation containing at or about 5%, 6%, 7%, 7.5%, 8%, 9% or 10% dimethylsulfoxide, or a range defined by any of the foregoing, such as at or about 7.5% DMSO. In some aspects, the compositions are stored in a formulation containing at or about 0.5%, 1%, 2% or 2.5% (v/v) of 25% human albumin, or a range defined by any of the foregoing, such as at or about 1% (v/v) 25% human albumin. The cells are generally then frozen to -80° C. at a rate of 1° per minute and stored in the vapor phase of a liquid nitrogen storage tank.

In some embodiments, the isolation and/or selection results in one or more input compositions of enriched T cells, e.g., CD3+ T cells, CD4+ T cells, and/or CD8+ T cells. In some embodiments, two or more separate input composition are isolated, selected, enriched, or obtained from a single biological sample. In some embodiments, separate input compositions are isolated, selected, enriched, and/or obtained from separate biological samples collected, taken, and/or obtained from the same subject.

In certain embodiments, the one or more input compositions is or includes a composition of enriched T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD3+ T cells. In particular embodiment, the input composition of enriched T cells consists essentially of CD3+ T cells.

In certain embodiments, the one or more input compositions is or includes a composition of enriched CD4+ T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD4+ T cells. In certain embodiments, the input composition of CD4+ T cells includes less than 40%, less than 35%, less than 30%, less than 25%, less than 20%, less than 15%, less than 10%, less than 5%, less than 1%, less than 0.1%, or less than 0.01% CD8+ T cells, and/or contains no CD8+ T cells, and/or is free or substantially free of CD8+ T cells. In some embodiments, the composition of enriched T cells consists essentially of CD4+ T cells.

In certain embodiments, the one or more compositions is or includes a composition of CD8+ T cells that is or includes at least 60%, at least 65%, at least 70%, at least 75%, at least 80%, at least 85%, at least 90%, at least 95%, at least 98%, at least 99%, at least 99.5%, at least 99.9%, or at or at about 100% CD8+ T cells. In certain embodiments, the composition of CD8+ T cells contains less than 40%, less than 35%, less than 30%, less than 25%, less than 20%, less than 15%, less than 10%, less than 5%, less than 1%, less than 0.1%, or less than 0.01% CD4+ T cells, and/or contains no CD4+ T cells, and/or is free of or substantially free of CD4+ T cells. In some embodiments, the composition of enriched T cells consists essentially of CD8+ T cells.

### 2. Activation and Stimulation

In some embodiments, the cells are incubated and/or cultured prior to or in connection with genetic engineering. The incubation steps can include culture, cultivation, stimulation, activation, and/or propagation. The incubation and/or engineering may be carried out in a culture vessel, such as a unit, chamber, well, column, tube, tubing set, valve, vial, culture dish, bag, or other container for culture or cultivating cells. In some embodiments, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some embodiments, the stimulating conditions or agents include one or more agent, e.g., ligand, which is capable of stimulating or activating an intracellular signaling domain of a TCR complex. In some aspects, the agent turns on or initiates TCR/CD3 intracellular signaling cascade in a T cell. Such agents can include antibodies, such as those specific for a TCR, e.g. anti-CD3. In some embodiments, the stimulating conditions include one or more agent, e.g. ligand, which is capable of stimulating a costimulatory receptor, e.g., anti-CD28. In some embodiments, such agents and/or ligands may be, bound to solid support such as a bead, and/or one or more cytokines. Optionally, the expansion method may further comprise the step of adding anti-CD3 and/or anti-CD28 antibody to the culture medium (e.g., at a concentration of at least about 0.5 ng/ml). In some embodiments, the stimulating agents include IL-2, IL-15 and/or IL-7. In some aspects, the IL-2 concentration is at least about 10 units/mL.

In some aspects, incubation is carried out in accordance with techniques such as those described in US Patent No. 6,040,177 to Riddell et al., Klebanoff et al.(2012) J Immunother. 35(9): 651-660, Terakura et al. (2012) Blood.1:72-82, and/or Wang et al. (2012) J Immunother. 35(9):689-701.

In some embodiments, the T cells are expanded by adding to a culture-initiating composition feeder cells, such as non-dividing peripheral blood mononuclear cells (PBMC), (e.g., such that the resulting population of cells contains at least about 5, 10, 20, or 40 or more PBMC feeder cells for each T lymphocyte in the initial population to be expanded); and incubating the culture (e.g. for a time sufficient to expand the numbers of T cells). In some aspects, the non-dividing feeder cells can comprise gamma-irradiated PBMC feeder cells. In some embodiments, the PBMC are irradiated with gamma rays in the range of about 3000 to 3600 rads to prevent cell division. In some aspects, the feeder cells are added to culture medium prior to the addition of the populations of T cells.

In some embodiments, the stimulating conditions include temperature suitable for the growth of human T lymphocytes, for example, at least about 25 degrees Celsius, generally at least about 30 degrees, and generally at or about 37 degrees Celsius. Optionally, the incubation may further comprise adding non-dividing EBV-transformed lymphoblastoid cells (LCL) as feeder cells. LCL can be irradiated with gamma rays in the range of about 6000 to 10,000 rads. The LCL feeder cells in some aspects is provided in any suitable amount, such as a ratio of LCL feeder cells to initial T lymphocytes of at least about 10:1.

In embodiments, antigen-specific T cells, such as antigen-specific CD4⁺ and/or CD8⁺ T cells, are obtained by stimulating naive or antigen specific T lymphocytes with antigen. For example, antigen-specific T cell lines or clones can be generated to cytomegalovirus antigens by isolating T cells from infected subjects and stimulating the cells in vitro with the same antigen.

In some embodiments, at least a portion of the incubation in the presence of one or more stimulating conditions or a stimulatory agents is carried out in the internal cavity of a centrifugal chamber, for example, under centrifugal rotation, such as described in International Publication Number WO2016/073602. In some embodiments, at least a portion of the incubation performed in a centrifugal chamber includes mixing with a reagent or reagents to induce stimulation and/or activation. In some embodiments, cells, such as selected cells, are mixed with a stimulating condition or stimulatory agent in the centrifugal chamber. In some aspects of such processes, a volume of cells is mixed with an amount of one or more stimulating conditions or agents that is far less than is normally employed when performing similar stimulations in a cell culture plate or other system.

In some embodiments, the stimulating agent is added to cells in the cavity of the chamber in an amount that is substantially less than (e.g. is no more than 5%, 10%, 20%, 30%, 40%, 50%, 60%, 70% or 80% of the amount) as compared to the amount of the stimulating agent that is typically used or would be necessary to achieve about the same or similar efficiency of selection of the same number of cells or the same volume of cells when selection is performed without mixing in a centrifugal chamber, e.g. in a tube or bag with periodic shaking or rotation. In some embodiments, the incubation is performed with the addition of an incubation buffer to the cells and stimulating agent to achieve a target volume with incubation of the reagent of, for example, 10 mL to 200 mL, such as at least or at least about or about or 10 mL, 20 mL, 30 mL, 40 mL, 50 mL, 60 mL, 70 mL, 80 mL, 90 mL, 100 mL, 150 mL or 200 mL. In some embodiments, the incubation buffer and stimulating agent are pre-mixed before addition to the cells. In some embodiments, the incubation buffer and stimulating agent are separately added to the cells. In some embodiments, the stimulating incubation is carried out with periodic gentle mixing condition, which can aid in promoting energetically favored interactions and thereby permit the use of less overall stimulating agent while achieving stimulating and activation of cells.

In some embodiments, the incubation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some embodiments, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In some embodiments, the total duration of the incubation, e.g. with the stimulating agent, is between or between about 1 hour and 96 hours, 1 hour and 72 hours, 1 hour and 48 hours, 4 hours and 36 hours, 8 hours and 30 hours or 12 hours and 24 hours, such as at least or at least about 6 hours, 12 hours, 18 hours, 24 hours, 36 hours or 72 hours. In some embodiments, the further incubation is for a time between or about between 1 hour and 48 hours, 4 hours and 36 hours, 8 hours and 30 hours or 12 hours and 24 hours, inclusive.

In particular embodiments, the stimulating conditions include incubating, culturing, and/or cultivating a composition of enriched T cells with and/or in the presence of one or more cytokines. In particular embodiments, the one or more cytokines are recombinant cytokines. In some embodiments, the one or more cytokines are human recombinant cytokines. In certain embodiments, the one or more cytokines bind to and/or are capable of binding to receptors that are expressed by and/or are endogenous to T cells. In particular embodiments, the one or more cytokines is or includes a member of the 4-alpha-helix bundle family of cytokines. In some embodiments, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF).

In some embodiments, the stimulation results in activation and/or proliferation of the cells, for example, prior to transduction.

### 3. Vectors and Methods for Genetic Engineering

In some embodiments, engineered cells, such as T cells, used in connection with the provided methods, uses, articles of manufacture or compositions are cells have been genetically engineered to express a recombinant receptor, e.g., a CAR or a TCR described herein. In some embodiments, the cells are engineered by introduction, delivery or transfer of nucleic acid sequences that encode the recombinant receptor and/or other molecules.

In some embodiments, methods for producing engineered cells includes the introduction of a polynucleotide encoding a recombinant receptor (e.g. anti-CD19 CAR) into a cell, e.g., such as a stimulated or activated cell. In particular embodiments, the recombinant proteins are recombinant receptors, such as any described. Introduction of the nucleic acid molecules encoding the recombinant protein, such as recombinant receptor, in the cell may be carried out using any of a number of known vectors. Such vectors include viral and non-viral systems, including lentiviral and gammaretroviral systems, as well as transposon-based systems such as PiggyBac or Sleeping Beauty-based gene transfer systems. Exemplary methods include those for transfer of nucleic acids encoding the receptors, including via viral, e.g., retroviral or lentiviral, transduction, transposons, and electroporation. In some embodiments, the engineering produces one or more engineered compositions of enriched T cells.

In certain embodiments, the one or more compositions of stimulated T cells are or include two separate stimulated compositions of enriched T cells. In particular embodiments, two separate compositions of enriched T cells, e.g., two separate compositions of enriched T cells that have been selected, isolated, and/or enriched from the same biological sample, are separately engineered. In certain embodiments, the two separate compositions include a composition of enriched CD4+ T cells. In particular embodiments, the two separate compositions include a composition of enriched CD8+ T cells. In some embodiments, two separate compositions of enriched CD4+ T cells and enriched CD8+ T cells are genetically engineered separately.

In some embodiments, gene transfer is accomplished by first stimulating the cell, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications. In certain embodiments, the gene transfer is accomplished by first incubating the cells under stimulating conditions, such as by any of the methods described.

In some embodiments, methods for genetic engineering are carried out by contacting one or more cells of a composition with a nucleic acid molecule encoding the recombinant protein, e.g. recombinant receptor. In some embodiments, the contacting can be effected with centrifugation, such as spinoculation (e.g. centrifugal inoculation). Such methods include any of those as described in International Publication Number WO2016/073602. Exemplary centrifugal chambers include those produced and sold by Biosafe SA, including those for use with the Sepax^{®} and Sepax^{®} 2 system, including an A-200/F and A-200 centrifugal chambers and various kits for use with such systems. Exemplary chambers, systems, and processing instrumentation and cabinets are described, for example, in US Patent No. 6,123,655, US Patent No. 6,733,433 and Published U.S. Patent Application, Publication No.: US 2008/0171951, and published international patent application, publication no. WO 00/38762, the contents of each of which are incorporated herein by reference in their entirety. Exemplary kits for use with such systems include, but are not limited to, single-use kits sold by BioSafe SA under product names CS-430.1, CS-490.1, CS-600.1 or CS-900.2.

In some embodiments, the contacting can be effected with centrifugation, such as spinoculation (e.g., centrifugal inoculation). In some embodiments, the composition containing cells, the vector, e.g., viral particles and reagent can be rotated, generally at relatively low force or speed, such as speed lower than that used to pellet the cells, such as from or from about 600 rpm to 1700 rpm (e.g., at or about or at least 600 rpm, 1000 rpm, or 1500 rpm or 1700 rpm). In some embodiments, the rotation is carried at a force, e.g., a relative centrifugal force, of from or from about 100 g to 3200 g (e.g., at or about or at least at or about 100 g, 200 g, 300 g, 400 g, 500 g, 1000 g, 1500 g, 2000 g, 2500 g, 3000 g or 3200 g), as measured for example at an internal or external wall of the chamber or cavity. The term "relative centrifugal force" or RCF is generally understood to be the effective force imparted on an object or substance (such as a cell, sample, or pellet and/or a point in the chamber or other container being rotated), relative to the earth's gravitational force, at a particular point in space as compared to the axis of rotation. The value may be determined using well-known formulas, taking into account the gravitational force, rotation speed and the radius of rotation (distance from the axis of rotation and the object, substance, or particle at which RCF is being measured).

In some embodiments, the system is included with and/or placed into association with other instrumentation, including instrumentation to operate, automate, control and/or monitor aspects of the transduction step and one or more various other processing steps performed in the system, e.g. one or more processing steps that can be carried out with or in connection with the centrifugal chamber system as described herein or in International Publication Number WO2016/073602. This instrumentation in some embodiments is contained within a cabinet. In some embodiments, the instrumentation includes a cabinet, which includes a housing containing control circuitry, a centrifuge, a cover, motors, pumps, sensors, displays, and a user interface. An exemplary device is described in US Patent No. 6,123,655, US Patent No. 6,733,433 and US 2008/0171951.

In some embodiments, the system comprises a series of containers, e.g., bags, tubing, stopcocks, clamps, connectors, and a centrifuge chamber. In some embodiments, the containers, such as bags, include one or more containers, such as bags, containing the cells to be transduced and the viral vector particles, in the same container or separate containers, such as the same bag or separate bags. In some embodiments, the system further includes one or more containers, such as bags, containing medium, such as diluent and/or wash solution, which is pulled into the chamber and/or other components to dilute, resuspend, and/or wash components and/or compositions during the methods. The containers can be connected at one or more positions in the system, such as at a position corresponding to an input line, diluent line, wash line, waste line and/or output line.

In some embodiments, the chamber is associated with a centrifuge, which is capable of effecting rotation of the chamber, such as around its axis of rotation. Rotation may occur before, during, and/or after the incubation in connection with transduction of the cells and/or in one or more of the other processing steps. Thus, in some embodiments, one or more of the various processing steps is carried out under rotation, e.g., at a particular force. The chamber is typically capable of vertical or generally vertical rotation, such that the chamber sits vertically during centrifugation and the side wall and axis are vertical or generally vertical, with the end wall(s) horizontal or generally horizontal.

In some embodiments, during at least a part of the genetic engineering, e.g. transduction, and/or subsequent to the genetic engineering the cells are transferred to a bioreactor bag assembly for culture of the genetically engineered cells, such as for cultivation or expansion of the cells.

In some embodiments, recombinant nucleic acids are transferred into cells using recombinant infectious virus particles, such as, e.g., vectors derived from simian virus 40 (SV40), adenoviruses, adeno-associated virus (AAV). In some embodiments, recombinant nucleic acids are transferred into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557.

In some embodiments, the retroviral vector has a long terminal repeat sequence (LTR), e.g., a retroviral vector derived from the Moloney murine leukemia virus (MoMLV), myeloproliferative sarcoma virus (MPSV), murine embryonic stem cell virus (MESV), murine stem cell virus (MSCV) or spleen focus forming virus (SFFV). Most retroviral vectors are derived from murine retroviruses. In some embodiments, the retroviruses include those derived from any avian or mammalian cell source. The retroviruses typically are amphotropic, meaning that they are capable of infecting host cells of several species, including humans. In one embodiment, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505.

In some embodiments, the viral vector particles contain a genome derived from a retroviral genome based vector, such as derived from a lentiviral genome based vector. In some aspects of the provided viral vectors, the heterologous nucleic acid encoding a recombinant receptor, such as an antigen receptor, such as a CAR, is contained and/or located between the 5' LTR and 3' LTR sequences of the vector genome.

In some embodiments, the viral vector genome is a lentivirus genome, such as an HIV-1 genome or an SIV genome. For example, lentiviral vectors have been generated by multiply attenuating virulence genes, for example, the genes env, vif, vpu and nef can be deleted, making the vector safer for therapeutic purposes. Lentiviral vectors are known. See Naldini et al., (1996 and 1998); Zufferey et al., (1997); Dull et al., 1998, U.S. Pat. Nos. 6,013,516; and 5,994,136). In some embodiments, these viral vectors are plasmid-based or virus-based, and are configured to carry the essential sequences for incorporating foreign nucleic acid, for selection, and for transfer of the nucleic acid into a host cell. Known lentiviruses can be readily obtained from depositories or collections such as the American Type Culture Collection ("ATCC"; 10801 University Blvd., Manassas, Va. 20110-2209), or isolated from known sources using commonly available techniques.

Non-limiting examples of lentiviral vectors include those derived from a lentivirus, such as Human Immunodeficiency Virus 1 (HIV-1), HIV-2, an Simian Immunodeficiency Virus (SIV), Human T-lymphotropic virus 1 (HTLV-1), HTLV-2 or equine infection anemia virus (E1AV). For example, lentiviral vectors have been generated by multiply attenuating the HIV virulence genes, for example, the genes env, vif, vpr, vpu and nef are deleted, making the vector safer for therapeutic purposes. Lentiviral vectors are known in the art, see Naldini et al., (1996 and 1998); Zufferey et al., (1997); Dull et al., 1998, U.S. Pat. Nos. 6,013,516; and 5,994,136). In some embodiments, these viral vectors are plasmid-based or virus-based, and are configured to carry the essential sequences for incorporating foreign nucleic acid, for selection, and for transfer of the nucleic acid into a host cell. Known lentiviruses can be readily obtained from depositories or collections such as the American Type Culture Collection ("ATCC"; 10801 University Blvd., Manassas, Va. 20110-2209), or isolated from known sources using commonly available techniques.

In some embodiments, the viral genome vector can contain sequences of the 5' and 3' LTRs of a retrovirus, such as a lentivirus. In some aspects, the viral genome construct may contain sequences from the 5' and 3' LTRs of a lentivirus, and in particular can contain the R and U5 sequences from the 5' LTR of a lentivirus and an inactivated or self-inactivating 3' LTR from a lentivirus. The LTR sequences can be LTR sequences from any lentivirus from any species. For example, they may be LTR sequences from HIV, SIV, FIV or BIV. Typically, the LTR sequences are HIV LTR sequences.

In some embodiments, the nucleic acid of a viral vector, such as an HIV viral vector, lacks additional transcriptional units. The vector genome can contain an inactivated or self-inactivating 3' LTR (Zufferey et al. J Virol 72: 9873, 1998; Miyoshi et al., J Virol 72:8150, 1998). For example, deletion in the U3 region of the 3' LTR of the nucleic acid used to produce the viral vector RNA can be used to generate self-inactivating (SIN) vectors. This deletion can then be transferred to the 5' LTR of the proviral DNA during reverse transcription. A self-inactivating vector generally has a deletion of the enhancer and promoter sequences from the 3' long terminal repeat (LTR), which is copied over into the 5' LTR during vector integration. In some embodiments enough sequence can be eliminated, including the removal of a TATA box, to abolish the transcriptional activity of the LTR. This can prevent production of full-length vector RNA in transduced cells. In some aspects, the U3 element of the 3' LTR contains a deletion of its enhancer sequence, the TATA box, Sp1, and NF-kappa B sites. As a result of the self-inactivating 3' LTR, the provirus that is generated following entry and reverse transcription contains an inactivated 5' LTR. This can improve safety by reducing the risk of mobilization of the vector genome and the influence of the LTR on nearby cellular promoters. The self-inactivating 3' LTR can be constructed by any method known in the art. In some embodiments, this does not affect vector titers or the in vitro or in vivo properties of the vector.

Optionally, the U3 sequence from the lentiviral 5' LTR can be replaced with a promoter sequence in the viral construct, such as a heterologous promoter sequence. This can increase the titer of virus recovered from the packaging cell line. An enhancer sequence can also be included. Any enhancer/promoter combination that increases expression of the viral RNA genome in the packaging cell line may be used. In one example, the CMV enhancer/promoter sequence is used (U.S. Pat. No. 5,385,839 and U.S. Pat. No. 5,168,062).

In certain embodiments, the risk of insertional mutagenesis can be minimized by constructing the retroviral vector genome, such as lentiviral vector genome, to be integration defective. A variety of approaches can be pursued to produce a non-integrating vector genome. In some embodiments, a mutation(s) can be engineered into the integrase enzyme component of the pol gene, such that it encodes a protein with an inactive integrase. In some embodiments, the vector genome itself can be modified to prevent integration by, for example, mutating or deleting one or both attachment sites, or making the 3' LTR-proximal polypurine tract (PPT) non-functional through deletion or modification. In some embodiments, non-genetic approaches are available; these include pharmacological agents that inhibit one or more functions of integrase. The approaches are not mutually exclusive; that is, more than one of them can be used at a time. For example, both the integrase and attachment sites can be non-functional, or the integrase and PPT site can be non-functional, or the attachment sites and PPT site can be non-functional, or all of them can be non-functional. Such methods and viral vector genomes are known and available (see Philpott and Thrasher, Human Gene Therapy 18:483, 2007; Engelman et al. J Virol 69:2729, 1995; Brown et al J Virol 73:9011 (1999); WO 2009/076524; McWilliams et al., J Virol 77:11150, 2003; Powell and Levin J Virol 70:5288, 1996).

In some embodiments, the vector contains sequences for propagation in a host cell, such as a prokaryotic host cell. In some embodiments, the nucleic acid of the viral vector contains one or more origins of replication for propagation in a prokaryotic cell, such as a bacterial cell. In some embodiments, vectors that include a prokaryotic origin of replication also may contain a gene whose expression confers a detectable or selectable marker such as drug resistance.

The viral vector genome is typically constructed in a plasmid form that can be transfected into a packaging or producer cell line. Any of a variety of known methods can be used to produce retroviral particles whose genome contains an RNA copy of the viral vector genome. In some embodiments, at least two components are involved in making a virus-based gene delivery system: first, packaging plasmids, encompassing the structural proteins as well as the enzymes necessary to generate a viral vector particle, and second, the viral vector itself, i.e., the genetic material to be transferred. Biosafety safeguards can be introduced in the design of one or both of these components.

In some embodiments, the packaging plasmid can contain all retroviral, such as HIV-1, proteins other than envelope proteins (Naldini et al., 1998). In other embodiments, viral vectors can lack additional viral genes, such as those that are associated with virulence, e.g., vpr, vif, vpu and nef, and/or Tat, a primary transactivator of HIV. In some embodiments, lentiviral vectors, such as HIV-based lentiviral vectors, comprise only three genes of the parental virus: gag, pol and rev, which reduces or eliminates the possibility of reconstitution of a wild-type virus through recombination.

In some embodiments, the viral vector genome is introduced into a packaging cell line that contains all the components necessary to package viral genomic RNA, transcribed from the viral vector genome, into viral particles. Alternatively, the viral vector genome may comprise one or more genes encoding viral components in addition to the one or more sequences, e.g., recombinant nucleic acids, of interest. In some aspects, in order to prevent replication of the genome in the target cell, however, endogenous viral genes required for replication are removed and provided separately in the packaging cell line.

In some embodiments, a packaging cell line is transfected with one or more plasmid vectors containing the components necessary to generate the particles. In some embodiments, a packaging cell line is transfected with a plasmid containing the viral vector genome, including the LTRs, the cis-acting packaging sequence and the sequence of interest, i.e. a nucleic acid encoding an antigen receptor, such as a CAR; and one or more helper plasmids encoding the virus enzymatic and/or structural components, such as Gag, pol and/or rev. In some embodiments, multiple vectors are utilized to separate the various genetic components that generate the retroviral vector particles. In some such embodiments, providing separate vectors to the packaging cell reduces the chance of recombination events that might otherwise generate replication competent viruses. In some embodiments, a single plasmid vector having all of the retroviral components can be used.

In some embodiments, the retroviral vector particle, such as lentiviral vector particle, is pseudotyped to increase the transduction efficiency of host cells. For example, a retroviral vector particle, such as a lentiviral vector particle, in some embodiments is pseudotyped with a VSV-G glycoprotein, which provides a broad cell host range extending the cell types that can be transduced. In some embodiments, a packaging cell line is transfected with a plasmid or polynucleotide encoding a non-native envelope glycoprotein, such as to include xenotropic, polytropic or amphotropic envelopes, such as Sindbis virus envelope, GALV or VSV-G.

In some embodiments, the packaging cell line provides the components, including viral regulatory and structural proteins, that are required in trans for the packaging of the viral genomic RNA into lentiviral vector particles. In some embodiments, the packaging cell line may be any cell line that is capable of expressing lentiviral proteins and producing functional lentiviral vector particles. In some aspects, suitable packaging cell lines include 293 (ATCC CCL X), 293T, HeLA (ATCC CCL 2), D17 (ATCC CCL 183), MDCK (ATCC CCL 34), BHK (ATCC CCL-10) and Cf2Th (ATCC CRL 1430) cells.

In some embodiments, the packaging cell line stably expresses the viral protein(s). For example, in some aspects, a packaging cell line containing the gag, pol, rev and/or other structural genes but without the LTR and packaging components can be constructed. In some embodiments, a packaging cell line can be transiently transfected with nucleic acid molecules encoding one or more viral proteins along with the viral vector genome containing a nucleic acid molecule encoding a heterologous protein, and/or a nucleic acid encoding an envelope glycoprotein.

In some embodiments, the viral vectors and the packaging and/or helper plasmids are introduced via transfection or infection into the packaging cell line. The packaging cell line produces viral vector particles that contain the viral vector genome. Methods for transfection or infection are well known. Non-limiting examples include calcium phosphate, DEAE-dextran and lipofection methods, electroporation and microinjection.

When a recombinant plasmid and the retroviral LTR and packaging sequences are introduced into a special cell line (e.g., by calcium phosphate precipitation for example), the packaging sequences may permit the RNA transcript of the recombinant plasmid to be packaged into viral particles, which then may be secreted into the culture media. The media containing the recombinant retroviruses in some embodiments is then collected, optionally concentrated, and used for gene transfer. For example, in some aspects, after cotransfection of the packaging plasmids and the transfer vector to the packaging cell line, the viral vector particles are recovered from the culture media and titered by standard methods used by those of skill in the art.

In some embodiments, a retroviral vector, such as a lentiviral vector, can be produced in a packaging cell line, such as an exemplary HEK 293T cell line, by introduction of plasmids to allow generation of lentiviral particles. In some embodiments, a packaging cell is transfected and/or contains a polynucleotide encoding gag and pol, and a polynucleotide encoding a recombinant receptor, such as an antigen receptor, for example, a CAR. In some embodiments, the packaging cell line is optionally and/or additionally transfected with and/or contains a polynucleotide encoding a rev protein. In some embodiments, the packaging cell line is optionally and/or additionally transfected with and/or contains a polynucleotide encoding a non-native envelope glycoprotein, such as VSV-G. In some such embodiments, approximately two days after transfection of cells, *e.g.,* HEK 293T cells, the cell supernatant contains recombinant lentiviral vectors, which can be recovered and titered.

Recovered and/or produced retroviral vector particles can be used to transduce target cells using the methods as described. Once in the target cells, the viral RNA is reverse-transcribed, imported into the nucleus and stably integrated into the host genome. One or two days after the integration of the viral RNA, the expression of the recombinant protein, e.g., antigen receptor, such as CAR, can be detected.

In some embodiments, the provided methods involve methods of transducing cells by contacting, e.g., incubating, a cell composition comprising a plurality of cells with a viral particle. In some embodiments, the cells to be transfected or transduced are or comprise primary cells obtained from a subject, such as cells enriched and/or selected from a subject.

In some embodiments, the concentration of cells to be transduced of the composition is from or from about 1.0 x 10⁵ cells/mL to 1.0 x 10⁸ cells/mL, such as at least or at least about or about 1.0 x 10⁵ cells/mL, 5 x 10⁵ cells/mL, 1 x 10⁶ cells/mL, 5 x 10⁶ cells/mL, 1 x 10⁷ cells/mL, 5 x 10⁷ cells/mL or 1 x 10⁸ cells/mL.

In some embodiments, the viral particles are provided at a certain ratio of copies of the viral vector particles or infectious units (IU) thereof, per total number of cells to be transduced (IU/cell). For example, in some embodiments, the viral particles are present during the contacting at or about or at least at or about 0.5, 1, 2, 3, 4, 5, 10, 15, 20, 30, 40, 50, or 60 IU of the viral vector particles per one of the cells.

In some embodiments, the titer of viral vector particles is between or between about 1 x 10⁶ IU/mL and 1 x 10⁸ IU/mL, such as between or between about 5 x 10⁶ IU/mL and 5 x 10⁷ IU/mL, such as at least 6 x 10⁶ IU/mL, 7 x 10⁶ IU/mL, 8 x 10⁶ IU/mL, 9 x 10⁶ IU/mL, 1 x 10⁷ IU/mL, 2 x 10⁷ IU/mL, 3 x 10⁷ IU/mL, 4 x 10⁷ IU/mL, or 5 x10⁷ IU/mL.

In some embodiments, transduction can be achieved at a multiplicity of infection (MOI) of less than 100, such as generally less than 60, 50, 40, 30, 20, 10, 5 or less.

In some embodiments, the method involves contacting or incubating, the cells with the viral particles. In some embodiments, the contacting is for 30 minutes to 72 hours, such as 30 minute to 48 hours, 30 minutes to 24 hours or 1 hour to 24 hours, such as at least or at least about 30 minutes, 1 hour, 2 hours, 6 hours, 12 hours, 24 hours, 36 hours or more.

In some embodiments, contacting is performed in solution. In some embodiments, the cells and viral particles are contacted in a volume of from or from about 0.5 mL to 500 mL, such as from or from about 0.5 mL to 200 mL, 0.5 mL to 100 mL, 0.5 mL to 50 mL, 0.5 mL to 10 mL, 0.5 mL to 5 mL, 5 mL to 500 mL, 5 mL to 200 mL, 5 mL to 100 mL, 5 mL to 50 mL, 5 mL to 10 mL, 10 mL to 500 mL, 10 mL to 200 mL, 10 mL to 100 mL, 10 mL to 50 mL, 50 mL to 500 mL, 50 mL to 200 mL, 50 mL to 100 mL, 100 mL to 500 mL, 100 mL to 200 mL or 200 mL to 500 mL.

In certain embodiments, the input cells are treated, incubated, or contacted with particles that comprise binding molecules that bind to or recognize the recombinant receptor that is encoded by the viral DNA.

In some embodiments, the incubation of the cells with the viral vector particles results in or produces an output composition comprising cells transduced with the viral vector particles.

In some embodiments, recombinant nucleic acids are transferred into T cells via electroporation (see, e.g., Chicaybam et al, (2013) PLoS ONE 8(3): e60298 and Van Tedeloo et al. (2000) Gene Therapy 7(16): 1431-1437). In some embodiments, recombinant nucleic acids are transferred into T cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material in immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA coprecipitation (Brash et al., Mol. Cell Biol., 7: 2031-2034 (1987)).

Other approaches and vectors for transfer of the nucleic acids encoding the recombinant products are those described, e.g., in international patent application, Publication No.: WO2014055668, and U.S. Patent No. 7,446,190.

In some embodiments, the cells, e.g., T cells, may be transfected either during or after expansion e.g. with a T cell receptor (TCR) or a chimeric antigen receptor (CAR). This transfection for the introduction of the gene of the desired receptor can be carried out with any suitable retroviral vector, for example. The genetically modified cell population can then be liberated from the initial stimulus (the anti-CD3/anti-CD28 stimulus, for example) and subsequently be stimulated with a second type of stimulus e.g. via a de novo introduced receptor). This second type of stimulus may include an antigenic stimulus in form of a peptide/MHC molecule, the cognate (cross-linking) ligand of the genetically introduced receptor (e.g. natural ligand of a CAR) or any ligand (such as an antibody) that directly binds within the framework of the new receptor (e.g. by recognizing constant regions within the receptor). See, for example, Cheadle et al, "Chimeric antigen receptors for T-cell based therapy" Methods Mol Biol. 2012; 907:645-66 or Barrett et al., Chimeric Antigen Receptor Therapy for Cancer Annual Review of Medicine Vol. 65: 333-347 (2014).

In some cases, a vector may be used that does not require that the cells, e.g., T cells, are activated. In some such instances, the cells may be selected and/or transduced prior to activation. Thus, the cells may be engineered prior to, or subsequent to culturing of the cells, and in some cases at the same time as or during at least a portion of the culturing.

Among additional nucleic acids, e.g., genes for introduction are those to improve the efficacy of therapy, such as by promoting viability and/or function of transferred cells; genes to provide a genetic marker for selection and/or evaluation of the cells, such as to assess *in vivo* survival or localization; genes to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also the publications of PCT/US91/08442 and PCT/US94/05601 by Lupton et al. describing the use of bifunctional selectable fusion genes derived from fusing a dominant positive selectable marker with a negative selectable marker. *See,* e.g., Riddell et al., US Patent No. 6,040,177, at columns 14-17.

### 4. Cultivation, Expansion and Formulation of Engineered Cells

In some embodiments, the methods for generating the engineered cells, e.g., for cell therapy in accord with any of provided methods, uses, articles of manufacture or compositions, include one or more steps for cultivating cells, e.g., cultivating cells under conditions that promote proliferation and/or expansion. In some embodiments, cells are cultivated under conditions that promote proliferation and/or expansion subsequent to a step of genetically engineering, e.g., introducing a recombinant polypeptide to the cells by transduction or transfection. In particular embodiments, the cells are cultivated after the cells have been incubated under stimulating conditions and transduced or transfected with a recombinant polynucleotide, e.g., a polynucleotide encoding a recombinant receptor. Thus, in some embodiments, a composition of CAR-positive T cells that has been engineered by transduction or transfection with a recombinant polynucleotide encoding the CAR, is cultivated under conditions that promote proliferation and/or expansion.

In certain embodiments, the one or more compositions of engineered T cells are or include two separate compositions of enriched T cells, such as two separate compositions of enriched T cells that have been engineered with a polynucleotide encoding a recombinant receptor, e.g. a CAR. In particular embodiments, two separate compositions of enriched T cells, e.g., two separate compositions of enriched T cells selected, isolated, and/or enriched from the same biological sample, are separately cultivated under stimulating conditions, such as subsequent to a step of genetically engineering, e.g., introducing a recombinant polypeptide to the cells by transduction or transfection. In certain embodiments, the two separate compositions include a composition of enriched CD4+ T cells, such as a composition of enriched CD4+ T cells that have been engineered with a polynucleotide encoding a recombinant receptor, e.g. a CAR. In particular embodiments, the two separate compositions include a composition of enriched CD8+ T cells, such as a composition of enriched CD4+ T cells that have been engineered with a polynucleotide encoding a recombinant receptor, e.g. a CAR. In some embodiments, two separate compositions of enriched CD4+ T cells and enriched CD8+ T cells, such as a composition of enriched CD4+ T cells and a composition of enriched CD8+ T cells that have each been separately engineered with a polynucleotide encoding a recombinant receptor, e.g. a CAR, are separately cultivated, e.g., under conditions that promote proliferation and/or expansion.

In some embodiments, cultivation is carried out under conditions that promote proliferation and/or expansion. In some embodiments, such conditions may be designed to induce proliferation, expansion, activation, and/or survival of cells in the population. In particular embodiments, the stimulating conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to promote growth, division, and/or expansion of the cells.

In particular embodiments, the cells are cultivated in the presence of one or more cytokines. In particular embodiments, the one or more cytokines are recombinant cytokines. In some embodiments, the one or more cytokines are human recombinant cytokines. In certain embodiments, the one or more cytokines bind to and/or are capable of binding to receptors that are expressed by and/or are endogenous to T cells. In particular embodiments, the one or more cytokines, e.g. a recombinant cytokine, is or includes a member of the 4-alpha-helix bundle family of cytokines. In some embodiments, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF). In some embodiments, the one or more recombinant cytokine includes IL-2, IL-7 and/or IL-15. In some embodiments, the cells, e.g., engineered cells, are cultivated in the presence of a cytokine, e.g., a recombinant human cytokine, at a concentration of between 1 IU/mL and 2,000 IU/mL, between 10 IU/mL and 100 IU/mL, between 50 IU/mL and 200 IU/mL, between 100 IU/mL and 500 IU/mL, between 100 IU/mL and 1,000 IU/mL, between 500 IU/mL and 2,000 IU/mL, or between 100 IU/mL and 1,500 IU/mL.

In some embodiments, the cultivation is performed under conditions that generally include a temperature suitable for the growth of primary immune cells, such as human T lymphocytes, for example, at least about 25 degrees Celsius, generally at least about 30 degrees, and generally at or about 37 degrees Celsius. In some embodiments, the composition of enriched T cells is incubated at a temperature of 25 to 38°C, such as 30 to 37°C, for example at or about 37 °C ± 2 °C. In some embodiments, the incubation is carried out for a time period until the culture, e.g. cultivation or expansion, results in a desired or threshold density, number or dose of cells. In some embodiments, the incubation is greater than or greater than about or is for about or 24 hours, 48 hours, 72 hours, 96 hours, 5 days, 6 days, 7 days, 8 days, 9 days or more.

In particular embodiments, the cultivation is performed in a closed system. In certain embodiments, the cultivation is performed in a closed system under sterile conditions. In particular embodiments, the cultivation is performed in the same closed system as one or more steps of the provided systems. In some embodiments the composition of enriched T cells is removed from a closed system and placed in and/or connected to a bioreactor for the cultivation. Examples of suitable bioreactors for the cultivation include, but are not limited to, GE Xuri W25, GE Xuri W5, Sartorius BioSTAT RM 20 | 50, Finesse SmartRocker Bioreactor Systems, and Pall XRS Bioreactor Systems. In some embodiments, the bioreactor is used to perfuse and/or mix the cells during at least a portion of the cultivation step.

In some embodiments, the mixing is or includes rocking and/or motioning. In some cases, the bioreactor can be subject to motioning or rocking, which, in some aspects, can increase oxygen transfer. Motioning the bioreactor may include, but is not limited to rotating along a horizontal axis, rotating along a vertical axis, a rocking motion along a tilted or inclined horizontal axis of the bioreactor or any combination thereof. In some embodiments, at least a portion of the incubation is carried out with rocking. The rocking speed and rocking angle may be adjusted to achieve a desired agitation. In some embodiments the rock angle is 20°, 19°, 18°, 17°, 16°, 15°, 14°, 13°, 12°, 11°, 10°, 9°, 8°, 7°, 6°, 5°, 4°, 3°, 2° or 1°. In certain embodiments, the rock angle is between 6-16°. In other embodiments, the rock angle is between 7-16°. In other embodiments, the rock angle is between 8-12°. In some embodiments, the rock rate is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 rpm. In some embodiments, the rock rate is between 4 and 12 rpm, such as between 4 and 6 rpm, inclusive.

In some embodiments, the bioreactor maintains the temperature at or near 37°C and CO2 levels at or near 5% with a steady air flow at, at about, or at least 0.01 L/min, 0.05 L/min, 0.1 L/min, 0.2 L/min, 0.3 L/min, 0.4 L/min, 0.5 L/min, 1.0 L/min, 1.5 L/min, or 2.0 L/min or greater than 2.0 L/min. In certain embodiments, at least a portion of the cultivation is performed with perfusion, such as with a rate of 290 ml/day, 580 ml/day, and/or 1160 ml/day, e.g., depending on the timing in relation to the start of the cultivation and/or density of the cultivated cells. In some embodiments, at least a portion of the cell culture expansion is performed with a rocking motion, such as at an angle of between 5° and 10°, such as 6°, at a constant rocking speed, such as a speed of between 5 and 15 RPM, such as 6 RMP or 10 RPM.

In some embodiments, the methods for manufacturing, generating or producing a cell therapy and/or engineered cells, in accord with the provided methods, uses or articles of manufacture, may include formulation of cells, such as formulation of genetically engineered cells resulting from the processing steps prior to or after the incubating, engineering, and cultivating, and/or one or more other processing steps as described. In some embodiments, one or more of the processing steps, including formulation of cells, can be carried out in a closed system. In some cases, the cells are processed in one or more steps (e.g. carried out in the centrifugal chamber and/or closed system) for manufacturing, generating or producing a cell therapy and/or engineered cells may include formulation of cells, such as formulation of genetically engineered cells resulting from the transduction processing steps prior to or after the culturing, e.g. cultivation and expansion, and/or one or more other processing steps as described. In some embodiments, the genetically engineered cells are formulated as unit dose form compositions including the number of cells for administration in a given dose or fraction thereof.

In some embodiments, the dose of cells comprising cells engineered with a recombinant antigen receptor, e.g. CAR or TCR, is provided as a composition or formulation, such as a pharmaceutical composition or formulation. Such compositions can be used in accord with the provided methods, such as in the treatment of diseases, conditions, and disorders, or in detection, diagnostic, and prognostic methods, and uses and articles of manufacture. In some cases, the cells can be formulated in an amount for dosage administration, such as for a single unit dosage administration or multiple dosage administration.

In some embodiments, the cells can be formulated into a container, such as a bag or vial. In some embodiments, the vial may be an infusion vial. In some embodiments, the vial is formulated with a single unit dose of the engineered cells, such as including the number of cells for administration in a given dose or fraction thereof.

In some embodiments, the cells are formulated in a pharmaceutically acceptable buffer, which may, in some aspects, include a pharmaceutically acceptable carrier or excipient. In some embodiments, the processing includes exchange of a medium into a medium or formulation buffer that is pharmaceutically acceptable or desired for administration to a subject. In some embodiments, the processing steps can involve washing the transduced and/or expanded cells to replace the cells in a pharmaceutically acceptable buffer that can include one or more optional pharmaceutically acceptable carriers or excipients. Exemplary of such pharmaceutical forms, including pharmaceutically acceptable carriers or excipients, can be any described below in conjunction with forms acceptable for administering the cells and compositions to a subject. The pharmaceutical composition in some embodiments contains the cells in amounts effective to treat or prevent the disease or condition, such as a therapeutically effective or prophylactically effective amount.

In some embodiments, the formulation buffer contains a cryopreservative. In some embodiments, the cell are formulated with a cyropreservative solution that contains 1.0% to 30% DMSO solution, such as a 5% to 20% DMSO solution or a 5% to 10% DMSO solution. In some embodiments, the cryopreservation solution is or contains, for example, PBS containing 20% DMSO and 8% human serum albumin (HSA), or other suitable cell freezing media. In some embodiments, the cryopreservative solution is or contains, for example, at least or about 7.5% DMSO. In some embodiments, the processing steps can involve washing the transduced and/or expanded cells to replace the cells in a cryopreservative solution. In some embodiments, the cells are frozen, e.g., cryoprotected or cryopreserved, in media and/or solution with a final concentration of or of about 12.5%, 12.0%, 11.5%, 11.0%, 10.5%, 10.0%, 9.5%, 9.0%, 8.5%, 8.0%, 7.5%, 7.0%, 6.5%, 6.0%, 5.5%, or 5.0% DMSO, or between 1% and 15%, between 6% and 12%, between 5% and 10%, or between 6% and 8% DMSO. In particular embodiments, the cells are frozen, e.g., cryoprotected or cryopreserved, in media and/or solution with a final concentration of or of about 5.0%, 4.5%, 4.0%, 3.5%, 3.0%, 2.5%, 2.0%, 1.5%, 1.25%, 1.0%, 0.75%, 0.5%, or 0.25% HSA, or between 0.1% and 5%, between 0.25% and 4%, between 0.5% and 2%, or between 1% and 2% HSA.

In some embodiments, the formulation is carried out using one or more processing step including washing, diluting or concentrating the cells, such as the cultured or expanded cells. In some embodiments, the processing can include dilution or concentration of the cells to a desired concentration or number, such as unit dose form compositions including the number of cells for administration in a given dose or fraction thereof. In some embodiments, the processing steps can include a volume-reduction to thereby increase the concentration of cells as desired. In some embodiments, the processing steps can include a volume-addition to thereby decrease the concentration of cells as desired. In some embodiments, the processing includes adding a volume of a formulation buffer to transduced and/or expanded cells. In some embodiments, the volume of formulation buffer is from or from about 10 mL to 1000 mL, such as at least or at least about or about or 50 mL, 100 mL, 200 mL, 300 mL, 400 mL, 500 mL, 600 mL, 700 mL, 800 mL, 900 mL or 1000 mL.

In some embodiments, such processing steps for formulating a cell composition is carried out in a closed system. Exemplary of such processing steps can be performed using a centrifugal chamber in conjunction with one or more systems or kits associated with a cell processing system, such as a centrifugal chamber produced and sold by Biosafe SA, including those for use with the Sepax^{®} or Sepax 2^{®} cell processing systems. An exemplary system and process is described in International Publication Number WO2016/073602. In some embodiments, the method includes effecting expression from the internal cavity of the centrifugal chamber a formulated composition, which is the resulting composition of cells formulated in a formulation buffer, such as pharmaceutically acceptable buffer, in any of the above embodiments as described. In some embodiments, the expression of the formulated composition is to a container, such as the vials of the biomedical material vessels described herein, that is operably linked as part of a closed system with the centrifugal chamber. In some embodiments, the biomedical material vessels are configured for integration and or operable connection and/or is integrated or operably connected, to a closed system or device that carries out one or more processing steps. In some embodiments, the biomedical material vessel is connected to a system at an output line or output position. In some cases, the closed system is connected to the vial of the biomedical material vessel at the inlet tube. Exemplary close systems for use with the biomedical material vessels described herein include the Sepax^{®} and Sepax^{®} 2 system.

In some embodiments, the closed system, such as associated with a centrifugal chamber or cell processing system, includes a multi-port output kit containing a multi-way tubing manifold associated at each end of a tubing line with a port to which one or a plurality of containers can be connected for expression of the formulated composition. In some aspects, a desired number or plurality of vials, can be sterilely connected to one or more, generally two or more, such as at least 3, 4, 5, 6, 7, 8 or more of the ports of the multi-port output. For example, in some embodiments, one or more containers, e.g., biomedical material vessels, can be attached to the ports, or to fewer than all of the ports. Thus, in some embodiments, the system can effect expression of the output composition into a plurality of vials of the biomedical material vessels.

In some aspects, cells can be expressed to the one or more of the plurality of output containers, e.g., vials, in an amount for dosage administration, such as for a single unit dosage administration or multiple dosage administration. For example, in some embodiments, the vials, may each contain the number of cells for administration in a given dose or fraction thereof. Thus, each vial, in some aspects, may contain a single unit dose for administration or may contain a fraction of a desired dose such that more than one of the plurality of vials, such as two of the vials, or 3 of the vials, together constitute a dose for administration. In some embodiments, 4 vials together constitute a dose for administration.

Thus, the containers, e.g. bags or vials, generally contain the cells to be administered, e.g., one or more unit doses thereof. The unit dose may be an amount or number of the cells to be administered to the subject or twice the number (or more) of the cells to be administered. It may be the lowest dose or lowest possible dose of the cells that would be administered to the subject. In some aspects, the provided articles of manufacture includes one or more of the plurality of output containers.

In some embodiments, each of the containers, e.g. bags or vials, individually comprises a unit dose of the cells. Thus in some embodiments, each of the containers comprises the same or approximately or substantially the same number of cells. In some embodiments, each unit dose contains at or about or at least or at least about 1 x 10⁶, 2 x 10⁶, 5 x 10⁶, 1 x 10⁷, 5 x 10⁷, or 1 x 10⁸ engineered cells, total cells, T cells, or PBMCs. In some embodiments, each unit dose contains at or about or at least or at least about 1 x 10⁶, 2 x 10⁶, 5 x 10⁶, 1 x 10⁷, 5 x 10⁷, or 1 x 10⁸ CAR+ T cells that are CD3+, such as CD4+ or CD8+, or a viable subset thereof.

In some embodiments, the volume of the formulated cell composition in each container, e.g. bag or vial, is between at or about 10 mL and at or about 100 mL, such as at or about or at least or at least about 20 mL, 30 mL, 40 mL, 50 mL, 60 mL, 70 mL, 80 mL, 90 mL or 100 mL. In some embodiments, the volume of the formulated cell composition in each container, e.g. bag or vial, is between at or about 1 mL and at or about 10 mL, such as between at or about 1 mL and at or about 5 mL. In some embodiments, the volume of the formulated cell composition in each container, e.g. bag or vial, is between at or about 4 mL and at or about 5 mL. In some embodiments, the volume of the formulated cell composition in each container, e.g. bag or vial, is or is about 4.4 mL. In some embodiments, the volume of the formulated cell composition in each container, e.g. bag or vial, is or is about 4.5 mL. In some embodiments, the volume of the formulated cell composition in each container, e.g. bag or vial, is or is about 4.6 mL. In some embodiments, the volume of the formulated cell composition in each container, e.g. bag or vial, is or is about 4.7 mL. In some embodiments, the volume of the formulated cell composition in each container, e.g. bag or vial, is or is about 4.8 mL. In some embodiments, the volume of the formulated cell composition in each container, e.g. bag or vial, is or is about 4.9 mL. In some embodiments, the volume of the formulated cell composition in each container, e.g. bag or vial, is or is about 5.0 mL.

In some embodiments, the formulated cell composition has a concentration of greater than at or about 0.5 x 10⁶ recombinant receptor-expressing (e.g. CAR⁺)/CD3+ cells or such viable cells per mL, greater than at or about 1.0 x 10⁶ recombinant receptor-expressing (e.g. CAR⁺)/CD3+ cells or such viable cells per mL, greater than at or about 1.5 x 10⁶ recombinant receptor-expressing (e.g. CAR⁺)/CD3+ cells or such viable cells per mL, greater than at or about 2.0 x 10⁶ recombinant receptor-expressing (e.g. CAR⁺)/CD3+ cells or such viable cells per mL. greater than at or about 2.5 x 10⁶ recombinant receptor-expressing (e.g. CAR⁺)/CD3+ cells or such viable cells per mL, greater than at or about 2.6 x 10⁶ recombinant receptor-expressing (e.g. CAR⁺)/CD3+ cells or such viable cells per mL, greater than at or about 2.7 x 10⁶ recombinant receptor-expressing (e.g. CAR⁺)/CD3+ cells or such viable cells per mL, greater than at or about 2.8 x 10⁶ recombinant receptor-expressing (e.g. CAR⁺)/CD3+ cells or such viable cells per mL, greater than at or about 2.9 x 10⁶ recombinant receptor-expressing (e.g. CAR⁺)/CD3+ cells or such viable cells per mL greater than at or about 3.0 x 10⁶ recombinant receptor-expressing (e.g. CAR⁺)/CD3+ cells or such viable cells per mL, greater than at or about 3.5 x 10⁶ recombinant receptor-expressing (e.g. CAR⁺)/CD3+ cells or such viable cells per mL, greater than at or about 4.0 x 10⁶ recombinant receptor-expressing (e.g. CAR⁺)/CD3+ cells or such viable cells per mL, greater than at or about 4.5 x 10⁶ recombinant receptor-expressing (e.g. CAR⁺)/CD3+ cells or such viable cells per mL or greater than at or about 5 x 10⁶ recombinant receptor-expressing (e.g. CAR⁺)/CD3+ cells or such viable cells per mL. In some embodiments, the CD3+ cells are CD4+ T cells. In some embodiments, the CD3+ cells are CD8+ T cells. In some embodiments, the CD3+ T cells are CD4+ and CD8+ T cells.

In some embodiments, the cells in the container, e.g. bag or vials, can be cryopreserved. In some embodiments, the container, e.g. vials, can be stored in liquid nitrogen until further use.

In some embodiments, such cells produced by the method, or a composition comprising such cells, are administered to a subject for treating a disease or condition, for example, in accord with the methods, uses and articles of manufacture described herein.

### IV. COMPOSITIONS AND FORMULATIONS

In some embodiments, the dose of cells comprising cells engineered with a recombinant antigen receptor, e.g. CAR or TCR, is provided as a composition or formulation, such as a pharmaceutical composition or formulation. Exemplary compositions and formulations are described above, including those produced in connection with methods of engineering the cells. Such compositions can be used in accord with the provided methods or uses, and/or with the provided articles of manufacture or compositions, such as in the prevention or treatment of diseases, conditions, and disorders, or in detection, diagnostic, and prognostic methods.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some aspects, the choice of carrier is determined in part by the particular cell or agent and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulation or composition may also contain more than one active ingredient useful for the particular indication, disease, or condition being prevented or treated with the cells or agents, where the respective activities do not adversely affect one another. Such active ingredients are suitably present in combination in amounts that are effective for the purpose intended. Thus, in some embodiments, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. In some embodiments, the agents or cells are administered in the form of a salt, e.g., a pharmaceutically acceptable salt. Suitable pharmaceutically acceptable acid addition salts include those derived from mineral acids, such as hydrochloric, hydrobromic, phosphoric, metaphosphoric, nitric, and sulphuric acids, and organic acids, such as tartaric, acetic, citric, malic, lactic, fumaric, benzoic, glycolic, gluconic, succinic, and arylsulphonic acids, for example, p-toluenesulphonic acid.

The pharmaceutical composition in some embodiments contains agents or cells in amounts effective to treat or prevent the disease or condition, such as a therapeutically effective or prophylactically effective amount. Therapeutic or prophylactic efficacy in some embodiments is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition.

The agents or cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some embodiments, they are administered by parenteral, intrapulmonary, and intranasal, and, if desired for local treatment, intralesional administration. Parenteral infusions include intramuscular, intravenous, intraarterial, intraperitoneal, or subcutaneous administration. In some embodiments, a given dose is administered by a single bolus administration of the cells or agent. In some embodiments, it is administered by multiple bolus administrations of the cells or agent, for example, over a period of no more than 3 days, or by continuous infusion administration of the cells or agent.

For the prevention or treatment of disease, the appropriate dosage may depend on the type of disease to be treated, the type of agent or agents, the type of cells or recombinant receptors, the severity and course of the disease, whether the agent or cells are administered for preventive or therapeutic purposes, previous therapy, the subject's clinical history and response to the agent or the cells, and the discretion of the attending physician. The compositions are in some embodiments suitably administered to the subject at one time or over a series of treatments.

The cells or agents may be administered using standard administration techniques, formulations, and/or devices. Provided are formulations and devices, such as syringes and vials, for storage and administration of the compositions. With respect to cells, administration can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell or an agent that treats or ameliorates symptoms of neurotoxicity), it will generally be formulated in a unit dosage injectable form (solution, suspension, emulsion).

Formulations include those for oral, intravenous, intraperitoneal, subcutaneous, pulmonary, transdermal, intramuscular, intranasal, buccal, sublingual, or suppository administration. In some embodiments, the agent or cell populations are administered parenterally. The term "parenteral," as used herein, includes intravenous, intramuscular, subcutaneous, rectal, vaginal, and intraperitoneal administration. In some embodiments, the agent or cell populations are administered to a subject using peripheral systemic delivery by intravenous, intraperitoneal, or subcutaneous injection.

Compositions in some embodiments are provided as sterile liquid preparations, e.g., isotonic aqueous solutions, suspensions, emulsions, dispersions, or viscous compositions, which may in some aspects be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the agent or cells in a solvent, such as in admixture with a suitable carrier, diluent, or excipient such as sterile water, physiological saline, glucose, dextrose, or the like.

The formulations to be used for in vivo administration are generally sterile. Sterility may be readily accomplished, e.g., by filtration through sterile filtration membranes.

In some embodiments, the dose of cells administered is in a cryopreserved composition. In some aspects, the composition is administered after thawing the cryopreserved composition. In some embodiments, the composition is administered within at or about 30 minutes, 45 minutes, 60 minutes, 90 minutes, 120 minutes, 150 minutes or 180 minutes after thawing. In some embodiments, the composition is administered within at or about 120 minutes after thawing.

In some embodiments, the dose of cells is administered with a syringe. In some embodiments, the syringe has a volume of at or about 0.5, 1, 2, 2.5, 3, 4, 5, 7.5, 10, 20 or 25 mL, or a range defined by any of the foregoing.

### V. COMBINATION THERAPY

In some embodiments of the methods, articles of manufacture, uses or compositions, the cell therapy, e.g. dose of T cells (e.g. CAR⁺ T cells) is administered to subjects in combination with an additional therapeutic agent or therapy, generally other than the cell therapy or another cell therapy, such as other than a CAR⁺ T cell therapy. In some embodiments, the cell therapy, e.g. dose of genetically engineered T cells, such as CAR⁺ T cells, in the provided methods or uses, and/or with the articles of manufacture or compositions, is administered as part of a combination treatment or combination therapy, such as simultaneously with, sequentially with or intermittently with, in any order, one or more additional therapeutic intervention. In some embodiments, the one or more additional therapeutic intervention includes any agent or treatment for treating or preventing the disease or condition, such as diffuse large B-cell lymphoma (DLBCL) or a subtype thereof or the B cell malignancy, e.g. NHL, and/or any agent or treatment to increase the efficacy, persistence, and/or activity of the engineered cell therapy.

In some embodiments, an additional therapeutic agent or therapy is administered to subjects who are or are likely to be or who are predicted to be poor responders and/or who do not, are likely not to and/or who are predicted not to respond or do not respond within a certain time and/or to a certain extent to treatment with the cell therapy, e.g. dose of T cells (e.g. CAR⁺ T cells). In some embodiments, the additional therapeutic agent is administered to subjects who do not or are not likely to or are not predicted to exhibit a complete response or overall response, such as within 1 month, within two months or within 3 months after initiation of administration of the cell therapy. In some embodiments, the additional therapeutic agent is administered to subjects who exhibit or are likely to exhibit or who are predicted to exhibit progressive disease (PD), such as within 1 month, two months or 3 months, following administration of the cell therapy. In some embodiments, a subject is likely or predicted not to exhibit a response or a certain response based on a plurality of similarly situated subjects so treated or previously treated with the cell therapy.

In some embodiments, it is observed that a subject that may or that is more likely to exhibit a poor response to cell therapy, e.g. dose of T cells (e.g. CAR⁺ T cells) includes a subject with NHL that is or has been identified to have stable or progressive disease (SD/PD) following treatment with a prior therapy, optionally a prior therapy with a chemotherapeutic agent, that is or has been identified with an Eastern Cooperative Oncology Group Performance Status (ECOG) status of 2, that is or has been identified as having a transformed follicular lymphoma (tFL), or that is or has been identified has having a DLBCL transformed from MZL and CLL. In some embodiments, the provided methods include selecting a subject that is or is likely to exhibit a poor response to a cell therapy when the cell therapy is administered alone, and administering the cell therapy in combination with an additional agent or therapy, such as any as described. In some embodiments, the a subject for treatment in the provided combination therapy methods is a subject that is selected as having a B cell malignancy, such as NHL, and that has stable or progressive disease (SD/PD) following treatment with a prior therapy, optionally a prior therapy with a chemotherapeutic agent, that has an Eastern Cooperative Oncology Group Performance Status (ECOG) status of 2, that has a transformed follicular lymphoma (tFL), or that has a DLBCL transformed from MZL and CLL. In some embodiments, the additional agent or therapy can be administered prior to, concomitantly with or at the same time and/or subsequently to initiation of administration of the cell therapy, e.g. dose of T cells (e.g. CAR⁺ T cells).

In certain embodiments, it is found that the pharmacokinetics (PK) of the cell therapy in the blood of subjects following administration of the cell therapy is similar or not substantially different between subjects that respond (e.g. exhibit a CR or OR) versus do not respond (e.g. exhibit PD) to the cell therapy. In some embodiments, such observations indicate that the cell therapy has or is expanding in the subject but may not exhibit optimal efficacy.

In some contexts, optimal efficacy of a cell therapy can depend on the ability of the administered cells to recognize and bind to a target, *e.g.,* target antigen, to traffic, localize to and successfully enter appropriate sites within the subject, tumors, and environments thereof. In some contexts, optimal efficacy can depend on the ability of the administered cells to become activated, expand, to exert various effector functions, including cytotoxic killing and secretion of various factors such as cytokines, to persist, including long-term, to differentiate, transition or engage in reprogramming into certain phenotypic states (such as long-lived memory, less-differentiated, and effector states), to avoid or reduce immunosuppressive conditions in the local microenvironment of a disease, to provide effective and robust recall responses following clearance and re-exposure to target ligand or antigen, and avoid or reduce exhaustion, anergy, peripheral tolerance, terminal differentiation, and/or differentiation into a suppressive state.

In some aspects, the efficacy of the immunotherapy, e.g., T cell therapy, may be limited by the immunosuppressive activity or factors present in the local microenvironment of the disease or disorder, e.g., the TME. In some aspects, the TME contains or produces factors or conditions that can suppress the activity, function, proliferation, survival and/or persistence of T cells administered for T cell therapy.

In some embodiments, administration of an additional agent or therapy, prior to, concomitantly with or at the same time and/or subsequently to initiation of administration of the cell therapy, e.g. dose of T cells (e.g. CAR⁺ T cells) can result in improved activity, efficacy and/or persistence of the cell therapy and/or improve responses of the treated subject. In some embodiments, the additional agent for combination treatment or combination therapy enhances, boosts and/or promotes the efficacy and/or safety of the therapeutic effect of the cell therapy, e.g. engineered T cell therapy, such as CAR⁺ T cells. In some embodiments, the additional agent enhances or improves the efficacy, survival or persistence of the administered cells, e.g., cells expressing the recombinant receptor, e.g. CAR.

In some embodiments, the additional agent of therapy is an antibody or a cytotoxic or therapeutic agent, e.g., a chemotherapeutic agent. In some embodiments, the one or more additional agents for treatment or therapy is an immunomodulatory agent, immune checkpoint inhibitor, adenosine pathway or adenosine receptor antagonist or agonist and kinase inhibitors. In some embodiments, the combination treatment or combination therapy includes an additional treatment, such as a surgical treatment, transplant, and/or radiation therapy.

In some embodiments, the additional agent is selected from among a protein phosphatase inhibitor, a kinase inhibitor, a cytokine, an immunomodulator, or an agent that decreases the level or activity of a regulatory T (Treg) cell. In some embodiments, the additional agent enhances safety, by virtue of reducing or ameliorating adverse effects of the administered cell therapy. In some embodiments, the additional agent can treat the same disease, condition or a comorbidity. In some embodiments, the additional agent can ameliorate, reduce or eliminate one or more toxicities, adverse effects or side effects that are associated with administration of the cells, e.g., CAR-expressing cells.

In some embodiments, the additional therapy, treatment or agent includes chemotherapy, radiation therapy, surgery, transplantation, adoptive cell therapy, antibodies, cytotoxic agents, chemotherapeutic agents, cytokines, growth inhibitory agents, anti-hormonal agents, kinase inhibitors, anti-angiogenic agents, cardioprotectants, immunostimulatory agents, immunosuppressive agents, immune checkpoint inhibitors, antibiotics, angiogenesis inhibitors, metabolic modulators or other therapeutic agents or any combination thereof. In some embodiments, the additional agent is a protein, a peptide, a nucleic acid, a small molecule agent, a cell, a toxin, a lipid, a carbohydrate or combinations thereof, or any other type of therapeutic agent, e.g. radiation. In some embodiments, the additional therapy, agent or treatment includes surgery, chemotherapy, radiation therapy, transplantation, administration of cells expressing a recombinant receptor, e.g., CAR, kinase inhibitor, immune checkpoint inhibitor, mTOR pathway inhibitor, immunosuppressive agents, immunomodulators, antibodies, immunoablative agents, antibodies and/or antigen binding fragments thereof, antibody conjugates, other antibody therapies, cytotoxins, steroids, cytokines, peptide vaccines, hormone therapy, antimetabolites, metabolic modulators, drugs that inhibit either the calcium dependent phosphatase calcineurin or the p70S6 kinase FK506) or inhibit the p70S6 kinase, alkylating agents, anthracyclines, vinca alkaloids, proteosome inhibitors, GITR agonists, protein tyrosine phosphatase inhibitors, protein kinase inhibitors, an oncolytic virus, and/or other types of immunotherapy. In some embodiments, the additional agent or treatment is bone marrow transplantation, T cell ablative therapy using chemotherapy agents such as, fludarabine, external-beam radiation therapy (XRT), cyclophosphamide, and/or antibody therapy.

In some embodiments, the additional agent is a kinase inhibitor, e.g., an inhibitor of Bruton's tyrosine kinase (Btk), e.g., ibrutinib. In some embodiments, the additional agent is an adenosine pathway or adenosine receptor antagonist or agonist. In some embodiments, the additional agent is an immunomodulator such as thalidomide or a thalidomide derivative (e.g., lenalidomide). In some embodiments, the additional therapy, agent or treatment is a cytotoxic or chemotherapy agent, a biologic therapy (e.g., antibody, e.g., monoclonal antibody, or cellular therapy), or an inhibitor (e.g., kinase inhibitor).

In some embodiments, the additional agent is a chemotherapeutic agent. Exemplary chemotherapeutic agents include an anthracycline (e.g., doxorubicin, such as liposomal doxorubicin); a vinca alkaloid (e.g., vinblastine, vincristine, vindesine, vinorelbine); an alkylating agent (e.g., cyclophosphamide, decarbazine, melphalan, ifosfamide, temozolomide); an immune cell antibody (e.g., alemtuzamab, gemtuzumab, rituximab, tositumomab); an antimetabolite (including, e.g., folic acid antagonists, pyrimidine analogs, purine analogs and adenosine deaminase inhibitors such as fludarabine); a TNFR glucocorticoid induced TNFR related protein (GITR) agonist; a proteasome inhibitor (e.g., aclacinomycin A, gliotoxin or bortezomib); an immunomodulatory such as thalidomide or a thalidomide derivative (e.g., lenalidomide).

In some embodiments, the additional agent is an immunomodulatory agent. In some embodiments, the combination therapy includes an immunomodulatory agent that can stimulate, amplify and/or otherwise enhance an anti-tumor immune response, e.g. anti-tumor immune response from the administered engineered cells, such as by inhibiting immunosuppressive signaling or enhancing immunostimulant signaling. In some embodiments, the immunomodulatory agent is a peptide, protein or is a small molecule. In some embodiments, the protein can be a fusion protein or a recombinant protein. In some embodiments, the immunomodulatory agent binds to an immunologic target, such as a cell surface receptor expressed on immune cells, such a T cells, B cells or antigen-presenting cells. For example, in some embodiments, the immunomodulatory agent is an antibody or antigen-binding antibody fragment, a fusion protein, a small molecule or a polypeptide. In some embodiments, the binding molecules, recombinant receptors, cells and/or compositions are administered in combination with an additional agent that is an antibody or an antigen-binding fragment thereof, such as a monoclonal antibody.

In some embodiments, the immunomodulatory agent blocks, inhibits or counteracts a component of the immune checkpoint pathway. The immune system has multiple inhibitory pathways that are involved in maintaining self-tolerance and for modulating immune responses. Tumors can use certain immune-checkpoint pathways as a major mechanism of immune resistance, particularly against T cells that are specific for tumor antigens (Pardoll (2012) Nature Reviews Cancer 12:252-264), e.g., engineered cells such as CAR-expressing cells. Because many such immune checkpoints are initiated by ligand-receptor interactions, they can be readily blocked by antibodies against the ligands and/or their receptors. In contrast to the majority of anti-cancer agents, checkpoint inhibitors do not necessarily target tumor cells directly, but rather target lymphocyte receptors or their ligands in order to enhance the endogenous antitumor activity of the immune system.

In some embodiments, the additional agent is an immunomodulatory agent that is an antagonist molecule or is an immune checkpoint inhibitor capable of inhibiting or blocking a function of a molecule, or signaling pathway, involving an immune checkpoint molecule. In some embodiments, the immune checkpoint molecule or pathway is PD-1, PD-L1, PD-L2, CTLA-4, LAG-3, TIM3, VISTA, adenosine 2A Receptor (A2AR), or adenosine or a pathway involving any of the foregoing. In certain embodiments, antagonistic molecules blocking an immune checkpoint pathway, such as small molecules, nucleic acid inhibitors (e.g., RNAi) or antibody molecules, are becoming promising avenues of immunotherapy for cancer and other diseases.

In some embodiments, the immune checkpoint inhibitor is a molecule that totally or partially reduces, inhibits, interferes with or modulate one or more checkpoint proteins. Checkpoint proteins regulate T-cell activation or function. These proteins are responsible for co-stimulatory or inhibitory interactions of T-cell responses. Immune checkpoint proteins regulate and maintain self-tolerance and the duration and amplitude of physiological immune responses.

Immune checkpoint inhibitors include any agent that blocks or inhibits in a statistically significant manner, the inhibitory pathways of the immune system. Such inhibitors may include small molecule inhibitors or may include antibodies, or antigen binding fragments thereof, that bind to and block or inhibit immune checkpoint receptors, ligands and/or receptor-ligand interaction. In some embodiments, modulation, enhancement and/or stimulation of particular receptors can overcome immune checkpoint pathway components. Illustrative immune checkpoint molecules that may be targeted for blocking, inhibition, modulation, enhancement and/or stimulation include, but are not limited to, PD-1 (CD279), PD-L1 (CD274, B7-H1), PDL2 (CD273, B7-DC), CTLA-4, LAG-3 (CD223), TIM-3, 4-1BB (CD137), 4-1BBL (CD137L), GITR (TNFRSF18, AITR), CD40, OX40 (CD134, TNFRSF4), CXCR2, tumor associated antigens (TAA), B7-H3, B7-H4, BTLA, HVEM, GAL9, B7H3, B7H4, VISTA, KIR, 2B4 (belongs to the CD2 family of molecules and is expressed on all NK, γδ, and memory CD8⁺ (αβ) T cells), CD160 (also referred to as BY55), CGEN-15049, CEACAM (e.g., CEACAM-1, CEACAM-3 and/or CEACAM-5), TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and a transforming growth factor receptor (TGFR; e.g., TGFR beta). Immune checkpoint inhibitors include antibodies, or antigen binding fragments thereof, or other binding proteins, that bind to and block or inhibit and/or enhance or stimulate the activity of one or more of any of the said molecules.

Exemplary immune checkpoint inhibitors include Tremelimumab (CTLA-4 blocking antibody, also known as ticilimumab, CP-675,206), anti-OX40, PD-L1 monoclonal antibody (Anti-B7-H1; MEDI4736), MK-3475 (PD-1 blocker), nivolumab (anti-PD-1 antibody), CT-011 (anti-PD-1 antibody), BY55 monoclonal antibody, AMP224 (anti-PD-L1 antibody), BMS-936559 (anti-PD-L1 antibody), MPLDL3280A (anti-PD-L1 antibody), MSB0010718C (anti-PD-L1 antibody) and ipilimumab (anti-CTLA-4 antibody, also known as Yervoy^{®}, MDX-010 and MDX-101). Exemplary of immunomodulatory antibodies include, but are not limited to, Daclizumab (Zenapax), Bevacizumab (Avastin ^{®}), Basiliximab, Ipilimumab, Nivolumab, pembrolizumab, MPDL3280A, Pidilizumab (CT-011), MK-3475, BMS-936559, MPDL3280A (Atezolizumab), tremelimumab, IMP321, BMS-986016, LAG525, urelumab, PF-05082566, TRX518, MK-4166, dacetuzumab (SGN-40), lucatumumab (HCD122), SEA-CD40, CP-870, CP-893, MEDI6469, MEDI6383, MOXR0916, AMP-224, MSB0010718C (Avelumab), MEDI4736, PDR001, rHIgM12B7, Ulocuplumab, BKT140, Varlilumab (CDX-1127), ARGX-110, MGA271, lirilumab (BMS-986015, IPH2101), IPH2201, ARGX-115, Emactuzumab, CC-90002 and MNRP1685A or an antibody-binding fragment thereof. Other exemplary immunomodulators include, e.g., afutuzumab (available from Roche^{®}); pegfilgrastim (Neulasta^{®}); lenalidomide (CC-5013, Revlimid^{®}); thalidomide (Thalomid^{®}), actimid (CC4047); and IRX-2 (mixture of human cytokines including interleukin 1, interleukin 2, and interferon.gamma., CAS 951209-71-5, available from IRX Therapeutics).

In some embodiments, the additional agent that is administered in accord with the provided methods, and/or with the provided articles of manufacture or compositions, is an agent that binds to and/or inhibits Programmed cell death 1 (PD-1). PD-1 is an immune checkpoint protein that is expressed in B cells, NK cells, and T cells (Shinohara et al., 1995, Genomics 23:704-6; Blank et al., 2007, Cancer Immunol Immunother 56:739-45; Finger et al., 1997, Gene 197:177-87; Pardoll (2012) Nature Reviews Cancer 12:252-264). The major role of PD-1 is to limit the activity of T cells in peripheral tissues during inflammation in response to infection, as well as to limit autoimmunity. PD-1 expression is induced in activated T cells and binding of PD-1 to one of its endogenous ligands acts to inhibit T-cell activation by inhibiting stimulatory kinases. PD-1 also acts to inhibit the TCR "stop signal". PD-1 is highly expressed on Treg cells and may increase their proliferation in the presence of ligand (Pardoll (2012) Nature Reviews Cancer 12:252-264). Anti-PD 1 antibodies have been used for treatment of melanoma, non-small-cell lung cancer, bladder cancer, prostate cancer, colorectal cancer, head and neck cancer, triple-negative breast cancer, leukemia, lymphoma and renal cell cancer (Topalian et al., 2012, N Engl J Med 366:2443-54; Lipson et al., 2013, Clin Cancer Res 19:462-8; Berger et al., 2008, Clin Cancer Res 14:3044-51; Gildener-Leapman et al., 2013, Oral Oncol 49:1089-96; Menzies & Long, 2013, Ther Adv Med Oncol 5:278-85). Exemplary anti-PD-1 antibodies include nivolumab (Opdivo by BMS), pembrolizumab (Keytruda by Merck), pidilizumab (CT-011 by Cure Tech), lambrolizumab (MK-3475 by Merck), and AMP-224 (Merck), nivolumab (also referred to as Opdivo, BMS-936558 or MDX1106; Bristol-Myers Squibb) is a fully human IgG4 monoclonal antibody which specifically blocks PD-1. Nivolumab (clone 5C4) and other human monoclonal antibodies that specifically bind to PD-1 are described in US 8,008,449 and WO2006/121168. Pidilizumab (CT-011; Cure Tech) is a humanized IgG1k monoclonal antibody that binds to PD-1. Pidilizumab and other humanized anti-PD-1 monoclonal antibodies are described in WO2009/101611. Pembrolizumab (formerly known as lambrolizumab, and also referred to as Keytruda, MK03475; Merck) is a humanized IgG4 monoclonal antibody that binds to PD-1. Pembrolizumab and other humanized anti-PD-1 antibodies are described in US 8,354,509 and WO2009/114335. Other anti-PD-1 antibodies include AMP 514 (Amplimmune), among others, e.g., anti-PD-1 antibodies described in US 8,609,089, US 2010028330, US 20120114649 and/or US 20150210769. AMP-224 (B7-DCIg; Amplimmune; e.g., described in WO2010/027827 and WO2011/066342), is a PD-L2 Fc fusion soluble receptor that blocks the interaction between PD-1 and B7-H1.

In some embodiments, the additional agent that is administered in accord with the provided methods, and/or with the provided articles of manufacture or compositions, is an agent that binds to or inhibits PD-L1 (also known as CD274 and B7-H1) and/or PD-L2 (also known as CD273 and B7-DC). PD-L1 and PD-L2 are ligands for PD-1, found on activated T cells, B cells, myeloid cells, macrophages, and some types of tumor cells. Anti-tumor therapies have focused on anti-PD-L1 antibodies. The complex of PD-1 and PD-L1 inhibits proliferation of CD8⁺ T cells and reduces the immune response (Topalian et al., 2012, N Engl J Med 366:2443-54; Brahmer et al., 2012, N Eng J Med 366:2455-65). Anti-PD-L1 antibodies have been used for treatment of non-small cell lung cancer, melanoma, colorectal cancer, renal-cell cancer, pancreatic cancer, gastric cancer, ovarian cancer, breast cancer, and hematologic malignancies (Brahmer et al., 2012, N Eng J Med 366:2455-65; Ott et al., 2013, Clin Cancer Res 19:5300-9; Radvanyi et al., 2013, Clin Cancer Res 19:5541; Menzies & Long, 2013, Ther Adv Med Oncol 5:278-85; Berger et al., 2008, Clin Cancer Res 14:13044-51). Exemplary anti-PD-L1 antibodies include MDX-1105 (Medarex), MEDI4736 (Medimmune) MPDL3280A (Genentech), BMS-935559 (Bristol-Myers Squibb) and MSB0010718C. MEDI4736 (Medimmune) is a human monoclonal antibody that binds to PD-L1, and inhibits interaction of the ligand with PD-1. MDPL3280A (Genentech/Roche) is a human Fc optimized IgG1 monoclonal antibody that binds to PD-L1. MDPL3280A and other human monoclonal antibodies to PD-L1 are described in U.S. Patent No. 7,943,743 and U.S Publication No. 20120039906. Other anti-PD-L1 binding agents include YW243.55.S70 (see WO2010/077634) and MDX-1105 (also referred to as BMS-936559, and, e.g., anti-PD-L1 binding agents described in WO2007/005874).

In some embodiments, the additional agent that is administered in accord with the provided methods, and/or with the provided articles of manufacture or compositions, is an agent that is an inhibitor of Cytotoxic T-lymphocyte-associated antigen (CTLA-4), also known as CD152, or binds to CTLA-4. CTLA-4 is a co-inhibitory molecule that functions to regulate T-cell activation. CTLA-4 is a member of the immunoglobulin superfamily that is expressed exclusively on T-cells. CTLA-4 acts to inhibit T-cell activation and is reported to inhibit helper T-cell activity and enhance regulatory T-cell immunosuppressive activity. Although the precise mechanism of action of CTLA-4 remains under investigation, it has been suggested that it inhibits T cell activation by outcompeting CD28 in binding to CD80 and CD86, as well as actively delivering inhibitor signals to the T cell (Pardoll (2012) Nature Reviews Cancer 12:252-264). Anti-CTLA-4 antibodies have been used in clinical trials for the treatment of melanoma, prostate cancer, small cell lung cancer, non-small cell lung cancer (Robert & Ghiringhelli, 2009, Oncologist 14:848-61; Ott et al., 2013, Clin Cancer Res 19:5300; Weber, 2007, Oncologist 12:864-72; Wada et al., 2013, J Transl Med 11:89). A significant feature of anti-CTLA-4 is the kinetics of anti-tumor effect, with a lag period of up to 6 months after initial treatment required for physiologic response. In some cases, tumors may actually increase in size after treatment initiation, before a reduction is seen (Pardoll (2012) Nature Reviews Cancer 12:252-264). Exemplary anti-CTLA-4 antibodies include ipilimumab (Bristol-Myers Squibb) and tremelimumab (Pfizer). Ipilimumab has recently received FDA approval for treatment of metastatic melanoma (Wada et al., 2013, J Transl Med 11:89).

In some embodiments, the additional agent that is administered in accord with the provided methods, and/or with the provided articles of manufacture or compositions, is an agent that bind to and/or inhibits Lymphocyte activation gene-3 (LAG-3), also known as CD223. LAG-3 is another immune checkpoint protein. LAG-3 has been associated with the inhibition of lymphocyte activity and in some cases the induction of lymphocyte anergy. LAG-3 is expressed on various cells in the immune system including B cells, NK cells, and dendritic cells. LAG-3 is a natural ligand for the MHC class II receptor, which is substantially expressed on melanoma-infiltrating T cells including those endowed with potent immune-suppressive activity. Exemplary anti-LAG-3 antibodies include BMS-986016 (Bristol-Myers Squib), which is a monoclonal antibody that targets LAG-3. IMP701 (Immutep) is an antagonist LAG-3 antibody and IMP731 (Immutep and GlaxoSmithKline) is a depleting LAG-3 antibody. Other LAG-3 inhibitors include IMP321 (Immutep), which is a recombinant fusion protein of a soluble portion of LAG-3 and Ig that binds to MHC class II molecules and activates antigen presenting cells (APC). Other antibodies are described, e.g., in WO2010/019570 and US 2015/0259420.

In some embodiments, the additional agent that is administered in accord with the provided methods, and/or with the provided articles of manufacture or compositions, is an agent that bins to and/or inhibits T-cell immunoglobulin domain and mucin domain-3 (TIM-3). TIM-3 was initially identified on activated Th1 cells, has been shown to be a negative regulator of the immune response. Blockade of TIM-3 promotes T-cell mediated anti-tumor immunity and has anti-tumor activity in a range of mouse tumor models. Combinations of TIM-3 blockade with other immunotherapeutic agents such as TSR-042, anti-CD137 antibodies and others, can be additive or synergistic in increasing anti-tumor effects. TIM-3 expression has been associated with a number of different tumor types including melanoma, NSCLC and renal cancer, and additionally, expression of intratumoral TIM-3 has been shown to correlate with poor prognosis across a range of tumor types including NSCLC, cervical, and gastric cancers. Blockade of TIM-3 is also of interest in promoting increased immunity to a number of chronic viral diseases. TIM-3 has also been shown to interact with a number of ligands including galectin-9, phosphatidylserine and HMGB1, although which of these, if any, are relevant in regulation of anti-tumor responses is not clear at present. In some embodiments, antibodies, antibody fragments, small molecules, or peptide inhibitors that target TIM-3 can bind to the IgV domain of TIM-3 to inhibit interaction with its ligands. Exemplary antibodies and peptides that inhibit TIM-3 are described in US 2015/0218274, WO2013/006490 and US 2010/0247521. Other anti-TIM-3 antibodies include humanized versions of RMT3-23 (Ngiow et al., 2011, Cancer Res, 71:3540-3551), and clone 8B.2C12 (Monney et al., 2002, Nature, 415:536-541). Bi-specific antibodies that inhibit TIM-3 and PD-1 are described in US 2013/0156774.

In some embodiments, the additional agent that is administered in accord with the provided methods, and/or with the provided articles of manufacture or compositions, is an agent that is a CEACAM inhibitor (e.g., CEACAM-1, CEACAM-3, and/or CEACAM-5 inhibitor). In some embodiments, the inhibitor of CEACAM is an anti-CEACAM antibody molecule. Exemplary anti-CEACAM-1 antibodies are described in WO 2010/125571, WO 2013/082366 WO 2014/059251 and WO 2014/022332, e.g., a monoclonal antibody 34B1, 26H7, and 5F4; or a recombinant form thereof, as described in, e.g., US 2004/0047858, US 7,132,255 and WO 99/052552. In some embodiments, the anti-CEACAM antibody binds to CEACAM-5 as described in, e.g., Zheng et al. PLoS One. (2011) 6(6): e21146), or crossreacts with CEACAM-1 and CEACAM-5 as described in, e.g., WO 2013/054331 and US 2014/0271618.

In some embodiments, the additional agent that is administered in accord with the provided methods, and/or with the provided articles of manufacture or compositions, is an agent that binds to and/or inhibits 4-1BB, also known as CD137. 4-1BB is a transmembrane glycoprotein belonging to the TNFR superfamily. 4-1BB receptors are present on activated T cells and B cells and monocytes. An exemplary anti-4-1BB antibody is urelumab (BMS-663513), which has potential immunostimulatory and antineoplastic activities.

In some embodiments, the additional agent that is administered in accord with the provided methods, and/or with the provided articles of manufacture or compositions, is an agent that binds to and/or inhibits Tumor necrosis factor receptor superfamily, member 4 (TNFRSF4), also known as OX40 and CD134. TNFRSF4 is another member of the TNFR superfamily. OX40 is not constitutively expressed on resting naive T cells and acts as a secondary co-stimulatory immune checkpoint molecule. Exemplary anti-OX40 antibodies are MEDI6469 and MOXR0916 (RG7888, Genentech).

In some embodiments, the additional agent that is administered in accord with the provided methods, and/or with the provided articles of manufacture or compositions, is an agent or a molecule that decreases the regulatory T cell (Treg) population. Methods that decrease the number of (e.g., deplete) Treg cells are known and include, e.g., CD25 depletion, cyclophosphamide administration, and modulating Glucocorticoid-induced TNFR family related gene (GITR) function. GITR is a member of the TNFR superfamily that is upregulated on activated T cells, which enhances the immune system. Reducing the number of Treg cells in a subject prior to apheresis or prior to administration of engineered cells, e.g., CAR-expressing cells, can reduce the number of unwanted immune cells (e.g., Tregs) in the tumor microenvironment and reduces the subject's risk of relapse. In some embodiments, the additional agent includes a molecule targeting GITR and/or modulating GITR functions, such as a GITR agonist and/or a GITR antibody that depletes regulatory T cells (Tregs). In some embodiments, the additional agent includes cyclophosphamide. In some embodiments, the GITR binding molecule and/or molecule modulating GITR function (e.g., GITR agonist and/or Treg depleting GITR antibodies) is administered prior to the engineered cells, e.g., CAR-expressing cells. For example, in some embodiments, the GITR agonist can be administered prior to apheresis of the cells. In some embodiments, cyclophosphamide is administered to the subject prior to administration (e.g., infusion or re-infusion) of the engineered cells, e.g., CAR-expressing cells or prior to apheresis of the cells. In some embodiments, cyclophosphamide and an anti-GITR antibody are administered to the subject prior to administration (e.g., infusion or re-infusion) of the engineered cells, e.g., CAR-expressing cells or prior to apheresis of the cells.

In some embodiments, the additional agent that is administered in accord with the provided methods, and/or with the provided articles of manufacture or compositions, is an agent that is a GITR agonist. Exemplary GITR agonists include, e.g., GITR fusion proteins and anti-GITR antibodies (e.g., bivalent anti-GITR antibodies) such as, e.g., a GITR fusion protein described in U.S. Patent No. 6,111,090, European Patent No. 090505B1, U.S Patent No. 8,586,023, PCT Publication Nos.: WO 2010/003118 and 2011/090754, or an anti-GITR antibody described, e.g., in U.S. Patent No. 7,025,962, European Patent No. 1947183B1, U.S. Patent No. 7,812,135, U.S. Patent No. 8,388,967, U.S. Patent No. 8,591,886, European Patent No. EP 1866339, PCT Publication No. WO 2011/028683, PCT Publication No. WO 2013/039954, PCT Publication No. WO2005/007190, PCT Publication No. WO 2007/133822, PCT Publication No. WO2005/055808, PCT Publication No. WO 99/40196, PCT Publication No. WO 2001/03720, PCT Publication No. WO99/20758, PCT Publication No. WO2006/083289, PCT Publication No. WO 2005/115451, U.S. Patent No. 7,618,632, and PCT Publication No. WO 2011/051726. An exemplary anti-GITR antibody is TRX518.

In some embodiments, the additional agent that is administered in accord with the provided methods, and/or with the provided articles of manufacture or compositions, enhances tumor infiltration or transmigration of the administered cells, e.g., CAR-expressing cells. For example, in some embodiments, the additional agent stimulates CD40, such as CD40L, e.g., recombinant human CD40L. Cluster of differentiation 40 (CD40) is also a member of the TNFR superfamily. CD40 is a costimulatory protein found on antigen-presenting cells and mediates a broad variety of immune and inflammatory responses. CD40 is also expressed on some malignancies, where it promotes proliferation. Exemplary anti-CD40 antibodies are dacetuzumab (SGN-40), lucatumumab (Novartis, antagonist), SEA-CD40 (Seattle Genetics), and CP-870,893. In some embodiments, the additional agent that enhances tumor infiltration includes tyrosine kinase inhibitor sunitnib, heparanase, and/or chemokine receptors such as CCR2, CCR4, and CCR7.

In some embodiments, the additional agent that is administered in accord with the provided methods, and/or with the provided articles of manufacture or compositions, is an immunomodulatory agent that is a structural or functional analog or derivative of thalidomide and/or an inhibitor of E3 ubiquitin ligase. In some embodiments, the immunomodulatory agent binds to cereblon (CRBN). In some embodiments, the immunomodulatory agent binds to the CRBN E3 ubiquitin-ligase complex. In some embodiments, the immunomodulatory agent binds to CRBN and the CRBN E3 ubiquitin-ligase complex. In some embodiments, the immunomodulatory agent up-regulates the protein or gene expression of CRBN. In some aspects, CRBN is the substrate adaptor for the CRL4^{CRBN} E3 ubiquitin ligase, and modulates the specificity of the enzyme. In some embodiments, binding to CRB or the CRBN E3 ubiquitin ligase complex inhibits E3 ubiquitin ligase activity. In some embodiments, the immunomodulatory agent induces the ubiqutination of KZF1 (Ikaros) and IKZF3 (Aiolos) and/or induces degradation of IKZF1 (Ikaros) and IKZF3 (Aiolos). In some embodiments, the immunomodulatory agent induces the ubiquitination of casein kinase 1A1 (CK1α) by the CRL4^{CRBN} E3 ubiquitin ligase. In some embodiments, the ubiquitination of CK1α results in CK1α degradation.

In some embodiments, the immunomodulatory agent is an inhibitor of the Ikaros (IKZF1) transcription factor. In some embodiments, the immunomodulatory agent enhances ubiquitination of Ikaros. In some embodiments, the immunomodulatory agent enhances the degradation of Ikaros. In some embodiments, the immunomodulatory agent down-regulates the protein or gene expression of Ikaros. In some embodiments, administration of the immunomodulatory agent causes a decrease in Ikaros protein levels.

In some embodiments, the immunomodulatory agent is an inhibitor of the Aiolos (IKZF3) transcription factor. In some embodiments, the immunomodulatory agent enhances ubiquitination of Aiolos. In some embodiments, the immunomodulatory agent enhances the degradation of Aiolos. In some embodiments, the immunomodulatory agent down-regulates the protein or gene expression of Aiolos. In some embodiments, administration of the immunomodulatory agent causes a decrease in Aiolos protein levels.

In some embodiments, the immunomodulatory agent is an inhibitor of both the Ikaros (IKZF1) and Aiolos (IKZF3) transcription factors. In some embodiments, the immunomodulatory agent enhances ubiquitination of both Ikaros and Aiolos. In some embodiments, the immunomodulatory agent enhances the degradation of both Ikaros and Aiolos. In some embodiments, the immunomodulatory agent enhances ubiquitination and degradation of both Ikaros and Aiolos. In some embodiments, administration of the immunomodulatory agent causes both Aiolos protein levels and Ikaros protein levels to decrease.

In some embodiments, the immunomodulatory agent is a selective cytokine inhibitory drug (SelCID). In some embodiments, the immunomodulatory agent inhibits the activity of phosphodiesterase-4 (PDE4). In some embodiments, the immunomodulatory agent suppresses the enzymatic activity of the CDC25 phosphatases. In some embodiments, the immunomodulatory agent alters the intracellular trafficking of CDC25 phosphatases.

In some embodiments, the immunomodulatory agent is thalidomide (2-(2,6-dioxopiperidin-3-yl)-lH-isoindole- 1,3(2H)-dione) or an analog or derivative of thalidomide. In certain embodiments, a thalidomide derivative includes structural variants of thalidomide that have a similar biological activity. Exemplary thalidomide derivatives include, but are not limited to lenalidomide (REVLIMMUNOMODULATORY COMPOUND^{™}; Celgene Corporation), pomalidomide (also known as ACTIMMUNOMODULATORY COMPOUND^{™} or POMALYST^{™} (Celgene Corporation)), CC-1088, CDC-501, and CDC- 801, and the compounds disclosed in U.S. Pat. Nos. 5,712,291; 7,320,991; and 8,716,315; U.S. Appl. No. 2016/0313300; and PCT Pub. Nos. WO 2002/068414 and WO 2008/154252.

In some embodiments, the immunomodulatory agent is 1-oxo- and 1,3 dioxo-2-(2,6-dioxopiperldin-3-yl) isoindolines substituted with amino in the benzo ring as described in U.S. Pat. No. 5,635,517 which is incorporated herein by reference.

In some embodiments, the immunomodulatory agent is a compound of the following formula: 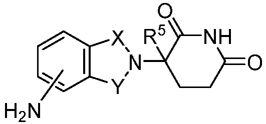 wherein one of X and Y is -C(O)- and the other of X and Y is -C(O)- or -CH₂-, and R⁵ is hydrogen or lower alkyl, or a pharmaceutically acceptable salt thereof. In some embodiments, X is -C(O)- and Y is - CH₂-. In some embodiments, both X and Y are -C(O)-. In some embodiments, R⁵ is hydrogen. In other embodiments, R⁵ is methyl.

In some embodiments, the immunomodulatory compound is a compound that belongs to a class of substituted 2-(2, 6-dioxopiperidin-3-yl)phthalimmunomodulatory compounds and substituted 2-(2,6-dioxopiperldin-3-yl)-1-oxoisoindoles, such as those described in U.S. Pat. Nos. 6,281,230; 6,316,471; 6,335,349; and 6,476,052, and International Patent Application No. PCT/US97/13375 (International Publication No. WO 98/03502), each of which is incorporated herein by reference.

In some embodiments, the immunomodulatory agent is a compound of the following formula: 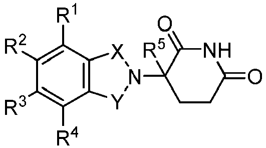 wherein one of X and Y is -C(O)- and the other of X and Y is -C(O)- or -CH₂-;
(1) each of R¹, R², R³, and R⁴ are independently halo, alkyl of 1 to 4 carbon atoms, or alkoxy or 1 to 4 carbon atoms, or
(2) one of R¹, R³, R⁴, and R⁵ is -NHR^{a} and the remaining of R¹, R², R³, and R⁴ is are hydrogen, wherein R^{a} is hydrogen or alkyl of 1 to 8 carbon atoms;
   R⁵ is hydrogen or alkyl of 1 to 8 carbon atoms, benzyl, or halo;
   provided that R⁵ is other than hydrogen if X and Y are -C(O)- and (i) each of R¹, R², R³, and R⁴ is fluoro; or (ii) one of R¹, R², R³, and R⁴ is amino;
   or a pharmaceutically acceptable salt thereof.

In some embodiments, the immunomodulatory agent is a compound that belongs to a class of isoindole-immunomodulatory compounds disclosed in U.S. Pat. No. 7,091,353, U.S. Patent Publication No. 2003/0045552, and International Application No. PCT/USOI/50401 (International Publication No. WO02/059106), each of which are incorporated herein by reference. For example, in some embodiments, the immunomodulatory agent is [2-(2,6-dioxo-piperidin-3-yl)-1,3-dioxo-2,3-dihydro-1H-isoindol-4-ylmethyl]-amide; (2-(2,6-dioxo-piperidin-3-yl)-1,3-dioxo-2,3-dihydro-1H-isoindol-4-ylmethyl)-carbamic acid tert-butyl ester; 4-(aminomethyl)-2-(2,6-dioxo(3-piperidyl))-isoindoline-1,3-dione; N-(2-(2,6-dioxo-piperidin-3-yl)-1,3-dioxo-2,3-dihydro-1H-isoindol-4-ylmethyl)-acetamide; N-{(2-(2,6-dioxo(3-piperidyl)-1,3-dioxoisoindolin-4-yl)methyl}cyclopropyl-carboxamide; 2-chloro-N-{(2-(2,6-dioxo(3-piperidyl))-1,3-dioxoisoindolin-4-yl)methyl}acetamide; N-(2-(2,6-dioxo(3-piperidyl))-1,3-dioxoisoindolin-4-yl)-3-pyridylcarboxamide; 3-{1-oxo-4-(benzylamino)isoindolin-2-yl}piperidine-2,6-dione; 2-(2,6-dioxo(3-piperidyl))-4-(benzylamino)isoindoline-1,3-dione; N-{(2-(2,6-dioxo(3-piperidyl))-1,3-dioxoisoindolin-4-yl)methyl}propanamide; N-{(2-(2,6-dioxo(3-piperidyl))-1,3-dioxoisoindolin-4-yl)methyl}-3-pyridylcarboxamide; N-{(2-(2,6-dioxo(3-piperidyl))-1,3-dioxoisoindolin-4-yl)methyl}heptanamide; N-{(2-(2,6-dioxo(3-piperidyl))-1,3-dioxoisoindolin-4-yl)methyl}-2-furylcarboxamide; {N-(2-(2,6-dioxo(3-piperidyl))-1,3-dioxoisoindolin-4-yl)carbamoyl}methyl acetate; N-(2-(2,6-dioxo(3-piperidyl))-1,3-dioxoisoindolin-4-yl)pentanamide; N-(2-(2,6-dioxo(3-piperidyl))-1,3-dioxoisoindolin-4-yl)-2-thienylcarboxamide; N-{[2-(2,6-dioxo(3-piperidyl))-1,3-dioxoisoindolin-4-yl]methyl)(butylamino)carboxamide; N-{[2-(2,6-dioxo(3-piperidyl))-1,3-dioxoisoindolin-4-yl]methyl}(octylamino)carboxamide; or N-{[2-(2,6-dioxo(3-piperidyl))-1,3-dioxoisoindolin-4-yl]methyl}(benzylamino)carboxamide.

In some embodiments, the immunomodulatory agent is a compound that belongs to a class of isoindole-immunomodulatory compounds disclosed in U.S. Patent Application Publication Nos. 2002/0045643, International Publication No. WO 98/54170, and U.S. Pat. No. 6,395,754, each of which is incorporated herein by reference. In some embodiments, the immunomodulatory agent is a tetra substituted 2-(2,6-dioxopiperdin-3-yl)-1-oxoisoindolines described in U.S. Pat. No. 5,798,368, which is incorporated herein by reference. In some embodiments, the immunomodulatory agent is 1-oxo and 1,3-dioxo-2-(2,6-dioxopiperidin-3-yl) isoindolines disclosed in U.S. Pat. No. 6,403,613, which is incorporated herein by reference. In some embodiments the immunomodulatory agent is a 1-oxo or 1,3-dioxoisoindoline substituted in the 4- or 5-position of the indoline ring as described in U.S. Pat. No. 6,380,239 and U.S. Pat. No. 7,244,759, both of which are incorporated herein by reference.

In some embodiments, the immunomodulatory agent is 2-(4-amino-1-oxo-1,3-dihydro-isoindol-2-yl)-4-carbamoyl-butyric acid or 4-(4-amino-1-oxo-1,3-dihydro-isoindol-2-yl)-4-carbamoyl-butyric acid. In some embodiments, the immunomodulatory compound is 4-carbamoyl-4-{4-[(furan-2-yl-methyl)-amino]-1,3-dioxo-1,3-dihydro-isoindol-2-yl}-butyric acid, 4-carbamoyl-2-{4-[(furan-2-yl-methyl)-amino]-1,3-dioxo-1,3-dihydro-isoindol-2-yl}-butyric acid, 2-{4-[(furan-2-yl-methyl)-amino]-1,3-dioxo-1,3-dihydro-isoindol-2-yl}-4-phenylcarbamoyl-butyric acid, or 2-{4-[(furan-2-yl-methyl)-amino]-1,3-dioxo-1,3-dihydro-isoindol-2-yl}-pentanedioic acid.

In some embodiments, the immunomodulatory agent is a isoindoline-1-one or isoindoline-1,3-dione substituted in the 2-position with 2,6-dioxo-3-hydroxypiperidin-5-yl as described in U.S. Pat. No. 6,458,810, which is incorporated herein by reference. In some embodiments, the immunomodulatory compound is 3-(5-amino-2-methyl-4-oxo-4H-quinazolin-3-yl)-piperidine-2,6-dione, or an enantiomer or a mixture of enantiomers thereof; or a pharmaceutically acceptable salt, solvate, hydrate, co-crystal, clathrate, or polymorph thereof. In some embodiments, the immunomodulatory compound is 3-[4-(4-morpholin-4-ylmethyl-benzyloxy)-1-oxo-1,3-dihydro-isoindol-2-yl]-piperidine-2,6-dione.

In some embodiments, the immunomodulatory agent is as described in Oshima, K. et al., Nihon Rinsho., 72(6):1130-5 (2014); Millrine, D. et al., Trends Mol Med., 23(4):348-364 (2017); and Collins, et al., Biochem J., 474(7):1127-1147 (2017).

In some embodiments, the immunomodulatory agent is lenalidomide, pomalidomide, avadomide, a stereoisomer of lenalidomide, pomalidomide, avadomide or a pharmaceutically acceptable salt, solvate, hydrate, co-crystal, clathrate, or polymorph thereof. In some embodiments, the immunomodulatory compound is lenalidomide, a stereoisomer of lenalidomide or a pharmaceutically acceptable salt, solvate, hydrate, co-crystal, clathrate, or polymorph thereof. In some embodiments, the immunomodulatory compound is lenalidomide, or ((RS)-3-(4-Amino-1-oxo-1,3-dihydro-2H-isoindol-2-yl)piperidine-2,6-dione).

In some embodiments, the additional agent includes thalidomide drugs or analogs thereof and/or derivatives thereof, such as lenalidomide, pomalidomide or apremilast. See, e.g., Bertilaccio et al., Blood (2013) 122:4171, Otahal et al., Oncoimmunology (2016) 5(4):e1115940; Fecteau et al., Blood (2014) 124(10):1637-1644 and Kuramitsu et al., Cancer Gene Therapy (2015) 22:487-495). Lenalidomide ((RS)-3-(4-Amino-1-oxo-1,3-dihydro-2H-isoindol-2-yl)piperidine-2,6-dione; also known as Revlimid) is a synthetic derivative of thalidomide, and has multiple immunomodulatory effects, including enforcement of immune synapse formation between T cell and antigen presenting cells (APCs). For example, in some cases, lenalidomide modulates T cell responses and results in increased interleukin (IL)-2 production in CD4⁺ and CD8⁺ T cells, induces the shift of T helper (Th) responses from Th2 to Th1, inhibits expansion of regulatory subset of T cells (Tregs), and improves functioning of immunological synapses in follicular lymphoma and chronic lymphocytic leukemia (CLL) (Otahal et al., Oncoimmunology (2016) 5(4):e1115940). Lenalidomide also has direct tumoricidal activity in patients with multiple myeloma (MM) and directly and indirectly modulates survival of CLL tumor cells by affecting supportive cells, such as nurse-like cells found in the microenvironment of lymphoid tissues. Lenalidomide also can enhance T-cell proliferation and interferon-γ production in response to activation of T cells via CD3 ligation or dendritic cell-mediated activation. Lenalidomide can also induce malignant B cells to express higher levels of immunostimulatory molecules such as CD80, CD86, HLA-DR, CD95, and CD40 (Fecteau et al., Blood (2014) 124(10):1637-1644). In some embodiments, lenalidomide is administered at a dosage of from about 1 mg to about 20 mg daily, e.g., from about 1 mg to about 10 mg, from about 2.5 mg to about 7.5 mg, from about 5 mg to about 15 mg, such as about 5 mg, 10 mg, 15 mg or 20 mg daily. In some embodiments, lenalidomide is administered at a dose of from about 10 µg/kg to 5 mg/kg, e.g., about 100 µg/kg to about 2 mg/kg, about 200 µg/kg to about 1 mg/kg, about 400 µg/kg to about 600 µg/kg, such as about 500 µg/kg.

In some embodiments, the additional agent that is administered in accord with the provided methods, and/or with the provided articles of manufacture or compositions, is a B-cell inhibitor. In some embodiments, the additional agent is one or more B-cell inhibitors selected from among inhibitors of CD10, CD19, CD20, CD22, CD34, CD123, CD79a, CD79b, CD179b, FLT-3, or ROR1, or a combination thereof. In some embodiments, the B-cell inhibitor is an antibody (e.g., a mono- or bispecific antibody) or an antigen binding fragment thereof. In some embodiments, the additional agent is an engineered cell expressing recombinant receptors that target B-cell targets, e.g., CD10, CD19, CD20, CD22, CD34, CD123, CD79a, CD79b, CD179b, FLT-3, or ROR1.

In some embodiments, the additional agent that is administered in accord with the provided methods, and/or with the provided articles of manufacture or compositions, is a CD20 inhibitor, e.g., an anti-CD20 antibody (e.g., an anti-CD20 mono- or bi-specific antibody) or a fragment thereof. Exemplary anti-CD20 antibodies include but are not limited to rituximab, ofatumumab, ocrelizumab (also known as GA101 or RO5072759), veltuzumab, obinutuzumab, TRU-015 (Trubion Pharmaceuticals), ocaratuzumab (also known as AME-133v or ocaratuzumab), and Pro131921 (Genentech). See, e.g., Lim et al. Haematologica. (2010) 95(1):135-43. In some embodiments, the anti-CD20 antibody comprises rituximab. Rituximab is a chimeric mouse/human monoclonal antibody IgG1 kappa that binds to CD20 and causes cytolysis of a CD20 expressing cell. In some embodiments, the additional agent includes rituximab. In some embodiments, the CD20 inhibitor is a small molecule.

In some embodiments, the additional agent that is administered in accord with the provided methods, and/or with the provided articles of manufacture or compositions, is a CD22 inhibitor, e.g., an anti-CD22 antibody (e.g., an anti-CD22 mono- or bi-specific antibody) or a fragment thereof. Exemplary anti-CD22 antibodies include epratuzumab and RFB4. In some embodiments, the CD22 inhibitor is a small molecule. In some embodiments, the antibody is a monospecific antibody, optionally conjugated to a second agent such as a chemotherapeutic agent. For instance, in some embodiments, the antibody is an anti-CD22 monoclonal antibody-MMAE conjugate (e.g., DCDT2980S). In some embodiments, the antibody is an scFv of an anti-CD22 antibody, e.g., an scFv of antibody RFB4. In some embodiments, the scFv is fused to all of or a fragment of Pseudomonas exotoxin-A (e.g., BL22). In some embodiments, the scFv is fused to all of or a fragment of (e.g., a 38 kDa fragment of) Pseudomonas exotoxin-A (e.g., moxetumomab pasudotox). In some embodiments, the anti-CD22 antibody is an anti-CD19/CD22 bispecific antibody, optionally conjugated to a toxin. For instance, in some embodiments, the anti-CD22 antibody comprises an anti-CD19/CD22 bispecific portion, (e.g., two scFv ligands, recognizing human CD19 and CD22) optionally linked to all of or a portion of diphtheria toxin (DT), e.g., first 389 amino acids of diphtheria toxin (DT), DT 390, e.g., a ligand-directed toxin such as DT2219ARL). In some embodiments, the bispecific portion (e.g., anti-CD 19/anti-CD22) is linked to a toxin such as deglycosylated ricin A chain (e.g., Combotox).

In some embodiments, the additional agent that is administered in accord with the provided methods, and/or with the provided articles of manufacture or compositions, is a cytokine or is an agent that induces increased expression of a cytokine in the tumor microenvironment. Cytokines have important functions related to T cell expansion, differentiation, survival, and homeostasis. Cytokines that can be administered to the subject receiving the combination therapy in the provided methods or uses, recombinant receptors, cells and/or compositions provided herein include one or more of IL-2, IL-4, IL-7, IL-9, IL-15, IL-18, and IL-21. In some embodiments, the cytokine administered is IL-7, IL-15, or IL-21, or a combination thereof. In some embodiments, administration of the cytokine to the subject that has sub-optimal response to the administration of the engineered cells, e.g., CAR-expressing cells improves efficacy and/or anti-tumor activity of the administered cells, e.g., CAR-expressing cells.

In some embodiments, the additional agent that is administered in accord with the provided methods, and/or with the provided articles of manufacture or compositions, is a cytokine, such as a protein that act on another cell as intercellular mediators. Examples of such cytokines are lymphokines, monokines, and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha and -beta; mullerian-inhibiting substance; mouse gonadotropin-associated peptide; inhibin; activin; vascular endothelial growth factor; integrin; thrombopoietin (TPO); nerve growth factors such as NGF-beta; platelet-growth factor; transforming growth factors (TGFs) such as TGF-alpha and TGF-beta; insulin-like growth factor-I and -II; erythropoietin (EPO); osteoinductive factors; interferons such as interferon-alpha, beta, and -gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; IL-15, a tumor necrosis factor such as TNF-alpha or TNF-beta; and other polypeptide factors including LIF and kit ligand (KL). As used herein, the term cytokine includes proteins from natural sources or from recombinant cell culture, and biologically active equivalents of the native sequence cytokines. For example, the immunomodulatory agent is a cytokine and the cytokine is IL-4, TNF-α, GM-CSF or IL-2.

In some embodiments, the additional agent that is administered in accord with the provided methods, and/or with the provided articles of manufacture or compositions, includes an interleukin-15 (IL-15) polypeptide, an interleukin -15 receptor alpha (IL-15Rα) polypeptide, or combination thereof, e.g., hetIL-15 (Admune Therapeutics, LLC). hetIL-15 is a heterodimeric non-covalent complex of IL-15 and IL-15Rα. hetIL-15 is described in, e.g., U.S. 8,124,084, U.S. 2012/0177598, U.S. 2009/0082299, U.S. 2012/0141413, and U.S. 2011/0081311. In some embodiments, the immunomodulatory agent can contain one or more cytokines. For example, the interleukin can include leukocyte interleukin injection (Multikine), which is a combination of natural cytokines.

In some embodiments, the additional agent that is administered in accord with the provided methods, and/or with the provided articles of manufacture or compositions, is a modulator of adenosine levels and/or an adenosine pathway component. Adenosine can function as an immunomodulatory agent in the body. For example, adenosine and some adenosine analogs that non-selectively activate adenosine receptor subtypes decrease neutrophil production of inflammatory oxidative products (Cronstein et al., Ann. N.Y. Acad. Sci. 451:291, 1985; Roberts et al., Biochem. J., 227:669, 1985; Schrier et al., J. Immunol. 137:3284, 1986; Cronstein et al., Clinical Immunol. Immunopath. 42:76, 1987). In some cases, concentration of extracellular adenosine or adenosine analogs can increase in specific environments, e.g., tumor microenvironment (TME). In some cases, adenosine or adenosine analog signaling depends on hypoxia or factors involved in hypoxia or its regulation, e.g., hypoxia inducible factor (HIF). In some embodiments, increase in adenosine signaling can increase in intracellular cAMP and cAMP-dependent protein kinase that results in inhibition of proinflammatory cytokine production, and can lead to the synthesis of immunosuppressive molecules and development of Tregs (Sitkovsky et al., Cancer Immunol Res (2014) 2(7):598-605). In some embodiments, the additional agent can reduce or reverse immunosuppressive effects of adenosine, adenosine analogs and/or adenosine signaling. In some embodiments, the additional agent can reduce or reverse hypoxia-driven A2-adenosinergic T cell immunosuppression. In some embodiments, the additional agent is selected from among antagonists of adenosine receptors, extracellular adenosine-degrading agents, inhibitors of adenosine generation by CD39/CD73 ectoenzymes, and inhibitors of hypoxia-HIF-1α signaling. In some embodiments, the additional agent is an adenosine receptor antagonist or agonist.

In some embodiments, the additional agent that is administered in accord with the provided methods, and/or with the provided articles of manufacture or compositions, is an agent that inhibits the activity and/or an amount of an adenosine receptor. Particular embodiments contemplate that inhibition or reduction of extracellular adenosine or the adenosine receptor by virtue of an inhibitor of extracellular adenosine (such as an agent that prevents the formation of, degrades, renders inactive, and/or decreases extracellular adenosine), and/or an adenosine receptor inhibitor (such as an adenosine receptor antagonist) can enhance immune response, such as a macrophage, neutrophil, granulocyte, dendritic cell, T- and/or B cell-mediated response. In addition, inhibitors of the Gs protein mediated cAMP dependent intracellular pathway and inhibitors of the adenosine receptor-triggered Gi protein mediated intracellular pathways, can also increase acute and chronic inflammation.

In some embodiments, the additional agent that is administered in accord with the provided methods, and/or with the provided articles of manufacture or compositions, is an adenosine receptor antagonist or agonist, e.g., an antagonist or agonist of one or more of the adenosine receptors A2a, A2b, A1, and A3. A1 and A3 inhibit, and A2a and A2b stimulate, respectively, adenylate cyclase activity. Certain adenosine receptors, such as A2a, A2b, and A3, can suppress or reduce the immune response during inflammation. Thus, antagonizing immunosuppressive adenosine receptors can augment, boost or enhance immune response, e.g., immune response from administered cells, e.g., CAR-expressing T cells. In some embodiments, the additional agent inhibits the production of extracellular adenosine and adenosine-triggered signaling through adenosine receptors. For example, enhancement of an immune response, local tissue inflammation, and targeted tissue destruction can be enhanced by inhibiting or reducing the adenosine-producing local tissue hypoxia; by degrading (or rendering inactive) accumulated extracellular adenosine; by preventing or decreasing expression of adenosine receptors on immune cells; and/or by inhibiting/antagonizing signaling by adenosine ligands through adenosine receptors.

In some embodiments, the additional agent that is administered in accord with the provided methods, and/or with the provided articles of manufacture or compositions, is an adenosine receptor antagonist. In some embodiments, the antagonist is small molecule or chemical compound of an adenosine receptor, such as the A2a, A2b, or A3 receptor. In some embodiments, the antagonist is a peptide, or a pepidomimetic, that binds the adenosine receptor but does not trigger a Gi protein dependent intracellular pathway. Examples of such antagonists are described in U.S. Pat. Nos. 5,565,566; 5,545, 627, 5,981,524; 5,861,405; 6,066,642; 6,326,390; 5,670,501; 6,117,998; 6,232,297; 5,786,360; 5,424,297; 6,313,131, 5,504,090; and 6,322,771.

In some embodiments, the additional agent is an A2 receptor (A2R) antagonist, such as an A2a antagonist. Exemplary A2R antagonists include, but are not limited to, KW6002 (istradefyline), SCH58261, caffeine, paraxanthine, 3,7-dimethyl-1-propargylxanthine (DMPX), 8-(m-chlorostyryl) caffeine (CSC), MSX-2, MSX-3, MSX-4, CGS-15943, ZM-241385, SCH-442416, preladenant, vipadenant (BII014), V2006, ST-1535, SYN-115, PSB-1115, ZM241365, FSPTP, and an inhibitory nucleic acid targeting A2R expression, e.g., siRNA or shRNA, or any antibodies or antigen-binding fragment thereof that targets an A2R. In some embodiments, the additional agent is an A2R antagonist described in, e.g., Ohta et al., Proc Natl Acad Sci U S A (2006) 103:13132-13137; Jin et al., Cancer Res. (2010) 70(6):2245-2255; Leone et al., Computational and Structural Biotechnology Journal (2015) 13:265-272; Beavis et al., Proc Natl Acad Sci U S A (2013) 110:14711-14716; and Pinna, A., Expert Opin Investig Drugs (2009) 18:1619-1631; Sitkovsky et al., Cancer Immunol Res (2014) 2(7):598-605; US 8,080,554; US 8,716,301; US 20140056922; WO2008/147482; US 8,883,500; US 20140377240; WO02/055083; US 7,141,575; US 7,405,219; US 8,883,500; US 8,450,329 and US 8,987,279).

In particular embodiments, an adenosine receptor antagonist that is an antisense molecule, inhibitory nucleic acid molecule (e.g., small inhibitory RNA (siRNA)) or catalytic nucleic acid molecule (e.g. a ribozyme) that specifically binds mRNA encoding an adenosine receptor. In some embodiments, the antisense molecule, inhibitory nucleic acid molecule or catalytic nucleic acid molecule binds nucleic acids encoding A2a, A2b, or A3. In some embodiments, an antisense molecule, inhibitory nucleic acid molecule or catalytic nucleic acid targets biochemical pathways downstream of the adenosine receptor. For example, the antisense molecule or catalytic nucleic acid can inhibit an enzyme involved in the Gs protein- or Gi protein-dependent intracellular pathway. In some embodiments, the additional agent includes dominant negative mutant form of an adenosine receptor, such as A2a, A2b, or A3.

In some embodiments, the additional agent that is administered in accord with the provided methods, and/or with the provided articles of manufacture or compositions, is an agent that inhibits extracellular adenosine. Agents that inhibit extracellular adenosine include agents that render extracellular adenosine non-functional (or decrease such function), such as a substance that modifies the structure of adenosine to inhibit the ability of adenosine to signal through adenosine receptors. In some embodiments, the additional agent is an extracellular adenosine-generating or adenosine-degrading enzyme, a modified form thereof or a modulator thereof. For example, in some embodiments, the additional agent is an enzyme (e.g. adenosine deaminase) or another catalytic molecule that selectively binds and destroys the adenosine, thereby abolishing or significantly decreasing the ability of endogenously formed adenosine to signal through adenosine receptors and terminate inflammation.

In some embodiments, the additional agent is an adenosine deaminase (ADA) or a modified form thereof, e.g., recombinant ADA and/or polyethylene glycol-modified ADA (ADA-PEG), which can inhibit local tissue accumulation of extracellular adenosine. ADA-PEG has been used in treatment of patients with ADA SCID (Hershfield (1995) Hum Mutat. 5:107). In some embodiments, an agent that inhibits extracellular adenosine includes agents that prevent or decrease formation of extracellular adenosine, and/or prevent or decrease the accumulation of extracellular adenosine, thereby abolishing, or substantially decreasing, the immunosuppressive effects of adenosine. In some embodiments, the additional agent specifically inhibits enzymes and proteins that are involved in regulation of synthesis and/or secretion of pro-inflammatory molecules, including modulators of nuclear transcription factors. Suppression of adenosine receptor expression or expression of the Gs protein- or Gi protein-dependent intracellular pathway, or the cAMP dependent intracellular pathway, can result in an increase/enhancement of immune response.

In some embodiments, the additional agent can target ectoenzymes that generate or produce extracellular adenosine. In some embodiments, the additional agent targets CD39 and CD73 ectoenzymes, which function in tandem to generate extracellular adenosine. CD39 (also called ectonucleoside triphosphate diphosphohydrolase) converts extracellular ATP (or ADP) to 5'AMP. Subsequently, CD73 (also called 5'nucleotidase) converts 5'AMP to adenosine. The activity of CD39 is reversible by the actions of NDP kinase and adenylate kinase, whereas the activity of CD73 is irreversible. CD39 and CD73 are expressed on tumor stromal cells, including endothelial cells and Tregs, and also on many cancer cells. For example, the expression of CD39 and CD73 on endothelial cells is increased under the hypoxic conditions of the tumor microenvironment. Tumor hypoxia can result from inadequate blood supply and disorganized tumor vasculature, impairing delivery of oxygen (Carroll and Ashcroft (2005), Expert. Rev. Mol. Med. 7(6):1-16). Hypoxia also inhibits adenylate kinase (AK), which converts adenosine to AMP, leading to very high extracellular adenosine concentration. Thus, adenosine is released at high concentrations in response to hypoxia, which is a condition that frequently occurs the tumor microenvironment (TME), in or around solid tumors. In some embodiments, the additional agent is one or more of anti-CD39 antibody or antigen binding fragment thereof, anti-CD73 antibody or antigen binding fragment thereof, e.g., MEDI9447 or TY/23, α-β-methylene-adenosine diphosphate (ADP), ARL 67156, POM-3, IPH52 (see, e.g., Allard et al. Clin Cancer Res (2013) 19(20):5626-5635; Hausler et al., Am J Transl Res (2014) 6(2):129-139; Zhang, B., Cancer Res. (2010) 70(16):6407-6411).

In some embodiments, the additional agent that is administered in accord with the provided methods, and/or with the provided articles of manufacture or compositions, is a chemotherapeutic agent (sometimes referred to as a cytotoxic agent). In particular embodiments, the chemotherapeutic agent is any agent known to be effective for the treatment, prevention or amelioration of hyperproliferative disorders such as cancer. Chemotherapeutic agents include, but are not limited to, small molecules, synthetic drugs, peptides, polypeptides, proteins, nucleic acids (e.g., DNA and RNA polynucleotides including, but not limited to, antisense nucleotide sequences, triple helices and nucleotide sequences encoding biologically active proteins, polypeptides or peptides), antibodies, synthetic or natural inorganic molecules, mimetic agents, and synthetic or natural organic molecules. In particular embodiments, chemotherapeutic drugs include alkylating agents, anthracyclines, cytoskeletal disruptors (taxanes), epothilones, histone deacetylase inhibitors, topoisomerase inhibitors, topoisomerase II inhibitors, kinase inhibitors, nucleotide analogs and precursor analogs, peptide antibiotics, platinum-based agents, and vinca alkaloids and derivatives.

Chemotherapeutic agents may include, but are not limited to, abarelix, aldesleukin, alemtuzumab, alitretinoin, allopurinol, altretamine, amifostine, anastrozole, arsenic trioxide, asparaginase, BCG live, bevaceizumab, bexarotene, bleomycin, bortezomib, busulfan, calusterone, camptothecin, capecitabine, carboplatin, carmustine, celecoxib, cetuximab, chlorambucil, cinacalcet, cisplatin, cladribine, cyclophosphamide, cytarabine, dacarbazine, dactinomycin, darbepoetin alfa, daunorubicin, denileukin diftitox, dexrazoxane, docetaxel, doxorubicin, dromostanolone, Elliott's B solution, epirubicin, epoetin alfa, estramustine, etoposide, exemestane, filgrastim, floxuridine, fludarabine, fluorouracil, fulvestrant, gemcitabine, gemtuzumab ozogamicin, gefitinib, goserelin, hydroxyurea, ibritumomab tiuxetan, idarubicin, ifosfamide, imatinib, interferon alfa-2a, interferon alfa-2b, irinotecan, letrozole, leucovorin, levamisole, lomustine, meclorethamine, megestrol, melphalan, mercaptopurine, mesna, methotrexate, methoxsalen, methylprednisolone, mitomycin C, mitotane, mitoxantrone, nandrolone, nofetumomab, oblimersen, oprelvekin, oxaliplatin, paclitaxel, pamidronate, pegademase, pegaspargase, pegfilgrastim, pemetrexed, pentostatin, pipobroman, plicamycin, polifeprosan, porfimer, procarbazine, quinacrine, rasburicase, rituximab, sargramostim, streptozocin, talc, tamoxifen, tarceva, temozolomide, teniposide, testolactone, thioguanine, thiotepa, topotecan, toremifene, tositumomab, trastuzumab, tretinoin, uracil mustard, valrubicin, vinblastine, vincristine, vinorelbine, and zoledronate.

In some embodiments, the additional agent is an inhibitor of hypoxia inducible factor 1 alpha (HIF-1α) signaling. Exemplary inhibitors of HIF-1α include digoxin, acriflavine, sirtuin-7 and ganetespib.

In some embodiments, the additional agent includes a protein tyrosine phosphatase inhibitor, e.g., a protein tyrosine phosphatase inhibitor described herein. In some embodiments, the protein tyrosine phosphatase inhibitor is an SHP-1 inhibitor, e.g., an SHP-1 inhibitor described herein, such as, e.g., sodium stibogluconate. In some embodiments, the protein tyrosine phosphatase inhibitor is an SHP-2 inhibitor, e.g., an SHP-2 inhibitor described herein.

In some embodiments, the additional agent is a kinase inhibitor. Kinase inhibitors, such as a CDK4 kinase inhibitor, a BTK kinase inhibitor, a MNK kinase inhibitor, or a DGK kinase inhibitor, can regulate the constitutively active survival pathways that exist in tumor cells and/or modulate the function of immune cells. In some embodiments, the kinase inhibitor is a Bruton's tyrosine kinase (BTK) inhibitor, e.g., ibrutinib. In some embodiments, the kinase inhibitor is a phosphatidylinositol-4,5-bisphosphate 3-kinase (PI3K) inhibitor. In some embodiments, the kinase inhibitor is a CDK4 inhibitor, e.g., a CDK4/6 inhibitor. In some embodiments, the kinase inhibitor is an mTOR inhibitor, such as, e.g., rapamycin, a rapamycin analog, OSI-027. The mTOR inhibitor can be, e.g., an mTORC1 inhibitor and/or an mTORC2 inhibitor, e.g., an mTORC1 inhibitor and/or mTORC2 inhibitor. In some embodiments, the kinase inhibitor is an MNK inhibitor, or a dual PI3K/mTOR inhibitor. In some embodiments, other exemplary kinase inhibitors include the AKT inhibitor perifosine, the mTOR inhibitor temsirolimus, the Src kinase inhibitors dasatinib and fostamatinib, the JAK2 inhibitors pacritinib and ruxolitinib, the PKCβ inhibitors enzastaurin and bryostatin, and the AAK inhibitor alisertib.

In some embodiments, the kinase inhibitor is a BTK inhibitor selected from ibrutinib (PCI-32765); GDC-0834; RN-486; CGI-560; CGI-1764; HM-71224; CC-292; ONO-4059; CNX-774; and LFM-A13. In some embodiments, the BTK inhibitor does not reduce or inhibit the kinase activity of interleukin-2-inducible kinase (ITK), and is selected from GDC-0834; RN-486; CGI-560; CGI-1764; HM-71224; CC-292; ONO-4059; CNX-774; and LFM-A13.

In some embodiments, the kinase inhibitor is a BTK inhibitor, e.g., ibrutinib (1-[(3R)-3-[4-Amino-3-(4-phenoxyphenyl)-1H-pyrazolo[3,4-d]pyrimidin-1-yl]piperidin-1-yl]prop-2-en-1-one; also known as PCI-32765). In some embodiments, the kinase inhibitor is a BTK inhibitor, e.g., ibrutinib (PCI-32765), and the ibrutinib is administered at a dose of about 250 mg, 300 mg, 350 mg, 400 mg, 420 mg, 440 mg, 460 mg, 480 mg, 500 mg, 520 mg, 540 mg, 560 mg, 580 mg, 600 mg (e.g., 250 mg, 420 mg or 560 mg) daily for a period of time, e.g., daily for 21 day cycle, or daily for 28 day cycle. In some embodiments, 1, 2, 3, 4, 5, 6, 7, 8, 9, 10, 11, 12 or more cycles of ibrutinib are administered. In some embodiments, the BTK inhibitor is a BTK inhibitor described in International Application WO 2015/079417.

In some embodiments, the kinase inhibitor is a PI3K inhibitor. PI3K is central to the PI3K/Akt/mTOR pathway involved in cell cycle regulation and lymphoma survival. Exemplary PI3K inhibitor includes idelalisib (PI3Kδ inhibitor). In some embodiments, the additional agent is idelalisib and rituximab.

In some embodiments, the additional agent is an inhibitor of mammalian target of rapamycin (mTOR). In some embodiments, the kinase inhibitor is an mTOR inhibitor selected from temsirolimus; ridaforolimus (also known as AP23573 and MK8669); everolimus (RAD001); rapamycin (AY22989); simapimod; AZD8055; PF04691502; SF1126; and XL765. In some embodiments, the additional agent is an inhibitor of mitogen-activated protein kinase (MAPK), such as vemurafenib, dabrafenib, and trametinib.

In some embodiments, the additional agent is an agent that regulates pro- or anti-apoptotic proteins. In some embodiments, the additional agent includes a B-cell lymphoma 2 (BCL-2) inhibitor (e.g., venetoclax, also called ABT-199 or GDC-0199; or ABT-737). Venetoclax is a small molecule (4-(4-{[2-(4-Chlorophenyl)-4,4-dimethyl-1-cyclohexen-1-yl]methyl}-1-piperazinyl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide) that inhibits the anti-apoptotic protein, BCL-2. Other agents that modulate pro- or anti-apoptotic protein include BCL-2 inhibitor ABT-737, navitoclax (ABT-263); Mcl-1 siRNA or Mcl-1 inhibitor retinoid N-(4-hydroxyphenyl) retinamide (4-HPR) for maximal efficacy. In some embodiments, the additional agent provides a pro-apoptotic stimuli, such as recombinant tumor necrosis factor-related apoptosis-inducing ligand (TRAIL), which can activate the apoptosis pathway by binding to TRAIL death receptors DR-4 and DR-5 on tumor cell surface, or TRAIL-R2 agonistic antibodies.

In some embodiments, the additional agent includes a cytotoxic agent, e.g., CPX-351 (Celator Pharmaceuticals), cytarabine, daunorubicin, vosaroxin (Sunesis Pharmaceuticals), sapacitabine (Cyclacel Pharmaceuticals), idarubicin, or mitoxantrone. In some embodiments, the additional agent includes a hypomethylating agent, e.g., a DNA methyltransferase inhibitor, e.g., azacitidine or decitabine.

In another embodiment, the additional therapy is a transplantation, e.g., allogeneic stem cell transplant.

In some embodiments, the additional therapy is a lymphodepleting therapy. In some embodiments, lymphodepletion is performed on a subject, e.g., prior to administering engineered cells, e.g., CAR-expressing cells. In some embodiments, the lymphodepletion comprises administering one or more of melphalan, Cytoxan, cyclophosphamide, and fludarabine. In some embodiments, a lymphodepleting chemotherapy is administered to the subject prior to, concurrently with, or after administration (e.g., infusion) of engineered cells, e.g., CAR-expressing cells. In an example, the lymphodepleting chemotherapy is administered to the subject prior to administration of engineered cells, e.g., CAR-expressing cells.

In some embodiments, the additional agent is an oncolytic virus. In some embodiments, oncolytic viruses are capable of selectively replicating in and triggering the death of or slowing the growth of a cancer cell. In some cases, oncolytic viruses have no effect or a minimal effect on non-cancer cells. An oncolytic virus includes but is not limited to an oncolytic adenovirus, oncolytic Herpes Simplex Viruses, oncolytic retrovirus, oncolytic parvovirus, oncolytic vaccinia virus, oncolytic Sinbis virus, oncolytic influenza virus, or oncolytic RNA virus (e.g., oncolytic reovirus, oncolytic Newcastle Disease Virus (NDV), oncolytic measles virus, or oncolytic vesicular stomatitis virus (VSV)).

Other exemplary combination therapy, treatment and/or agents include anti-allergenic agents, anti-emetics, analgesics and adjunct therapies. In some embodiments, the additional agent includes cytoprotective agents, such as neuroprotectants, free-radical scavengers, cardioprotectors, anthracycline extravasation neutralizers and nutrients.

In some embodiments, an antibody used as an additional agent is conjugated or otherwise bound to a therapeutic agent, e.g., a chemotherapeutic agent (e.g., Cytoxan, fludarabine, histone deacetylase inhibitor, demethylating agent, peptide vaccine, anti-tumor antibiotic, tyrosine kinase inhibitor, alkylating agent, anti-microtubule or anti-mitotic agent), anti-allergic agent, anti-nausea agent (or anti-emetic), pain reliever, or cytoprotective agent described herein. In some embodiments, the additional agent is an antibody-drug conjugate.

Any of the additional agents described herein can be prepared and administered as a combination therapy described in the provided methods, uses, articles of manufacture or compositions, such as in pharmaceutical compositions comprising one or more agents of the combination therapy and a pharmaceutically acceptable carrier, such as any described herein. In some embodiments, the combination therapy in the provided methods, uses, articles of manufacture or compositions can be administered simultaneously, concurrently or sequentially, in any order with the additional agents, therapy or treatment, wherein such administration provides therapeutically effective levels each of the agents in the body of the subject. In some embodiments, the additional agent can be co-administered with the combination therapy in the provided methods, uses, articles of manufacture or compositions, for example, as part of the same pharmaceutical composition or using the same method of delivery. In some embodiments, the additional agent is administered simultaneously with the cell therapy, e.g. dose of engineered T cells (e.g. CAR⁺ T cells), but in separate compositions. In some embodiments, the additional agent is incubated with the engineered cell, e.g., CAR-expressing cells, prior to administration of the cells.

In some examples, the one or more additional agents are administered subsequent to or prior to the administration of the cell therapy, e.g. dose of engineered T cells (e.g. CAR⁺ T cells), separated by a selected time period. In some examples, the time period is 1 day, 2 days, 3 days, 4 days, 5 days, 6 days, 1 week, 2 weeks, 3 weeks, 1 month, 2 months, or 3 months. In some examples, the one or more additional agents are administered multiple times. In some embodiments, the additional agent is administered prior to the cell therapy, e.g. dose of engineered T cells (CAR⁺ T cells) in the provided methods, uses, articles of manufacture or compositions, e.g., two weeks, 12 days, 10 days, 8 days, one week, 6 days, 5 days, 4 days, 3 days, 2 days or 1 day before the administration. In some embodiments, the additional agent is administered after the cell therapy, e.g. dose of engineered T cells (e.g. CAR⁺ T cells) in the provided methods, uses, articles of manufacture or compositions, e.g., two weeks, 12 days, 10 days, 8 days, one week, 6 days, 5 days, 4 days, 3 days, 2 days or 1 day after the administration.

The dose of the additional agent can be any therapeutically effective amount, e.g., any dose amount described herein, and the appropriate dosage of the additional agent may depend on the type of disease to be treated, the type, dose and/or frequency of the binding molecule, recombinant receptor, cell and/or composition administered, the severity and course of the disease, previous therapy, the patient's clinical history and response to cell therapy, e.g. dose of engineered T cells (CAR⁺ T cells), and the discretion of the attending physician.

### VI. ARTICLES OF MANUFACTURE AND KITS

Also provided are articles of manufacture and kits containing engineered cells expressing a recombinant receptor or compositions thereof, and optionally instructions for use, for example, instructions for administering, according to the provided methods.

In some embodiments, provided are articles of manufacture and/or kits that include a composition comprising a therapeutically effective amount of any of the engineered cells described herein, and instructions for administering, to a subject for treating a disease or condition. In some embodiments, the instructions can specify some or all of the elements of the methods provided herein. In some embodiments, the instructions specify particular instructions for administration of the cells for cell therapy, e.g., doses, timing, selection and/or identification of subjects for administration and conditions for administration. In some embodiments, the articles of manufacture and/or kits further include one or more additional agents for therapy, e.g., lymphodepleting therapy and/or combination therapy, such as any described herein and optionally further includes instructions for administering the additional agent for therapy. In some embodiments, the articles of manufacture and/or kits further comprise an agent for lymphodepleting therapy, and optionally further includes instructions for administering the lymphodepleting therapy. In some embodiments, the instructions can be included as a label or package insert accompanying the compositions for administration.

In some embodiments, the instructions specify the criteria for selection or identification of subjects for therapy. In some embodiments, such criteria include subjects having a B cell malignancy, such as a large B cell lymphoma. In some embodiments, such criteria include subjects having diffuse large B-cell lymphoma (DLBCL) or a subtype thereof or NHL or sub-type thereof and/or a high-risk NHL. In some embodiments, the instructions specify that the subjects to be treated include subjects having a disease or condition characterized or determined to be aggressive NHL, diffuse large B cell lymphoma (DLBCL), primary mediastinal large B cell lymphoma (PMBCL), T cell/histocyte-rich large B cell lymphoma (TCHRBCL), Burkitt's lymphoma (BL), mantle cell lymphoma (MCL), and/or follicular lymphoma (FL). In particular embodiments, the subject to be treated include subjects with aggressive NHL, in particular, with diffuse large B-cell lymphoma (DLBCL), not otherwise specified (NOS) and in some aspects including de novo or transformed from indolent. In some aspects, the subject or population to be treated may include and/or further include subjects with primary mediastinal B-cell lymphoma (PMBCL) or follicular lymphoma grade 3B (FL3B). In some aspects, the subject has follicular lymphoma (FL).

In some embodiments, the instructions specify that the subjects to be treated include subjects having a DLBCL, NOS, which in some cases, can be characterized as a DLBCL that is not a DLBCL with predominant extranodal location, a DLBCL that is not a large-cell lymphoma of terminally differentiated B cells, or a DLBCL that is not a B cell neoplasm with features intermediated between DLBCL and other lymphoid tumors.

In some embodiments, the instructions specify that the subjects to be treated include subjects having a DLBCL, not otherwise specified (DLBCL, NOS), which in some cases, can be characterized as a DLBCL that is not a T cell/histocyte-rich large B cell lymphoma (TCHRBCL), that is not a primary DLBCL of the central nervous system (CNS), that is not a primary cutaneous DLBCL, leg type, or a Epstein-Barr virus (EBV)-positive DLBCL (e.g., an EBV-positive DLBCL of the elderly), and optionally a DLBCL that is not a DLBCL associated with chronic inflammation.

In some embodiments, the instructions specify that the subjects to be treated include subjects having a DLBCL, NOS, which in some cases, can be characterized as a high-grade B cell lymphoma that is not a B-lymphoblastic leukemia/lymphoma (B-LBL), a high-grade B cell lymphoma that is not a Burkitt lymphoma, or a high-grade B cell lymphoma that is not a high-grade B cell lymphoma with MYC and BCL2 and/or BCL6 rearrangements. In some aspects, DLBCL is be a DLBCL, NOS, which in some cases, can be characterized as a high-grade B cell lymphoma that is not a B-lymphoblastic leukemia/lymphoma (B-LBL), a high-grade B cell lymphoma that is not a Burkitt lymphoma, or a high-grade B cell lymphoma that is not a high-grade B cell lymphoma with MYC and BCL2 and/or BCL6 rearrangements.

In some embodiments, the instructions specify that the subjects to be treated include subjects having a DLBCL, NOS, which in some cases, can be characterized as being germinal center B-cell-like (GCB) and activated B-cell-like (ABC) based on the molecular and/or cytogenetic features of the cells of origin.

In some embodiments, the instructions specify that the subjects to be treated include subjects having a DLBCL. In some aspects, the DLBCL is a DLBCL, not otherwise specified (NOS), which in some cases, can be characterized as de novo or transformed from an indolent disease. In some embodiments, the DLBCL is a de novo or a primary DLBCL. In some embodiments, the disease or condition to be treated (such as the lymphoma such as the DLBCL) is transformed from a different subtype of disease or condition, such as transformed from an indolent lymphoma, such as a follicular lymphoma (FL). In some embodiments, such other indolent lymphomas can include, for example, marginal zone B-cell lymphoma (MZL) and chronic lymphocytic leukemia/small-cell lymphocytic lymphoma (CLL/SLL). In some embodiments, the disease or condition to be treated is DLBCL transformed from follicular lymphoma (tFL); in some aspects, it is a DLBCL transformed from another indolent lymphoma. In some embodiments, the disease or condition to be treated is a DLBCL transformed from FL. In some aspects, the disease or condition to be treated is a DLBCL transformed from an indolent lymphoma other than a FL.

In some embodiments, the instructions specify that the subjects to be treated include subjects having a DLBCL that is transformed from another indolent lymphoma, such as DLBCL transformed from marginal zone lymphoma (tMZL) or DLBCL transformed from chronic lymphocytic leukemia (tCLL; Richter's). In some cases, the disease or condition to be treated is DLBCL tMZL or DLBCL tCLL. In some embodiments, it is a disease or condition to be treated transformed from an indolent lymphoma other than FL. In some embodiments, it is a DLBCL or a large B cell lymphoma, such as a DLBCL or a large B cell lymphoma transformed from an FL or other indolent lymphoma.

In some embodiments, the instructions specify that the subjects to be treated include subjects having a high-grade B cell lymphoma with MYC and BCL2 and/or BCL6 rearrangements, optionally with DLBCL histology. In some embodiments, the disease or condition to be treated is a DLBCL NOS (de novo or transformed from indolent). In some embodiments, the disease or condition is primary mediastinal B-cell lymphoma (PMBCL) or follicular lymphoma grade 3B (FL3B). In some embodiments, the disease or condition is follicular lymphoma (FL). In some embodiments, it is a DLBCL with CNS involvement.

In some embodiments, the instructions specify that the subjects to be treated include subjects that has or has been identified as having as having a double/triple hit lymphoma or a lymphoma of the double/triple hit molecular subtypes. In some embodiments, the lymphoma is a double hit lymphoma characterized by the presence of MYC (myelocytomatosis oncogene), BCL2 (B-cell lymphoma 2), and/or BCL6 (B-cell lymphoma 6) gene rearrangements (e.g., translocations). In some embodiments, the gene rearrangement affects the MYC/8q24 locus in combination with another gene rearrangement. For example, the other gene rearrangement includes t(14;18)(q32;q21) involving BCL2. In some embodiments, the gene rearrangements affect the MYC/8q24 locus in combination with BCL6/3q27. In some embodiments, the lymphoma is a triple hit lymphoma characterized by the presence of MYC, BCL2, and BCL6 gene rearrangements; see, e.g., Aukema et al., (2011) Blood 117:2319-2331.

In some embodiments, the subject or population to be treated include those subjects having poor performance status. In some aspects, the population to be treated includes, e.g., subjects having an Eastern Cooperative Oncology Group Performance Status (ECOG) that is anywhere from 0-2. In other aspects of any of the embodiments, the subjects to be treated include ECOG 0-1 or do not include ECOG2 subjects. In some embodiments, of any of the embodiments, the subjects to be treated have failed two or more prior therapies. In some embodiments, the subject does not have DLBCL transformed from marginal zone lymphoma (MZL) and chronic lymphocytic leukemia (CLL; Richter's) and/or has a DLBCL characterized as de novo or transformed from an indolent disease. In some embodiments, the subject has mantle cell lymphoma (MCL). In some of any embodiments, the subjects has follicular lymphoma (FL). In some aspects of such embodiments the subject is ECOG 0-1 or does not have or is not suspected or characterized as having DLBCL transformed from MZL or CLL. In some embodiments, the instructions specify the administration of the cell therapy is for a subject that is or has been identified as having a double/triple hit lymphoma (or high-grade B-cell lymphoma, with MYC and BCL2 and/or BCL6 rearrangements with DLBCL histology) has been identified as having a chemorefractory lymphoma, (e.g., chemorefractory DLBCL) and/or that has not achieved complete remission (CR) in response to a prior therapy.

In some embodiments, the instructions specify the dose of cells to be administered. For example, in some embodiments, the dose specified in the instructions include a total recombinant receptor (e.g., CAR)-expressing cells between at or about 1 x 10⁶ and at or about 5 x 10⁸, e.g., in the range of from at or about 1 x 10⁷ to at or about 2 x 10⁸ such cells, such as at or about 1 x 10⁷, 5 x 10⁷, 1 x 10⁸ or 1.5 x 10⁸ total such cells, or the range between any two of the foregoing values. In some embodiments, the patient is administered multiple doses, and each of the doses or the total dose can be within any of the foregoing values.

In some embodiments, the article of manufacture or kit comprises a container, optionally a vial comprising a plurality of CD4⁺ T cells expressing a recombinant receptor (e.g. CAR), and a container, optionally a vial comprising a plurality of CD8⁺ T cells expressing a recombinant receptor (e.g. CAR). In some embodiments, the article of manufacture or kit comprises a container, optionally a vial comprising a plurality of CD4⁺ T cells expressing a recombinant receptor (e.g. CAR), and further comprises, in the same container, a plurality of CD8⁺ T cells expressing a recombinant receptor (e.g. CAR). In some embodiments, a cryoprotectant is included with the cells. In some aspects the container is a bag. In some aspects, the container is a vial.

In some embodiments, the container such as the vial comprises greater than or greater than at or about 10 x 10⁶ T cells or recombinant receptor (e.g. CAR)-expressing T cells, greater than or greater than at or about 15 x 10⁶ T cells or recombinant receptor (e.g. CAR)-expressing T cells, greater than or greater than at or about 25 x 10⁶ T cells or recombinant receptor (e.g. CAR)-expressing T cell. In some aspects, the vial comprises between at or about 10 million cells per ml and at or about 70 million cells per ml, between at or about 10 million cells per ml and at or about 50 million cells per ml, between at or about 10 million cells per ml and at or about 25 million cells per ml, between at or about 10 million cells per ml and at or about 15 million cells per ml, 15 million cells per ml and at or about 70 million cells per ml, between at or about 15 million cells per ml and at or about 50 million cells per ml, between at or about 15 million cells per ml and at or about 25 million cells per ml, between at or about 25 million cells per ml and at or about 70 million cells per ml, between at or about 25 million cells per ml and at or about 50 million cells per ml, and between at or about 50 million cells per ml and at or about 70 million cells per ml. In some aspects, the concentration of cells in the container is of viable cells in the container.

In some embodiments, the container such as the vial comprises greater than at or about 0.5 x 10⁶ recombinant receptor-expressing (e.g. CAR⁺)/CD3+ cells or such viable cells per mL, greater than at or about 1.0 x 10⁶ recombinant receptor-expressing (e.g. CAR⁺)/CD3+ cells or such viable cells per mL, greater than at or about 1.5 x 10⁶ recombinant receptor-expressing (e.g. CAR⁺)/CD3+ cells or such viable cells per mL, greater than at or about 2.0 x 10⁶ recombinant receptor-expressing (e.g. CAR⁺)/CD3+ cells or such viable cells per mL. greater than at or about 2.5 x 10⁶ recombinant receptor-expressing (e.g. CAR⁺)/CD3+ cells or such viable cells per mL, greater than at or about 2.6 x 10⁶ recombinant receptor-expressing (e.g. CAR⁺)/CD3+ cells or such viable cells per mL, greater than at or about 2.7 x 10⁶ recombinant receptor-expressing (e.g. CAR⁺)/CD3+ cells or such viable cells per mL, greater than at or about 2.8 x 10⁶ recombinant receptor-expressing (e.g. CAR⁺)/CD3+ cells or such viable cells per mL, greater than at or about 2.9 x 10⁶ recombinant receptor-expressing (e.g. CAR⁺)/CD3+ cells or such viable cells per mL greater than at or about 3.0 x 10⁶ recombinant receptor-expressing (e.g. CAR⁺)/CD3+ cells or such viable cells per mL, greater than at or about 3.5 x 10⁶ recombinant receptor-expressing (e.g. CAR⁺)/CD3+ cells or such viable cells per mL, greater than at or about 4.0 x 10⁶ recombinant receptor-expressing (e.g. CAR⁺)/CD3+ cells or such viable cells per mL, greater than at or about 4.5 x 10⁶ recombinant receptor-expressing (e.g. CAR⁺)/CD3+ cells or such viable cells per mL or greater than at or about 5 x 10⁶ recombinant receptor-expressing (e.g. CAR⁺)/CD3+ cells or such viable cells per mL. In some embodiments, the CD3+ cells are CD4+ T cells. In some embodiments, the CD3+ cells are CD8+ T cells. In some embodiments, the CD3+ T cells are CD4+ and CD8+ T cells.

In some embodiments, the plurality of vials or plurality of cells or unit dose of cells specified for administration, collectively, comprises a dose of cells comprising from or from about 1 x 10⁵ to or to about 5 x 10⁸ total recombinant receptor (e.g. CAR)-expressing T cells or total T cells, 1 x 10⁵ to or to about1 x 10⁸ total recombinant receptor (e.g. CAR)-expressing T cells or total T cells, from or from about 5 x 10⁵ to or to about1 x 10⁷ total recombinant receptor (e.g. CAR)-expressing T cells or total T cells, or from or from about 1 x 10⁶ to or to about1 x 10⁷ total recombinant receptor (e.g. CAR)-expressing T cells or total T cells, each inclusive. In some embodiments, the T cells are CD3+ cells. In some embodiments, the CD3+ cells are CD8+ T cells. In some embodiments, the CD3+ T cells are CD4+ and CD8+ T cells. In some embodiments, the plurality of vials or plurality of cells or unit dose of cells specified for administration include one or more unit doses of recombinant receptor (e.g. CAR)-expressing CD3+ CD4+ T cells and one or more unit doses of recombinant receptor (e.g. CAR)-expressing CD3+ CD8+ T cells. In some embodiments, the number of cells of each unit dose are viable cells.

In some aspects, the article comprises one or more unit dose of the CD4⁺ and CD8⁺ cells or of the CD4⁺receptor⁺ (e.g. CAR+) cells and CD8⁺receptor⁺ (e.g. CAR+) cells, wherein the unit dose comprises between at or about 1 x 10⁷ and at or about 2 x 10⁸ recombinant receptor (e.g. CAR)-expressing T cells, between at or about 5 x 10⁷ and at or about 1.5 x 10⁸ recombinant receptor (e.g. CAR)-expressing T cells, at or about 5 x 10⁷ recombinant receptor (e.g. CAR)-expressing T cells, at or about 1 x 10⁸ recombinant receptor (e.g. CAR)-expressing T cells, or at or about 1.5 x 10⁸ recombinant receptor (e.g. CAR)-expressing T cells, optionally wherein the information in the article specifies administration of one or of a plurality of unit doses and/or a volume corresponding to such one or plurality of unit doses. In some cases, the article comprises one or more unit doses of the CD8⁺ cells, wherein the dose comprises between at or about 5 x 10⁶ and at or about 1 x 10⁸ recombinant receptor (e.g. CAR)-expressing CD8⁺ T cells, the dose comprises between at or about 1 x 10⁷ and at or about 0.75 x 10⁸ recombinant receptor (e.g. CAR)-expressing CD8⁺ T cells, the dose comprises at or about 2.5 x 10⁷ recombinant receptor (e.g. CAR)-expressing CD8⁺ T cells, or the dose comprises at or about 5 x 10⁷ recombinant receptor (e.g. CAR)-expressing CD8⁺ T cells, or the dose comprises at or about 0.75 x 10⁸ recombinant receptor (e.g. CAR)-expressing CD8⁺ T cells, optionally wherein the information in the article specifies administration of one or of a plurality of unit doses and/or a volume corresponding to such one or plurality of unit doses. In some cases, the article comprises one or more unit doses of the CD4⁺ cells, wherein the dose comprises between at or about 5 x 10⁶ and at or about 1 x 10⁸ recombinant receptor (e.g. CAR)-expressing CD4⁺ T cells, the dose comprises between at or about 1 x 10⁷ and at or about 0.75 x 10⁸ recombinant receptor (e.g. CAR)-expressing CD4⁺ T cells, the dose comprises at or about 2.5 x 10⁷ recombinant receptor (e.g. CAR)-expressing CD4⁺ T cells, or the dose comprises at or about 5 x 10⁷ recombinant receptor (e.g. CAR)-expressing CD4⁺ T cells, or the dose comprises at or about 0.75 x 10⁸ recombinant receptor (e.g. CAR)-expressing CD4⁺ T cells, optionally wherein the information in the article specifies administration of one or of a plurality of unit doses and/or a volume corresponding to such one or plurality of unit doses. In some embodiments, the cells in the article, collectively, comprise a dose of cells comprising no more than at or about 1 x 10⁸ total recombinant receptor (e.g. CAR)-expressing T cells or total T cells or CD3+ cells, no more than at or about 1 x 10⁷ total recombinant receptor (e.g. CAR)-expressing T cells or total T cells or CD3+ cells, no more than at or about 0.5 x 10⁷ total recombinant receptor (e.g. CAR)-expressing T cells or total T cells or CD3+ cells, no more than at or about 1 x 10⁶ total recombinant receptor (e.g. CAR)-expressing T cells or total T cells or CD3+, no more than at or about 0.5 x 10⁶ total recombinant receptor (e.g. CAR)-expressing T cells or total T cells or CD3+ cells. In some embodiments, the number of cells of each unit dose are viable cells.

In some embodiments, each vial or the plurality of vials or plurality of cells or unit dose of cells specified for administration, collectively, comprises a flat dose of cells or fixed dose of cells such that the dose of cells is not tied to or based on the body surface area or weight of a subject.

In some embodiments, a unit dose of a cell is or comprises the number or amount of cells, such as engineered T cells, that can be administered to a subject or a patient in a single dose. In some embodiments, as unit dose is a fraction of the number of cells for administration in a given dose.

In some embodiments, each vial or the plurality of vials or plurality of cells or unit dose of cells specified for administration, collectively, comprises a dose that includes fewer than about 5 x 10⁸ total recombinant receptor (e.g., CAR)-expressing cells, T cells, or peripheral blood mononuclear cells (PBMCs), e.g., in the range of at or about 1 x 10⁶ to at or 5 x 10⁸ such cells, such as at or about 2 x 10⁶, 5 x 10⁶, 1 x 10⁷, 2.5 x 10⁷, 5 x 10⁷, 7.5 x 10⁷, 1 x 10⁸, 1.5 x 10⁸, or 5 x 10⁸ total such cells, or the range between any two of the foregoing values.

In some embodiments, each vial or the plurality of vials or plurality of cells or unit dose of cells specified for administration, collectively, comprises a dose of genetically engineered cells comprising from at or about 1 x 10⁵ to at or about 5 x 10⁸ total CAR-expressing T cells, from at or about 1 x 10⁵ to at or about 2.5 x 10⁸ total CAR-expressing T cells, from at or about 1 x 10⁵ to at or about 1 x 10⁸ total CAR-expressing T cells, from at or about 1 x 10⁵ to at or about 5 x 10⁷ total CAR-expressing T cells, from at or about 1 x 10⁵ to at or about 2.5 x 10⁷ total CAR-expressing T cells, from at or about 1 x 10⁵ to at or about 1 x 10⁷ total CAR-expressing T cells, from at or about 1 x 10⁵ to at or about 5 x 10⁶ total CAR-expressing T cells, from at or about 1 x 10⁵ to at or about 2.5 x 10⁶ total CAR-expressing T cells, from at or about 1 x 10⁵ to at or about 1 x 10⁶ total CAR-expressing T cells, from at or about 1 x 10⁶ to at or about 5 x 10⁸ total CAR-expressing T cells, from at or about 1 x 10⁶ to at or about 2.5 x 10⁸ total CAR-expressing T cells, from at or about 1 x 10⁶ to at or about 1 x 10⁸ total CAR-expressing T cells, from at or about 1 x 10⁶ to at or about 5 x 10⁷ total CAR-expressing T cells, from at or about 1 x 10⁶ to at or about 2.5 x 10⁷ total CAR-expressing T cells, from at or about 1 x 10⁶ to at or about 1 x 10⁷ total CAR-expressing T cells, from at or about 1 x 10⁶ to at or about 5 x 10⁶ total CAR-expressing T cells, from at or about 1 x 10⁶ to at or about 2.5 x 10⁶ total CAR-expressing T cells, from at or about 2.5 x 10⁶ to at or about 5 x 10⁸ total CAR-expressing T cells, from at or about 2.5 x 10⁶ to at or about 2.5 x 10⁸ total CAR-expressing T cells, from at or about 2.5 x 10⁶ to at or about 1 x 10⁸ total CAR-expressing T cells, from at or about 2.5 x 10⁶ to at or about 5 x 10⁷ total CAR-expressing T cells, from at or about 2.5 x 10⁶ to at or about 2.5 x 10⁷ total CAR-expressing T cells, from at or about 2.5 x 10⁶ to at or about 1 x 10⁷ total CAR-expressing T cells, from at or about 2.5 x 10⁶ to at or about 5 x 10⁶ total CAR-expressing T cells, from at or about 5 x 10⁶ to at or about 5 x 10⁸ total CAR-expressing T cells, from at or about 5 x 10⁶ to at or about 2.5 x 10⁸ total CAR-expressing T cells, from at or about 5 x 10⁶ to at or about 1 x 10⁸ total CAR-expressing T cells, from at or about 5 x 10⁶ to at or about 5 x 10⁷ total CAR-expressing T cells, from at or about 5 x 10⁶ to at or about 2.5 x 10⁷ total CAR-expressing T cells, from at or about 5 x 10⁶ to at or about 1 x 10⁷ total CAR-expressing T cells, from at or about 1 x 10⁷ to at or about 5 x 10⁸ total CAR-expressing T cells, from at or about 1 x 10⁷ to at or about 2.5 x 10⁸ total CAR-expressing T cells, from at or about 1 x 10⁷ to at or about 1 x 10⁸ total CAR-expressing T cells, from at or about 1 x 10⁷ to at or about 5 x 10⁷ total CAR-expressing T cells, from at or about 1 x 10⁷ to at or about 2.5 x 10⁷ total CAR-expressing T cells, from at or about 2.5 x 10⁷ to at or about 5 x 10⁸ total CAR-expressing T cells, from at or about 2.5 x 10⁷ to at or about 2.5 x 10⁸ total CAR-expressing T cells, from at or about 2.5 x 10⁷ to at or about 1 x 10⁸ total CAR-expressing T cells, from at or about 2.5 x 10⁷ to at or about 5 x 10⁷ total CAR-expressing T cells, from at or about 5 x 10⁷ to at or about 5 x 10⁸ total CAR-expressing T cells, from at or about 5 x 10⁷ to at or about 2.5 x 10⁸ total CAR-expressing T cells, from at or about 5 x 10⁷ to at or about 1 x 10⁸ total CAR-expressing T cells, from at or about 1 x 10⁸ to at or about 5 x 10⁸ total CAR-expressing T cells, from at or about 1 x 10⁸ to at or about 2.5 x 10⁸ total CAR-expressing T cells, from at or about or 2.5 x 10⁸ to at or about 5 x 10⁸ total CAR-expressing T cells.

In some embodiments, each vial or the plurality of vials or plurality of cells or unit dose of cells specified for administration, collectively, comprises a dose of genetically engineered cells comprising at least or at least about 1 x 10⁵ CAR-expressing cells, at least or at least about 2.5 x 10⁵ CAR-expressing cells, at least or at least about 5 x 10⁵ CAR-expressing cells, at least or at least about 1 x 10⁶ CAR-expressing cells, at least or at least about 2.5 x 10⁶ CAR-expressing cells, at least or at least about 5 x 10⁶ CAR-expressing cells, at least or at least about 1 x 10⁷ CAR-expressing cells, at least or at least about 2.5 x 10⁷ CAR-expressing cells, at least or at least about 5 x 10⁷ CAR-expressing cells, at least or at least about 1 x 10⁸ CAR-expressing cells, at least or at least about 2.5 x 10⁸ CAR-expressing cells, or at least or at least about 5 x 10⁸ CAR-expressing cells. In some embodiments, the number of cells is the number of such cells that are viable cells.

In some embodiments, the instructions for administration of a dose of engineered cell specify administering a number of cell of from or from about 1 x 10⁵ to or to about 5 x 10⁸ total recombinant receptor-expressing cells, total T cells, or total peripheral blood mononuclear cells (PBMCs), from or from about 5 x 10⁵ to or to about 1 x 10⁷ total recombinant receptor-expressing cells, total T cells, or total peripheral blood mononuclear cells (PBMCs) or from or from about 1 x 10⁶ to or to about 1 x 10⁷ total recombinant receptor-expressing cells, total T cells, or total peripheral blood mononuclear cells (PBMCs), each inclusive. In some embodiments, the cell therapy comprises administration of a dose of cells comprising a number of cells at least or at least about 1 x 10⁵ total recombinant receptor-expressing cells, total T cells, or total peripheral blood mononuclear cells (PBMCs), such at least or at least 1 x 10⁶, at least or at least about 1 x 10⁷, at least or at least about 1 x 10⁸ of such cells. In some embodiments, the number is with reference to the total number of CD3⁺ or CD8⁺ or CD4+ and CD8+, in some cases also recombinant receptor-expressing (e.g. CAR⁺) cells. In some embodiments, the instructions for administration of a dose specify administering a number of cell from or from about 1 x 10⁵ to or to about 5 x 10⁸ CD3⁺ or CD8⁺ or CD4+ and CD8+ total T cells or CD3⁺, CD8⁺ or CD4+ and CD8+ recombinant receptor (e.g. CAR)-expressing cells, from or from about 5 x 10⁵ to or to about 1 x 10⁷ CD3⁺, CD8⁺ or CD4⁺ and CD8⁺ total T cells or CD3⁺, CD8⁺ or CD4⁺ and CD8⁺ recombinant receptor (e.g. CAR)-expressing cells, or from or from about 1 x 10⁶ to or to about 1 x 10⁷ CD3⁺, CD8⁺ or CD4⁺ and CD8⁺ total T cells or CD3⁺, CD8⁺ or CD4⁺ and CD8⁺ recombinant receptor (e.g. CAR)-expressing cells, each inclusive. In some embodiments, the instructions for administration of a dose specify administering a number of cells from or from about 1 x 10⁵ to or to about 5 x 10⁸ total CD3⁺/CAR⁺ or CD8⁺/CAR⁺ or CD4⁺/CD8⁺/CAR⁺ cells, from or from about 5 x 10⁵ to or to about 1 x 10⁷ total CD3⁺/CAR⁺ or CD8⁺/CAR⁺ or CD4⁺/CD8⁺/CAR⁺ cells, or from or from about 1 x 10⁶ to or to about 1 x 10⁷ total CD3⁺/CAR⁺ or CD8⁺/CAR⁺ or CD4⁺/CD8⁺/CAR⁺ cells, each inclusive. In some embodiments, the number of cells is the number of such cells that are viable cells.

In some embodiments, the instructions for administration of a dose specify administering a number of cells comprising at least or at least about 2.5 x 10⁷ CD3+/CAR⁺, CD8⁺/CAR⁺, or CD4⁺/CD8⁺/CAR⁺ T cells, at least or at least about 5 x 10⁷ CD3+/CAR⁺, CD8⁺/CAR⁺, or CD4⁺/CD8⁺/CAR⁺ T cells, or at least or at least about 1 x 10⁸ CD3+/CAR⁺, CD8⁺/CAR⁺, or CD4⁺/CD8⁺/CAR⁺ T cells. In some embodiments, instructions for administration of a dose specify administering a number of cells comprising at or about 2.5 x 10⁷ CD3+/CAR⁺, CD8⁺/CAR⁺, or CD4⁺/CD8⁺/CAR⁺ T cells, at or about 5 x 10⁷ CD3+/CAR⁺, CD8⁺/CAR⁺, or CD4⁺/CD8⁺/CAR⁺ T cells, or at or about 1 x 10⁸ CD3+/CAR⁺, CD8⁺/CAR⁺, or CD4⁺/CD8⁺/CAR⁺ T cells. In some embodiments, the number of cells is the number of such cells that are viable cells.

In some embodiments, instructions for administration of a dose specify administering a number of cells that is or is about 5 x 10⁷ CD3+ CAR+ viable cells, that includes a separate dose of at or about 2.5 x 10⁷ CD4+ CAR+ viable cells and at or about 2.5 x 10⁷ CD8+CAR+ viable cells. In some embodiments, instructions for administration of a dose specify administering a number of cells that is or is about 1 x 10⁸ CD3+CAR+ viable cells, that includes a separate dose of at or about 5 x 10⁷ CD4+CAR+ viable cells and at or about 5 x10⁷ CD8+CAR+ viable cells. In some embodiments, instructions for administration of a dose specify administering a number of cells that is or is about 1.5 x 10⁸ CD3+CAR+ viable cells, that includes a separate dose of at or about 0.75 x 10⁸ CD4+CAR+ viable cells and at or about 0.75 x10⁸ CD8+CAR+ viable cells.

In some embodiments, the instructions can specify dosage regimen and timing of the administration. For example, in some embodiments, the instructions can specify administering to the subject multiple doses, e.g., two or more doses, of the cells. In some embodiments, the instructions specify the timing of the multiple doses, e.g., the second dose being administered approximately 4, 5, 6, 7, 8, 9, 10, 11, 12, 13, 14, 15, 16, 17, 18, 19, 20 or 21 days after the first dose; and/or the dosage amount in each dose.

In some embodiments, the article of manufacture or kit comprises a plurality of CD4⁺ T cells expressing a recombinant receptor, and instructions for administering, to a subject having a disease or condition, all or a portion of the plurality of CD4⁺ T cells and further administering CD8⁺ T cells expressing a recombinant receptor. In some embodiments, the instructions specify administering the CD4⁺ T cells prior to administering the CD8⁺ cells. In some cases, the instructions specify administering the CD8⁺ T cells prior to administering the CD4⁺ cells. In some embodiments, the article of manufacture or kit comprises a plurality of CD8⁺ T cells expressing a recombinant receptor, and instructions for administering, to a subject having a disease or condition, all or a portion of the plurality of CD8⁺ T cells and CD4⁺ T cells expressing a recombinant receptor. In some embodiments, the instructions specify dosage regimen and timing of the administration of the cells.

In some aspects, the instructions specify administering all or a portion of the CD4⁺ T cells and the all or a portion of the CD8⁺ T cells with 48 hours apart, such as no more than 36 hours apart, no more than 24 hours apart, no more than 12 hours, apart, such as 0 to 12 hours apart, 0 to 6 hours apart or 0 to 2 hours apart. In some cases, the instructions specify administering the CD4⁺ T cells and the CD8⁺ T cells no more than 2 hours, no more than 1 hour, no more than 30 minutes, no more than 15 minutes, no more than 10 minutes or no more than 5 minutes apart.

In some embodiments, the instructions specify the dose or number of cells or cell type(s) and/or a ratio of cell types, e.g., individual populations or sub-types, such as the CD4⁺ to CD8⁺ ratio. In some embodiments, the populations or sub-types of cells, such as CD8⁺ and CD4⁺ T cells. For example, in some embodiments, the instructions specify that the cells are administered at or within a tolerated range of an output ratio of multiple cell populations or sub-types, such as CD4⁺ and CD8⁺ cells or sub-types, of between at or about 5:1 and at or about 5:1 (or greater than about 1:5 and less than about 5:1), or between at or about 1:3 and at or about 3:1 (or greater than about 1:3 and less than about 3:1), such as between at or about 2:1 and at or about 1:5 (or greater than about 1:5 and less than about 2:1, such as at or about 5:1, 4.5:1, 4:1, 3.5:1, 3:1, 2.5:1, 2:1, 1.9:1, 1.8:1, 1.7:1, 1.6:1, 1.5:1, 1.4:1, 1.3:1, 1.2:1, 1.1:1, 1:1, 1:1.1, 1:1.2, 1:1.3, 1:1.4, 1:1.5, 1:1.6, 1:1.7, 1:1.8, 1:1.9: 1:2, 1:2.5, 1:3, 1:3.5, 1:4, 1:4.5, or 1:5. In some aspects, the tolerated difference is within about 1%, about 2%, about 3%, about 4% about 5%, about 10%, about 15%, about 20%, about 25%, about 30%, about 35%, about 40%, about 45%, about 50% of the desired ratio, including any value in between these ranges.

In some embodiments, the articles of manufacture and/or kits further include one or more additional agents for therapy, e.g., lymphodepleting therapy and/or combination therapy, as described herein, and optionally instructions for administering the additional agents. In some examples, the articles of manufacture may further contain one or more therapeutic agents. In some embodiments, the therapeutic agent is an immunomodulatory agent, a cytotoxic agent, an anti-cancer agent or a radiotherapeutic.

In some embodiments, the articles of manufacture and/or kits further include one or more agents or treatments for treating, preventing, delaying, reducing or attenuating the development or risk of development of a toxicity and/or instructions for the administration of one or more agents or treatments for treating, preventing, delaying, reducing or attenuating the development or risk of development of a toxicity in the subject. In some embodiments, the agent is or comprises an anti-IL-6 antibody or anti-IL-6 receptor antibody. For example, in some embodiments, the agent or treatment is or comprises an agent selected from among tocilizumab, siltuximab, clazakizumab, sarilumab, olokizumab (CDP6038), elsilimomab, ALD518/BMS-945429, sirukumab (CNTO 136), CPSI-2634, ARGX-109, FE301 and FM101. For example, in some embodiments, the agent or treatment is or comprises one or more of a steroid; an antagonist or inhibitor of a cytokine receptor or cytokine selected from among IL-10, IL-10R, IL-6, IL-6 receptor, IFNγ, IFNGR, IL-2, IL-2R/CD25, MCP-1, CCR2, CCR4, MIP1β, CCR5, TNFalpha, TNFR1, IL-1, and IL-1Ralpha/IL-1beta; or an agent capable of preventing, blocking or reducing microglial cell activity or function.

In some embodiments, the agent capable of preventing, blocking or reducing microglial cell activity or function is selected from an anti-inflammatory agent, an inhibitor of NADPH oxidase (NOX2), a calcium channel blocker, a sodium channel blocker, inhibits GM-CSF, inhibits CSF1R, specifically binds CSF-1, specifically binds IL-34, inhibits the activation of nuclear factor kappa B (NF-κB), activates a CB₂ receptor and/or is a CB₂ agonist, a phosphodiesterase inhibitor, inhibits microRNA-155 (miR-155) or upregulates microRNA-124 (miR-124). In some cases, the agent is selected from minocycline, naloxone, nimodipine, Riluzole, MOR103, lenalidomide, a cannabinoid (optionally WIN55 or 212-2), intravenous immunoglobulin (IVIg), ibudilast, anti-miR-155 locked nucleic acid (LNA), MCS110, PLX-3397, PLX647, PLX108-D1, PLX7486, JNJ-40346527, JNJ28312141, ARRY-382, AC-708, DCC-3014, 5-(3-methoxy-4-((4-methoxybenzyl)oxy)benzyl) pyrimidine-2,4-diamine (GW2580), AZD6495, Ki20227, BLZ945, emactuzumab, IMC-CS4, FPA008, LY-3022855, AMG-820 and TG-3003. In some embodiments, the agent is an inhibitor of colony stimulating factor 1 receptor (CSF1R). For example, the agent PLX-3397, PLX647, PLX108-D1, PLX7486, JNJ-40346527, JNJ28312141, ARRY-382, AC-708, DCC-3014, 5-(3-methoxy-4-((4-methoxybenzyl)oxy)benzyl)pyrimidine-2,4-diamine (GW2580), AZD6495, Ki20227, BLZ945 or a pharmaceutical salt or prodrug thereof; emactuzumab, IMC-CS4, FPA008, LY-3022855, AMG-820 and TG-3003 or is an antigen-binding fragment thereof, or a combination of any of the foregoing.

In some embodiments, the articles of manufacture and/or kits further include one or more reagents for assaying biological samples, e.g., biological samples from subjects who are candidates for administration or who have been administered the therapy, and optionally instructions for use of the reagents or assays. In some embodiments, the biological sample is or is obtained from a blood, plasma or serum sample In some embodiments, the reagents can be used prior to the administration of the cell therapy or after the administration of cell therapy, for diagnostic purposes, to identify subjects and/or to assess treatment outcomes and/or toxicities. For example, in some embodiments, the article of manufacture and/or kits further contain reagents for measuring the level of particular biomarkers, e.g., cytokines or analytes, that are associated with toxicity, and instructions for measuring. In some embodiments, the reagents include components for performing an *in vitro* assay to measure the biomarkers (e.g. analytes), such as an immunoassay, an aptamer-based assay, a histological or cytological assay, or an mRNA expression level assay. In some embodiments, the *in vitro* assay is selected from among an enzyme linked immunosorbent assay (ELISA), immunoblotting, immunoprecipitation, radioimmunoassay (RIA), immunostaining, flow cytometry assay, surface plasmon resonance (SPR), chemiluminescence assay, lateral flow immunoassay, inhibition assay and avidity assay. In some aspects, the reagent is a binding reagent that specifically binds the biomarkers (e.g. analytes). In some cases, the binding reagent is an antibody or antigen-binding fragment thereof, an aptamer or a nucleic acid probe.

In some embodiments, the articles of manufacture and/or kits comprise one or more reagent capable of detecting one or more analytes, and instructions for using the reagent to assay a biological sample from a subject that is a candidate for treatment, wherein the one or more analytes is selected from LDH, ferritin, CRP, IL-6, IL-7, IL-8, IL-10, IL-15, IL-16, TNF-alpha, IFN-gamma, MCP-1, MIP-1beta, eotaxin, G-CSF, IL-1Ralpha, IL-1Rbeta, IP-10, perforin, and D-dimer (fibrin degradation product). In some embodiments, the one or more analytes include LDH, ferritin and/or CRP. In some embodiments, instructions for assaying presence or absence, level, amount, or concentration of an analyte in the subject compared to a threshold level of the analyte is also included. In some embodiments, the instructions specify methods for carrying out assessment or monitoring of such analytes, e.g. LDH, ferritin and/or CRP, according to any of the provided methods.

In some embodiments, the instructions are included which specify, if the level, amount or concentration of the analyte in the sample is at or above a threshold level for the analyte, administering to the subject an agent or other treatment capable of treating, preventing, delaying, reducing or attenuating the development or risk of development of a toxicity (i) prior to, (ii) within one, two, or three days of, (iii) concurrently with and/or (iv) at first fever following, the initiation of administration of the cell therapy to the subject. In some cases, the instructions specify that if the level, amount or concentration of the analyte in the sample is at or above a threshold level for the analyte, the cell therapy is administered to the subject at a reduced dose or at a dose that is not associated with risk of developing toxicity or severe toxicity, or is not associated with a risk of developing a toxicity or severe toxicity in a majority of subjects, and/or a majority of subjects having a disease or condition that the subject has or is suspected of having, following administration of the cell therapy. In some cases, the instructions specify that if the level, amount or concentration of the analyte in the sample is at or above a threshold level for the analyte, the cell therapy is administered in an inpatient setting and/or with admission to the hospital for one or more days, optionally wherein the cell therapy is otherwise to be administered to subjects on an outpatient basis or without admission to the hospital for one or more days.

In some embodiments, the instructions for administering the cell therapy specify, if the level, amount or concentration of the analyte in the sample, is below a threshold level, administering to the subject the cell therapy, optionally at a non-reduced dose, optionally on an outpatient basis or without admission to the hospital for one or more days. In some embodiments, the instructions for administering the cell therapy specify, if the level, amount or concentration of the analyte in the sample, is below a threshold level, prior to or concurrently with administering the cell therapy and/or prior to the development of a sign or symptom of a toxicity other than fever, an agent or treatment capable of treating, preventing, delaying, or attenuating the development of the toxicity is not administered to the subject. In some aspects, the instructions for administering the cell therapy specify that if the level, amount or concentration of the analyte in the sample, is below a threshold level, the administration of the cell therapy is to be or may be administered to the subject on an outpatient setting and/or without admission of the subject to the hospital overnight or for one or more consecutive days and/or is without admission of the subject to the hospital for one or more days.

The articles of manufacture and/or kits may further include a cell therapy and/or further include instructions for use with, prior to and/or in connection with treatment with the cell therapy. In some embodiments, the instructions are included for administering the agent and the instructions specify if the level, amount or concentration of the analyte in the sample, is at or above a threshold level administering to the subject the agent. In some aspects, the instructions further specify administering a cell therapy to the subject, wherein administration of the agent is to be carried out (i) prior to, (ii) within one, two, or three days of, (iii) concurrently with and/or (iv) at first fever following, the initiation of administration of the cell therapy to the subject.

The articles of manufacture and/or kits may include a container and a label or package insert on or associated with the container. Suitable containers include, for example, bottles, vials, syringes, IV solution bags, etc. The containers may be formed from a variety of materials such as glass or plastic. The container in some embodiments holds a composition which is by itself or combined with another composition effective for treating, preventing and/or diagnosing the condition. In some embodiments, the container has a sterile access port. Exemplary containers include an intravenous solution bags, vials, including those with stoppers pierceable by a needle for injection, or bottles or vials for orally administered agents. The label or package insert may indicate that the composition is used for treating a disease or condition. The article of manufacture may include (a) a first container with a composition contained therein, wherein the composition includes engineered cells expressing a recombinant receptor; and (b) a second container with a composition contained therein, wherein the composition includes the second agent. In some embodiments, the article of manufacture may include (a) a first container with a first composition contained therein, wherein the composition includes a subtype of engineered cells expressing a recombinant receptor; and (b) a second container with a composition contained therein, wherein the composition includes a different subtype of engineered cells expressing a recombinant receptor. The article of manufacture may further include a package insert indicating that the compositions can be used to treat a particular condition. Alternatively, or additionally, the article of manufacture may further include another or the same container comprising a pharmaceutically-acceptable buffer. It may further include other materials such as other buffers, diluents, filters, needles, and/or syringes.

### VII. EXEMPLARY EMBODIMENTS

Among the provided embodiments are:
1. A method of assessing the risk of developing a toxicity after administration of a cell therapy, the method comprising:
   (a) assessing one or more parameters in a subject, wherein the one or more parameters is selected from the level, amount or concentration of LDH, ferritin or C-reactive protein (CRP) in a biological sample from the subject, or is a sum of product dimensions (SPD) of a tumor in the subject;
      wherein the subject is a candidate for treatment with the cell therapy, said cell therapy comprising a dose of genetically engineered cells expressing a recombinant receptor; and
      wherein the assessing is carried out prior to administering the cell therapy and/or said biological sample or tumor does not comprise the recombinant receptor and/or said engineered cells; and
   (b) identifying if the subject is as at risk of toxicity wherein the subject is at risk of toxicity if the one or more parameters is above a threshold level and the subject is not at risk of toxicity if the one or more parameters is at or below a threshold level, wherein:
      (i) the parameter is a sum of product dimensions (SPD), and the threshold level is above at or about 30 cm², 40 cm², 50 cm², 60 cm² or 70 cm²;
      (ii) the parameter is LDH, and the threshold level is above at or about 300 units per liter, 400 units per liter, 500 units per liter or 600 units per liter;
      (iii) the parameter is ferritin, and the threshold level is above at or about 1000 nanograms per milliliter, 2000 nanograms per milliliter, 3000 nanograms per milliliter, 4000 nanograms per milliliter, 5000 nanograms per milliliter, 6000 nanograms per milliliter, 7000 nanograms per milliliter or 8000 nanograms per milliliter; and/or
      (iv) the parameter is CRP, and the threshold level is above at or about 5 milligrams per liter, 10 milligrams per liter, 15 milligrams per liter, 20 milligrams per liter, 25 milligrams per liter, 30 milligrams per liter, 40 milligrams per liter or 50 milligrams per liter.
2. A method of identifying a subject, the method comprising:
   (a) assessing one or more parameters in a subject, wherein the one or more parameters is selected from the level, amount or concentration of LDH, ferritin or C-reactive protein (CRP) in a biological sample from the subject, or is a sum of product dimensions (SPD) of a tumor in the subject;
      wherein the subject is a candidate for treatment with the cell therapy, said cell therapy comprising a dose of genetically engineered cells expressing a recombinant receptor; and
      wherein the assessing is carried out prior to administering the cell therapy and/or said biological sample or tumor does not comprise the recombinant receptor and/or said engineered cells; and
   (b) identifying a subject who has a risk of developing a toxicity after administration of a cell therapy, wherein the subject is at risk of toxicity if the one or more parameters is above a threshold level and the subject is not at risk of toxicity if the one or more parameters is at or below a threshold level, wherein:
      (i) the parameter is a sum of product dimensions (SPD), and the threshold level is above at or about 30 cm², 40 cm², 50 cm², 60 cm² or 70 cm²;
      (ii) the parameter is LDH, and the threshold level is above at or about 300 units per liter, 400 units per liter, 500 units per liter or 600 units per liter;
      (iii) the parameter is ferritin, and the threshold level is above at or about 1000 nanograms per milliliter, 2000 nanograms per milliliter, 3000 nanograms per milliliter, 4000 nanograms per milliliter, 5000 nanograms per milliliter, 6000 nanograms per milliliter, 7000 nanograms per milliliter or 8000 nanograms per milliliter; and/or
      (iv) the parameter is CRP, and the threshold level is above at or about 5 milligrams per liter, 10 milligrams per liter, 15 milligrams per liter, 20 milligrams per liter, 25 milligrams per liter, 30 milligrams per liter, 40 milligrams per liter or 50 milligrams per liter.
3. A method of treatment, comprising:
   (a) assessing one or more parameters in a subject, wherein the one or more parameters is selected from the level, amount or concentration of LDH, ferritin or C-reactive protein (CRP) in a biological sample from the subject, or is a sum of product dimensions (SPD) of a tumor in the subject;
      wherein the subject is a candidate for treatment with the cell therapy, said cell therapy comprising a dose of genetically engineered cells expressing a recombinant receptor; and
      wherein the assessing is carried out prior to administering the cell therapy and/or said biological sample or tumor does not comprise the recombinant receptor and/or said engineered cells; and
   (b) identifying if the subject is as at risk of toxicity wherein the subject is at risk of toxicity if the one or more parameters is above a threshold level and the subject is not at risk of toxicity if the one or more parameters is at or below a threshold level, wherein:
      (i) the parameter is a sum of product dimensions (SPD), and the threshold level is above at or about 30 cm², 40 cm2, 50 cm², 60 cm² or 70 cm²;
      (ii) the parameter is LDH, and the threshold level is above at or about 300 units per liter, 400 units per liter, 500 units per liter or 600 units per liter;
      (iii) the parameter is ferritin, and the threshold level is above at or about 1000 nanograms per milliliter, 2000 nanograms per milliliter, 3000 nanograms per milliliter, 4000 nanograms per milliliter, 5000 nanograms per milliliter, 6000 nanograms per milliliter, 7000 nanograms per milliliter or 8000 nanograms per milliliter; and/or
      (iv) the parameter is CRP, and the threshold level is above at or about 5 milligrams per liter, 10 milligrams per liter, 15 milligrams per liter, 20 milligrams per liter, 25 milligrams per liter, 30 milligrams per liter, 40 milligrams per liter or 50 milligrams per liter; and
   (c) following or based on the results of the assessment, administering to the subject the cell therapy, and, optionally, an agent or other treatment capable of treating, preventing, delaying, reducing or attenuating the development or risk of development of a toxicity.
4. The method of any of embodiments 1-3, wherein the parameter is SPD, and the threshold level is 50 cm².
5. The method of any of embodiments 1-3, the parameter is LDH, and the threshold level is 500 units per liter.
6. The method of any of embodiments 1-5, wherein the one or more parameter is SPD and LDH, and the threshold level for SPD is 50 cm² and the threshold level for LDH is 500 units per liter.
7. The method of any of embodiments 1-3, wherein the parameter is ferritin, and the threshold level is 5000 nanograms per milliliter.
8. The method of any of embodiments 1-3, wherein the parameter is CRP, and the threshold level is 10 milligrams per liter.
9. The method of any of embodiments 1-3, 7 and 8, wherein the one or more parameter is ferritin and CRP, and the threshold level for ferritin is 5000 nanograms per milliliter and the threshold level for CRP is 10 milligrams per liter.
10. The method of any of embodiments 1-9, wherein the biological sample is a blood or plasma sample.
11. The method of any of embodiments 1-10, wherein the SPD is measured based on CT and/or MRI imaging or other imaging of the body.
12. The method of any of embodiments 1-11, wherein the level, amount or concentration of one or more analyte and/or the SPD is measured prior to treatment, prior to apheresis, or prior to cell product manufacturing.
13. A method of assessing the risk of developing a toxicity after administration of a cell therapy, the method comprising:
   (a) assessing the peak concentration of genetically engineered cells expressing a recombinant receptor in a biological sample from a subject that has been previously administered a cell therapy comprising the genetically engineered cells; and
   (b) identifying if the subject is as at risk of toxicity wherein the subject is at risk of toxicity if the one or more parameters is above a threshold level and the subject is not at risk of toxicity if the peak concentration of genetically engineered cells is at or below a threshold level, wherein: the threshold level is above at or about 300 cells per microliter, 400 cells per microliter, 500 cells per microliter, 600 cells per microliter, 700 cells per microliter, 800 cells per microliter, 900 cells per microliter or 1000 cells per microliter.
14. A method of identifying a subject, the method comprising:
   (a) assessing the peak concentration of genetically engineered cells expressing a recombinant receptor in a biological sample from a subject that has been previously administered a cell therapy comprising the genetically engineered cells; and
   (b) identifying a subject who has a risk of developing a toxicity after administration of a cell therapy, wherein the subject is at risk of toxicity if the one or more parameters is above a threshold level and the subject is not at risk of toxicity if the peak concentration of genetically engineered cells is at or below a threshold level, wherein: the threshold level is above at or about 300 cells per microliter, 400 cells per microliter, 500 cells per microliter, 600 cells per microliter, 700 cells per microliter, 800 cells per microliter, 900 cells per microliter or 1000 cells per microliter.
15. A method of treatment, comprising:
   (a) assessing the peak concentration of genetically engineered cells expressing a recombinant receptor in a biological sample from a subject that has been previously administered a cell therapy comprising the genetically engineered cells; and
   (b) identifying if the subject is as at risk of toxicity wherein the subject is at risk of toxicity if the one or more parameters is above a threshold level and the subject is not at risk of toxicity if the peak concentration of genetically engineered cells is at or below a threshold level, wherein: the threshold level is above at or about 300 cells per microliter, 400 cells per microliter, 500 cells per microliter, 600 cells per microliter, 700 cells per microliter, 800 cells per microliter, 900 cells per microliter or 1000 cells per microliter; and
   (c) following or based on the results of the assessment, administering to the subject the cell therapy, and, optionally, an agent or other treatment capable of treating, preventing, delaying, reducing or attenuating the development or risk of development of a toxicity.
16. The method of any of embodiments 13-15, wherein threshold level is 500 cells per microliter.
17. The method of any of embodiments 1-16, further comprising monitoring the subject for symptoms of toxicity if the subject is administered a cell therapy and is identified as having a risk of developing a toxicity.
18. The method of any of embodiments 1-17, wherein the toxicity is neurotoxicity or CRS.
19. The method of any of embodiments 1-18, wherein the toxicity is a grade 1 or higher neurotoxicity or CRS.
20. The method of any of embodiments 1-19, wherein the toxicity is a severe neurotoxicity or is a grade 2 or higher neurotoxicity, a grade 3 or higher neurotoxicity, or a grade 4 or higher neurotoxicity
21. The method of any of embodiments 1-20, wherein the toxicity is a grade 1 or higher neurotoxicity.
22. The method of any of embodiments 1-20, wherein the toxicity is a severe neurotoxicity or a grade 3 or higher neurotoxicity.
23. The method of any of embodiments 1-19, wherein the toxicity is a severe CRS or is a grade 2 or higher CRS, a grade 3 or higher CRS, or a grade 4 or higher CRS.
24. The method of any of embodiments 1-19 and 23, wherein the toxicity is a grade 1 or higher CRS.
25. The method of any of embodiments 1-19 and 23, wherein the toxicity is a severe CRS or a grade 3 or higher CRS.
26. The method of any of embodiments 1-25, wherein if the subject is identified as having a risk of developing a toxicity, administering to the subject:
   (a) (1) an agent or other treatment capable of treating, preventing, delaying, reducing or attenuating the development or risk of development of a toxicity and (2) the cell therapy, wherein administration of the agent is to be administered (i) prior to, (ii) within one, two, or three days of, (iii) concurrently with and/or (iv) at first fever following, the initiation of administration of the cell therapy to the subject; and/or
   (b) a cell therapy at a reduced dose or at a dose that is not associated with risk of developing toxicity or severe toxicity, or is not associated with a risk of developing a toxicity or severe toxicity in a majority of subjects, and/or a majority of subjects having a disease or condition that the subject has or is suspected of having, following administration of the cell therapy; and/or
   (c) administering to the subject a cell therapy in an inpatient setting and/or with admission to the hospital for one or more days, optionally wherein the cell therapy is otherwise to be administered to subjects on an outpatient basis or without admission to the hospital for one or more days.
27. The method of embodiment 26, wherein the agent or other treatment is an anti-IL-6 antibody or an anti-IL-6 receptor antibody.
28. The method of embodiment 27, wherein the agent or other treatment is or comprises an agent selected from among tocilizumab, siltuximab, clazakizumab, sarilumab, olokizumab (CDP6038), elsilimomab, ALD518/BMS-945429, sirukumab (CNTO 136), CPSI-2634, ARGX-109, FE301 and FM101.
29. The method of embodiment 27 or embodiment 28, wherein the agent or other treatment is tocilizumab.
30. The method of embodiment 26, wherein the agent or other treatment is or comprises one or more steroids.
31. The method of embodiment 30, wherein the steroid is dexamethasone or methylprednisolone.
32. The method of embodiment 30 or embodiment 31, wherein the steroid is dexamethasone.
33. The method of embodiment 26, wherein the agent or other treatment comprises administration of a vasopressor.
34. The method of embodiment 26, wherein the agent or other treatment comprises intubation.
35. The method of embodiment 26, wherein the agent or other treatment comprises dialysis.
36. The method of any of embodiments 1-35, wherein the recombinant receptor specifically binds to an antigen associated with the disease or condition or expressed in cells of the environment of a lesion associated with the disease or condition.
37. The method of any of embodiments 1-36, wherein the disease or condition is a cancer.
38. The method of any of embodiments 1-37, wherein the disease or condition is a myeloma, a leukemia or a lymphoma.
39. The method of any of embodiments 1-38a, wherein the disease or condition is a B cell malignancy and/or is acute lymphoblastic leukemia (ALL), adult ALL, chronic lymphoblastic leukemia (CLL), non-Hodgkin lymphoma (NHL), or a large B cell lymphoma.
40. The method of any of embodiments 1-39, wherein the disease or condition is a large B cell lymphoma.
41. The method of embodiment 40, wherein the large B cell lymphoma is a Diffuse Large B-Cell Lymphoma (DLBCL).
42. The method of embodiment 41, wherein the DLBCL is a DLBCL, not otherwise specified (NOS), a de novo DLBCL or a DLBCL transformed from indolent lymphoma.
43. The method of embodiment 41 or embodiment 42, wherein the DLBCL is a de novo DLBCL.
44. The method of embodiment 41 or embodiment 42, wherein the DLBCL is a DLBCL transformed from follicular lymphoma (tFL).
45. The method of embodiment 41 or embodiment 42, wherein the DLBCL is a DLBCL transformed from marginal zone lymphoma (tMZL) or a DLBCL transformed from chronic lymphocytic leukemia (tCLL; Richter's).
46. The method of any of embodiments 1-39, wherein the disease or condition is primary mediastinal B-cell lymphoma (PMBCL) or a follicular lymphoma (FL), optionally follicular lymphoma grade 3B (FL3B).
47. The method of any of embodiments 1-46, wherein the antigen is a B cell antigen, optionally CD19.
48. The method of any of embodiments 1-47, wherein the recombinant receptor is a chimeric antigen receptor (CAR).
49. The method of any of embodiments 1-48, wherein the engineered cells comprise T cells, optionally CD4⁺ T cells and/or CD8⁺ T cells.
50. The method of any of embodiments 1-49, wherein the cell therapy comprises administering a dose of CD4⁺ and CD8⁺ T cells is administered to a subject, each of the CD4⁺ and the CD8⁺ T cells, individually, comprising a receptor that specifically binds to a target antigen expressed by the disease or condition or a cell or tissue thereof and/or that is associated with the disease or condition, wherein the administration comprises administering a plurality of separate compositions, the plurality of separate compositions comprising a first composition comprising CD8⁺ T cells and a second composition comprising CD4⁺ T cells.
51. The method of embodiment 50, wherein the initiation of the administration of the first composition is carried out prior to the initiation of the administration of the second composition.
52. The method of embodiment 50 or embodiment 51, wherein the administration of the first composition and the administration of the second composition are carried out no more than 48 hours apart.
53. The method of any of embodiments 50-52, wherein the administration of the first composition and the administration of the second composition are carried out no more than 36 hours apart, no more than 24 hours apart, no more than 12 hours apart, no more than 6 hours apart, no more than 4 hours apart, no more than 2 hours apart, no more than 1 hour apart or no more than 30 minutes apart.
54. The method of any of embodiments 50-53, wherein the receptor comprised by the CD4⁺ T cells and/or the receptor comprised by the CD8⁺ T cells comprises a recombinant receptor that is the same and/or wherein the CD4⁺ T cells and/or the CD8⁺ T cells are genetically engineered to express a recombinant receptor that is the same.
55. The method of any of embodiments 50-54, wherein:
   the dose of CD4⁺ and CD8⁺ T cells comprises a defined ratio of CD4⁺ cells expressing a recombinant receptor to CD8⁺ cells expressing a recombinant receptor and/or of CD4⁺ cells to CD8⁺ cells, that is or is approximately 1:1 or is between approximately 1:3 and approximately 3:1; and/or
   the CD4⁺ T cells comprising the receptor in the one of the first and second compositions and the CD8⁺ T cells comprising the receptor in the other of the first and second compositions are present at a defined ratio that is or is approximately 1:1 or is between approximately 1:3 and approximately 3:1; and/or
   the CD4⁺ T cells comprising the receptor and the CD8⁺ T cells comprising the receptor administered in the first and second compositions are present at a defined ratio, which ratio is or is approximately 1:1 or is between approximately 1:3 and approximately 3:1.
56. The method of embodiment 55, wherein the defined ratio is or is approximately 1:1.
57. The method of any of embodiments 50-56, wherein the dose of CD4⁺ and CD8⁺ T cells comprises:
   between at or about 1 x 10⁷ and at or about 2 x 10⁸ total recombinant receptor-expressing T cells, inclusive;
   between at or about 2.5 x 10⁷ and at or about 1.5 x 10⁸ total recombinant receptor-expressing T cells, inclusive;
   between at or about 5 x 10⁷ and at or about 1 x 10⁸ total recombinant receptor-expressing T cells, inclusive;
   at or about 5 x 10⁷ total recombinant receptor-expressing T cells;
   at or about 1 x 10⁸ total recombinant receptor-expressing T cells; or at or about 1.5 x 10⁸ total recombinant receptor-expressing T cells.
58. The method of any of embodiments 50-57, wherein the dose of CD4⁺ and CD8⁺ T cells comprises at or about 5 x 10⁷ total recombinant receptor-expressing T cells.
59. The method of any of embodiments 50-57, wherein the dose of CD4⁺ and CD8⁺ T cells comprises at or about 1 x 10⁸ total recombinant receptor-expressing T cells.
60. The method of any of embodiments 50-57, wherein the dose of CD4⁺ and CD8⁺ T cells comprises at or about 1.5 x 10⁸ total recombinant receptor-expressing T cells.
61. The method of any of embodiments 50-60, wherein the dose of CD4⁺ and CD8⁺ T cells comprises:
   between at or about 1 x 10⁷ and at or about 1 x 10⁸ recombinant receptor-expressing CD8⁺ T cells, inclusive;
   between at or about 1.25 x 10⁷ and at or about 7.5 x 10⁷ recombinant receptor-expressing CD8⁺ T cells, inclusive;
   between at or about 2.5 x 10⁷ and at or about 5 x 10⁷ recombinant receptor-expressing CD8⁺ T cells, inclusive;
   at or about 2.5 x 10⁷ recombinant receptor-expressing CD8⁺ T cells;
   at or about 5 x 10⁷ recombinant receptor-expressing CD8⁺ T cells; or at or about 7.5 x 10⁷ recombinant receptor-expressing CD8⁺ T cells.
62. The method of any of embodiments 50-61, wherein the dose of CD4⁺ and CD8⁺ T cells comprises at or about 2.5 x 10⁷ recombinant receptor-expressing CD8⁺ T cells.
63. The method of any of embodiments 50-61, wherein the dose of CD4⁺ and CD8⁺ T cells comprises at or about 5 x 10⁷ recombinant receptor-expressing CD8⁺ T cells.
64. The method of any of embodiments 50-61, wherein the dose of CD4⁺ and CD8⁺ T cells comprises at or about 7.5 x 10⁷ recombinant receptor-expressing CD8⁺ T cells.
65. The method of any of embodiments 47-64, wherein the T cells are primary T cells obtained from a subject or are autologous to the subject.
66. The method of any of embodiments 1-65, wherein the subject is a human subject.
67. An article of manufacture comprising a composition comprising genetically engineered cells expressing a recombinant receptor and optionally an agent or other treatment capable of treating, preventing, delaying, reducing or attenuating the development or risk of development of a toxicity; and instructions for assessing the risk of developing a toxicity, identifying a subject or treating a subject in accord with the method of any of embodiments 1-66.
68. A method of treating a subject having or suspected of having a disease or condition, the method comprising administering to the subject a dose of CD4⁺ and CD8⁺ T cells, each of the CD4⁺ and the CD8⁺ T cells, individually, comprising a recombinant receptor that specifically binds to a target antigen expressed by the disease or condition or a cell or tissue thereof and/or that is associated with the disease or condition, wherein the administration comprises administering a plurality of separate compositions, the plurality of separate compositions comprising a first composition comprising CD8⁺ T cells and a second composition comprising CD4⁺ T cells.
69. A method of treating a subject having or suspected of having a disease or condition that is a B cell malignancy, the method comprising administering to the subject a dose of CD4⁺ and CD8⁺ T cells, each of the CD4⁺ and the CD8⁺ T cells, individually, comprising a recombinant receptor that specifically binds to a target antigen expressed by the disease or condition or a cell or tissue thereof and/or that is associated with the disease or condition, wherein the administration comprises administering a plurality of separate compositions, the plurality of separate compositions comprising a first composition comprising CD8⁺ T cells and a second composition comprising CD4⁺ T cells.
70. A method of treating a subject having or suspected of having a disease or condition that is a large B cell lymphoma, the method comprising administering to the subject a dose of CD4⁺ and CD8⁺ T cells, each of the CD4⁺ and the CD8⁺ T cells, individually, comprising a recombinant receptor that specifically binds to a target antigen expressed by the disease or condition or a cell or tissue thereof and/or that is associated with the disease or condition, wherein the administration comprises administering a plurality of separate compositions, the plurality of separate compositions comprising a first composition comprising CD8⁺ T cells and a second composition comprising CD4⁺ T cells.
71. A method of treating a subject having or suspected of having a disease or condition that is a non-Hodgkin lymphoma (NHL) or a large B cell lymphoma, the method comprising administering to the subject a dose of T cells comprising T cells expressing a chimeric antigen receptor (CAR) that specifically binds to a target antigen expressed by the NHL, wherein:
   the dose of T cells comprises between at or about 1 x 10⁷ CAR-expressing T cells and at or about 2 x 10⁸ CAR-expressing T cells, inclusive; and
   the dose of T cells comprises CD4⁺ and CD8⁺ T cells, each of the CD4⁺ and the CD8⁺ T cells, individually, comprising a receptor that specifically binds to a target antigen expressed by the disease or condition or a cell or tissue thereof and/or that is associated with the disease or condition, wherein the administration comprises administering a plurality of separate compositions, the plurality of separate compositions comprising a first composition comprising CD8⁺ T cells and a second composition comprising CD4⁺ T cells.
72. The method of embodiment 71, wherein the dose of T cells comprises a defined ratio of CD4⁺ cells expressing the CAR to CD8⁺ cells expressing the CAR and/or of CD4⁺ cells to CD8⁺ cells, which ratio is approximately 1:1 or is between approximately 1:3 and approximately 3:1.
73. A method of treating a subject having a disease or condition that is a non-Hodgkin lymphoma (NHL) or a large B cell lymphoma, the method comprising administering to the subject a dose of T cells comprising T cells expressing a chimeric antigen receptor (CAR) that specifically binds to a target antigen expressed by the NHL, the dose of T cells comprising a defined ratio of CD4⁺ cells expressing the CAR to CD8⁺ cells expressing the CAR and/or of CD4⁺ cells to CD8⁺ cells, which ratio is approximately or is 1:1, wherein the administration comprises administering a plurality of separate compositions, the plurality of separate compositions comprising a first composition comprising CD8⁺ T cells and a second composition comprising CD4⁺ T cells.
74. A method of treating a subject having a disease or condition that is a non-Hodgkin lymphoma (NHL) or a large B cell lymphoma, the method comprising administering to the subject a dose of T cells comprising T cells expressing a chimeric antigen receptor (CAR) that specifically binds to a target antigen expressed by the NHL, wherein:
   the dose of T cells comprises between at or about 5 x 10⁷ recombinant receptor-expressing T cells and at or about 1.5 x 10⁸ recombinant receptor-expressing T cells, inclusive, said dose comprising a defined ratio of CD4⁺ cells expressing the recombinant receptor to CD8⁺ cells expressing the recombinant receptor and/or of CD4⁺ cells to CD8⁺ cells, which ratio is approximately or is 1:1; and
   the method results in (1) a complete response (CR) in at least 35%, at least 40% or at least 50% of subjects treated and/or objective response (OR) in at least 50%, at least 60% or at least 70% of subjects treated and (2) results in no more than 50% of subjects exhibiting a cytokine release syndrome (CRS) higher than grade 2 and/or a neurotoxicity higher than grade 2, wherein the administration comprises administering a plurality of separate compositions, the plurality of separate compositions comprising a first composition comprising CD8⁺ T cells and a second composition comprising CD4⁺ T cells.
75. A method of treating a subject, the method comprising administering, to a subject that has a disease or condition that is a lymphoma, a dose of T cells comprising T cells expressing a chimeric antigen receptor (CAR) that specifically binds to a target antigen expressed by the lymphoma, wherein the lymphoma in the subject is associated with or involves central nervous system (CNS) involvement; and the dose of T cells comprises CD4⁺ and CD8⁺ T cells, each of the CD4⁺ and the CD8⁺ T cells, individually, comprising a receptor that specifically binds to a target antigen expressed by the disease or condition or a cell or tissue thereof and/or that is associated with the disease or condition, wherein the administration comprises administering a plurality of separate compositions, the plurality of separate compositions comprising a first composition comprising CD8⁺ T cells and a second composition comprising CD4⁺ T cells.
76. The method of embodiment 75, wherein, at or prior to the time of administration of the dose of cells, the subject comprises a brain lesion, optionally a temporal lobe brain lesion.
77. The method of embodiment 75 or embodiment 76, wherein the lymphoma is a B cell malignancy.
78. The method of any of embodiments 75-77, wherein the lymphoma is non-Hodgkin lymphoma (NHL) or a large B cell lymphoma.
79. The method of any of embodiments 68-78, wherein the initiation of the administration of the first composition is carried out prior to the initiation of the administration of the second composition.
80. The method of any of embodiments 68-79, wherein the administration of the first composition and the administration of the second composition are carried out no more than 48 hours apart.
81. The method of any of embodiments 68-80, wherein the administration of the first composition and the administration of the second composition are carried out no more than 36 hours apart, no more than 24 hours apart, no more than 12 hours apart, no more than 6 hours apart, no more than 4 hours apart, no more than 2 hours apart, no more than 1 hour apart or no more than 30 minutes apart.
82. The method of any of embodiments 68-81, wherein the administration of the first composition and the administration of the second composition are carried out between at or about 0 and at or about 48 hours, between at or about 0 and at or about 36 hours, between at or about 0 and at or about 24 hours, between at or about 0 and at or about 12 hours, between at or about 0 and at or about 6 hours, between at or about 0 and at or about 2 hours, between at or about 0 and at or about 1 hours, between at or about 0 and at or about 30 minutes, between at or about 30 minutes and at or about 48 hours, between at or about 30 minutes and at or about 36 hours, between at or about 30 minutes and at or about 24 hours, between at or about 30 minutes and at or about 12 hours, between at or about 30 minutes and at or about 6 hours, between at or about 30 minutes and at or about 4 hours, between at or about 30 minutes and at or about 2 hours, between at or about 30 minutes and at or about 1 hour, between at or about 1 hours and at or about 48 hours, between at or about 1 hour and at or about 36 hours, between at or about 1 hour and at or about 24 hours, between at or about 1 hour and at or about 12 hours, between at or about 1 hour and at or about 6 hours, between at or about 1 hour and at or about 4 hours, between at or about 1 hour and at or about 2 hours, between at or about 2 hours and at or about 48 hours, between at or about 2 hours and at or about 36 hours, between at or about 2 hours and at or about 24 hours, between at or about 2 hours and at or about 12 hours, between at or about 2 hours and at or about 6 hours, between at or about 2 hours and at or about 4 hours, between at or about 4 hours and at or about 48 hours, between at or about 4 hours and at or about 36 hours, between at or about 4 hours and at or about 24 hours, between at or about 4 hours and at or about 12 hours, between at or about 4 hours and at or about 6 hours, between at or about 6 hours and at or about 48 hours, between at or about 6 hours and at or about 36 hours, between at or about 6 hours and at or about 24 hours, between at or about 6 hours and at or about 12 hours, between at or about 12 hours and at or about 48 hours, between at or about 12 hours and at or about 36 hours, between at or about 12 hours and at or about 24 hours, between at or about 24 hours and at or about 48 hours, between at or about 24 hours and at or about 36 hours or between at or about 36 hours and at or about 48 hours.
83. The method of any of embodiments 68-82, wherein:
   the administration of the first composition and the administration of the second composition are carried out on the same day, are carried out between about 0 and about 12 hours apart, between about 0 and about 6 hours apart or between about 0 to 2 hours apart; or
   the initiation of administration of the first composition and the initiation of administration of the second composition are carried out between about 1 minute and about 1 hour apart or between about 5 minutes and about 30 minutes apart.
84. The method of any of embodiments 68-83, wherein the first composition and second composition are administered no more than 2 hours, no more than 1 hour, no more than 30 minutes, no more than 15 minutes, no more than 10 minutes or no more than 5 minutes apart.
85. The method of any of embodiments 68-84, wherein the receptor comprised by the CD4⁺ T cells and/or the receptor comprised by the CD8⁺ T cells comprises a recombinant receptor that is the same and/or wherein the CD4⁺ T cells and/or the CD8⁺ T cells are genetically engineered to express a recombinant receptor that is the same.
86. The method of any of embodiments 68-71 and 75-85, wherein:
   the dose of CD4⁺ and CD8⁺ T cells comprises a defined ratio of CD4⁺ cells expressing a recombinant receptor to CD8⁺ cells expressing a recombinant receptor and/or of CD4⁺ cells to CD8⁺ cells, that is or is approximately 1:1 or is between approximately 1:3 and approximately 3:1; and/or
   the CD4⁺ T cells comprising the receptor in the one of the first and second compositions and the CD8⁺ T cells comprising the receptor in the other of the first and second compositions are present at a defined ratio that is or is approximately 1:1 or is between approximately 1:3 and approximately 3:1; and/or
   the CD4⁺ T cells comprising the receptor and the CD8⁺ T cells comprising the receptor administered in the first and second compositions are present at a defined ratio, which ratio is or is approximately 1:1 or is between approximately 1:3 and approximately 3:1.
87. The method of embodiment 86, wherein the defined ratio is or is approximately 1:1.
88. The method of any of embodiments 68-70, 72, 73 and 75-87, wherein the dose of CD4⁺ and CD8⁺ T cells comprises:
   between at or about 1 x 10⁷ and at or about 2 x 10⁸ total recombinant receptor-expressing T cells, inclusive;
   between at or about 2.5 x 10⁷ and at or about 1.5 x 10⁸ total recombinant receptor-expressing T cells, inclusive;
   between at or about 5 x 10⁷ and at or about 1 x 10⁸ total recombinant receptor-expressing T cells, inclusive;
   at or about 5 x 10⁷ total recombinant receptor-expressing T cells;
   at or about 1 x 10⁸ total recombinant receptor-expressing T cells; or
   at or about 1.5 x 10⁸ total recombinant receptor-expressing T cells.
89. The method of any of embodiments 68-88, wherein the dose of CD4⁺ and CD8⁺ T cells comprises at or about 5 x 10⁷ total recombinant receptor-expressing T cells.
90. The method of any of embodiments 68-88, wherein the dose of CD4⁺ and CD8⁺ T cells comprises at or about 1 x 10⁸ total recombinant receptor-expressing T cells.
91. The method of any of embodiments 68-88, wherein the dose of CD4⁺ and CD8⁺ T cells comprises at or about 1.5 x 10⁸ total recombinant receptor-expressing T cells.
92. The method of any of embodiments 68-89, wherein the dose of CD4⁺ and CD8⁺ T cells comprises:
   between at or about 1 x 10⁷ and at or about 1 x 10⁸ recombinant receptor-expressing CD8⁺ T cells, inclusive;
   between at or about 1.25 x 10⁷ and at or about 7.5 x 10⁷ recombinant receptor-expressing CD8⁺ T cells, inclusive;
   between at or about 2.5 x 10⁷ and at or about 5 x 10⁷ recombinant receptor-expressing CD8⁺ T cells, inclusive;
   at or about 2.5 x 10⁷ recombinant receptor-expressing CD8⁺ T cells;
   at or about 5 x 10⁷ recombinant receptor-expressing CD8⁺ T cells; or at or about 7.5 x 10⁷ recombinant receptor-expressing CD8⁺ T cells.
93. The method of any of embodiments 68-92, wherein the dose of CD4⁺ and CD8⁺ T cells comprises at or about 2.5 x 10⁷ recombinant receptor-expressing CD8⁺ T cells.
94. The method of any of embodiments 68-92, wherein the dose of CD4⁺ and CD8⁺ T cells comprises at or about 5 x 10⁷ recombinant receptor-expressing CD8⁺ T cells.
95. The method of any of embodiments 68-92, wherein the dose of CD4⁺ and CD8⁺ T cells comprises at or about 7.5 x 10⁷ recombinant receptor-expressing CD8⁺ T cells.
96. The method of any of embodiments 68-95, wherein the recombinant receptor specifically binds to an antigen associated with the disease or condition or expressed in cells of the environment of a lesion associated with the disease or condition.
97. The method of any of embodiments 68 and 79-96, wherein the disease or condition is a cancer.
98. The method of any of embodiments 68 and 79-95, wherein the disease or condition is a myeloma, a leukemia or a lymphoma.
99. The method of any of embodiments 68 and 79-96, wherein the disease or condition is a B cell malignancy and/or is acute lymphoblastic leukemia (ALL), adult ALL, chronic lymphoblastic leukemia (CLL), non-Hodgkin lymphoma (NHL), or a large B cell lymphoma.
100. The method of any of embodiments 68 and 79-99, wherein the disease or condition is a large B cell lymphoma.
101. The method of any of embodiments 70-74 and 78-100, wherein the large B cell lymphoma is a Diffuse Large B-Cell Lymphoma (DLBCL).
102. The method of embodiment 101, wherein the DLBCL is a DLBCL, not otherwise specified (NOS), a de novo DLBCL or a DLBCL transformed from indolent lymphoma.
103. The method of embodiment 101 or embodiment 102, wherein the DLBCL is a de novo DLBCL.
104. The method of embodiment 101 or embodiment 102, wherein the DLBCL is a DLBCL transformed from follicular lymphoma (tFL).
105. The method of embodiment 101 or embodiment 102, wherein the DLBCL is a DLBCL transformed from marginal zone lymphoma (tMZL) or a DLBCL transformed from chronic lymphocytic leukemia (tCLL; Richter's).
106. The method of any of embodiments 71-74 and 99, wherein the NHL or the large B cell lymphoma is selected from the group consisting of aggressive NHL, diffuse large B cell lymphoma (DLBCL), NOS (de novo or transformed from indolent), primary mediastinal large B cell lymphoma (PMBCL), mantle cell lymphoma (MCL), and/or follicular lymphoma (FL), optionally follicular lymphoma Grade 3B (FL3B).
107. The method of any of embodiments 68-99, wherein the disease or condition is primary mediastinal B-cell lymphoma (PMBCL) or a follicular lymphoma (FL), optionally follicular lymphoma grade 3B (FL3B).
108. The method of any of embodiments 68-107, wherein the antigen is a B cell antigen, optionally CD19.
109. The method of any of embodiments 68-70 and 79-108, wherein the recombinant receptor is a chimeric antigen receptor (CAR).
110. The method of any of embodiments 68-109, wherein the T cells are primary T cells obtained from a subject.
111. The method of any of embodiments 68-110, wherein the T cells are autologous to the subject.
112. The method of any of embodiments 68-111, wherein at least 40%, at least 50%, at least 60%, at least 70% of the subjects who, at or prior to the administration of the dose of cells had or were identified to have a double/triple hit lymphoma or relapse following administration of an autologous stem cell transplant (ASCT), achieved an OR or an OR that is durable for at or greater than 3 months or at or greater than 6 months.
113. The method of any of embodiments 68-112, wherein:
   at least 35%, at least 40% or at least 50% of subjects treated according to the method achieve a complete response (CR);
   at least 60%, 70%, 80%, 90%, or 95% of subjects achieving a CR exhibit a CR that is durable for at or greater than 3 months or at or greater than 6 months; and/or
   at least 60%, 70%, 80%, 90%, or 95% of subjects achieving a CR by one month and/or by 3 months remain in response, remain in CR, and/or survive or survive without progression, for at or greater than 3 months and/or at or greater than 6 months and/or at greater than 9 months after achieving the CR; and/or
   at least 50%, at least 60% or at least 70% of the subjects treated according to the method achieve objective response (OR);
   at least 60%, 70%, 80%, 90%, or 95% of subjects achieving an OR exhibit an OR that is durable for at or greater than 3 months or at or greater than 6 months; and/or
   at least 35%, at least 40%, or at least 50% of subjects achieving an OR remain in response or survive for at or greater than 3 months and/or at or greater than 6 months after achieving the OR; and/or
   at least 40%, at least 50%, at least 60%, at least 70% of the subjects who, at or prior to the administration of the dose of cells had or were identified to have a double/triple hit lymphoma or relapse, following administration of an autologous stem cell transplant (ASCT), achieved an OR, or an OR that is durable for at or greater than 3 months or at or greater than 6 months.
114. The method of any of embodiments 68-113, wherein the cells are autologous to the subject, and
   no minimum absolute lymphocyte count (ALC) for apheresis is required and/or specified for production of the therapy; and/or
   the cells are produced by a process which, for at least 90%, 91%, 92%, 93%, 94%, 95%, 96%, 97%, 98%, 99%, or 100% of subjects having the disease or condition or of the selected population of subjects, is capable of generating a cell product for administration according to the method.
115. The method of any of embodiment 68-114, wherein:
   greater than or greater than about 50%, about 60%, about 70%, or about 80% of the subjects treated according to the method do not exhibit a grade 3 or greater cytokine release syndrome (CRS) and/or do not exhibit a grade 3 or greater neurotoxicity and/or greater than 40% or 50% or 55% do not exhibit any neurotoxicity or CRS.
116. The method of any of embodiments 74 and 79-115, wherein:
   the CR or the OR is durable for greater than 3 months or greater than 6 months; and/or
   at least 20%, at least 25%, at least 35%, at least 40% or at least 50% of subjects treated according to the method achieve a CR that is durable for greater than 3 months or greater than 6 months; and/or
   at least 60%, 70%, 80%, 90%, or 95% of subjects treated with the method and who achieve a CR, remain in CR or remain in response or remain surviving for at or greater than 3 months or at or greater than 6 months or at or greater than 9 months; and/or
   at least 60%, 70%, 80%, 90%, or 95% of subjects treated with the method who achieve a CR by one month and/or by 3 months remain in response, remain in CR, and/or survive or survive without progression, for greater at or greater than 3 months and/or at or greater than 6 months and/or at greater than 9 months; and/or
   at least 50%, at least 60% or at least 70% of the subjects treated according to the method achieve objective response (OR);
   at least 60%, 70%, 80%, 90%, or 95% of subjects achieve an OR that is durable for at or greater than 3 months or at or greater than 6 months; and/or
   at least at least 35%, at least 40%, or at least 50% of subjects treated with the method and achieving an OR remain in response or survive for at or greater than 3 months and/or at or greater than 6 months.
117. The method of any of embodiments 68-116, wherein:
   at or prior to administration of the dose of cells, the subject is or has been identified as having a lymphoma associated with or involving central nervous system (CNS) involvement; and/or
   at least 70%, at least 80%, at least 90% or at least 95% of subjects treated according to the method who, at or prior to the administration of the dose of cells exhibited or were identified to exhibit a lymphoma with CNS involvement, achieved a resolution of the CNS disease.
118. The method of any of embodiments 75-117, wherein:
   at least 35%, at least 40% or at least 50% of subjects treated according to the method achieve a complete response (CR) or remission of CNS disease;
   at least 60%, 70%, 80%, 90%, or 95% of subjects who achieve a CR remain in CR for at or greater than 3 months or at or greater than 6 months; and/or
   at least 60%, 70%, 80%, 90%, or 95% of subjects achieving a CR or remission of CNS disease by one month and/or by 3 months remain in response, remain in CR, and/or survive or survive without progression, for greater at or greater than 3 months and/or at or greater than 6 months and/or at greater than 9 months; and/or
   at least 50%, at least 60% or at least 70% of the subjects treated according to the method achieve objective response (OR) or remission of CNS disease;
   at least 60%, 70%, 80%, 90%, or 95% of subjects achieving the OR, for at or greater than 3 months or at or greater than 6 months; and/or
   at least 60%, 70%, 80%, 90%, or 95% of subjects achieving OR or remission of CNS disease remain in response or survive for at or greater than 3 months and/or at or greater than 6 months; and/or
   the brain lesion is reduced in size or volume by greater than or greater than about 25%, 50%, 75% or more; and/or
   reduction or remission or clearance of CNS disease is achieved in at least 35%, at least 40% or at least 50% of subjects treated according to the method.
119. The method of any of embodiments 68-118, wherein:
   greater than or greater than about 30%, 35%, 40%, or 50% of the subjects treated according to the method do not exhibit any grade of cytokine release syndrome (CRS) or neurotoxicity; and/or
   at least at or about 45%, 50%, 60%, 65%, 70%, 75%, 80%, 85%, 90%, or 95% of subjects treated according to the method do not exhibit onset of CRS earlier than 3 days following initiation of the administration and/or do not exhibit onset of neurotoxicity earlier than 5 days following initiation of the administration; and/or
   the median onset of neurotoxicity among subjects treated according to the method is at or after the median peak of, or median time to resolution of, CRS in subjects treated according to the method and/or the median onset of neurotoxicity among subjects treated according to the method is greater than at or about 8, 9, 10, or 11 days.
120. The method of any of embodiments 68-119, wherein prior to initiation of administration of the dose of cells, the subject has not been administered an agent or treatment capable of treating, preventing, delaying, reducing or attenuating the development or risk of development of a toxicity following administration of the dose of cells.
121. The method of embodiment 120, wherein the agent is or comprises an anti-IL-6 antibody, anti-IL-6 receptor antibody or a steroid.
122. The method of embodiment 120 or embodiment 121, wherein the agent is or comprises tocilizumab, siltuximab, dexamethasone or methylprednisolone.
123. The method of any of embodiments 68-122, wherein:
   the administration and any follow up is carried out on an outpatient basis and/or without requiring admission to or an overnight stay at a hospital; and
   if the subject exhibits a sustained fever or a fever that is or has not been reduced or not reduced by more than 1°C after treatment with an antipyretic, the subject is admitted to the hospital or to an overnight stay at a hospital and/or is administered an agent or treatment for the treatment or prevention or reduction or attenuation of a neurotoxicity and/or a cytokine release syndrome or risk thereof.
124. The method of any of embodiments 68-123, wherein the subject is or has been identified as having an Eastern Cooperative Oncology Group Performance Status (ECOG) status of 0, 1 or 2.
125. The method of any of embodiments 68-124, wherein, at or immediately prior to the time of the administration of the dose of cells the subject has relapsed following remission after treatment with, or become refractory to, one or more prior therapies for the disease or condition, optionally one, two or three prior therapies other than another dose of cells expressing the CAR.
126. The method of any of embodiments 68-125, wherein, at or prior to the administration of the dose of cells:
   the subject is or has been identified as having a double/triple hit lymphoma; and/or
   the subject is or has been identified as having a chemorefractory lymphoma, optionally a chemorefractory DLBCL; and/or
   the subject has not achieved complete remission (CR) in response to a prior therapy; and/or
   the subject has relapsed within 1 year or less than 1 year after receiving an autologous stem cell transplant (ASCT).
127. The method of any of embodiments 68-126, comprising, prior to administration of the dose of cells, identifying or selecting a subject for the administration of the dose of cells that has:
   a double/triple hit lymphoma;
   a chemorefractory lymphoma, optionally a chemorefractory DLBCL;
   not achieved complete remission (CR) in response to a prior therapy for treating the malignancy, optionally the NHL; and/or
   has relapsed within 1 year or less than 1 year after receiving an autologous stem cell transplant (ASCT); and/or
   has a lymphoma associated with or involving central nervous system (CNS) involvement.
128. The method of any of embodiments 68-127, further comprising administering to the subject an additional therapeutic agent or therapy, optionally other than a cell therapy, optionally other than CAR⁺ T cell therapy.
129. The method of any of embodiments 48-66 and 71-128, wherein:
   the CAR comprises an extracellular antigen-binding domain specific for the antigen, a transmembrane domain, a cytoplasmic signaling domain derived from a costimulatory molecule, which optionally is a 4-1BB, and a cytoplasmic signaling domain derived from a primary signaling ITAM-containing molecule, which optionally is a CD3zeta;
   the CAR comprises, in order, an extracellular antigen-binding domain specific for the antigen, a transmembrane domain, a cytoplasmic signaling domain derived from a costimulatory molecule, and a cytoplasmic signaling domain derived from a primary signaling ITAM-containing molecule; or
   the CAR comprises an extracellular antigen-recognition domain that specifically binds to the antigen and an intracellular signaling domain comprising a CD3-zeta (CD3ζ) chain and a costimulatory signaling region that is a signaling domain of CD28 or 4-1BB.
130. The method of embodiment 129, wherein the antigen-binding domain is an scFv.
131. The method of embodiment 130, wherein the scFv comprises an amino acid sequence of RASQDISKYLN (SEQ ID NO: 35), an amino acid sequence of SRLHSGV (SEQ ID NO: 36), and/or an amino acid sequence of GNTLPYTFG (SEQ ID NO: 37) and/or an amino acid sequence of DYGVS (SEQ ID NO: 38), an amino acid sequence of VIWGSETTYYNSALKS (SEQ ID NO: 39), and/or an amino acid sequence of YAMDYWG (SEQ ID NO: 40) or wherein the scFv comprises a variable heavy chain region of FMC63 and a variable light chain region of FMC63 and/or a CDRL1 sequence of FMC63, a CDRL2 sequence of FMC63, a CDRL3 sequence of FMC63, a CDRH1 sequence of FMC63, a CDRH2 sequence of FMC63, and a CDRH3 sequence of FMC63 or binds to the same epitope as or competes for binding with any of the foregoing, and optionally wherein the scFv comprises, in order, a V_{H}, a linker, optionally comprising SEQ ID NO: 24, and a V_{L}, and/or the scFv comprises a flexible linker and/or comprises the amino acid sequence set forth as SEQ ID NO: 43.
132. The method of any of embodiments 129-131, wherein the costimulatory signaling region is a signaling domain of CD28 or 4-1BB.
133. The method of any of embodiments 129-132, wherein the costimulatory signaling region is a signaling domain of 4-1BB.
134. The method of any of embodiments 129-133, wherein the costimulatory domain comprises SEQ ID NO: 12 or a variant thereof having at least 85%, 86%, 87%, 88%, 89%, 90%, 91%, 92%, 93%, 94%, 95%, 96%, 97%, 98%, 99% or more sequence identity thereto.
135. The method of any of embodiments 129-134, wherein the primary signaling domain is a CD3zeta signaling domain.
136. The method of any of embodiments 129-135, wherein the primary signaling domain comprises SEQ ID NO: 13, 14 or 15 having at least 85%, 86%, 87%, 88%, 89%, 90%, 91%, 92%, 93%, 94%, 95%, 96%, 97%, 98%, 99% or more sequence identity thereto.
137. The method of any of embodiments 129-136, wherein the CAR further comprises a spacer between the transmembrane domain and the scFv.
138. The method of embodiment 137, wherein the spacer is a polypeptide spacer that comprises or consists of all or a portion of an immunoglobulin hinge or a modified version thereof, optionally an IgG4 hinge, or a modified version thereof.
139. The method of embodiment 137 or embodiment 138, wherein the spacer is about 15 amino acids or less, and does not comprise a CD28 extracellular region or a CD8 extracellular region.
140. The method of any of embodiments 137-139, wherein the spacer is at or about 12 amino acids in length.
141. The method of any of embodiments 137-140, wherein:
   the spacer has or consists of the sequence of SEQ ID NO: 1, a sequence encoded by SEQ ID NO: 2, SEQ ID NO: 30, SEQ ID NO: 31, SEQ ID NO: 32, SEQ ID NO: 33, SEQ ID NO: 34, or a variant of any of the foregoing having at least 85%, 86%, 87%, 88%, 89%, 90%, 91%, 92%, 93%, 94%, 95%, 96%, 97%, 98%, 99% or more sequence identity thereto; and/or
   the spacer comprises or consists of the formula X₁PPX₂P, where X₁ is glycine, cysteine or arginine and X₂ is cysteine or threonine.
142. The method of any of embodiments 48-66 and 71-141, wherein:
   the CAR comprises an scFv specific for the antigen, a transmembrane domain, a cytoplasmic signaling domain derived from a costimulatory molecule, which optionally is or comprises a 4-1BB, and a cytoplasmic signaling domain derived from a primary signaling ITAM-containing molecule, which optionally is or comprises a CD3zeta signaling domain and optionally further comprises a spacer between the transmembrane domain and the scFv;
   the CAR comprises, in order, an scFv specific for the antigen, a transmembrane domain, a cytoplasmic signaling domain derived from a costimulatory molecule, which optionally is or comprises a 4-1BB signaling domain, and a cytoplasmic signaling domain derived from a primary signaling ITAM-containing molecule, which optionally is a CD3zeta signaling domain; or
   the CAR comprises, in order, an scFv specific for the antigen, a spacer, a transmembrane domain, a cytoplasmic signaling domain derived from a costimulatory molecule, which optionally is a 4-1BB signaling domain, and a cytoplasmic signaling domain derived from a primary signaling ITAM-containing molecule, which optionally is or comprises a CD3zeta signaling domain; and wherein:
   the spacer is a polypeptide spacer that (a) comprises or consists of all or a portion of an immunoglobulin hinge or a modified version thereof or comprises about 15 amino acids or less, and does not comprise a CD28 extracellular region or a CD8 extracellular region, (b) comprises or consists of all or a portion of an immunoglobulin hinge, optionally an IgG4 hinge, or a modified version thereof and/or comprises about 15 amino acids or less, and does not comprise a CD28 extracellular region or a CD8 extracellular region, or (c) is at or about 12 amino acids in length and/or comprises or consists of all or a portion of an immunoglobulin hinge, optionally an IgG4, or a modified version thereof; or (d) has or consists of the sequence of SEQ ID NO: 1, a sequence encoded by SEQ ID NO: 2, SEQ ID NO: 30, SEQ ID NO: 31, SEQ ID NO: 32, SEQ ID NO: 33, SEQ ID NO: 34, or a variant of any of the foregoing having at least 85%, 86%, 87%, 88%, 89%, 90%, 91%, 92%, 93%, 94%, 95%, 96%, 97%, 98%, 99% or more sequence identity thereto, or (e) comprises or consists of the formula X₁PPX₂P, where X₁ is glycine, cysteine or arginine and X₂ is cysteine or threonine; and/or
   the costimulatory domain comprises SEQ ID NO: 12 or a variant thereof having at least 85%, 86%, 87%, 88%, 89%, 90%, 91%, 92%, 93%, 94%, 95%, 96%, 97%, 98%, 99% or more sequence identity thereto; and/or
   the primary signaling domain comprises SEQ ID NO: 13, 14 or 15 having at least 85%, 86%, 87%, 88%, 89%, 90%, 91%, 92%, 93%, 94%, 95%, 96%, 97%, 98%, 99% or more sequence identity thereto; and/or
   the scFv comprises an amino acid sequence of RASQDISKYLN (SEQ ID NO: 35), an amino acid sequence of SRLHSGV (SEQ ID NO: 36), and/or an amino acid sequence of GNTLPYTFG (SEQ ID NO: 37) and/or an amino acid sequence of DYGVS (SEQ ID NO: 38), an amino acid sequence of VIWGSETTYYNSALKS (SEQ ID NO: 39), and/or an amino acid sequence of YAMDYWG (SEQ ID NO: 40) or wherein the scFv comprises a variable heavy chain region of FMC63 and a variable light chain region of FMC63 and/or a CDRL1 sequence of FMC63, a CDRL2 sequence of FMC63, a CDRL3 sequence of FMC63, a CDRH1 sequence of FMC63, a CDRH2 sequence of FMC63, and a CDRH3 sequence of FMC63 or binds to the same epitope as or competes for binding with any of the foregoing, and optionally wherein the scFv comprises, in order, a V_{H}, a linker, optionally comprising SEQ ID NO: 24, and a V_{L}, and/or the scFv comprises a flexible linker and/or comprises the amino acid sequence set forth as SEQ ID NO: 43.
143. The method of any of embodiments 1-66 and 68-142, wherein, prior to the administration, the subject has been preconditioned with a lymphodepleting therapy comprising the administration of fludarabine and/or cyclophosphamide.
144. The method of any of embodiments 50-66 and 68-143, further comprising, immediately prior to the administration of a dose of the cells, administering a lymphodepleting therapy to the subject comprising the administration of fludarabine and/or cyclophosphamide.
145. The method of embodiment 144, wherein:
   the administration of the cell dose and/or the lymphodepleting therapy is carried out via outpatient delivery.
146. The method of embodiment 144 or embodiment 145, wherein, if the subject exhibits a sustained fever or a fever that is or has not been reduced or not reduced by more than 1°C after treatment with an antipyretic, the subject is admitted to the hospital or to an overnight stay at a hospital and/or is administered an agent or treatment for the treatment or prevention or reduction or attenuation of a neurotoxicity and/or a cytokine release syndrome or risk thereof.
147. The method of any of embodiments 50-66 and 68-146, wherein the dose of cells is administered parenterally, optionally intravenously.
148. The method of any of embodiments 68-147, wherein the subject is a human subject.
149. An article of manufacture comprising a composition comprising genetically engineered cells expressing a recombinant receptor; and optionally instructions for administering a dose of the cells in accord with the method of any of embodiments 68-148.

### VIII. DEFINITIONS

The terms "polypeptide" and "protein" are used interchangeably to refer to a polymer of amino acid residues, and are not limited to a minimum length. Polypeptides, including the provided receptors and other polypeptides, e.g., linkers or peptides, may include amino acid residues including natural and/or non-natural amino acid residues. The terms also include post-expression modifications of the polypeptide, for example, glycosylation, sialylation, acetylation, and phosphorylation. In some aspects, the polypeptides may contain modifications with respect to a native or natural sequence, as long as the protein maintains the desired activity. These modifications may be deliberate, as through site-directed mutagenesis, or may be accidental, such as through mutations of hosts which produce the proteins or errors due to PCR amplification.

As used herein, a "subject" is a mammal, such as a human or other animal, and typically is human. In some embodiments, the subject, e.g., patient, to whom the agent or agents, cells, cell populations, or compositions are administered, is a mammal, typically a primate, such as a human. In some embodiments, the primate is a monkey or an ape. The subject can be male or female and can be any suitable age, including infant, juvenile, adolescent, adult, and geriatric subjects. In some embodiments, the subject is a non-primate mammal, such as a rodent.

As used herein, "treatment" (and grammatical variations thereof such as "treat" or "treating") refers to complete or partial amelioration or reduction of a disease or condition or disorder, or a symptom, adverse effect or outcome, or phenotype associated therewith. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The terms do not imply complete curing of a disease or complete elimination of any symptom or effect(s) on all symptoms or outcomes.

As used herein, "delaying development of a disease" means to defer, hinder, slow, retard, stabilize, suppress and/or postpone development of the disease (such as cancer). This delay can be of varying lengths of time, depending on the history of the disease and/or individual being treated. In some embodiments, sufficient or significant delay can, in effect, encompass prevention, in that the individual does not develop the disease. For example, a late stage cancer, such as development of metastasis, may be delayed.

"Preventing," as used herein, includes providing prophylaxis with respect to the occurrence or recurrence of a disease in a subject that may be predisposed to the disease but has not yet been diagnosed with the disease. In some embodiments, the provided cells and compositions are used to delay development of a disease or to slow the progression of a disease.

As used herein, to "suppress" a function or activity is to reduce the function or activity when compared to otherwise same conditions except for a condition or parameter of interest, or alternatively, as compared to another condition. For example, cells that suppress tumor growth reduce the rate of growth of the tumor compared to the rate of growth of the tumor in the absence of the cells.

An "effective amount" of an agent, e.g., a pharmaceutical formulation, cells, or composition, in the context of administration, refers to an amount effective, at dosages/amounts and for periods of time necessary, to achieve a desired result, such as a therapeutic or prophylactic result.

A "therapeutically effective amount" of an agent, e.g., a pharmaceutical formulation or cells, refers to an amount effective, at dosages and for periods of time necessary, to achieve a desired therapeutic result, such as for treatment of a disease, condition, or disorder, and/or pharmacokinetic or pharmacodynamic effect of the treatment. The therapeutically effective amount may vary according to factors such as the disease state, age, sex, and weight of the subject, and the populations of cells administered. In some embodiments, the provided methods involve administering the cells and/or compositions at effective amounts, e.g., therapeutically effective amounts.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount. In the context of lower tumor burden, the prophylactically effective amount in some aspects will be higher than the therapeutically effective amount.

The term "about" as used herein refers to the usual error range for the respective value readily known to the skilled person in this technical field. Reference to "about" a value or parameter herein includes (and describes) embodiments that are directed to that value or parameter per se.

As used herein, the singular forms "a," "an," and "the" include plural referents unless the context clearly dictates otherwise. For example, "a" or "an" means "at least one" or "one or more."

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

As used herein, a composition refers to any mixture of two or more products, substances, or compounds, including cells. It may be a solution, a suspension, liquid, powder, a paste, aqueous, nonaqueous or any combination thereof.

As used herein, "enriching" when referring to one or more particular cell type or cell population, refers to increasing the number or percentage of the cell type or population, e.g., compared to the total number of cells in or volume of the composition, or relative to other cell types, such as by positive selection based on markers expressed by the population or cell, or by negative selection based on a marker not present on the cell population or cell to be depleted. The term does not require complete removal of other cells, cell type, or populations from the composition and does not require that the cells so enriched be present at or even near 100% in the enriched composition.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control or fluorescence minus one (FMO) gating control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control or fluorescence minus one (FMO) gating control under otherwise identical conditions, and/or at a level substantially lower than that for cell known to be positive for the marker, and/or at a level substantially similar as compared to that for a cell known to be negative for the marker.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

Unless defined otherwise, all terms of art, notations and other technical and scientific terms or terminology used herein are intended to have the same meaning as is commonly understood to which the claimed subject matter pertains. In some cases, terms with commonly understood meanings are defined herein for clarity and/or for ready reference, and the inclusion of such definitions herein should not necessarily be construed to represent a substantial difference over what is generally understood.

All publications, including patent documents, scientific articles and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### IX. EXAMPLES

The following examples are included for illustrative purposes only and are not intended to limit the scope of the invention.

### X. EXAMPLES

The following examples are included for illustrative purposes only and are not intended to limit the scope of the invention.

### Example 1 Administration of Anti-CD19 CAR-Expressing Cells to Subjects with Relapsed and Refractory Non-Hodgkin's Lymphoma (NHL)

### A. Subjects and Treatment

Therapeutic CAR⁺ T cell compositions containing autologous T cells expressing a chimeric antigen-receptor (CAR) specific for CD19 were administered to subjects with B cell malignancies.

### Example 1.A.1

Results are described in this Example 1.A.1 for evaluation through a particular time-point (1.A.1) in an ongoing clinical study administering such therapy to patients with B cell Malignancies. Specifically, a cohort (full cohort) of (at this time-point, fifty-five (55)) adult human subjects with relapsed or refractory (R/R) aggressive non-Hodgkin's lymphoma (NHL), including diffuse large B-cell lymphoma (DLBCL), de novo or transformed from indolent lymphoma (NOS), high-grade B-cell lymphoma, with MYC and BCL2 and/or BCL6 rearrangements with DLBCL histology (double/triple hit), DLBCL transformed from chronic lymphocytic leukemia (CLL) or marginal zone lymphomas (MZL), primary mediastinal large b-cell lymphoma (PMBCL), and follicular lymphoma grade 3b (FL3B) after failure of 2 lines of therapy. Among the subjects treated were those having Eastern Cooperative Oncology Group (ECOG) scores of between 0 and 2 (median follow-up 3.2 months). Subjects with MCL after 1 line of therapy also were included in the study (see Example 2), however, the full cohort did not include subjects with mantle cell lymphoma (MCL). No subjects were excluded based on prior allogeneic stem cell transplantation (SCT), secondary central nervous system (CNS) involvement or an ECOG score of 2, and there was no minimum absolute lymphocyte count (ALC) for apheresis required.

Outcomes were separately assessed for a core subset of subjects within the full cohort (subjects within the full cohort excluding those subjects with a poor performance status (ECOG 2), DLBCL transformed from marginal zone lymphomas (MZL) and/or chronic lymphocytic leukemia (CLL, Richter's), and subjects with primary mediastinal large b-cell lymphoma (PMBCL), and follicular lymphoma grade 3b (FL3B) (core cohort)). The core cohort includes subjects with DLBCL, NOS and transformed follicular lymphoma (tFL) or high grade B-cell lymphoma (double/triple hit) or high-grade B-cell lymphoma, with MYC and BCL2 and/or BCL6 rearrangements with DLBCL histology (double/triple hit) and with Eastern Cooperative Oncology Group performance status (ECOG PS) of 0 or 1. At the timepoint in Example 1.A.1, outcomes for 44 subjects within this core cohort were assessed.

The demographics and baseline characteristics of the full and core cohort subjects at the timepoint in Example 1.A.1 are set forth in **Table E1.**

| **Table E1. Demographics and Baseline Characteristics** | | | |
|---|---|---|---|
| **Characteristic** | | **FULL N=55** | **CORE N=44** |
| Median Age, years (range) | | 61 (29-82) | 61 (29-82) |
| | ≥ 65 years, n (%) | 22 (40) | 17 (39) |
| Male/Female, n (%) | | 38/17 (69/31) | 28/16 (64/36) |
| Months from diagnosis, median (range) | | 17 (3-259) | 20 (8-259) |
| B-NHL Subtype, n (%) | | | |
| DLBCL, NOS | | 40 (73) | 35 (80) |
| Transformed DLBCL | | 14 (26) | 8 (18) |
| Follicular, Grade 3B | | 1 (2) | 1 (2) |
| Molecular Subtype, n (%) | | | |
| Double/triple hit | | 15 (27) | 12 (27) |
| Double expressor | | 6 (11) | 4 (9) |
| Patient Characteristics, n (%) | | | |
| Chemorefractory^{†} | | 42 (76) | 34 (77) |
| ECOG 0-1 | | 48 (87) | 44 (100) |
| ECOG 2 | | 7 (13) | 0 |
| Prior lines of therapy. median (range) | | 3 (1-11) | 3 (1-8) |
| | < 5 lines of therapy | 44 (80) | 37 (84) |
| Any HSCT | | 27 (49) | 22 (50) |
| | Allogeneic | 4 (7) | 3 (7) |
| | Autologous | 24 (44) | 20 (45) |

*SD or PD to last chemo-containing regimen or relapse <12 months after autologous SCT

The therapeutic T cell compositions administered had been generated by a process including immunoaffinity-based (e.g., immunomagnetic selection) enrichment of CD4⁺ and CD8⁺ cells from leukapheresis samples from the individual subjects to be treated. Isolated CD4⁺ and CD8⁺ T cells were separately activated and independently transduced with a viral vector (e.g., lentiviral vector) encoding an anti-CD19 CAR, followed by separate expansion and cryopreservation of the engineered cell populations in a low-volume. The CAR contained an anti-CD19 scFv derived from a murine antibody (variable region derived from FMC63, V_{L}-linker-V_{H} orientation), an immunoglobulin-derived spacer, a transmembrane domain derived from CD28, a costimulatory region derived from 4-1BB, and a CD3-zeta intracellular signaling domain. The viral vector further contained sequences encoding a truncated receptor, which served as a surrogate marker for CAR expression; separated from the CAR sequence by a T2A ribosome skip sequence.

The cryopreserved cell compositions were thawed prior to intravenous administration. The therapeutic T cell dose was administered as a defined cell composition by administering a formulated CD4⁺ CAR⁺ cell population and a formulated CD8⁺ CAR⁺ population administered at a target ratio of approximately 1:1. Subjects were administered a single or double dose of CAR-expressing T cells (each single dose via separate infusions of CD4⁺ CAR-expressing T cells and CD8⁺ CAR-expressing T cells, respectively) as follows: a single dose of dose level 1 (DL-1) containing 5 x 10⁷ total CAR-expressing T cells (n=30 for subjects assessed in Example 1.A.1), a double dose of DL1 in which each dose was administered approximately fourteen (14) days apart (n=6 for subjects assessed in Example 1.A.1, administered on day 1 and day 14, including one subject that inadvertently received two DL2 doses via the two-dose schedule, due to a dosing error), or a single dose of dose level 2 (DL-2) containing 1 x 10⁸ total CAR-expressing T cells (n=18 for subjects assessed in Example 1.A.1). The target dose level and the numbers of T cell subsets for the administered compositions are set forth in **Table E2**.

| **Table E2. Target dose levels and number of T cell subsets for cell compositions containing anti-CD19 CAR T cells** | | | |
|---|---|---|---|
| **Dose level** | **Helper T cell (T_{H}) Dose (CD4⁺CAR⁺)** | **Cytotoxic T Cell (T_{C}) Dose (CD8⁺CAR⁺)** | **Total T Cell Dose (CD3⁺ CAR⁺)** |
| 1 | 25 x 10⁶ | 25 x 10⁶ | 50 x 10⁶ |
| 2 | 50 x 10⁶ | 50 x 10⁶ | 100 x 10⁶ |

Beginning at prior to CAR⁺ T cell infusion, subjects received a lymphodepleting chemotherapy with fludarabine (flu, 30 mg/m²) and cyclophosphamide (Cy, 300mg/m²) for three (3) days. The subjects received CAR-expressing T cells 2-7 days after lymphodepletion.

### Example 1.A.2

For Example 1.A.2, at a subsequent point in time in the clinical study described in this Example 1 above, results were analyzed. At this analysis time point in Example 1.A.2, 74 patients had been treated (51 male, 23 female). The subjects included sixty-nine (69) subjects in the full DLBCL cohort (including 67 DLBCL NOS (45 de novo, 14 transformed from FL, 8 transformed from CLL or MZL), 1 FL grade 3B, and 1 PMBCL), and 5 subjects in the MCL cohort. Among subjects in the full (DLBCL) cohort, median age was 61 years (range 26, 82), median prior therapies was 3 (range 1, 12), 46 (67%) were chemorefractory, 32 (46%) had any prior transplant, and at least 16 (23%) patients had double/triple hit lymphoma. Forty-nine (49) subjects in the core cohort were assessed at this timepoint in 1.A.2.

### B. Safety

The presence or absence of treatment-emergent adverse events (TEAE) following administration of the CAR-T cell therapy was assessed. Subjects also were assessed and monitored for neurotoxicity ( neurological complications including symptoms of confusion, aphasia, encephalopathy, myoclonus seizures, convulsions, lethargy, and/or altered mental status), graded on a 1-5 scale, according to the National Cancer Institute-Common Toxicity Criteria (CTCAE) scale, version 4.03 (NCI-CTCAE v4.03). Common Toxicity Criteria (CTCAE) scale, version 4.03 (NCI-CTCAE v4.03). See Common Terminology for Adverse Events (CTCAE) Version 4, U.S. Department of Health and Human Services, Published: May 28, 2009 (v4.03: June 14, 2010); and Guido Cavaletti & Paola Marmiroli Nature Reviews Neurology 6, 657-666 (December 2010). Cytokine release syndrome (CRS) also was determined and monitored, graded based on severity. See Lee et al, Blood. 2014;124(2):188-95. In some cases, adverse events data were reported and collected starting at lymphodepletion to 90 days after CAR⁺ T cell administration.

### Example 1.B.1

### Example 1.B.1 describes results based on the analysis time-point in Example 1.A.1.

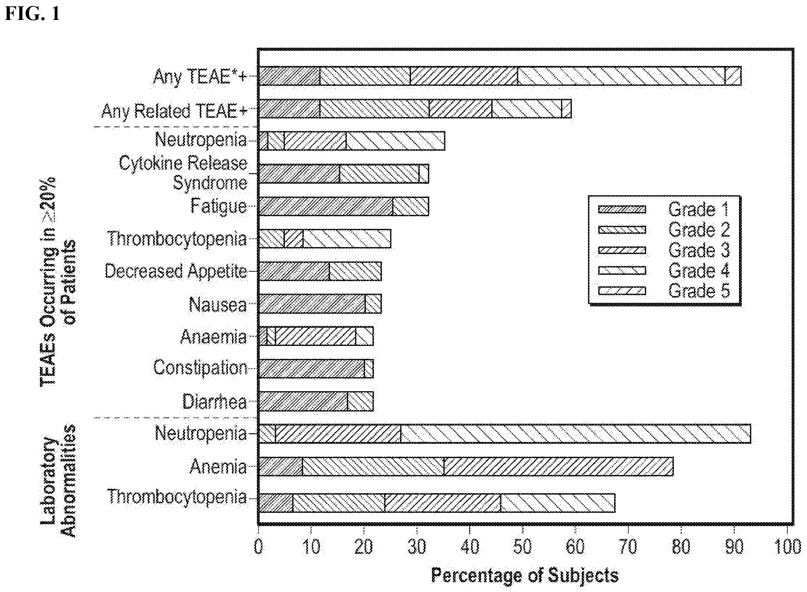

**FIG. 1** depicts the percentage of such subjects who were observed to have experienced laboratory abnormalities and TEAEs, which occurred in ≥20% of subjects. In addition to the TEAEs shown in **FIG. 1****,** the following event terms were observed at Grade 3-4 in ≥5% of patients: white blood cell count decreased (13.6%), encephalopathy (12%), hypertension (7%). Degree of toxicities observed were consistent between dose levels 1 and 2.

In 84% of the full cohort subjects in Example 1.B.1 analysis, severe (grade 3 or higher) cytokine release syndrome (CRS) and severe neurotoxicity were not observed. Additionally, it was observed that 60% of the full cohort subjects did not develop any grade of CRS or neurotoxicity. No differences in incidence of CRS, neurotoxicity (NT), sCRS, or severe neurotoxicity (sNT) were observed between dose levels. **Table E3** summarizes the incidence of cytokine release syndrome (CRS) and neurotoxicity adverse events in patients 28 days after receiving at least one dose of CAR-T cells. As shown in **Table E3,** no sCRS (Grade 3-4) was observed in any subjects that received a single dose of DL2 or double dose of DL1. Severe neurotoxicity or severe CRS (grade 3-4) was observed in 16% (9/55) of the full cohort of subjects and in 18% (8/44) of the subjects in the core subset. 11% (n=6) of subjects received tocilizumab, 24% (n=13) of subjects received dexamethasone. Among the ECOG2 subjects within the full cohort, observed rates of CRS and neurotoxicity were 71% and 29%, respectively.

| **Table E3. Assessment of Presence or Absence of CRS and Neurotoxicity Adverse Events for Example 1.B.1** | | | | | | |
|---|---|---|---|---|---|---|
| | | **FULL** | | | | **CORE** |
| | | **All Dose Levels** | **DL1S** | **DL2S** | **DL1D^{†}** | |
| Safety, N | | 55 | 30 | 19 | 6 | 44 |
| | sCRS or sNT, n (%) | 9 (16) | 6 (20) | 2 (11) | 1 (17) | 8 (18) |
| | CRS or NT. n (%) | 22 (40) | 12 (40) | 7 (37) | 3 (50) | 15 (34) |

| CRS | | | | | | |
|---|---|---|---|---|---|---|
| | Grade 1-2, n (%) | 18 (33) | 10 (33) | 5 (26) | 3 (50) | 12 (27) |
| | Grade 3-4, n (%) | 1 (2) | 1 (3) | 0 | 0 | 1 (2) |

| Neurotoxicity | | | | | | |
|---|---|---|---|---|---|---|
| | Grade 1-2, n (%) | 3 (6) | 1 (3) | 2 (11) | 0 | 2 (5) |
| | Grade 3-4, n (%) | 9 (16) | 6 (20) | 2 (11) | 1 (17) | 8 (18) |

| | | | | | | |
|---|---|---|---|---|---|---|
| Includes one patient treated at DL2 2-dose schedule due to dosing error | | | | | | |

**FIG. 2** shows a Kaplan meier curve depicting observed time to onset of CRS and/or neurotoxicity for the analysis in 1.B.1. As shown, the observed median times to onset of CRS and to onset of neurotoxicity were 5 and 11 days, respectively, with only 11% of patients experiencing onset of CRS less than 72 hours after initiation of the administration of the cell therapy. The median time to resolution of CRS and neurotoxicity to Grade 1 or better was 5 and 7 days, respectively. The median time to complete resolution of CRS and neurotoxicity was 5 and 11 days, respectively. The results were consistent with a conclusion that there was a low rate of early onset of any CRS or neurotoxicity in the subjects.

### Example 1.B.2

Example 1.B.2 describes assessment at the time-point in Example 1.A.2. Up to this time point, adverse event (AE) data were collected from lymphodepletion (LD) to 90 days post administration of CAR-expressing T cells. At the second time point, 69 subjects in the DLBCL cohort (full cohort) were evaluated for safety, 38 that had received DL1 single dose, 25 having received DL2 single dose, and 6 having received DL1 double dose schedule. The most common TEAEs other than CRS or NT included neutropenia (41%, 28/69), fatigue (30%, 21/69), thrombocytopenia (30%, 21/69), and anemia (26%, 18/69). One Grade 5 TEAE of diffuse alveolar damage was observed.

No acute infusional toxicity was observed, and the majority of subjects in the full cohort, 64% (44/69), were observed to have no CRS or NT, indicating that outpatient delivery of CAR-expressing T cells may be possible. Rates of CAR T cell-associated toxicities, including CRS and NT, did not differ between dose levels. Safety profile was observed to be similar across cohorts and dose levels. Among the 25 subjects in the full cohort (36%) who experienced any grade CRS or NT, 21 (30%) had CRS and 14 (20%) had NT. No subjects had Grade 3 CRS and only one (1%, 1/69) had Grade 4 CRS and required ICU care; the other 29% (20/69) had Grade 1-2 CRS. Of the 20% of subjects with NT, 6% (4/69) had Grade 1-2 and 14% (10/69) had Grade 3-4; 2 (3%) had seizure. No Grade 5 CRS or grade 5 NT was observed. No incidences of cerebral edema were observed. All CRS and NT events were resolved except one case of Grade 1 tremor, which was ongoing at the time of analysis. Median time to onset of first CRS and NT was 5 days (range 2, 12) and 10 days (range 5, 23), respectively. In the first 72 hours post infusion, no subjects were observed to have NT, and only 10% (7/69) were observed to have CRS (all Grade 1); NT was preceded by CRS in >70% of subjects. Overall, thirteen (13) subjects (19%) required intervention for CRS or NT with anti-cytokine therapy (tocilizumab alone 1 (1%), dexamethasone alone 6 (9%), or both 6 (9%)) and only one required any vasopressor support. Median doses of tocilizumab and dexamethasone were 1 and 6, respectively. Median CRS and NT duration was 5 days and 11 days, respectively. Analysis of the core cohort (n=49) also showed similar rates of CRS and NT.

In this assessment, low incidences and late onsets of CRS and/or NT were observed, at both dose levels, supported the feasibility of outpatient infusion, such as with hospital admission at the first sign of fever or fever lasting beyond a certain period of time. No Grade 5 CRS or grade 5 NT was observed, and all severe CRS and severe NT were resolved. Further, approximately 2 out of 3 patients had no CRS or NT, supporting that the cells can be administered on outpatient basis. At the time of assessment in 1.B.2, four subjects had been treated in the outpatient setting. Further, no meaningful differences in toxicity was observed in subjects receiving DL1 or DL2, indicating achievement of higher response rates without an increased risk of toxicity or safety concerns.

### C. Response Outcomes following Treatment

Subjects were monitored for response, including by assessing tumor burden at 1, 3, 6, 7, 12, 18, and 24 months after administration of the CAR⁺ T cells.

### Example 1.C.1

Example 1.C.1 describes results based on the analysis time-point in Example 1.A.1 and 1.B.1.

Response rates are listed in **Table E4.** High durable response rates were observed in the cohort of subjects, which included subjects heavily pretreated or, with poor prognosis and/or with relapsed or refractory disease. For subjects across all doses in the Core (n=44) cohort, the observed overall response rate (ORR) was 86% and the observed complete response (CR) rate was 59%. At three months for the core cohort, the overall response rate (ORR) was 66%; the three-month CR rate was 50% among the core cohort. In the core cohort, the 3 month ORR was 58% (11/19) at dose level 1 and 78% at dose level 2; the 3 month CR rate was 42% (8/19) for dose level 1 and 56% (5/9) for dose level 2, consistent with a suggested dose response effect on treatment outcome. Additionally, the results were consistent with a relationship between dose and durability of response.

| **Table E4. Response** | | | | | | | | |
|---|---|---|---|---|---|---|---|---|
| | **FULL** | | | | **CORE** | | | |
| | **All Dose Levels** | **DL1S** | **DL2S** | **DL1D^{c}** | **All Dose Levels** | **DL1S** | **DL2S** | **DL1D^{a}** |
| Best Overall Response, N^{a} | 54 | 30 | 18 | 6 | 44 | 25 | 15 | 4 |
| ORR, % (95% CI) | 76 (62, 87) | 80 (61, 92) | 72 (47, 90) | 67 (23, 96) | 86 (73, 95) | 84 (64, 95) | 87 (60, 98) | 100 (40, 100) |
| CR,% (95% CI) | 52 (38, 66) | 53 (34, 72) | 50 (26, 74) | 50 (12, 88) | 59 (43, 74) | 56 (35, 76) | 60 (32, 84) | 75 (19, 99) |
| ≥ 3 mos f/u, n^{b} | 41 | 24 | 11 | 6 | 32 | 19 | 9 | 4 |
| 3 mo ORR,% (95% CI) | 51 (35, 67) | 46 (26, 67) | 64 (31, 89) | 50 (12, 88) | 66 (47, 81) | 58 (34, 80) | 78 (40, 97) | 75 (19, 99) |
| 3 mo CR,% (95% CI) | 39 (24, 56) | 33 (16, 55) | 46 (17, 77) | 50 (12, 88) | 50 (32, 68) | 42 (20, 67) | 56 (21, 86) | 75 (19, 99) |

| | | | | | | | | |
|---|---|---|---|---|---|---|---|---|
| DL1S: DL1 1-dose schedule; DL2S: DL2 1-dose schedule; DL1D: DL1 2-dose schedule; ^{a}Included patients with event of PD, death, or 28 day restaging scans. Treated patients 28 days prior to data snapshot were not included. ^{b}The denominator is number of patients who received the CAR T-cell therapy ≥ 3 months ago, prior to data snapshot date assessment at Month 3 or prior assessment of PD or death. ^{c} Includes one patient treated at DL2 2-dose schedule due to dosing error | | | | | | | | |

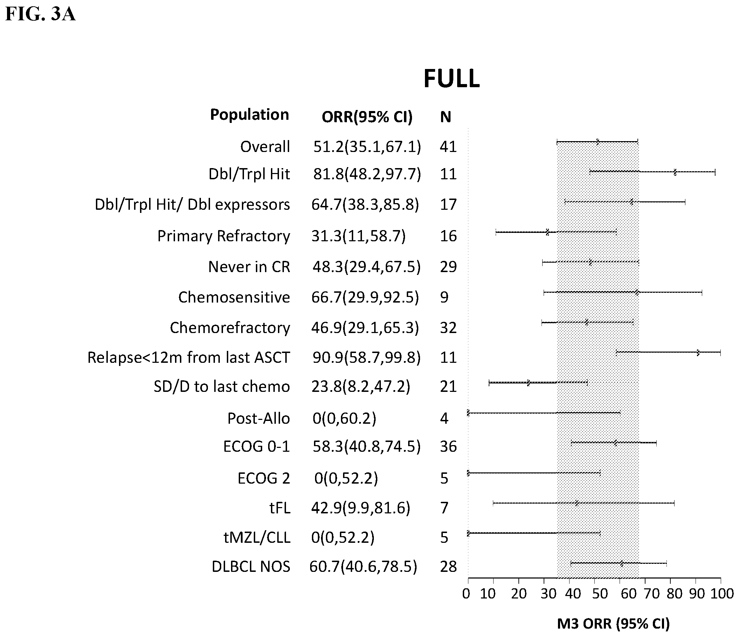

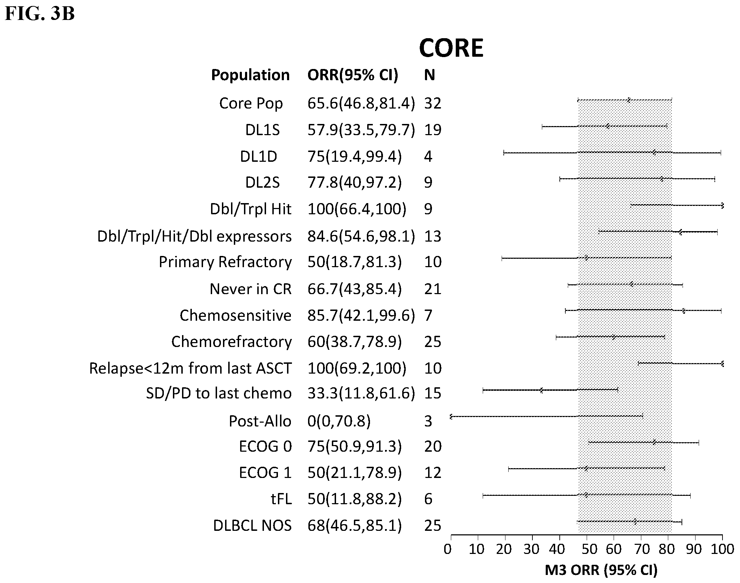

Overall response rates among various subgroups of subjects in the full and core cohorts are shown in **FIG. 3A** and **3B****,** respectively. In poor-risk DLBCL subgroups, response rates were generally high. An ORR of greater than 50% was observed at 3 months in patients with double/triple hit molecular subtype, that had primary refractory or chemorefractory DLBCL or that never before had achieved a CR. Complete resolution of CNS involvement by lymphoma was observed in 2 patients.

Among the subjects treated six months or greater prior to the particular time-point of the evaluation, of the ten (10) patients that had been in response at three months, 9 (90%) remained in response at six months. At the evaluation time-point, 97% of subjects in the core subset who had responded were alive and in follow-up, median follow-up time 3.2 months.

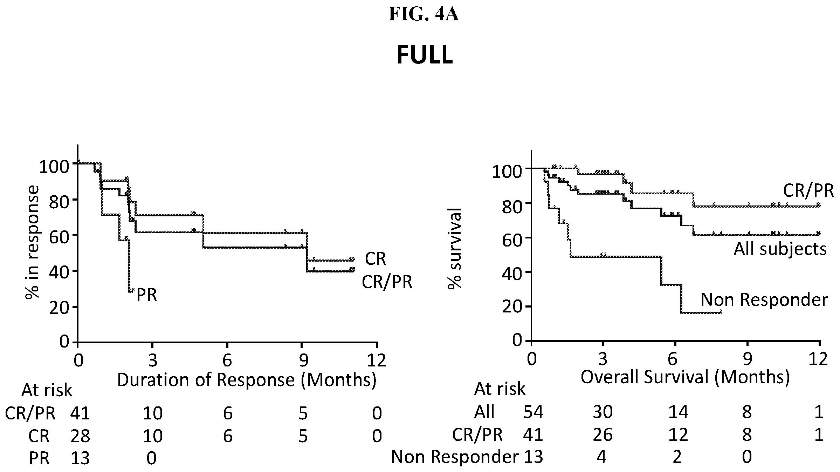

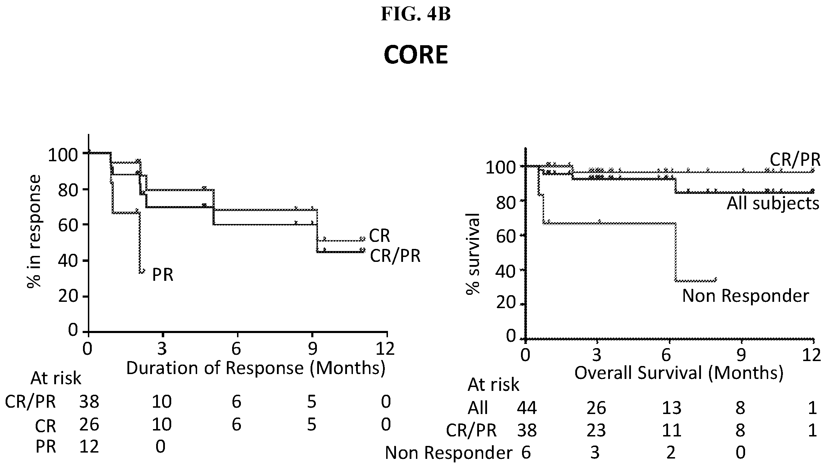

Results for the duration of response and overall survival (grouped by best overall response (non-responder, CR/PR, CR and/or PR)) are shown for full and core cohorts of subjects, in **FIGS. 4A** and **4B****,** respectively. As shown, prolonged survival was observed in responders, with increased durability of response in subjects with CRs. All patients in response at three months remained alive at the time of evaluation, although 5/6 subjects with poor performance status (ECOG 2) had expired.

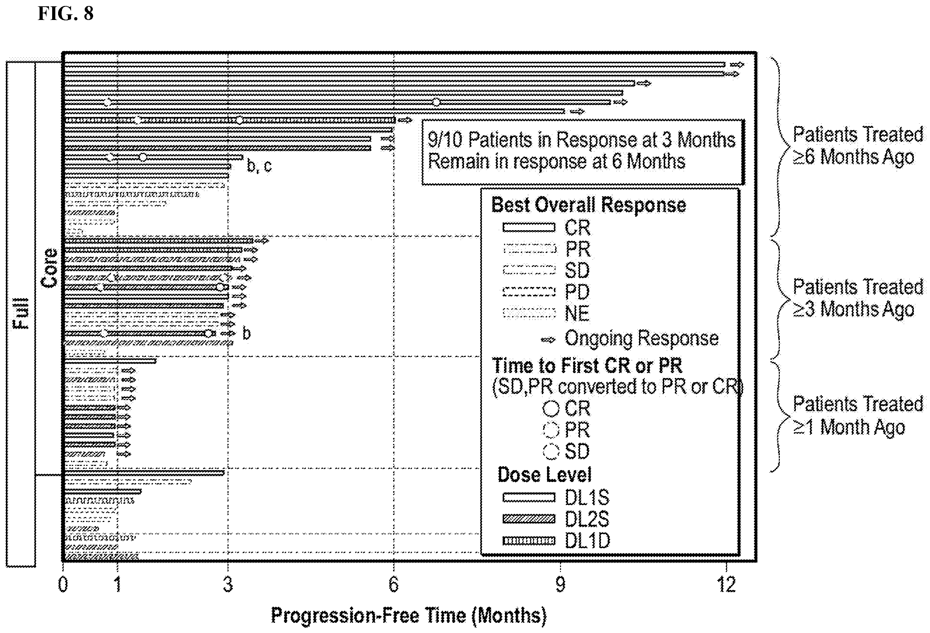

**FIG. 8** shows a graph plotting progression-free time (months) for individual subjects within the full and core cohorts. Each bar represents a single patient. Shading indicates best overall response (in each case, unless otherwise indicated, achieved at 1 month); texture indicates dose (solid=dose level 1, single dose; cross-hatched, dose-level 2, single dose; vertical hatched=dose level 1, two-dose). Horizontal arrows indicate an ongoing response. Certain individual subjects were initially assessed (e.g., at 1-month) as exhibiting stable disease (SD) or Partial Response (PR), and were later observed to have achieved a PR (e.g., conversion of SD to PR) or CR. In such cases, shading of the individual patient bar, as noted, indicates best overall response, and dots (same correspondence of shading to response achieved) along each individual subject bar, indicate when each SD, PR, and/or CR was observed to have occurred in the subject.

Complete resolution of CNS involvement by lymphoma was observed in two patients. CAR⁺ cells in one subject were observed to have expanded following biopsy after relapse. The subject who exhibited expansion following biopsy had chemorefractory transformed DLBCL (germinal center subtype with a BCL2 rearrangement and multiple copies of *MYC* and *BCL6*). The subject had been administered the CAR⁺ T cells at DL-1 and the numbers of CD3⁺/CAR⁺, CD4⁺/CAR⁺, CD8⁺/CAR⁺ T cells in peripheral blood were measured at certain time points, are shown in **FIG. 6A****.** The subject had previously been treated with, and was refractory to, five prior lines of therapy including dose-adjusted etoposide, doxorubicin, and cyclophosphamide with vincristine and prednisone plus rituximab (DA-EPOCH-R) and intermediate-intensity allogeneic stem-cell transplantation from an 8/8 HLA-matched unrelated donor. Following allogeneic stem cell transplantation and prior to receiving CAR⁺ T cells, the subject showed 100% donor chimerism in all blood lineages, had ceased taking immunosuppressive therapy, and did not have graft versus host disease (GVHD). Prior to administration of CAR⁺ T cells, the subject had a periauricular mass and temporal lobe lesion observed by positron-emission tomography and computed tomography (PET-CT) (**FIG. 6B**) and confirmed by magnetic resonance imaging (MRI) (**FIG. 6D**).

After receiving anti-CD19 CAR-T cell treatment, the subject achieved CR 28 days post-infusion, as shown by PET-CT (**FIG. 6C**) and brain MRI (**FIG. 6E**), with no observed signs of neurotoxicity or CRS. Three months post-infusion of the CAR-T cells, relapse of the periauricular mass was noted in this subject (**FIG. 6F**), and an incisional biopsy was performed. As shown in **FIG. 6A****,** following biopsy, the visible tumor receded with no further therapy. Pharmacokinetic analysis showed a marked re-expansion of the CAR⁺ T cells in peripheral blood (to a level higher than initial expansion observed, with peak levels observed at about 113 days post-infusion) i, which coincided with tumor regression. The subject then went on to achieve a second CR, as confirmed by restaging PET-CT one month following the biopsy (**FIG. 6G**), and remained in CR at 6 months post CAR-T cell infusion. Further assessment of the subject showed that the CNS response was durable and the subject remained in CR at 12 months.

The results in the subject having received the biopsy followed by observed re-expansion of CAR⁺ T cells are consistent with a conclusion that re-expansion and activation of CAR⁺T cells can be initiated *in vivo* following reduction or loss of functional or active CAR⁺ T cells and/or relapse following anti-tumor response to CAR-T cell therapy. Further, following re-expansion *in vivo* late after initial CAR⁺ T cell infusion, the CAR⁺ T cells are able to re-exert anti-tumor activity. This result supports that CAR⁺ T cell re-expansion and activation can be triggered *in vivo* and that methods of reactivating CAR⁺ T cells, may further augment their efficacy.

The complete responses in the two DLBCL subjects with CNS involvement were observed without development of any grade of neurotoxicity. These results are consistent with the observation that CAR⁺ T cells of embodiments provided herein are capable of readily accessing the CNS and exerting effector function to reduce or eliminate CNS tumors, without increasing or without substantially increasing risk of toxicity such as neurotoxicity. In other studies, among subjects having ALL treated with anti-CD19 CAR T cells, no clear correlation has been observed between incidence of neurotoxicity and the presence of CNS leukemia in the brain (which has been observed to respond to such CAR T cell therapy). Thus, whereas neurotoxicity can occur in some contexts following treatment with CAR-T therapies, such neurotoxicity may not necessarily be the result of target expression in the brain or activity of the CAR T cells in the CNS, and may not result from "on-target" toxicity by the CAR⁺ T cells.

### Example 1.C.2

Example 1.C.2 describes results based on the analysis time-point in Example 1.A.2 and 1.B.2.

Up to the time point in Example 1.C.2, 68 subjects in the full DLBCL cohort was evaluated for response. Overall or objective response (OR), 3-month, and 6-month objective response rates were 75% (51/68), 49% (27/55), and 40% (14/35), respectively. Complete response (CR) rate, 3-month CR rate, and 6-month CR rate were 56% (38/68), 40% (22/55), and 37% (13/35), respectively. A trend toward improved response rate at 3 months was observed in subjects treated at DL2 compared to DL1: 63% (12/19; 95% CI 38, 84) vs 40% (12/30; 95% CI 23, 59) for ORR with p=0.148, and 58% (11/19; 95% CI 34, 80) vs 27% (8/30; 95% CI: 12, 46) for CR with p=0.0385. Among 16 double/triple hit lymphoma subjects, ORR was 81%, and 3-month CR rate was 60%.

In the core cohort (n=49 for the time-point in Example 1.C.2), OR, 3-month, and 6-month OR rates were 84% (41/49), 65% (26/40), and 57% (13/23), respectively. CR rate, 3-month CR rate, and 6-month CR rate were 61% (30/49), 53% (21/40), and 52% (12/23), respectively. A similar trend in improved durable ORR and CR at 3 months at higher doses was observed. Specifically, for patients in the CORE cohort administered DL2, 3-month ORR was 80% (12/15; 95% CI 52, 96) and 3-month CR was 73% (11/15; 95% CI 45, 92), compared to 3-month ORR and CR rates of 52% (11/21; 95% CI 30, 74) and 33% (7/21; 95% CI 15, 57) in CORE cohort subjects administered DL1, with p=0.159 and p=0.0409 respectively. Among subjects in the CORE cohort having received DL2 and with 3-month follow-up (n=15), 3-month ORR was 80% and 3-month CR was 73%.

Median DOR in the full cohort and core cohorts at this time-point in 1.C.2 was 5.0 and 9.2 months, respectively; median duration of CR was 9.2 months in the full cohort. Median duration of CR had not been reached in the core cohort. Median overall survival (OS) was 13.7 months in the full cohort and had not been reached in the core cohort. 6-month OS was 75% in the full cohort, with median follow-up of 5.8 months. 6-month OS was 88% in the core cohort, with median follow up of 5.6 months.

### D. Assessment of CAR⁺ T cells in Blood

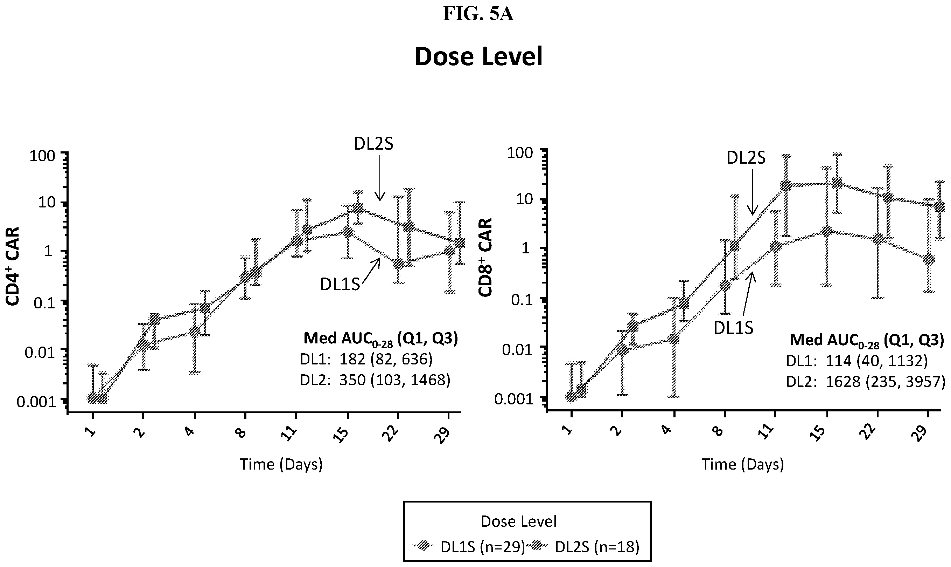

Based on data from the time-point described in Example 1.A.1, 1.B.1 and 1.C.1, pharmacokinetic analysis was carried out to assess numbers of CAR⁺ T cells in peripheral blood at various time points post-treatment. Results from the fifty-five (55) subjects assessed at the time-point in Example 1.A.1 in the DLBCL cohort and four (4) subjects (assessed at that same time-point) in the mantle cell lymphoma (MCL) cohort, as described in Example 2 below were analyzed. Pharmacokinetics (PK) measurements were carried out using validated flow cytometry to detect a marker expressed in the CAR construct and quantitative PCR-based assays to detect the integration of the CAR construct. B cell aplasia was assessed by flow cytometry using anti-CD19 antibodies. As shown in **FIG. 5A****,** CD4⁺ and CD8⁺ CAR-expressing cells, as measured by the number of cells/µL blood (median ± quartiles) plotted on a log scale, were detected throughout the course of assessment at both administered dose levels. Subjects receiving DL2 relative to DL1 had higher median Cₘₐₓ and median AUC₀₋₂₈ for CD3⁺/CAR⁺, CD4⁺/CAR⁺, and CD8⁺/CAR⁺ T cell subsets in peripheral blood (AUC₀₋₂₈: DL2 vs. DL1 was 1836 vs. 461, 350 vs. 182, and 1628 vs. 114, for CD3⁺, CD4⁺, and CD8⁺, respectively; p < 0.05 for CD8⁺; Cₘₐₓ: DL2 vs. DL1 was 99.8 vs. 27.9, 15.1 vs. 5.2, and 73.1 vs. 5.5 cells/µL, respectively). Median time to maximum CD3⁺ CAR⁺ T cell expansion was 15 days (range 8-29) and did not differ between dose levels. CD4⁺ and CD8⁺ CAR-expressing T cells homed to the bone marrow at relatively similar levels.

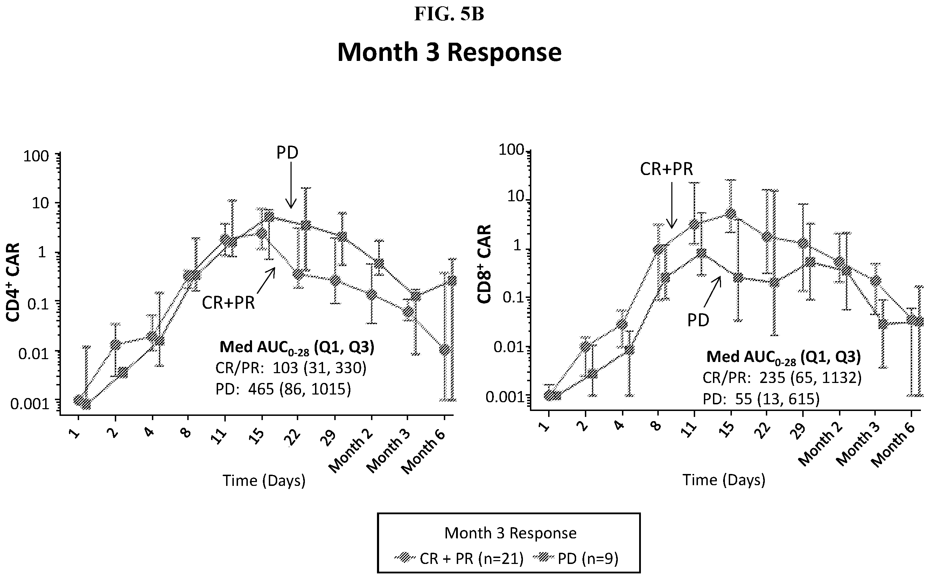

An increased median area under the curve (AUC) (CD8⁺ CAR⁺ T cell numbers over time in the blood) was observed among subjects administered the higher dose level, as compared to the lower dose level, without an observed increase in toxicity. Higher peak CD8⁺/CAR⁺ T cell exposure was observed in responders (CR/PR) than non-responders (PD); persistence of cells over the time of assessment, including out to 3 and 6 months, was observed even in subjects whose disease had progressed **(****FIG. 5B**). Median Cₘₐₓ and median AUC₀₋₂₈ of CD8⁺ CAR⁺ T cells were higher in responding subjects and with durable response at month 3 (CD8⁺ Cₘₐₓ median = 20.8 vs. 5.5; CD8⁺ AUC₀₋₂₈ median = 235 vs. 55 in CR/PR at Month 3 vs. PD at Month 3). Among subjects that were evaluated for CAR T cell persistence, 90% and 93% of 29 subjects had detectable CD8⁺ and CD4⁺ CAR⁺ T cells, respectively, at month 3; 63% and 58% of 19 subjects had detectable CD8⁺ and CD4⁺ CAR⁺ T cells, respectively, at month 6. At months 3 and 6, no statistically significant differences in the persistence of CAR⁺ T cells were observed between subjects with durable response or relapse. CAR⁺ T cells were detectable at time of relapse in 89% of 11 subjects with PK, even though B cell aplasia (<1 cell/µl) was demonstrated in nearly all subjects 97% (34/35) at month 3, and 100% (24/24) at month 6.

Higher Cₘₐₓ and AUC₀₋₂₈ at DL2 as compared to DL1 was not observed to be associated with increased CRS or NT. For any NT or for > Grade 2 CRS, median AUCs of CD4⁺/CAR⁺ and CD8⁺/CAR⁺ T cells were 5 to 10 fold and 3 to 5 fold higher, respectively, than the median AUC for DL2. Higher disease burden and baseline levels of inflammatory cytokines was observed to be associated with higher peak levels of CAR⁺ T cells, higher cytokine peak levels, and higher incidences of CRS and NT. The results were consistent with a conclusion that the higher Cmax and median AUC₀₋₂₈ at DL2 did not increase CRS or NT.

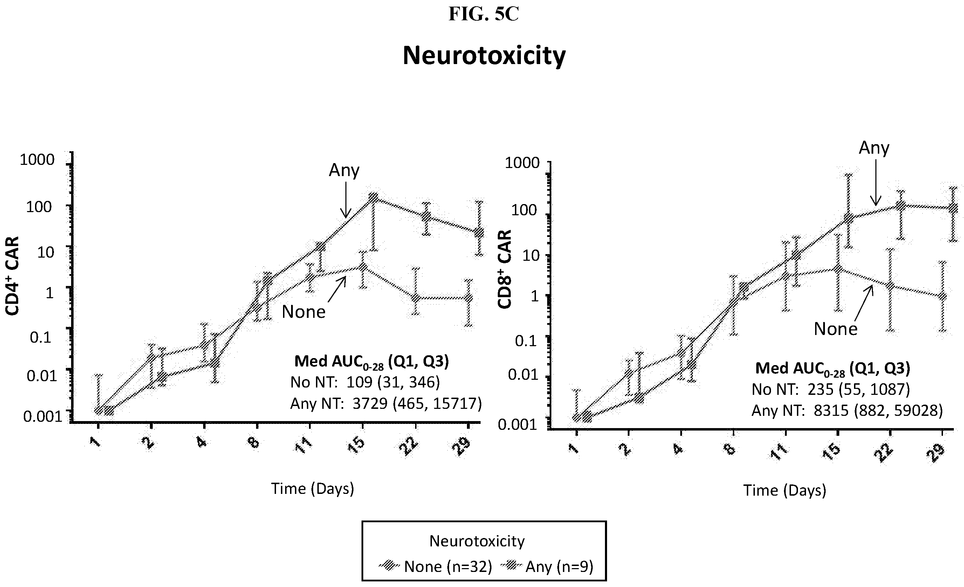

The results were consistent with a conclusion that treatment resulted in prolonged exposure and persistence of the engineered cells, even in subjects with poor responses. In some embodiments, combination approaches are used, such as administration of an immune checkpoint modulator or other immune modulatory agent, e.g., following relapse or disease progression, at a time at which engineered cells persist in the subject, e.g., as measured by levels of cells in peripheral blood. In some aspects, the cells, having persisted for a prolonged period, re-expand or become activated and/or exhibit anti-tumor function, following administration of the other agent or treatment. Higher median CD4⁺ and CD8⁺ CAR⁺ T cell numbers were generally observed over time in blood of subjects who developed neurotoxicity (**FIG. 5C**). Results indicated that the CAR⁺ T cells exhibited expansion and persistence, durability of response at 3 months that increased at higher dose levels, without increased toxicity. Results were observed that were consistent with a suggestion that high peak levels of CAR⁺ T cells and cytokines in the blood may be associated with NT and CRS, and may be influenced by baseline subject factors. It was observed that CAR⁺ T cells were present at the time of relapse, indicating that combination or retreatment approaches may provide certain advantages.

### E. Blood Analytes and Neurotoxicity, CRS and Response

Various pre-treatment blood analytes, including cytokines, were measured in the serum of subjects (those assessed at the time-point in Example 1.A.1), prior to administration of the CAR⁺ T cells. Cytokines were measured using a multiplex cytokine assay. Potential correlations to risk of developing neurotoxicity were assessed using statistical analysis based on univariate nonparametric tests.

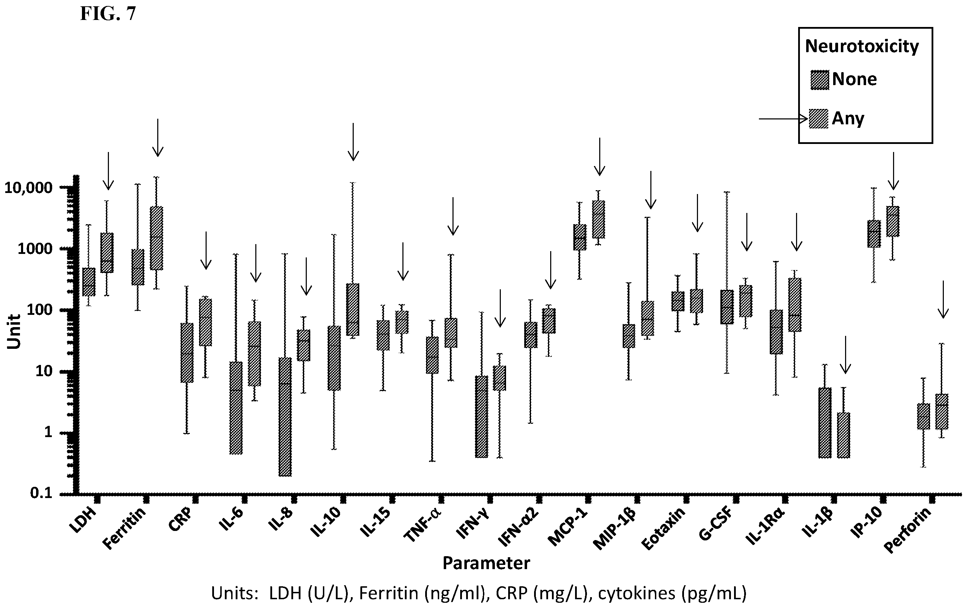

**FIG. 7** shows median levels of the assessed analytes in units (LDH, U/L; ferritin, ng/mL; CRP, mg/L; cytokines, pg/mL) in subjects that did not develop a neurotoxicity versus subjects that did develop a neurotoxicity following CAR⁺ T cell therapy. Levels of certain blood analytes, including LDH, Ferritin, CRP, IL-6, IL-8, IL-10, TNF-α, IFN-α2, MCP-1 and MIP-1β, were observed to be associated with level of risk of developing neurotoxicity (Wilcoxon p values <0.05,without multiplicity adjustment). In particular, the results were consistent with a conclusion that pre-treatment levels of LDH, which in some embodiments is a surrogate for disease burden, may be useful for potential neurotoxicity risk assessment and/or risk-adapted dosing or adjustment of treatment of certain subjects. In addition, tumor burden measured before administration of the CAR-T cell composition correlated (Spearman p values <0.05) with the risk of developing neurotoxicity. In some aspects, LDH levels may be assessed alone and/or in combination with another pre-treatment parameter, such as another measure or indicator of disease burden, such as a volumetric tumor measurement such as sum of product dimensions (SPD) or other CT-based or MRI-based volumetric measurement of disease burden. In some aspects, one or more parameters indicative of disease burden are assessed, and in some contexts may indicate the presence, absence or degree of risk of developing neurotoxicity following the T cell therapy. In some aspects, the one or more parameters include LDH and/or a volumetric tumor measurement.

In an additional analysis, fifty-five (55) subjects in the DLBCL cohort at the time-point in Example 1.A.1, and four (4) subjects in the mantle cell lymphoma (MCL) described in Example 2 below were included in analysis for correlation with safety evaluations. In the 59 subjects evaluated for safety, CRS was observed in 32% (30% Grade 1-2, 0% Grade 3, 2% Grade 4); NT was observed in 20% (5% Grade 1-2, 10% Grade 3, 5% Grade 4). Dose level did not correlate with CRS or NT (p=0.565 and p=1.00, respectively). Subject factors that correlate with any grade CRS and NT were poorer performance status (e.g. ECOG Status 2) (p=0.03) and higher disease burden (p <0.05) as measured by the sum of the products of diameters (SPD) based on imaging results. Pre-CAR⁺ T cell infusion clinical laboratory parameters and cytokine measurements for pre-CAR⁺ T cell infusion that were observed to be associated with the occurrence of any grade NT included higher serum LDH, ferritin, and CRP, and higher plasma IL-6, IL-8, IL-10, TNF-α, IFN-α2, MCP-1, and MIP-1β (p<0.05 for each). Higher pre-CAR⁺ T cell infusion plasma levels of IL-8, IL-10, and CXCL10 were also associated with Grade 3-4 NT (p<0.05 for each).

Of the 54 subjects in the DLBCL cohort that were evaluated for response, higher ECOG scores and DLBCL transformed from CLL or MZL correlated with lower durable response at month 3 (p=0.02 for both). Pre-CAR⁺ T cell infusion parameters associated with best ORR included lower values of ferritin, LDH, CXCL10, G-CSF, and IL-10, and those associated with durable response at 3 months included lower ferritin, CRP, LDH, CXCL10, IL-8, IL-10, IL-15, MCP-1, MIP-1β, TNF-α, and higher pre-CAR⁺ T infusion hemoglobin and albumin (p<0.05 for each).

In some cases, the apheresis sample and CAR⁺ T cell composition for administration was assessed and correlated with clinical outcomes. The results showed that T cell memory subsets and T cell functionality may correlate with certain clinical outcomes.

The results showed that certain baseline patient characteristics, including inflammatory state and high tumor burden prior to treatment, may be useful for the identification of patients at risk for increased toxicity following administration of CAR-expressing T cells. Low tumor burden and low inflammatory state were observed to be associated with improved toxicity profile and better durability of response. The results support that treating subjects earlier in the course of therapy and/or assessing a panel of clinical and laboratory biomarkers to risk stratify subjects for potential early intervention may mitigate the risk of toxicity and improve durability of response.

### Example 2 Administration of anti-CD19 CAR-Expressing Cells to Subjects with Mantle Cell Lymphoma (MCL)

Therapeutic CAR⁺ T cell compositions containing autologous CD4 and CD8 T cells expressing a chimeric antigen-receptor (CAR) specific for CD19, generated as described in Example 1, were administered to human subjects with mantle cell lymphoma (MCL) that had failed (relapsed/refractory, R/R) after ≥ 1 prior lines of therapy. Eligible patients had confirmed MCL (cyclin D1 expression or evidence of t[11;14]) with R/R disease after at least 1 prior line of therapy. No subjects were excluded based on prior allogeneic stem cell transplantation (SCT), secondary central nervous system (CNS) involvement, and there was no minimum absolute lymphocyte count (ALC) for apheresis required. Subjects also were not excluded based on an ECOG score of 2, which was later amended to also include subjects with an ECOG score of 0-1. Bridging chemotherapy during manufacturing of the T cell composition was allowed.

### Example 2.A

Therapeutic CAR⁺ T cell compositions containing autologous T cells expressing a chimeric antigen-receptor (CAR) specific for CD19, generated as described in Example 1, were administered to four (4) human subjects with mantle cell lymphoma (MCL) that had failed at least 1 line of therapy. The cryopreserved cell compositions were thawed prior to intravenous administration. The therapeutic T cell composition was administered as a defined composition cell product with formulated CD4⁺ and CD8⁺ populations of CAR⁺ engineered T cells derived from the same subject administered at a target ratio of approximately 1:1. Subjects were administered a dose of CAR-expressing T cells (as a split or separate dose of the CD4⁺ and CD8⁺ CAR-expressing T cells) at a single dose of dose level 1 (DL1) containing 5 x 10⁷ CAR-expressing T cells. Beginning at three (3) days prior to CAR⁺ T cell infusion, subjects received a lymphodepleting chemotherapy with fludarabine (flu, 30 mg/m²) and cyclophosphamide (Cy, 300 mg/m²).

Subjects were monitored for response and toxicities as described in Example 1. No CRS or neurotoxicity was observed in any of the subjects. Of the 4 subjects that were treated, two (2) subjects achieved PR (not durable) and two (2) patients had progressive disease.

### Example 2.B

Safety and efficacy were assessed at a subsequent time-point in the clinical study described in Example 2.A for nine (9) human subjects with mantle cell lymphoma (MCL) that had failed at least 1 prior line of therapy. The median age was 66 years old (range 58-78) of which 7 subjects were male. Histologies included blastoid (n=3) and pleiomorphic (n=1) variants. Eight (8) subjects had documented Ki67 >30% (range 40%-80%), and 1 subject had a TP53 mutation. The subjects at this time-point had received a median of 5 (range 3-7) prior therapies, including 3 subjects that had received prior hematopoietic stem cell transplant. All 9 subjects had been previously treated with ibrutinib, of which 4 had a best response of progressive disease on ibrutinib. Six of the nine subjects (67%) received bridging chemotherapy.

After receiving lymphodepleting chemotherapy (LDC) as described in Example 2.A, subjects were administered a dose of CAR-expressing T cells (by separate administration of a dose of the CD4⁺ and CD8⁺ CAR-expressing T cells) at a single dose of 5 x 10⁷ CAR-expressing T cells (DL1, about 2.5 x 10⁷ CD4+CAR+ T cells and 2.5 x 10⁷ CD8⁺ CAR⁺ T cells, 6 subjects) generally as described in Example 1.A, or at a single dose of 1 x 10⁸ CAR-expressing T cells (DL2, about 5 x 10⁷ CD4+CAR+ T cells and 5 x 10⁷ CD8⁺ CAR⁺ T cells, 3 subjects).

Four (4) of the nine subjects (44%) had serious treatment-emergent adverse events (TEAEs), 5 of the nine subjects (56%) had grade (G) 3/4 TEAEs, primarily anemia, neutropenia, and hypophosphatemia (22% each), and 3 of the nine subjects (33%) had grade 1 (G1) cytokine release syndrome (CRS). Median time to CRS onset was 6 days (range 2-7) and median time to resolution was 6 days (range 2-6). One (1) subject received tocilizumab and corticosteroids. There were no neurological events. Four (4) subjects from the DL1 cohort died (3 from disease progression and 1 after receiving new anticancer therapy post administration of the dose of anti-CD19 CAR-expressing T cells).

The overall response rate was 78% (7 out of 9 subjects, including 4 out of the 6 subjects in the DL1 cohort (median follow-up of 12.4 months [95% CI: 9.2-12.4]) and all 3 subjects in the DL2 cohort (median follow-up of 1.4 months [95% CI: 1.0-1.4]). Two (2) subjects in the DL1 cohort maintained a durable CR until last follow-up (day 281 and 378, respectively).

The median time to peak CAR⁺ T cell expansion was 9.5 days (range 9-10) for DL1 and 17.5 days (10-27) for DL2.

Together, these data demonstrate that administration of the anti-CD19 CAR-T cell therapeutic T cell composition to subjects with R/R MCL, resulted in tolerable toxicity and clinical response.

### Example 2.C

Safety and response outcome were assessed at a subsequent time-point in the clinical study described in Example 2.A for seventeen (17) human subjects with R/R mantle cell lymphoma (MCL) that had failed ≥1 prior lines of therapy. Subjects were monitored for response and safety as described in Example 1, including by evaluating efficacy according to the Lugano criteria, and grade of CRS and NE by criteria of Lee et al. (2014) and CTCAE, version 4.03, respectively. Subjects were monitored for dose-limiting toxicities (DLTs) for 28 days, and for treatment-emergent adverse events (TEAEs) for 90 days after anti-CD19 CAR-T cell therapeutic T cell composition administration. The Kaplan-Meier method was used to determine duration of response (DOR), overall survival (OR), and progression-free survival (PFS).

Therapeutic T cell compositions were prepared from 25 subjects as described in Example 1 by immunoaffinity-based (e.g., immunomagnetic selection) enrichment of CD4⁺ and CD8⁺ cells from leukapheresis samples from the individual subjects to be treated. Isolated CD4⁺ and CD8⁺ T cells were separately activated and independently transduced with a viral vector (e.g., lentiviral vector) encoding the anti-CD19 CAR, followed by separate expansion and cryopreservation of the engineered cell populations in a low-volume. Prior to administration of the therapeutic T cell composition, treatment of 7 subjects was discontinued due to disease progression and death (n=3), no longer met inclusion criteria due to secondary malignancy (n=1) or achieved CR during intervening chemotherapy (n=3).

After receiving lymphodepleting chemotherapy (LDC) as described in Example 2.A, the remaining subjects were administered a dose of CAR-expressing T cells (by separate administration of a dose of the CD4⁺ and CD8⁺ CAR-expressing T cells) at a single dose of 5 x 10⁷ CAR-expressing T cells (DL1, about 2.5 x 10⁷ CD4+CAR+ T cells and 2.5 x 10⁷ CD8⁺ CAR⁺ T cells, 6 subjects) generally as described in Example 1.A, or at a single dose of 1 x 10⁸ CAR-expressing T cells (DL2, about 5 x 10⁷ CD4+CAR+ T cells and 5 x 10⁷ CD8⁺ CAR⁺ T cells, 11 subjects). One additional subject in dose level 1 (DL1) received a nonconforming CAR-expressing T cell product with reduced CD8⁺ CAR⁺ cell viability and demonstrated a high tumor proliferation index (Ki67 90%); this subject was not included in safety and efficacy analyses.

The demographics and subject characteristics of the subjects treated at this time-point are set forth in **Table E4.1.** At baseline, most subjects had received multiple lines of prior therapy, including ibrutinib, and the majority of subjects had poor risk factors including blastoid/pleiomorphic variants, TP53 mutation, or a Ki67 >30%. Ten of the seventeen subjects (59%) received bridging anticancer therapy.

| **Table E4.1 Subject Demographics and Clinical Characteristics at Baseline** | | | | |
|---|---|---|---|---|
| | | **DL1 (n=6)** | **DL2 (n=11)** | **All Subjects (N=17)** |
| Age, median (range), vears | | 67 (58-78) | 66 (53-80) | 66 (53-80) |
| Male, n | | 5 | 8 | 13 (76%) |
| SPD (cm²) prior to LDC; median (range) | | 48 (2-80) | 41 (0-210)^{a} | 41 (0-210) |
| LDH > 500 IU/mL (pre-LDC), n | | 3 | 1 | 4 (24%) |
| Prior therapies; median (range) | | 4 (3-6) | 4 (1-8) | 4 (1-8) |
| Prior HSCT,^{b} n | | 2 | 4 | 6 (35%) |
| Prior ibrutinib, n | | 6 | 10 | 16 (94%) |
| | Refractory to prior ibrutinib ^{c} | 4 | 3 | 7 (41%) |
| Prior venetoclax, n | | 0 | 2 | 2 (12%) |
| | Refractory to prior venetoclax^{c} | NA | 1 | 1 (6%) |
| Ki67 >30%, ^{d}n/N | | 6/6 | 7/9 | 13/15 (87%) |
| TP53 mutation, ^{d,e} n | | 1 | 0 | 1 |
| Variant type, ^{d} n | | | | |
| | Blastoid | 3 | 1 | 4 |
| | Pleomorphic | 1 | 0 | 1 |
| CNS disease at time of CAR-expressing T cell administration, n | | 0 | 1 | 1 (6%) |
| Prior bone marrow involvement. ^{d,f} n | | 2/6 | N/A | 2/17 (33%) |
| | Prior pleural effusions, ^{d.g} n | 1 | 5 | 6 |
| Bridging chemotherapy | | 4 | 6 | 10 (59%) |
| ^{a}One subject had nonmeasurable disease (splenic focus, extranodal) | | | | |
| ^{b} One subject received allogenic HSCT. | | | | |
| ^{c}Refractory was defined as best response of progressive disease. | | | | |
| ^{d}Based on the number of subjects with available database entries; data was not available for all subjects | | | | |
| ^{e}This subject also had Ki67 of 40% | | | | |
| ^{f}Not captured after a protocol amendment | | | | |
| ^{g}Pleural effusions were only captured when reported as an adverse event starting prior to anti-CD19 CAR-T cell therapeutic T cell composition administration. | | | | |
| CNS, central nervous system; DL, dose level; HSCT, hematopoietic stem cell transplantation; LDC, lymphodepleting chemotherapy; LDH, lactate dehydrogenase; NA, not applicable; SPD, sum of the product of the longest perpendicular dimensions. | | | | |

### A. Safety

Adverse events in this subject population at this timepoint are set forth in **Table E4.2.** The most common grade 3 or 4 treatment-emergent adverse events (TEAEs) were cytopenias. One subject had a grade 5 TEAE of alveolar damage on Day 38 after receiving a new anticancer therapy (rituximab and lenalidomide) on Day 31 post CAR-expressing T cell administration, and was considered not to be related to the CAR-expressing T cell dose. One dose-limiting toxicity (DLT) of grade 5 tumor lysis syndrome occurred at day 10-12 in a subject that received administration of CAR-expressing T cells at DL2. This subject had high tumor burden, sum of the product of the longest perpendicular dimensions of 210 cm², pleural effusion, bone marrow infiltration, and gastric involvement. The subject refused intubation and later died.

| **Table E4.2 Treatment-Emergent Adverse Events** | | | | |
|---|---|---|---|---|
| | | DL1 (n=6) | DL2 (n=11) | All Subjects (N=17) |
| All TEAEs | | 6 | 11 | 17 (100%) |
| | Grade 3 or 4 TEAEs | 4 | 9 | 13 (76%) |
| | Grade 5 TEAEs^{a} | 0 | 1 | 1 (6%) |
| Serious TEAEs | | 3 | 7 | 10 (59%) |

| Most common grade 3 or 4 TEAEs | | | | |
|---|---|---|---|---|
| Thrombocytopenia | | 1 | 6 | 7 (41%) |
| Anemia | | 2 | 4 | 6 (35%) |
| Neutropenia | | 2 | 4 | 6 (35%) |
| DLTs^{b} | | 0 | 1 | 1 (6%) |
| ^{a} One subject had grade 5 DLT of tumor lysis syndrome. The subject refused intubation and later died. DL, dose level; DLT, dose limiting toxicity; TEAE, treatment-emergent adverse effect. | | | | |

As shown in **Table E4.3,** severe CRS (grade 4) was observed in one subject treated at DL2. NEs, including severe NEs in 2 subjects, occurred in subjects treated at DL2. The subject that received a nonconforming product did not experience CRS or NE.

| **Table E4.3 Adverse Events of Specific Interest** | | | | |
|---|---|---|---|---|
| | | **DL1 (n=6)** | **DL2 (n=11)** | **All Subjects (N=17)** |
| Any grade CRS, n | | 1 | 6 | 7 (41%) |
| | Grade 3 or 4 CRS | 0 | 1 | 1 (6%) |
| Median time to onset, days (range) | | 6 (6-6) | 7 (2-10) | 7 (2-10) |
| Median time to resolution, days (range) | | 2 (2-2) | 5 (2-8) | 4 (2-8) |
| Any grade NEs, n | | 0 | 3 | 3 (18%) |
| | Grade 3 or 4 NEs | 0 | 2 | 2 (12%) |
| Median time to onset, days (range) | | NA | 9 (7-25) | 9 (7-25) |
| Median time to resolution, days (range) | | NA | 3 (1-3) | 3 (1-3) |
| Tocilizumab use, n | | 0 | 3 | 3 (18%) |
| Corticosteroid use, n | | 0 | 3 | 3 (18%) |
| CRS, cytokine release syndrome; DL, dose level; NA, no applicable; NE, neurological event; NR, not reported. | | | | |

### B. Response

The overall response rates for subjects of this population at this timepoint are set forth in **Table E4.4.** Seven subjects had ongoing CR at the last time point assessed in this study: one subject through Day 365 and one subject through Day 545 at DL1, and two subjects through Day 180 and three subjects through Day 90 at DL2. Median time to complete response (CR) was 1 month, and the median duration of response (DOR) has not yet been reached at this time point of the clinical study (observed range 1.0-16.2+). At DL1, subjects maintained response up to the last time point of evaluation (Day 545) and in DL2 subjects maintained response up to Day 180. Of the 17 subjects, 5 did not respond to treatment: 1 subject with secondary central nervous system (CNS) involvement did not respond (stable disease on Day 29). Two subjects had central nervous system (CNS) disease at relapse: 1 subject had both CNS and systemic disease at day 90 and 1 subject had isolated CNS disease at Day 180.

| **Table E4.4 Overall Response Rates** | | | |
|---|---|---|---|
| | **DL1^{a} (n=6)** | **DL2 (n=11)** | **All Subjects (N=17)** |
| Best ORR, n (%) | 4 (67) | 8 (73) | 12 (71) |
| Best CR, n (%) | 2 (33) | 7 (64) | 9 (53) |
| Median (range) time to CR, months | 3.6 (1-6.3) | 0.9 (0.9-4.9) | 1 (0.9-6.3) |
| Median (range) PFS, months | 2.6(0.7-18.2+) | 5.8 (0.4-6.0+) | 5.8 (0.4-18.2+) |
| Median (range) OS, months | 10.9 (1.2-18.2+) | NR (0.4-9.2+) | 11.1 (0.4-18.2+) |
| Median (range) follow-up, months | 18.0 (1.2-18.2+) | 6.2 (0.4-9.2+) | 8.4 (0.4-18.2+) |
| CR, complete response; DL, dose level; NR, not reached; ORR, overall response rate; OS, overall survival; PFS, progression-free survival. + Indicates ongoing response or survival. | | | |

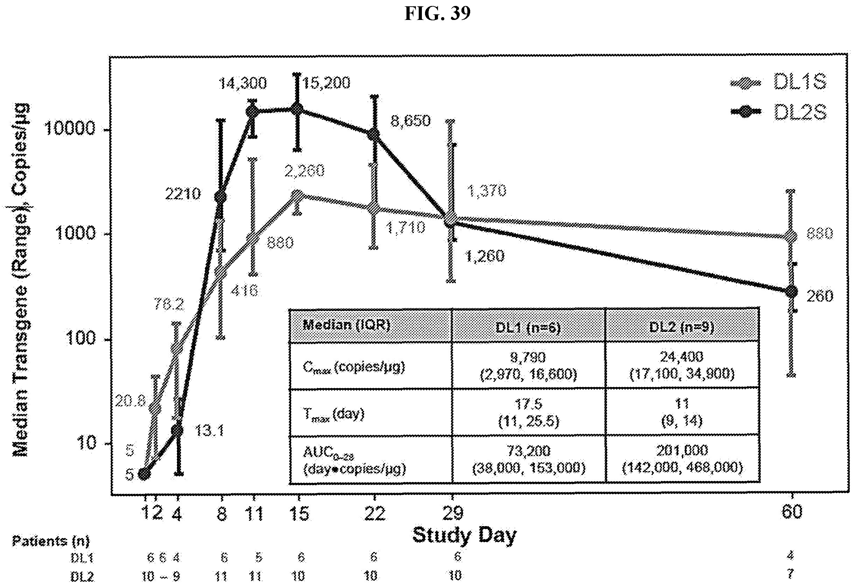

CAR T cell expansion by dose level is shown in **FIG. 39**. Pharmacokinetic (PK) parameters from 2 subjects were not calculated because the last PK sampling time was before Day 29. The results show an increased cell expansion at DL2. Without wishing to be bound by theory this increased expansion at DL2 may be associated with increased CRS and NEs observed at this dose level; however, this observation is based on a small number of subjects.

Together, these data are consistent with the observation that administration of the anti-CD19 CAR-T cell therapeutic T cell composition to subjects with R/R MCL, resulted in tolerable toxicity, including a low incidence of grade 3 or 4 CRS and NEs. The incidence of CRS and NE was similar to that observed in the study described in Example 6 in subjects (n=102) with aggressive B-cell NHL that had been treated with a similar anti-CD19 CAR T cell composition, in which 1% developed grade 3/4 CRS and 13% grade 3/4 NE (see **Table E19**). The results also showed favorable response rates in subjects with R/R MCL, including a best ORR of 71% and CR rate of 53% at the time point in this study. At this time point, 7 subjects (41%) are in ongoing CR and median duration of CR has not yet been reached.

### Example 3 Further Assessment of Response, Safety, Pharmacokinetics, Pharmacodynamics and Blood Analytes in Subjects with Relapsed and Refractory Non-Hodgkin's Lymphoma (NHL) After Administration of Anti-CD19 CAR-Expressing Cells

Response outcomes, safety outcomes, pharmacokinetic and pharmacodynamics parameters, and blood analytes were assessed in patients at a subsequent point in time in the clinical study described in Example 1 above.

### A. Subjects and Treatment

The analysis at this time point presented in this example is based on assessment of a total of 91 subjects in the full DLBCL cohort (88 (65 from the CORE cohort) assessed for response and 91 (67 from the CORE cohort) assessed for safety) that had been administered the anti-CD19 CAR-expressing cells. The FULL cohort included DLBCL, NOS de novo and transformed from any indolent lymphoma, ECOG 0-2; the CORE cohort for analysis included subjects having DLBCL, NOS and transformed from follicular lymphoma (tFL) or high grade B-cell lymphoma and with Eastern Cooperative Oncology Group performance status (ECOG PS) of 0 or 1. Approximately 90% of treated patients in the CORE cohort had at least 1 poor-risk disease feature predictive of short median overall survival (OS) of 3-6 months, such as double/triple hit expressors, primary refractory disease, refractory to 2 or more lines of therapy, never achieved CR, or never received autologous stem cell transplant (ASCT). Subjects with MCL after 1 line of therapy also were included in the study. In some embodiments a cohort of subjects having Diffuse large B-cell lymphoma (DLBCL) not otherwise specified (NOS; de novo and transformed from follicular lymphoma tFL) or high-grade B-cell lymphoma, with MYC and BCL2 and/or BCL6 rearrangements with DLBCL histology, and excluding subjects with ECOG score of 2 or subjects who have received prior hematopoietic stem cell transplantation (HSCT), are administered CAR-T compositions as provided herein. In some embodiments, subjects of the CORE cohort are administered anti-CD19 CAR⁺ T cells at a single dose of DL2 (1 x 10⁸ total CAR-expressing T cells).

At this time point, a total of 140 subjects had been leukapheresed, of which 10 were awaiting manufactured composition, 2 had withdrawn before manufacturing, and 2 had compositions unavailable. Of another 18 subjects whose products were available, 4 were awaiting treatment, 4 had withdrawn, and 10 had developed progressive disease or had died. A total of 108 subjects had been administered the anti-CD19 CAR-expressing cells, of which 6 were not evaluable and 11 received non-conforming anti-CD19 CAR-expressing cells (compositions not necessarily meeting certain specifications but deemed to be safe for administration). Subjects had received DL1 (n=45), double dose of DL1 (n=6) or DL2 (n=40). Six (6) subjects with mantle cell lymphoma (MCL) had been administered CAR⁺ cells at DL1 (five treated with conforming product, one treated with non-conforming product), and five (5) had completed 28 days of follow-up. One MCL subject had developed CRS, and none had received tocilizumab or dexamethasone. Product had been available for 98% of apheresed subjects (126/128) in the DLBCL cohort.

The subjects at this time-point included 5 patients that had been treated in the outpatient setting (including four (4) subjects treated with DL1, one (1) treated with DL2; four (4) of which were included in the CORE cohort). For subjects treated in the outpatient setting, median age was 57 years old (range 26-61), 3 had DLBCL, NOS, 1 had tFL, and 1 had PMBCL. All five (5) subjects had an ECOG scores of 0 or 1. Data on outpatient results included results for three (3) additional subjects that had been treated in the outpatient setting (total of eight (8) subjects) and whose data became available after the time point for the analysis in this Example.

The demographics and baseline characteristics of the full and core cohort subjects at the timepoint are set forth in **Table E5.**

| **Table E5. Patient Characteristics: DLBCL Cohort** | | | |
|---|---|---|---|
| **Characteristic** | | **FULL (n = 91)** | **CORE (n = 67)** |
| Median Age, years (range), | | 61 (20-82) | 60 (20-82) |
| | ≥ 65 years, n (%) | 34 (37) | 24 (36) |
| | Male/Female, n (%) | 61/30 (67/33) | 46/21 (69/31) |

| **B-NHL subtype, n (%)** | | | |
|---|---|---|---|
| DLBCL, NOS de novo | | 59 (65) | 51 (76) |
| Transformed from FL (tFL) | | 19 (21) | 16 (24) |
| Transformed from MZL (tMZL) /CLL (tCLL) | | 6 (7) / 4 (4) | 0 |
| Follicular, Grade 3B/PMBCL | | 1 (1) / 2 (2) | 0 |

| **Molecular subtype, n (%)** | | | |
|---|---|---|---|
| Double/triple hit [High grade B-cell lymphoma]^{a} | | 18 (20) | 16 (24) |

| **Patient characteristics, n (%)** | | | |
|---|---|---|---|
| Chemorefractory^{b} | | 61 (67) | 44 (66) |
| ECOG PS 0-1 / 2 (pre-LD) | | 81 (89) / 10 (11) | 67 (100) / 0 |
| IPI 3-5 / Disease stage 3-4 | | 38 (42) / 70 (77) | 24 (36) / 49 (73) |
| CNS involvement | | 2 (2) | 2 (3) |
| Prior lines of therapy, median (range) | | 3 (1-12) | 3 (1-8) |
| Never achieved CR | | 47 (52) | 34 (51) |
| Any HSCT | | 39 (43) | 28 (42) |
| | Prior Autologous | 36 (40) | 28 (42) |
| | Prior Allogeneic | 5 (5) | 0 |

| | | | |
|---|---|---|---|
| HSCT, hematopoietic stem cell transplantation; LD, lymphodepletion. ^{a} At trial initiation, included in DLBCL, NOS histology; based on most recent WHO criteria (Swerdlow et al., (2016) Blood 127(20):2375-2390), are now considered "high-grade B-cell lymphoma, with MYC and BCL2 and/or BCL6 rearrangements with DLBCL histology (double/triple hit). ^{b} SD or PD to last chemotherapy-containing regimen or relapse <12 months after autologous SCT. | | | |

### B. Safety and Response Outcomes after Treatment

As shown in **Table E6,** the objective response rate (ORR) was 74%, including 52% subjects who showed a complete response (CR). The incidence of any grade of cytokine release syndrome (CRS) was 35%, with 1% severe CRS; and the incidence of any grade of neurotoxicity (NT) was 19%, with 1% severe NT.

| **Table E6. Response and Safety After CAR⁺ Cell Administration** | | | | |
|---|---|---|---|---|
| | **FULL** | **CORE** | | |
| | **All Dose Levels** | **All Dose Levels^{a}** | **DL1S** | **DL2S** |
| **Best Overall Response (BOR), n^{b}** | 88 | 65 | 34 | 27 |
| ORR, % (95% CI) | 74 (63,83) | 80(68, 89) | 77 (59,89) | 82 (62, 94) |
| CR,% (95% CI) | 52 (41,63) | 55(43, 68) | 47 (30, 65) | 63 (42, 81) |
| **Safety, n^{c}** | 91 | 67 | 34 | 29 |
| Any CRS,% (95% CI) | 35 (25, 46) | 36 (24, 48) | 41 (25, 59) | 24 (10, 44) |
| sCRS(grade 3-4),% (95% CI) | 1 (0, 6) | 1 (0, 8) | 38 (0, 15) | 0 |
| Any NT, % (95% CI) | 19 (11,28) | 21 (12, 33) | 24 (11, 41) | 17 (6, 36) |
| sNT (grade 3-4),% (95% CI) | 12 (6, 21) | 15 (7, 26) | 21 (9, 38) | 7 (1, 23) |
| ^{a} Four patients treated on DL1D (dose level 1, two-dose schedule) with similar outcomes. | | | | |

^{b} Includes patients with event of PD, death, or 28-day restaging scans. One patient did not have restaging scans available. ^{c} Includes all subjects who have received at least one dose of conforming CAR-expressing cell product 28 days prior to data snapshot date or died.

As shown in **Table E7,** high rates of response and low severe toxicity was observed in the full DLBCL population.

| **Table E7. Response After CAR⁺ Cell Administration** | | | | | |
|---|---|---|---|---|---|
| | | **By Diagnosis** | | | |
| | **FULL** | **DLBCL, NOS** | **tFL** | **tCLL/MZL** | **FL3B/PMBCL** |
| **BOR, n^{a}** | 88 | 57 | 19 | 10 | 2 |
| ORR,% (95% CI) | 74 (63,83) | 74 (60, 85) | 84 (60, 97) | 50 (19, 81) | 100 (16, 100) |
| CR,% (95% CI) | 52 (41,63) | 51 (37, 64) | 63 (38, 84) | 30 (7, 65) | 100 (16, 100) |
| **Safety, n^{b}** | 91 | 59 | 19 | 10 | 3 |
| Any CRS,% (95% CI) | 35 (25, 46) | 34 (22, 47) | 42 (20, 67) | 20 (3, 56) | 67 (9, 99) |
| sCRS (grade 3-4),% (95% CI) | 1 (0, 6) | 2 (0, 9) | 0 | 0 | 0 |
| Any NT,% (95% CI) | 19 (11,28) | 20 (11, 33) | 21 (6, 46) | 10 (0, 45) | 0 |
| sNT (grade 3-4),% (95% CI) | 12 (6, 21) | 14 (6, 25) | 11 (1, 33) | 10 (0, 45) | 0 |

| | | | | | |
|---|---|---|---|---|---|
| ^{a} Includes patients with event of PD, death, or 28-day restaging scans. One patient did not have restaging scans available. ^{b} Includes all subjects who have received at least one dose of conforming CAR⁺ expressing cells 28 days prior to data snapshot date or died. | | | | | |

As shown in **Table E8,** high rate of response and a dose-dependent response was observed in the CORE cohort of subjects.

| **Table E8. Durable Response After CAR⁺ Cell Administration** | | | |
|---|---|---|---|
| | **Dose Levels^{a}** | **DL1S** | **DL2S** |
| **BOR, n^{b}** | 65 | 34 | 27 |
| ORR (95% CI),% | 80(68, 89) | 77 (59,89) | 82 (62, 94) |
| CR (95% CI),% | 55(43, 68) | 47 (30, 65) | 63 (42, 81) |
| **≥ 3-mo f/u, n^{c}** | 52 | 29 | 19 |
| 3-mo ORR (95% CI),% | 65 (51,78) | 59 (39, 77) | 74 (49. 91) |
| 3-mo CR (95% CI).% | 54(40, 68) | 41 (24,61) | 68 (43. 87) |
| **≥ 6-mo f/u, n^{d}** | 38 | 20 | 14 |
| 6-mo ORR (95% CI),% | 47(31, 64) | 40 (19, 64) | 50 (23, 77) |
| 6-mo CR (95% CI).% | 42 (26, 59) | 30 (12, 54) | 50 (23, 77) |

| | | | |
|---|---|---|---|
| ^{a} Four patients (CORE) treated on DL1D with similar outcomes. ^{b} Includes patients with event of PD, death, or 28-day restaging scans. One patient did not have restaging scans available. ^{c} The denominator is number of patients who received CAR⁺ cells ≥ 3 months ago, prior to data snapshot date, with an efficacy assessment at month 3 or prior assessment of PD or death. ^{d} The denominator is number of patients who received CAR⁺ cells ≥ 6 months ago, prior to data snapshot date, with an efficacy assessment at month 6 or prior assessment of PD or death. | | | |

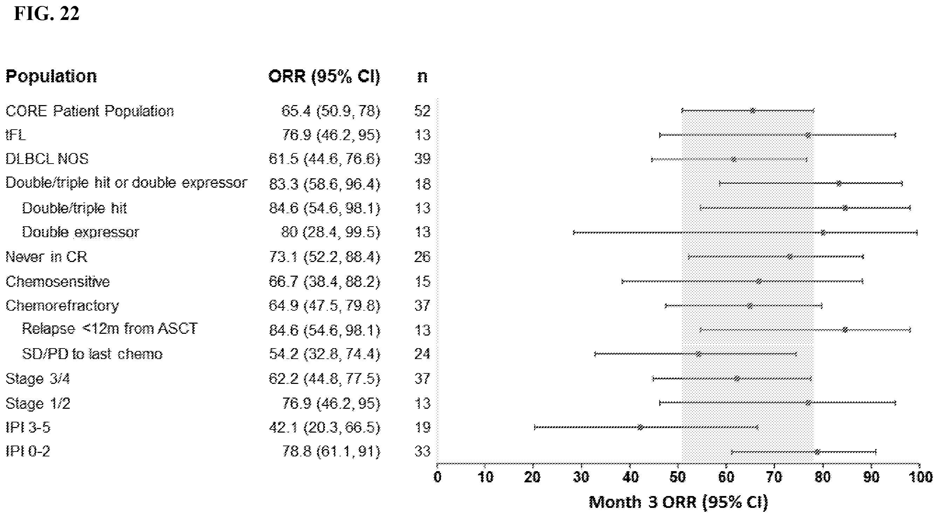

Three-month objective response rates (ORR) among various subgroups of subjects in the poor-risk DLBCL subgroups, that included all DLBCL patients treated at all dose levels in the core cohort, are shown in **FIG. 22****.** The results showed high durable ORR in the poor-risk DLBCL subgroup.

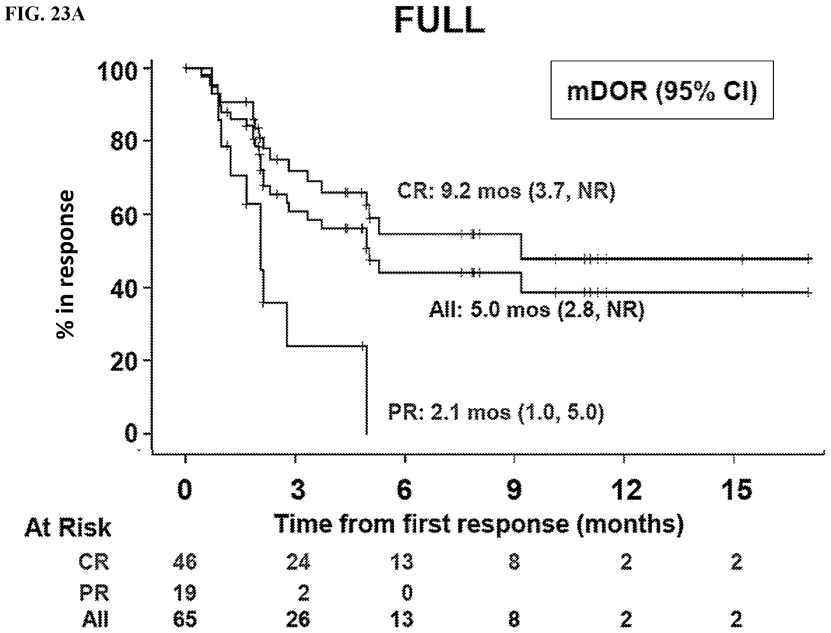

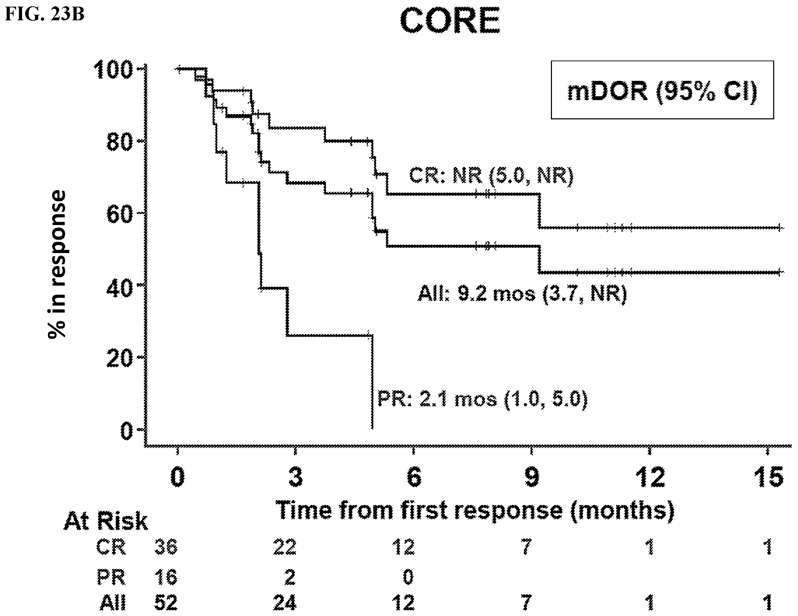

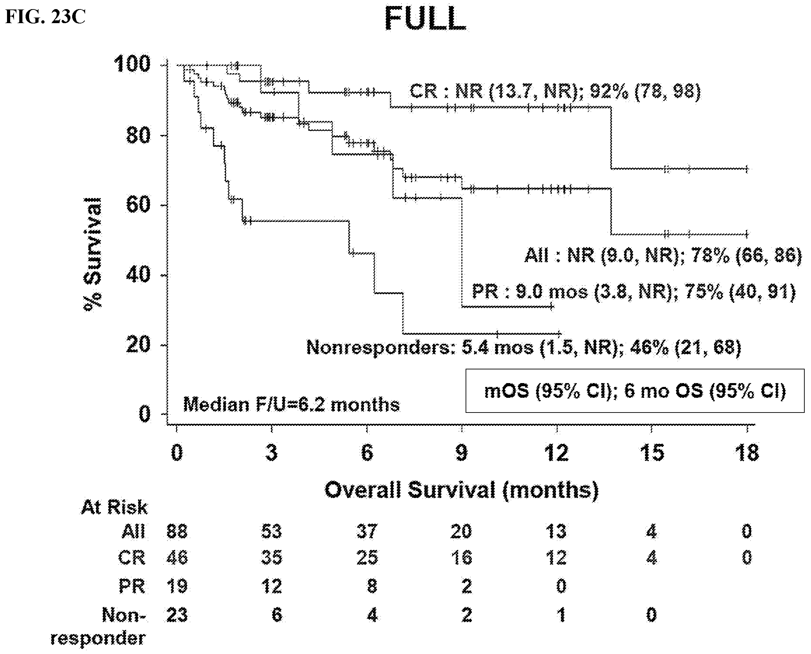

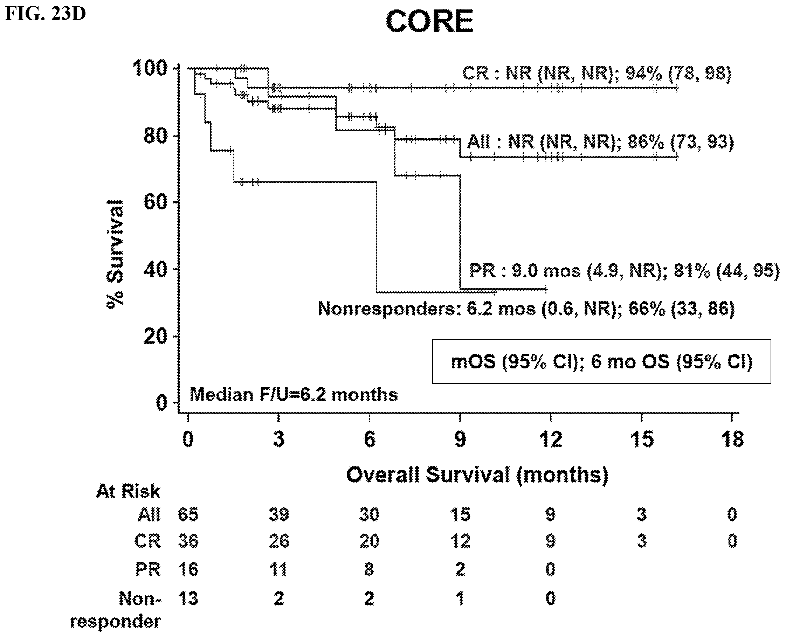

Results for the duration of response (DOR) and overall survival (grouped by best overall response (non-responder, CR/PR, CR and/or PR)) are shown for the full cohort and the core cohort cohorts of subjects, in **FIGS. 23A-23D****.** The results also showed 80% (16/20) of subjects with a CR at 3 months stay in CR at 6 months, and 92% (11/12) of subjects with a response (CR or PR) at 6 months continue to show a response longer term.

**FIG. 24** depicts the percentage of subjects at this timepoint who were observed to have experienced laboratory abnormalities and treatment-emergent adverse events (TEAEs) (data for 5 patients with MCL treated with conforming product at DL1 with at least 28 days of follow-up are not included). In addition to the TEAEs shown in **FIG. 24****,** the following event terms were observed at Grade 3-4 in ≥5% of patients: encephalopathy (8%), Pancytopenia (5%) and Febrile neutropenia (7%). Eight patients (9%) had infusional toxicity, defined as AE on day of administration related to CAR⁺ cell administration, including flushing, headache, fever, pyrexia, chills, rigors, vomiting, rash, hives, pruritis, hypotension, wheezing, bronchospasm, shortness of breath, nausea, vomiting, back pain, cough, and infusion-related reaction. Events included chills (2), pyrexia (5), flushing (1), headache (1), hypotension (1), infusion related reaction (1), rash (1), pruritis (1), and vomiting (1), with 6 grade 1 events, 1 grade 2 (chills), and 1 grade 3 (hypotension) event. TEAE in the core cohort did not differ substantially from those in the full cohort. The most common related TEAEs in the subjects treated in the outpatient setting group were CRS, hypotension, vomiting, anemia, and dyspnea.

**Table E9** sets forth the TEAEs and neurotoxicity that occurred in 25 percent or more subjects in the FULL or CORE cohort, for subjects who received DL1S and DL2S. No apparent dose-toxicity relationship was observed in the DLBCL population.

| **Table E9. TEAEs ≥25% in FULL cohort, CORE cohort, and CORE cohort by dose level.** | | | | |
|---|---|---|---|---|
| **Term, n (%)** | **FULL (N = 91)** | **CORE^{a} (n = 67)** | **CORE DL1S (n = 34)** | **CORE DL2S (n = 29)** |
| | 85 (93) | 63 (94) | 33 (97) | 26 (90) |
| Anemia^{b} | 64 (70) | 48 (72) | 28 (82) | 19 (66) |
| Thrombocytopenia^{b} | 48 (53) | 41 (61) | 20 (59) | 19 (66) |
| Fatigue | 34 (37) | 25 (37) | 11 (32) | 12 (41) |
| CRS | 32 (35) | 24 (36) | 14 (41) | 7 (24) |
| Nausea | 25 (27) | 19 (28) | 12 (35) | 5 (17) |
| Diarrhea | 23 (25) | 16 (24) | 7 (21) | 7 (24) |

| | | | | |
|---|---|---|---|---|
| a Includes 4 patients treated at dose level 1, two-dose schedule. b Laboratory anomalies. | | | | |

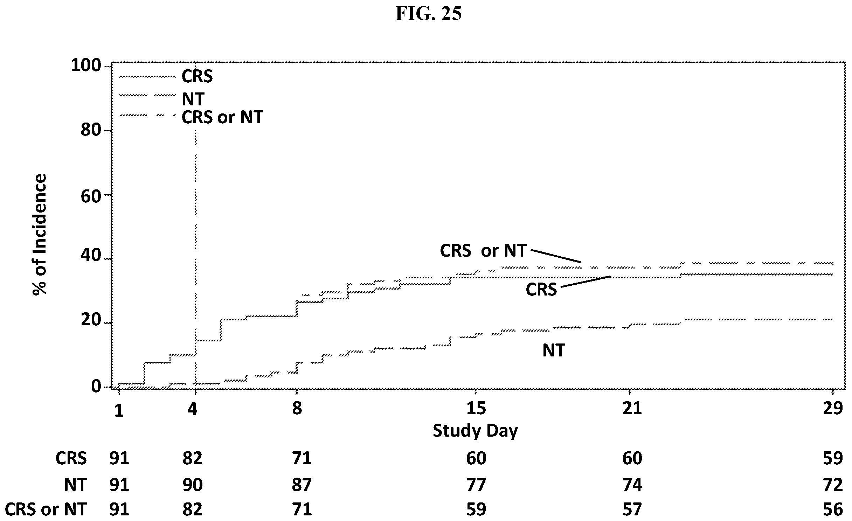

**FIG. 25** depicts the number and percentage of subjects that were observed to have CRS and/or NT at various time points after administration of CAR⁺ cells. In this assessment, the median time to onset of first of CRS or NT event was observed to be 5 (range 1-14) or 10 (range 3-23) days, respectively. Within the first 72 hours after CAR⁺ cell administration, 1 patient had NT (grade 1), and only 14% (13 of 91) had CRS (7 grade 1; 6 grade 2). The median duration (Q1,Q3) of CRS or NT was 5 (4, 8) or 10.5 (7, 19) days, respectively. NT was preceded by CRS in 12 of 17 cases (71%). All evaluable NT events were resolved at the time of analysis except one grade 1 tremor and 2 patients died from progressive disease with ongoing NT (based on safety database of reported events including additional subjects analyzed after the analysis timepoint described in this Example).

In the full cohort (n=91), selected subjects with onset of CRS or NT were administered anti-cytokine therapy with tocilizumab and/or dexamethasone as follows: Tocilizumab alone, 4% (n = 4); Dexamethasone alone, 9% (n = 8); Tocilizumab and dexamethasone, 8% (n = 7). The median number of dexamethasone doses was 6 (range, 2-99); and the median number of tocilizumab doses was 1 (range, 1-3).

**Table E10** shows toxicity outcomes in subjects in the CORE cohort that received a single dose at DL1 or DL2. No deaths occurred from CRS or NT. The median time to onset of CRS was 5 days (range, 2 -14) and NT was 11.5 days (range, 5 -23). In the CORE cohort, 13% (n = 9) received tocilizumab, and 18% (n = 12) received dexamethasone to ameliorate toxicity. Eighteen percent of subjects (12 of 67) exhibited neurotoxicity terms consistent with encephalopathy, including encephalopathy (13%), 6% (4 of 67) had aphasia and 3% (2 of 67) had seizures. In **Table E10,** the number of subjects or% of total subjects (parentheses) exhibiting an indicated toxicity outcome is shown at all dose levels or specifically in subjects administered DL1 or DL2. Also shown in brackets is the upper and lower 95% confidence interval.

| **Table E10. Toxicity in Core Cohort Receiving Different Dose Levels.** | | | |
|---|---|---|---|
| | **All Dose Levels^{a} n=67** | **DL1S n=34** | **DL2S n=29** |
| **CRS, n (%) [95%CI]** | | | |
| Any Grade | 24 (36) [24, 48] | 14 (41) [25, 59] | 7 (24) [10, 44] |
| Grade ½ | 23 (34) [23, 47] | 13 (38) [22,56] | 7 (24) [10, 44] |
| Grade 3/4 (sCRS) | 1 (1) [0, 8] | 1 (3) [0, 15] | 0 |

| **Neurotoxicity^{b}, n (%) [95% CI]** | | | |
|---|---|---|---|
| Any Grade | 14 (21) [12, 33] | 8 (24) [11, 41] | 5 (17) [6, 36] |
| Grade ½ | 4 (6) [2, 15] | 1 (3) [0, 15] | 3 (10) [2, 27] |
| Grade 3/4 (sNT) | 10 (15) [7, 26] | 7 (21) [9, 38] | 2 (7) [1, 23] |

| **Any, n (%) [95%CI]** | | | |
|---|---|---|---|
| CRS or NT, n (%) | 28 (42) [30, 54] | 15 (44) [27, 62] | 10 (34) [18, 54] |
| sCRS or sNT, n (%) | 10 (15) [7, 26] | 7 (21) [9, 38] | 2 (7) [1, 23] |

| | | | |
|---|---|---|---|
| ^{a} Four patients treated on DL1D with similar outcomes. ^{b} Includes confusional state, encephalopathy, aphasia, ataxia, cerebellar syndrome, delirium, depressed level of consciousness, dizziness, flat affect, hand-eye coordination impaired, memory impairment, tremor, agitation, disturbance in attention, dysarthria, mental status changes, muscular weakness, seizure, somnolence, and urinary incontinence. | | | |

Among twelve (12) subjects receiving nonconforming products, 10 at DL1 and 2 at DL2, all had 28-day follow-up. CRS was observed in 33% of the subjects (4/12), and NT was not observed in any of the subjects. Two subjects received tocilizumab and 3 subjects received dexamethasone. The toxicity rates were comparable to those observed in the larger cohort of subjects administered conforming product. In the subjects receiving nonconforming products, pharmacokinetic (PK) expansion was higher in subjects with CRS/NT, subjects with high tumor burden or LDH levels.

### C. Assessment of Outpatient Administration

Data for a total of eight (8) subjects were evaluated at this timepoint that had been treated in the outpatient setting (median age of 58.5 and ECOG of 0 or 1) at multiple clinical sites, including 3 subjects whose data was available subsequent to the time point analyzed for purposes of this Example. The mean length of hospitalization was 15.6 days for subjects treated in the inpatient setting (SD 9.6, n = 86) and 9.3 days for subjects treated in the outpatient setting (SD 11.9, n = 8). A 40% reduction in length of hospitalization was observed in subjects treated in the outpatient setting. The median number of days prior to hospitalization after outpatient CAR⁺ T cell administration was 5 days (range: 4-22). None required admission to the intensive care unit (ICU) after outpatient administration.

Among those of the 8 subjects treated in the outpatient setting with more than 28-day post-administration follow-up, 1 remained outpatient throughout the duration of the dose-limiting toxicity period. Seven (7) patients were admitted with fevers (1 on study day 4, the rest on study day ≥ 5), 6 patients were admitted with CRS (4 grade 1, 2 grade 2) and 2 patients with grade 1 NT. No patient experienced severe CRS or NT. One (1) patient was treated with tocilizumab without dexamethasone for CRS (grade 2), and no patients were treated with dexamethasone for CRS or NT. One patient was admitted 3 days after CAR⁺ T cell administration.

Among 91 subjects treated in the inpatient and outpatient settings, 11 subjects (12%) required ICU admission for management of toxicity; 8 subjects (9%) required ICU admission for management of CRS or NT; 2 subjects (2%) required ICU admission for management of acute respiratory events (one related to CAR⁺ T cell administration, one unrelated). Six (6) subjects (6%) were intubated (based on safety database of reported events including additional subjects analyzed after the analysis timepoint described in this Example; n=94); 7 subjects (7%) received vasopressors (based on safety database of reported events, defined as exhibiting hypotension in the first 28 days after CAR⁺ T cell administration, in the TEAE assessment); and 2 subjects (2%) underwent hemofiltration (based on safety database of reported events). The results showed that very few patients required ICU-level care and associated procedures. The results supported the feasibility of outpatient administration, with safe management of toxicity in the outpatient setting, appropriate education and outpatient monitoring.

The assessment of outpatient administration supported the feasibility of safe outpatient administration. 30% of the subjects were not re-admitted.

### D. Pharmacokinetic Assessment

Numbers of CAR⁺ T cells in peripheral blood and bone marrow at time points before administration (pre-treatment or pre-lymphodepleting chemotherapy (LDC)) and various time points post-treatment (with day of administration as day 1) in 87 subjects in the DLBCL cohort with evaluable PK, by flow cytometry using an antibody specific for the truncated receptor used as a surrogate marker, and quantitative polymerase chain reaction (qPCR) using primers specific for a woodchuck hepatitis virus post-transcriptional regulatory element (WPRE) present in the vector encoding the chimeric antigen receptor (CAR). The area under the curve plotting numbers per microliter for the indicated CAR⁺ cell population between days 0 and 28 (AUC₀₋₂₈) and the maximum or peak blood concentration of CAR⁺ cells (Cₘₐₓ; CAR⁺ cells/µL blood) were assessed. B-cell aplasia was assessed in peripheral blood by flow cytometry, by staining with CD19. Cytokines were measured using a multiplex cytokine assay. For safety analysis, the data from all subjects receiving different dose levels were pooled. For response analysis, data were stratified by dose level. Statistical analysis was two-sided without multiplicity adjustment.

**FIG. 9A** shows detected numbers of CAR T cells per microliter of blood at various indicated time-points, as assessed by qPCR or flow cytometry. **FIG 9B** shows CAR⁺ cells per microliter of blood versus microliter of bone marrow at day 11 ^{±} 3. As shown in **FIG. 9A****,** levels of CAR-expressing cells in samples from subjects were observed both by flow cytometry-based assays and qPCR-based assays. As shown in **FIG. 9B****,** all subjects (n= 86 and 85 for flow cytometry and qPCR, respectively, excluding one patient that did not have flow cytometry results available and 2 patients that did not have qPCR results available) with PK results assessed, showed detectable numbers of the CAR-expressing cells in the blood and bone marrow. Results were consistent with an observation that CAR⁺ T cells had trafficked similarly to the bone marrow and blood.

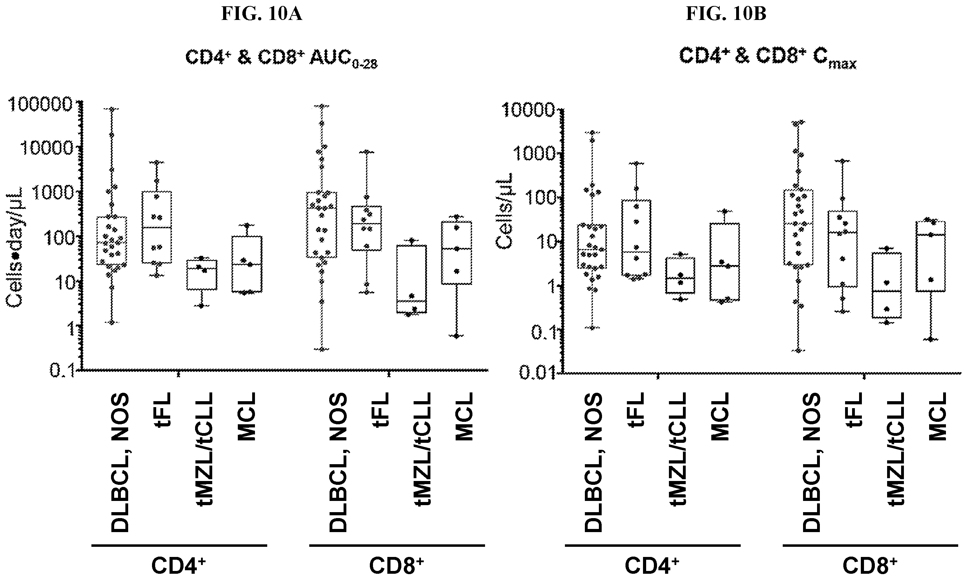

Levels over time of CD4⁺ and CD8⁺ CAR-expressing cells (as assessed by AUC₀₋₂₈ and Cₘₐₓ) were compared in different patient subgroups receiving dose level 1 (DL1): diffuse large B-cell lymphoma de novo (DLBCL, NOS) or transformed from follicular lymphoma (tFL) (CORE; N=32), DLBCL transformed from marginal zone lymphoma or chronic lymphocytic leukemia (tMZL/tCLL; N=4), or mantle cell lymphoma (MCL; N=5), who had received CAR-expressing T cells at DL1. As shown in **FIGS. 10A** and **10B****,** AUC₀₋₂₈ and Cₘₐₓ. varied among subjects in different disease subgroups, with expansion of CD4⁺and CD8⁺ CAR-expressing cells trending lower in non-CORE subsets. PMBCL (n = 2) and FL3B (n = 1) not shown due to limited patient numbers. Expansion in subjects receiving DL2 was similar to in subjects receiving DL1.

### E. Pharmacokinetic Assessment by Dose Level

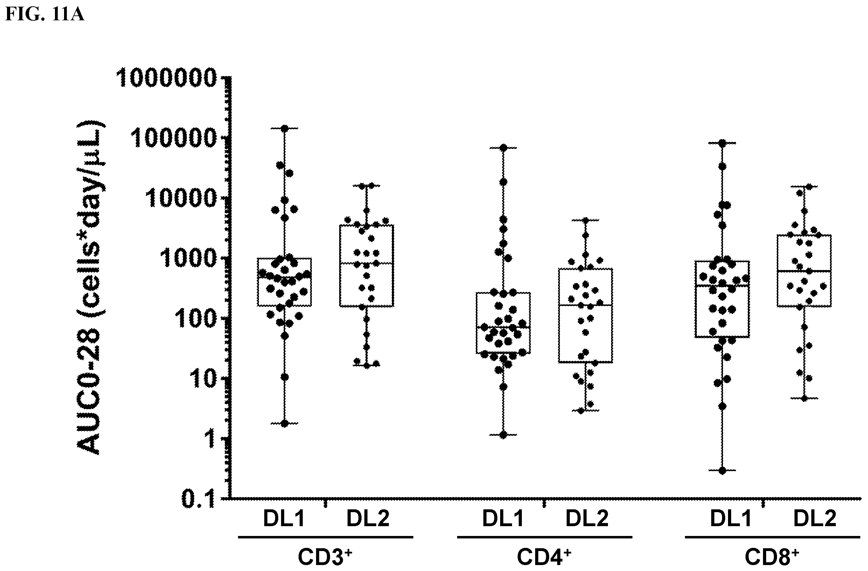

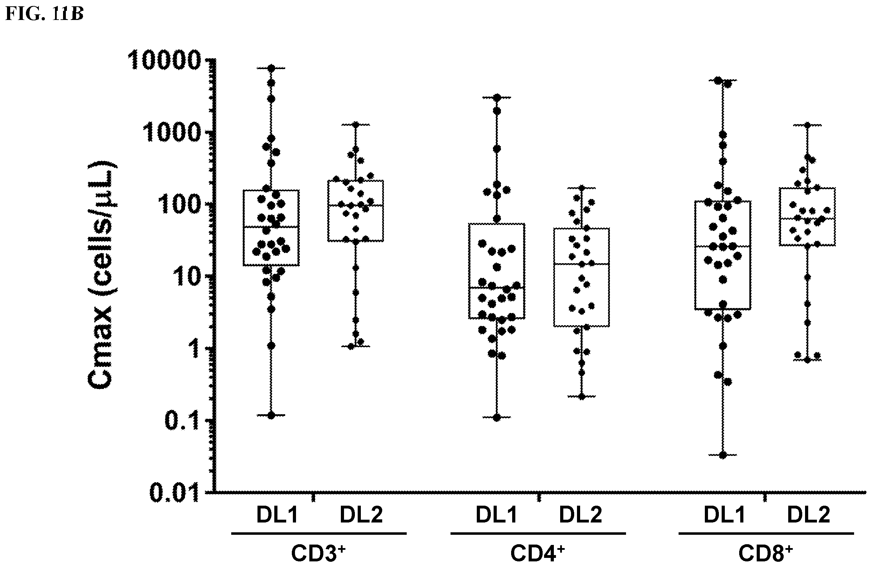

AUC₀₋₂₈ and Cₘₐₓ for CD3⁺, CD4⁺ and CD8⁺ CAR-expressing cells were also compared for subjects having received dose level 1 (DL1; n=32) and those having received dose level 2 (DL2; n=27), in the CORE cohort (subjects with DLBCL, NOS or high grade B-cell lymphoma (double/triple hit); N=59). As shown in **FIGS. 11A** and **11B** and in **Table E11**, a higher median AUC₀₋₂₈ was observed for CD3⁺, CD4⁺ and CD8⁺ CAR-expressing cells was observed in subjects that received DL2, compared to subjects who had received DL1. Similarly, a trend of higher expansion in subjects who had received DL2 was observed in the full DLBCL cohort. A higher durability of response (DOR) at 3 months also was observed among subjects who had received DL2 as compared to those having received DL1, without an increase in toxicity. The median time to Cₘₐₓ (Tₘₐₓ) for CD4⁺ and CD8⁺ CAR⁺ cells was similar between subjects who received DL1 and DL2.

Increased CAR⁺ T cell exposure was observed in DL2 versus DL1, corresponding to an increased durability of response without increased toxicity in DL2 subjects.

| **Table E11. Pharmacokinetics in Subjects Grouped by Dose Levels in Core cohort** | | | |
|---|---|---|---|
| | **DL1S (n = 32)** | **DL2S (n = 27)** | **Total, DL1S and DL2S (n = 59)** |
| **CD3⁺** | | | |
| **Cₘₐₓ, median (cells/µL)** | 48.2 | 96.2 | 65.8 |
| Q1, Q3 | 15.6, 151.3 | 30.2, 219.5 | 19.0, 204.2 |
| Min, max | 0.1, 7726.3 | 1.1, 1280.9 | 0.1, 7726.3 |
| **Tₘₐₓ, median (days)** | 14.5 | 15.0 | 15.0 |
| Q1, Q₃ | 11, 15 | 11, 15 | 11, 15 |
| Min, max | 9, 24 | 8, 31 | 8, 31 |
| **AUC₀₋₂₈, median (cells*day/µL)** | 477.7 | 823.1 | 542.4 |
| Q1, Q3 | 165.9, 999.3 | 155.8, 3628.3 | 155.8, 3381.9 |
| Min, max | 1.8, 142816.7 | 16.5, 16087.8 | 1.8, 142816.7 |

| **CD4⁺** | | | |
|---|---|---|---|
| **Cₘₐₓ, median (cells/µL)** | 7.0 | 14.9 | 7.7 |
| Q1, Q3 | 2.6, 46.0 | 2.0,46.8 | 2.5, 46.8 |
| Min, max | 0.1, 3039.9 | 0.2, 169.4 | 0.1, 3039.9 |
| **Tₘₐₓ, median (days)** | 14.0 | 15.0 | 15.0 |
| Q1, Q3 | 11, 15 | 11, 15 | 11, 15 |
| Min, max | 8, 24 | 8, 31 | 8, 31 |
| **AUC₀₋₂₈, median (cells*day/µL)** | 71.1 | 166.1 | 91.5 |
| Q1, Q3 | 26.4, 274.7 | 18.1,679.0 | 23.9,368.8 |
| Min, max | 1.2, 68990.3 | 2.9, 4266.8 | 1.2, 68990.3 |

| **CD8⁺** | | | |
|---|---|---|---|
| **Cₘₐₓ, median (cells/µL)** | 26.1 | 62.8 | 43.6 |
| Q1, Q3 | 3.7, 111.2 | 26.2,171.7 | 9.1,151.6 |
| Min, max | 0.0, 5237.6 | 0.7,1261.8 | 0.0, 5237.6 |
| **Tₘₐₓ, median (days)** | 15.0 | 15.0 | 15.0 |
| Q1, Q3 | 11, 16 | 11, 17 | 11, 16 |
| Min, max | 4, 28 | 8, 31 | 4, 31 |
| **AUC₀₋₂₈, median (cells*day/µL)** | 347.2 | 606.6 | 412.2 |
| Q1, Q3 | 52.1.871.4 | 155.7,2463.4 | 72.1,1852.5 |
| Min, max | 0.3, 81865.9 | 4.7, 15570.0 | 0.3, 81865.9 |

### F. Persistence

Persistence of CAR-expressing cells and CD19⁺ B cell aplasia (low numbers or absence of CD19⁺ B cells) was assessed at various time points in evaluable subjects with DLBCL that had been administered CAR⁺ T cells, based on detectable CD3⁺, CD4⁺ or CD8⁺ CAR-expressing cell levels and levels of CD19⁺ B-cells detected in the blood, respectively. The results are set forth in **Table E12**. Among subjects evaluated at progression (time of progression regardless of BOR; n=37), a median of 0.17 CD4⁺ CAR⁺ cells/µL (range, 0-65.5 cells/µL) and a median of 0.15 CD8⁺ CAR⁺ cells/µL (range, 0-131.8 cells/µL) were observed at progression. Among subjects evaluated at relapse (at the time of progression after achieving CR) (n=12), a median of 0.17/µL (range, 0-35.1 cells/µL) CD4⁺ CAR-expressing cells and a median of 0.20 cells/µL (range, 0-131.8 cells/µL) CD8⁺ CAR-expressing cells were observed at relapse Long-term persistence of CAR-expressing cells was observed in 75% of evaluable subjects with DLBCL at 12 months. Long-term persistence of B cell aplasia also was observed in 75% of the subjects at 12 months, and in subjects regardless of relapse status. The results are consistent with a conclusion that the anti-CD19 CAR-expressing cells exhibited long-term persistence in most subjects, and suggest the potential for ongoing, low-level disease control even in relapsed patients.

Of subjects who relapsed, 91.7% (11/12) had detectable CAR-expressing cells in the blood at the time of relapse. This result is consistent with a conclusion that a combination therapy or other intervention in some embodiments may be used to augment and/or boost CAR-expressing cells such as those that may be exhausted.

| **Table E12. CAR⁺ Cell Long-Term Persistence and CD19 Aplasia** | | | | | | |
|---|---|---|---|---|---|---|
| | **Month 3** | **Month 6** | **Month 9** | **Month 12** | **At Progression** | **At Relapse** |
| CAR T persistence in evaluable patients, n | 50 | 30 | 18 | 12 | 37 | 12 |
| CD3⁺,% | 100 | 80.0 | 77.8 | 75.0 | 91.9 | 91.7 |
| CD4⁺,% | 88.0 | 63.3 | 50.0 | 41.7 | 83.8 | 83.3 |
| CD8⁺,% | 90.0 | 70.0 | 55.6 | 50.0 | 83.8 | 75.0 |
| CD19⁺ B-cell aplasia (< 1 cell/µL),% | 96.0 | 93.3 | 77.8 | 75.0 | 97.3 | 100 |

### G Pharmacokinetic Assessment and Toxicity

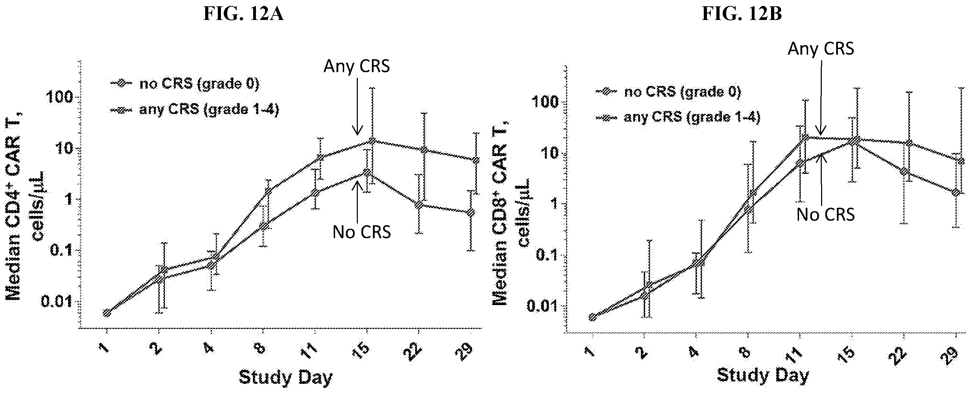

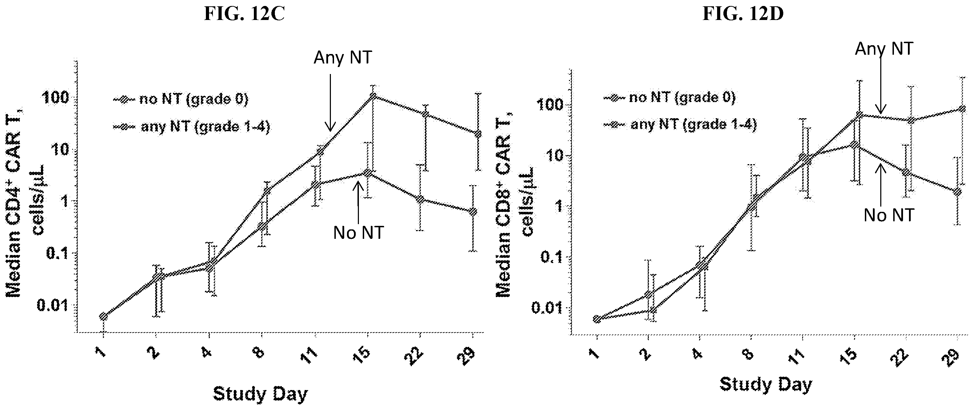

AUC₀₋₂₈ and Cₘₐₓ of CD4⁺ and CD8⁺ CAR-expressing cells was also compared for subjects in the core cohort with any grade (in this assessment, any of grade 1-4; no grade 5 CRS or NT observed) cytokine release syndrome (CRS) or neurotoxicity (NT) to subjects that were not assessed as exhibiting any grade of CRS or NT. The median CD4⁺ CAR⁺ AUC₀₋₂₈ (Q1, Q3) was 59 (18, 210) for no CRS (grade 0; n=43), and 267 (91, 1510) for any CRS (grades 1-4; n=20) (p = 0.001); the median CD8⁺ CAR⁺ AUC₀₋₂₈ (Q1, Q3) was 310 (36, 900) for no CRS (grade 0; n=43), and 605 (174, 5619) for any CRS (grades 1-4; n=20) (p = 0.021); the median CD4⁺ CAR⁺ AUC₀₋₂₈ (Q1, Q3) was 71 (23, 244) for no NT (grade 0; n=50), and 1269 (184, 3057) for any NT (grades 1-4; n=13) (p = 0.003); the median CD8⁺ CAR⁺ AUC₀₋₂₈ (Q1, Q3) was 304 (43, 799) for no NT (grade 0; n=50), and 2463 (607, 7691) for any NT (grades 1-4; n=13) (p = 0.004). As described above and shown in **FIGS. 12A-12D**, higher CD4⁺ and CD8⁺ CAR-expressing cell levels over time were associated with CRS and NT.

### H. Pharmacokinetic Assessment and Response

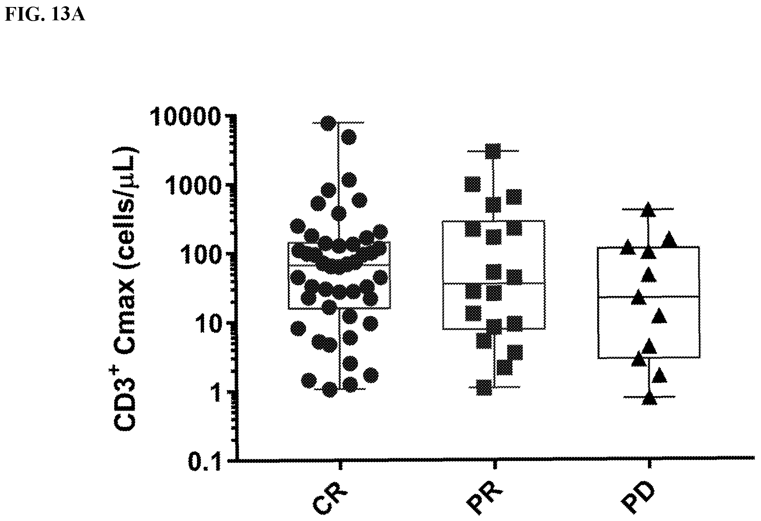

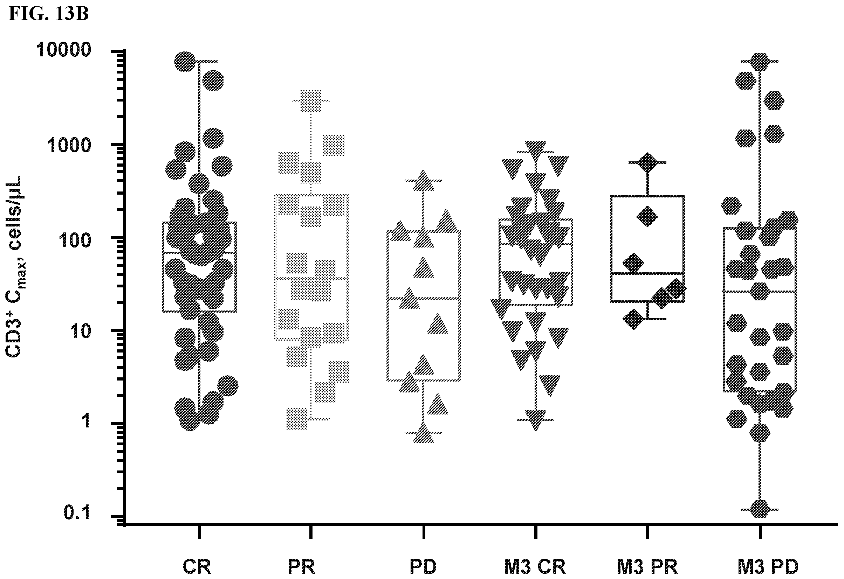

The number of peak CD3⁺ CAR⁺ cells/µL (CD3⁺ Cₘₐₓ) was assessed over time in subjects who had a best overall response (BOR) of CR, PR or PD. As shown in **FIGS. 13A** and **13B****,** a trend towards better BOR was observed in subjects with higher expansion, with variability among subjects.

### I. Pharmacokinetic Assessment by Blood Analytes and Patient Parameters

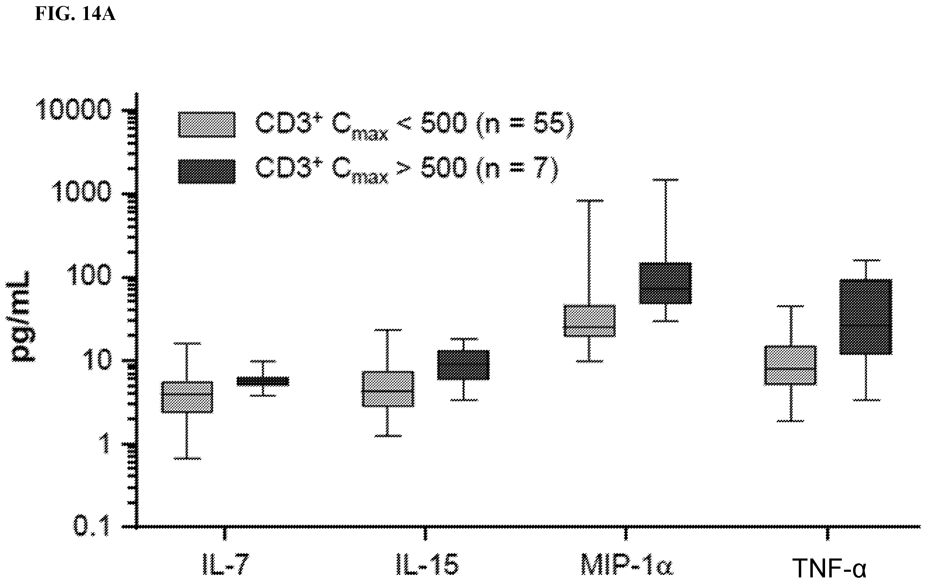

Pre-CAR⁺ T cell treatment (pre-lymphodepleting chemotherapy) plasma cytokine levels, including interleukin-7 (IL-7), IL-15, macrophage inflammatory protein (MIP-1α), were assessed in subjects that exhibited a CAR⁺CD3⁺ blood Cₘₐₓ > 500 CAR⁺ T cells/µL (N=55) as compared to in subjects that exhibited CAR⁺CD3⁺ blood Cₘₐₓ < 500 CAR⁺ T cells/µL (N=7). As shown in **FIG. 14A****,** elevated pre-CAR⁺ T cell treatment cytokine plasma levels were observed to be associated with CAR⁺CD3⁺ Cₘₐₓ > 500 CAR⁺ T cells/µL (Wilcoxon P values <.05 (without multiplicity of adjustment); except for IL-7 p = 0.07).

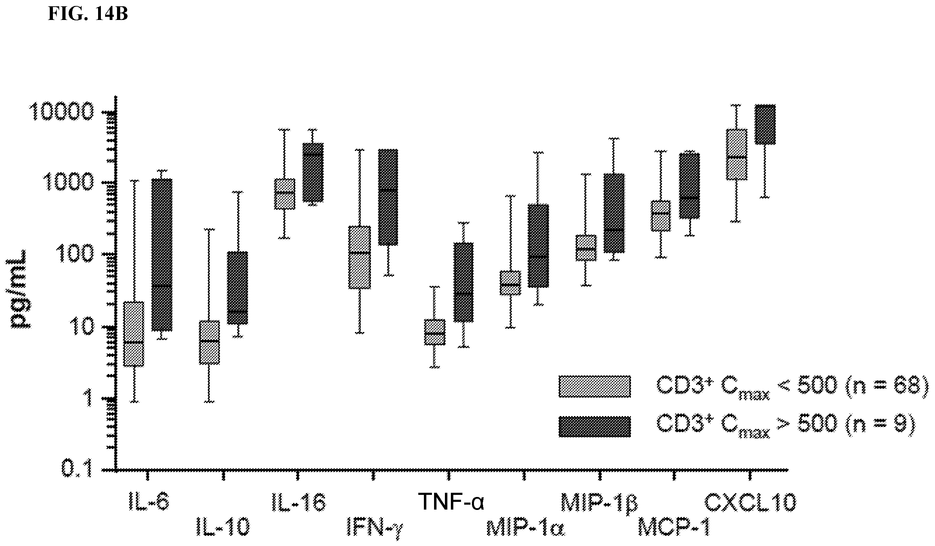

Peak levels of various plasma cytokines (IL-6, IL-10, IL-16, interferon gamma (IFN-γ), tumor necrosis factor alpha (TNF-α), MIP-1α, MIP-1β, Monocyte chemoattractant protein-1 (MCP-1), and C-X-C motif chemokine 10 (CXCL10)) were also assessed in subjects that exhibited CAR⁺ CD3⁺ blood Cₘₐₓ > 500 CAR⁺ T cells/µL (N=68) as compared to subjects that exhibited CAR⁺ CD3⁺ blood Cₘₐₓ < 500 CAR⁺ T cells/µL; N=9). As shown in **FIG. 14B****,** higher peak cytokine levels were observed to be associated with CAR⁺CD3⁺ Cₘₐₓ > 500 CAR⁺ T cells/µL (Wilcoxon P values < 0.05; without multiplicity of adjustment).

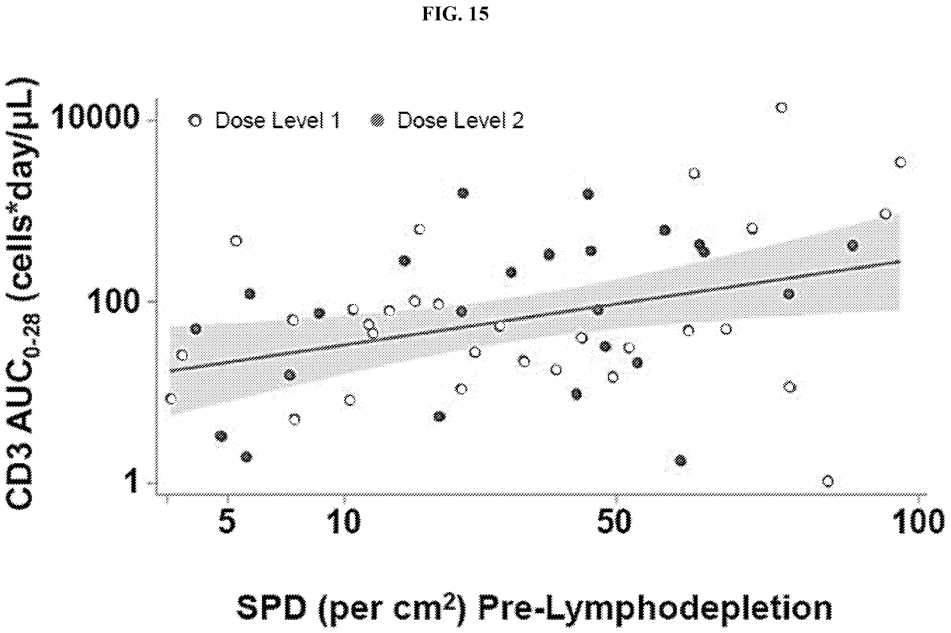

Relationship between pre-CAR⁺ T cell treatment (pre-lymphodepleting chemotherapy (LDC)) volumetric tumor measurement sum of product dimensions (SPD), as an indicator of tumor burden, and AUC₀₋₂₈ of CD3⁺ CAR⁺ T cells, representing CAR⁺ T exposure over time, was assessed. As shown in **FIG. 15****,** a positive correlation was observed between baseline SPD and CD3⁺ AUC₀₋₂₈, with a Spearman correlation of 0.32 and p = 0.019.

### J. Pre-treatment patient parameters and response and toxicity outcomes

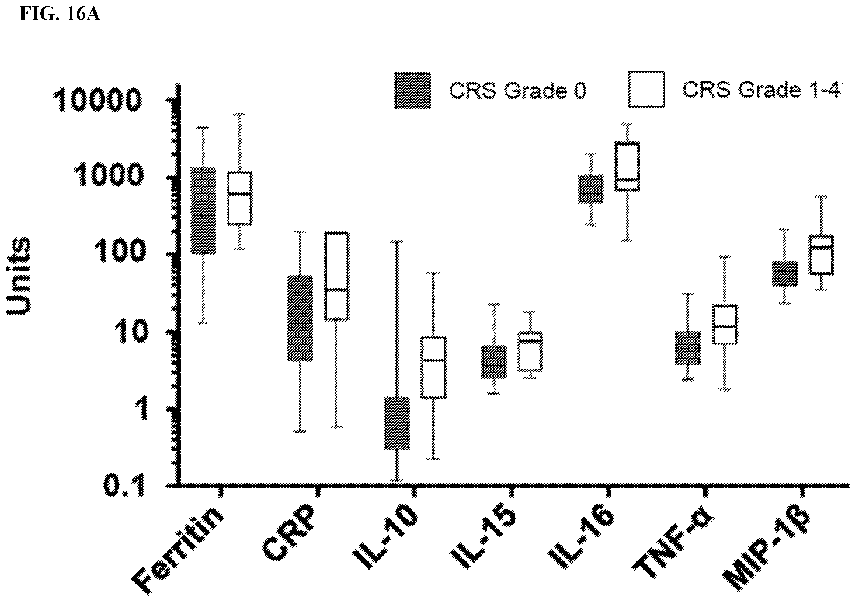

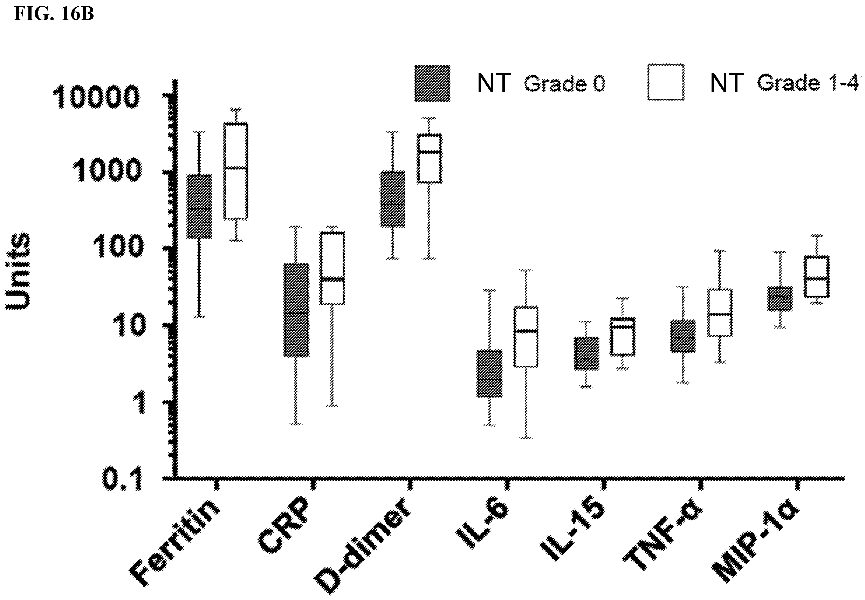

Pre-CAR⁺ T cell treatment (pre-LDC) analyte levels, including cytokines and inflammatory markers such as Ferritin, C-reactive protein (CRP), D-dimer (fibrin degradation product), IL-6, IL-10, IL-15, IL-16 TNF-α, MIP-1α, and MIP-1β, were compared for subjects with any grade (here, grade 1-4) cytokine release syndrome (CRS) or neurotoxicity (NT) to subjects that did not have any CRS or NT (grade 0). In this cohort, among subjects with CRS grade 1-4, all but one CRS events were determined to be grade 1 or 2. As shown in **FIG. 16A** (CRS) and **FIG. 16B** (NT), higher peak plasma cytokine levels and inflammatory marker levels were observed to be associated with CRS and NT, based on univariate analysis (Wilcoxon P values < 0.05 for all analytes except ferritin for CRS (p = 0.14) and CRP for CRS (p = 0.09)). For CRS, after adjusting tumor burden in a multivariable analysis, MIP-1β, IL-10 and TNF had p < 0.05; for NT, IL-15, IL-6, MIP-1α, and TNF had p < 0.05.

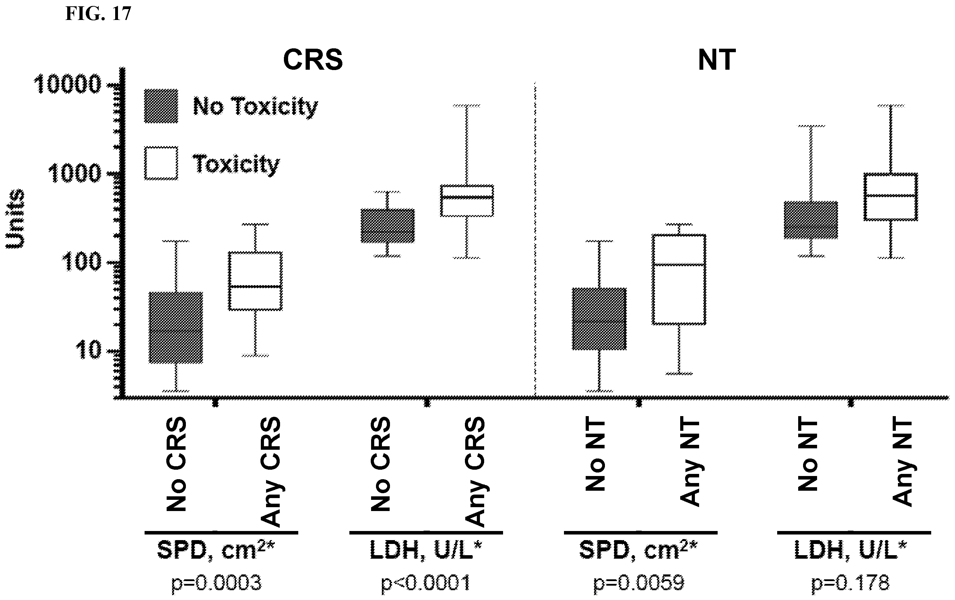

Pre-treatment (pre-LDC) patient parameters, such as levels of lactate dehydrogenase (LDH) and a volumetric tumor measurement such as sum of product dimensions (SPD), as an indicator of tumor burden, were compared between subjects that were not observed to have developed CRS or neurotoxicity versus subjects that were observed to have developed CRS or NT. As shown in **FIG. 17****,** subjects with CRS or NT exhibited higher levels of pre-treatment patient parameters such as SPD (cm²) and LDH (U/L) levels; such levels were observed to be correlated with CRS or NT, with univariate statistical analysis. Other patient parameters that were observed to be associated with CRS and NT include shorter time since diagnosis (p=0.05 and p=0.09, for CRS and NT, respectively). Patient parameters that were observed not to be associated with CRS or NT included age (p=0.19 and p=0.54, respectively) and prior numbers of therapies (p=0.67 and p=0.59, respectively), disease stage 0-2 vs 3-4 (p=0.79, p=0.51), and patient weight (p=0.35 and p=0.44, respectively).

**FIG. 18A** shows pre-treatment SPD and LDH levels among individual patients (dots; with shading of individual dots indicating whether the individual patients did or did and did not exhibit any grade neurotoxicity (left-hand panel) or did or did not exhibit any grade CRS (right-hand panel). In **FIG. 18A****,** dotted lines on the y and x axes delineate SPD ≥50 cm² and LDH ≥500 U/L, respectively. As shown in **FIG. 18A****,** an SPD of approximately 50 cm² or higher, and/or an LDH of approximately 500 U/L or higher, were observed to be associated with risk of NT and CRS. Calculated odds ratio estimates for developing CRS or NT in subjects above or below the SPD and LDH levels indicated by dotted lines in **FIG. 18A****,** with 95% confidence intervals (CI), are depicted in **FIGS. 18B** and **18C****.** An odds ratio over 1 indicated an increased probability or likelihood of developing CRS or NT. As shown, SPD of 50 cm² or higher, and LDH of 500 U/L or higher, were observed to be associated with increased risk of developing CRS or NT. SPD of 50 cm² or higher and LDH of 500 U/L or higher was observed to be associated with an approximately 8-fold increased risk in developing any grade CRS and NT, and SPD of lower than 50 cm² and LDH of lower than 500 U/L showed a reduced risk of any grade CRS and NT. The results were consistent with an association of baseline patient parameters, including high tumor burden and inflammatory biomarkers, with CAR⁺ T cell expansion and increased rates of CRS and neurotoxicity.

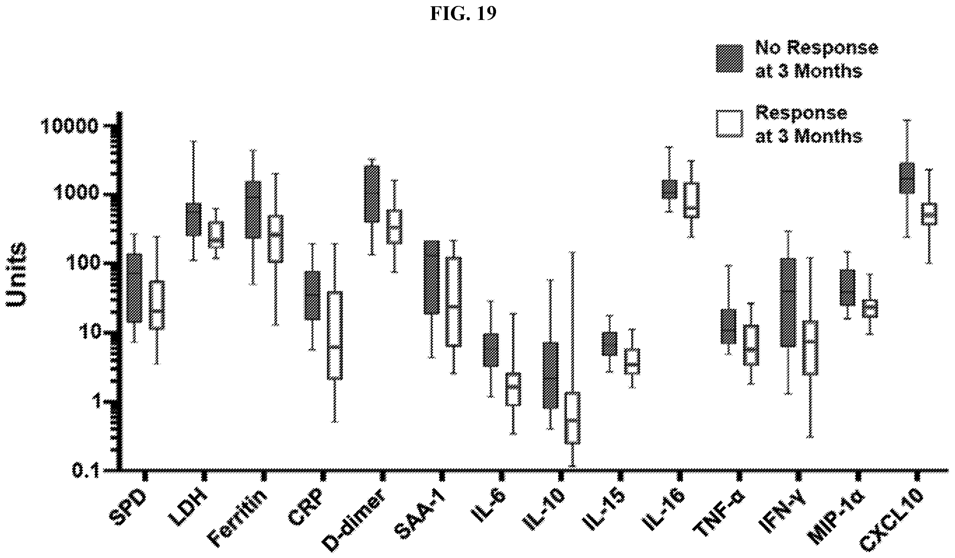

Various pre-treatment (pre-LDC) patient parameters, including markers associated with tumor burden (SPD), inflammatory cytokines and other blood analytes, including LDH, ferritin, CRP, D-dimer, SAA-1, IL-6, IL-10, IL-15, IL-16, TNF-α, IFN-γ, MIP-1α and CXCL10, were compared for subjects with and without a durable response at 3 months, with univariate statistical analysis. As shown in **FIG. 19****,** certain markers of tumor burden, markers of inflammation or inflammatory cytokines were observed to be lower in subjects that exhibited a durable response (p value < 0.05 for all parameters except SPD (p = 0.1274)). Similar results were observed in subjects receiving DL2, when analyzed alone. An inverse association of baseline patient parameters, including high tumor burden and inflammatory biomarkers, with durable response was observed. In some aspects, such inverse association may be due to higher expansion and exhaustion of CAR⁺ T cells.

Relationships between patient factors, clinical correlates and blood analytes to developing of degrees of CRS and NT were assessed using statistical analysis based on univariate nonparametric tests.

**Table E13** lists the results of the univariate analysis. In this assessment, age < 40 years and no prior HSCT correlated with incidence of CRS or NT. Subjects with age < 40 years were not observed to have statistically different rates of higher tumor burden than older patients. Subjects with ECOG score of 2 did not have statistically different rates of higher tumor burden compared to subjects with ECOG score 0-1. Those without prior HSCT or double/triple hit or double expressor were not associated with CRS or NT.

| **Table E13. Univariate Analysis of Key Subgroups** | | | | | | | |
|---|---|---|---|---|---|---|---|
| | | **CRS** | | | **NT** | | |
| **Variable, n (%)** | | **Any Grade** | **Grade 1/2** | **Grade 3/4** | **Any Grade** | **Grade 1/2** | **Grade 3/4** |
| FULL Population (N = 91) | | | | | | | |
| Age | | | | | | | |
| | < 40 years (n = 8) | 5 (63) | 4 (50) | 1 (13) | 3 (38) | 0 | 3 (38) |
| | 40-64 years (n = 49) | 19 (39) | 19 (39) | 0 | 9 (18) | 5 (10) | 4 (8) |
| | ≥ 65 years (n = 34) | 8 (24) | 8 (24) | 0 | 5 (15) | 1 (3) | 4 (12) |

| Pre-LD ECOG PS | | | | | | | |
|---|---|---|---|---|---|---|---|
| | 0-1 (n = 81) | 28 (35) | 27 (33) | 1 (1) | 15 (19) | 4 (5) | 11 (14) |
| | 2 (n = 10) | 4 (40) | 4 (40) | 0 | 2 (20) | 2 (20) | 0 |

| Double/triple hit or double expressor | | | | | | | |
|---|---|---|---|---|---|---|---|
| | Yes (n = 30) | 12 (40) | 12 (40) | 0 | 6 (20) | 3 (10) | 3 (10) |
| | No (n = 22) | 6 (27) | 6 (27) | 0 | 4 (18) | 2 (9) | 2 (9) |

| Prior HSCT | | | | | | | |
|---|---|---|---|---|---|---|---|
| | Yes (n = 39) | 10 (26) | 10 (26) | 0 | 5 (13) | 4 (10) | 1 (3) |
| | No (n = 52) | 22 (42) | 21 (40) | 1 (2) | 12 (23) | 2 (4) | 10 (19) |

### K. Peak Blood Analytes, Response and Toxicity

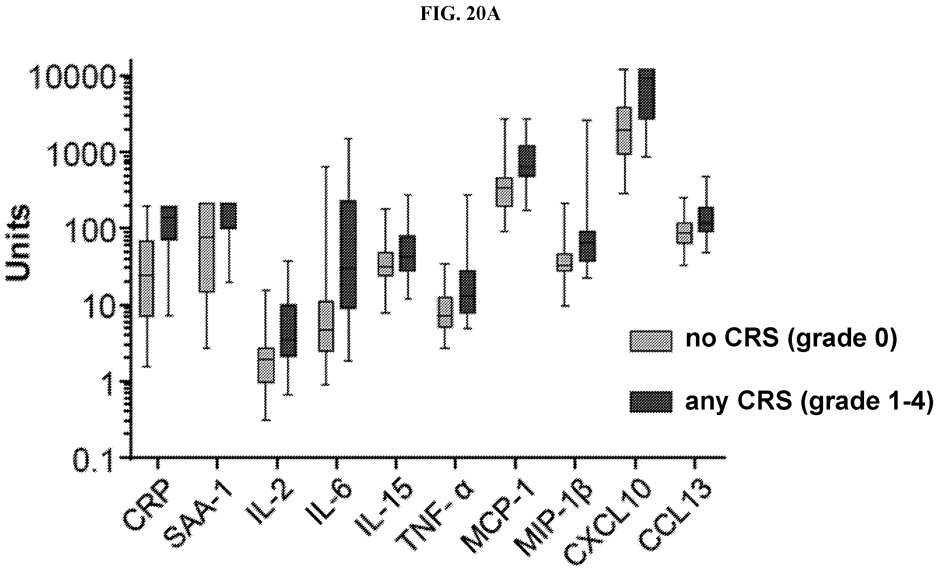

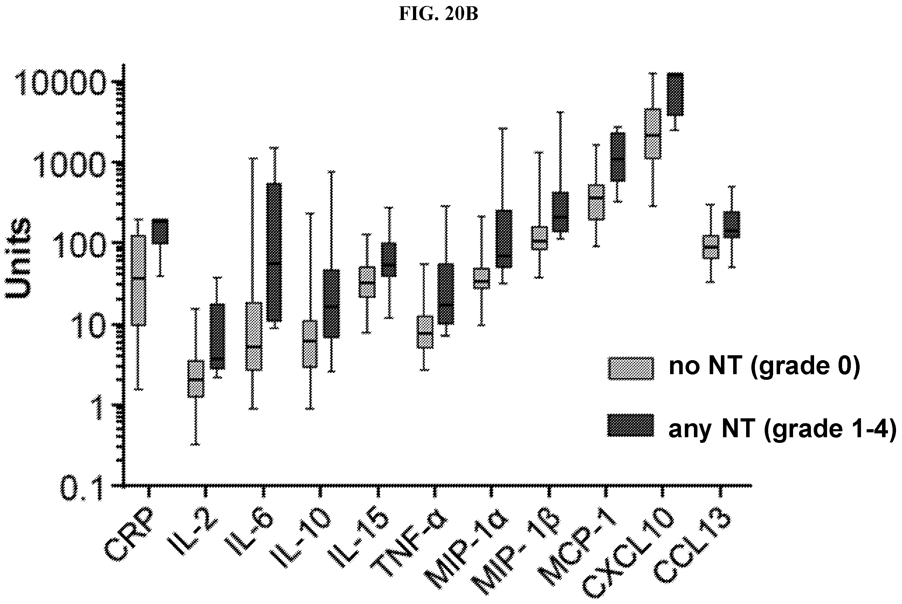

Peak post-treatment plasma levels of blood analytes, including cytokines and inflammatory markers such as CRP, Serum Amyloid A1 (SAA-1), IL-2, IL-6, IL-10, IL-15, TNF-α, MIP-1α, MIP-1β, MCP-1, CXCL10 and C-C Motif Chemokine Ligand 13 (CCL13) were compared for subjects with grade 1-4 cytokine release syndrome (CRS) or neurotoxicity (NT) to subjects that were not observed to have any CRS or NT. As shown in **FIG. 20A** (CRS; CRS grade 0, n=51; CRS grades 1-4, n=28) and **FIG. 20B** (NT; NT grade 0, n=63; NT grades 1-4, n=16), higher peak plasma cytokine levels and inflammatory marker levels were observed to be associated with CRS and NT (Wilcoxon P values < 0.001 for no CRS vs. any CRS and for no NT vs. any NT, except IL-15 (P= 0.05 and 0.006, respectively)).

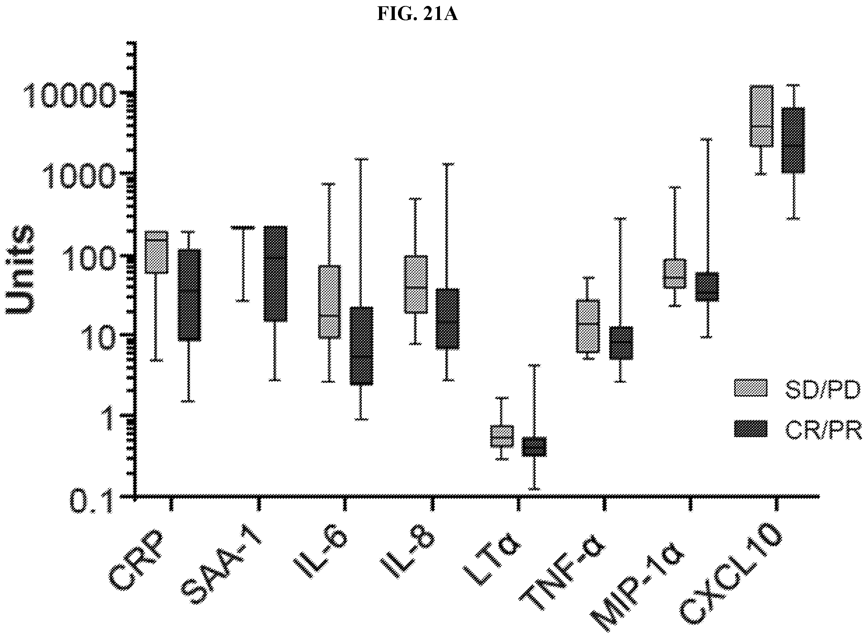

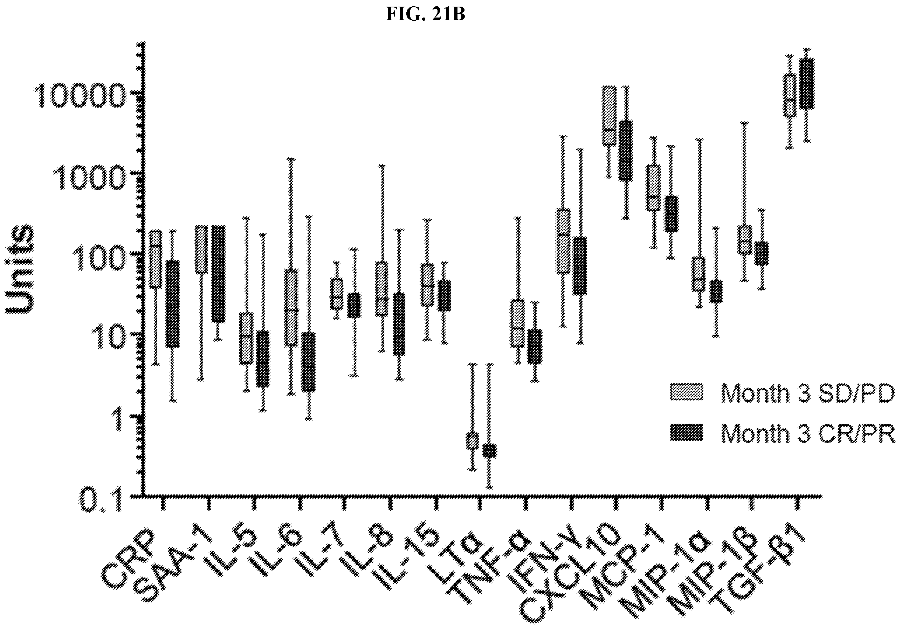

Peak plasma levels of blood analytes, including cytokines and inflammatory markers such as CRP, SAA-1, IL-5, IL-6, IL-7, IL-8, IL-15, Lymphotoxin-alpha (LT-α), TNF-α, IFN-γ, MIP-1α, MIP-1β, MCP-1, CXCL10, and Transforming growth factor beta (TGF-β), were assessed for subjects with a best overall response (BOR) of complete response (CR) or partial response (PR) (N=57) compared to levels in subjects with stable disease (SD) or progressive disease (PD) (N=17); or for subjects with a 3-month SD or PD (SD/PD) (N=31), compared to subjects who exhibited CR/PR at 3-months (N=35). As shown in **FIG. 21A** (best overall response (BOR)) and **FIG. 21B** (month 3 response), lower peak plasma cytokine levels and inflammatory marker levels were observed to be associated with better BOR and response at month 3 (Wilcoxon P values < 0.05 without multiplicity of adjustment).

In this study, administration of the anti-CD19 CAR⁺ cell compositions was administered to subjects with relapsed/refractory aggressive non-Hodgkin lymphoma (NHL) that have poor-risk disease features. Responses, including durable responses, were observed, including 81% ORR, 63% CR at DL2, with 80% of patients in CR at 3 months remaining in CR at 6 months at all dose levels, median DOR of subjects treated at all dose levels of 9.2 months, with medium duration of CR not having been reached at the time point of analysis in this example. The results also were consistent with manageable toxicity levels and a favorable safety profile that in some embodiments may be consistent with outpatient administration. Low rates of severe CRS (1%) and severe neurotoxicity (12%) were observed, with few events in first 72 hours. Results were consistent with feasibility of outpatient administration.

Pharmacokinetic assessments showed that higher expansion of CAR⁺ T cells was generally associated with increased rates of CRS and NT. Subjects receiving DL2 showed higher CAR T exposure compared to subjects receiving DL1, which generally corresponded to increased durability of response without increased incidence of toxicity. In some aspects, pre-treatment, such as pre-LDC, patient factors, including homeostatic and inflammatory cytokines and tumor burden, were observed to be associated with and/or drive very high expansion and toxicity. The administered CAR⁺ T cells were shown to expand in the blood and bone marrow of all patients, with variability among subjects and between disease types. The administered CAR⁺ T cells also exhibited long-term persistence, with 75% (9/12) of evaluable patients having detectable CAR T cells at 12 months. CAR T cells and B cell aplasia were observed to be still present at time of relapse (11/12 and 12/12 patients, respectively), supporting that tumors may evade CAR T cell action and that combination strategies may be effective to prevent relapse or augment, boost or enhance exhausted CAR T cells. In general, a trend of higher response was observed with higher expansion, with variability among subjects, supporting that other patient factors and/or disease characteristics, e.g., tumor burden, may be contribute to determining response.

### Example 4 Attributes of Therapeutic T Cell Composition for Administration and Process for Generation of Composition

Exemplary therapeutic T cell compositions containing autologous T cells expressing a chimeric antigen-receptor (CAR) specific for CD19, used for administration in Examples 1 and 2 above were assessed for greater than one hundred phenotypic, functional, and cell health related attributes, using flow cytometry and in vitro assays. Therapeutic cell compositions generated for subjects enrolled in a clinical study evaluating anti-CD19 CAR-T cell therapy for treatment of relapsed/refractory B-cell non-Hodgkin lymphoma were examined (N=63; core cohort). Cells were assessed before and after engineering, for various attributes. Exemplary attributes that were assessed are set forth in **Table E14.** Memory and cell health phenotypes of the CAR T cells were examined using flow cytometry. T cell functionality was assessed using in vitro antigen-specific bioassays. Characterization and release testing was conducted on therapeutic T cell compositions that had undergone a representative number of freeze-thaw cycles.

| **Table E14: Representative characterization attributes measured in therapeutic cell compositions containing anti-CD19 CAR T cells** | |
|---|---|
| **Cell Composition Characterization Class** | **Representative Attribute** |
| Cell Health | Viability |
| | Active intracellular caspase-3 |
| | Annexin V |
| Memory phenotype | CCR7 (C-C chemokine receptor type 7) |
| Cell function | Inflammatory cytokines such as TNF-α (tumor necrosis factor α) |

For generation of cell compositions for administration, autologous cells were isolated from the subjects via leukapheresis. Leukapheresis samples were subjected to a process for generation of CAR-expressing cells. The process involved washing of cells using an automated wash and immunoaffinity based selection for purification of CD4⁺ and CD8⁺ T cells, resulting in two compositions, enriched for CD8⁺ (in which a median of 99%, Inter Quartile Range (IQR) 98-100%, of cells were CD8⁺) and CD4⁺ (in which a median of 99%, IQR 99-100%, cells were CD4⁺) cells, respectively.

Cells of the enriched CD4⁺ and CD8⁺ compositions were separately subjected to lentiviral transduction with a vector encoding an anti-CD19 CAR with a 41BB costimulatory domain. Transduced populations then were separately incubated in the presence of stimulating reagents for cell expansion. Expanded CD8⁺ and CD4⁺ cells were formulated and cryopreserved separately and stored prior to administration. To minimize variations, between lots and/or cell compositions derived from different patients, such as those having different patient attributes, in parameters indicative of cell health, cells were held at constant volumes across lots. Cell products exhibited a tight range of viable cell concentrations (based on an assessment of cell compositions for one group of subjects, CD8⁺: median 31 x 10⁶ cells/mL, IQR 28-40 x 10⁶ cells/mL, N=38; CD4⁺: median 35 x 10⁶ cells/mL, IQR 31-40 x 10⁶, N=36).

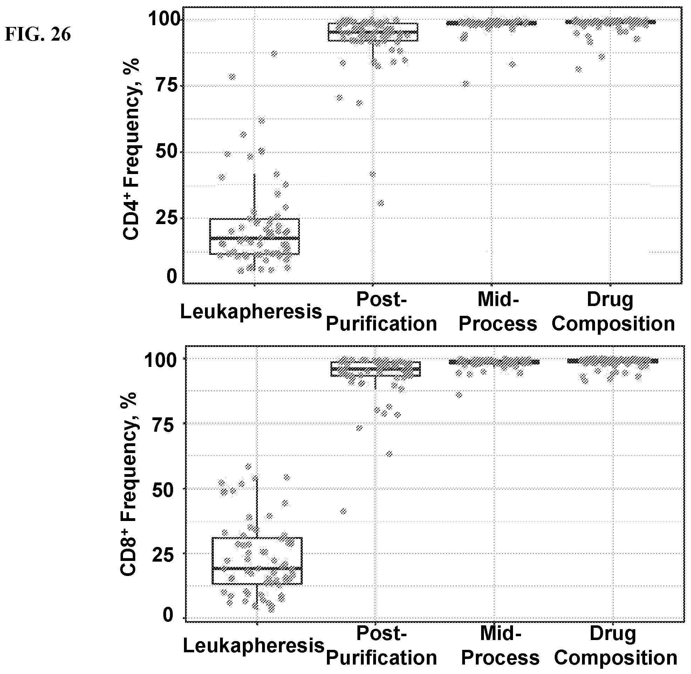

As shown in **FIG. 26** and summarized in **Table E15,** the automated T cell purification resulted in pure CD8⁺ and CD4⁺ T cell populations. This strategy reduced the probability of transducing non-T cells and resulted in high T cell purities in therapeutic cell composition independent of the expansion duration.

| **Table E15: T cell purity (% of total leukocytes) by process step** | | | | | |
|---|---|---|---|---|---|
| | | **Leukapheresis** | **Post-Purification** | **Mid-Process** | **Drug Composition** |
| **CD4⁺ T Cell frequency** | **Median** | 17.6 | 95.3 | 98.9 | 99.2 |
| | **IQR** | 11.7-24.7 | 92.1-98.5 | 98.1-99.2 | 98.7-99.6 |
| **CD8⁺ T Cell frequency** | **Median** | 19.2 | 96.0 | 98.9 | 99.3 |
| | **IQR** | 13.2-30.9 | 93.4-98.6 | 98.0-99.2 | 98.4-99.7 |

At the site of administration, cell compositions were thawed and administered separately, according to a target volume of each composition corresponding to the number of CD8⁺CAR⁺ and CD4⁺CAR⁺ cells in the appropriate dose (such as for DL1, containing 5 x 10⁷ total CAR-expressing T cells (2.5 x 10⁷ each of CAR-expressing CD4⁺ and CAR-expressing CD8⁺ cells), or DL2, containing 1 x 10⁸ total CAR-expressing T cells (5 x 10⁷ each of CAR-expressing CD4⁺ and CAR-expressing CD8⁺ cells)).

During the clinical trial there was a process change from a high-volume formulation to low-volume formulation. The post change therapeutic cell composition was formulated at a constant low volume, with a tightly controlled range of viable cell concentrations. In some cases, a low-volume formulation was used instead of a high-volume formulation. Parameters indicative of health of the CAR-expressing T cells in the compositions for administration were assessed, such as by measuring, postthaw, viability, cell surface Annexin V expression and levels of active intracellular Caspase 3, in cell compositions that were formulated with high volume and low volume.

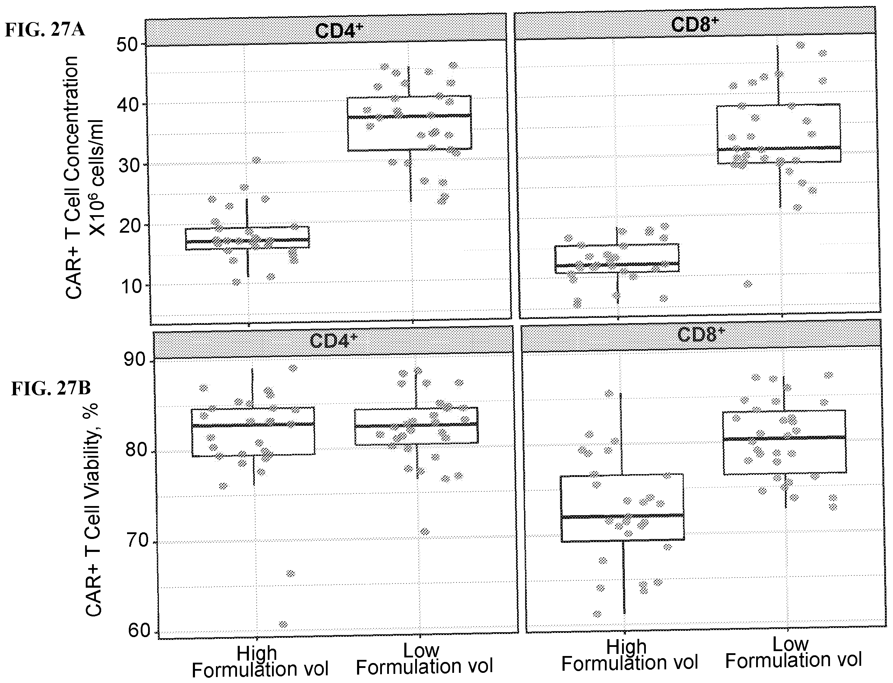

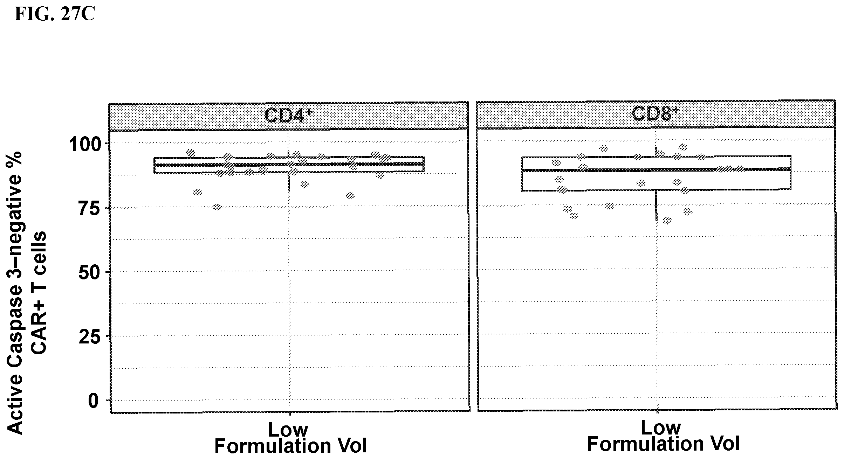

The process change from the high-volume formulation to the low-volume formulation resulted in increased process robustness and decreased variability of the cell health attributes. Values for concentration, percentage of viable cells, and percentage of active caspase-3 negative cells among CD4⁺ and CD8⁺ T cells from individual cell compositions are shown in **FIGS. 27A-27C** and are summarized in **Table E16.** The median percentage of Annexin V-expressing cells was 11% (IQR 9-18%; N=33) of CD8⁺CAR⁺ T cells and 10% (IQR 8-17%; N=31) of CD4⁺CAR⁺ T cells. Caspase 3 expression was observed to be similar to Annexin V expression. The shift to low-volume formulation resulted in increased robustness of the process and reduced the variance of cell health attributes.

| **Table E16: Cell Health Attributes** | | | | | |
|---|---|---|---|---|---|
| | | **CD4⁺** | | **CD8⁺** | |
| | | **High Formulation Volume** | **Low Formulation Volume** | **High Formulation Volume** | **Low Formulation Volume** |
| **Cell Concentration x10⁶ cells/mL** | **Median** | 17.1 | 37.2 | 12.1 | 30.8 |
| | **IQR** | 15.9-19.3 | 31.7-40.4 | 10.9-15.3 | 28.5-38.0 |
| **Cell viability%** | **Median** | 82.8 | 82.5 | 72.0 | 80.3 |
| | **IQR** | 79.5-84.7 | 80.4-84.3 | 69.3-76.6 | 76.4-83.3 |
| **% Caspase-3 negative cells** | **Median** | | 82.8 | | 82.5 |
| | **IQR** | | 79.5-84.7 | | 80.4-84.3 |

The quantities of CAR⁺CD4⁺ and CAR⁺CD8⁺ T cells in the composition for administration were precisely controlled. The number of cells actually administered to an exemplary set of subjects was observed to be within 8% or less of the target number of cells for a given dose:
- 2.4-2.7 x 10⁷ (target ±8%) CD4⁺CAR⁺ T cells and 2.4-2.7 x 10⁷ (target ±8%) CD8⁺CAR⁺ T cells for subjects administered cells at DL1 (n=48)
- 4.6-5.1 x 10⁷ (target ±8%) CD4⁺CAR⁺ T cells or 4.6-5.1 x 10⁷ (target ±8%) CD8⁺CAR⁺ T cells for subjects administered cells at DL2 (n=20).

The range of administered dose was found to have low variability in a different exemplary set of subjects:
- 48-52 ×10⁶ CD3⁺CAR⁺T cell at DL1 (n =34)
- 96-101×10⁶ CD3⁺CAR⁺T cells at DL2 (n = 29)
- 24-27 x 10⁶ CD4⁺CAR⁺ or CD8⁺CAR⁺ T cells at DL1 (n = 34)
- 46-51 x 10⁶ CD4⁺CAR⁺ or CD8⁺CAR⁺ T cells at DL2 (n = 29).

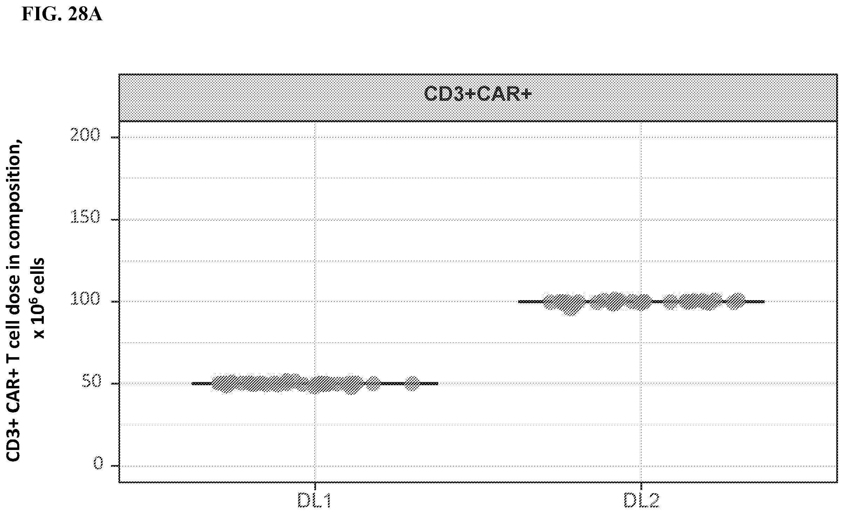

As shown in **FIG. 28A****,** CAR-expressing T cell compositions administered to subjects were observed to exhibit high T cell purity and low variance between lots. In view, e.g., of process and product controls, therapeutic cell compositions containing CAR T cells were observed to have low lot-to-lot variability in cell-specific T cell function.

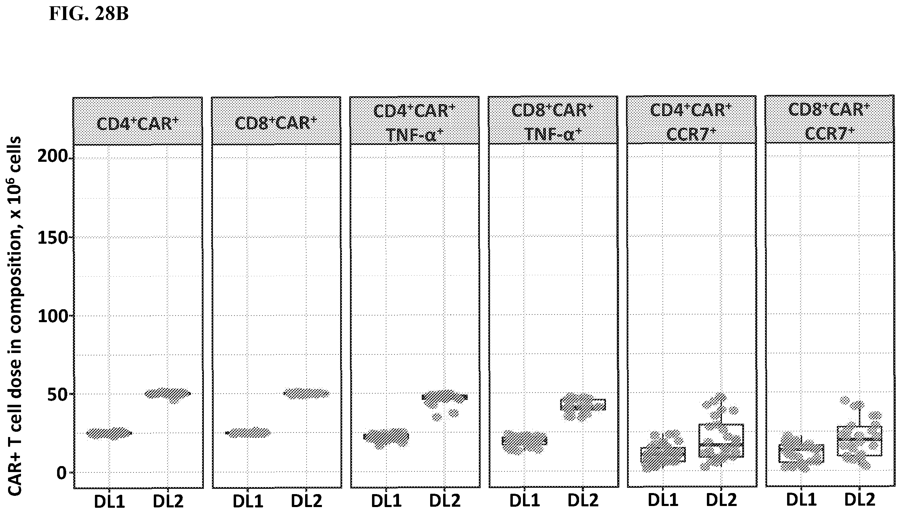

*In vitro* antigen-specific cytokine accumulation and intracellular cytokine staining (ICS) showed a similar low variance between lots for cytokine production for multiple cytokines (IL-2, TNF-α and IFN-y). In an exemplary ICS experiment, cells from compositions were stimulated with CD19, stained for cytokines, including TNF-α, and surface proteins, including C-C chemokine receptor type 7 (CCR7) as a memory phenotype marker, and analyzed by flow cytometry. The number of cells in the composition for administration that were positive for the cytokines or surface proteins were determined. **FIG. 28B** shows the number of CD4⁺CAR⁺ and CD8⁺CAR⁺ cells, CD4⁺CAR⁺TNF-α⁺ and CD8⁺CAR⁺TNF-α⁺ cells, CD4⁺CAR⁺CCR7⁺ and CD8⁺CAR⁺CCR7⁺ and present in CAR T cell compositions for administration at DL1 and DL2. These results are summarized in **Table E17.** The results show low variability in the number of CD4⁺CAR⁺ and CD8⁺CAR⁺ cells, CD4⁺CAR⁺TNF-α⁺ and CD8⁺CAR⁺TNF-α⁺ cells, CD4⁺CAR⁺CCR7⁺ and CD8⁺CAR⁺CCR7⁺ cells. For example, a tight range for the number of cells positive for TNF-α production was observed (n = 61).

| **Table E17: Controlled dose, T cell phenotypes, and cell specific function (x 10⁶ cells)** | | | | | | | | | | | | |
|---|---|---|---|---|---|---|---|---|---|---|---|---|
| | **CD4⁺CAR⁺** | | **CD8⁺CAR⁺** | | **CD4⁺CAR⁺ TNF-α⁺** | | **CD8⁺CAR⁺ TNF-α⁺** | | **CD4⁺CAR⁺ CCR7⁺** | | **CD8⁺CAR⁺ CCR7⁺** | |
| | **DL1** | **DL2** | **DL1** | **DL2** | **DL1** | **DL2** | **DL1** | **DL2** | **DL1** | **DL2** | **DL1** | **DL2** |
| **Median** | 25.1 | 50.1 | 25.0 | 50.0 | 23.0 | 47.4 | 19.9 | 41.7 | 11.1 | 15.8 | 8.9 | 15.0 |
| **IQR** | 24.7-25.3 | 49.6-50.0 | 24.8-25.2 | 49.6-50.4 | 21.3-23.5 | 46.6-48.5 | 18.3-21.8 | 39.9-46.0 | 5.5-14.1 | 11.0-26.0 | 3.0-14.8 | 8.6-25.3 |

A parameter indicative of production by CAR⁺ cells of tumor necrosis factor alpha (TNFα) after stimulation with CD19 showed a narrow range among different lots, with relative standard deviation (RSD) of 37% for CD4⁺CAR⁺ T cells (N=59) and 51% for CD8⁺CAR⁺ T cells (N=61).

The results were consistent with an observation that a composition that contains a precise and consistent dose of CD4⁺ and CD8⁺ CAR T cells, control and optimization of CD4⁺ and CD8⁺ T cell culture conditions, low variability of cytokine production, and/or constant formulation and volume of the composition for administration can lead to consistent cell health in the composition. In provided embodiments, aspects of such manufacturing and control process contribute to low variability in attributes of such cell compositions engineered using cells from, and generated for administration to, a number of different subjects. Such aspects in some aspects include the use of a precise, consistent flat dose of administered CD4⁺ and CD8⁺ cells among subjects; control and optimization of CD4⁺ and CD8⁺ T cell culture conditions such as those that result in low between-drug product lot variability of phenotypes (e.g., CCR7) and in vitro function (e.g., IL-2, TNF-α and IFN-γ production after antigen stimulation) such as among different subjects; and the use of constant formulation and volume of drug product which can result in or contribute to consistency among therapeutic cell compositions generated by the method in attributes indicative of cell health.

### Example 5 Biomarker Assessment in Pre- and Post-administration Tumor Biopsies from Subjects with Relapsed and Refractory Non-Hodgkin's Lymphoma (NHL) for Administration of Anti-CD19 CAR-Expressing Cells

Expression of several biomarkers was assessed in tumor biopsies collected from subjects before and/or after administration of CAR-expressing cells.

### A. Tumor Biopsy Samples

Tumor biopsies were collected from selected subjects with relapsed or refractory (R/R) diffuse large B-cell lymphoma (DLBCL) or mantle cell lymphoma (MCL) who received treatment with therapeutic CAR⁺ T cell compositions containing autologous T cells expressing a chimeric antigen-receptor (CAR) specific for CD19, described above in Examples 1 and 2 above, based on the time point of assessment in Example 1.A.2. Tumor biopsies were obtained prior to administration of the CAR⁺ T cells (pre-treatment) and at 7 to 20 days after administration (post-treatment). Results are described in this example for evaluation through the time-point in Example 1.A.2, in an ongoing study. Results from 43 biopsies (26 pre-treatment; 17 post-treatment and 15 matched pairs) from 28 total subjects (25 DLBCL and 3 MCL) were examined.

### B. Assessment of Biomarkers, Response and Safety Outcomes

Infiltration of CAR⁺ T cell in the tumor biopsy was quantified using in situ hybridization (ISH) probes specific to the mRNA encoding the anti-CD19 CAR. CAR⁺ T cells, non-CAR T cells and B cells were enumerated using multiplex immunofluorescence (IF) assays detecting for a cell surface surrogate marker for CAR-expressing cells, CD4, CD8, CD19, CD20, CD73, FOXP3, CD163, IDO and PD-L1. Tumor biopsy sections were stained with hematoxylin and eosin (H&E) and assessed for tissue quality and tumor identification. Immunofluorescence images were analyzed using an image analysis software. Potential correlations to response outcomes were assessed using statistical analysis based on univariate t-tests, and the p-values were 2-sided without multiplicity adjustment.

Subjects were assessed for response and safety outcomes, including by assessing the tumor burden at various time points after administration of the CAR⁺ T cells, including at 3 months after administration, and determining whether the subject had progressive disease (PD), stable disease (SD), partial response (PR), or complete response (CR). Safety outcomes evaluated included neurotoxicity (neurological complications including symptoms of confusion, aphasia, encephalopathy, myoclonus seizures, convulsions, lethargy, and/or altered mental status), graded on a 1-5 scale, according to the National Cancer Institute-Common Toxicity Criteria (CTCAE) scale, version 4.03 (NCI-CTCAE v4.03).

### C. Results

The observed objective response rate (ORR; including CR and PR) was 71% (20/28) in the subjects for which biopsies were assessed. Grade 1, 2 CRS was observed in 36% (10/28; grade 1, 2) of the subjects for which biopsies were assessed, and Grades 2-4 NT was observed in 18% (5/28) of the subjects for which biopsies were assessed.

Pre-treatment tumor biopsies were observed to contain varying cellular compositions: tumor cells (median: 77%; range 5-96%), CD4⁺ cells (0.90%; 0.02-15%), and CD8⁺ cells (1.5%; 0-23%). The results showed that subjects with a CR or a PR at 3 months after CAR⁺ T cell administration had a higher percentage of endogenous CD4⁺ cells in pre-treatment tumors compared those with a PD (CR, PR median: 7.9%; PD median: 0.38%; p < 0.0001). Percentages of CD8⁺ cells in pre-treatment tumors did not differ between the 3 month response groups (CR, PR median: 1.9%; PD median: 0.47%; p = 0.6496).

In the post-treatment biopsies, CAR⁺ T cell were observed to have infiltrated the tumor, and constituted up to 22% of cells in the biopsy sample. The level of tumor infiltration in post-treatment samples (7 to 20 days after administration) was observed to be higher in subjects that went on to achieve a CR (median: 3.9%) or a PR (median: 1.1%) compared to subjects that went on to achieve a best overall response (BOR) of SD or PD (median: 0.51%). Although both CD4⁺ and CD8⁺ CAR T cells were observed to have infiltrated the tumor area at the post-treatment time point (7 to 20 days after administration), subjects that went on to achieve a CR were observed to have higher ratio of CD8⁺ CAR⁺ T cells to CD4⁺ CAR⁺ T cells, at this post-treatment timepoint, as compared to subjects that went on to achieve a BOR of SD or PD (CR median: 0.83; SD, PD median: 0.14; p = 0.0097).

Comparing matched pre- and post-treatment biopsies from individual subjects, results showed a trend towards subjects ultimately achieving a BOR of CR or PR having a larger post-treatment increase in CD8⁺ cells (CAR⁺ T and non-CAR T) in tumors, as compared to subjects ultimately achieving a BOR of SD or PD (CR, PR median change: +5.3%; SD, PD median change: +0.06%; p = 0.1225).

Expression of immunosuppressive factors, including CD73, FOXP3, CD163, IDO and PD-L1, varied among subjects at pre-treatment (CD73 (median: 1.5%; range 0-42%), FOXP3 (0.10%; 0-1.5%), IDO (0.06%; 0-11%), CD163 (1.2%; 0-24%) and PD-L1 (0.16%; 0-56%)) and post-treatment (CD73 (1.6%; 0-53%), FOXP3 (0.09%; 0-4.3%), IDO (0.28%; 0-15%), CD163 (3.6%; 0-22%) and PD-L1 (3.3%; 0-65%)). Post-treatment increases in CD8⁺ cells in matched biopsies were observed to be associated with post-treatment increases in IDO (R² = 0.64) and PD-L1 (R² = 0.61) expression. This result is consistent with a conclusion that infiltration of CD8⁺ CAR⁺ cells at the time assessed may indicate potential likelihood of achieving a degree of response or duration of response, and that the presence and/or activity of such cells may result in upregulation of TME factors.

### D. Conclusion

Durable response at month 3 after CAR⁺ T cell administration was observed to be associated with higher levels of CD4⁺ cells in pre-treatment tumors. In post-treatment tumor cells, CAR⁺ T cells, both CD4⁺ and CD8⁺, were observed to infiltrate the tumor and adjacent tissue. ORR was associated with an increase in CAR⁺ T cells in the tumor biopsy. An increase of CD8⁺ levels in the post-treatment tumor biopsy compared to CD8⁺ levels in the pre-treatment tumor biopsy was associated with increased IDO and PD-L1 expression. In some embodiments, therapies targeting these pathways, such as those administered at the time of or following administration of the CAR-T cells, may enhance one or more therapeutic outcomes or duration thereof following CAR⁺ T cell administration.

### Example 6 Further Assessment of Response and Safety Outcomes in Subjects with Relapsed and Refractory Non-Hodgkin's Lymphoma (NHL) After Administration of Anti-CD19 CAR-Expressing Cells

Response and safety outcomes were assessed in patients at a subsequent point in time in the clinical study described in Examples 1 and 3 above.

### A. Subjects and Treatment

The analysis at this time point presented in this example is based on assessment of a total of 102 subjects in the FULL cohort (73 in the CORE cohort) that had been administered the anti-CD19 CAR-expressing cells. The FULL cohort included subjects who had DLBCL (DLBCL, NOS de novo and transformed from follicular lymphoma; high grade B-cell lymphoma (double/triple hit); DLBCL transformed from CLL or MZL; PMBCL; and FL3B, ECOG 0-2, after 2 lines of therapy; the CORE cohort for analysis included subjects having DLBCL, NOS and transformed from follicular lymphoma (tFL) or high grade B-cell lymphoma (double/triple hit) and with Eastern Cooperative Oncology Group performance status (ECOG PS) of 0 or 1. Subjects with MCL after 1 line of therapy also were included in the study. Approximately 90% of treated patients in the FULL and the CORE cohort had at least one poor-risk disease feature predictive of short median overall survival (OS) of 3-6 months (see Crump et al., Blood (2017) 130:1800-1808 and Van de Neste et al., Bone Marrow Transplant. (2016) 51(1):51-7), such as double/triple hit expressors, primary refractory disease, refractory to 2 or more lines of therapy, never achieved CR, never received autologous stem cell transplant (ASCT) or an ECOG PS of 2.

At this time point, a total of 134 subjects had been leukapheresed, of which 2 had compositions unavailable. Product was available for 99% of apheresed subjects (132/134) in the DLBCL cohort. Of another 18 subjects whose products were available, 5 had withdrawn, and 13 had developed progressive disease or had died. A total of 114 subjects had been administered the anti-CD19 CAR-expressing cells, of which 12 received non-conforming anti-CD19 CAR-expressing cells (compositions not necessarily meeting certain specifications but deemed to be safe for administration). Subjects had received DL1 (n=45), double dose of DL1 (n=6) or DL2 (n=51). Seven (7) subjects with mantle cell lymphoma (MCL) had been administered CAR⁺ cells at DL1. At this time point, eight (8) subjects were treated in an outpatient setting.

The demographics and baseline characteristics of the FULL and CORE cohort subjects at the timepoint are set forth in **Table E18.**

| **Table E18. Patient Characteristics: DLBCL Cohort** | | | |
|---|---|---|---|
| **Characteristic** | | **FULL (n = 102)** | **CORE (n = 73)** |
| Median age (range), years | | 61 (20-82) | 60 (20-82) |
| | ≥0 (20-82) (range | 37 (36) | 24 (33) |

| **B-NHL Subtype, n (%)** | | | |
|---|---|---|---|
| DLBCL, NOS de novo | | 63 (62) | 53 (73) |
| Transformed from FL (tFL) | | 23 (23) | 20 (27) |
| Transformed from MZL (tMZL) /CLL (tCLL) | | 6 (6)/6 (6) | 0 |
| Follicular, grade 3B/PMBCL | | 1 (1)/3 (3) | 0 |

| Molecular **Subtype,** n **(%)** | | | |
|---|---|---|---|
| Double/triple hit | | 19 (19) | 16 (22) |

| **Patient Characteristics, n (%)L** | | | |
|---|---|---|---|
| ECOG PS 0-1 | | 93 (91) | 73 (100) |
| IPI 3-5 | | 43 (42) | 26 (36) |
| CNS involvement | | 2 (2) | 1 (1) |
| Chemorefractory^{b} | | 71 (70) | 49 (67) |
| Prior lines of therapy, median (range) | | 3 (1-8) | 3 (2-8) |
| Never achieved CR | | 49 (48) | 36 (49) |
| Any HSCT | | 41 (40) | 28 (38) |
| | Prior autologous | 38 (37) | 28 (38) |
| | Prior allogeneic | 5 (5) | 0 |

| | | | |
|---|---|---|---|
| HSCT, hematopoietic stem cell transplant. IPI, International Prognostic Index; SD, stable disease; WHO, World Health Organization. ^{a} At trial initiation, included in DLBCL, NOS histology; based on most recent WHO criteria (Swerdlow et al., (2016) Blood 127(20):2375-2390), are now considered high-grade B-cell lymphoma, with *myc* and *bcl2* and/or *bcl6* rearrangements with DLBCL histology (double/triple hit). ^{b} SD or PD to last chemotherapy-containing regimen or relapse < 12 months after autologous SCT. | | | |

### B. Safety and Response Outcomes after Treatment

**Table E19** shows the safety outcome of the FULL and CORE cohort. As shown, no deaths from CRS or NT were observed. In the FULL cohort, the median time to onset of CRS was 5 days (range, 2-12 days) and NT was 10 days (range, 3-23 days). In the FULL cohort, 17% (n = 17) received tocilizumab and 21% (n = 21) received corticosteroids as a toxicity intervention. In the CORE cohort, no increase in CRS or NT was observed at DL2, compared to DL1.

| **Table E19. Safety Outcomes After CAR⁺ Cell Administration** | | | | | |
|---|---|---|---|---|---|
| | | **FULL** | **CORE** | | |
| | | **All Dose Levels n = 102** | **a All Dose Levels n = 73** | **DL1S n = 33** | **DL2S n = 37** |
| **CRS, n (%)** | | | | | |
| | Any grade | 38 (37) | 27 (37) | 14 (42) | 11 (30) |
| | Grade ½ | 37 (36) | 26 (36) | 13 (39) | 11 (30) |
| | Grade 3/4 (sCRS) | 1 (1) | 1 (1) | 1 (3) | 0 |

| **Neurotoxicity, n (%)** | | | | | |
|---|---|---|---|---|---|
| | Any grade | 23 (23) | 18 (25) | 8 (24) | 9 (24) |
| | Grade ½ | 10 (10) | 7 (10) | 1 (3) | 6 (16) |
| | Grade 3/4 (sNT) | 13 (13) | 11 (15) | 7 (21) | 3 (8) |

| **Any, n (%)** | | | | | |
|---|---|---|---|---|---|
| | CRS or NT | 44 (43) | 32 (44) | 15 (45) | 15 (41) |
| | sCRS or sNT | 13 (13) | 11 (15) | 7 (21) | 3 (8) |

| | | | | | |
|---|---|---|---|---|---|
| ^{a} Three patients treated on DL1D (dose level 1, two-dose schedule) with similar outcomes. | | | | | |

**FIG. 29** depicts the percentage of subjects in the FULL cohort at this timepoint (n = 102) who were observed to have experienced laboratory abnormalities and treatment-emergent adverse events (TEAEs) (data for 6 subjects with MCL treated with conforming product at DL1 with at least 28 days of follow-up are not included; showing TEAEs and laboratory abnormalities occurring in 20% or more of the subjects).

As shown in **Table E20,** high rates of response was observed in subjects with relapsed or refractory (R/R) DLBCL. The results are consistent with a dose response effect on treatment outcome in the CORE cohort. Subjects with a tumor burden above a threshold (as indicated by the volumetric tumor measurement of sum of product dimensions (SPD) of more than 50 cm²) was similarly distributed between subjects receiving DL1 and DL2 (approximately 1/3 of the subjects in each group).

| **Table E20. Response After CAR⁺ Cell Administration** | | | | |
|---|---|---|---|---|
| | **FULL** | **CORE** | | |
| | **All Dose Levels (n=102)** | **a All Dose Levels (n=73)** | **DL1S (n=33)** | **DL2S (n=37)** |
| ORR (95% CI), % | 75 (65-83) | 80 (68-88) | 79 (61-91) | 78 (62-90) |
| CR (95% CI), % | 55 (45-65) | 59 (47-70) | 55 (36-72) | 62 (45-78) |
| 3-mo ORR (95% CI), % | 51 (41-61) | 59 (47-70) | 52 (34-69) | 65 (48-80) |
| 3-mo CR (95% CI), % | 38 (29-48) | 45 (34-57) | 36 (20-55) | 51 (34-68) |
| 6-mo ORR (95% CI), % | 40 (31-50) | 47 (35-59) | 42 (26-61) | 49 (32-66) |
| 6-mo CR (95% CI), % | 34 (25-44) | 41 (30-53) | 33 (18-52) | 46 (30-63) |

| | | | | |
|---|---|---|---|---|
| ^{a} Three patients treated on DL1D (dose level 1, two-dose schedule) with similar outcomes. | | | | |

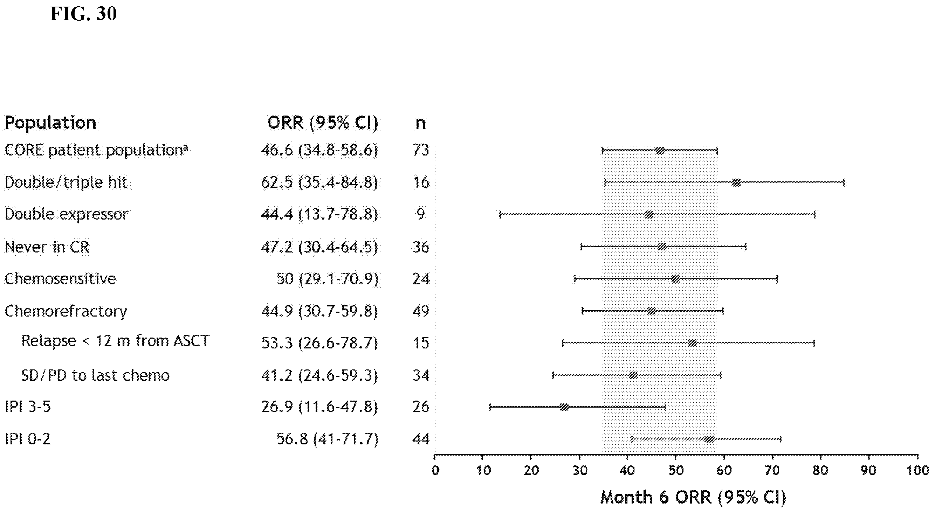

Six-month objective response rates (ORR) among various subgroups of subjects in the poor-risk DLBCL subgroups, that included all DLBCL patients treated at all dose levels in the CORE cohort, are shown in **FIG. 30****.** The results showed high durable ORR in the poor-risk DLBCL subgroup for anti-CD19 CAR+ T cell administration.

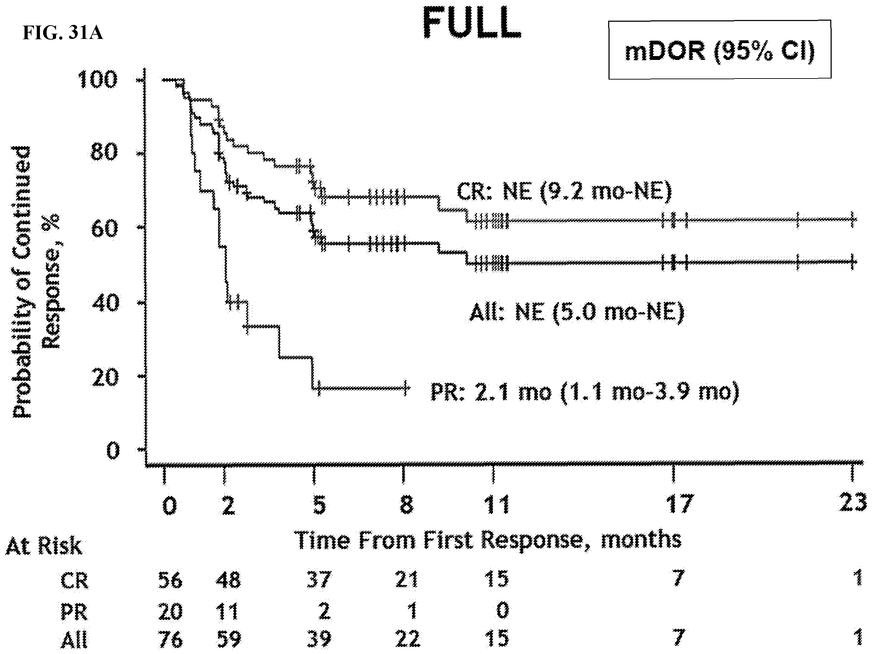

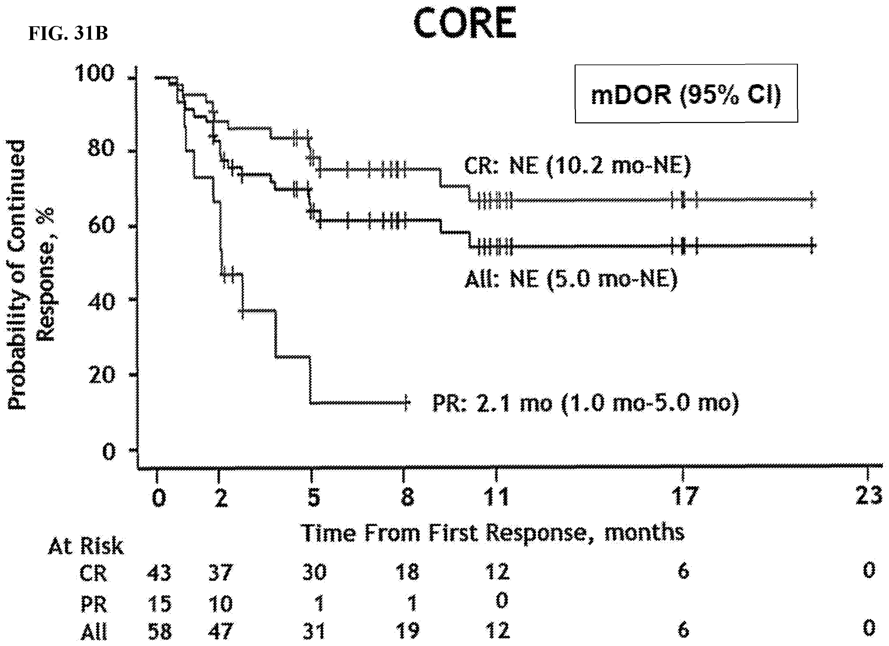

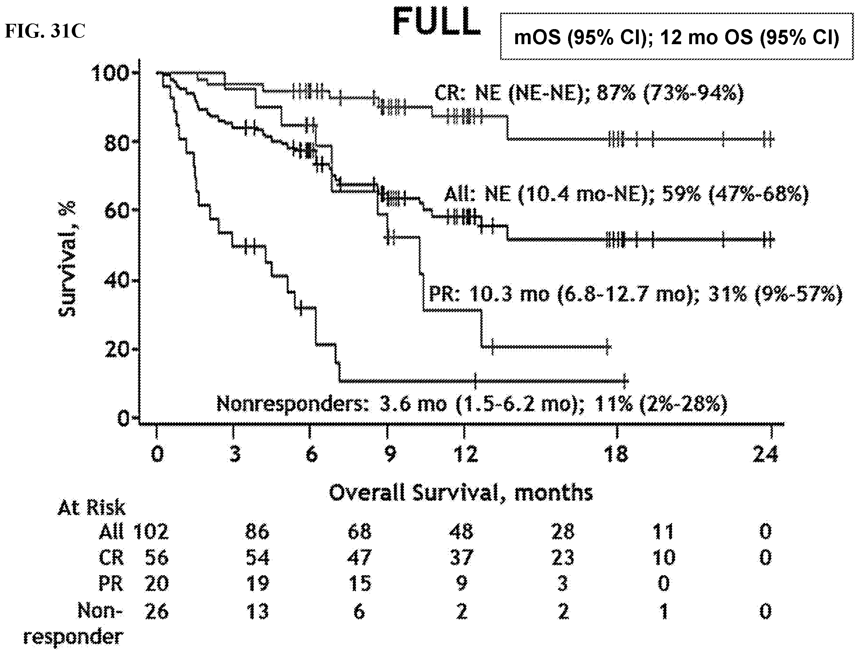

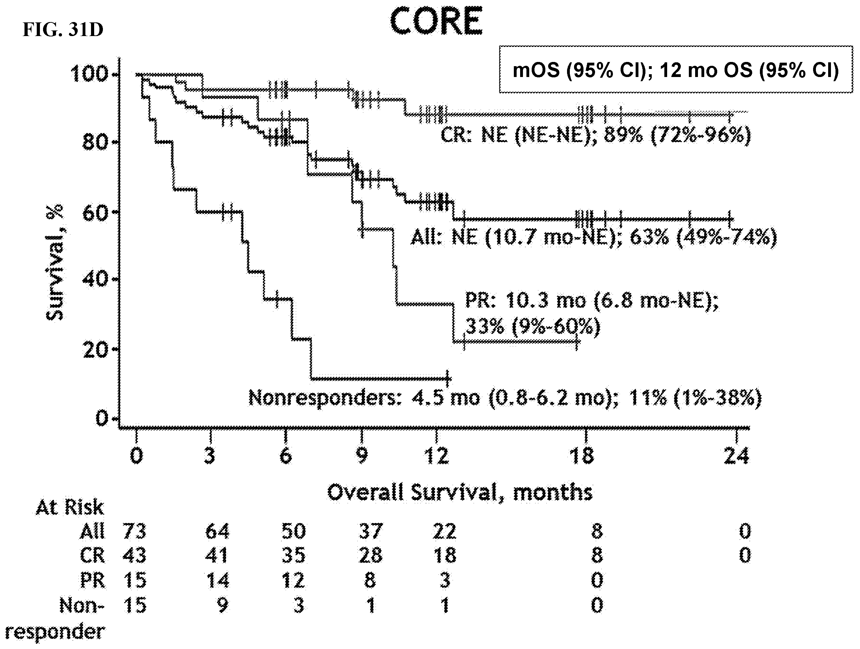

Results for the duration of response (DOR, with median follow-up of 8 months) and overall survival (grouped by best overall response (non-responder, CR/PR, CR and/or PR), with median follow-up of 12 months) are shown for the full cohort and the core cohort cohorts of subjects, in **FIGS. 31A-****31D.** The results showed that in the CORE cohort, 88% of subjects with CR at 3 months continued to show CR at 6 months, and 93% of subjects who exhibited CR at 6 months continued to show a response longer term.

The results were consistent with an observation that administration of anti-CD19 CAR+ cell compositions that contains a precise and consistent dose of CD4⁺ and CD8⁺ CAR+ T cells results in durable response in subjects with R/R aggressive NHL with poor prognosis and/or heavy pretreatment. The results showed a favorable durable response rate in the CORE cohort, with 49% ORR and 46% CR rate at 6 months, and 93% of the subjects (at all dose levels) in CR at 6 months remained in response at this time point. The results also were consistent with manageable toxicity and a favorable safety profile, including low rates of severe CRS (1%) and severe neurotoxicity (13%), which, in some aspects, supports outpatient administration.

### Example 7 Assessment of Resource Utilization and Pre-treatment Parameters and Expansion in Subjects with Relapsed and Refractory Non-Hodgkin's Lymphoma (NHL) After Administration of Anti-CD19 CAR-Expressing Cells

The utilization of various healthcare resources and various pre-treatment parameters, such as blood analytes and disease burden, and cell expansion, were assessed in patients described in the clinical studies described in Examples above in subjects with relapsed/refractory NHL who had received the anti-CD19 CAR-expressing T cell therapeutic compositions.

### Example 7.I.A. Subjects and Treatment

A first analysis was carried out at a specific point in time in the clinical study described in Examples 1, 3 and 6. The analysis at this time point presented in this example is based on assessment of a total of 114 subjects in the FULL cohort. At this time point, 96 subjects had been treated in the inpatient setting, and 18 subjects had been treated in an outpatient setting.

The demographics and baseline characteristics of the subjects treated in the inpatient and outpatient settings at the timepoint are set forth in **Table E21.**

| **Table E21. Patient Characteristics** | | |
|---|---|---|
| **Demographics/Characteristics** | **Inpatient Administration (n=96)** | **Outpatient Administration (n=18)** |
| Median age (range), years | 61 (20-82) | 60 (23-76) |
| Male, n (%) | 66 (69) | 13 (72) |

| **B-NHL Subtype, n (%)** | | |
|---|---|---|
| DLBCL, NOS de novo | 60 (63) | 9 (50) |
| Transformed from FL | 22 (23) | 5 (28) |
| Transformed from MZL/CLL | 9 (9) | 0 (0) |
| Follicular, grade 3B/PMBCL | 2 (2) | 4 (22) |

| **Molecular Subtype, n (%)** | | |
|---|---|---|
| Double/triple hit | 19 (20) | 2 (11) |
| **Patient Characteristics** | 81 (84) | 18 (100) |
| CNS involvement, n (%) | 2 (2) | 0 (0) |
| Chemorefractory, n (%) | 66 (69) | 14 (78) |
| Prior lines of therapy, median (range) | 3 (1-8) | 2 (1-5) |
| Never achieved CR, n (%) | 47 (49) | 9 (50) |
| Any HSCT, n (%) | 39 (41) | 6 (33) |
| Prior autologous | 36 (38) | 6 (33) |
| Prior allogeneic | 5 (5) | 0 (0) |
| CLL, chronic lymphocytic lymphoma; DLBCL, diffuse large B-cell lymphoma; EGOG PS, Eastern Cooperative Oncology Group Performance Score: FL, follicular lymphoma; FL3B, follicular lymphoma grade 3B; MZL, marginal zone lymphoma; NOS, not otherwise specified; PMBCL, primary mediastinal B-cell lymphoma; SCT, stem cell transplantation; IPI, International Prognostic Index. At trial initiation, included in DLBCL, NOS histology; based on most recent WHO criteria,3 are now considered high-grade B-cell lymphoma, with MYC and BCL2 and/or BCL6 rearrangements with DLBCL histology (double/triple hit). b SD or PD to last chemo-containing regimen or relapse <12 months after autologous HSCT. | | |

### Example 7.I.B. Safety Outcome

**Table E22** shows particular safety-related outcomes (incidence of CRS and neurologic events) of the subjects who had been treated by administration in the inpatient or outpatient setting. ICU admission during a CRS or neurological events occurred in 9 subjects (9.4%) receiving inpatient treatment and in 1 patient (5.6%) receiving outpatient treatment.

| **Table E22. Cytokine Release Syndrome and Neurologic Events by Site of Administration** | | |
|---|---|---|
| | **Inpatient Administration (n=96)** | **Outpatient Administration (n=18)** |
| **CRS^{a}, n (%)** | | |
| Any grade | 35 (36.5) | 6 (33.3) |
| Grade 1-2 | 34 (35.4) | 5 (27.8) |
| Grade 3-4 (sCRS) | 1 (1.0) | 1 (5.6) |

| **Neurologic events,^{b} n (%)** | | |
|---|---|---|
| Any grade | 20 (20.8) | 7 (38.9) |
| Grade 1-2 | 8 (8.3) | 5 (27.8) |
| Grade 3-4 (sNE) | 12 (12.5) | 2 (11.1) |

| **Any, n (%)** | | |
|---|---|---|
| CRS or NE | 39 (40.6) | 9 (50) |
| sCRS or sNE | 12 (12.5) | 2 (11.1) |
| CRS, cytokine release syndrome; NE, neurologic event; sCRS, serious CRS; sNE, serious NE. | | |
| ^{a}Graded per Lee, et al. Blood, 2014.7 | | |
| ^{b}Graded per Common Terminology Criteria for Adverse Events (CTCAE), version 4.03. | | |

### Example 7.I.C. Assessment of Biomarkers, Expansion and Resource Utilization

Expression of biomarkers LDH, CRP and ferritin was assessed in tumor biopsies collected from subjects, and tumor burden in the subject was assessed by a volumetric tumor measurement of sum of product dimensions (SPD), before (pre-treatment) administration of CAR-expressing cells. The maximum serum concentration (Cₘₐₓ; CD3+ CAR⁺ cells/µL blood) was also determined in the subjects after administration. The subjects were grouped by SPD, LDH, CRP and Cₘₐₓ at or below, or above, a threshold value, or by inpatient or outpatient treatment, and the association with various parameters indicative of healthcare resource utilization (inpatient days, days in the intensive care unit (ICU), tocilizumab use (agent for toxicity intervention, (%)) and vasopressor, intubation or dialysis use (treatment for toxicity intervention, (%)) was assessed.

**FIGS. 32A-32F** show the use of various healthcare resource parameters in subjects grouped by: Cₘₐₓ of at or below 500 cells/µL or above 500 cells/µL CAR⁺ cells/µL **(****FIG. 32A****);** SPD at or below 50 cm² or above 50 cm² **(****FIG. 32B****);** LDH at or below 500 units/L or above 500 units/L **(****FIG. 32C****);** CRP at or below 10 mg/L and ferritin at or below 5000 ng/mL or CRP above 10 mg/L and ferritin above 5000 ng/mL **(****FIG. 32D****),** and inpatient setting or outpatient setting **(****FIGS. 32E-32F****).**

The result were consistent with reduced healthcare resource utilization (e.g., reduced use of toxicity treatment or intervention) in subjects with lower pre-treatment tumor burden, expression of certain blood analytes such as LDH, CRP and/or ferritin, and/or lower peak CAR+ T cell numbers following treatment. In subjects treated in the outpatient setting, there was a 42% reduction in hospital length of stay, as compared to those treated in the inpatient setting. In general, the use of toxicity intervention, e.g., tocilizumab, intubation, vasopressors, and dialysis) was low in both inpatient and outpatient treatment groups, with the use of vasopressors observed to be higher in outpatient treatment groups. Results were consistent with the feasibility of outpatient administration.

### Example 7.II.

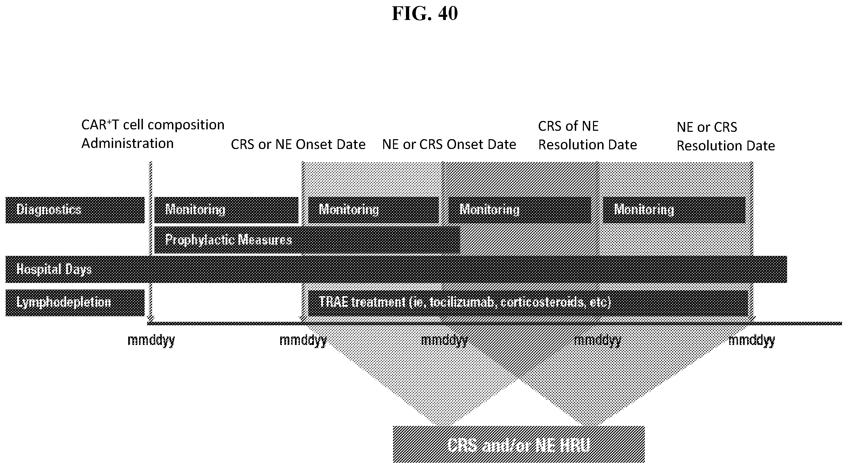

In the same clinical study described in Example 7.I above, incidence, healthcare resource utilization (HRU), and costs of cytokine release syndrome (CRS) and neurological events (NE) were assessed in a subset of 102 subjects who had received a conforming dose of anti-CD19 CAR-expressing CD4/CD8 cell compositions as described in Example 6. Information about patient characteristics, AE incidence, laboratory reports, facility use and prescription information were captured. Healthcare Resource Utilization (HRU) within the dates of onset and resolution of CRS and NEs were identified for each subject using methods as described in **FIG. 40****.** The HRU was identified for each subject. Trial management guidelines were cross-checked to determine whether HRU was CRS- and/or NE-related. Costs associated with the grade of CRS or NE were applied using public databases and literature on national average costs **(Table E22.1).**

Baseline subject demographics and clinical characteristics were assessed by CRS and/or NE profile. Adverse event (AE) incidence was evaluated as described in Example 1, including by grade of CRS and NE by criteria of Lee et al. (2014) and CTCAE, version 4.03, respectively. The occurrence of CRS only, NE(s) only, nonconcurrent CRS and NE(s) (i.e. no overlap between start and end dates) and concurrent CRS and NE(s) was determined.

HRU was determined and costs associated with CRS and NEs were estimated, including based on diagnostics, medication (e.g. Tocilizumab and corticosteroid utilization), and facility utilization (e.g. median length of stay (LOS) by grade of CRS and/or NEs, and intensive care unit (ICU) utilization). Median total cost by grade of CRS and/or NEs was estimated, and analyzed according to CRS and/or NE profile.

| **Table E22.1 Cost Inputs^{a}** | | |
|---|---|---|
| **Input** | **Value (Range)** | **Reference** |
| Cost per office visit | $114 | 2018 OPPS National Reimbursement Rate |
| Cost per inpatient day^{b} | $2,668 | Health Care Utilization Project - National Inpatient Sample 2015 |
| Cost per ICU day^{b} | $6,546 | Estimated per ICU day cost w/o mechanical ventilation^{d} |
| Diagnostic laboratory tests | ($6-$23) | 2018 Clinical Laboratory Fee Schedule by HCPCS code, national reimbursement rate |
| Diagnostic tests | ($360-$486) | 2018 OPPS and Physician Fee Schedule national reimbursement rates |
| Medication/drugs^{c} | ($0.01-$4,914) Per dose | IBM Truven Micromedex RED BOOK^{®}, WAC Price |
| Tocilizumab 80 mg/4-mL vial^{d} | $436.81 | IBM Truven Micromedex RED BOOK^{®}, WAC Price |
| ^{a}These costs were applied to any HRU that met the temporality and guideline requirements from the provider perspective. | | |
| ^{b}Inflated to 2018 US$. | | |
| ^{c}Costs at prescribed amount. | | |
| ^{d}Tocilizumab is dosed at 8mg/kg and subjects may require more than one dose. | | |
| HCPCS, Healthcare Common Procedure Coding System; HRU, healthcare resource utilization; ICU, intensive care unit: OPPS, Outpatient Prospective Payment System; WAC, wholesale acquisition cost. | | |

Patient characteristics by AE profile is shown in **Table E22.2.** Of the 102 subjects assessed, 44 experienced CRS and/or NE(s), 21 subjects (21%) had CRS only (no G≥3), 6 subjects (6%) had NE only (no G≥4), 6 subjects (6%) had nonconcurrent CRS and NE, and 11 subjects (11%) had concurrent CRS and NE (including 1 G4 CRS). Of 38/102 (37%) subjects who experienced CRS, 1 had grade 3/4 CRS. Of 23/102 subjects (23%) who experienced NEs, 10 (10%) were grade 1/2 and 13 (13%) were grade 3/4. Of the subset of 44 subjects, the majority in each adverse event profile had DLBCL not otherwise specified. Of these 44 subjects, 70% had G≤2 events (31 out of 44 subjects). Of the subjects with both CRS and NE, 94% had CRS first (16 out of 17 subjects). Of the subjects with nonconcurrent events, NE developed a median of 2 days after CRS resolved. Of the 10 out of 11 subjects (91%) with concurrent events and CRS before NE, NE developed a median of 3.5 days after CRS onset.

| **Table E22.2 Sub ect Characteristics (N=44) with CRS and/or NEs by AE Profile** | | | | | |
|---|---|---|---|---|---|
| | **Grade^{a}** | **n(%)^{b}** | **Median Age (Range)** | **Male n (%)** | **Type of NHL, DLBCL-NOS, n (%)** |
| CRS only | Any | 21 (48) | 55.7 (26-74) | 11 (52.4) | 13 (61.9) |
| | 1 | 13(30) | 57.5 (47-74) | 7 (53.8) | 8 (61.5) |
| | 2 | 8(18) | 52.8 (26-68) | 4 (50) | 5 (62.5) |
| NE(s) only | Any | 6(14) | 64.3 (47-84) | 4 (66.7) | 3 (50) |
| | 1 | 1(2) | 47 | 0 | 0 |
| | 2 | 3(7) | 62.7 (57-73) | 2 (66.70 | 2 (66.7) |
| | 3 | 2(5) | 75.5 (67-84) | 2 (100) | 1 (50) |
| Nonconcurrent CRS & NE(s) | Any | 6(14) | 55.8 (33-73) | 6 (100) | 3 (50) |
| | CRS & NE(s) ≤2 | 2(5) | 57 (55-59) | 2 (100) | 0 |
| | CRS or NE(s) ≥3 | 4(9) | 54.5 (29-73) | 4 (100) | 3 (75) |
| Concurrent CRS & NE(s) | Any | 11(25) | 54.5 (29-79) | 8 (72.7) | 8 (72.7) |
| | CRS & NE(s) ≤2 | 4(9) | 62.25 (51-75) | 4 (100) | 4 (100) |
| | CRS or NE(s) ≥3 | 6(14) | 53.7 (37-79) | 3 (50) | 3 (50) |
| | CRS & NE(s) ≥3 | 1(2) | 29 | 1 (100) | 1 (100) |
| ^{a}Lee Criteria were used to grade CRS, while the CTCAE version 4.03 was used to grade NEs. Determined by maximum grade. | | | | | |
| ^{b}All percentages are calculated using n/44 subjects. | | | | | |
| AE, adverse event; CRS, cytokine release syndrome; DLBCL, diffuse large B-cell lymphoma; NE, neurological event; NHL, non-Hodgkin lymphoma; NOS, not otherwise specified | | | | | |

**Table E22.3** sets forth HRU by grade of toxicity. Of the 38 subjects with CRS, 11 (29%) received tocilizumab (11/102, 11% of subjects assessed at this time point), and of the 44 with CRS and/or NE, 21 (48%) received a corticosteroid (e.g. dexamethasone) (21/102, 21% of subjects assessed at this timepoint). As shown in **Table E22.3,** rates of corticosteroid use increased across all CRS and/or AE profiles as the grades of AEs increased.

| **Table E22.3 HRC by Grade (N=44)** | | | | | | |
|---|---|---|---|---|---|---|
| | Grade | n | Median LOS, Days (Range) | ICU Use, n (%) | Tocilizumab, n (%) | Corticosteroid, n (%) |
| CRS only | 1 | 13 | 5 (1-11) | 0 | 2 (15.4) | 0 |
| | 2 | 8 | 6 (4-8) | 2 (25) | 1 (12.5) | 4 (50) |
| NE(s) only | 1 | 1 | 0 | 0 | 0 | 0 |
| | 2 | 3 | 9 (6-18) | 1 (33) | 0 | 2 (66.7) |
| | 3 | 2 | 16.5 (12-21) | 2 (100) | 0 | 2 (100) |
| Nonconcurrent CRS & NE(s) | CRS & NE(s) ≤2 | 2 | 6.5 (2-11) | 0 | 1 (50) | 1 (50) |
| | CRS or NE(s) ≥3 | 4 | 16.5 (11-25) | 1 (25) | 1 (25) | 4 (100) |
| Concurrent CRS & NE(s) | CRS & NE(s) ≤2 | 4 | 10.5 (5-13) | 0 | 2 (50) | 1 (25) |
| | CRS or NE(s) ≥3 | 6 | 16 (13-56) | 3 (50) | 3 (50) | 6 (100) |
| | CRS & NE(s) ≥3 | 1 | 56 | 1 (100) | 1 (100) | 1 (100) |
| CRS, cytokine release syndrome; HRU, healthcare resource utilization; ICU, intensive care unit; LOS, length of stay; NE, neurological event. | | | | | | |

**Table E23** summarizes the HCRU and median costs for each toxicity incidence among treated subjects. Overall median costs for G≤2 vs G≥3 CRS and/or NE were $16,533 vs $70,666. As shown in **Table E23,** median total management costs for CRS and/or NE were largely driven by hospitalizations, and ranged $177-24,945 for G≤2 CRS or NE only, $31,018 for G≤2 concurrent CRS and NE, and $45,384-263,743 for G≥3 CRS and/or NE.

| **Table E23. Median Costs by Grade (N=44)** | | | | | | |
|---|---|---|---|---|---|---|
| | **Grade** | **n** | **Diagnostics** | **Drugs** | **Facility ^{a}** | **Total** |
| CRS only | 1 | 13 | $70 | $332 | $13,341 | $13,743 |
| | 2 | 8 | $249 | $175 | $16,009 | $16,434 |
| NE(s) only | 1 | 1 | $63 | $0 | $114 | $177 |
| | 2 | 3 | $527 | $406 | $24,013 | $24,945 |
| | 3 | 2 | $1390 | $845 | $76,223 | $79,223 |
| | CRS & NE ≤2 | 2 | $771 | $2,205 | $17,856 | $20,832 |
| Nonconcurrent CRS & NE | CRS or NE ≥3 | 4 | $859 | $444 | $44,081 | $45,384 |
| Concurrent CRS & NE | CRS & NE ≤2 | 4 | $606 | $2,169 | $28,243 | $31,018 |
| | CRS or NE ≥3 | 6 | $1450 | $5,128 | $75,653 | $82,231 |
| | CRS & NE ≥3 | 1 | $3407 | $10,092 | $250,244 | $263,743 |
| ^{a}Facility costs represent office visits, hospitalizations, and intensive care unit stays. | | | | | | |
| CRS, cytokine release syndrome; NE, neurological event. | | | | | | |

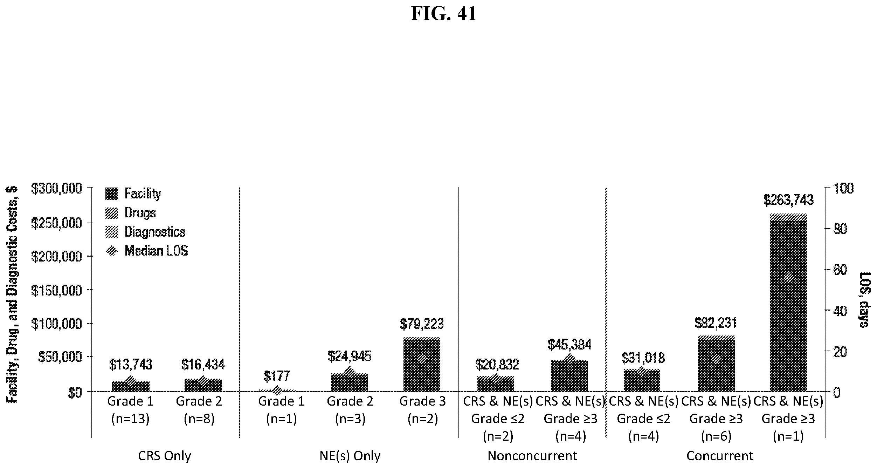

As shown in **FIG. 41****,** as the severity of AE increased, total LOS increased and ICU utilization rates increased for the CRS and NE groups. For subjects with concurrent CRS and NEs, health resource utilization in all categories increased with 1 or more grade ≥3 AEs.

The analysis was based on a time point in a clinical study in which of subjects treated with a dose of CAR-expressing T cells, 70% of CRS and/or NEs were grade ≤2. Hospitals and ICU LOS were associated with CRS and/or NE management costs. Costs for medications (including tocilizumab) were not a major component of costs, whereas facility costs, primary inpatient and ICU stays, made up an average of 92.6% of total management costs. Estimated costs of AE management in subjects in this study and at this time point varied substantially, ranging from $177 to $263,743 for this group of subjects. Grade ≥3 events resulted in a 327% increase in overall median costs vs G≤2 events ($70,666 vs $16,533). 53.8% of subjects (7/13) with grade ≥3 events were admitted to the ICU for AE management, compared with 9.7% of subjects (3/31) who had grade ≤2 events

These results are consistent with an observation that CAR T cell therapy associated with decreased incidence of AEs and lower grade CRS and NEs may limit HRU and costs.

### Example 8 Assessment of Health Status in Patients With Relapsed and Refractory Non-Hodgkin's Lymphoma (NHL) After Administration of Anti-CD19 CAR-Expressing Cells

Therapeutic CAR⁺ T cell compositions containing autologous CD4+ and CD8+ T cells expressing a chimeric antigen-receptor (CAR) specific for CD19 were administered to subjects with relapsed and refractory non-Hodgkin lymphoma that had failed 2 prior lines of therapy in the clinical study substantially as described in Examples 1, 3 and 6.

### A. Health Related Quality of Life (HRQL) and Symptom (Sx) Impact

Symptom impact, functional scales and global health status were assessed in subjects after treatment. European Organization for Research and Treatment Core Quality of Life Questionnaire version 3.0 (EORTC QLQ-C30) was administered at baseline (BL), day 29 (1 month), and months 2 and 3 to assess symptom impact, functional scales and global health status, with a point score range of 0-100, with higher scores indicating improved HRQL or lower symptom burden. Increases from BL ≥10 in functional and quality of life (QL) scores or decreases from BL≥10 points in Sx scores were considered clinically meaningful improvements. At a particular timepoint in the clinical study, 87 of 127 subjects had been evaluated at baseline and at one or more of the other times (BL+≥ 1 post-BL assessment). The mean age range of the evaluable subjects was 62 years old (standard deviation of 11 years).

As shown in **Table E24,** symptom scores generally improved post-infusion. Mean global health status, and emotional and cognitive function scores improved vs BL from months 1-3 post-infusion. Physical, role, and social function scores declined at month 1 vs. BL, followed by improvements vs. BL in month 2-3. The proportion of subjects achieving clinically meaningful improvement in particular domains 3 months post-infusion were: fatigue, 50%; pain, 51%; physical function, 22%; and global health status, 41%.

These results show that in this subset of subjects, symptoms and HQRL improved in surviving subjects, with clinically meaningful improvements in prespecified domains 2-3 months post-infusion.

| **Table E24. EORTC QLQ-30 scores** | | | | |
|---|---|---|---|---|
| **Mean (SD)** | **BL (n/N=87/87)** | **Mo 1 (n/N=73/86)** | **Mo 2 (n/N=62/82)** | **Mo (n/N=51/70)** |
| Global health status* | 65 (20) | 67 (23) | 76 (19) | 74 (19) |
| Physical* | 80 (17) | 73 (26) | 81 (21) | 80 (20) |
| Role | 70 (28) | 67 (31) | 80 (28) | 75 (28) |
| Emotional | 82 (17) | 83 (19) | 88 (15) | 86 (16) |
| Cognitive | 84 (17) | 87 (19) | 91 (16) | 88 (18) |
| Social | 71 (24) | 68 (29) | 77 (27) | 80 (25) |
| Fatigue* | 37 (22) | 38 (27) | 27 (22) | 28 (25) |
| Pain* | 26 (26) | 20 (26) | 17 (22) | 19 (23) |
| *Prespecified domains. n/N = completed QLQ-C30/total on study | | | | |

### B. Preference-Weighted Health Status after Treatment

The health utility index score and visual analog scale [VAS] (EQ-5D-5L), which measure patients' well-being and perceived health status, was analyzed in treated subjects at baseline, day 29 (month 1), and months 2, 3, 6 and every 3 months afterward. EQ-5D-5L index score was calculated using widely employed US preference weights. At a particular timepoint in the clinical study, 90 subjects had been evaluated at baseline and at one or more of the other times (BL+ ≥ 1 post-BL assessment). Mean age range was 61.7 years old (standard deviation (SD) of 11.2 years).

As shown in **Table E25,** patients had been heavily pretreated and 90% had ≥1 poor-risk disease features predictive of short overall survival.

| **Table E25. Patient characteristics (EQ-5D-5L Evaluable Population)** | |
|---|---|
| **Demographic/Characteristic** | **N=90** |
| Median age (range), y | 64 (24-79) |
| Male, n (%) | 63 (70) |

| B-cell NHL subtype, n (%) | |
|---|---|
| DLBCL, NOS de novo | 71(79) |
| Follicular lymphoma | 18(20) |
| High-grade lymphoma with DLBCL histology | 1(1) |

| Molecular subtype, n (%) | |
|---|---|
| Double/triple hit | 14(16) |

| Patient characteristics | |
|---|---|
| ECOG PS 0-1 (pre-LD), n (%) | 87(97) |
| CNS involvement, n (%) | 1(1) |
| Chemorefractory, n (%) | 62(69) |
| Never achieved CR, n (%) | 31(34) |
| Median months (range) from diagnosis to therapeutic CAR⁺ T cell infusion | 21.8(5,202) |
| Median number of prior therapies (range) | 3(2,8) |
| Previous hematopoietic stem cell transplantation, n (%) | 33(37) |
| CNS, central nervous system; CR, complete response; DLBCL, diffuse large B-cell lymphoma; ECOG, Eastern Cooperative Oncology Group; LD, lymphodepletion; NHL, non-Hodgkin lymphoma; NOS, not otherwise specified; PS, performance status. | |

EQ-5D health status evaluation was composed of 5 dimensions of health including mobility, self-care, usual activities, pain/discomfort and anxiety/depression, and was scored as a single summary index using a US value set on a -0.109 to 1 scale. A score of 0 indicated death, 1 indicated "full health", and negative scores reflect states perceived to be worse than death. A change from baseline of ≥0.07 in the EQ-5D-5L single summary index score was specified a priori as an indicator of clinically meaningful change (see e.g., Pickard AS, et al. Health Qual Life Outcomes. 2007; 5:70).

For EQ-VAS evaluation, subjects were asked to assess their health on the day of assessment by placing an X on a scale numbered from 0-100, where zero represents the worst health imaginable, and a score of 100 represents the best health imaginable (i.e., higher scores indicating improved health). Analysis of observed scores included descriptive statistics (e.g. n, mean, standard deviation, minimum, percentiles and maximum) of observed scores and change from baseline at each time point, and were evaluated by 2-sided Wilcoxon signed rank test to assess change from baseline.

Patient reported outcomes (PRO) were evaluated through month 6, with compliance rates of >57% **(Table E26).**

| **Table E26. EQ-5D-5L Compliance Rates** | |
|---|---|
| **Assessment Time Point** | **Compliance Rate, n/N (%)** |
| Baseline | 90/90 (100) |
| Month 1 | 76/89 (85.4) |
| Month 2 | 64/74 (75.3) |
| Month 3 | 52/73 (71.2) |
| Month 6 | 23/40 (57.5) |

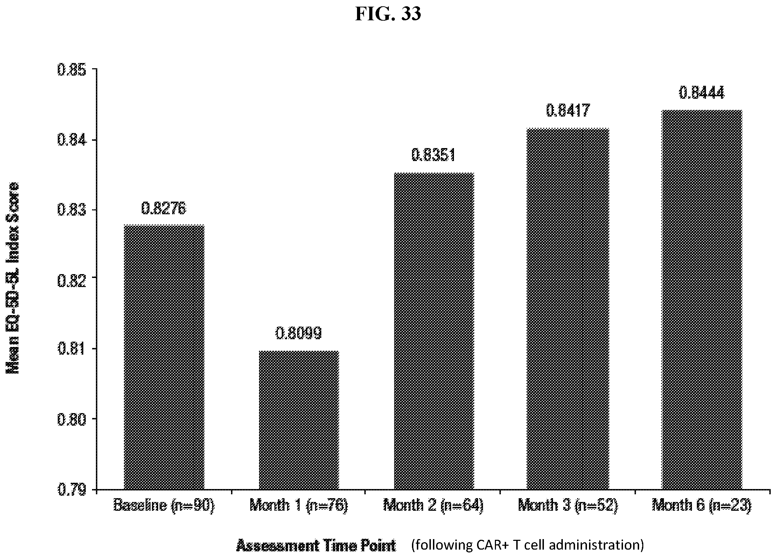

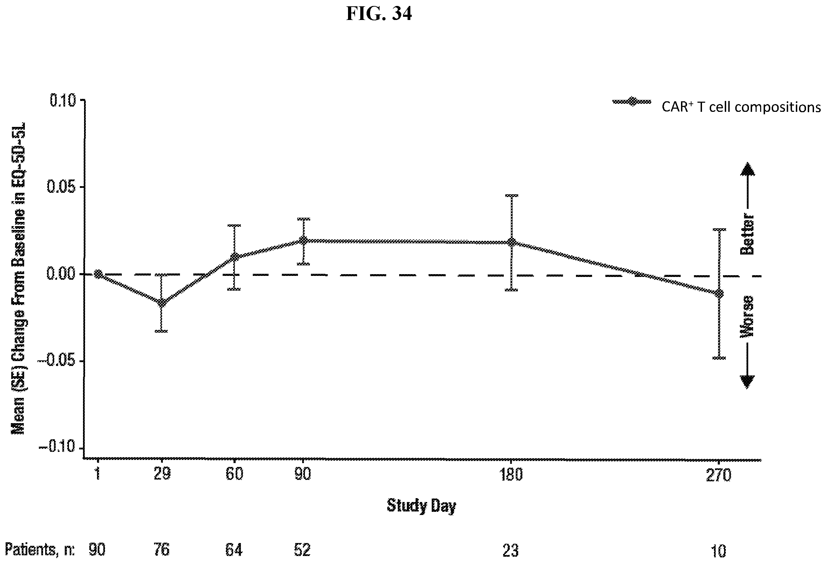

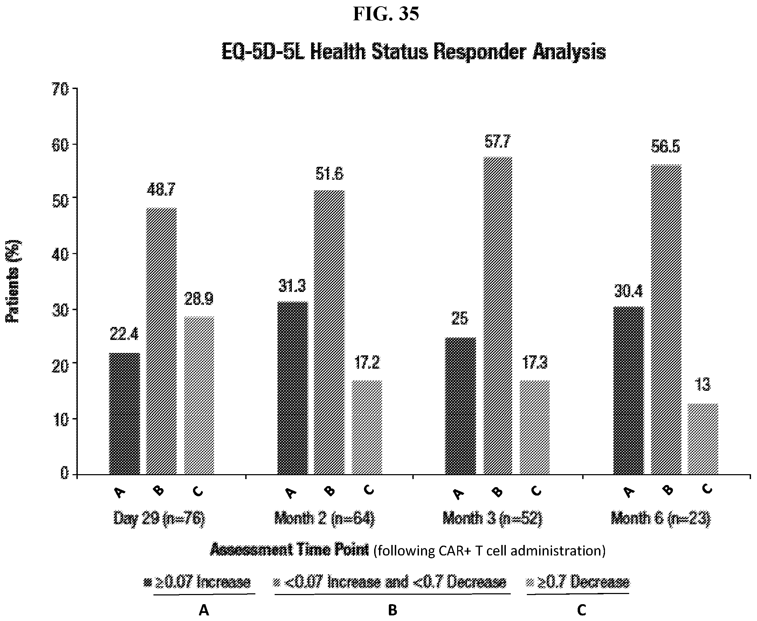

As shown in **FIG. 33****,** mean preference-weighted health status score was 0.83 (SD of 0.104 at baseline), which decreased at month 1 post-infusion (-0.012 [SD 0.144]), and increased from baseline at months 2 (0.010 [SD 0.149]) through month 6 (0.019 [SD 0.133]) post-infusion. The mean baseline EQ-5D health state utility score at baseline was lower than the population norm for the US (mean EQ-5D index score = 0.867, see e.g. Janssen B, et al. Chapter 3. Population norms for the EQ-5D. 2013 Sep 26.In: Szende A, et al, eds. Self-Reported Population Health: An International Perspective based on EQ-5D [Internet]. Dordrecht, Netherlands: Springer; 2014.). Mean change from baseline demonstrated a decrease at month 1, followed by increases from baseline from months 2-6 post-infusion **(****FIG. 34****).** At month 6, 30% of patients experienced improvements of ≥0.07, which are believed to represent a clinically meaningful improvement **(****FIG. 35****).**

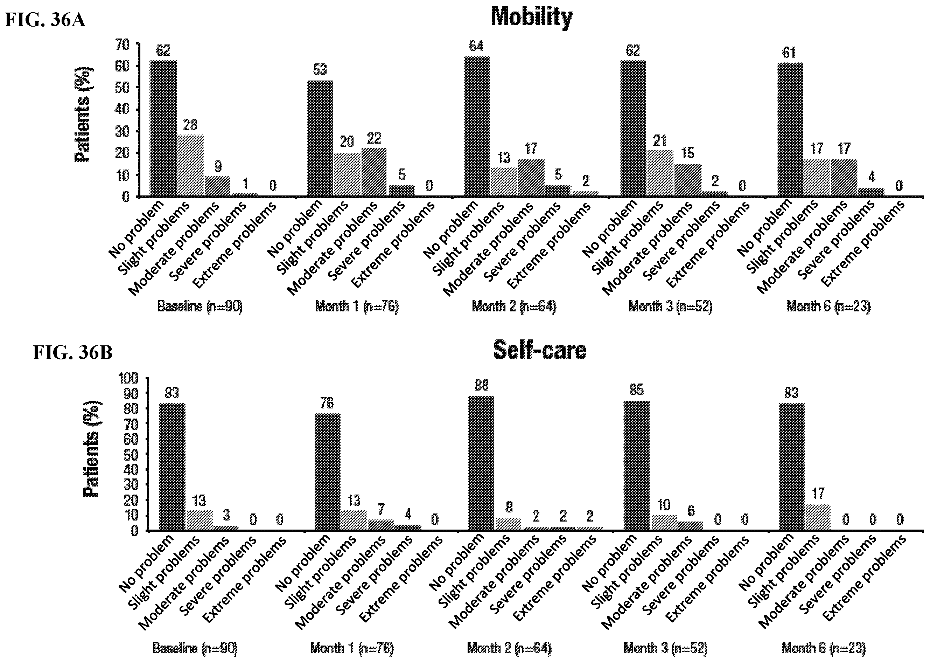

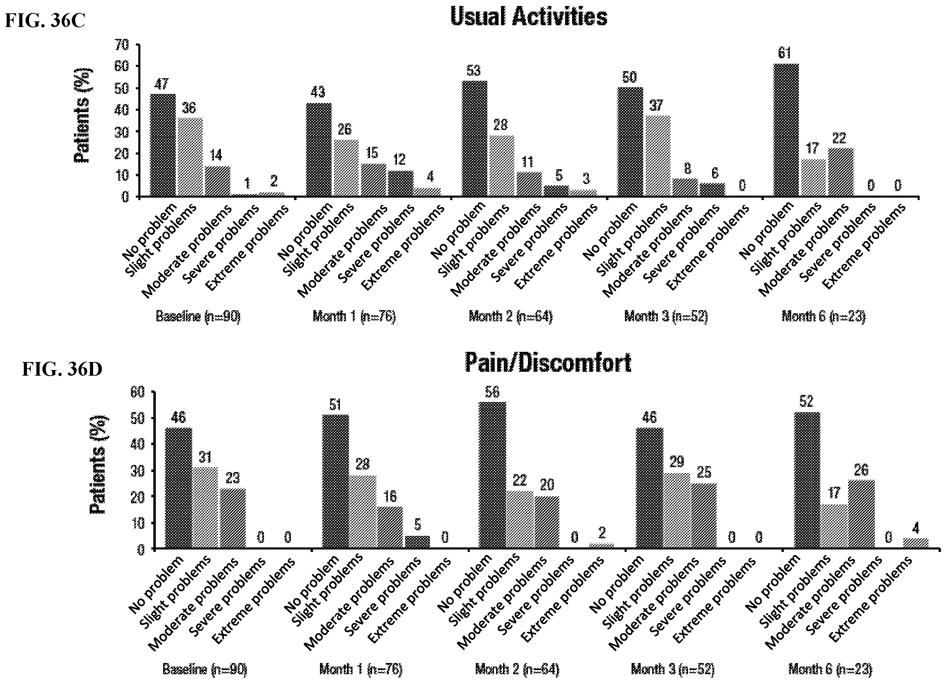

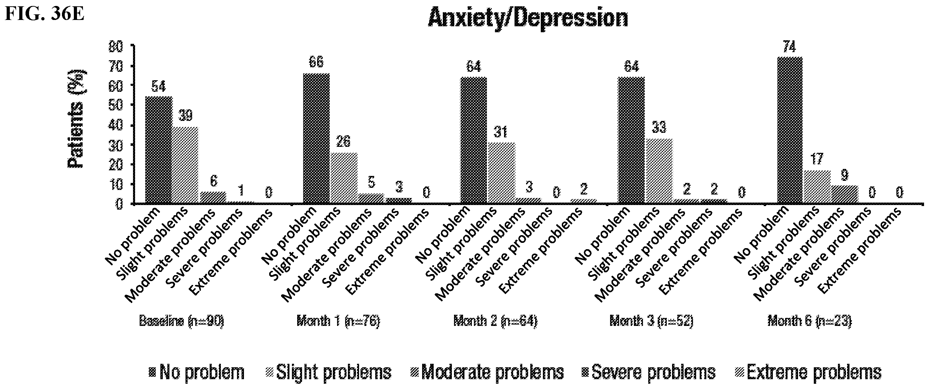

The percentage of patients reporting moderate, severe, or extreme problems with the mobility, self-care, and usual activities dimensions of the EQ-5D-5L increased at month 1 post-infusion **(****FIGS. 36A-36C****),** and the percentage of patients reporting no pain/discomfort and no anxiety/depression increased from baseline to 6 months post-infusion **(****FIGS. 36D-36E****).**

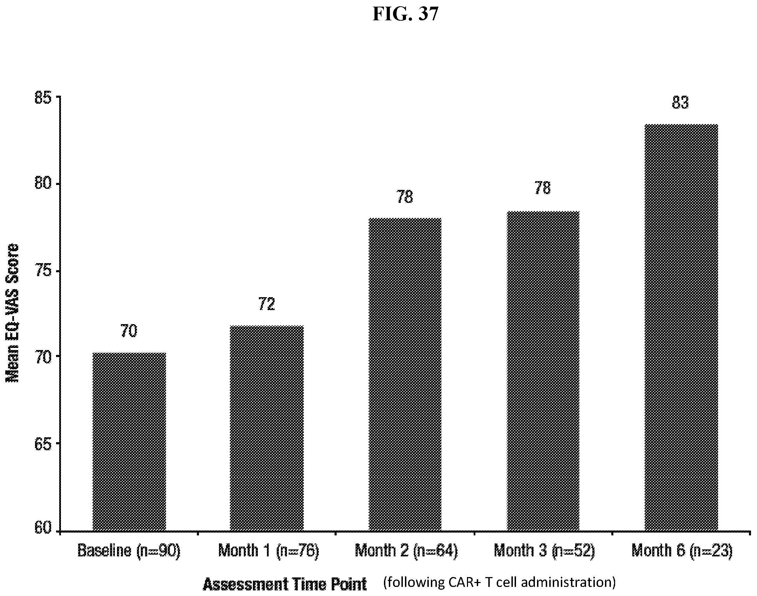

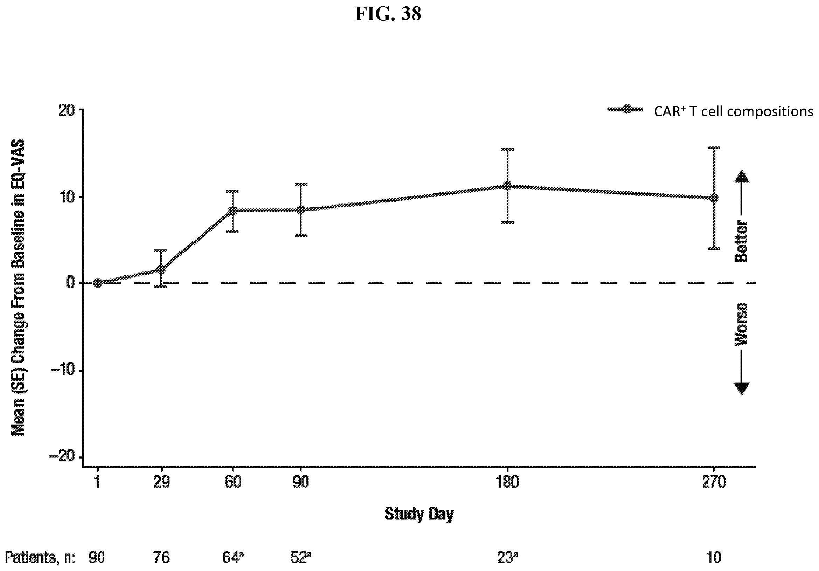

As shown in **FIG. 37****,** mean global health rating scores (EQ-VAS) increased monotonically from 70.2 at baseline to 83.4 at 6 months post-infusion, exceeding anchor and distribution based estimates of minimally important difference (8-12 and 7-10, respectively). The mean baseline EQ-VAS ratings were lower than the population norm for the US (mean EQ-VAS=80) (see e.g. Jansen B, et al.). Mean change from baseline in EQ-VAS score demonstrated an improvement in global health status from months 1-6 post-infusion **(****FIG. 38****).**

These results show that in this subset of subjects, mean preference-weighted health status decreased 1-month post-infusion, but increased during months 2-6, with clinically meaningful improvements exceeding a >0.07 threshold in 30% of patients 6 months post-infusion. The global health rating scores increased from baseline to month 6 post-infusion.

### C. Health Related Quality of Life (HQRL) and Preference-Weighted Health Status after 12 Months of Treatment

Quality of life, symptoms and health utility impact were assessed in subjects at a later timepoint in the same study described in Example 8.A and 8.B above. At this time point in the clinical study, parameters were assessed in subjects at baseline (BL; prior to CAR⁺ T cell infusion), day 29 (month 1), and months 2, 3, 6, 9, 12, 18, and 24 post-CAR⁺ T cell infusion. A change of ≥10 points (increase for functional/healthy status and decrease for symptoms) on the scale score from BL was considered clinically meaningful (i.e., a change that a patient would identified as important). The proportions of subjects with clinically meaningful change (i.e., 10-point improvement or worsening) in prespecified domains of EORTC QLQ-C30 were calculated based on change scores from BL to 6 and 12 months. Additionally, mean change from BL was calculated for the health state index score (EQ-5D) and EQ-VAS from the EQ-5D-5L.

At the time of assessment, 181 of 268 subjects (mean age, 60 years) were evaluable for the EORTC QLQ-C30 questionnaire and 186 subjects (mean age, 60 years) were evaluable for the EQ-5D-5L questionnaire. The demographics and baseline characteristics at a time point at which the median on-study follow up time was 8.7 and 8.8 months for EORTC QLQ-C30 and EQ-5D-5L evaluable populations, respectively, is shown in **Table E26.1.** Patient reported outcome survey participation rates were a minimum of 63% for subjects with at least 6 months follow up; 75% of subjects with at least 12 months of follow up completed the surveys.

| **[1201] Table E26.1 Demographics and Baseline Characteristics** | | | |
|---|---|---|---|
| **[1202]** | **[1203] Category** | **[1204] EORTC QLQ-C30 (n=181)** | **[1205] EQ-5D-5L (n=186)** |
| **[1206]** Age (years), mean (SD) | **[1207]** | **[1208]** 60.2 (14.0) | **[1209]** 60.1 (13.9) |
| **[1210]** Sex, n(%) | **[1211]** Male | **[1212]** 117(65) | **[1213]** 121(65) |
| **[1214]** Race, n(%) | **[1215]** White | **[1216]** 155(86) | **[1217]** 158(85) |
| **[1218]** Type of B-cell lymphoma, n(%) | **[1219]** DLBCL, NOS | **[1220]** 98(54) | **[1221]** 99(53) |
| | **[1222]** HGL, including double/triple hit | **[1223]** 19(10) | **[1224]** 21(11) |
| | **[1225]** DLBCL transformed from indolent lymphoma | **[1226]** 50(28) | **[1227]** 51(27) |
| | **[1228]** PMBCL | **[1229]** 13(7) | **[1230]** 14(8) |
| | **[1231]** FL3B | **[1232]** 1(1) | **[1233]** 1(1) |
| **[1234]** ECOG PS at screening, n(%) | **[1235]** 0 | **[1236]** 73(40) | **[1237]** 73(39) |
| | **[1238]** 1 | **[1239]** 107(59) | **[1240]** 112(60) |
| **[1241]** LDH ≥500 U/L,^{a} n(%) | **[1242]** | **[1243]** 29(16) | **[1244]** 30(16) |
| **[1245]** SPD ≥50 cm²,^{a} n (%) | **[1246]** | **[1247]** 44(25) | **[1248]** 45(25) |
| **[1249]** CRP ≥20 mg/L, ^{a} n(%) | **[1250]** | **[1251]** 97(45) | **[1252]** 101(54) |
| **[1253]** Refractory or relapsed, n(%) | **[1254]** Refractory | **[1255]** 141(78) | **[1256]** 145(78) |
| | **[1257]** Relapsed | **[1258]** 40 /922) | **[1259]** 41(22) |
| **[1260]** Months from eligible diagnosis to CAR-T+ cell infusion, mean (SD) | **[1261]** | **[1262]** 32.3 (35.7) | **[1263]** 32.3(35.4) |
| ^{a}Percentages based on number of patients with non-missing results. | | | |
| CRP, C-reactive protein; ECOG PS, Eastern Cooperative Oncology Group performance status; FL3B, follicular lymphoma grade 3B; HGL, high-grade lymphoma; LDC, lymphodepleting | | | |
| **[1264]** chemotherapy; LDH, lactate dehydrogenase; PMBCL, primary mediastinal B-cell lymphoma; SD, standard deviation; SPD, sum of the product of perpendicular diameters. | | | |

The results at this timepoint are summarized in **Table E26.2.** For prespecified domains of interest in the EORTC QLQ-C30, mean scores in global health status showed improvement from Months 1 to 12 compared to BL, while physical functioning declined at Month 1, followed by improvements compared to BL at Months 2 to 12. Reported fatigue symptom burden remained similar at Month 1 followed by improvement from Months 2 to 12 post-infusion compared to BL. Although mean pain symptoms were consistently lower from Months 1 to 12 compared to BL, mean symptom burden fluctuated. The mean change from BL score ranged between 4.3 to 16.9 in global health status, -4.8 to 6.5 in physical functioning, -15.5 to 0.0 for fatigue, and -8.8 to -1.8 in pain scores through Month 12. Compared to the results from the general population in the United States, based on the EORTC QLQ-C30 reference values, subjects in the study had comparable mean EORTC QLQ-C30 scores for most of the domains at BL, which were maintained or improved through Month 12. A portion of subjects demonstrated clinically meaningful improvement at Months 6 and 12 in global health status (52% and 61%), physical functioning (30% and 37%), fatigue (53% and 61%), and pain (35% and 47%), respectively. Mean EQ-5D-5L US-based health utility index scores decreased 1 month after CAR⁺ T cell infusion, which was followed by fluctuations in scores between Months 2 and 3 then an improvement from Months 6 to 12 compared to BL. Mean EQ-VAS scores showed improvement from Months 1 to 12 compared to BL.

| **Table E26.2 Mean (SD) Summary of EORTC QLQ-C30 Scores, EQ-5D-5L Index Scores and EQ-5D-VAS** | | | | | | | |
|---|---|---|---|---|---|---|---|
| | | EORTC QLQ-C30 Domains of Interest | | | | EQ-5D-5L | |
| Domain | Statistics | Global Health Status | Physical Functioning | Fatigue | Pain | Health Index Score | EQ-VAS |
| General Population Norm Data, USA ^{a,b} | Mean (SD) | 63.9 (22.9) | 80.8 (25.2) | 31.9 (27.8) | 27.5 (30.2) | NA | NA |
| Baseline | N | 181 | 181 | 181 | 181 | 186 | 186 |
| | Mean (SD) | 62.3 (20.3) | 77.8 (19.2) | 38.2 (21.8) | 25.6 (25.8) | 0.817 (0.120) | 68.3 (19.5) |
| Month 1 | N | 160 | 160 | 160 | 160 | 165 | 164 |
| | Mean (SD) | 66.4 (21.9) | 72.7 (24.8) | 37.7 (26.0) | 20.0 (24.8) | 0.794 (0.157) | 70.3 (20.2) |
| Month 2 | N | 146 | 147 | 147 | 147 | 150 | 149 |
| | Mean (SD) | 74.5 (18.9) | 80.8 (19.7) | 27.1 (21.1) | 17.7 (21.7) | 0.837 (0.136) | 77.1 (17.2) |
| Month 3 | N | 135 | 134 | 134 | 135 | 138 | 138 |
| | Mean (SD) | 72.7 (19.7) | 79.6 (19.5) | 30.2 (22.8) | 22.7 (24.2) | 0.822 (0.145) | 75.4 (18.9) |
| Month 6 | N | 81 | 81 | 81 | 81 | 85 | 84 |
| | Mean (SD) | 74.3 (18.6) | 80.2 (18.9) | 28.5 (20.9) | 17.9 (23.2) | 0.833 (0.122) | 78.0 (18.0) |
| Month 9 | N | 65 | 65 | 65 | 65 | 65 | 65 |
| | Mean (SD) | 75.8 (16.5) | 81.1 (18.1) | 27.5 (19.7) | 24.4 (24.7) | 0.833 (0.122) | 80.1 (15.6) |
| Month 12 | N | 38 | 38 | 38 | 38 | 38 | 38 |
| | Mean (SD) | 79.8 (20.1) | 83.2 (20.3) | 23.4 (23.5) | 18.4 (25.9) | 0.848 (0.157) | 82.1 (17.8) |
| NA= not available | | | | | | | |
| ^{a}EORTC QLQ-C30 Normal data for US general population (N=1009) (Nolte S et al. Eur J Cancer. 2019; 107:153-163) | | | | | | | |
| ^{b}The subject-reported outcome assessments were implemented into the study after a protocol change; therefore, only subjects enrolled after this change were eligible for the assessments. Number od assessments declines over 12 months due to loss of follow-up, deaths, length of time of study, and other reasons. | | | | | | | |

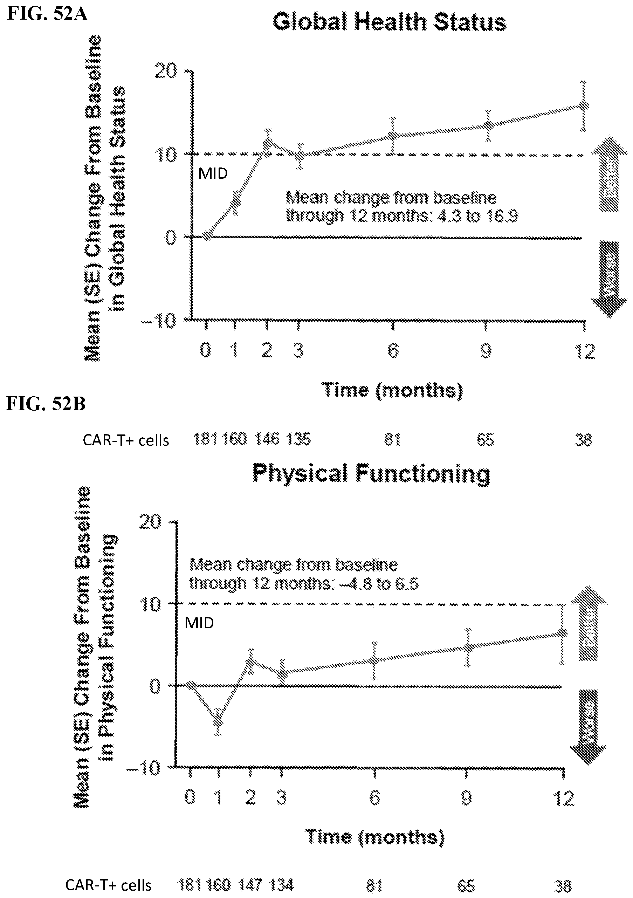

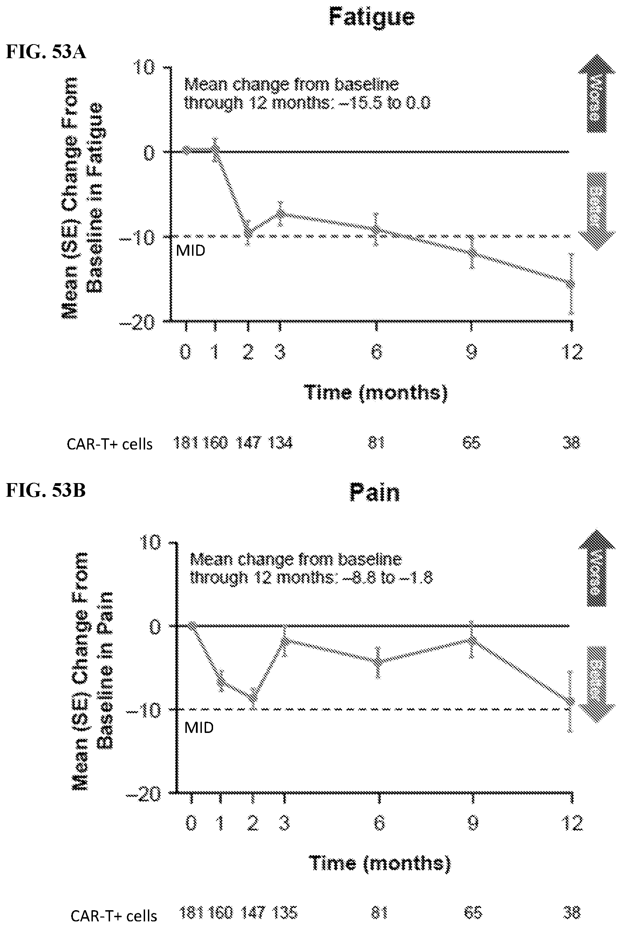

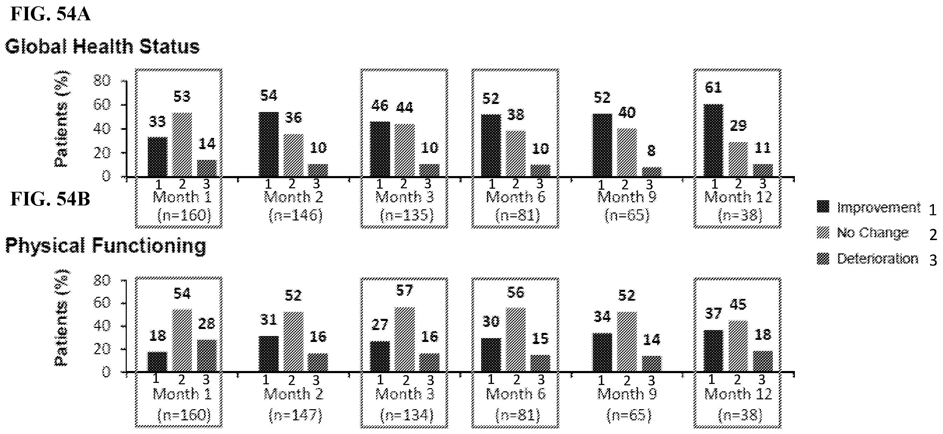

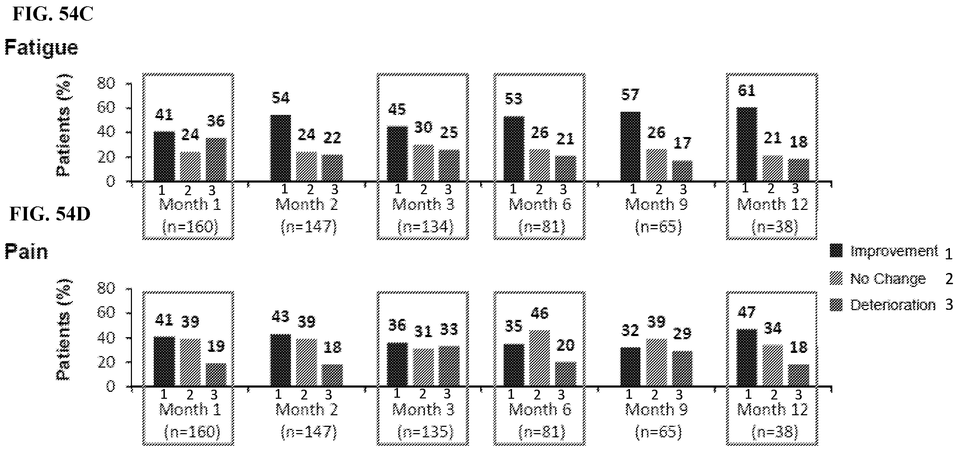

For EORTC QLQ-C30, a change from baseline of ≥10 points (MID) was considered a clinically meaningful difference. Mean change from baseline through 12 months in global health status was 4.3 to 16.9 **(****FIG. 52A****),** physical functioning was -4.8 to 6.5 **(****FIG. 52B****),** fatigue was -15.5 to 0.0 **(****FIG. 53A****)** and pain was -8.8 to -1.8 **(****FIG. 53B****).** A higher proportion of subjects experienced clinically meaningful improvements than deterioration across all timepoints in this study in areas including global health status **(****FIG. 54A****),** physical functioning **(****FIG. 54B****;** with the exception at Month 1), fatigue **(****FIG. 54C****)** and pain **(****FIG. 54D****).**

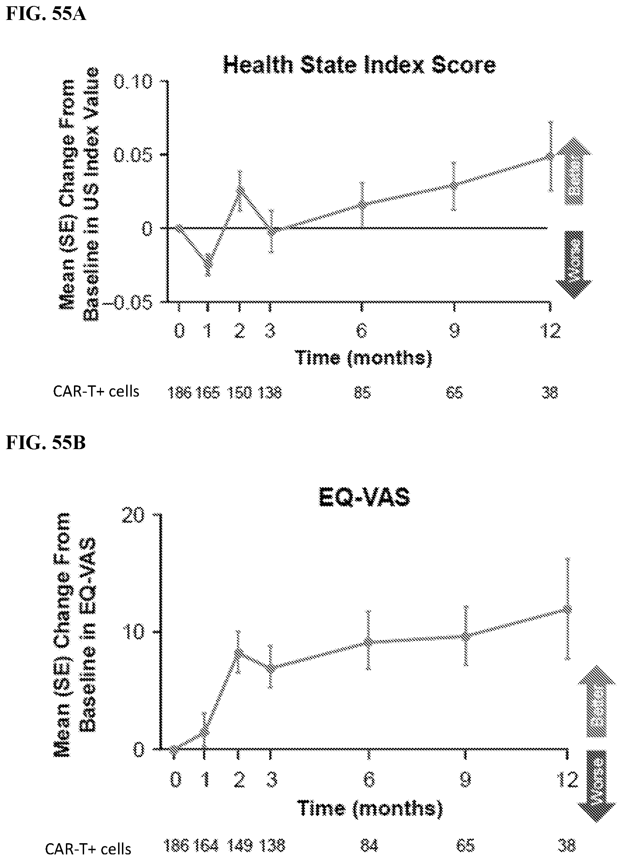

Mean change from baseline in health index score decreased at month 1, followed by fluctuations in scores between months 2 and 3 and improvement at months 6 through 12 **(****FIG. 55A****).** Mean change from baseline in EQ-VAS increased through month 1 and beyond, ranging from 9.1 to 11.9 at months 6 and 12, respectively **(****FIG. 55B****).**

The results showed that treatment with anti-CD19 CAR-expressing T cells was shown to significantly improve patients' experience as measured by patient-reported outcomes. The results showed that in this study, subjects in the DLBCL cohort experienced an improvement in HRQL and health utility through Month 12, even though some reported a detriment at Month 1. Reduced fatigue and pain symptom burden through Month 12 after CAR⁺ T cell infusion was also observed. HRQL and symptom burden (EORTC QLQ-C30) improved as early as Month 1 and through 12 months after anti-CD19 CAR-expressing T cell administration. The proportion of patients with clinically meaningful improvements in their HRQL and symptom burden were greater than the proportion of patients with deterioration. In addition, patients with clinical responses were more likely to experience clinically meaningful improvements in their HRQL than patients who did not have a clinical response. After administration of anti-CD19 CAR-expressing T cells, health status index scores (EQ-5D-5L) and self-rated health (EQ-VAS) improved as early as Month 1, and then improved steadily from Months 3 through 12. These results support the finding that notable proportions of subjects demonstrated clinically meaningful improvements at months 6 and 12 post-CAR⁺ T cell infusion.

### Example 9 Administration of Anti-CD19 CAR-Expressing Cells to Subjects with Relapsed and Refractory Non-Hodgkin Lymphoma (NHL) and Secondary CNS Lymphoma

Therapeutic CAR⁺ T cell compositions containing autologous CD4+ and CD8+ T cells expressing a chimeric antigen-receptor (CAR) specific for CD19 were administered to subjects with relapsed and refractory non-Hodgkin lymphoma that had failed 2 prior lines of therapy in the clinical study substantially as described in Examples 1, 3 and 6. In the study, eligible subjects for treatment included subjects with secondary CNS lymphoma which could be present at the initiation of the study or, if secondary CNS lymphoma developed during the clinical study, subjects could continue to receive the anti-CD19 CAR therapeutic T cell composition. Subjects initially treated with a single initial infusion of CAR⁺ T cell compositions and those that received re-treatment were included. Subjects were monitored for response and safety as described in Example 1, including by evaluating efficacy according to the Lugano criteria, and grade of CRS and NE by criteria of Lee et al. (2014) and CTCAE, version 4.03, respectively.

In the study, the subjects had been administered a single dose of anti-CD19 CAR-expressing cells as a defined composition by separate administration of a formulated CD4⁺ CAR⁺ cell population and a formulated CD8⁺ CAR⁺ population administered at a target ratio of approximately 1:1 (each single dose via separate infusions of CD4⁺ CAR-expressing T cells and CD8⁺ CAR-expressing T cells, respectively) as follows: a single dose of dose level 1 (DL-1) containing 5 x 10⁷ total CAR-expressing T cells or a single dose of dose level 2 (DL-2) containing 1 x 10⁸ total CAR-expressing T cells Subjects who achieved a complete response after the first dose but later progressed could receive a second dose (retreatment).

**Table E27** presents the patient demographics of subjects analyzed in this study who had secondary CNS lymphoma, which included 3 patients at DL1 and 6 patients at DL2 at this time of analysis. The median age of patients with secondary lymphoma was 60 years, and they had received a median of 3 prior therapies. The onset of secondary CNS lymphoma varied: of subjects treated, nine (9) subjects had secondary CNS lymphoma either at initial treatment (n=6), before retreatment (n=2) or cycle (n=1). A tenth patient had a diagnosis of secondary CNS lymphoma at time of initial treatment, but was later diagnosed with isolated brachial plexus involvement (no CNS involvement) and was not included in the analysis.

| **Table E27. Subject Demographics Summary (DLBCL Subjects)** | | | | |
|---|---|---|---|---|
| **Demographics** | **Initial Infusion (n=6)^{a}** | **Re-Treatment ^{a,b} (n=2)** | **All subjects with DLBCL (n=8)** | **Patient with MCL (n=1) ^{c}** |
| Age, median (range), years | 62 (47-3) | 52 (50-52) | 60 (47-73) | 59 |
| Male subjects, n | 3 | 1 | 4 | 1 |
| Median SPD ^{d} (range), cm² | 19 (5-134) | 37 (20-54) | 20 (5-134) | 99 |
| Median LDH (range), U/L | 247 (154-1085) | 505.5 (505-506) | 303.5 (154-1085) | 526 |
| Prior number of therapies, median (range) | 3 (2-5) | 3.5 (3-4) | 3 (2-5) | 2 |
| Prior HSCT, n | 2 | 1 | 3 | 1 |
| Refractory to prior treatment, n (%) | 6 | 1 | 7 | NA |
| Double/triple hit lymphoma, n (%) | 1 | 1 | 2 | NA |
| Parenchymal involvement only, n | 1 | 1 | 2 | 1 |
| Leptomeningeal involvement only, n | 3 | 1 | 4 | 0 |
| Both parenchymal and leptomeningeal involvement, n | 1 | 0 | 1 | 0 |
| ^{a}Includes only subjects with DLBCL. ^{b} Retreatment was defined as a second dose of CAR+ T cells given to subjects who achieved a complete response after the first dose but later progressed. ^{C} subject with MCL had CNS involvement at initial CAR+ T cell infusion. ^{d} SPD measures only non CNS sites of lymphoma involvement. | | | | |
| HSCT, hematopoietic stem cell transplantation; LDH, lactate dehydrogenase; NA, not available SPD, sum of the product of the longest perpendicular dimensions. | | | | |

As shown in **Table E28,** subjects with R/R DLBCL and with secondary CNS lymphoma showed a high degree of heterogeneity, including with respect to different histologies and various types of CNS involvement. Different histologies included DLBCL transformed from follicular lymphoma, DLBCL not otherwise specified and high-grade B cell lymphoma with MYC and BCL2 and/or BCL6 rearrangements. Various types of CNS involvement were observed that included leptomeningeal disease, focal parenchymal lesions, multiple lesions, dural involvement and nerve involvement.

| **Table E28. Individual Subject Characteristics: Active CNS Involvement at the Time of CAR+ T cell composition Administration.^{a}** | | | | | | | | | |
|---|---|---|---|---|---|---|---|---|---|
| **Subje ct num ber** | **Dose Level** | **Age (years) Sex** | **Primary Disease** | **Active Secondary CNS Lymphoma at Time of CAR+ T cell composition treatment** | **Type of CNS involvement** | **SPD (cm²)** | **LDH (U/m L)** | **Number of Prior Therapi es** | **Prior HSCT, Y/N** |
| 1 | DL1 | 67 Female | DLBCL (tFL) | Initial treatment | Parenchymal (Right temporal lobe) | 16.8 | 154 | 5 | Y (Allo) |
| 2 | DL1 | 60 male | DLBCL (tFL) | Initial treatment | Dural involvement, leptomeningea l/ parenchymal enhancement ^{c} | 5.2 | 238 | 3 | Y (Auto) |
| 3 | DL2 | 73 male | DLBCL NOS | Initial treatment | Leptomeninge al d | 20.4 | 231 | 2 | N |
| 4 | DL2 | 47 female | DLBCL NOS | Initial treatment | Leptomeninge al and parenchymal | 9.0 | 352 | 3 | N |
| 5 | DL2 | 64 male | HGBCL, DH | Initial treatment | Leptomeninge al | 132.4 | 255 | 2 | N |
| 6 | DL2 | 60 female | DLBCL NOS | Initial treatment | Leptomeninge al | 133.9 | 1085 | 3 | N |
| 7 | DL2 | 59 male | MCL | Initial treatment | Parenchymal | 98.9 | 526 | 2 | Y (Auto) |
| 8 | DL1 | 52 female | DLBCL NOS^{b} | Retreatment after PD on D280 | Leptomeninge al | 53.7 | 506 | 3 | Y (Auto) |
| 9 | DL2 | 50 male | HGBCL^{c} | Retreatment after PD on D90 | Parenchymal | 19.6 | 505 | 4 | N |
| ^{a}Two additional patients had CNS involvement; at screening in 1 patient that resolved prior to receipt of CAR+T cell compositions, and during an earlier line of therapy in 1 patient. ^{b}SPD is based on systemic disease. ^{c}CSF was negative, and there was leptomeningeal enhancement. ^{d}Patient had Meckel's cave involvement that resolved. ^{e} CNS disease was detected prior to retreatment administered on Day 568; data presented in the table are after retreatment following relapse involving the CNS. ^{f}DLBCL spread to CNS was detected after the first dose of CAR+ T cell compositions was administered during evaluation for possible neurotoxicity manifested by somnolence, confusion, partial visual field loss with "nonspecific high signal that could be related to subtle temporal edema" with leptomeningeal involvement on imaging. The patient had CR on Day 29 after first treatment. After retreatment on Day 133, no neurotoxicity was reported but DLBCL progressed 29 days after retreatment | | | | | | | | | |
| Allo, allogeneic; Auto, autologous; CNS, central nervous system; CSF, cerebrospinal fluid; DL, dose level; DLBCL, diffuse large B-cell lymphoma; HSCT, hematopoietic stem cell transplantation; LDH, lactate dehydrogenase; MCL, mantle cell lymphoma; NOS, not otherwise specified; NR, not reported; PD, progressive disease; SD, stable disease; SPD, sum of the product of the longest perpendicular dimensions: tFL, transformed from follicular lymphoma. | | | | | | | | | |

The median time to peak CAR⁺ T cell expansion was 12.5 days (median of 7-112 days). **Table E29** provides a summary of treatment-emergent adverse events in the group of subjects with secondary CNS lymphoma and the time point assessed in this study. As shown in Table E29, of the subjects with secondary CNS lymphoma, 1 of 9 had grade (G)2 cytokine release syndrome (CRS) and G3 NE; the onset of CRS occurred on Day 1 (grade 1), with progression to grade 2 on Day 2 and the onset of grade 3 NE occurred on Day 6 with decreased level of consciousness (Days 6-13) and dysphagia (Days 17-18). Additional NEs of low grade included confusion, tremors, dysarthria, lethargy, agitation, rigidity, medial gaze, bilateral nystagmus, left pupil dilated, spasticity, dysphonia, and dysphagia.

No subjects that had been subject to retreatment had CRS or NEs, however all 3 re-treatment subjects had low-grade CRS at the time of initial treatment. One re-treatment subject had an NE of G2 temporal edema when receiving initial treatment with the anti-CD19 CAR+ therapeutic T cell composition and before formal diagnosis of secondary CNS lymphoma. Among the assessed subjects with secondary CNS lymphoma, subjects received prophylactic levetiracetam, and 1 subject received corticosteroids and tocilizumab.

| **Table E29. Individual Subject Outcomes- Treatment Emergent Adverse Events** | | | | | | | | |
|---|---|---|---|---|---|---|---|---|
| **Subject Numbe r** | **Dose Level** | **Age (years) Sex** | **Primary Disease** | **Type of CNS Involvement** | **CRS^{a}** | **NEs^{a}** | **TEAEs in SOC Nervous System/ Psychiatric Disorders (Any Grade)** | **Serious AEs** |
| 1 | DL1 | 67 female | DLBCL (tFL) | Parenchyma (Right temporal lobe) | None | None | Syncope, hemorrhage intracranialb, dizziness, anxiety | Thrombocytop enia, syncope, hemorrhage intracranialb |
| 2 | DL1 | 60 male | DLBCL (tFL) | Dural involvement, leptomeningel/ parenchymal enhancement c | None | None | None | Febrile neutropenia, atypical pneumonia, pneumonia |
| 3 | DL2 | 73 male | DLBCL NOS | Leptomeningeal d | None | None | None | None |
| 4 | DL2 | 47 female | DLBCL NOS | Leptomeningeal and parenchymal | None | None | Peripheral neuropathy, headache | None |
| 5 | DL2 | 64 male | HGBCL, DH | Leptomeningeal | None | None | VIth nerve paralysis, dipleagia, confusional state, dysarthria, headache, insomnia, lethargy | Diplegia |
| 6 | DL2 | 60 female | DLBCL NOS | Leptomeningeal | Yes Grade 2 Tocilizu mab Siltuxi mab | Yes Grade 3 Dexamethazon e Solume drol | Depressed level of consciousness, agitation, nystagmus, confusional state, dysarthria, lethargy, muscle spasticity, tremor, facial paralysis, headache, huypoesthesia | Depressed level of consciousness |
| 7 | DL2 | 59 male | MCL | Parenchymal | None | None | Anxiety, hypoesthesia, headache, insomnia | None |
| 8 | DL1 | 52 female | DLBCL NOSc | Leptomeningeal | None | None | Somnolence | Bronchopulmo nary aspergillosis |
| 9 | DL2 | 50 male | High-grade DLBCL d | Parenchymal | None | None | Dizziness, headache, peripheral sensory neuropathy | Headache, nausea, vomiting |
| aWorst grade and intervention are shown. bAlthough serious, the event of intracranial hemorrhage was considered Grade 1 and causally associated with the subject's chronicthrombocytopenia. cCNS disease was detected prior to retreatment administered on Day 568; data presented in the table are after re-treatment following relapse involving the CNS. dSubject with Meckel's cave involvement that resolved. fDLBCL spread to CNS was detected after the first dose of CAR+ T cell compositions was administered during evaluation for possible neurotoxicity manifest by somnolence, confusion, partial visual field loss with "nonspecific high signal that could be related to subtle temporal edema" with leptomeningeal involvement on imaging. The subject had a CR on Day 29 after first treatment. After retreatment on Day 133, no neurotoxicity was reported but DLBCL progressed 29 days after first treatment. After retreatment on Day 133, no neurotoxicity was reported but DLBCL progressed 29 days after retreatment. | | | | | | | | |
| AE, adverse event; CNS, central nervous system; CSF, cerebrospinal fluid; CR, complete response; CRS, cytokine release syndrome; DL, dose level; DLBCL, diffuse large B-cell lymphoma; MCL, mantle cell lymphoma; NE, neurological event; NOS, not otherwise specified; SOC, system organ class; TEAE, treatment-emergent adverse event; tFL, transformed from follicular lymphoma. | | | | | | | | |

Other reported toxicities were predominantly cytopenias, and there were no treatment-related deaths related to AEs **(Table E30).**

| **Table E30. Summary of AEs (DLBCL Subjects)** | | | | |
|---|---|---|---|---|
| | **Initial Infusion ^{a}, (n=6)** | **Re-treatment^{a,b} (n=2)** | **All Subjects with DLBCL (n=8)** | **Patient with MCL (n=1)^{c}** |
| All TEAEs | 6 | 2 | 8 | 1 |
| Grade 3 or 4 | 5 | 2 | 7 | 1 |
| Grade 5 | 0 | 0 | 0 | 0 |
| SAEs | 3 | 2 | 5 | 0 |
| DLT | 0 | 0 | 0 | 0 |
| a Includes only subjects with DLBCL. b Retreatment was defined as a second dose of CAR+ T cells given to subjects who achieved a complete response after the first dose but later progressed AE, adverse event; DLT, dose-limiting toxicity; SAE, serious adverse event; TEAE, treatment-emergent adverse event. | | | | |

Response was evaluated using the Lugano criteria, and are summarized in **Tables E31** and **E32.** Four (4) subjects with secondary CNS lymphoma responded to treatment and of these all had a best response of complete response, of which 2 are ongoing at 270 and 545 days post-treatment. All 4 responses occurred after initial treatment with the therapeutic T cell compositions. None of the retreated subjects responded.

| **Table E31. Summary of Efficacy (DLBCL Subjects)** | | | | | |
|---|---|---|---|---|---|
| | | **Initial Infusion ^{a,} (n=6)** | **Re-treatment^{a,b} (n=2)** | **All Subjects with DLBCL (n=8)** | **Patient with MCL (n=1)^{c}** |
| Best ORR,^{d} n | | 4 | 0 | 4 | 0 |
| | Best CRR, n | 4 | 0 | 4 | 0 |
| Time to CR, median (range) | | 1.4 (0.9-8.7) | 0 | 1.4 (0.9-8.7) | NA |
| PFS, median (95% Cl), months | | 2.9 (0.2-NR) | NA | 2.9 (0.2-NR) | NA |
| OS | | 10.7 (0.5-NR) | NA | 10.7 (0.5-NR) | NA |
| Median follow-up | | 24.1 (12.3-24.1) | NA | 24.1 (12.3-24.1) | NA |
| Subjects still in response, n | | 2 | 0 | 2 | 0 |
| Subjects died (disease progression), n | | 4 | 2 | 6 | 0 |
| ^{a}Includes only subjects with DLBCL. ^{b} Retreatment was defined as a second dose of CAR+ T cells given to subjects who achieved a complete response after the first dose but later progressed. ^{C} best response was stable disease. ^{d} Best ORR was considered the response to the dose of CAR+T cells that was administered after secondary CNS lymphoma was diagnosed. | | | | | |
| CR, complete response; CRR, CR rate; NA, not available; NR, not reached; ORR, overall response rate; OS, overall survival; PFS, progression-free survival. | | | | | |

| **Table E32. Individual Sub ect Outcomes- Efficacy and Duration of Response** | | | | | | | | |
|---|---|---|---|---|---|---|---|---|
| **Subject Numbe r** | **Dos e level** | **Age (years ) sex** | **Primary disease** | **Active Secondary CNS Lymphoma at time of CAR+ T cell compositio n treatment** | **Type of CNS involvement** | **Best Respons e ^{a}** | **Duration of Respons e (days)** | **Status of Last Visit/Site of Progressio n** |
| 1 | DL1 | 67 Femal e | DLBCL (tFL) | Initial treatment | Parenchyma (Right temporal lobe) | CR^{b} | 65 | Death Syst |
| 2 | DL1 | 60 Male | DLBCL (fTL) | Initial treatment | Dural involvement, leptomeningel/ parenchymal enhancement ^{c} | CR | 533+ (ongoing) | Alive |
| 3 | DL2 | 72 Male | DLBCL NOS | Initial treatment | Leptomeningea l ^{d} | CR | 239+ (ongoing) | Alive |
| 4 | DL2 | 47 Femal e | DLBCL NOS | Initial treatment | Leptomeningea l and parenchymal | PD | NA | Death Syst + CNS |
| 5 | DL2 | 64 Male | HGBCL , DH | Initial treatment | Leptomeningea 1 | PD | NA | Death Clin Prog |
| 6 | DL2 | 60 Femal e | DLBCL NOS | Initial treatment | Leptomeningea 1 | CR | 59 | Death Syst + CNS |
| 7 | DL2 | 59 Male | MCL | Initial treatment | Parenchymal | SD | NA | Alive Syst + CNS |
| 8 | DL1 | 53 Femal e | DLBCL NOS ^{e} | Retreatment after PD on D181 | Leptomeningea 1 | PD | NA | Death Syst + CNS |
| 9 | DL2 | 50 Male | HGBCL ^{f} | Retreatment after PD on D89 | Parenchymal | PD | NA | Death Syst + CNS |
| ^{a}Best ORR was considered the response to the dose of CAR+ T cell compositions that was administered after secondary CNS lymphoma was diagnosed ^{b} Progressed on Day 92 as diagnosed by biopsy. After the biopsy the patient went back into CR and maintained that CR until Day 366 when she relapsed systemically, but not in the CNS. ^{c}CNS disease was detected prior to retreatment administered on Day 568; data presented in the table are after retreatment following relapse involving the CNS· ^{d}Patient had Meckel's cave involvement that resolved prior to receipt of CAR+T cell compositions. ^{e}CSF was negative, and there was leptomeningeal enhancement. | | | | | | | | |
| ^{f}DLBCL spread to CNS was detected after the first dose of CAR+T cell compositions was administered during evaluation for possible neurotoxicity manifested by somnolence, confusion, partial visual field loss with "nonspecific high signal that could be related to subtle temporal edema" with leptomeningeal involvement on imaging. The patient had CR on Day 29 after first treatment. After retreatment on Day 133, no neurotoxicity was reported but DLBCL progressed 29 days after retreatment. | | | | | | | | |
| Clin Prog.clinical progression. CNS, central nervous system; CR, complete response; CSF, cerebrospinal fluid; DL, dose level; DH, double hit; DLBCL, diffuse large B-cell lymphoma; MCL, mantle cell lymphoma; NA, not available; NOS, not otherwise specified; PD, progressive disease; SD, stable disease; tFL, transformed from follicular lymphoma. | | | | | | | | |

These results are consistent with a finding that the administered anti-CD19 CAR+ T cell composition was able to be safely delivered to subjects with secondary CNS lymphoma, which is a population with a highly unmet medical need as there are limited treatment options and a poor prognosis. The administered anti-CD19 CAR+ T cell demonstrated manageable toxicity and clinical activity in this subject population. No excess NE was observed in this population, with only 1 subject demonstrating severe NEs (grade 3) and CRS (grade 2). Response or resistance to anti-CD19 CAR-expressing T cells was similar in sites of systemic and CNS lymphoma in all subjects. In addition, 4 out of 6 subjects with secondary CNS lymphoma at the time of initial treatment responded, with 2 having an ongoing response at the time of last analysis on Day 270 and Day 545 of CAR-T cell administration, respectively.

### Example 10 Comparison of Anti-CD19 CAR-Expressing Cells versus Standard of Care (SOC) Therapy in High Risk, Transplant-Eligible Subjects with Relapsed and Refractory Non-Hodgkin Lymphoma (NHL)

Therapeutic CAR⁺ T cell compositions containing autologous CD4+ and CD8+ T cells expressing a chimeric antigen-receptor (CAR) specific for CD19, employed in the studies described in Examples 1, 3 and 6 above, are administered to subjects with relapsed and refractory non-Hodgkin lymphoma that had failed 2 prior lines of therapy. Eligible subjects for treatment include subjects with aggressive B-cell non-Hodgkin lymphoma (NHL) (diffuse large B-cell lymphoma (DLBCL), not otherwise specified [de novo or transformed indolent NHL], double/triple-hit lymphoma, primary mediastinal large B-cell lymphoma, T cell/histiocyte-rich large B-cell lymphoma, or follicular lymphoma grade 3B) and are relapsed and refractory (R/R) within 12 months of achieving a complete response (CR) after first-line therapy with a CD20 antibody and an anthracycline. Exclusion criteria include ineligibility for autologous stem cell transplantation (ASCT), planned allogeneic stem cell transplantation, prior gene or CD19-targeted therapy, or active infection.

Subjects are assigned to two different groups. Group 1 receives 3 cycles of a standard of care (SOC) therapy. The SOC for transplant-eligible subjects with relapsed/refractory DLBCL include platinum-based regimens (e.g., rituximab, dexamethasone, cytarabine (AraC), and cisplatin (R-DHAP), rituximab, ifosfamide, carboplatin and etoposide (R-ICE), or rituximab, gemcitabine, dexamethasone, and cisplatin (R-GDP)), followed by carmustine, etoposide, cytarabine, and melphalan (BEAM) high-dose chemotherapy and hematopoietic stem cell transplant (HSCT) in responding subjects. Group 2 undergoes lymphodepletion with fludarabine/cyclophosphamide followed by anti-CD19 CAR+ T cell infusion at a target dose of 100×10⁶ CD8+ and CD4+ CAR+ T cells. During manufacturing of the CAR+ T cells, subjects may receive an SOC therapy regimen.

### Example 11 Analysis of Pre-Treatment Biopsy Gene Expression Profiles and Clinical Response

Subjects with relapsed or refractory (R/R) aggressive non-Hodgkin's lymphoma (NHL) that were administered therapeutic engineered T cell compositions containing anti-CD19 CAR T cells, generally as described in Examples 1, 3 and 6 above, were assessed for gene expression profiles in pretreatment tumor biopsies.

After administration of the CAR T cell composition, subjects were monitored for clinical response, including at 3 months after administration, and response to the CAR T cell composition was determined by assessing whether the subject had progressive disease (PD) or complete response (CR).

Tumor biopsies from 50 subjects with diffuse large B-cell lymphoma (DLBCL) were collected prior to administration of the lymphodepleting chemotherapy and at day 11 post-CAR T cell administration, and analyzed by RNA sequencing (RNA-seq) for gene expression on the complementary DNA (cDNA) samples prepared from the RNA isolated from the tumor biopsies. Gene expression levels determined using RNA-seq from the pre-treatment tumor biopsies were correlated *post facto* to response following administration of the autologous therapeutic CAR T cell composition.

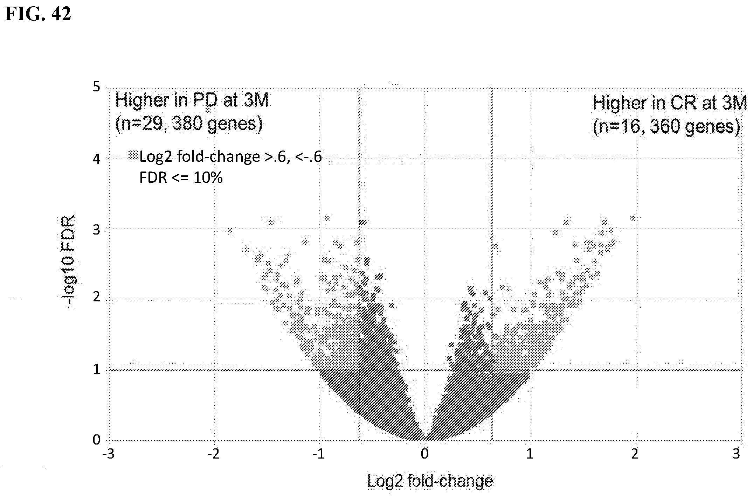

**FIG. 42** shows differential gene expression profiles in pre-treatment tumor biopsies in subjects showing CR or PD at 3 months post-treatment. Setting a Log₂ fold-change cutoff of greater than 0.6 or less than -0.6 and a false discovery rate (FDR) of less than or equal to 10% revealed 360 genes that were highly expressed in pre-treatment biopsies from subjects showing CR at 3 months post-treatment (n = 16) and 380 genes that were highly expressed in pre-treatment biopsies from subjects with PD at 3 months post-treatment (n = 29). Of the differentially expressed genes, expression of genes associated with T cells was higher in pre-treatment biopsies from subjects that exhibited CR at 3 months post-treatment. Expression of EZH2, a gene encoding histone lysine methyltransferase enhancer of zeste homolog 2, and genes that are targets of EZH2 was higher in pre-treatment biopsies for subjects that showed PD at 3 months post-treatment.

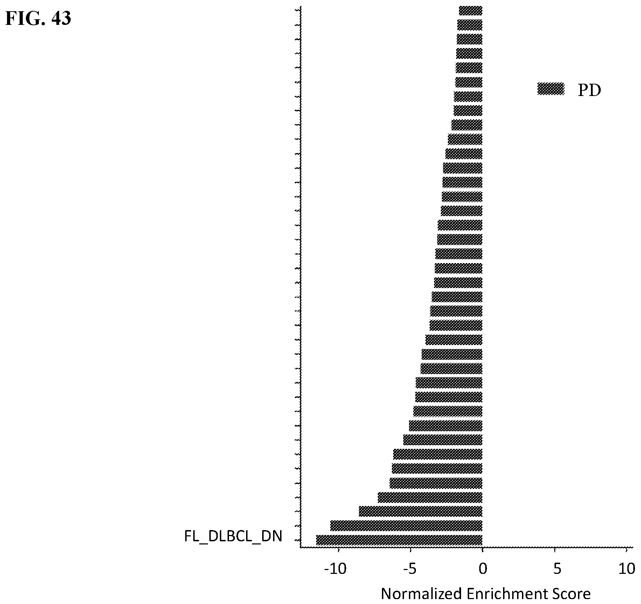

As shown in **FIG. 43****,** a gene set identified as genes that were expressed at a higher level in DLBCL compared to FL (designated "FL_DLBCL_DN"gene set; from an analysis of differential gene expression in 75 available DLBCL cell line samples and 75 available FL cell line samples as described in Example 4 below), was also found to be highly enriched for genes associated with biopsies from subjects exhibiting PD 3 months post-treatment.

These results support that genes expressed at a higher level in DLBCL compared to FL, and EZH2 target genes, were enriched in pre-treatment biopsy samples among genes associated with PD at 3 months in subjects following administration of CAR-T cells.

### Example 12 Gene Expression Analysis of Diffuse Large B-Cell Lymphoma (DLBCL) vs. Follicular Lymphoma (FL)

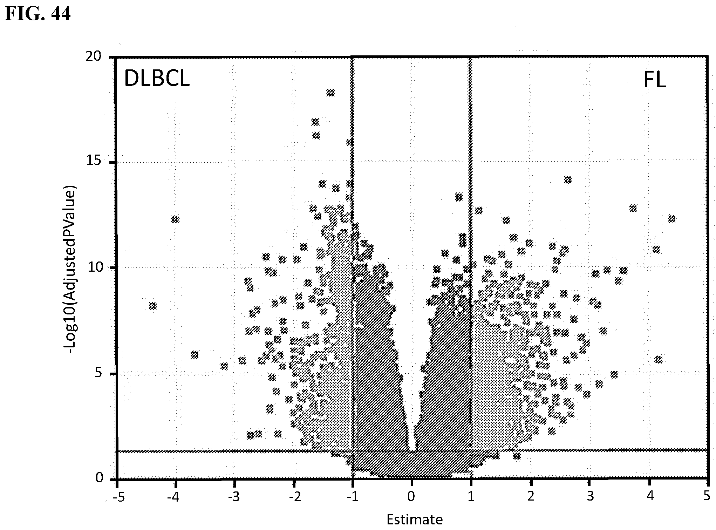

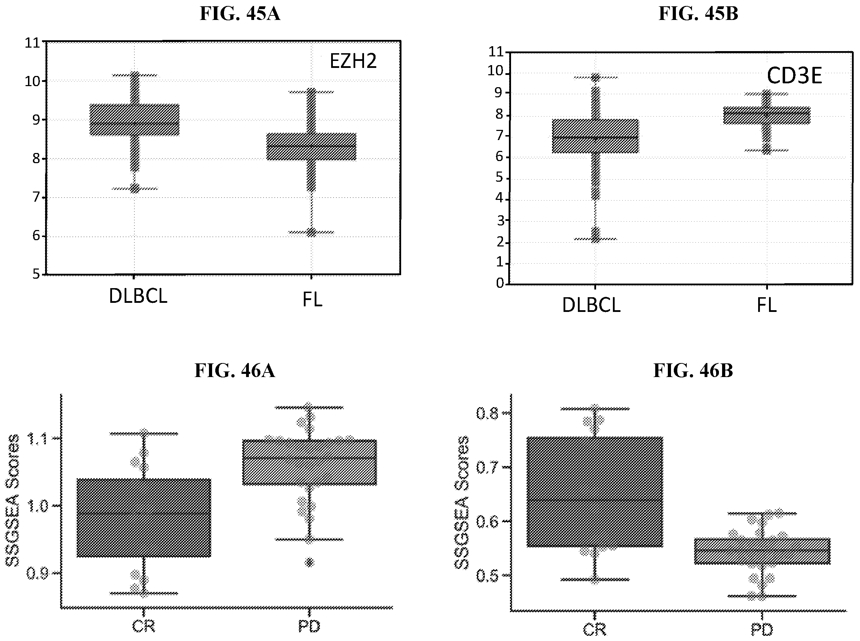

RNA-Seq for gene expression was carried out on the complementary DNA (cDNA) samples prepared from RNA isolated from about 75 Follicular Lymphoma (FL) and about 75 diffuse large B-cell lymphoma (DLBCL) available cell line samples, described above in Example 11. The cell lines analyzed included both germinal center B-cell-like (GCB) and activated B-cell (ABC) subtypes of DLBCL. As shown in **FIG. 44****,** differential gene expression was observed in DLBCL and FL tumor samples with different sets of genes elevated in the DLBCL and FL cell lines. Among the genes that exhibited differential expression between FL and DLBCL cell lines were EZH2 **(****FIG. 45A****;** encoding histone lysine methyltransferase enhancer of zeste homolog 2) and CD3E **(****FIG. 45B****;** encoding CD3e molecule, epsilon in the CD3-TCR complex). As shown, FL tumors showed lower expression levels of EZH2 and higher expression levels of CD3E compared to DLBCL cell lines.

Gene expression profiles by RNA-Seq on pre-treatment tumor biopsies, from subjects described in Example 11 who then were administered an anti-CD19 therapeutic T cell composition as described, were analyzed for expression of genes from the study described in **FIG. 44** that were found to be elevated in DLBCL (designated "DLBCL gene set") versus in FL (designated "FL gene set"). A single-sample Gene Set Enrichment Analysis (ssGSEA) was carried out to calculate separate enrichment scores for each pairing of a sample and the respective gene set. As shown in **FIG. 46A****,** subjects who went on to exhibit CR in the study described in Example 11 had a lower ssGSEA score for the DLBCL gene set compared to subjects who went on to exhibit PD. Conversely, as shown in **FIG. 46B****,** subjects who went on to exhibit CR in the study described in Example 11 had a higher ssGSEA score for the FL gene set compared to subjects who went on to exhibit PD.

These data demonstrate that the gene expression profile of subjects with DLBCL and FL are different. These data are consistent with an observation that subjects with FL or subjects with higher expression of genes found to be elevated in FL subjects compared to DLBCL subjects may be less resistant to T cell infiltration into the tumor environment than subjects with DLBCL or subjects expressing genes found to be elevated in DLBCL compared to FL.

### Example 13 Assessment of Safety and Efficacy in Subjects with Relapsed and Refractory Non-Hodgkin's Lymphoma (NHL) After Administration of Anti-CD19 CAR-Expressing Cells

Therapeutic CAR⁺ T cell compositions containing autologous CD4+ and CD8+ T cells expressing a chimeric antigen-receptor (CAR) specific for CD19 were administered to subjects with relapsed and refractory non-Hodgkin lymphoma, including subject with relapsed and refractory large B cell lymphoma (LBCL), that had failed 2 prior lines of therapy in the clinical study substantially as described in Examples 1, 3 and 6.

### A. Subjects and Treatment

The data presented at this time point represent results from268 subjects that received the therapeutic CAR+ T cell compositions, unless otherwise specified as 269 subjects. This group of subjects included the subset of subjects in which results are depicted in Examples 1, 3 and 6.

Subjects in the Large B-cell lymphoma (LBCL cohort; previously also referred to as DLBCL cohort) included subjects that were 18 years or older with relapsed/refractory (R/R) DLBCL not otherwise specified (NOS; including transformed from any indolent lymphoma), high-grade B cell lymphoma (HGBCL) with MYC and BCL2 and/or BCL6 rearrangements (Swerdlow SH et al. Blood. 2016;127:2375-90), primary mediastinal B cell lymphoma (PMBCL), or follicular lymphoma grade 3B (FL3B). Subjects had R/R disease after 2 or more lines of therapy and ECOG PS 0-2. Subjects with grade 3/4 cytopenias, mild-moderate organ dysfunction, and secondary CNS lymphoma were eligible. Bridging therapy was allowed but subjects had to have PET-positive disease before lymphodepletion (LD) with fludarabine and cyclophosphamide. Subjects who achieved stable disease (SD) as the best response after a first infusion of engineered T cells expressing the same anti-CD19 CAR described herein, were eligible to receive a second infusion of the engineered cells. Subjects must have received prior anti-CD20-containing chemoimmunotherapy (e.g. anthracycline and rituximab (or other CD20-targeted agent) and subsequently relapsed, and could have received prior autologous or allogeneic HSCT. Subjects with moderate renal (creatinine clearance <60 mL/min) and cardiac (left ventricular ejection fraction <50%) dysfunction, low absolute lymphocyte count (no minimum count required), and secondary CNS involvement were included. There were no lower threshold for ALC, ANC, platelets, or hemoglobin. Long-term follow up of outcomes in subjects in the DLBCL cohort were assessed.

For the Mantle cell lymphoma (MCL) Cohort: MCL (diagnosis must have been confirmed with cyclin D1 expression or evidence of t(11;14) by cytogenetics, fluorescence in situ hybridization [FISH], or polymerase chain reaction [PCR]) with relapsed or refractory disease after at least 1 prior line of MCL therapy.

Other criteria included positron emission tomography (PET)-positive disease according to the "Recommendations for Initial Evaluation, Staging, and Response Assessment of Hodgkin and Non-Hodgkin Lymphoma: The Lugano Classification"(Cheson BD et al. J Clin Oncol. 2014. 32:3059-68); archived tumor biopsy tissue available from the last relapse and corresponding pathology report available or, if at least 1 tumor-involved site was deemed accessible at time of screening, willing to undergo pretreatment biopsy (excisional when possible) for disease confirmation. If the subject had never had a complete response (CR), a sample from the most recent biopsy was acceptable; Eastern Cooperative Oncology Group (ECOG) performance status of 0 or 1 (note, ECOG status of 2 was also allowed until Protocol Amendment 5); adequate organ function, defined as: Assessed by the investigator to have had adequate bone marrow function to receive lymphodepleting chemotherapy (LDC); Serum creatinine ≤1.5 × age-adjusted upper limit of normal (ULN) or calculated creatinine clearance (CrCl; Cockcroft and Gault) >30 mL/min/1.73 m²; Alanine aminotransferase (ALT) ≤5 × ULN and total bilirubin <2.0 mg/dL (or <3.0 mg/dL for patients with Gilbert's syndrome or lymphomatous infiltration of the liver); Adequate pulmonary function, defined as National Cancer Institute (NCI) Common Terminology Criteria for Adverse Events (CTCAE) grade ≤1 dyspnea and oxygen saturation (SaO₂) ≥92% on room air; Adequate cardiac function, defined as left ventricular ejection fraction (LVEF) ≥40% as assessed by echocardiogram or multiple uptake gated acquisition (MUGA) scan performed within 1 month of determination of eligibility; Adequate vascular access for leukapheresis procedure (either peripheral line or surgically placed line). Subjects who received previous CD19-targeted therapy must have had CD19-positive lymphoma confirmed on a biopsy since completing the prior CD19-targeted therapy.

Subjects were treated with the anti-CD19 therapeutic CAR T cell compositions as described in Example 1. In particular, the anti-CD19 CAR+ T cells construct incorporated a 4-1BB costimulatory domain. For all treated subjects, the therapeutic T cell compositions were manufactured as separate CD4+ and CD8+ compositions in which CD8+ and CD4+ T-cell subsets from each patient were purified and transduced with the anti-CD19 CAR, expanded, and cryopreserved separately, at individual target numbers of CD8+ and CD4+ CAR T+ cell The separate manufacture of the CD4 and CD8 CAR+ T cell compositions, including separate transduction to express the anti-CD19 CAR and expansion separately in vitro, allowed administration of the CD4+ and CD8+ CAR-engineered T cells at approximately equal target doses (approximately 1:1). Consequently, a low variability was observed in the administered total and CD8+ CAR+ T-cell doses.

Subjects were treated with intravenous fludarabine (30 mg/m²/day for 3 days) plus cyclophosphamide (300 mg/m²/day for 3 days) prior to treatment with CAR T cells. Lymphodepleting chemotherapy was completed between 2 and 7 days before CAR T cell administration. Throughout the study, fludarabine administration was to be based on the fludarabine package insert; fludarabine doses were adjusted in patients with renal insufficiency, in accordance with the fludarabine label. Doses of lymphodepleting chemotherapy are described in **Table E32.1.**

| **Table E32.1. Reduced, Delayed, or Eliminated Doses of Lymphodepleting Chemotherapy ^{a}** | |
|---|---|
| | LBCL-treated Subjects^{a} |
| | N=268 |
| Subjects with | |
| Reduced dose of fludarabine or cyclophosphamide,^{b} n (%) | 18 (7) |
| Eliminated dose of fludarabine or cyclophosphamide,^{c} n (%) | 2 (0.7) |
| LBCL, large B-cell lymphoma. | |
| ^{a}Subjects could be included in >1 category. | |
| ^{b}Received at least one reduced dose of fludarabine or cyclophosphamide but did not miss a dose. | |
| ^{c}Missed at least one dose of fludarabine or cyclophosphamide. | |
| ^{d}Received at least one delayed dose of fludarabine or cyclophosphamide. | |

Bridging therapy to control disease was allowed and was administered per the investigator's decision after leukapheresis and before lymphodepleting chemotherapy. Bridging therapies are described in **Table E32.2.**

| **Table E32.2. Anticancer Bridging Therapy.** | | |
|---|---|---|
| | | **DLBCL-treated Patients N=268** |
| **Received bridging therapy, n (%)^{a}** | | |
| Yes | | 159 (59) |
| No | | 109 (41) |
| **Type of treatment, n (%)^{b}** | | |
| Systemic therapy only | | 140 (88) |
| Radiotherapy only | | 6 (4) |
| Both | | **13 (8)** |
| **Most frequently used therapies (in ≥3%), n (%)^{b}** | | |
| Rituximab-GEMOX | | 24 (15) |
| Dexamethasone | | 18 (11) |
| XRT | | 17 (11) |
| Rituximab | | 14 (9) |
| Prednisone | | 12 (8) |
| BR | | 10 (6) |
| Lenalidomide | | 9 (6) |
| GEMOX | | 7 (4) |
| Brentuximab vedotin | | 5 (3) |
| Ibrutinib | | 5 (3) |
| Bendamustine | | 4 (3) |
| Gemcitabine + rituximab | | 4 (3) |
| BR, bendamustine plus rituximab; GEMOX, gemcitabine plus oxaliplatin; LBCL, large B-cell lymphoma; XRT, radiotherapy. | | |
| LBCL-treated set includes all patients who received at least one dose of CAR+T cell compositions. | | |
| ^{a}Bridging therapy that was started after consent and prior to lymphodepleting chemotherapy. | | |
| ^{b}The denominator is the number of patients who received bridging therapy. | | |

### B. Baseline Characteristics

At this time point for analysis, a total of 342 subjects had been leukapheresed, of which 268 received CAR⁺ T cell compositions (or in some cases, 269 subjects as indicated), 24 received nonconforming product (i.e., the CD8+ or CD4+ component did not meet release criteria), across all dose levels, 2 had compositions unavailable, and 48 did not receive CAR T-cells primarily because of death or lymphoma complications before infusion (33 had died and did not receive CAR-expressing T cells). In an exemplary manufacturing process for generating CAR-expressing cells, median time from leukapheresis to harvest criteria and product availability was 24 days.

Patient characteristics are shown in **Table E32.3** (n=268) **and Table E32.4** (n=269). The median age was 63 (range, 18-86) years, including older subpopulations (41% were ≥65 years old and 10% were ≥75 years old); 65% of subjects were male. Subjects had moderate comorbidities (19% and 5% of patients had impaired renal and cardiac function, respectively). Subjects were heavily pretreated (the median number of prior lines of therapy was 3) and had aggressive disease: 26% had ≥4 prior lines of systemic therapy (median of 3; range, 1-8); 34% underwent prior autologous HSCT and 3% prior allogeneic HSCT; 67% were chemorefractory (chemotherapy-refractory to the last line of therap); 44% had never achieved CR. Of all subjects, 255 were evaluable for response. Subjects had serum creatinine levels of ≤1.5 x age adjusted ULN or a creatinine clearance rate (CrCl) >30 ml/min/1.73m². At screening, 58% of subjects had ECOG PS 1; 59% required bridging therapy; 22% had LDH ≥500 U/L and 28% had SPD ≥50 cm² before lymphodepletion.

| **Table E32.3 Patient Characteristics at Baseline** | |
|---|---|
| **Characteristic** | **CAR⁺ T cell-Treated Patients N=268** |
| Males - no. (%) | 174 (65) |

| Age, yrs. | |
|---|---|
| Median (range) | 63 (18-86) |
| ≥65 - no. (%) | 111 (41) |
| ≥75 - no. (%) | 27 (10) |

| LBCL subtypes - no. (%) | |
|---|---|
| DLBCL NOS | 137 (51) |
| DLBCL transformed from FL | 60 (22) |
| DLBCL transformed from other indolent lymphomas | 18 (7) |
| HGBCL^{†} | 36 (13) |
| PMBCL | 15 (6) |
| FL grade 3B | 2 (1) |

| ECOG PS at screening - no. (%) | |
|---|---|
| 0 | 109 (41) |
| 1 | 155 (58) |
| 2 | 4 (1) |
| Pre-LDC SPD ≥50 cm² - no. (%)^{‡} | 72 (28) |
| Pre-LDC CRP, mg/L - median (range) | 27.90 (0.25-2158.00) |
| Pre-LDC creatinine clearance <60 mL/min - no. (%)^{§} | 51 (19) |
| LVEF <50% - no. (%)^{∥} | 13 (5) |

| Prior lines of therapy | |
|---|---|
| Median (range) | 3.0 (1-8) |
| ≥4 - no. (%) | 70 (26) |
| Chemotherapy-refractory - no. (%) | 180 (67) |
| Received prior HSCT - no. (%) | 94 (35) |
| Autologous HSCT | 90 (34) |
| Allogeneic HSCT | 9 (3) |
| Never achieved CR with prior therapy - no. (%) | 119 (44) |
| Received bridging therapy - no. (%) | 159 (59) |
| Secondary CNS lymphoma - no. (%) | 7 (3) |
| * CNS denotes central nervous system, CR complete response, CRP C-reactive protein, DLBCL NOS diffuse large B-cell lymphoma not otherwise specified, ECOG PS Eastern Cooperative Oncology Group performance status, FL follicular lymphoma, HGBCL high-grade B-cell lymphoma with *MYC* and *BCL2* and/or *BCL6* rearrangements, HSCT hematopoietic stem cell transplantation, LBCL large B-cell lymphoma, LDC lymphodepleting chemotherapy, LVEF left ventricular ejection fraction, PMBCL primary mediastinal large B-cell lymphoma, and SPD sum of product diameter. | |
| ^{†}Patients with DLBCL transformed from indolent lymphomas with *MYC* and *BCL2* or *BCL6* rearrangements were not included as HGBCL. | |
| ^{‡}Not all patients were evaluable for SPD (n=256). | |
| ^{§}Creatinine clearance had to be ≥40 mL/min per eligibility criteria. | |
| ^{∥}LVEF had to be ≥40% per eligibility criteria | |

| **Table E32.4 Baseline characteristics** | |
|---|---|
| **Characteristic** | **All LBCL Patients, N=269** |
| Age, median (range) | 63 (18-86) |
| ≥65 years, n (%) | 112 (42) |
| ≥75 years, n (%) | 27 (10) |

| NHL subtypes, n (%) | |
|---|---|
| DLBCL NOS | 137 (51) |
| Transformed from FL / other indolent lymphomas | 60 (22) / 18 (7) |
| HGBCL^{a} / PMBCL / FL3B | 36 (13) / 15 (6) / 3 (1) |
| Secondary CNS lymphoma, n (%) | 7 (3) |
| ECOG PS 0-1 / 2 at screening, n (%) | 265 (99) / 4 (1) |
| Pre-LDC SPD ≥50 cm², n (%)^{b} | 73 (28) |
| Creatinine clearance >30 to <60 mL/min, n (%) | 51 (19) |
| LVEF ≥40% to <50%, n (%) | 13 (5) |
| Prior therapies, median (range) | 3 (1-8) |
| ≥4 prior therapies, n (%) | 71 (26) |
| Received prior HSCT, n (%) | 94 (35) |
| Autologous / allogeneic HSCT | 90 (33) / 9 (3) |
| Chemotherapy-refractory, n (%)^{c} | 181 (67) |
| Never achieved CR with prior therapy, n (%) | 119 (44) |
| Received bridging therapy), n (%) | 159 (59) |
| ^{a} Subjects with DLBCL transformed from indolent lymphomas with *MYC* and *BCL2* or *BCL6* rearrangements are not included as HGBCL. ^{b}N=256. ^{c}The status was chemotherapy-refractory if the patient achieved SD or PD to last chemotherapy-containing regimen or relapsed <12 months after auto-HSCT; otherwise the status was chemotherapy-sensitive. | |
| NOS, not otherwise specified; FL(3B), follicular lymphoma (grade 3B); HGBCL, high-grade B cell lymphoma; LDC, lymphodepleting chemotherapy; PMBCL, primary mediastinal large B-cell lymphoma; SPD, sum of product of diameter | |

***C. Assessment of Dose and Outcomes*** Subjects had received DL1 of 50 x 10⁶ (n=51), DL2 of 100 x 10⁶ (n=176) or DL3 of 150 x 10⁶ (n=41). Twenty-five subjects (25) were treated in the outpatient setting. The data in this example are pooled from all dose levels.

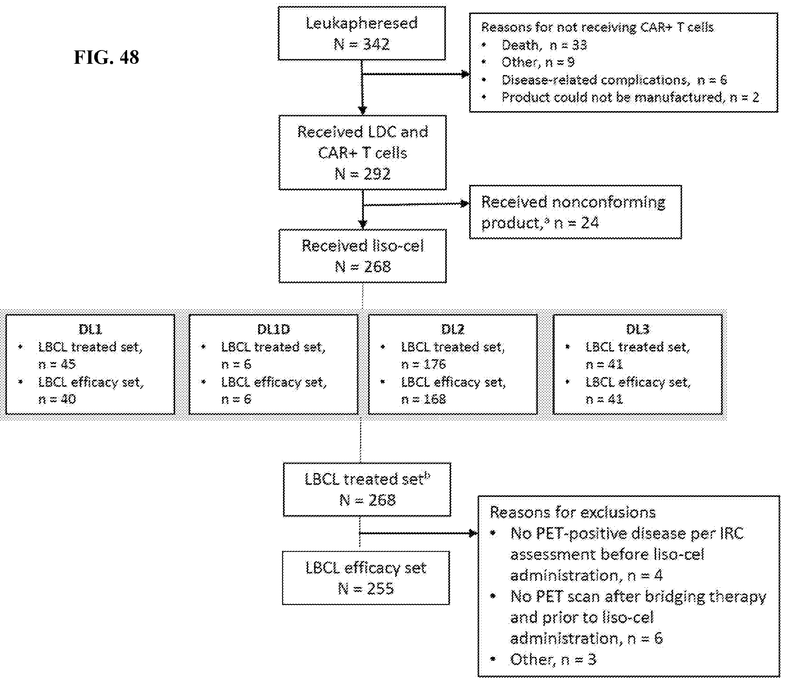

Three single-dose schedules were tested. Dose level 1 was tested as both a single dose given at Day 1 and as a two-dose schedule, with a second dose of CAR T cells given on Day 15 **(Table E33.1).** All dose levels were expanded, and DL2 was chosen as the target DL for dose confirmation. Efficacy and safety in dose-finding cohorts were analyzed using the Bayesian method (Quintana M, et al. Contemp Clin Trials 2016;48:153-65) to identify a recommended regimen, which was further evaluated in dose-confirmation. The study met all primary and secondary response endpoints. Primary endpoints were treatment emergent adverse effects (TEAEs) and overall response rate (ORR). Secondary endpoints included CR rate, duration of response (DOR), PFS, PFS ratio, and OS. TEAEs, including investigator-identified neurological events (NE) related to CAR⁺ T cell compositions, were graded using CTCAE criteria (e.g. v4.03.20); CRS was graded per the Lee criteria (2014); and parameters related to cellular kinetics, B-Cell aplasia, and immunogenicity were assessed as described below. ORR was assessed by independent review using the Lugano criteria. Data from all CAR⁺ T cell treated subjects in the LBCL cohort were analyzed across DLs. A diagram of the treatment regimen is described in **FIG. 48****.**

| **Table E33.1 Dose Schedules** | | | | |
|---|---|---|---|---|
| | | **Target Dose of CAR+ T cells** | | |
| **Dose Level** | **Study Day of Administration** | **Total** | **CD8+** | **CD4+** |
| DL1 | Day 1 | 50 × 10⁶ | 25 × 10⁶ | 25 × 10⁶ |
| DL1D | Day 1 and Day 15 | 50 × 10⁶ and 50 × 10⁶ | 25 × 10⁶ and 25 × 10⁶ | 25 × 10⁶ and 25 × 10⁶ |
| DL2 | Day 1 | 100 × 10⁶ | 50 × 10⁶ | 50 × 10⁶ |
| DL3 | Day 1 | 150 × 10⁶ | 75 × 10⁶ | 75 × 10⁶ |
| DL1, dose level 1 (single dose); DL1D, dose level 1 (2 doses); DL2, dose level 2 (single dose); DL3, dose level 3 (single dose). | | | | |

Dose limiting toxicities (DLT) and Adverse Events (AEs) were defined as follows: Any treatment-emergent grade 4 or 5 AEs related to CAR+ T cells; any treatment-emergent grade 3 AEs related to CAR+ T cells that did not resolve to grade ≤2 within 7 days; any treatment-emergent grade 3 seizures that did not resolve to grade ≤2 within 3 days; any treatment-emergent autoimmune toxicity grade ≥3, regardless of attribution (excluding B-cell aplasia). In the 2-dose schedule, inability to receive the second CAR+ T cell dose due to toxicity related to CAR+ T cells. The following expected events were not considered DLTs: Grade 3 or 4 laboratory values that were not considered clinically significant by the investigator, even if they met AE reporting criteria; Grade 4 infusional toxicities that were reversible to grade ≤2 in 8 hours; Grade 3 or 4 fever or febrile neutropenia for ≤2 weeks; Grade 3 transaminases for ≤2 weeks; Grade 3 bone pain due to T-cell expansion in marrow compartments for ≤2 weeks; Grade 3 or 4 tumor lysis syndrome (TLS) for ≤2 weeks; Grade 3 or 4 hypotension (without other cytokine release syndrome [CRS] symptoms) requiring a single vasopressor for support that resolved to grade <3 in ≤72 hours; Grade 3 or 4 CRS with hypotension alone requiring a single vasopressor for support (not requiring intubation) that resolved to grade <3 in ≤72 hours; Grade 3 or 4 encephalopathy for ≤7 days and resolved to baseline in ≤28 days from the beginning of the grade ≥3 event; Grade 3 chills; Grade 3 or 4 lymphopenia; Grade 3 or 4 leukopenia; Grade 3 or 4 B-cell aplasia and hypogammaglobulinemia.

Treatment-emergent adverse events (TEAEs) were defined as adverse events that started any time from initiation of CAR+ T cell administration through and including 90 days following the final administration of CAR+ T cells. Any adverse event that occurred after the initiation of another anticancer treatment or after CAR+ T cell retreatment was not considered a CAR+ T cell TEAE. TEAEs, laboratory abnormalities, and neurological events (defined as investigator-assessed neurological events related to CAR+ T cells) were graded using National Cancer Institute Common Terminology Criteria for Adverse Events v4.03 (National Cancer Institute Common Terminology Criteria for Adverse Events v4.03.2009. October 23,2019), CRS was graded according to the Lee criteria (Lee et al. Blood. 2014; 188-95).

Duration of response was defined as the time from first complete response or partial response to disease progression or death. Progression-free survival was defined as the time from first infusion of CAR T cells to progression of disease or death. Overall survival was defined as the time from first infusion of CAR T cells to the date of death.

### D. Safety Outcomes

Among 139 subjects treated in dose-finding (n=59) and dose-expansion (n=80), 9 (6%) experienced DLTs **(Table E33.2).** No maximum-tolerated dose was identified. DL2 (100×10⁶ CAR+ T-cells) was the dose evaluated in dose-confirmation. No clear relationship was observed between dose-related toxicity and efficacy across all DLs, except for an elevated rate of grade 1 and 2 CRS at DL3 **(Table E33.2).** No DLTs occurred at DL1D (dose level 1, double dose). All events of grade 3 encephalopathy resolved. The event of grade 4 encephalopathy was not resolved at time of death due to disease progression. Both events of grade 4 thrombocytopenia recovered/resolved. Data from all subjects were combined.

| **Table E33.2 Dose-Limiting Toxicities During Dose-Finding and Dose-Expansion** | | | |
|---|---|---|---|
| | | **DLTs** | |
| | | **Event** | **Worst Grade** |
| *Dose-Finding (N=59)* | | | |
| | **DL1** | | |
| | Patient 1 | Encephalopathy | 4 |
| | Patient 2 | Seizure | 4 |
| | Patient 3 | Acute kidney injury | 4 |
| | DL2 **DL2** | | |
| | Patient 4 | Thrombocytopenia | 4 |
| | **DL3** | | |
| | Patient 5 | Encephalopathy | 3 |

| *Dose-Expansion (N=80)* | | | |
|---|---|---|---|
| | **L1** | | |
| | Patient 6 | Encephalopathy | 3 |
| | | CRS | 4 |
| | | Renal failure | 4 |
| | Patient 7 | Diffuse alveolar damage | 5 |
| | Patient 8 | Thrombocytopenia | 4 |
| | | Mental status changes | 3 |

| | **DL2** | | |
|---|---|---|---|
| | Patient 9 | Encephalopathy | 3 |

CRS, cytokine release syndrome; DL1, dose level 1 (single dose); DL2, dose level 2 (single dose); DLT, dose-limiting toxicity.

The adverse events outcomes of all of the subjects treated at the timepoint are set forth in **Table E33.3.**

At screening, 58% of subjects had ECOG PS 1; 59% required bridging therapy; 22% had LDH ≥500 U/L and 28% had SPD ≥50 cm² before LD. Among 268 subjects who have received CAR+ T cell compositions assessed in the safety analysis, the most frequent treatment-emergent adverse events (TEAEs) were neutropenia (63%), anemia (48%), fatigue (44%), CRS (42%), and nausea (33%). Safety assessment showed 79% of subjects had grade ≥3 TEAEs, primarily cytopenias (neutropenia, 60%; anemia, 37%; thrombocytopenia, 27%). CRS or NE occurred in 47% of subjects. Any grade CRS occurred in 42% of subjects at a median onset of 5 days; only 2% had grade ≥3 CRS. NEs occurred in 30% of subjects (grade ≥3, 10%) at a median onset of 9 days. 19% of subjects received tocilizumab and 21% received corticosteroids for CRS and/or NEs. Seven subjects (3%) had grade 5 TEAEs, including one grade 5 DLT, of which 4 were attributed to the CAR+ T cell composition and LDC, as shown in **Table E33.3.**

Seven subjects (3%) had grade 5 TEAEs, including one grade 5 DLT; four grade 5 TEAEs that occurred were related to CAR⁺ T cell compositions and the lymphodepleting therapy (LD) (diffuse alveolar damage, pulmonary hemorrhage, multiple organ dysfunction syndrome, cardiomyopathy). Three grade 5 TEAEs were unrelated to CAR⁺ T cell compositions: fludarabine leukoencephalopathy, septic shock, and progressive multifocal leukoencephalopathy. Prolonged grade ≥3 cytopenia (based on laboratory assessment at Day 29) was reported in 37% of subjects. Safety was similar between age groups, histologies, and subjects with organ dysfunction

**Table E33A-1** shows treatment adverse events for the group of 268 subjects and **Table E33A-2** show treatment-emergent adverse events across all subject subgroups at another timepoint in the same study (n=269). **Table E33B-1** (n=268) and **Table E33B-2** (n=269) show other adverse events of special interest. Laboratory based hypogammaglobulinemia (IgG<500 mg/dL) was present in 49% (127 of 258) at baseline, 58% (136 of 236 subjects) on study day 29, and 61% (8 of 112 subjects) at study day 365.

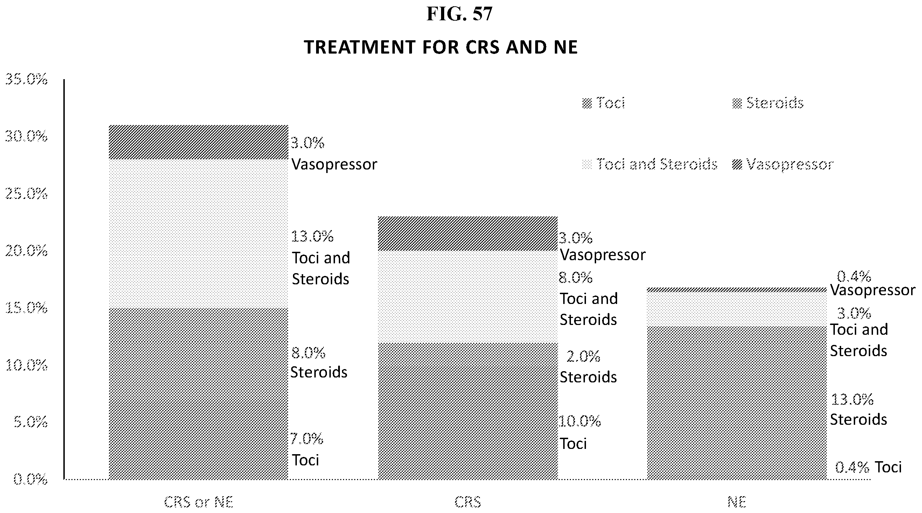

**Table E33B-3** shows incidence and management or CRS and NEs. CRS and NE were reversible, with 1 subject having unresolved NE (grade 1 tremor) at the time point of analysis and 8 subjects had ongoing CRS/NE at time of death for other reasons. Treatment for CRS and NEs are described in **FIG. 57****,** one subject received anakinra and siltuximab for CRS and one subject received siltuximab for NE. **Table E33C** shows treatment emergent symptoms of cytokine release syndrome (CRS) which occurred in ≥2% of patients. **Table E33D** shows treatment-emergent neurological events by group which occurred in ≥5 patients.

**Tables E33E-1 and Table E33E-2** show a comparison of adverse events by dose level. **Table E33F** shows a comparison of safety among patient subgroups. **Table E33G** shows safety outcomes of outpatients, retreated patients and those who received non-conforming product.

Fewer than one-half (47%) of all patients developed CRS and/or NEs **(Tables E33B-E33D),** with a median onset of 5 and 9 days, respectively. Forty-two percent of patients had any-grade CRS. Grade 3 or 4 CRS was rare, occurring in only 2% of patients. CRS was managed with tocilizumab and/or corticosteroids in 20% of patients; 10% received tocilizumab only. Any-grade NEs were seen in 30% of patients and usually occurred during or after CRS (73%); 10% had grade 3 or 4 NEs. No patients had grade 5 CRS or NEs. Other TEAEs of special interest included prolonged cytopenias (37% of patients), hypogammaglobulinemia (14%), and grade ≥3 infections (12%). Eighteen patients (7%) were admitted to the intensive care unit during the initial hospitalization period after CAR+T cell infusion.

| **Table E33.3. Adverse Events** | | |
|---|---|---|
| | **All Treated Subjects N= 268** | |
| **TEAEs of Special Interest** | **Any Grade** | **Grade ≥3** |
| CRS, n (%) | 113 (42) | 6 (2) |
| Time to onset, median (range) days | 5 (1-14) | 6.5 (3-12) |
| NE, n (%) | 80 (30) | 27 (10) |
| Time to NE onset, median (range) days | 9 (1-66) | 9 (2-44) |
| Any tocilizumab, n (%) | 52 (19) | |
| Tocilizumab alone, n (%) | 18 (7) | |
| Any corticosteroids, n (%) | 56 (21) | |
| Corticosteroids alone, n (%) | 22 (8) | |
| Both tocilizumab and corticosteroids, n (%) | 34 (13) | |
| Infections, n (%) | 110 (41) | 33 (12) |
| Prolonged grade ≥3 cytopenia, ^{a} n (%) | - | 100 (37) |
| Tumor lysis syndrome, n (%) | 2 (1) | 2 (1) |
| ^{a} Prolonged cytopenias were defined as grade ≥3 laboratory assessments at study day 29. CRS, cytokine release syndrome;; NE, neurological events; TEAEs, treatment-emergent adverse events. | | |

| **Table E33A-1. Treatment-Emergent Adverse Events** | | | |
|---|---|---|---|
| | **CAR+T cell-Treated Patients N=268** | | |
| | **Any-Grade TEAE** | **Grade 1 or 2 TEAE** | **Grade ≥3 TEAE** |
| Patients with TEAEs - no. (%) | 266 (99) | 54 (20) | 212 (79) |
| Neutropenia | 168 (63) | 8 (3) | 160 (60) |
| Anemia | 128 (48) | 28 (10) | 100 (37) |
| Fatigue | 119 (44) | 115 (43) | 4 (1) |
| Cytokine release syndrome | 113 (42) | 107 (40) | 6 (2) |
| Nausea | 89 (33) | 85 (32) | 4 (1) |
| Thrombocytopenia | 84 (31) | 12 (4) | 72 (27) |
| Headache | 80 (30) | 77 (29) | 3 (1) |
| Decreased appetite | 76 (28) | 69 (26) | 7 (3) |
| Diarrhea | 71 (26) | 70 (26) | 1 (<1) |
| Constipation | 62 (23) | 62 (23) | 0 |
| Dizziness | 60 (22) | 59 (22) | 1 (<1) |
| Hypotension | 59 (22) | 51 (19) | 8 (3) |
| Cough | 56 (21) | 56 (21) | 0 |
| Vomiting | 56 (21) | 55 (21) | 1 (<1) |
| Hypokalemia | 52 (19) | 46 (17) | 6 (2) |
| Leukopenia | 43 (16) | 5 (2) | 38 (14) |

| | Grade 5 TEAE | | |
|---|---|---|---|
| Patients with TEAEs - no. (%) | 7 (3) | | |
| Diffuse alveolar damage‡,§ | 1 (<1) | | |
| Pulmonary hemorrhage§ | 1 (<1) | | |
| Multiple organ dysfunction syndrome§ | 1 (<1) | | |
| Leukoencephalopathy∥ | 1 (<1) | | |
| Septic shock∥ | 1 (<1) | | |
| Cardiomyopathy§ | 1 (<1) | | |
| Progressive multifocal leukoencephalopathy∥ | 1 (<1) | | |
| *LBCL denotes large B-cell lymphoma, LDC lymphodepleting chemotherapy, and TEAE treatment-emergent adverse event. | | | |
| †In at least 15% of patients. | | | |
| ‡Dose-limiting toxicity. | | | |
| §Considered related to both CAR+T cell compositions and LDC by the investigator. | | | |
| ∥Considered related to LDC (septic shock and leukoencephalopathy) or unrelated to both CAR+T cell compositions and LDC (progressive multifocal leukoencephalopathy). | | | |

| **Table E33A-2. Treatment-emergent adverse events in ≥25% of Subjects^{a}** | | |
|---|---|---|
| | **All LBCL Patients, N=269** | |
| | **Any Grade** | **Grade ≥3** |
| Any TEAEs, n (%) | 267 (99) | 213 (79) |
| Neutropenia | 169 (63) | 161 (60) |
| Anemia | 129 (48) | 101 (38) |
| Fatigue | 119 (44) | 4 (1) |
| Cytokine release syndrome | 113 (42) | 6 (2) |
| Nausea | 90 (33) | 4 (1) |
| Thrombocytopenia | 84 (31) | 72 (27) |
| Headache | 80 (30) | 3 (1) |
| Decreased appetite | 76 (28) | 7 (3) |
| Diarrhea | 71 (26) | 1 (<1) |
| ^{a}A TEAE was defined as an adverse event that started any time from initiation of CAR T cell administration through and including 90 days following the final cycle of CAR T cell. Any AE that occurred after the initiation of another anticancer treatment or CAR T cell retreatment was not considered a CAR T cell TEAE. | | |
| DLT, dose-limiting toxicity: TEAE, treatment-emergent adverse event. | | |

| **Table E33B-1. Treatment-Emergent Adverse Events of Special Interest** | |
|---|---|
| **Event** | **CAR+ T cell-Treated Patients N=268** |
| CRS^{†} | |
| Any grade - no. (%) | 113 (42) |
| Grade 3 - no. (%) | 4 (1) |
| Grade 4 - no. (%) | 2 (1) |
| Time to onset (days) - median (range) | 5 (1-14) |
| Time to resolution (days) - median (range) | 5 (1-17) |

| NEs^{†} | |
|---|---|
| Any grade - no. (%) | 80 (30) |
| Grade 3 - no. (%) | 23 (9) |
| Grade 4 - no. (%) | 4 (1) |
| Time to onset (days) - median (range) | 9 (1-66) |
| Time to resolution (days) - median (range) | 11 (1-86) |
| Tocilizumab and/or corticosteroid for CRS^{‡} - no. (%) | 53 (20) |
| Tocilizumab only | 27 (10) |
| Tocilizumab and corticosteroid | 21 (8) |
| Corticosteroid only | 5 (2) |
| Other medications for treatment of CRS - no. (%) | |
| Anakinra | 1 (<1) |
| Vasopressors | 7(3) |
| Siltuximab | 1 (<1) |
| Prolonged cytopenias^{§} - no. (%) | 100 (37) |
| Grade ≥3 infections^{∥} - no. (%) | 33 (12) |
| Hypogammaglobulinemia - no. (%) | 37 (14) |
| Tumor lysis syndrome - no. (%) | 2 (1) |
| Infusion-related reactions - no. (%) | 3 (1) |
| *CRS denotes cytokine release syndrome, LBCL large B-cell lymphoma, and NEs neurological events. ^{†}No grade 5 CRS or NEs occurred. CRS was graded according to Lee criteria.²¹ NEs were defined as investigator-assessed NEs related to CAR+T cell compositions. | |
| ^{‡}Patients with concurrent CRS and NEs may have received tocilizumab or corticosteroids for both events. | |
| ^{§}Prolonged cytopenias included grade ≥3 neutropenia, thrombocytopenia, or anemia not resolved at study Day 29 based on laboratory assessments. | |
| ^{∥}Grade ≥3 bacterial infections occurred in 11 (4%) patients; fungal infections in 2 (0.7%) patients and viral infections in 4 (1.5%) patients | |

| **Table E33B-2. Other Adverse Events of Special Interest.** | |
|---|---|
| **Incidence** | **All CAR-T cell-Treated Subjects N=269** |
| Prolonged grade ≥3 cytopenias,^{a} n (%) | 100 (37) |
| Grade ≥3 infections, n (%) | 33 (12) |
| Bacterial | 11 (4) |
| Viral | 4 (1) |
| Fungal | 2 (1) |
| Other | 16 (6) |

| Hypogammaglobulinemia, n (%) | |
|---|---|
| Adverse event reporting | 37 (14) |
| Laboratory assessment | XXX |
| Tumor lysis syndrome, n (%) | 2 (1) |
| Grade 3 or 4 | 2 (1) |
| Infusion-related reactions, n (%) | 3 (1) |
| Grade 3 or 4 | 0 |
| ^{a}Prolonged cytopenias included neutropenia, thrombocytopenia, or anemia not resolved at study Day 29 based on laboratory assessments. 55 (20.5%) subjects received IV immunoglobulin. | |

| **Table E33B-3. Incidence and Management of CRS and NEs.** | |
|---|---|
| | **All LBCL Patients N=269** |
| CRS^{a} | |
| Any grade, n (%) | 113 (42) |
| Grade 3, n (%) | 4 (1) |
| Grade 4, n (%) | 2 (1) |
| Time to onset (days), median (range) | 5 (1-14) |
| Time to resolution (days), median (range) | 5 (1-17) |

| NE^{b} | |
|---|---|
| Any grade, n (%) | 80 (30) |
| Grade 3, n (%) | 23 (9) |
| Grade 4, n (%) | 4 (1) |
| Time to onset (days), median (range) | 9 (1-66) |
| Time to resolution (days), median (range) | 11 (1-86) |
| ICU admissions, n(%) | 19(7) |
| For CRS and/or NE | 12(4) |
| Other reasons | 7(3) |
| ^{a}CRS was graded according to the Lee criteria (Lee DW, et al. *Blood* 2014;124:188-195); ^{b}NEs were based on investigator assessment and graded per National Cancer Institute Common Terminology Criteria for Adverse Events v4.03. | |

| **Table E33C. Treatment-Emergent Symptoms of Cytokine Release Syndrome (CRS)** | | |
|---|---|---|
| | **DLBCL-treated Patients, N=268** | |
| Patients with treatment-emergent CRS symptoms of any grade, n (%) | 113 (42) | |
| Patients with treatment-emergent CRS symptoms of grade ≥3, n (%) | 20 (7) | |

| **Patient Incidence, n (%)** | **Any grade** | **Grade 3 or 4** |
|---|---|---|
| Pyrexia | 107 (40) | 6 (2) |
| Hypotension | 55 (21) | 7 (3) |
| Tachycardia | 47 (18) | 0 |
| Chills | 34 (13) | 0 |
| Hypoxia | 26 (10) | 9 (3) |
| Fatigue | 17 (6) | 0 |
| Headache | 17 (6) | 3 (1) |
| Nausea | 8 (3) | 0 |
| Tachypnoea | 7 (3) | 1 (0.4) |
| Malaise | 6 (2) | 0 |
| Treatment-emergent was defined as a CRS symptom that occurred from the time of CAR+T cell composition infusion to within 90 days after the last dose of CAR+T cell composition. Any symptoms occurring after the initiation of another anticancer treatment or after retreatment were not considered treatment-emergent. Toxicity was graded using NCI CTCAE v4.03.³ | | |
| ^{a}Overall CRS grade is based on Lee et al⁴ and is not based on CTCAE grading of individual symptoms. | | |

| **Table E33D. Treatment Emergent Neurological Events by Group** | | |
|---|---|---|
| | **DLBCL-treated Patients, N=268** | |
| **Patient Incidence, n (%)^{a}** | **Any Grade** | **Grade 3 or 4** |
| Group: Encephalopathy | 57 (21) | 18 (7) |
| Confusional state | 30 (11) | 2 (0.7) |
| Encephalopathy | 17 (6) | 11 (4) |
| Mental status changes | 10 (4) | 4 (1.5) |
| Somnolence | 6 (2) | 1 (0.4) |
| Lethargy | 5 (2) | 0 |
| Other^{b} | 25 (9) | 2 (1) |
| Group: Aphasia | 26 (10) | 5 (2) |
| Aphasia | 22 (8) | 3 (1) |
| Dvsarthria | 5 (2) | 2 (1) |
| Other^{c} | 4 (1) | 0 |
| Group: Tremor | 26 (10) | 0 |
| Tremor | 25 (9) | 0 |
| Essential tremor | 1 (<1) | 0 |
| Group: Delirium | 16 (6) | 4 (1.5) |
| Agitation | 9 (3) | 3 (1) |
| Other^{d} | 11 (4) | 2 (1) |
| Group: Dizziness | 11 (4) | 2 (0.7) |
| Dizziness | 11 (4) | 1 (<1) |
| Syncope | 1 (<1) | 1 (<1) |
| Group: Headache | 9 (3) | 2 (1) |
| Headache | 9 (3) | 2 (1) |
| Migraine | 1 (<1) | 0 |
| Group: Ataxia/Gait Disturbance | 8 (3) | 1 (<1) |
| Ataxia | 6 (2) | 1 (<1) |
| Gait disturbance | 2 (1) | 0 |
| Group: Cerebellar | 6 (2) | 0 |
| Other^{e} | 9 (3) | 0 |
| Neurological events were defined as investigator-assessed neurological events related to CAR+T cell compositions and were graded using National Cancer Institute Common Terminology Criteria for Adverse Events v4.03⁶ | | |
| ^{a}Patients could experience more than one event. | | |
| ^{b}Other events occurred in 4 or fewer patients and included amnesia, cognitive disorder, depressed level of consciousness, memory impairment, flat effect, depersonalization or derealization disorder, disturbance in attention, incoherent, and hypersomnia. | | |
| ^{c}Other events occurred in 4 or fewer patients and included dysphemia, dysphonia, slow speech, and speech disorder. | | |
| ^{d}Other events occurred in 4 or fewer patients and included delirium, disorientation, delusion, hallucination, visual hallucination, and irritability. | | |
| ^{e}Other events occurred in 4 or fewer patients and included cerebellar syndrome, dysmetria, balance disorder, dyskinesia, and impaired hand-eye coordination. | | |

| **Table E33E-1. Comparison of Adverse Events by Dose Level.** | | | | | |
|---|---|---|---|---|---|
| | **DL1** | **DL1D** | **DL2** | **DL3** | **Total** |
| **LBCL-treated patients** | **n=45** | **n=6** | **n=176** | **n=41** | **N=268** |
| Any TEAE, n (%) | 44 (98) | 6 (100) | 176 (100) | 40 (98) | 266 (99) |
| Grade 3 or 4 | 35 (78) | 5 (83) | 134 (76) | 31 (76) | 205 (76) |
| Grade 5 | 1 (2) | 0 | 5 (3) | 1 (2) | 7 (3) |
| Serious AE | 16 (36) | 3 (50) | 83 (47) | 20 (49) | 122 (45.5) |

| CRS, n (%) | | | | | |
|---|---|---|---|---|---|
| Any grade | 18 (40) | 3 (50) | 66 (37.5) | 26 (63) | 113 (42) |
| Grade 3 or 4 | 1 (2) | 0 | 5 (3) | 0 | 6 (2) |

| NEs, n (%) | | | | | |
|---|---|---|---|---|---|
| Any grade | 10 (22) | 1 (17) | 53 (30) | 16 (39) | 80 (30) |
| Grade 3 or 4 | 7 (16) | 1 (17) | 16 (9) | 3 (7) | 27 (10) |
| AE, adverse event; CR, complete response; CRS, cytokine release syndrome; DL1, dose level 1 (single dose); DL1D, dose level 1 (2 doses); DL2, dose level 2 (single dose); DL3, dose level 3 (single dose); NEs, neurological events; ORR, objective response rate; TEAE, treatment-emergent adverse event. | | | | | |

| **Table E33E-1-2. Comparison of Adverse Events Across Single Dose Levels.** | | | |
|---|---|---|---|
| | **DL1** | **DL2** | **DL3** |
| **LBCL-treated patients** | **n=45** | **n=177** | **n=41** |
| Any TEAE, n (%) | 44 (98) | 177 (100) | 40 (98) |
| Grade 3 or 4 | 35 (78) | 135 (76) | 31 (76) |
| Grade 5 | 1 (2) | 5 (3) | 1 (2) |
| Serious AE | 16 (36) | 83 (47) | 20 (49) |

| CRS, n (%) | | | |
|---|---|---|---|
| Any grade | 18 (40) | 66 (37.5) | 26 (63) |
| Grade 3 or 4 | 1 (2) | 5 (3) | 0 |

| NEs, n (%) | | | |
|---|---|---|---|
| Any grade | 10 (22) | 53 (30) | 16 (39) |
| Grade 3 or 4 | 7 (16) | 16 (9) | 3 (7) |
| AE, adverse event; CR, complete response; CRS, cytokine release syndrome; DL1, dose level 1 (single dose); DL2, dose level 2 (single dose); DL3, dose level 3 (single dose); NEs, neurological events; TEAE, treatment-emergent adverse event. | | | |

| **Table E33F. Comparison of Safety in Patient Subgroups** | | | | | | |
|---|---|---|---|---|---|---|
| | | **TEAEs** | | **CRS** | **NEs** | |
| **Patient Incidence, n (%)** | **n** | **Grade 3-5** | **Any Grade** | **Grade 3 or 4** | **Any Grade** | **Grade 3 or 4** |
| Age | | | | | | |
| <65 years | 157 | 121 (77) | 72 (46) | 5 (3) | 46 (29) | 15 (10) |
| ≥65 years | 111 | 91 (82) | 41 (37) | 1 (1) | 34 (31) | 12 (11) |
| <75 years | 241 | 186 (77) | 101 (42) | 5 (2) | 68 (28) | 22 (9) |
| ≥75 years | 27 | 26 (96) | 12 (44) | 1 (4) | 12 (44) | 5 (19) |
| Pre-LDC CrCl | | | | | | |
| ≥60 mL/min | 217 | 165 (76) | 86 (40) | 5 (2) | 58 (27) | 17 (8) |
| <60 mL/min | 51 | 47 (92) | 27 (53) | 1 (2) | 22(43) | 10 (20) |
| LVEF at screening | | | | | | |
| ≥40% to <50% | 13 | 9 (69) | 4 (31) | 0 | 4 (31) | 1 (8) |
| ≥50% | 255 | 203 (80) | 109 (43) | 6 (2) | 76 (30) | 26 (10) |

| Response to prior therapy | | | | | | |
|---|---|---|---|---|---|---|
| Chemotherapy-refractory | 180 | 142 (79) | 76 (42) | 6 (3) | 50 (28) | 20 (11) |
| Chemotherapy-sensitive | 88 | 70 (80) | 37 (42) | 0 | 30 (34) | 7 (8) |

| Secondary CNS lymphoma | | | | | | |
|---|---|---|---|---|---|---|
| Yes | 7 | 7 (100) | 2 (29) | 0 | 2 (29) | 2 (29) |
| No | 261 | 205 (79) | 111 (42.5) | 6 (2) | 78 (30) | 25 (10) |
| Prior HSCT | | | | | | |
| Yes | 94 | 75 (80) | 36 (38) | 2 (2) | 29 (31) | 6 (6) |
| No | 174 | 137 (79) | 77 (44) | 4(2) | 51 (29) | 21 (12) |

| Received Bridging therapy | | | | | | |
|---|---|---|---|---|---|---|
| Yes | 159 | 137 (86) | 81 (51) | 5 (3) | 53 (33) | 21 (13) |
| No | 109 | 75 (69) | 32 (29) | 1 (0.9) | 27 (25) | 6 (5.5) |
| Pre-LDC SPD | | | | | | |
| ≥50 cm² | 72 | 63 (88) | 43 (60) | 3 (4) | 31 (43) | 12 (17) |
| <50 cm² | 184 | 141 (77) | 66 (36) | 3 (2) | 47 (25.5) | 15 (8) |
| Baseline CRP | | | | | | |
| <20 mg/L | 117 | 86 (74) | 35 (30) | 1 (0.9) | 23 (20) | 5 (4) |
| ≥20 mg/L | 150 | 125 (83) | 78 (52) | 5 (3) | 57 (38) | 22 (15) |
| Outpatients | 25 | 17 (68) | 12 (48) | 1 (4) | 11 (44) | 2 (8) |
| Received retreatment^{a} | 15 | 12 (80) | 5 (33) | 1 (8) | NA | NA |
| Received nonconforming product | 24 | 20 (83) | 7 (29) | 1 (4) | 3 (12.5) | 1 (4) |
| CNS, central nervous system; CR, complete response; CrCl, creatinine clearance; CRP, C-reactive protein; HSCT, hematopoietic stem cell transplantation; LCD, lymphodepleting chemotherapy; LVEF, left ventricular ejection fraction; NA, not available; NE, neurological events; SPD, sum of product of perpendicular diameters; TEAE, treatment-emergent adverse events. | | | | | | |
| ^{a}Patients who achieved CR after initial CAR+T cell composition infusion but subsequently developed progressive disease. | | | | | | |

| **Table E33G. Efficacy for Outpatients, Retreated Patients and Non-Conforming Product.** | | | |
|---|---|---|---|
| | **Outpatients** | **Retreated Patients^{a}** | **Patients Receiving Nonconforming Product^{b}** |
| | **n=25** | **n=15** | **n=24** |
| Safety, n % | | | |
| Any TEAE | 25 (100) | 14 (93) | 24 (100) |
| Grade 3-4 | 17 (68) | 12 (80) | 18 (75) |
| Grade 5 | 0 | 1 (7) | 1 (4)^{c} |
| Serious Aes | 18 (72) | NA | 11 (46) |

| CRS, n (%) | | | |
|---|---|---|---|
| Any grade | 12 (48) | 5 (33) | 7 (29) |
| Grade ≥3 | 1 (4) | 1 (7) | 1 (4) |

| NEs, n (%) | | | |
|---|---|---|---|
| Any grade | 11 (44) | NA | 3 (12.5) |
| Grade ≥3 | 2 (8) | NA | 1 (4)^{c} |
| CI, confidence interval; CR, complete response; CRS, cytokine release syndrome; NA, not available; NEs, neurological events; NR, not reached; ORR, objective response rate. | | | |
| ^{a}All analyses were based on safety and efficacy outcomes after retreatment. | | | |
| ^{b}Nonconforming product was defined as any product wherein one or both components did not meet the release specifications for CAR+T cell compositions. | | | |
| ^{c}One patient had a grade 5 TEAE of mucormycosis that started and ended on study Day 4 and which was not considered related to lymphodepleting chemotherapy. | | | |
| ^{d}Per investigator assessment. | | | |

### E. Efficacy and Response Outcomes

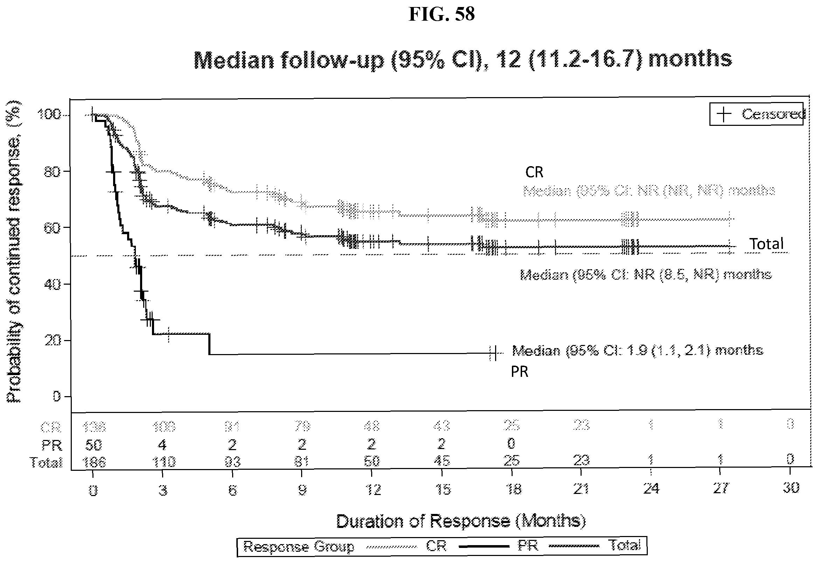

The efficacy and response outcomes of the subjects treated at the timepoint are set forth in **Table E33H-1.** Response and durability by IRC assessment are described in **Table E33H-2.** Probability of continued response and duration of response is described in **FIG. 58****.** Median follow-up (95% CI) was 12 months (11.2-16.7 range).

Among subjects evaluable for response (n=255), ORR was 73% (95% CI, 67-78); the CR rate was 53% (95% CI, 47-59). Responses were similar across all subject subgroups, e.g., including based on disease subtypes, age (e.g., subjects 65 years or older), subjects with high tumor burden, and chemotherapy-sensitive and -refractory status, as shown in **Table E33H. Table E331-1** and **E331-2** show comparison of efficacy by dose level. **Table E33J** shows a comparison of efficacy according to analysis set. **Table E33K** shows efficacy for outpatients, retreated patients and those who received non-conforming product. Efficacy was similar across a narrow range of DLs, with no statistically significant difference between individual DLs for ORR (P=0.68), PFS (P=0.46), and DOR (P=0.76), or between analysis sets. The prespecified primary endpoint of ORR for the PAS was met. Retreatment with CAR+T cell compositions (n=15) resulted in a low ORR (20%). Efficacy among patients who received nonconforming product (n=24) was similar to those who received CAR+ T cell compositions.

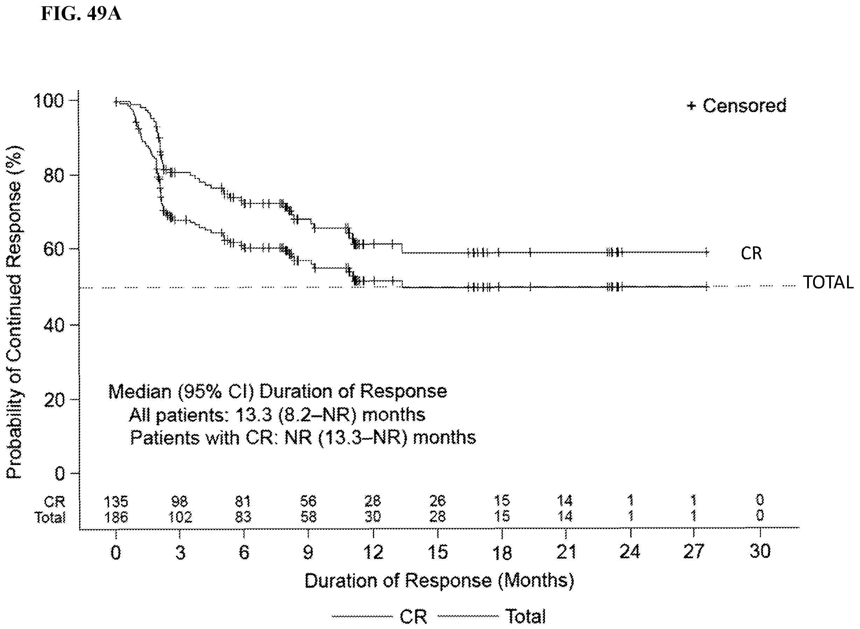

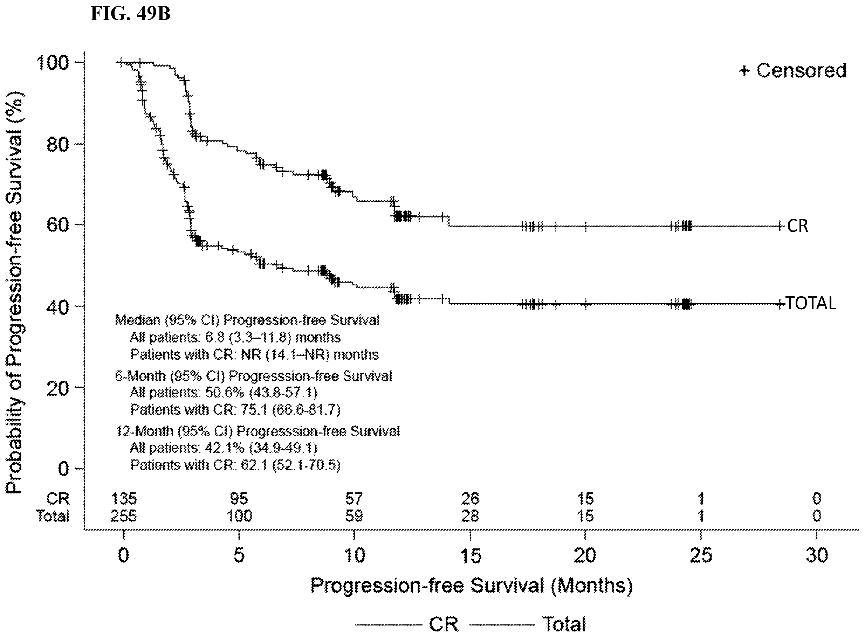

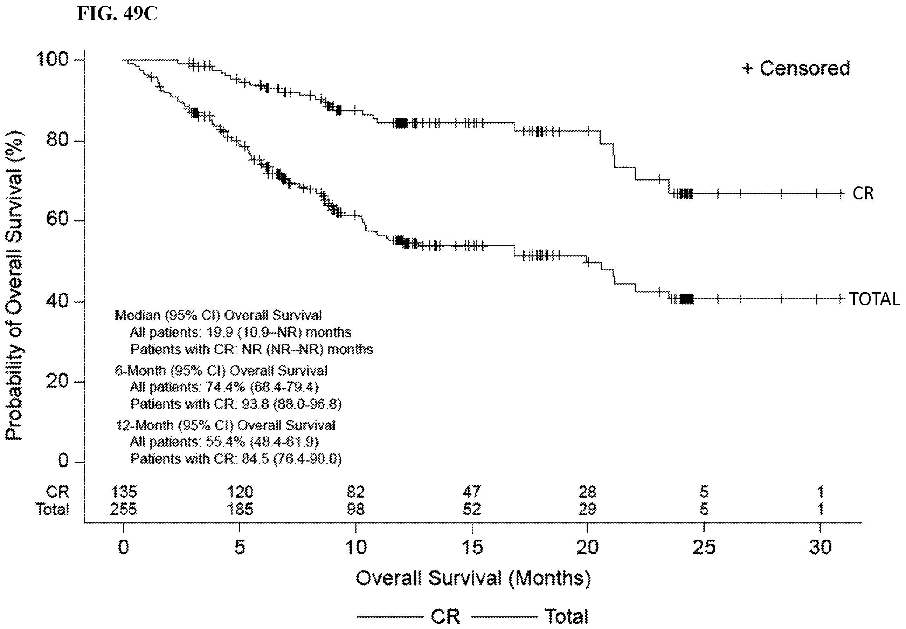

Responses were rapid and durable, with a median time to first response of 1 month (range, 0.7-6.0) and median DOR of 13.3 months (95% CI, 8.2-not reached [NR]) at median follow-up of 10.8 months. The median DOR in patients with CR was not reached (95% CI, 13.3-NR) **(****FIG. 49A****).** Median PFS was 6.8 months (95% CI, 3.3-11.8). Among patients with CR, the median PFS was not reached (95% CI, 14.1-NR) **(****FIG. 49B****).** The median PFS ratio was 0.72. Overall, PFS after CAR⁺ T cell infusion was substantially longer than PFS from the immediate prior therapy. The median OS was 19.1 months, with a median follow-up of 12.2 months, and the estimated 12-month OS was 55% (95% CI, 48.4-61.9). Among patients achieving CR, the estimated median OS was not reached (95% CI, NR-NR) **(****FIG. 49C****).** Efficacy was similar across a narrow range of DLs **(Table E331),** with no statistically significant difference between individual DLs for ORR (P=0.68), PFS (P=0.46), and DOR (P=0.76), or between analysis sets **(Table E33J).** Retreatment with CAR-T cell compositions (n=15) resulted in a low ORR (20%). Efficacy among patients who received nonconforming product (n=24) was similar to those who received CAR+T cell compositions **(Table E33K).**

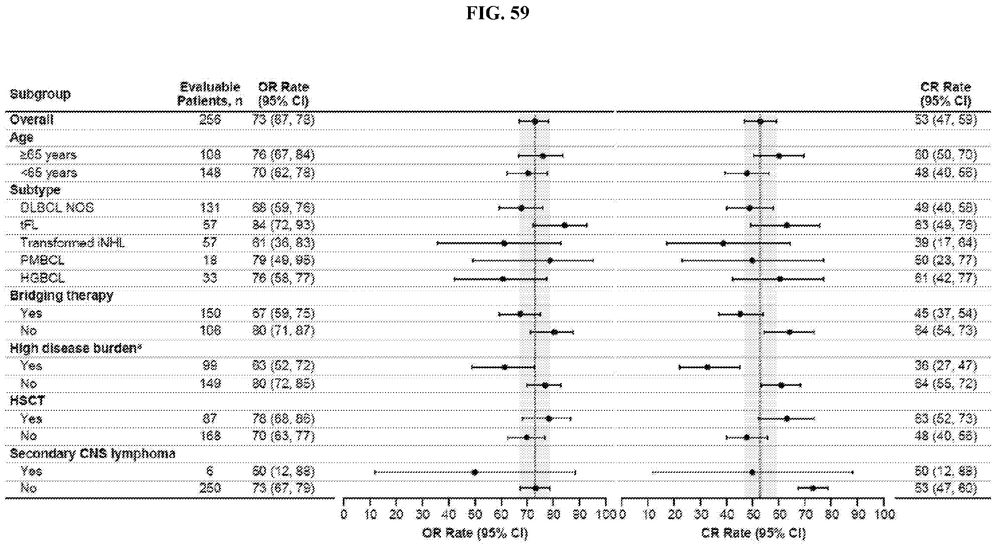

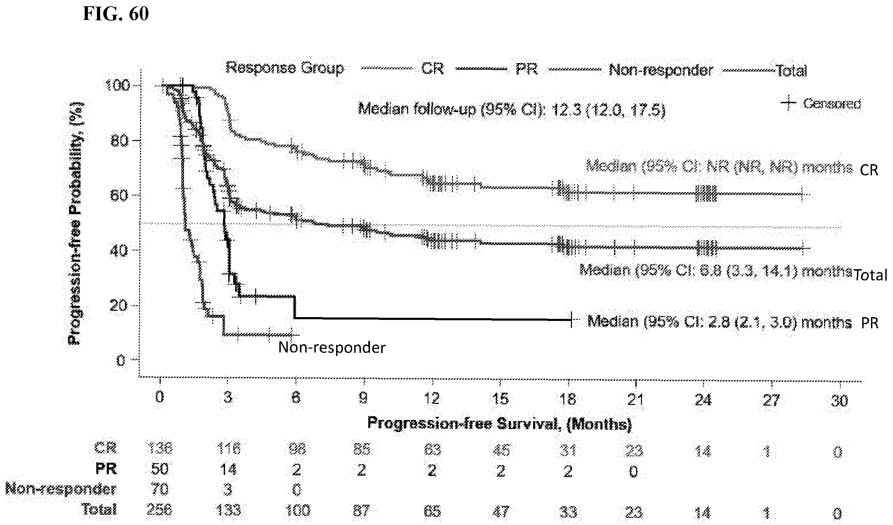

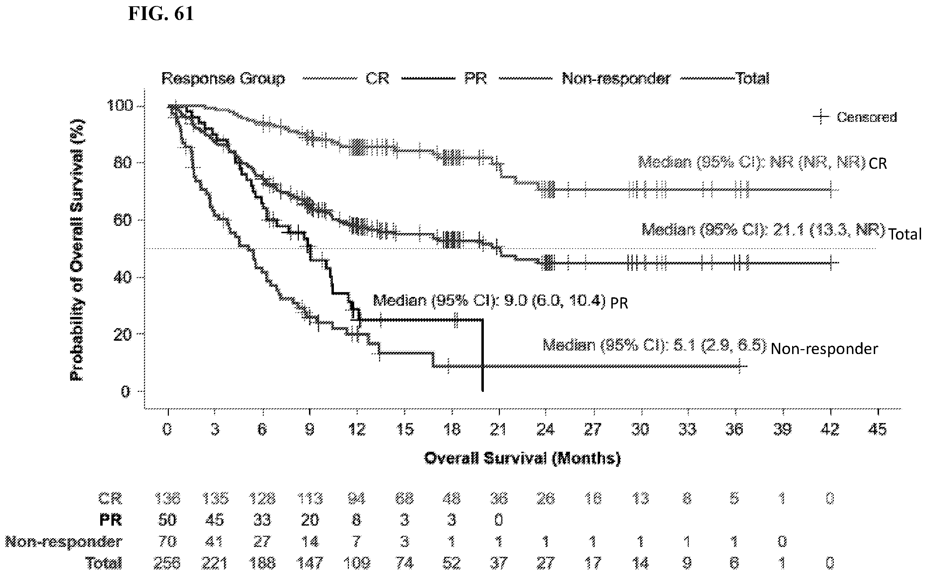

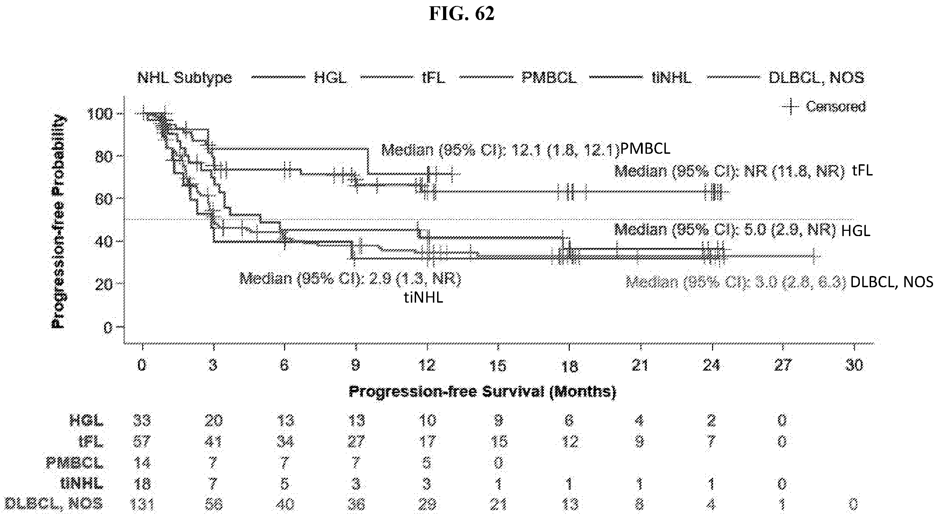

As shown in **FIG. 59****,** ORR did not differ by age, LBCL subtype, prior HSCT, or secondary CNS lymphoma. Subjects needing bridging therapy and those with high disease burden trended toward lower ORR. The pattern of CR rates for the subgroups was similar to that of ORR. PFS **(****FIG. 60** **and Table E33L)** and OS **(****FIG. 61** **and Table E33M)** were evaluated by objective response. At this time point of analysis, 37% of subjects had 12 months of on-study follow-up and 68% had 6 months of on-study follow up. PFS by histologic subtype is shown in **FIG. 62****.**

| **Table E33H-1. Response Outcomes** | | | |
|---|---|---|---|
| **Response** | **Evaluable Subjects N=255** | | **Evaluable Subjects N=256** |
| Response | ORR, n (%) | CR, n (%) | CR, n (%) |
| DLBCL cohort, all subjects | 186 (73) | 135 (53) | 136(53) |
| DLBCL NOS (n=131) | 89 (68) | 63 (48) | 64 (49) |
| Transformed from FL (n=57) | 48 (84) | 36 (63) | 36(63) |
| Transformed from indolent lymphoma (n=18) | 11 (61) | 7 (39) | 7(39) |
| PMBCL (n=14) | 11 (79) | 7 (50) | 7(50) |
| HGBCL (n=33) | 25 (76) | 20 (61) | 20(61) |
| FL3B (n=2) or (n=3) | 2 (100) | 2 (100) | 2(67) |
| Age ≥65 years (n=107) or (n=108) | 83 (78) | 65 (61) | 65(60) |
| LDH ≥500 U/L (n=57) | 36 (63) | 23 (40) | 23 (40) |
| SPD ≥50 cm² (n=69) or (n=70) | 43 (62) | 23 (330 | 23(33) |
| Chemosensitive (n=85) | 66 (78) | 46 (54) | NA |
| Chemorefractory (n=170) or (n=171) | 120 (71) | 89 (52) | 90(53) |
| Received prior HSCT (n=87) | 68 (78) | 55 (63) | 55(63) |
| Received bridging therapy (n=150) | 101 (67) | 67 (45) | 68(45) |
| ^{a} Prolonged cytopenias were defined as grade ≥3 laboratory assessments at study day 29. CR, complete response; CRS, cytokine release syndrome; DLBCL, diffuse large B cell lymphoma; FL, follicular lymphoma; FL3B, follicular lymphoma grade 3B; HGBCL, high-grade B cell lymphoma; HSCT, hematopoietic stem cell transplantation; LDH, lactate dehydrogenase; NE, neurological events; NOS, not otherwise specified; ORR, overall response rate; PMBCL, primary mediastinal B cell lymphoma; SPD, sum of the product of the greatest diameters; TEAEs, treatment-emergent adverse events. | | | |

| **Table E33H-2. Response and durability by IRC Assessment** | |
|---|---|
| **Efficacy-Evaluable Patients N=255** | |
| ORR (95% CI) | 73% (67-78) |
| CR rate (95% CI) | 53% (47-59) |
| Time to first CR or PR, median (range) | 0.95 (0.7-8.9) |
| DOR median (95% CI), months^{a} | |
| DOR at 6 months (95% CI) | 60.4 (52.6-67.3) |
| DOR at 12 months (95% CI) | 54.7 (46.7-62.0) |
| CI, confidence interval; CR, complete response; DOR, duration of response; NR, not reached; ORR, objective response rate. | |

| **Table E331-1. Comparison of Efficacy by Dose Level.** | | | | | |
|---|---|---|---|---|---|
| | **DL1** | **DL1D** | **DL2** | **DL3** | **Total** |
| **LBCL-treated efficacy-evaluable patients** | **n=40** | **n=6** | **n=168** | **n=41** | **N=255** |
| ORR, n (%) | 27 (67.5) | 4 (67) | 125 (74) | 30 (73) | 186 (73) |
| CR, n (%) | 24 (60) | 3 (50) | 88 (52) | 20 (49) | 135 (53) |
| AE, adverse event; CR, complete response; CRS, cytokine release syndrome; DL1, dose level 1 (single dose); DL1D, dose level 1 (2 doses); DL2, dose level 2 (single dose); DL3, dose level 3 (single dose); NEs, neurological events; ORR, objective response rate; TEAE, treatment-emergent adverse event. | | | | | |

| **Table E331-1-2. Comparison of Efficacy by Dose Level.** | | | |
|---|---|---|---|
| | **DL1** | **DL2** | **DL3** |
| **Efficacy-evaluable patients** | **n=40** | **n=169** | **n=41** |
| ORR, n (%) | 27 (67.5)^{a} | 125 (74) | 30 (73) |
| CR, n (%) | 24 (60) | 88 (52) | 21 (51) |
| ^{a}All percentages are rounded unless the decimal equals 0.5. CR, complete response; DL, dose level; ORR, objective response rate. | | | |

| **Table E33J. Comparison of Efficacy According to Analysis Set.** | | | |
|---|---|---|---|
| | **Analysis Set** | | |
| | **LBCL-Efficacy^{a} N=255** | **PAS^{b} N=133** | **Intent-to-Treat^{c} N=342** |
| Response, n (%) | | | |
| ORR | 186 (73) | 99 (74) | 208 (61) |
| CR | 135 (53) | 72 (54) | 149 (44) |
| PR | 51 (20) | 27 (20) | 59 (17) |
| SD | 28 (11) | 13 (10) | 32 (9) |
| PD | 28 (11) | 14 (11) | 33 (10) |
| DOR (months), median (95% CI) | 13.3 (8.2-NR) | 11.1 (8.0-NR) | 13.3 (8.2-NR) |
| PFS (months), median (95% CI) | 6.8 (3.3-11.8) | 9.0 (3.1-14.1) | 4.8 (4.3-7.1) |
| 6-month PFS, % | 50.6 | 53.0 | 46.3 |
| OS (months), median (95% CI) | 19.9 (10.9-NR) | 19.9 (10.4-NR) | 12.8 (10.6-21.1) |
| 6-month OS, % | 74.4 | 72.9 | 70.5 |
| CI, confidence interval; CR, complete response; DOR, duration of response; LBCL, large B-cell lymphoma; NR, not reported; ORR, objective response rate; OS, overall survival; PD, progressive disease; PFS, progression-free survival; PR, partial response; SD, stable disease. | | | |
| ^{a}LBCL-treated efficacy-evaluable set included all patients in the LBCL-treated set who had PET-positive disease present before CAR+T cell composition administration based on independent review committee assessment. | | | |
| ^{b}Primary analysis set (PAS) was a subset of the LBCL-treated analysis set treated at DL2S. | | | |
| ^{c}Intent-to-treat analysis set included all patients who were leukapheresed. | | | |

| **Table E33K. Efficacy Outcomes for Outpatients, Retreated Patients and Non-Conforming Product.** | | | |
|---|---|---|---|
| | **Outpatients n=25** | **Retreated Patients^{a} n=15** | **Patients Receiving Nonconforming Product^{b} n=24** |
| Efficacy, % (95% CI) | | | |
| ORR | 80 (59.3-93.2) | 20 (4.3-48.1)^{d} | 62.5 (40.6-81.2) |
| CR | 56 (34.9-75.6) | 13 (1.7-40.5)^{d} | 42 (22.1-63.4) |
| Duration of response, median (95% CI), months | NR (2.4-NR) | NA | NR (3.1-NR) |
| Progression-free survival, median (95% CI), months | 5.4 (3.0-NR) | NA | 4.6 (3.1, NR) |
| CR, complete response; NA, not available; NR, not reached; ORR, objective response rate. ^{d}Per investigator assessment. | | | |

| **Table E33L. PFS Evaluated by Objective Response** | |
|---|---|
| | **PFS** |
| 6 month (95% Cl), % | |
| All Subjects | 51.4 (44.6-57.7) |
| Subjects with CR | 76.1 (67.9-82.4) |

| 12 month (95% Cl), % | |
|---|---|
| All Subjects | 44.1 (37.3-50.7) |
| Subjects with CR | 65.1 (56.1-72.7) |

| **Table E33M. OS Evaluated by Objective Response** | |
|---|---|
| | **OS** |
| 6 month (95% Cl), % | |
| All Subjects | 74.4 (68.4-79.4) |
| Subjects with CR | 94.1(88.6-97.0) |

| 12 month (95% Cl), % | |
|---|---|
| All Subjects | 57.9 (51.3-63.8) |
| Subjects with CR | 85.5 (78.2-90.5) |

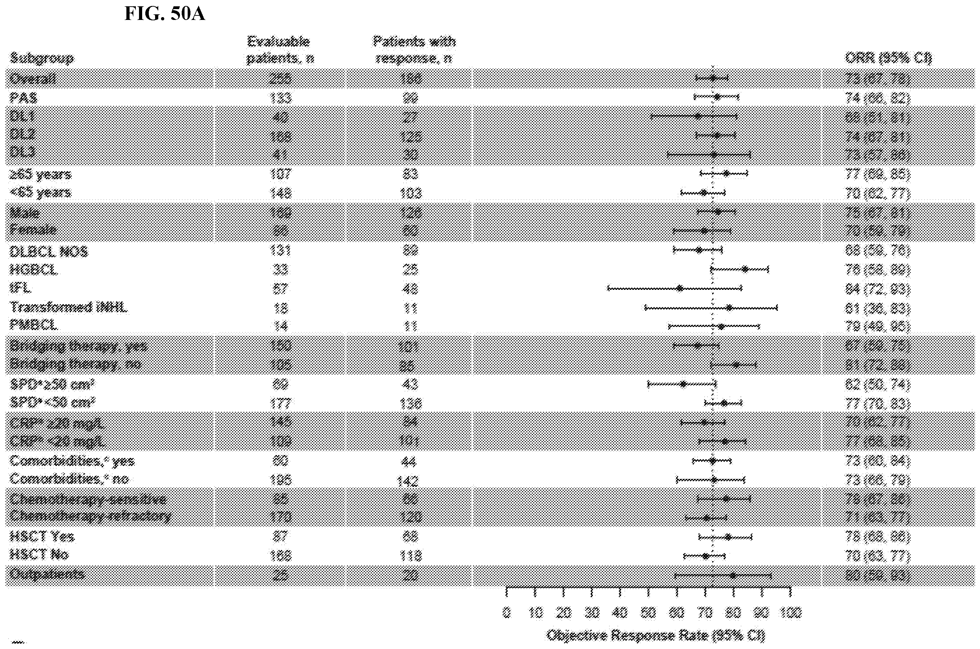

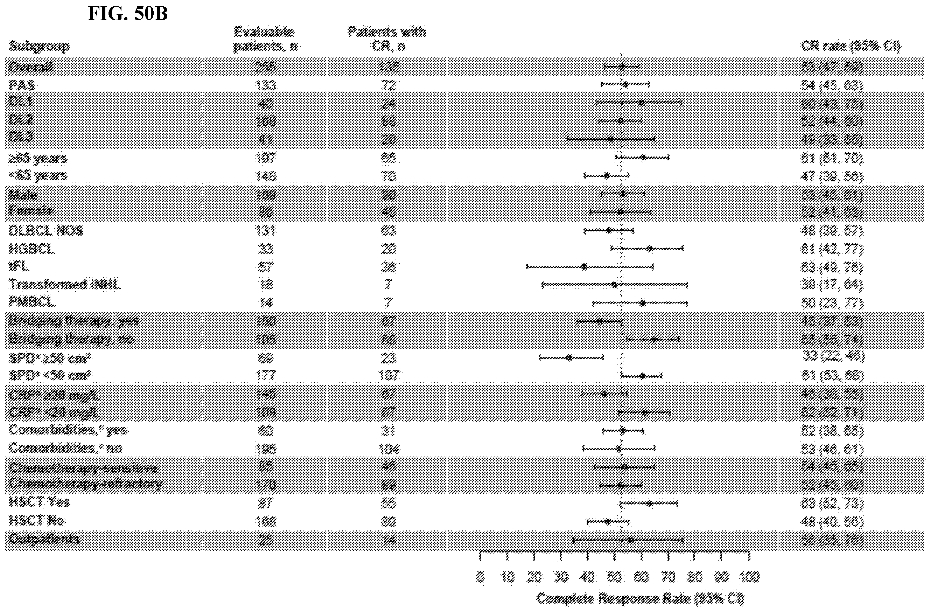

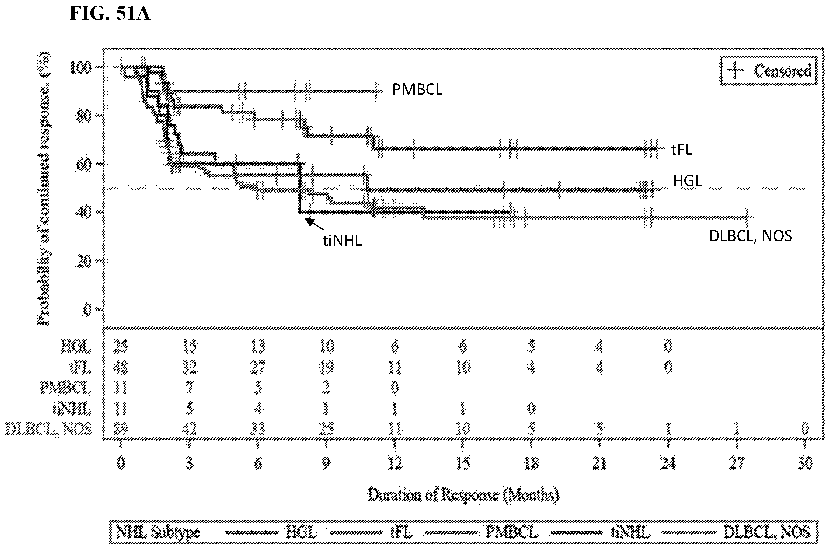

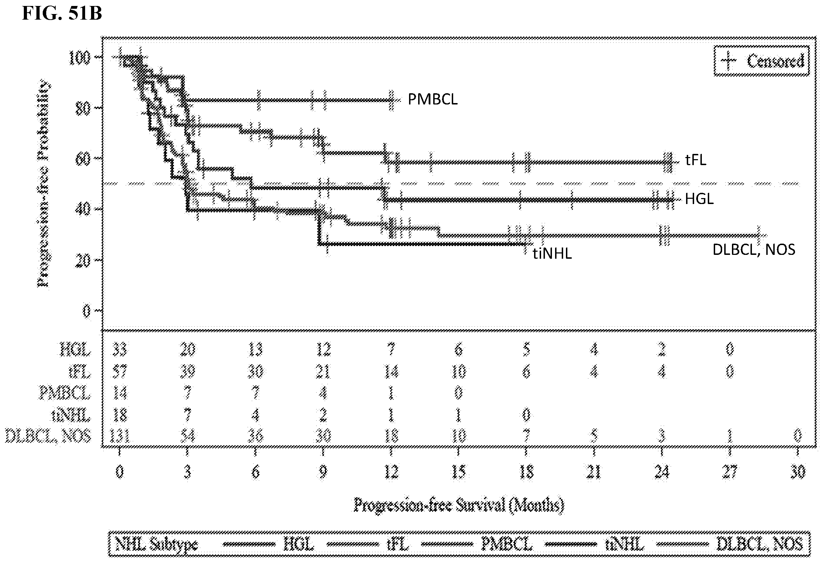

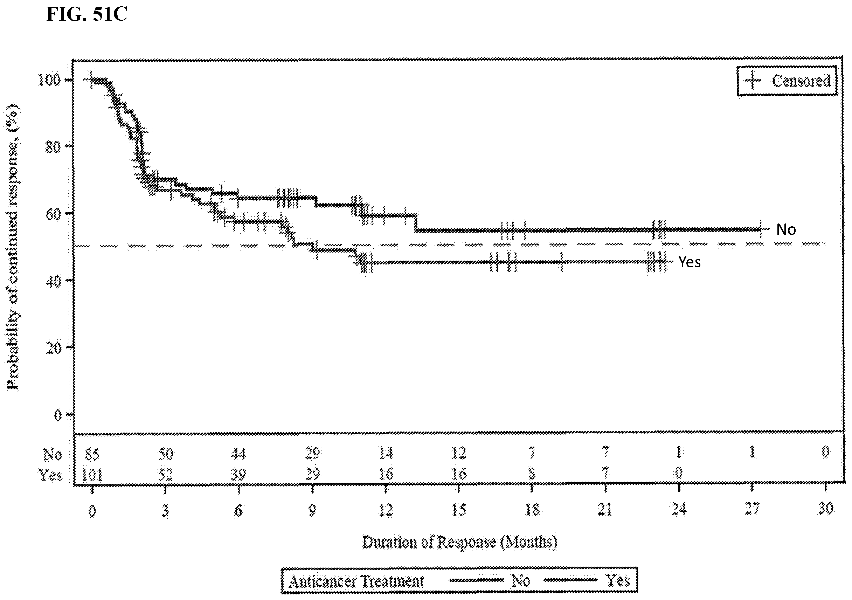

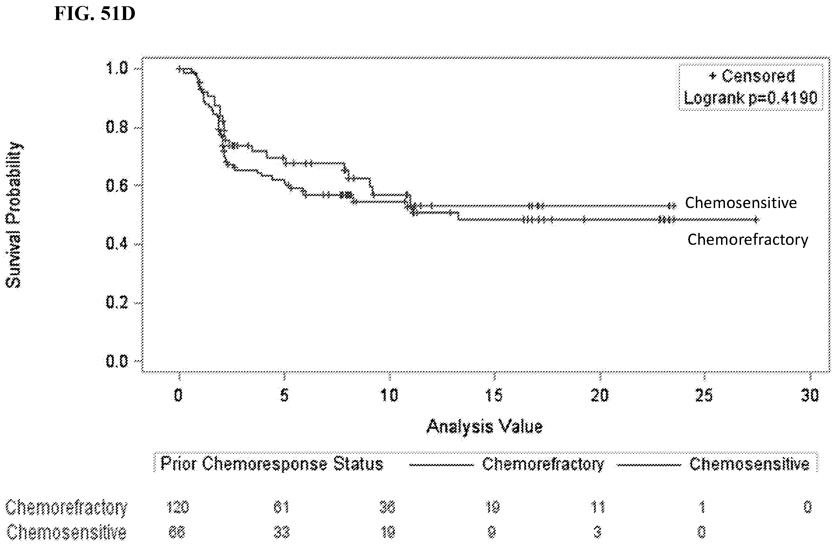

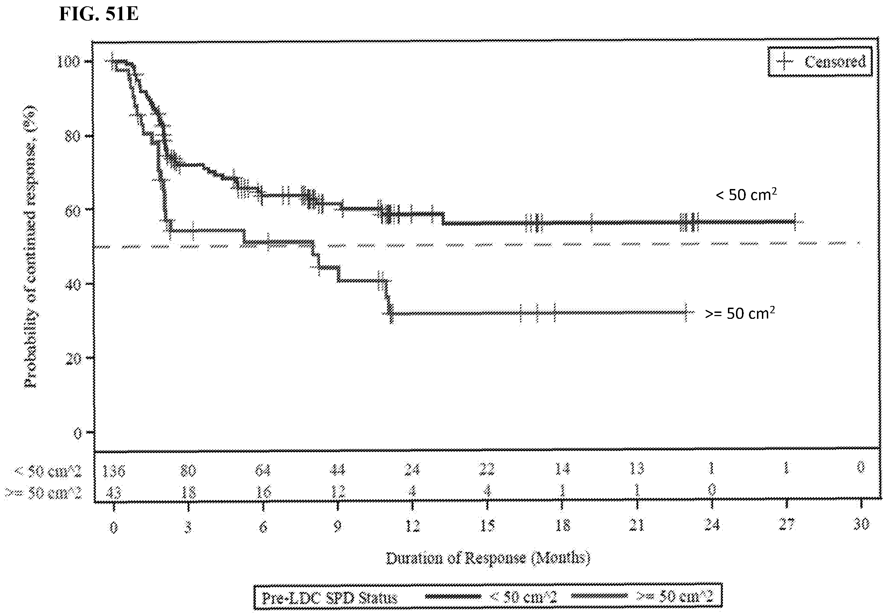

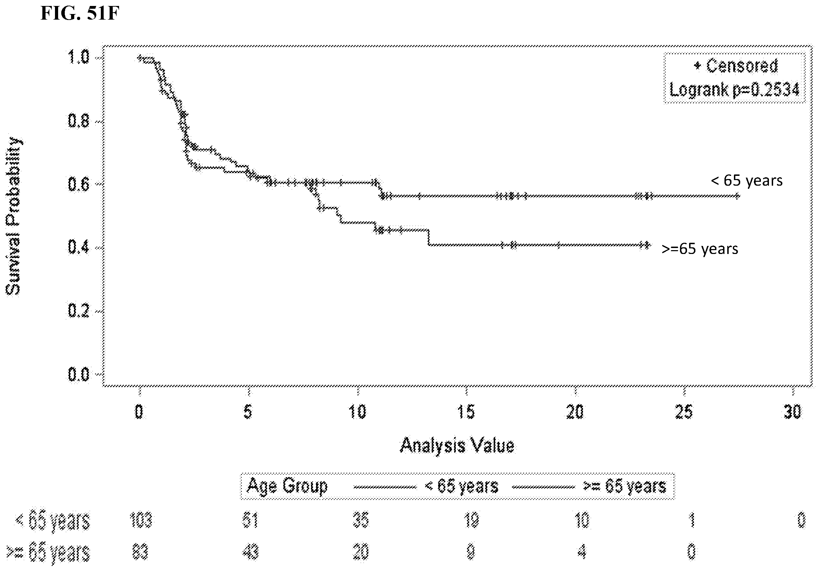

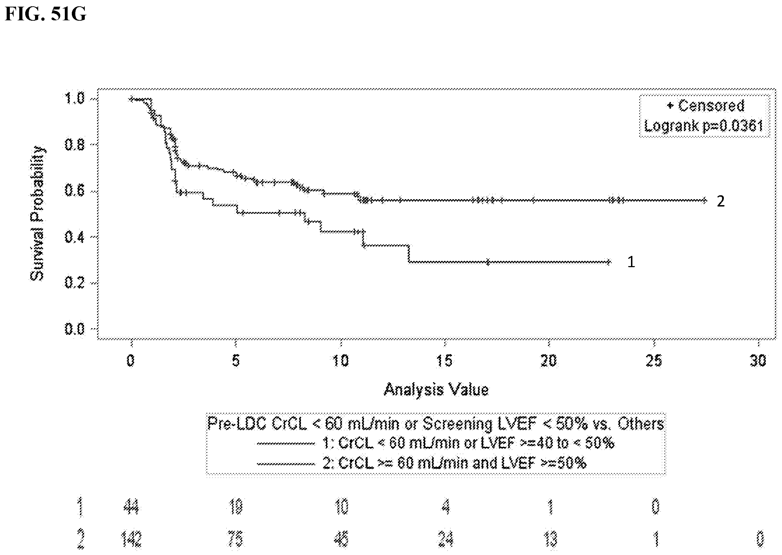

Responses were observed across subgroups, including outpatient infusion/monitoring and high-risk patients, such as uncommon histologic subtypes and HGBCL, age ≥65 years, chemotherapy-refractory disease, and those requiring bridging therapy. Among 6 patients with secondary CNS lymphoma evaluable for response, 3 achieved a CR. Similarly, responses were durable in high-risk subgroups of age ≥65 years, HGBCL, chemotherapy-refractory disease, and secondary CNS lymphoma **(****FIGS. 50A-50B****).** However, for patients with high tumor burden, PFS and DOR were shorter. As shown in **Table E33N,** PFS was shorter in patients requiring bridging therapy. **FIG. 51A** shows duration of response and progression free survival. **FIG. 51B** shows duration of response by histologic group. **FIG. 51C** shows duration of response by bridging therapy. **FIG. 51D** shows duration of response by chemotherapy-sensitive versus chemotherapy-refractory status. **FIG. 51E** shows duration of response by pre-LDC SPD status. **FIG. 51F** shows duration of response by age group. **FIG. 51G** shows duration of response in subjects with versus without comorbidities.

| **Table E33N. DOR and PFS by IRC Assessment.** | | | | |
|---|---|---|---|---|
| **Parameter** | **DOR** | | **PFS** | |
| | **Hazard Ratio (95% CI)** | **P value^{b}** | **Hazard Ratio (95% CI)** | **P value^{b}** |
| Pre-LDC SPD ≥50 cm² (reference: SPD <50 cm²) | 1.88 (1.16-3.06) | 0.0111 | 1.77 (1.22-2.58) | 0.0028 |
| Bridging therapy (reference: no) | 1.37 (0.87-2.16) | 0.1805 | 1.45 (1.01-2.09) | 0.0436 |
| Responded to last prior therapy (reference: refractory) | 0.68 (0.39-1.20) | 0.1886 | 0.68 (0.43-1.08) | 0.1015 |
| CI, confidence interval; DOR, duration of response; IRC, independent review committee; LBCL, large B-cell lymphoma; LDC, lymphodepleting chemotherapy; PFS, progression-free survival; SPD, sum of product of perpendicular diameters. | | | | |
| ^{a}Ad hoc analysis (Cox proportional hazards model) using the LBCL-treated efficacy evaluable analysis set. ^{b}P values are descriptive. | | | | |

### F. Cellular Kinetics, B cell Aplasia and Immunogenicity

Cellular kinetics of CAR + T cells in blood were based on all patients in the LBCL-treated cohort who received CAR + T cells as a single infusion and who had baseline (before CAR + T cell infusion) and post-baseline measurements at least in the first 28 days. Data were assessed using a validated real-time quantitative PCR assay for detection of the CAR + T cell transgene.

B-cell aplasia was defined as CD19+ B-cells representing <3% of peripheral blood lymphocytes (Mueller KT et al. Clin Cancer Res. 2018.;24:6175-84)

Anti-CAR-expressing T cell antibodies were detected using a electrochemiluminescence immunoassay. Immunogenicity was assessed by calculating the prevalence of anti-CAR-expressing T cell antibodies (pre-existing antibodies that bind to CAR-expressing T cells measured before the first CAR-expressing T cell infusion) and the incidence of anti-CAR-expressing T cells antibodies (treatment-induced or treatment-boosted antibodies that bind to CAR-expressing T cells at any time point after the first infusion of CAR-expressing T cells).

Among 238 evaluable patients, median time to CAR T-cell peak expansion across all DLs was 12 days (interquartile range, 10-15); median maximum expansion (Cₘₐₓ) was 23,963.7 copies/µg, and median area under the curve during the first 28 days following infusion (AUC_{0-28d}) was 214,283.0 day*copies/µg. Expansion was similar across all DLs **(Table E33O**).

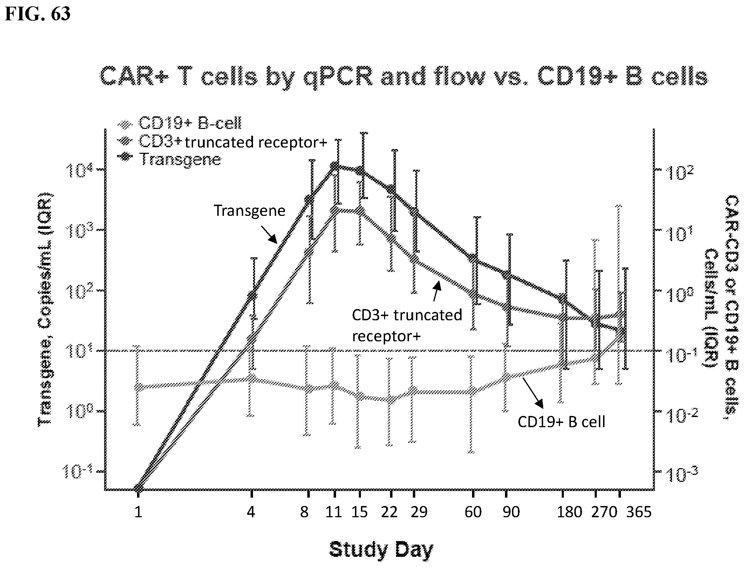

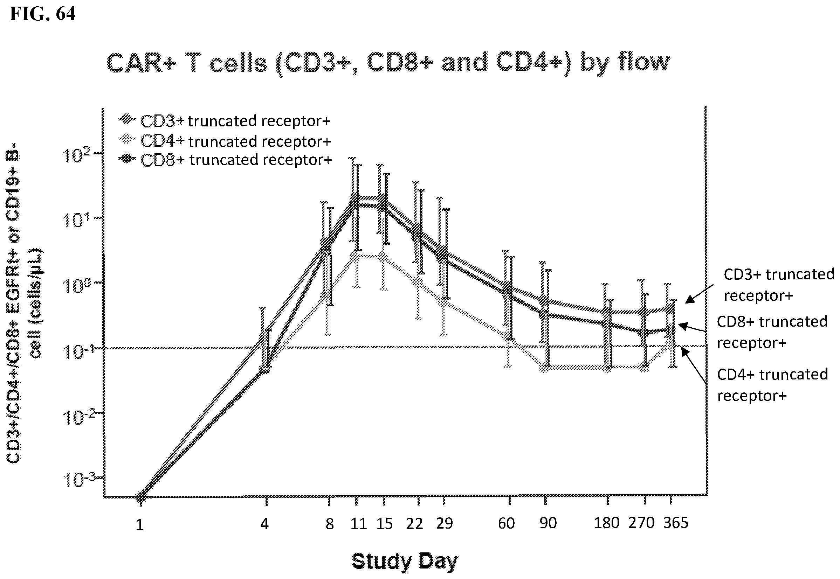

CAR+ T cells showed long-term persistence at 1 year in 59% (n=22/37). CD19+ B cell levels remained low for 2-3 months before increasing, and the kinetics of CAR+ CD4+ and CD8+ cell expansion was similar. Results of CAR+ T cells analyzed by qPCR and flow cytometry vs. CD19+ B cells analyzed for 261 subjects are shown in **FIG. 63** **and Table E33P.** Results for CAR+ T cells (CD3+, CD8+ and CD4+) by flow cytometry are shown in **FIG. 64****.**

Responders (CR or PR; n=175) had higher median Cₘₐₓ (4.06-fold) and AUC_{0-28d} (2.59-fold) than nonresponders (n=50). Higher median Cₘₐₓ was also associated with higher baseline tumor burden (2.45-fold), any-grade CRS (2.31-fold), any-grade NE (3.46-fold), and grade ≥3 NE (5.06-fold) **(Table E33Q).** CAR+ T-cells showed long-term persistence at 1 year in 59% (n=22/37) of patients; 73% (n=36/49) had persistent B-cell aplasia at 1 year. Anti-CAR+T cell antibodies were detected in 11% of patients (n=28/261) before first CAR+T cell compositions infusion and in 11% (n=27/257) at any time after infusion (most were treatment-induced). The relationship between anti-CAR+T cell antibody status and efficacy, safety, or cellular kinetics was inconclusive because of the small number of patients with positive antibody tests.

| **Table E33O. Cellular Kinetic Parameters by Dose Level** | | | | |
|---|---|---|---|---|
| | Evaluable LBCL Patients^{a} | | | |
| Parameter | DL1 | DL2 | DL3 | DL1 + DL2 + DL3 |
| | n=40 | n=166 | n=32 | n=238 |
| Cₘₐₓ (copies/µg) | | | | |
| Median | 20958.2 | 25098.5 | 23548.8 | 23963.7 |
| Q1, Q3 | 5634.7, 71868.6 | 9806.3, 79118.5 | 6374.9, 71267.3 | 8159.3, 78748.2 |

| Tₘₐₓ (day) | | | | |
|---|---|---|---|---|
| Median | 14.0 | 11.0 | 10.0 | 12.0 |
| Q1, Q3 | 12.0, 19.5 | 10.0, 14.0 | 7.5, 14.0 | 10.0, 15.0 |

| AUC_{0-28d} (day*copies/µg) | | | | |
|---|---|---|---|---|
| Median | 186994.0 | 229062.6 | 185393.9 | 214283.0 |
| Q1, Q3 | 41717.3, 510264.9 | 96750.8, 689751.7 | 54491.0, 849879.3 | 77281.7, 689751.7 |
| AUC_{0-28d}, area under the curve during the first 28 days following infusion (ie, from Day 1 to Day 29); Cₘₐₓ, maximum expansion; DL1, dose level 1 (single dose); DL2, dose level 2 (single dose); DL3, dose level 3 (single dose); LBCL, large B-cell lymphoma; Q1, Q3, first and third quartiles; qPCR, quantitative polymerase chain reaction; Tₘₐₓ, time to maximum expansion. | | | | |
| ^{a}Number of patients with cellular kinetic parameters. Noncompartmental cellular kinetic parameters were calculated for patients who had cellular kinetic measurement on Day 29 or later. | | | | |

| **Table E33P. Pharmacokinetic Analysis of CAR+ T cells.** | |
|---|---|
| | **[1333] PK Analysis Set (qPCR) (n=261)** |
| Tₘₐₓ, median (IQR) | **[1334]** 12(10-15) |
| Cₘₐₓ, median | **[1335]** 23,963.7 copies/µg |
| AUC_{0-28d}, median | **[1336]** 214,283.0 day*copies/µg |

| **Table E33Q. Median Cₘₐₓ and AUC_{0-28d} Fold-Change in Subject Subgroups** | | | |
|---|---|---|---|
| | **n** | **Fold-Change in median Cmax** | **Fold-Change in median AUC_{0-28d}** |
| SPD at baseline | | | |
| ≥50 cm² vs <50 cm² | 59 | 2.45 | 2.86 |
| | 167 | | |
| Any grade CRS vs no CRS^{a} | 95 | 2.31 | 2.41 |
| | 143 | | |
| Any grade NE vs no NE | 70 | 3.46 | 3.88 |
| | 168 | | |
| Grade ≥3 NE vs grade 0-2 NE | 19 | 5.06 | 5.99 |
| | 219 | | |
| Responders (CR+PR) vs Nonresponders | 175 | 4.06 | 2.59 |
| | 50 | | |
| AUC, area under the concentration curve; Cₘₐₓ, maximum concentration; CR, complete response; CRS, cytokine release syndrome; NE, neurological events; PR, partial response; SPD, sum of product of perpendicular diameters. | | | |
| ^{a}The analysis of any grade CRS vs grade ≥3 CRS is not included because there were too few patients with grade ≥3 CRS. | | | |

### G. Conclusion

The results reported in this example of CD19-directed CAR T-cell therapy in subjects with relapsed/refractory aggressive LBCL show that the treatment with anti-CD19 CAR+ T cells resulted in durable response, with increased CAR T-cell expansion in vivo, and CAR T-cells persisted long-term after anti-CD19 CAR+ T cell infusion. These results were observed in a population of heavily pretreated subjects with aggressive, high-risk disease, most of whom were chemotherapy-refractory and required immediate treatment for disease control with bridging therapy. The study also included subject subgroups with characteristics of poor prognosis, such as secondary CNS involvement, DLBCL transformed from indolent lymphomas other than FL, FL3B and moderate comorbidities. The subject population treated in the study was generally older, including those aged over 75 years. These results were observed with low incidence of severe CRS and NEs. Fewer than one-half of all subjects in the study described herein developed CRS or NEs, and the incidence of grade 3 or higher CRS and NEs was low (2% and 10%, respectively), and no fatal CRS or NE occurred. The low overall incidence and severity of CRS and NEs, along with their late onset (median, 5 and 9 days, respectively), support outpatient administration/monitoring in select subjects. Safety and efficacy outcomes in the 25 subjects who received anti-CD19 CAR+ T cells in the outpatient setting in various studies described herein were similar to the entire treated population of subjects. The results demonstrated that administration of anti-CD19 CAR-expressing cells demonstrated durable response with a favorable safety profile in subjects with R/R large B cell NHL. Low incidence and late onset of CRS and NE permitted outpatient administration. These results show that clinically meaningful responses were observed across histologic subgroups and in subjects with poor prognosis, including subjects who were refractory, elderly, comorbid, and/or had high tumor burden. Treatment with CAR+ T cell compositions as described herein resulted in a rapid, high rate of durable CR and low incidence of severe CRS and NEs among patients with high-risk, aggressive relapsed/refractory LBCL in this study. Clinically meaningful activity was observed across patient subgroups with unmet medical need, including uncommon DLBCL histologic subtypes and those with poor prognostic characteristics. The low incidence of severe CRS and NEs and later time to onset allowed for outpatient administration/monitoring. The results support the utility of the described compositions and methods for treating a larger groups of subjects and potential sites of care.

### Example 14 Administration of Anti-CD19 CAR-Expressing Cells for Treatment of Second-line Transplant-Noneligible (TNE) Subjects with Relapsed and Refractory Non-Hodgkin Lymphoma (NHL)

Therapeutic CAR⁺ T cell compositions containing autologous CD4+ and CD8+ T cells expressing a chimeric antigen-receptor (CAR) specific for CD19 employed in the studies described in Examples 1, 3 and 6 above, were administered as a second-line therapy to subjects with transplant non-eligible (TNE) relapsed and refractory (R/R) non-Hodgkin lymphoma (NHL), such as relapsed and refractory (R/R) large B cell lymphoma, that had failed a first-line therapy.

Eligible subjects for treatment included subjects with relapsed/refractory large B cell non-Hodgkin lymphoma (NHL) (diffuse large B-cell lymphoma (DLBCL), not otherwise specified [de novo or transformed indolent NHL, high grade lymphoma with MYC and BCL2 and/or BCL6 [double/triple-hit lymphoma], or follicular lymphoma grade 3B) and had received only 1 prior line of immunochemotherapy containing an anthracycline and a CD20-targeted agent (e.g., R-CHOP; rituximab, cyclophosphamide, doxorubicin hydrochloride (hydroxydaunomycin), vincristine sulfate (oncovin) and prednisone). Subjects included those that were deemed ineligible for high-dose chemotherapy followed by HSCT (e.g., based on age, performance status and/or comorbidities) if they met at least one of the following TNE criteria while still fulfilling criteria for CAR T cell therapy: age ≥70 years, Eastern Cooperative Oncology Group (ECOG) performance status of 2, and/or impaired pulmonary (DLCO ≤60%, but SaO₂≥92% on room air and CTCAE ≤1 dyspnea), cardiac (LVEF 240% and <50%), renal (creatinine clearance >30 and <60 mL/min), or hepatic function (AST/ALT >2 and ≤5 x ULN) (see **Table E34A).** Eligible subjects for treatment also included those with positron emission tomography (PET)-positive disease, histological confirmation of diagnosis at last relapse (e.g., having enough tumor material must be available for central confirmation of diagnosis, otherwise a new tumor biopsy is mandated), an ECOG PS of 0, or 1, or 2, and/or adequate vascular access for leukapheresis procedure (either peripheral line or surgically-placed line). Subjects who have previously received administration of T cells expressing the same anti-CD19 CAR as described above and achieved a complete response and had relapsed were eligible for retreatment.

Primary end points included objective response rate (ORR), and secondary end points included adverse events (AE), complete response rate (CRR), duration of response (DR), pharmacokinetic profile, estimated progression free survival, event free survival, overall survival, laboratory anomalies and health related quality of life. Efficacy was evaluated according to the 2014 Lugano criteria (Cheson BD, et al. J Clin Oncol. 2014; 32:3059-3067) and CRS was graded using the Lee criteria (Lee DW, et al. Blood. 2014;124:188-195). NE were adverse events (AE) that were considered related to CAR⁺ T cell compositions. AEs, except for CRS, were graded using the National Cancer Institute Commin Terminology Criteria for Adverse Events, version 4.03. Subjects were monitored for treatment-emergent AEs (TEAEs) for 90 days after CAR⁺ T cell composition administration. Subjects could be treated as outpatients at the investigator's discretion. Subjects were considered to be monitored as outpatients if the patients left the clinic or were discharged from the hospital or left the clinic after the end of the post-infusion observation period. Subjects were considered to be monitored as inpatients if the subjects were admitted to the hospital during CAR⁺ T cell composition infusion or immediately after the post-infusion observation period. Pharmacokinetic (PK) parameters were determined by flow cytometry.

| **Table E34A. TNE Criteria** | |
|---|---|
| Criteria ^{a} | Details |
| Age | Age ≥70 years |
| ECOG PS | ECOG PS of 2 |
| Impaired pulmonary function | DLCO ≤60% adjusted for hemoglobin concentration (DLCO ≤60% but oxygen saturation ≥92% on room air and NCI CTCAE ≤1 dyspnea) |
| Impaired cardiac function | Left ventricular ejection fraction ≥40% to ≤50%; assessed by echocardiogram or multi-gated acquisition scan performed within 4 weeks of determination of eligibility |
| Impaired renal function | Creatinine clearance (Cockcroft and Gault or direct measurement) >30 and <60 mL/min |
| Impaired hepatic function | Aspartate aminotransferase and alanine aminotransferase >2 and ≤5 xULN |
| DLCO, diffusing capacity of the lungs for carbon monoxide; ECOG PS, Eastern Cooperative Oncology Group performance status; NCI CTCAE, National Cancer Institute Common Terminology Criteria for Adverse Events, v 4.03; ULN, upper limit of normal. | |
| ^{a} TNE criteria were developed based on published literature, treatment guidelines, and expert opinion from clinicians in the US and Europe. | |

### Example 14.A

At the first time point of analysis, a total of 10 subjects had been leukapheresed, of which 9 received CAR⁺ T cell compositions and 1 subject was awaiting treatment. For these subjects, CAR+T cell compositions were successfully manufactured for all subjects. Subjects received a target dose of DL2 (100 x 10⁶ CAR-expressing T cells) between 2 and 7 days after lymphodepletion with fludarabine/cyclophosphamide for 3 days, as described in Example 1. Five (5) subjects were infused and monitored as outpatients.

Median age was 71 (range, 64-79) years, and 5 subjects were male. Based on histological analysis, the subjects had DLBCL NOS (n=7) and transformed follicular lymphoma (tFL, n=2); two subjects had triple-hit (one of whom had tFL histology). Five subjects relapsed from, and 4 subjects had disease refractory to prior therapy. Median SPD and LDH were 26.6 cm² and 201 U/L, respectively. Four subjects had high tumor burden with SPD of ≥50 cm² (n=4) and LDH of ≥500 U/L (n=1). The median Hematopoietic Cell Transplantation Comorbidity Index (HCT-CI) score was 3 (range, 0-3).

Six subjects had one or more treatment-emergent adverse events (TEAEs) of grade ≥3, which were primarily cytopenias. Three subjects had prolonged cytopenias of grade ≥3 at Day 29. Two subjects had infections of any grade, and no subjects had a treatment-emergent infection of grade ≥3 occurred. No subjects had CRS or NEs, and no subjects received tocilizumab, corticosteroids, or vasopressors. There were no cases of macrophage activation syndrome, tumor lysis syndrome, infusion reactions, or grade 5 TEAEs. Among the 5 subjects treated and monitored as outpatients, none were admitted to hospital for adverse events within the first 29 days post CAR-expressing T cell infusion.

All 9 subjects achieved an objective response. Four subjects achieved complete response (CR); and at the time of analysis, continued to have ongoing CR. Five subjects achieved partial response (PR); with 2 subjects continuing to exhibit ongoing PR at the time of analysis. Results were similar in subjects treated in the inpatient setting compared to subjects in the outpatient setting. Median follow-up was 3.5 months. Median (range) time to peak CAR T cell expansion was 10 (7-21) days.

These results are consistent with an observation that an anti-CD19 CAR-expressing T cells can be successfully administered, including in the outpatient setting, as a second-line therapy in subjects with R/R aggressive B-cell NHL who were ineligible for high-dose chemotherapy and HSCT by a prespecified criteria.

### Example 14.B

At a second time point of analysis, a total of 19 subjects had been leukapheresed, of which 13 received CAR⁺ T cell compositions. For these subjects, CAR+T cell compositions were successfully manufactured for all subjects. Six subjects were treated and monitored as outpatients. All subjects that received CAR⁺ T cell compositions were included in the safety analysis, and 12 were included in the efficacy analysis. Of the 13 subjects that had received the CAR+ T cell composition at the time of analysis, 9 subjects had 3 month or more of follow up. No subjects received nonconforming product (e.g., product for which one or both of the CD8 or CD4 cell components did not meet the release specifications for CAR+T cell compositions), and there were no manufacturing failures.

Median age was 72 years. At screening, 8 subjects met 1 TNE criterion, 4 subjects met 2 TNE criteria, and 1 subject met ≥3 TNE criteria. Among the 6 subjects treated and monitored as outpatients, none were hospitalized for AEs within the first 29 days after CAR+T cell composition infusion **(Table E34B).** Eight subjects (61.5%) experienced grade 3 or 4 TEAEs, mostly cytopenias. No subjects died after CAR+T cell composition infusion **(Table E34C).**

| **Table E34B. Demographics and Clinical Characteristics at Baseline.** | |
|---|---|
| | **Subjects (N=13)** |
| Males, n (%) | 7 (54) |
| Age, years, median (range) | 72 (64-80) |

| Age, years, n(%) | |
|---|---|
| <65 | 1 98) |
| ≥65 | 12(92) |

| TNE criteria,^{a} n (%) | |
|---|---|
| Age ≥70 years | 8(61.5) |
| ECOG PS of 2 | 4(310 |
| DLCO ≤60% | 1(8) |
| LVEF <50% | 1(8) |
| Creatinine clearance <60 mL/min | 4(31) |
| AST/ALT >2 x ULN | 0 |
| B-cell NHL subtypes, n (%) | |
| DLBCL NOS | 7(54) |
| Transformed FL | 2(15) |
| High-grade lymphoma with DLBCL histology | 3(23) |
| FL3B | 1(8) |
| LDH ≥500 U/L, n(%) | 3(23) |
| SPD ≥50 cm², n (%) | 5(38) |
| Relapsed, n (%) b | 6(46) |
| Refractory, n (%) | 7(54) |
| HCT-CI score, median (range) | 3(0-9) |

| Treatment setting, n (%) | |
|---|---|
| Inpatient | 7(54) |
| Outpatient | 6(46) |
| ALT, alanine aminotransferase; AST, aspartate aminotransferase; DLBCL, diffuse large B-cell lymphoma; DLCO, diffusing capacity of the lungs for carbon monoxide; ECOG PS, Eastern Cooperative Oncology Group performance status; FL, follicular lymphoma; FL3B, follicular lymphoma grade 3B; HCT-CI, hematopoietic cell transplant-comorbidity index; LDH, lactate dehydrogenase; LVEF, left ventricular ejection fraction; NHL, non-Hodgkin lymphoma; NOS, not otherwise specified; SPD, sum of the product of perpendicular diameters; TNE, transplant noneligible; ULN, upper limit of normal. | |
| All percentages are rounded to whole numbers except those ending in.5. | |
| ^{a}TNE criteria as assessed at screening. bRelapsed vs refractory was defined as best response of complete response vs best response of partial response, stable disease, or progressive disease to last systemic or transplant treatment with curative intent | |

| **Table E34C. Treatment-Emergent Adverse Events** | | | | |
|---|---|---|---|---|
| | | **CAR+T cell treated subjects (N=13)** | | |
| | | **Any grade** | **Grade 3-4** | **Grade 5** |
| Any TEAEs, n (%) | | 12(92) | 8(61.5) | 0 |

| | TEAEs in ≥15% of subjects,^{a} n (%) | | | |
|---|---|---|---|---|
| Anemia | | 5(38) | 2(15) | 0 |
| Hypotension | | 4(31) | 0 | 0 |
| Neutropenia | | 4(31) | 4(31) | 0 |
| Thrombocytopenia | | 4(31) | 3(23) | 0 |
| CRS | | 3(23) | 0 | 0 |
| Dizziness | | 3(23) | 0 | 0 |
| Fatigue | | 3(23) | 0 | 0 |
| Insomnia | | 3(23) | 0 | 0 |
| Leukopenia | | 3(23) | 2(15) | 0 |
| Lymphopenia | | 3(23) | 3(23) | 0 |
| Nasal congestion | | 3(23) | 0 | 0 |
| Anal incontinence | | 2(15) | 0 | 0 |
| Cough | | 2(15) | 0 | 0 |
| Fall | | 2(15) | 0 | 0 |
| Hypertension | | 2(15) | 1(8) | 0 |
| Hypomagnasemia | | 2(15) | 0 | 0 |
| Neutrophil count decreased | | 2(15) | 2(15) | 0 |
| Pain in extremity | | 2(15) | 0 | 0 |
| Productive cough | | 2(15) | 0 | 0 |
| CRS, cytokine release syndrome; TEAEs, treatment-emergent adverse events. | | | | |
| All percentages are rounded to whole numbers except those ending in.5. | | | | |
| ^{a}TEAEs are listed in descending order of frequency for preferred term based on the any grade column. | | | | |

Among the evaluated subjects at this timepoint, no events of grade 3-4 CRS occurred, no subjects experienced NEs, and there were no cases of macrophage activation syndrome, tumor lysis syndrome, or occurrences of infusion reactions **(Table E34D).**

| **Table E34D. Adverse Events of Special Interest** | |
|---|---|
| **Event, n (%)** | **CAR+T cell treated subjects (N=13)** |
| CRS | |
| Grade 1-2 | 3(23) |
| Grade 3-5 | 0 |

| NEs^{a} | |
|---|---|
| Grade 1-2 | 0 |
| Grade 3-5 | 0 |
| Tocilizumab and corticosteroid for CRS^{b} | 2(15) |
| Prolonged grade ≥3 cytopenia ^{c} | 4(31) |
| Infections, grade ≥3 | 0 |
| CRS, cytokine release syndrome; NEs, neurological events. | |
| ^{a}NEs were treatment-related events defined by the investigator. ^{b}Tocilizumab and a corticosteroid were given for 2 events of grade 2 CRS with hypotension. ^{c}Prolonged cytopenias included neutropenia, thrombocytopenia, or anemia not resolved at study Day 29 based on laboratory assessments. | |

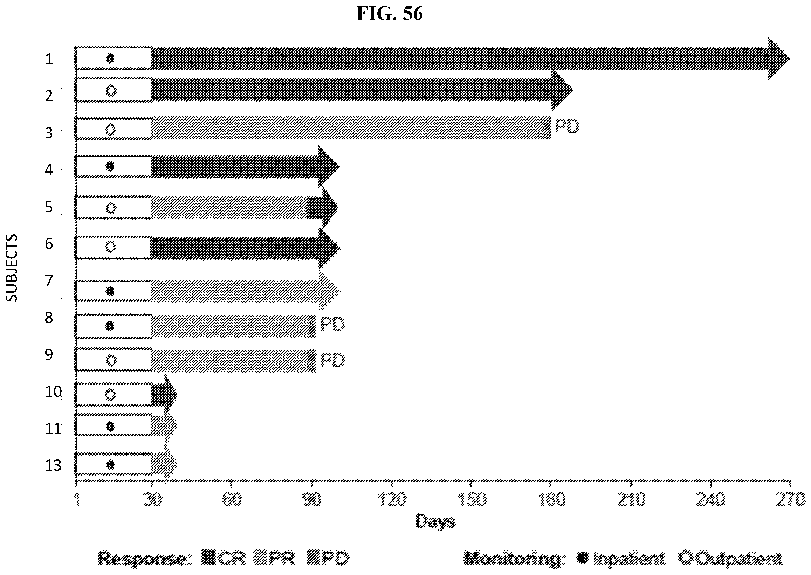

All subjects in this study at this timepoint that were evaluated for efficacy (12 subjects) achieved an objective response (100% [12/12]) and six subjects (50%) achieved a CR **(Table E34E).** Individual outcomes for each subject in this study at this timepoint are summarized in **Table E34F.** All 12 subjects achieved an early objective response by day 30, and seven of the 12 subjects (58%) maintained durable responses at 3 months after CAR+T cell infusion. One subject remained in CR for 9 months (270 days) and 1 subject remained in CR for 6 months (180 days) **(****FIG. 56****).** The time to maximum concentration (Tmax) of the CAR T cells in this study (11 [interquartile range (IQR): 11-15] days) was similar to that observed with the Tmax of the CAR T cell composition observed in a different study (e.g., described in Examples 1, 3, 6 and 13 above) of 12 [IQR: 10 15] days.

| **Table E34E. Best Overall Response ^{a}** | |
|---|---|
| **Response, n (%)** | **CAR+T cell treated subjects (N=12)^{b}** |
| ORR | 12 (100) |
| CR | 6 (50) |
| PR | 6(50) |
| SD | 0 |
| PD | 0 |
| CR, complete response; ORR, objective response rate; PD, progressive disease; PR, partial response; SD, stable disease. | |
| ^{a}Responses were calculated based on patients with an end-of visit window at Day 29 that occurred on or before the time point of analysis. ^{b}At the time point of analysis, one patient did not yet have a Day 29 efficacy assessment. | |

| **Table E34F. Individual Outcomes** | | | | | | | | |
|---|---|---|---|---|---|---|---|---|
| **Subject numbe r** | **Treatment setting** | **TNE criteria Met (value)** | **HCT -CI score** | **Tumor burden** | | **AEs Related to CAR+T cell compositions (Grade 3-4)^{a}** | **CRS or NE (Grad e)** | **Best Respon se^{c}** |
| | | | | **LDH, IU/L^{a}** | **SPD, cm^{2,b}** | | | |
| 1 | Inpatient | CrCl (55 mL/min) | 1 | 200 | 10 | None | 0 | CR |
| 2 | Outpatient | Age (71 years) | 0 | 195 | 22 | None | 0 | CR |
| 3 | Outpatient | CrCl (59 ml/min) | 3 | 355 | 27 | Neutropenia | 0 | PR |
| 4 | Inpatient | Age (71 years) | 0 | 568 | 91 | Neutropenia/ Thrombocytopenia | 0 | CR |
| 5 | Outpatient | Age (72 years) | 3 | 160 | 8 | Leukopenia/ Neutropenia/ Lymphopenia | 0 | CR |
| 6 | Outpatient | Age (72 years) DLCO (57%) | 3 | 201 | 5 | Thrombocytopenia Lymphocyte count decreased Neutrophil count decreased | 0 | CR |
| 7 | Inpatient | Age (79 years) ECOG PS (2) | 3 | 260 | 79 | None | 0 | PR |
| 8 | Inpatient | CrCl (58ml/min) LVEF (45%) | 3 | 341 | 191 | Anemia | 0 | PR |
| 9 | Outpatient | Age (77 years) | 0 | 201 | 89 | None | 0 | PR |
| 10 | Outpatient | Age (80 vears) | 6 | 332 | 21 | None | 0 | CR |
| 11 | Inpatient | Age (75 vears) | 1 | 1567 | 118 | None | 2 | PR |
| 12 | Inpatient | Age (72 years) ECOG PS (2) CrCl (53 mL/min) | 9 | 562 | 18 | None | 2^{d} | N/A ^{e} |
| 13 | Inpatient | ECOG PS (2) | N/A | 218 | 11 | None | 1 | PR |
| AE, adverse event; CR, complete response; CrCl,creatinine clearance; CRS, cytokine release syndrome; DLCO, diffusing capacity of the lungs for carbon monoxide; ECOG PS, Eastern Cooperative Oncology Group performance status; HCT-CI,hematopoietic cell transplant-comorbidity index; LDH, lactate dehydrogenase; LVEF left ventricular ejection fraction; N/A, not applicable; NE, neurological events; PD, progressive disease; PR, partial response; SPD, sum of the product of perpendicular diameters; TNE, transplant noneligible. | | | | | | | | |
| ^{a}High tumor burden based on **LDH** >500 IU/L. ^{b}High tumor burden based on SPD >50 cm2. ^{c}Data from electronic data capture. ^{d}Duration of follow-up for AE's was <28 days. ^{e}At the time point of analysis, this patient did not yet have a Day 29 efficacy assessment. | | | | | | | | |

The results showed that the most common grade 3 or 4 TEAEs were cytopenias, no subjects exhibited grade 3-4 CRS, no subjects exhibited neurological events, and no subjects had grade 5 TEAEs. The results also showed that administration of the anti-CD19 CAR-expressing T cell compositions resulted in durable responses, including CRs. In this study, all subjects (100%) achieved an early objective response by Day 30, six patients (50%) achieved a CR, seven patients (58%) maintained durable responses at 3 months after CAR-expressing T cell infusion, two patients have CRs ongoing for 6 and 9 months, respectively. These results are consistent with an observation that an anti-CD19 CAR-expressing T cells is successfully tolerated as a second-line therapy in subjects with R/R aggressive B-cell non-Hodgkin lymphoma (NHL) who were ineligible for high-dose chemotherapy and HSCT.

### Example 15 Administration of Anti-CD19 CAR-Expressing Cells for Treatment of Relapsed and Refractory (R/R) B-cell Non-Hodgkin Lymphoma (NHL) in the Outpatient Setting

Therapeutic CAR⁺ T cell compositions containing autologous CD4+ and CD8+ T cells expressing a chimeric antigen-receptor (CAR) specific for CD19 employed in the studies described in Examples 1, 3 and 6 above, were administered in an outpatient setting, as third-line or later therapy.

Eligible subjects for treatment included adult subjects with relapsed/refractory large B cell non-Hodgkin lymphoma (NHL) (diffuse large B cell lymphoma (DLBCL) not otherwise specified (NOS); including biopsy-confirmed transformed DLBCL from indolent lymphoma, high grade lymphoma with MYC and BCL2 and/or BCL6 [double/triple-hit lymphoma], primary mediastinal B-cell lymphoma (PMBCL), and follicular lymphoma Grade 3B). Subjects had been previously treated with an anthracycline and rituximab (or other CD20-targeted agent) and have had relapsed or refractory disease after at least 2 systemic lines of therapy for DLBCL or after autologous hematopoietic stem cell transplant (HSCT). Eligible subjects for treatment also included those with positron emission tomography (PET)-positive disease by Lugano classification, an Eastern Cooperative Oncology Group (ECOG) performance status of 0 or 1, subjects with adequate bone marrow, renal, hepatic, pulmonary and cardiac organ function, adequate vascular access for leukapheresis procedure and/or subjects who have received previous CD19-targeted therapy having a CD19-positive lymphoma confirmed on a biopsy since completing the prior CD19-targeted therapy.

Subjects underwent leukapheresis to isolate peripheral blood mononuclear cells (PBMCs) for the production of the CD19-targeted CAR. During production of cells expressing the CD19-targeted CAR, subjects were permitted to receive low-dose chemotherapy for disease control. Following successful manufacturing of an anti-CD19 CAR-expressing T cell composition, subjects received lymphodepleting chemotherapy, followed by one dose of 100 x 10⁶ CAR-expressing T cells administered by intravenous (IV) injection.

### Example 16 Assessment of Safety and Response Outcomes for Outpatient Subjects with Relapsed and Refractory Non-Hodgkin's Lymphoma (NHL) after Administration of Anti-CD19 CAR-Expressing Cells

Response and safety outcomes were assessed across groups of subjects with relapsed and refractory (R/R) Non-Hodgkin's Lymphoma (NHL), such as large B cell lymphoma,treated in an outpatient setting from clinical studies as described in Example 1, 3, 6, 13, 14 and 15 above.

As described in Examples 1, 3, 6 and 13 above, a defined composition of CD4+ and CD8+ cells expressing an anti-CD19 CAR achieved high response rates as a third line or later therapy in subjects with R/R large B cell NHL. With fewer than half of the subjects developing cytokine release syndrome (CRS) and/or neurological events (NE) (e.g., in 47% of subjects), low incidence of grade 3 or higher events (2% for CRS and 10% for NE, respectively) and in view of the delayed onset (median 5 and 10 days, respectively) outpatient treatment was permitted, with hospitalization at the first sign of fever or neurological symptoms.

### Example 16.A

A total of 37 subjects, across different studies, who were administered the CAR+ T cells in an outpatient setting were analyzed at this time point of analysis. In the studies, outpatient treatment was carried out at either a non-university setting or both a university and non-university medical center, among three groups of treated subjects. The groups are summarized in **Table E35.**

| **Table E35. Study Designs** | | | |
|---|---|---|---|
| **Study Type of Site (s)** | **Dose Level(s)** | **Eligibility Criteria** | **Objectives** |
| Group A University and non-university centers | 50 x 10⁶ CAR+ T cells | Large B-cell NHL after ≥2 lines of prior therapy (DLBCL cohort) | Safety Efficacy |
| | 100 x 10⁶ CAR+ T cells | | |
| | 150 x 10⁶ CAR+ T cells | | |
| Group B Non-university centers | 100 x 10⁶ CAR⁺ T cells | Large B-cell NHL after ≥2 lines of prior therapy | Safety Efficacy |
| Group C University and non-university centers | 100 x 10⁶ CAR⁺ T cells | Large B-cell NHL after only 1 line of prior therapy | Efficacy Safety |
| | | Ineligible for both high dose chemotherapy and HSCT | |
| | | Meet ≥1 TNE criteria | |
| CY, cyclophosphamide; DLBCL, diffuse large B-cell lymphoma; FLU, fludarabine; HSCT, hematopoietic stem cell transplantation; | | | |
| NHL, non-Hodgkin lymphoma; PET, positron emission tomography; TNE, transplant non-eligible. | | | |

One group (Group A) included a subset of 25 subjects with R/R large B cell NHL treated in an outpatient setting generally as described in Examples 1, 3, 6 and 13. Subjects had received CAR⁺ T cell compositions at DL1 of 50 x 10⁶, DL2 of 100 x 10⁶ or DL3 of 150 x 10⁶ CAR+ cells, as a third-line therapy or later (subjects had 2 or more prior lines of therapy and an ECOG PS of 1 or lower). Subjects in the mantle cell lymphoma (MCL) cohort were treated with ≥1 prior line of therapy.

Another group (Group B) included 7 subjects from a study in which CAR⁺ T cell compositions were administered as a third-line or later therapy, generally as described in Example 15. Subjects had been treated with anthracycline and rituximab (or other CD20-targeted agent) and had relapsed or refractory disease after at least 2 systemic lines of therapy for DLBCL or after autologous HSCT.

Another group (Group C) included 5 subjects from a study in which CAR⁺ T cell compositions were administered as a second line therapy for transplant noneligible (TNE) subjects. Subjects were deemed ineligible for high-dose chemotherapy followed by HSCT by meeting at least one of the TNE criteria described in Example 14, while still fulfilling criteria for CAR T cell therapy (Age ≥70 years, ECOG PS of 2, impaired pulmonary function (diffusing capacity of the lungs for carbon monoxide (DLCO) ≤60% adjusted for hemoglobin concentration (DLCO ≤60% but oxygen saturation ≥92% on room air and National Cancer Institute Common Terminology Criteria for Adverse Events [NCI CTCAE] ≤1 dyspnea),impaired cardiac function (left ventricular ejection fraction ≥40% to <50%), impaired renal function (creatinine clearance >30 and <60 mL/min) and impaired hepatic function (aspartate aminotransferase and alanine aminotransferase >2 and ≤5 × upper limit of normal). Subjects had received only 1 prior line of immunochemotherapy containing an anthracycline and a CD20-targeted agent (e.g., R-CHOP).

In the studies, eligible subjects had R/R large B-cell NHL, adequate organ function, and prior systemic chemoimmunotherapy (≥2 prior lines of therapy and ECOG PS ≤1 or 1 prior line of therapy and deemed TNE for autologous hematopoietic stem cell transplantation based on ECOG PS, organ function, and/or age). After lymphodepletion with fludarabine/cyclophosphamide as described in Examples above, CAR⁺ T cell compositions were administered at 1 of 3 dose levels (DL1 of 50 x 10⁶, DL2 of 100 x 10⁶ or DL3 of 150 x 10⁶ CAR+ cells) for the 25 in Group A, and at DL2 (100 x 10⁶ CAR+ T cells) in Groups B and C described above. For outpatient treatment, subjects had a caregiver for 30 days post CAR-expressing T cell infusion, received safety-monitoring education (recognizing critical adverse events; e.g., fever), and were within 1 hour travel to the site of care. All sites for outpatient treatment had a multidisciplinary CAR T cell therapy team and standard operating procedures for outpatient administration and adverse event monitoring. Cytokine release syndrome (CRS; Lee et al. (2014) criteria) and neurological events (NE; defined as related to CAR-expressing T cell administration; CTCAE criteria) were managed in the hospital.

At the time of analysis, 37 subjects across studies had received anti-CD19 CAR+ T cells on study Day 1 and were monitored as outpatients, including subjects 65 years old or older and those with high tumor burden. Patient characteristics, safety and response outcomes are shown in **Table E35.1** below. The most frequent grade ≥3 treatment-emergent AEs were cytopenias (neutropenia 43%, anemia 30%, thrombocytopenia 14%). Sixteen subjects had any grade CRS and 12 had any grade NE (19 subjects had CRS and/or NE). Severe CRS and/or NE occurred in only 2 subjects and were reversible (see **Table E35.1).** Three subjects received tocilizumab and corticosteroids and 4 subjects received corticosteroids alone for CRS and/or NE. No subjects received tocilizumab alone. Twenty-two of the 37 subjects (59%) required hospitalization at any time; all subjects that required hospitalization were from Group A or Group B. Three of those subjects (8%) were admitted on study Day 3 or earlier, all for CRS; 1 subject required ICU-level care (length of stay, 3 days). Median (range) time to hospitalization post treatment was 5 (2-22) days and median (range) length of stay was 6 (2-23) days. Fifteen (41%) of the 37 subjects, including all 5 subjects from Group C, were not admitted to hospital in the first 29 days post anti-CD19 CAR-expressing cell infusion.

Across all 3 groups, most subjects achieved an objective response, including CR (see **Table E35.1).** Median time to peak CAR+ T cell expansion in each study was 10 days (range: 3-21 in Group A; 7-10 in Group B; 7-10 in Group C).

| **Table E35.1 Subject Characteristics, Safety and Response Outcomes for Subjects Treated in an Outpatient Setting** | | | | |
|---|---|---|---|---|
| | **All Outpatient treated subjects (n=37)** | **Group C 2^{nd}-line transplant noneligible (TNE), Example 14 (N=5)** | **Group B 3^{rd} line or later therapy, Example 15 (N=7)** | **Group A Examples 1, 3, 6, 7 and 13 (N=25)** |
| Subject characteristics | | | | |
| Age, median (range) vears | 62 (24-82) | 72 (69-77) | 64 (49-78) | 60 (24-82) |
| ≥65, n (%) | 15 (41) | 5 (100) | 3 (43) | 7 (28) |
| Male, n (%) | 21 (57) | 3 (60) | 3 (43) | 15 (60) |
| Histology, n (%) | | | | |
| DLBCL NOS | 24 (65) | 4 (80) | 6 (86) | 14 956) |
| HGBCL | 3 (8) | 1 (20) | 0 | 2 (8) |
| Transformed DLBCL | 6 (16) | 0 | 1 (14) | 5 920) |
| PMBCL | 4(11) | 0 | 0 | 4 (16) |
| Prior lines of therapy, median (range) | 2 (1-5) | 1 (1-1) | 2 (2-4) | 2 (1-5) |
| Relapsed, ^{a} n (%) | 13 (35) | 4 (80) | 2 (29) | 7 (28) |
| Refractory, ^{a} n (%) | 24 (65) | 1 (20) | 5 (71) | 18 (72) |
| LDH ≥500 U/L, n (%) | 2 (5) | 0 | 1 (14) | 1 (4) |
| SPD ≥50 cm², n (%) | 10 (27) | 9 (20) | 3 (43) | 6 (24) |
| Safety | | | | |
| Any TEAEs grade ≥3, n (%) | 27 (73) | 3 (60) | 7 (100) | 17 (68) |
| TEAEs grade 5, n (%) | 0 | 0 | 0 | 0 |

| TEAEs of special interest, n (%) | | | | |
|---|---|---|---|---|
| CRS, any grade | 16 (43) | 0 | 4 (57) | 12 (48) |
| CRS, grade ≥3 | 1 (3) | 0 | 0 | 1 (4) |
| NE, any grade | 12 (32) | 0 | 1 (14) | 11 (44) |
| NE, grade ≥3 | 2 (5) | 0 | 0 | 2 (8) |
| CRS and/or NE, any grade | 19 (51) | 0 | 4 (57) | 15 (60) |
| CRS and/or NE, grade ≥3 | 2 (5) | 0 | 0 | 2 (8) |
| Response | N=34 | N= 5 | N=6 | N=23 |
| ORR, n (%) | 26 (76) | 5 (100) | 4 (67) | 17 (74) |
| CR, n (%) | 14 (41) | 2 (40) | 2 (33) | 10 (43) |
| PR, n (%) | 12 (35) | 3 (60) | 2 (33) | 7 (30) |

### Example 16.B

At a different time point in the same study as describe above in Example 16.A, a total of 44 subjects, across different studies, who were administered the CAR+ T cells in an outpatient setting were analyzed at this time point of analysis. In the studies, outpatient treatment was carried out at either a non-university setting or both a university and non-university medical center, among three groups of treated subjects. Outpatient groups included: Group A with a subset of 25 subjects from a total of 286 in the study (9%), Group B included a subset of 13 subjects from a total of 27 in the study (48%), and Group C included a subset of 6 subjects from a total of 13 in the study (46%). Efficacy was evaluated by positron emission tomography/computed tomography scan according to the 2014 Lugano criteria (Cheson BD et al. J Clin Oncol. 2104;32:3059-3067) based on investigator assessment at 1, 3, 6, 9, 12, 18 and 24 months after CAR+T cell infusion. AEs were graded using NCI CTCAE, version 4.03, except for CRS, which was graded using the criteria by Lee at al. (Lee DW et al. Blood. 2014:188-195). NEs were AEs per investigator definition that were considered related to CAR+T cell compositions per investigator assessment. TEAEs were defined as those that occur within 90 days after CAR+T cell composition administration. Pharmacokinetic parameters were determined by flow cytometry. All subjects with ≥30 days of follow up were included in the analysis.

Outpatient treatment requirements included safety monitoring education to recognize critical AEs (fever, CRS and NE symptoms) for which to seek medical attention, the presence of a caregiver for 30 days after CAR+T cell composition infusion and stay within 1-hour travel time to the site of care for 30 days after CAR+T cell composition infusion. Study site requirements included multidisciplinary CAR T cell therapy teams in the outpatient and inpatient setting. Logistics and communication flow of CAR T cell patient care between outpatient and inpatient facilities documented in the site readiness plan available 24 hours a day 7 days a week, coordination of care between outpatient and inpatient medical teams, standard operating procedure for outpatient AE monitoring, and on call medical coverage available 24 hours a day 7 days a week. Hospitalizations requires management of CRS, NEs and serious AEs.

Subjects were considered "outpatients" if they received CAR+T cell infusion in the outpatient setting or if the patient left the clinic or was discharged from the hospital at the end of the observation period on the day of infusion.

Baseline demographics and subject characteristics are shown in **Table E35A,** and a summary of safety is described in **Table E35B.** Adverse events of special interest are reported in **Table E35C,** and grade TEAEs reported in ≥20% of subjects is describes in **Table E35D.** The option for outpatient treatment for Group A was added during the study, and outpatient treatment was available from the beginning of the study for Groups B and C. Overall, 9% of subjects (25/286) were treated as outpatients in the study described in Examples 1, 3, 6 and 13 (Group A), 48% (13/27) for the study in Example 15 (Group B) and 46% (6/13) for the study in Example 14 (Group C).

| **Table E35A. Baseline demographics and subject characteristics** | | | | |
|---|---|---|---|---|
| | **All outpatients (n=44)** | **Group A (n=25)** | **Group B (n=13)** | **Group C (n=6)** |
| Age, median (range), years | 62 (24-82) | 62 (24-82) | 62 (50-78) | 72 (69-80) |
| ≥65 years, n (%) | 18(41) | 7 (28) | 5 (38) | 6 (100) |
| Male, n (%) | 27 (61) | 15 (60) | 8 (62) | 4 (67) |

| Histology, n (%) | | | | |
|---|---|---|---|---|
| DLBCL NOS | 27 (61) | 14(56) | 9(69) | 4(67) |
| DLBCL transformed from indolent histologies | 9(20) | 5(20) | 4(31) | 0 |
| HGBCL | 3(7) | 2(8) | 0 | 1(17) |
| PMBCL | 4(9) | 4(16) | 0 | 0 |
| FL3B | 1 (2) | 0 | 0 | 1(17) |
| ECOG PS of 0-1 / 2 at screening, n (%) | 44 (100)/0 | 25(100)/0 | 13(100)/0 | 6(100)/0 |
| TNE criteria,a n (%) | NA | NA | NA | |
| Age ≥70 years | | | | 4(67) |
| ECOG PS of 2 | | | | 0 |
| DLCO ≤60% | | | | 1(17) |
| LVEF <50% | | | | 0 |
| CrCl <60 mL/min | | | | 9(17) |
| AST/ALT >2 × ULN | | | | 0 |
| Prior lines of therapy, median (range) | 2 (1-5) | 2 (1-5) | 3(2-5)b | 1(1-1) |
| Received prior HSCT, n (%) | 13(30) | 9 (36) | 4(31) | NA |
| Chemotherapy-refractory,c n (%) | 28 (64) | 16 (64) | 10(77) | 2(33) |
| Chemotherapy-sensitive,c n (%) | 16(36) | 9 (36) | 3(23) | 4(67) |
| LDH ≥500 U/L, n (%) | 6(14) | 1 (4) | 5(38) | 0 |
| SPD ≥50 cm2, N/n (%) | 43/12 (28) | 25/6 (24) | 12/5 (42) | 6/1 (17) |
| CRP ≥20 mg/mL, n (%) | 17 (39) | 10(40) | 7(54) | 0 |
| ^{a}TNE criteria as assessed at screening. | | | | |
| ^{b}Based on 12 patients; data were missing for 1 patient. | | | | |
| ^{c}The status was chemotherapy-refractory if the patient achieved SD or PD to last chemotherapy-containing regimen or relapsed <12 months after auto-HSCT; otherwise, the status was chemotherapy-sensitive. | | | | |
| ALT, alanine aminotransferase; AST, aspartate aminotransferase; CrCl, creatinine clearance; CRP, C-reactive protein; DLBCL, diffuse large B-cell lymphoma; DLCO, diffusing capacity of the lungs for carbon monoxide; ECOG PS, Eastern Cooperative Oncology Group performance status; FL3B, follicular lymphoma Grade 3B; HGBCL, high-grade B-cell lymphoma; HSCT, hematopoietic stem cell transplantation; LDH, lactate dehydrogenase; | | | | |
| LVEF, left ventricular ejection fraction; NA, not applicable; NOS, not otherwise specified; PMBCL, primary mediastinal large B-cell lymphoma; SPD, | | | | |
| sum of the product of perpendicular diameters; TNE, transplant noneligible; ULN, upper limit of normal. | | | | |

| **Table E35B. Summary of Safety** | | | | |
|---|---|---|---|---|
| | **All outpatients (n=44)** | **Group A (n=25)** | **Group B (n=13)** | **Group C (n=6)** |
| Any TEAE, n (%) | 42 (95) | 25 (100) | 12 (92) | 5 (83) |
| TEAEs, grade ≥3 | 31 (70) | 17 (68) | 11 (85) | 3 (50) |
| TEAEs, grade 5 | 0 | 0 | 0 | 0 |
| SAEs | 24 (55) | 18 (72) | 6 (46) | 0 |
| SAE, serious adverse event; TEAE, treatment-emergent adverse event. | | | | |

| **Table E35C. Summary of Adverse Events of Special Interest** | | | | |
|---|---|---|---|---|
| | **All outpatients (n=44)** | **Group A (n=25)** | **Group B (n=13)** | **Group C (n=6)** |
| CRS and/or NEs, n (%) | | | | |
| Any grade | 25 (57) | 15 (60) | 7 (35) | 0 |
| Grade ≥3 | 3 (7) | 2 (8) | 0 | 0 |

| CRS | | | | |
|---|---|---|---|---|
| Any grade, n (%) | 17 (39) | 12 (48) | 5 (38) | 0 |
| Grade ≥3, n (%) | 1 (2) | 1 (4) | 0 | 0 |
| Time to onset, median (range), days | 5 (2-9) | 5 (2-8) | 5 (2-9) | NA |
| Time to resolution, median (range), days | 6 (1-16) | 6 (4-16) | 4 (1-9) | NA |

| NEs | | | | |
|---|---|---|---|---|
| Any grade, n (%) | 13 (30) | 11 (44) | 2 (15) | 0 |
| Grade ≥3, n (%) | 2 (5) | 2 (8) | 0 | 0 |
| Time to onset, median (range), days | 8 (3-22) | 8 (3-22) | 8.5 (8-9) | NA |
| Time to resolution, median (range), days | 16 (1-52) | 18 (2-52) | 2 (1-3) | NA |
| Tocilizumab and/or corticosteroid for CRS, n (%) | 5 (11) | 5 (20) | 2 (15) | 0 |
| Tocilizumab only | 0 | 0 | 0 | 0 |
| Tocilizumab and corticosteroid | 3 (7) | 1 (4) | 2 (15) | 0 |
| Corticosteroid only | 2 (5) | 4 (16) | 0 | 0 |
| Corticosteroids for NEs, n (%) | 4 (9) | 4 (16) | 0 | 0 |
| **Infection,** grade ≥3, n (%) | 7 (16) | 6 (24) | 1 (8) | 0 |
| Prolonged grade ≥3 cytopenia,a n (%) | 9 (20) | 3 (12) | 5 (38) | 1 (17) |
| aProlonged cytopenias included neutropenia, thrombocytopenia, or anemia not resolved at study Day 29 based on laboratory assessments. | | | | |
| CRS, cytokine release syndrome; NA, not applicable; NE, neurological event; TEAE, treatment-emergent adverse event. | | | | |

| **Table E35D. Any grade TEAEs Reported in ≥20% of all subjects** | | | | |
|---|---|---|---|---|
| | **All outpatients (n=44)** | **Group A (n=25)** | **Group B (n=13)** | **Group C (n=6)** |
| Fatigue | 25 (57) | 17 (68) | 6 (46) | 2 (33) |
| Neutropenia | 20 (45) | 11 (44) | 7 (54) | 2 (33) |
| Decreased appetite | 19 (43) | 14 (56) | 5 (38) | 0 |
| Cytokine release syndrome | 17 (39) | 12(48) | 5 (38) | 0 |
| Anemia | 16 (36) | 10 (40) | 5 (38) | 1 (17) |
| Constipation | 15 (34) | 9 (36) | 6 (46) | 0 |
| Nausea | 15 (34) | 12 (48) | 3 (23) | 0 |
| Headache | 14 (32) | 10 (40) | 4 (31) | 0 |
| Cough | 13 (30) | 10 (40) | 2 (15) | 1 (17) |
| Dizziness | 13 (30) | 10 (40) | 1 (8) | 2 (33) |
| Hypotension | 13 (30) | 11 (44) | 2 (15) | 0 |
| Thrombocytopenia | 11 (25) | 3 (12) | 6 (46) | 2 (33) |
| Vomiting | 10 (23) | 9 (36) | 1 (8) | 0 |
| Back pain | 9 (20) | 6 (24) | 2 (15) | 1 (17) |
| Diarrhea | 9 (20) | 7 (28) | 2 (15) | 0 |
| Hypomagnesemia | 9 (20) | 9 (36) | 0 | 0 |
| Tremor | 9 (20) | 6 (24) | 3 (23) | 0 |
| TEAE, treatment-emergent adverse event. | | | | |

A summary of hospitalizations for outpatients **(Table E35E)** and for inpatients **(Table E35F)** are described. The median hospitalization days per subject for inpatients across all participants in Group A was 15.0 days (range, 4-98; n=244) and for outpatients accords all studies was 6.0 days (range, 2-23; n=25), with a 60% reduction in inpatient hospitalization stays. For outpatients in Group A, median time to hospitalization was study day 5 (range 3-22), with 6 (24%) of subjects hospitalized on study day 4 or earlier and 1 (4%) required ICU admission. Among all 269 subjects treated in Group A, 19 (7%) required ICU admission for toxicity management, where 12 (4%) was for management of CRS and/or NEs and 7 (3%) was for management of other AEs. Efficacy **(Table E35G)** and pharmacokinetics **(Table E35H)** was assessed across all subgroups in the outpatient setting. Patients in the MCL cohort from the study described in Example 1, 3, 6 and 13 (also described in Example 2) have also been treated in the outpatient setting.

| **Table E35E. Summary of hospitalizations for outpatients** | | | | |
|---|---|---|---|---|
| | **All outpatients (n=44)** | **Group A (n=25)** | **Group B (n=13)** | **Group C (n=6)** |
| Hospitalization required at any time after CAR T cell infusion, n (%) | 24 (55) | 18 (72) | 6 (46) | 0 |
| Time to hospitalization after CAR T cell infusion, median (range), days | 5 (2-22) | 5 (3-22) | 4.5 (2-9) | NA |
| Hospitalization on study Day 4 or earlier, n (%) | 9 (20) | 6 (24) | 3 (23) | 0 |
| Length of hospital stay, median (range), days | 6.5 (1-23) | 6 (2-23) | 9 (4-16) | NA |
| ICU-level care required, n (%) | 2 (5) | 1 (4) | 1 (8) | 0 |
| Length of ICU stay, median (range), days | 4 (3-5) | 3 (3-3) | 5 (5-5) | NA |
| Reasons for initial hospitalization, n (%) | | | | NA |
| CRS and/or NE | 14 (32) | 10 (40) | 4 (31) | |
| CRS | 13 (30) | 9 (36) | 4 (31) | |
| NE | 1 (2) | 1 (4) | 0 | |
| Other | 10 (23) | 8 (32) | 2 (15) | |
| CRS, cytokine release syndrome; ICU, intensive care unit; NA, not applicable; NE, neurological event. | | | | |

| **Table E35F. Summary of hospitalizations for inpatients** | | | |
|---|---|---|---|
| | **Group A (n=25)** | **Group B (n=13)** | **Group C (n=6)^{a}** |
| Length of initial hospital stay during CAR T cell administration, days | | | |
| Median | 15 | 9 | 7 |
| Q1, Q3 | 11, 20.5 | 5, 11.5 | 4, 10 |
| Range | 4-98 | 2-21 | 3-21 |

| Length of initial hospital stay from time of CAR T cell administration, days | | | |
|---|---|---|---|
| Median | 11 | 8 | 6 |
| Q1, Q3 | 8, 15 | 5, 11.5 | 4, 9 |
| Range | 3-88 | 2-21 | 3-15 |

| ICU admissions during initial hospitalization for CAR T cell administration | | | |
|---|---|---|---|
| Number (%) | 18 (7) | 0 | 0 |
| Median days | 7.5 | NA | NA |
| Ql, Q3 days | 3, 18 | NA | NA |
| Range days | 1-56 | NA | NA |

| Reasons for initial hospitalization, n (%) Prophylaxis for CAR T cell | | | |
|---|---|---|---|
| administration | 225 (92) | 6 (86) | 5 (83) |
| Adverse event | 14 (6) | 1 (14) | 0 |
| Other | 5 (2) | 0 | 1 (17) |
| ^{a}A total of 7 patients were treated as inpatients; hospitalization data were not available for 1 patient at time point of analysis | | | |
| ICU, intensive care unit; NA, not applicable; Q, quartile. | | | |

| **Table E35G. Summary of efficacy** | | | | |
|---|---|---|---|---|
| | **All outpatients (n=44)** | **Group A (n=25)** | **Group B (n=13)** | **Group C (n=6)** |
| Response, n (%) | | | | |
| ORR | 35 (80) | 20 (80) | 9 (69) | 6 (100) |
| CR | 24 (55) | 14 (56) | 6 (46) | 4 (67) |
| PR | 11 (25) | 6 (24) | 3 (23) | 2 (33) |
| SD | 3(7) | 2 (8) | 1 (8) | 0 |
| PD | 5 (11) | 3 (12) | 2 (15) | 0 |
| Missing | 1 (2) | 0 | 1 (8) | 0 |
| CR, complete response; ORR, objective response rate; PD, progressive disease; PR, partial response; SD, stable disease. | | | | |

| **Table E35H. Summary of pharmacokinetics** | | | |
|---|---|---|---|
| | **Group A** | **Group B** | **Group C** |
| | (**n=25**) | (**n=13**) | **(n=6)** |
| Tmax, median (IQR) | 10.0 (10.0, 14.0) | 10 (7.0, 10.0) | 10.0 (10.0, 10.0) |
| Cmax, median (IQR) | 62.9 (24.3, 151.8) | 29.8 (14.7, 117.5) | 135.1 (64.5, 185.0) |
| AUC, median (IQR) | 474.8 (169.8, 1208.0) | 287.0 (128.6, 923.6) | 741.9 (318.7, 1135.1) |
| AUC, area under the concentration-time curve; Cmax, maximum concentration; IQR, interquartile range; Tmax, time to maximum concentration. | | | |

The results demonstrate that a subset of subjects with R/R large B cell NHL were successfully treated with an anti-CD19 CAR+ T cells and monitored for CAR T cell-related toxicity in the outpatient setting, including elderly subjects and subjects with high tumor burden. The results, including the safety profile, including the low rates of all-grade and severe CRS and NEs and their delayed onset, support outpatient administration for patients with R/R B-cell NHL treated with a composition containing anti-CD19 CAR-expressing cells. The results show that outpatient administration has been successfully implemented in multiple clinical studies across a variety of clinical sites (university/nonuniversity; dual/single entity) with adverse events managed safely by a multidisciplinary CAR T cell therapy team, and appropriate patient education and outpatient monitoring procedures, with no increase in severe toxicities, such as severe CRS or NEs The results also show, in a diverse patient population (elderly, comorbidities, high tumor burden), that severe CRS and NEs occurred with low incidence and were reversible. The results showed that severe CRS and NEs occurred with low incidence. The number of early hospitalizations was low, and 41% of subjects did not require hospitalization in the first month after anti-CD19 CAR+ T cell infusion, and ICU-level care was required in a very low percentage of subjects (5%). Most subjects achieved an objective response at the first time point (65%) and the second time point (80%) evaluated in this study.

### Example 17 Further Assessment of Biomarkers by Immunofluorescence (IF) and Gene Expression Analysis in Pre- and Post-administration Tumor Biopsies from Subjects for Administration of Anti-CD19 CAR-Expressing Cells

Expression of various biomarkers was assessed by multiplex immunofluorescence (IF) and gene expression analysis using RNA profiling, in tumor biopsies collected from subjects before and/or after administration of CAR-expressing T cells, in a subsequent analysis of a similar assessment described in Example 5 above.

### A. Tumor Biopsy Samples

Tumor biopsies were collected from selected subjects with relapsed or refractory (R/R) diffuse large B-cell lymphoma (DLBCL) from the clinical study described in Examples 1, 3, 6 and 13 above, who received treatment with anti-CD19 targeted therapeutic CAR⁺ T cell compositions, obtained at baseline prior to treatment and at approximately 11 days after administration (D11 post-treatment).

### B. Assessment of Cellular Markers

111 biopsies from 83 total subjects (n = 58 pre-treatment; n = 53 D11 post-infusion; 28 matched pairs) were examined using multiplexed IF.

The biopsies were assessed using the following three IF panels for detection: (1) B cell (CD19, CD20) and T cell lineage (CD4, CD8) markers and an antibody specific for a truncated receptor (surrogate marker) expressed by CAR+ cells, (2) biomarkers associated with immunosuppression (CD163, FoxP3, CD73, IDO1, PD-L1), and (3) biomarkers associated with immune cell function (CD3, Ki67, GZMB, PD-1) and an antibody specific for the truncated receptor. Fluorescently labeled antibody specific for the truncated receptor was used to identify CAR-expressing T cells within the tumor biopsies, generally as described in Example 5.B above.

Subjects were assessed for response and safety outcomes, including by assessing the tumor burden at various time points after administration of the CAR⁺ T cells, including at 3 months after administration, and determining whether the subject had progressive disease (PD), stable disease (SD), partial response (PR), or complete response (CR). Potential association between differences in marker densities for subjects with best overall response (BOR) of complete response (CR), and progressive disease (PD) were examined. Baseline and D11 biomarker expression were correlated with early responses at approximately 1 month (M1) post infusion (PD n=16; CR n=42) and durable responses at approximately 9 months (M9) post infusion (PD n=76; CR n=32; 55 subjects evaluated at both M1 and M9). The association between baseline and D11 biomarker expression densities, with spatial distinction between tumoral and peritumoral regions, and early and durable responses, were assessed. All comparisons were based on differences in median densities, and statistical significance were evaluated using uncorrected P values assessed via the (unpaired) Wilcoxon-Mann-Whitney nonparametric test.

Bulk tumor RNA profiling was performed in an overlapping subset of 50 baseline biopsies and 37 D11 post-treatment biopsies (11 matched pairs).

### C. Results

Biomarker expression signals in baseline biopsies that correlated with early response differed from those that correlated with durable complete response at 9 months (M9). A 21% higher presence of PD-1⁺ T cells at baseline was found to be associated with subjects who achieved early CR (at M1) as compared to subjects who had early PD (P=0.007). Subjects with durable CR (at M9) had 39% lower levels of tumor associated CD163⁺ macrophages at baseline (P=0.019) and 270% higher levels of CD73⁺ cells (P=0.028) than subjects with PD at M9. Evaluation of D11 post-treatment biopsies tumors showed that subjects with both early and durable CR had 28% higher levels of truncated receptor⁺ CD8⁺ T cells (indicative of CAR+ T cells) (P=0.022), and a 810% higher of truncated receptor CD4⁺ (non-CAR⁺ T cells) (P=0.009), but not truncated receptor⁻ CD8⁺ cells (P=0.28).

Changes in marker expression between baseline and D11 post-treatment biopsies were also investigated. Subjects with durable CR (at M9) were found to have decreased B-cell densities (P=0.029) and an increase in density of CD8⁺GZMB⁺, Ki67⁺, and/or PD1⁺ CAR⁺ cells (P=0.001) as well as non-CAR⁺ T cells (P=0.017) at D11. Subjects with durable CR at 9 months also were observed to have a 29% increase in tumor-associated CD163⁺ macrophages at D11 post infusion relative to baseline (P=0.033).

The general trends observed from IF assessment, which allows spatial assessment and multilabeled cells, were in agreement with those observed from bulk tumor RNA sequencing. Lower baseline expression of CD163 (P=0.021) and higher expression of CD73 (P=0.054) were observed in subjects with durable CR. Additionally, elevated expression of CD3E, CD4, and the CAR sequence (P<0.001) were also observed in subjects with durable CR at D11, consistent with the greater endogenous T cell and CAR⁺ T cell tumor infiltration in subjects that responded to treatment. Subjects with a CR at M9 exhibited increased CD163 expression measured at D11 relative to CD163 expression at baseline (P<0.001).

### D. Conclusion

The results showed increased infiltration of tumor-specific CAR⁺ T cells upon initial treatment with the anti-CD19 CAR-expressing cells. These results are consistent with a conclusion that an increased infiltration of tumor specific CAR T⁺ cells upon initial treatment helped establish an active immune response. Further, recruitment of additional functional endogenous T cells, in particular CD4+ T cells, were associated with a durable response. Higher numbers of stimulated and/or functional T cells and lower numbers of macrophages at baseline was also associated with a durable response to CAR T⁺ cell treatment. These results also support an observation that tumors in responders may have a baseline tumor microenvironment (TME) in which T cells can infiltrate and respond to antigen, promoting the entry of CAR T⁺ into tumors and the subsequent recruitment and stimulation of endogenous lymphocytes that support the function of CAR⁺ T cell function.

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.

### SEQUENCES

| **SEQ ID NO.** | **SEQUENCE** | **DESCRIPTION** |
|---|---|---|
| **1** | ESKYGPPCPPCP | spacer (IgG4hinge) (aa) Homo sapiens |
| **2** | GAATCTAAGTACGGACCGCCCTGCCCCCCTTGCCCT | spacer (IgG4hinge) (nt) homo sapiens |
| **3** | ESKYGPPCPPCPGQPREPQVYTLPPSQEEMTKNQVSLTCLVKGFYPSDIAVEWE SNGQPENNYKTTPPVLDSDGSFFLYSRLTVDKSRWQEGNVFSCSVMHEALHNHY TQKSLSLSLGK | Hinge-CH3 spacer Homo sapiens |
| **4** | ESKYGPPCPPCPAPEFLGGPSVFLFPPKPKDTLMISRTPEVTCVVVDVSQEDPE VQFNWYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEALHN HYTQKSLSLSLGK | Hinge-CH2-CH3 spacer Homo sapiens |
| **5** | RWPESPKAQASSVPTAQPQAEGSLAKATTAPATTRNTGRGGEEKKKEKEKEEQE ERETKTPECPSHTQPLGVYLLTPAVQDLWLRDKATFTCFVVGSDLKDAHLTWEV AGKVPTGGVEEGLLERHSNGSQSQHSRLTLPRSLWNAGTSVTCTLNHPSLPPQR LMALREPAAQAPVKLSLNLLASSDPPEAASWLLCEVSGFSPPNILLMWLEDQRE VNTSGFAPARPPPQPGSTTFWAWSVLRVPAPPSPQPATYTCVVSHEDSRTLLNA SRSLEVSYVTDH | IgD-hinge-Fc Homo sapiens |
| **6** | LEGGGEGRGSLLTCGDVEENPGPR | T2A artificial |
| **7** | MLLLVTSLLLCELPHPAFLLIPRKVCNGIGIGEFKDSLSINATNIKHFKNCTSI SGDLHILPVAFRGDSFTHTPPLDPQELDILKTVKEITGFLLIQAWPENRTDLHA FENLEIIRGRTKQHGQFSLAVVSLNITSLGLRSLKEISDGDVIISGNKNLCYAN TINWKKLFGTSGQKTKIISNRGENSCKATGQVCHALCSPEGCWGPEPRDCVSCR NVSRGRECVDKCNLLEGEPREFVENSECIQCHPECLPQAMNITCTGRGPDNCIQ CAHYIDGPHCVKTCPAGVMGENNTLVWKYADAGHVCHLCHPNCTYGCTGPGLEG CPTNGPKIPSIATGMVGALLLLLVVALGIGLFM | tEGFR artificial |
| **8** | FWVLVVVGGVLACYSLLVTVAFIIFWV | CD28 (amino acids 153-179 of Accession No. P10747) Homo sapiens |
| **9** | IEVMYPPPYLDNEKSNGTIIHVKGKHLCPSPLFPGPSKPFWVLVVVGGVLACYS LLVTVAFIIFWV | CD28 (amino acids 114-179 of Accession No. P10747) Homo sapiens |
| **10** | RSKRSRLLHSDYMNMTPRRPGPTRKHYQPYAPPRDFAAYRS | CD28 (amino acids 180-220 of P10747) Homo sapiens |
| **11** | RSKRSRGGHSDYMNMTPRRPGPTRKHYQPYAPPRDFAAYRS | CD28 (LL to GG) Homo sapiens |
| **12** | KRGRKKLLYIFKQPFMRPVQTTQEEDGCSCRFPEEEEGGCEL | 4-1BB (amino acids 214-255 of Q07011.1) Homo sapiens |
| **13** | RVKFSRSADAPAYQQGQNQLYNELNLGRREEYDVLDKRRGRDPEMGGKPRRKNP QEGLYNELQKDKMAEAYSEIGMKGERRRGKGHDGLYQGLSTATKDTYDALHMQA LPPR | CD3 zeta Homo sapiens |
| **14** | RVKFSRSAEPPAYQQGQNQLYNELNLGRREEYDVLDKRRGRDPEMGGKPRRKNP QEGLYNELQKDKMAEAYSEIGMKGERRRGKGHDGLYQGLSTATKDTYDALHMQA LPPR | CD3 zeta Homo sapiens |
| **15** | RVKFSRSADAPAYKQGQNQLYNELNLGRREEYDVLDKRRGRDPEMGGKPRRKNP QEGLYNELQKDKMAEAYSEIGMKGERRRGKGHDGLYQGLSTATKDTYDALHMQA LPPR | CD3 zeta Homo sapiens |
| **16** | RKVCNGIGIGEFKDSLSINATNIKHFKNCTSISGDLHILPVAFRGDSFTHTPPL DPQELDILKTVKEITGFLLIQAWPENRTDLHAFENLEIIRGRTKQHGQFSLAVV SLNITSLGLRSLKEISDGDVIISGNKNLCYANTINWKKLFGTSGQKTKIISNRG ENSCKATGQVCHALCSPEGCWGPEPRDCVSCRNVSRGRECVDKCNLLEGEPREF VENSECIQCHPECLPQAMNITCTGRGPDNCIQCAHYIDGPHCVKTCPAGVMGEN NTLVWKYADAGHVCHLCHPNCTYGCTGPGLEGCPTNGPKIPSIATGMVGALLLL LVVALGIGLFM | tEGFR artificial |
| **17** | EGRGSLLTCGDVEENPGP | T2A artificial |
| **18** | GSGATNFSLLKQAGDVEENPGP | P2A |
| **19** | ATNFSLLKQAGDVEENPGP | P2A |
| **20** | QCTNYALLKLAGDVESNPGP | E2A |
| **21** | VKQTLNFDLLKLAGDVESNPGP | F2A |
| **22** | PGGG-(SGGGG)5-P- wherein P is proline, G is glycine and S is serine | linker |
| **23** | GSADDAKKDAAKKDGKS | Linker |
| **24** | GSTSGSGKPGSGEGSTKG | Linker |
| **25** | gacatccagatgacccagaccacctccagcctgagcgccagcctgggcgaccgg gtgaccatcagctgccgggccagccaggacatcagcaagtacctgaactggtat cagcagaagcccgacggcaccgtcaagctgctgatctaccacaccagccggctg cacagcggcgtgcccagccggtttagcggcagcggctccggcaccgactacagc ctgaccatctccaacctggaacaggaagatatcgccacctacttttgccagcag ggcaacacactgccctacacctttggcggcggaacaaagctggaaatcaccggc agcacctccggcagcggcaagcctggcagcggcgagggcagcaccaaggcgag gtgaagctgcaggaaagcggccctggcctggtggcccccagccagagcctgagc gtgacctgcaccgtgagcggcgtgagcctgcccgactacggcgtgagctggatc cggcagcccccaggaagggcctggaatggctgggcgtgatctggggcagcgag accacctactacaacagcgccctgaagagccggctgaccatcatcaaggacaac agcaagagccaggtgttcctgaagatgaacagcctgcagaccgacgacaccgcc atctactactgcgccaagcactactactacggcggcagctacgccatggactac tggggccagggcaccagcgtgaccgtgagcagc | Sequence encoding scFv |
| **26** | X₁PPX₂P X₁ is glycine, cysteine or arginine X₂ is cysteine or threonine | Hinge |
| **27** | Glu Pro Lys Ser Cys Asp Lys Thr His Thr Cys Pro Pro Cys Pro | Hinge |
| **28** | Glu Arg Lys Cys Cys Val Glu Cys Pro Pro Cys Pro | Hinge |
| **29** | ELKTPLGDTHTCPRCPEPKSCDTPPPCPRCPEPKSCDTPPPCPRCPEPKSCDTP PPCPRCP | Hinge |
| **30** | Glu Ser Lys Tyr Gly Pro Pro Cys Pro Ser Cys Pro | Hinge |
| **31** | Glu Ser Lys Tyr Gly Pro Pro Cys Pro Pro Cys Pro | Hinge |
| **32** | Tyr Gly Pro Pro Cys Pro Pro Cys Pro | Hinge |
| **33** | Lys Tyr Gly Pro Pro Cys Pro Pro Cys Pro | Hinge |
| **34** | Glu Val Val Val Lys Tyr Gly Pro Pro Cys Pro Pro Cys Pro | Hinge |
| **35** | RASQDISKYLN | CDR L1 |
| **36** | SRLHSGV | CDR L2 |
| **37** | GNTLPYTFG | CDR L3 |
| **38** | DYGVS | CDR H1 |
| **39** | VIWGSETTYYNSALKS | CDR H2 |
| **40** | YAMDYWG | CDR H3 |
| **41** | EVKLQESGPGLVAPSQSLSVTCTVSGVSLPDYGVSWIRQPPRKGLEWLGVIWGS ETTYYNSALKSRLTIIKDNSKSQVFLKMNSLQTDDTAIYYCAKHYYYGGSYAMD YWGQGTSVTVSS | VH |
| **42** | DIQMTQTTSSLSASLGDRVTISCRASQDISKYLNWYQQKPDGTVKLLIYHTSRL HSGVPSRFSGSGSGTDYSLTISNLEQEDIATYFCQQGNTLPYTFGGGTKLEIT | VL |
| **43** | DIQMTQTTSSLSASLGDRVTISCRASQDISKYLNWYQQKPDGTVKLLIYHTSRL HSGVPSRFSGSGSGTDYSLTISNLEQEDIATYFCQQGNTLPYTFGGGTKLEITG STSGSGKPGSGEGSTKGEVKLQESGPGLVAPSQSLSVTCTVSGVSLPDYGVSWI RQPPRKGLEWLGVIWGSETTYYNSALKSRLTIIKDNSKSQVFLKMNSLQTDDTA IYYCAKHYYYGGSYAMDYWGQGTSVTVSS | scFv |
| **44** | KASQNVGTNVA | CDR L1 |
| **45** | SATYRNS | CDR L2 |
| **46** | QQYNRYPYT | CDR L3 |
| **47** | SYWMN | CDR H1 |
| **48** | QIYPGDGDTNYNGKFKG | CDR H2 |
| **49** | KTISSVVDFYFDY | CDR H3 |
| **50** | EVKLQQSGAELVRPGSSVKISCKASGYAFSSYWMNWVKQRPGQGLEWIGQIYPG DGDTNYNGKFKGQATLTADKSSSTAYMQLSGLTSEDSAVYFCARKTISSVVDFY FDYWGQGTTVTVSS | VH |
| **51** | DIELTQSPKFMSTSVGDRVSVTCKASQNVGTNVAWYQQKPGQSPKPLIYSATYR NSGVPDRFTGSGSGTDFTLTITNVQSKDLADYFCQQYNRYPYTSGGGTKLEIKR | VL |
| **52** | GGGGSGGGGSGGGGS | Linker |
| **53** | EVKLQQSGAELVRPGSSVKISCKASGYAFSSYWMNWVKQRPGQGLEWIGQIYPG DGDTNYNGKFKGQATLTADKSSSTAYMQLSGLTSEDSAVYFCARKTISSVVDFY FDYWGQGTTVTVSSGGGGSGGGGSGGGGSDIELTQSPKFMSTSVGDRVSVTCKA SQNVGTNVAWYQQKPGQSPKPLIYSATYRNSGVPDRFTGSGSGTDFTLTITNVQ SKDLADYFCQQYNRYPYTSGGGTKLEIKR | scFv |
| **54** | HYYYGGSYAMDY | CDR H3 |
| **55** | HTSRLHS | CDR L2 |
| **56** | QQGNTLPYT | CDR L3 |
| **57** | ACACGGCCTCGTGTATTACTGT | IGH primer |
| **58** | ACCTGAGGAGACGGTGACC | IGH Primer |

The invention further provides the following non-limiting embodiments:
1. A method of treating a subject having or suspected of having a disease or condition that is a relapsed or refractory large B cell lymphoma (r/r LBCL), the method comprising administering to the subject a dose of CD4⁺ and CD8⁺ T cells, wherein T cells of each dose comprises a chimeric antigen receptor (CAR) that specifically binds to CD19, wherein:
   the dose of T cells comprises between at or about 1 x 10⁷ CAR-expressing T cells and at or about 2 x 10⁸ CAR-expressing T cells, inclusive;
   the dose of T cells comprises a ratio of approximately 1:1 CD4⁺ T cells expressing the CAR to CD8⁺ T cells expressing the CAR; and
   the administration comprises administering a plurality of separate compositions, wherein the plurality of separate compositions comprises a first composition comprising CD8⁺ T cells and a second composition comprising CD4⁺ T cells.
2. The method of embodiment 1, wherein the large B cell lymphoma is selected from an aggressive non-Hodgkin lymphoma (NHL), diffuse large B cell lymphoma (DLBCL), optionally DLBCL NOS (de novo or transformed from indolent), high-grade B-cell lymphoma (HGBCL), double/triple hit lymphoma, primary mediastinal large B cell lymphoma (PMBCL), mantle cell lymphoma (MCL), transformed follicular lymphoma (tFL), and/or follicular lymphoma (FL), optionally follicular lymphoma Grade 3B (FL3B).
3. The method of embodiment 1 or embodiment 2, wherein the large B cell lymphoma is a Diffuse Large B-Cell Lymphoma (DLBCL).
4. The method of embodiment 3, wherein the DLBCL is a DLBCL NOS, a de novo DLBCL or a DLBCL transformed from indolent lymphoma.
5. The method of embodiment 3 or embodiment 4, wherein the DLBCL is a de novo DLBCL.
6. The method of embodiment 3 or embodiment 4, wherein the DLBCL is a DLBCL transformed from indolent lymphomas other than FL.
7. The method of embodiment 3 or embodiment 4, wherein the DLBCL is a DLBCL transformed from marginal zone lymphoma (tMZL) or a DLBCL transformed from chronic lymphocytic leukemia (tCLL; Richter's).
8. The method of embodiment 1 or embodiment 2, wherein the large B cell lymphoma is a high-grade B cell lymphoma (HGBCL).
9. The method of any of embodiments 1, 2 and 8, wherein the HGBCL has MYC and BCL2 and/or BCL6 rearrangements.
10. The method of any of embodiments 1, 2, 8 and 9, wherein the HGBCL has a DLBCL histology.
11. The method of embodiment 1 or embodiment 2, wherein the large B cell lymphoma is a double/triple hit lymphoma.
12. The method of embodiment 1 or embodiment 2, wherein the large B cell lymphoma is primary mediastinal B-cell lymphoma (PMBCL).
13. The method of embodiment 1 or embodiment 2, wherein the large B cell lymphoma is mantle cell lymphoma (MCL).
14. The method of embodiment 1 or embodiment 2, wherein the large B cell lymphoma is not a primary central nervous system lymphoma (PCNSL).
15. The method of embodiment 1 or embodiment 2, wherein the large B cell lymphoma is a transformed follicular lymphoma (tFL).
16. The method of embodiment 1 or embodiment 2, wherein the large B cell lymphoma is follicular lymphoma (FL).
17. A method of treatment, wherein the method comprises:
   (a) selecting a subject that has a follicular lymphoma (FL) for treatment;
   (b) administering to the subject a dose of T cells comprising T cells expressing a recombinant receptor that specifically binds to an antigen expressed by FL or a cell or tissue thereof and/or that is associated with FL.
18. The method of embodiment 17, wherein the dose of T cells comprises a dose of CD4⁺ and CD8⁺ T cells, wherein T cells of each dose comprises a recombinant receptor that specifically binds to an antigen expressed by FL or a cell or tissue thereof and/or that is associated with FL, wherein the administration comprises administering a plurality of separate compositions, wherein the plurality of separate compositions comprises a first composition comprising CD8⁺ T cells and a second composition comprising CD4⁺ T cells.
19. The method of embodiment 17 or embodiment 18, wherein the recombinant receptor is a chimeric antigen receptor (CAR).
20. The method of any of embodiments 17-19, wherein the antigen is CD19.
21. The method of any of embodiments 18-20, wherein the method comprises, prior to administration of the dose of cells, identifying or selecting for the administration of the dose of cells a subject that has a follicular lymphoma (FL).
22. The method of any of embodiments 16-21, wherein the FL is associated with co-expression of CD10, BCL6 and BCL2 within the follicles, and/or t(14;18)/(q32;q21) (IGH-BCL2) and/or BCL6 rearrangements.
23. The method of any of embodiments 16-22, wherein the FL is follicular lymphoma grade 3B (FL3B).
24. The method of any of embodiments 1-23, wherein, at or immediately prior to the time of the administration of the dose of cells, the subject has relapsed following remission after treatment with, or become refractory to, two or more prior therapy for the disease or condition other than another dose of cells expressing the CAR.
25. The method of any of embodiments 1-24, wherein, at or immediately prior to the time of the administration of the dose of cells, the subject has relapsed following remission after treatment with, or become refractory to, three or more prior therapy for the disease or condition other than another dose of cells expressing the CAR.
26. The method of any of embodiments 1-25, wherein, at or immediately prior to the time of the administration of the dose of cells, the subject has relapsed following remission after treatment with, or become refractory to, four or more prior therapy for the disease or condition other than another dose of cells expressing the CAR.
27. The method of any of embodiments 24-26, wherein the prior therapy comprises an anthracycline and a CD20-targeted agent.
28. The method of embodiment 27, wherein the one or more CD20-targeted agent comprises rituximab.
29. The method of embodiment 27 or embodiment 28, wherein the one or more CD20-targeted agent comprises R-CHOP (rituximab, cyclophosphamide, doxorubicin hydrochloride (hydroxydaunomycin), vincristine sulfate (oncovin) and prednisone).
30. The method of any of embodiments 24-29, wherein, if the prior therapy is a prior CD19-targeted therapy, a biological sample obtained from the subject after the prior CD19-targeted therapy comprises a cell expressing CD19.
31. The method of any of embodiments 24-30, wherein the prior therapy comprises an allogeneic or an autologous hematopoietic stem cell transplantation (HSCT).
32. The method of embodiment 31, wherein the subject has relapsed within 1 year or less than 1 year after receiving the HSCT.
33. The method of any of embodiments 24-32, wherein the subject has not achieved complete remission (CR) in response to the prior therapy.
34. The method of any of embodiments 1-33, wherein, at or prior to the administration of the dose of cells, the subject has been identified as having an aggressive disease or high-risk disease or as having poor prognosis.
35. The method of any of embodiments 1-34, wherein, at or prior to the administration of the dose of cells, the subject has been identified as having a chemorefractory disease or having a persistent or relapsed disease following chemotherapy.
36. The method of any of embodiments 1-35, wherein, at or prior to the administration of the dose of cells, the subject has been identified as having a chemorefractory lymphoma, optionally a chemorefractory DLBCL.
37. The method of any of embodiments 1-36, wherein, at or prior to the administration of the dose of cells, the subject has been identified as having a lymphoma associated with or involving central nervous system (CNS) involvement or a secondary CNS lymphoma.
38. The method of any of embodiments 1-37, wherein:
   at or prior to administration of the dose of cells, the subject is or has been identified as having a lymphoma associated with or involving central nervous system (CNS) involvement or a secondary CNS lymphoma; and/or
   at least 70%, at least 80%, at least 90% or at least 95% of subjects treated according to the method who, at or prior to the administration of the dose of cells exhibited or were identified to exhibit a lymphoma with CNS involvement or a secondary CNS lymphoma, achieved a resolution of the CNS disease.
39. The method of any of embodiments 1-38, wherein the subject is age 65 years or older.
40. The method of any of embodiments 1-39, wherein among the subjects treated, greater than at or about 35%, greater than at or about 40%, greater than at or about 45% or greater than at or about 50%, or any value between any of the foregoing, are aged 65 years or older.
41. The method of any of embodiments 1-39, wherein the subject is age 70 years or older.
42. The method of any of embodiments 1-41, wherein, at or prior to the administration of the dose of cells, the subject is or has been identified as having an impaired cardiac function, optionally with a left ventricular ejection fraction (LVEF) of less than at or about 50%.
43. The method of any of embodiments 1-42, wherein, at or prior to the administration of the dose of cells, the subject is or has been identified as having an impaired renal function, optionally with a calculated creatinine clearance of less than at or about 60 mL/min.
44. The method of any of embodiments 1-43, wherein, at or prior to the administration of the dose of cells, the subject is or has been identified as having an impaired pulmonary function, optionally with a diffusing capacity of the lungs for carbon monoxide (DLCO) of at or about 60% or less.
45. The method of any of embodiments 1-44, wherein, at or prior to the administration of the dose of cells, the subject is or has been identified as having an impaired hepatic function, optionally with an aspartate aminotransferase (AST) and alanine aminotransferase (ALT) of more than at or about twice the upper limit of normal (ULN).
46. The method of any of embodiments 1-45, wherein, at or prior to the administration of the dose of cells, the subject has received a bridging chemotherapy between the time of leukapheresis to produce the dose of CD4⁺ and CD8⁺ T cells and the administration of the dose of CD4⁺ and CD8⁺ T cells.
47. The method of any of embodiments 1-46, wherein, at or prior to the administration of the dose of cells, the subject has received a bridging chemotherapy for disease control after a prior therapy.
48. The method of embodiment 46 or embodiment 47, wherein the bridging chemotherapy is selected from among one or more of: Rituximab-gemcitabine plus oxaliplatin, Dexamethasone, radiotherapy, Rituximab, Prednisone, BR, Lenalidomide, gemcitabine plus oxaliplatin, Brentuximab vedotin, Ibrutinib, Bendamustine, and/or Gemcitabine + rituximab.
49. The method of any of embodiments 1-48, wherein, at or immediately prior to the time of the administration of the dose of cells, the subject is or has been identified as being ineligible for a high-dose chemotherapy.
50. The method of any of embodiments 1-49, wherein, at or immediately prior to the time of the administration of the dose of cells, the subject is or has been identified as being ineligible for a hematopoietic stem cell transplantation (HSCT).
51. The method of embodiment 49 or embodiment 50, wherein, at or immediately prior to the time of the administration of the dose of cells, the subject is or has been identified as being ineligible for both a high-dose chemotherapy and a hematopoietic stem cell transplantation (HSCT).
52. The method of any of embodiments 1-51, wherein, at or immediately prior to the time of the administration of the dose of cells, the subject is or has been identified as being ineligible for both a high-dose chemotherapy and a hematopoietic stem cell transplantation (HSCT), and the subject has relapsed following remission after treatment with, or become refractory to, one prior therapy for the disease or condition other than another dose of cells expressing the CAR.
53. The method of any of embodiments 1-52, wherein the subject is or has been identified as having an Eastern Cooperative Oncology Group Performance Status (ECOG PS) of 0, 1 or 2.
54. The method of any of embodiments 1-53, wherein the subject is or has been identified as having an Eastern Cooperative Oncology Group Performance Status (ECOG PS) of 0 or 1.
55. The method of any of embodiments 1-53, wherein the subject is or has been identified as having an Eastern Cooperative Oncology Group Performance Status (ECOG PS) of 2.
56. The method of any of embodiments 1-55, wherein, prior to the administration of the dose of cells, the subject is or has been identified as having a sum of product dimensions (SPD) of a tumor in the subject that is at or about 50 cm² or greater.
57. The method of any of embodiments 1-55, wherein, at or prior to the administration of the dose of cells, the subject has a positron emission tomography (PET)-positive disease.
58. The method of any of embodiments 1-56, wherein prior to administration of the dose of cells, identifying or selecting the subject for administration of the dose of cells.
59. The method of any of embodiments 1-57, wherein the method comprises, prior to administration of the dose of cells, identifying or selecting for the administration of the dose of cells a subject that has or is:
   a relapsed or refractory large B cell lymphoma; and/or
   an anthracycline and one or more CD20-targeted agent; and/or
   relapsed or refractory disease after two or more lines of therapy or after autologous HSCT; and/or
   having an ECOG performance status of 0, 1 or 2; and/or
   if the subject has received a prior CD19-targeted therapy, a biological sample obtained from the subject after the prior CD19-targeted therapy comprises a cell expressing CD19.
60. The method of any of embodiments 1-57, wherein the method comprises, prior to administration of the dose of cells, identifying or selecting for the administration of the dose of cells a subject that has or is:
   age 70 years or older; and/or
   an ECOG performance status of 2; and/or
   an impaired pulmonary function, optionally with a diffusing capacity of the lungs for carbon monoxide (DLCO) of at or about 60% or less; and/or
   an impaired cardiac function, optionally with a left ventricular ejection fraction (LVEF) of less than at or about 50%; and/or
   an impaired renal function, optionally with a calculated creatinine clearance of less than at or about 60 mL/min; and/or an impaired hepatic function, optionally with an aspartate aminotransferase (AST) and alanine aminotransferase (ALT) of more than at or about twice the upper limit of normal (ULN).
61. The method of any of embodiments 1-57, wherein the method comprises, prior to administration of the dose of cells, identifying or selecting for the administration of the dose of cells a subject that has:
   a double/triple hit lymphoma;
   a chemorefractory lymphoma, optionally a chemorefractory DLBCL;
   not achieved complete remission (CR) in response to a prior therapy for treating the malignancy, optionally the NHL; and/or
   has relapsed within 1 year or less than 1 year after receiving an autologous stem cell transplant (ASCT); and/or
   has a lymphoma associated with or involving central nervous system (CNS) involvement.
62. The method of any one of embodiments 1-61, wherein the dose of T cells is enriched for CD3+ T cells, CD4+ T cells, CD8+ T cells or CD4+ T cells and CD8+ T cells, optionally wherein greater than at or about 70%, 75%, 80%, 85%, 90%, 95% or 98% of the cells in the dose of T cells are CD3+ T cells, CD4+ T cells, CD8+ T cells or CD4+ T cells and CD8+ T cells.
63. The method of any of embodiments 1-17 and 19-62, wherein the initiation of the administration of the first composition is carried out prior to the initiation of the administration of the second composition.
64. The method of any of embodiments 1-17 and 19-63, wherein the administration of the first composition and the administration of the second composition are carried out no more than 48 hours apart.
65. The method of any of embodiments 1-17 and 19-64, wherein the administration of the first composition and the administration of the second composition are carried out no more than 36 hours apart, no more than 24 hours apart, no more than 12 hours apart, no more than 6 hours apart, no more than 4 hours apart, no more than 2 hours apart, no more than 1 hour apart or no more than 30 minutes apart.
66. The method of any of embodiments 1-17 and 19-64, wherein:
   the administration of the first composition and the administration of the second composition are carried out on the same day, are carried out between about 0 and about 12 hours apart, between about 0 and about 6 hours apart or between about 0 to 2 hours apart; or
   the initiation of administration of the first composition and the initiation of administration of the second composition are carried out between about 1 minute and about 1 hour apart or between about 5 minutes and about 30 minutes apart.
67. The method of any of embodiments 1-17 and 19-64, wherein the first composition and second composition are administered no more than 2 hours, no more than 1 hour, no more than 30 minutes, no more than 15 minutes, no more than 10 minutes or no more than 5 minutes apart.
68. The method of any of embodiments 1-67, wherein the recombinant receptor comprised by the CD4⁺ T cells and/or the recombinant receptor comprised by the CD8⁺ T cells is a recombinant receptor that is the same and/or wherein the CD4⁺ T cells and/or the CD8⁺ T cells are genetically engineered to express the recombinant receptor that is the same.
69. The method of any of embodiments 1-68, wherein the dose of CD4⁺ and CD8⁺ T cells comprises:
   between at or about 2.5 x 10⁷ and at or about 1.5 x 10⁸ total recombinant receptor-expressing T cells, inclusive;
   between at or about 5 x 10⁷ and at or about 1 x 10⁸ total recombinant receptor-expressing T cells, inclusive;
   at or about 5 x 10⁷ total recombinant receptor-expressing T cells;
   at or about 1 x 10⁸ total recombinant receptor-expressing T cells; or at or about 1.5 x 10⁸ total recombinant receptor-expressing T cells.
70. The method of any of embodiments 1-69, wherein the dose of CD4⁺ and CD8⁺ T cells comprises at or about 5 x 10⁷ total recombinant receptor-expressing T cells.
71. The method of any of embodiments 1-69, wherein the dose of CD4⁺ and CD8⁺ T cells comprises at or about 1 x 10⁸ total recombinant receptor-expressing T cells.
72. The method of any of embodiments 1-69, wherein the dose of CD4⁺ and CD8⁺ T cells comprises at or about 1.5 x 10⁸ total recombinant receptor-expressing T cells.
73. The method of any of embodiments 1-69, wherein the dose of CD4⁺ and CD8⁺ T cells comprises:
   between at or about 1.25 x 10⁷ and at or about 7.5 x 10⁷ recombinant receptor-expressing CD8⁺ T cells, inclusive;
   between at or about 2.5 x 10⁷ and at or about 5 x 10⁷ recombinant receptor-expressing CD8⁺ T cells, inclusive;
   at or about 2.5 x 10⁷ recombinant receptor-expressing CD8⁺ T cells;
   at or about 5 x 10⁷ recombinant receptor-expressing CD8⁺ T cells; or at or about 7.5 x 10⁷ recombinant receptor-expressing CD8⁺ T cells.
74. The method of any of embodiments 1-69, 70 and 73, wherein the dose of CD4⁺ and CD8⁺ T cells comprises at or about 2.5 x 10⁷ recombinant receptor-expressing CD8⁺ T cells.
75. The method of any of embodiments 1-69, 71 and 73, wherein the dose of CD4⁺ and CD8⁺ T cells comprises at or about 5 x 10⁷ recombinant receptor-expressing CD8⁺ T cells.
76. The method of any of embodiments 1-69, 72 and 73, wherein the dose of CD4⁺ and CD8⁺ T cells comprises at or about 7.5 x 10⁷ recombinant receptor-expressing CD8⁺ T cells.
77. The method of any of embodiments 1-76, wherein, prior to the administration, the subject has been preconditioned with a lymphodepleting therapy comprising the administration of fludarabine and/or cyclophosphamide.
78. The method of any of embodiments 1-77, wherein the method further comprises, immediately prior to the administration of a dose of the cells, administering a lymphodepleting therapy to the subject comprising the administration of fludarabine and/or cyclophosphamide.
79. The method of embodiment 77 or embodiment 78, wherein the administration of the dose of cells and/or the lymphodepleting therapy is carried out via outpatient delivery.
80. The method of embodiment 79, wherein the administration of the dose of cells and/or the lymphodepleting therapy is carried out in a non-tertiary care center.
81. The method of any of embodiments 1-80, wherein:
   the administration and any follow up is carried out on an outpatient basis and/or without requiring admission to or an overnight stay at a hospital; and
   if the subject exhibits a sustained fever or a fever that is or has not been reduced or not reduced by more than 1°C after treatment with an antipyretic, the subject is admitted to the hospital or to an overnight stay at a hospital and/or is administered an agent or treatment for the treatment or prevention or reduction or attenuation of a neurotoxicity and/or a cytokine release syndrome or risk thereof.
82. The method of any of embodiments 1-81, wherein prior to initiation of administration of the dose of cells, the subject has not been administered an agent or treatment for the treatment or prevention or reduction or attenuation of a neurotoxicity and/or a cytokine release syndrome or risk thereof.
83. The method of any of embodiments 1-82, further comprising administering to the subject an agent or treatment for the treatment or prevention or reduction or attenuation of a neurotoxicity and/or a cytokine release syndrome or risk thereof.
84. The method of any of embodiments 81-83, wherein the agent is or comprises an anti-IL-6 antibody, anti-IL-6 receptor antibody or a steroid.
85. The method of any of embodiments 81-84, wherein the agent is or comprises tocilizumab, siltuximab, dexamethasone or methylprednisolone.
86. The method of any of embodiments 81-85, wherein the agent is or comprises tocilizumab.
87. The method of any of embodiments 81-85, wherein the agent is or comprises dexamethasone.
88. The method of any of embodiments 1-87, wherein, at or prior to the administration of the dose of cells:
   the subject is or has been treated with an anthracycline and one or more CD20-targeted agent; and/or
   the subject is or has relapsed or refractory disease after two or more lines of therapy or after autologous HSCT; and/or
   the subject is or has been identified as having an ECOG performance status of 0, 1 or 2; and/or
   if the subject has received a prior CD19-targeted therapy, a biological sample obtained from the subject after the prior CD19-targeted therapy comprises a cell expressing CD19; and
   the administration of the dose of cells is carried out via outpatient delivery.
89. The method of any of embodiments 1-88, wherein the T cells are primary T cells obtained from a subject.
90. The method of any of embodiments 1-89, wherein the T cells are autologous to the subject.
91. The method of any of embodiments 1-90, wherein, at least 40%, at least 50%, at least 60%, at least 70% of the subjects who, at or prior to the administration of the dose of cells had or were identified to have a double/triple hit lymphoma or relapse following administration of an autologous stem cell transplant (ASCT), achieved an OR or an OR that is durable for at or greater than 3 months or at or greater than 6 months.
92. The method of any of embodiments 1-91, wherein:
   at least 35%, at least 40% or at least 50% of subjects treated according to the method achieve a complete response (CR);
   at least 60%, 70%, 80%, 90%, or 95% of subjects achieving a CR exhibit a CR that is durable for at or greater than 3 months or at or greater than 6 months; and/or
   at least 60%, 70%, 80%, 90%, or 95% of subjects achieving a CR by one month and/or by 3 months remain in response, remain in CR, and/or survive or survive without progression, for at or greater than 3 months and/or at or greater than 6 months and/or at greater than 9 months after achieving the CR; and/or
   at least 50%, at least 60% or at least 70% of the subjects treated according to the method achieve objective response (OR);
   at least 60%, 70%, 80%, 90%, or 95% of subjects achieving an OR exhibit an OR that is durable for at or greater than 3 months or at or greater than 6 months; and/or
   at least 35%, at least 40%, or at least 50% of subjects achieving an OR remain in response or survive for at or greater than 3 months and/or at or greater than 6 months after achieving the OR; and/or
   at least 40%, at least 50%, at least 60%, at least 70% of the subjects who, at or prior to the administration of the dose of cells had or were identified to have a double/triple hit lymphoma or relapse, following administration of an autologous stem cell transplant (ASCT), achieved an OR, or an OR that is durable for at or greater than 3 months or at or greater than 6 months.
93. The method of any of embodiments 1-92, wherein:
   at least 50% of subjects treated according to the method achieve a complete response (CR);
   at least 60% of subjects achieving a CR exhibit a CR that is durable for at or greater than 6 months; and/or
   at least 60% of subjects achieving a CR by 1 month and/or by 3 months remain in response, remain in CR, and/or survive or survive without progression, for at or greater than 6 months after achieving the CR; and/or
   at least 70% of the subjects treated according to the method achieve objective response (OR);
   at least 60%,of subjects achieving an OR exhibit an OR that is durable for at or greater than 6 months; and/or
   at least 50% of subjects achieving an OR remain in response or survive for at or greater than 6 months after achieving the OR; and/or
   at least 40% of the subjects who, at or prior to the administration of the dose of cells had or were identified to have a double/triple hit lymphoma or relapse, following administration of an autologous stem cell transplant (ASCT), achieved an OR, or an OR that is durable for at or greater than 3 months.
94. The method of any of embodiments 1-93, wherein the cells are autologous to the subject, and
   no minimum absolute lymphocyte count (ALC) for apheresis is required and/or specified for production of the therapy; and/or
   the cells are produced by a process which, for at least 90%, 91%, 92%, 93%, 94%, 95%, 96%, 97%, 98%, 99%, or 100% of subjects having the disease or condition or of the selected population of subjects, is capable of generating a cell product for administration according to the method.
95. The method of any of embodiments 92-94, wherein:
   the CR or the OR is durable for greater than 3 months or greater than 6 months; and/or
   at least 20%, at least 25%, at least 35%, at least 40% or at least 50% of subjects treated according to the method achieve a CR that is durable for greater than 3 months or greater than 6 months; and/or
   at least 60%, 70%, 80%, 90%, or 95% of subjects treated with the method and who achieve a CR, remain in CR or remain in response or remain surviving for at or greater than 3 months or at or greater than 6 months or at or greater than 9 months; and/or
   at least 60%, 70%, 80%, 90%, or 95% of subjects treated with the method who achieve a CR by one month and/or by 3 months remain in response, remain in CR, and/or survive or survive without progression, for greater at or greater than 3 months and/or at or greater than 6 months and/or at greater than 9 months; and/or
   at least 50%, at least 60% or at least 70% of the subjects treated according to the method achieve objective response (OR);
   at least 60%, 70%, 80%, 90%, or 95% of subjects achieve an OR that is durable for at or greater than 3 months or at or greater than 6 months; and/or
   at least at least 35%, at least 40%, or at least 50% of subjects treated with the method and achieving an OR remain in response or survive for at or greater than 3 months and/or at or greater than 6 months.
96. The method of any of embodiments 1-95, wherein:
   at least 35%, at least 40% or at least 50% of subjects treated according to the method achieve a complete response (CR) or remission of CNS disease;
   at least 60%, 70%, 80%, 90%, or 95% of subjects who achieve a CR remain in CR for at or greater than 3 months or at or greater than 6 months; and/or
   at least 60%, 70%, 80%, 90%, or 95% of subjects achieving a CR or remission of CNS disease by one month and/or by 3 months remain in response, remain in CR, and/or survive or survive without progression, for greater at or greater than 3 months and/or at or greater than 6 months and/or at greater than 9 months; and/or
   at least 50%, at least 60% or at least 70% of the subjects treated according to the method achieve objective response (OR) or remission of CNS disease;
   at least 60%, 70%, 80%, 90%, or 95% of subjects achieving the OR, for at or greater than 3 months or at or greater than 6 months; and/or
   at least 60%, 70%, 80%, 90%, or 95% of subjects achieving OR or remission of CNS disease remain in response or survive for at or greater than 3 months and/or at or greater than 6 months; and/or
   the brain lesion is reduced in size or volume by greater than or greater than about 25%, 50%, 75% or more; and/or
   reduction or remission or clearance of CNS disease is achieved in at least 35%, at least 40% or at least 50% of subjects treated according to the method.
97. The method of any of embodiment 1-96, wherein:
   greater than or greater than about 50%, about 60%, about 70%, or about 80% of the subjects treated according to the method do not exhibit a grade 3 or greater cytokine release syndrome (CRS) and/or do not exhibit a grade 3 or greater neurotoxicity and/or greater than 40% or 50% or 55% do not exhibit any neurotoxicity or CRS.
98. The method of any of embodiment 1-97, wherein greater than or greater than about 80% of the subjects treated according to the method do not exhibit a grade 3 or greater cytokine release syndrome (CRS) and/or do not exhibit a grade 3 or greater neurotoxicity.
99. The method of any of embodiments 1-98, wherein greater than 95% of the subjects treated according to the method do not exhibit grade 3 or greater CRS.
100. The method of any of embodiments 1-99, wherein greater than 85% of the subjects treated according to the method do not exhibit grade 3 or greater neurotoxicity.
101. The method of any of embodiments 1-100, wherein:
   greater than or greater than about 30%, 35%, 40%, or 50% of the subjects treated according to the method do not exhibit any grade of cytokine release syndrome (CRS) or neurotoxicity; and/or
   at least at or about 45%, 50%, 60%, 65%, 70%, 75%, 80%, 85%, 90%, or 95% of subjects treated according to the method do not exhibit onset of CRS earlier than 3 days following initiation of the administration and/or do not exhibit onset of neurotoxicity earlier than 5 days following initiation of the administration; and/or
   the median onset of neurotoxicity among subjects treated according to the method is at or after the median peak of, or median time to resolution of, CRS in subjects treated according to the method and/or the median onset of neurotoxicity among subjects treated according to the method is greater than at or about 8, 9, 10, or 11 days.
102. The method of any of embodiments 1-101, wherein:
   greater than or greater than about 50% of the subjects treated according to the method do not exhibit any grade of cytokine release syndrome (CRS) or neurotoxicity; and/or
   at least at or about 45% of subjects treated according to the method do not exhibit onset of CRS earlier than 3 days following initiation of the administration and/or do not exhibit onset of neurotoxicity earlier than 5 days following initiation of the administration; and/or
   the median onset of neurotoxicity among subjects treated according to the method is at or after the median peak of, or median time to resolution of, CRS in subjects treated according to the method and/or the median onset of neurotoxicity among subjects treated according to the method is greater than at or about 8 days.
103. The method of any of embodiments 1-102, wherein:
   at least 50% of subjects treated according to the method achieve a complete response (CR);
   at least 70% of the subjects treated according to the method achieve objective response (OR); and
   greater than or greater than about 50% of the subjects treated according to the method do not exhibit any grade of cytokine release syndrome (CRS) or neurotoxicity; and
   greater than or greater than about 80% of the subjects treated according to the method do not exhibit a grade 3 or greater cytokine release syndrome (CRS) and/or do not exhibit a grade 3 or greater neurotoxicity.
104. The method of any of embodiments 1-103, wherein:
   greater than or greater than about 25%, 30%, 35%, 40%, 45%, 50%, 55% or 60% of the subjects treated according to the method exhibit an improvement of 10 points or greater in European Organization for Research and Treatment Core Quality of Life Questionnaire version 3.0 (EORTC QLQ-C30) in global health status at months 6 or month 12 after administration compared to the score prior to treatment or at month 1 after treatment; and/or
   greater than or greater than about 25%, 30%, 35%, 40%, 45%, 50%, 55% or 60% of the subjects treated according to the method exhibit an improvement of 10 points or greater in EORTC QLQ-C30 in physical functioning at months 6 or month 12 after administration compared to the score prior to treatment or at month 1 after treatment; and/or
   greater than or greater than about 25%, 30%, 35%, 40%, 45%, 50%, 55% or 60% of the subjects treated according to the method exhibit an improvement of 10 points or greater in EORTC QLQ-C30 in fatigue at months 6 or month 12 after administration compared to the score prior to treatment or at month 1 after treatment; and/or
   greater than or greater than about 25%, 30%, 35%, 40%, 45%, 50%, 55% or 60% of the subjects treated according to the method exhibit an improvement of 10 points or greater in EORTC QLQ-C30 in pain at months 6 or month 12 after administration compared to the score prior to treatment or at month 1 after treatment; and/or
   greater than or greater than about 25%, 30%, 35%, 40%, 45%, 50%, 55% or 60% of the subjects treated according to the method exhibit an improvement of 10 points or greater in EORTC QLQ-C30 in pain at months 6 or month 12 after administration compared to the score prior to treatment or at month 1 after treatment.
105. The method of any of embodiments 1-104, wherein:
   the mean 5-level EuroQol-5D (EQ-5D-5L) score among subjects treated according to the method is the same or greater at months 6 or month 12 after administration compared to the score prior to treatment or at month 1 after treatment; and/or
   the mean EuroQol global visual analog scale (EQ-VAS) score among subjects treated according to the method is the same or greater at months 6 or month 12 after administration compared to the score prior to treatment or at month 1 after treatment.
106. The method of any of embodiments 1-105, wherein:
   the CAR comprises an extracellular antigen-binding domain specific for the antigen, a transmembrane domain, a cytoplasmic signaling domain derived from a costimulatory molecule, which optionally is a 4-1BB, and a cytoplasmic signaling domain derived from a primary signaling ITAM-containing molecule, which optionally is a CD3zeta;
   the CAR comprises, in order, an extracellular antigen-binding domain specific for the antigen, a transmembrane domain, a cytoplasmic signaling domain derived from a costimulatory molecule, and a cytoplasmic signaling domain derived from a primary signaling ITAM-containing molecule; or
   the CAR comprises an extracellular antigen-recognition domain that specifically binds to the antigen and an intracellular signaling domain comprising a CD3-zeta (CD3ζ) chain and a costimulatory signaling region that is a signaling domain of 4-1BB.
107. The method of any of embodiments 1-106, wherein the CAR comprises an extracellular antigen-binding domain specific for CD19, a transmembrane domain, a cytoplasmic signaling domain derived from a 4-1BB, and a cytoplasmic signaling domain derived from a CD3zeta.
108. The method of embodiment 107, wherein the antigen-binding domain is an scFv.
109. The method of embodiment 108, wherein the scFv comprises an amino acid sequence of RASQDISKYLN (SEQ ID NO: 35), an amino acid sequence of SRLHSGV (SEQ ID NO: 36), and/or an amino acid sequence of GNTLPYTFG (SEQ ID NO: 37) and/or an amino acid sequence of DYGVS (SEQ ID NO: 38), an amino acid sequence of VIWGSETTYYNSALKS (SEQ ID NO: 39), and/or an amino acid sequence of YAMDYWG (SEQ ID NO: 40) or wherein the scFv comprises a variable heavy chain region of FMC63 and a variable light chain region of FMC63 and/or a CDRL1 sequence of FMC63, a CDRL2 sequence of FMC63, a CDRL3 sequence of FMC63, a CDRH1 sequence of FMC63, a CDRH2 sequence of FMC63, and a CDRH3 sequence of FMC63 or binds to the same epitope as or competes for binding with any of the foregoing, and optionally wherein the scFv comprises, in order, a V_{H}, a linker, optionally comprising SEQ ID NO: 24, and a V_{L}, and/or the scFv comprises a flexible linker and/or comprises the amino acid sequence set forth as SEQ ID NO: 43.
110. The method of embodiment 108 or embodiment 109, wherein the scFv comprises a variable heavy chain region of FMC63 and a variable light chain region of FMC63.
111. The method of any of embodiments 106-110, wherein the costimulatory signaling region is a signaling domain of 4-1BB.
112. The method of any of embodiments 106-111, wherein the costimulatory domain comprises SEQ ID NO: 12 or a variant thereof having at least 85%, 86%, 87%, 88%, 89%, 90%, 91%, 92%, 93%, 94%, 95%, 96%, 97%, 98%, 99% or more sequence identity thereto.
113. The method of any of embodiments 106-112, wherein the primary signaling domain is a CD3zeta signaling domain.
114. The method of any of embodiments 106-113, wherein the primary signaling domain comprises SEQ ID NO: 13, 14 or 15 having at least 85%, 86%, 87%, 88%, 89%, 90%, 91%, 92%, 93%, 94%, 95%, 96%, 97%, 98%, 99% or more sequence identity thereto.
115. The method of any of embodiments 106-114, wherein the CAR further comprises a spacer between the transmembrane domain and the scFv.
116. The method of embodiment 115, wherein the spacer is a polypeptide spacer that comprises or consists of all or a portion of an immunoglobulin hinge or a modified version thereof, optionally an IgG4 hinge, or a modified version thereof.
117. The method of embodiment 115 or embodiment 116, wherein the spacer is at or about 12 amino acids in length.
118. The method of any of embodiments 115-117, wherein:
   the spacer has or consists of the sequence of SEQ ID NO: 1, a sequence encoded by SEQ ID NO: 2, SEQ ID NO: 30, SEQ ID NO: 31, SEQ ID NO: 32, SEQ ID NO: 33, SEQ ID NO: 34, or a variant of any of the foregoing having at least 85%, 86%, 87%, 88%, 89%, 90%, 91%, 92%, 93%, 94%, 95%, 96%, 97%, 98%, 99% or more sequence identity thereto; and/or
   the spacer comprises or consists of the formula X₁PPX₂P, where X₁ is glycine, cysteine or arginine and X₂ is cysteine or threonine.
119. The method of any of embodiments 115-118, wherein:
   the spacer is a polypeptide spacer that (a) comprises or consists of all or a portion of an immunoglobulin hinge or a modified version thereof or comprises about 15 amino acids or less, and does not comprise a CD28 extracellular region or a CD8 extracellular region, (b) comprises or consists of all or a portion of an immunoglobulin hinge, optionally an IgG4 hinge, or a modified version thereof and/or comprises about 15 amino acids or less, and does not comprise a CD28 extracellular region or a CD8 extracellular region, or (c) is at or about 12 amino acids in length and/or comprises or consists of all or a portion of an immunoglobulin hinge, optionally an IgG4, or a modified version thereof; or (d) has or consists of the sequence of SEQ ID NO: 1, a sequence encoded by SEQ ID NO: 2, SEQ ID NO: 30, SEQ ID NO: 31, SEQ ID NO: 32, SEQ ID NO: 33, SEQ ID NO: 34, or a variant of any of the foregoing having at least 85%, 86%, 87%, 88%, 89%, 90%, 91%, 92%, 93%, 94%, 95%, 96%, 97%, 98%, 99% or more sequence identity thereto, or (e) comprises or consists of the formula X₁PPX₂P, where X₁ is glycine, cysteine or arginine and X₂ is cysteine or threonine; and/or
   the costimulatory domain comprises SEQ ID NO: 12 or a variant thereof having at least 85%, 86%, 87%, 88%, 89%, 90%, 91%, 92%, 93%, 94%, 95%, 96%, 97%, 98%, 99% or more sequence identity thereto; and/or
   the primary signaling domain comprises SEQ ID NO: 13, 14 or 15 having at least 85%, 86%, 87%, 88%, 89%, 90%, 91%, 92%, 93%, 94%, 95%, 96%, 97%, 98%, 99% or more sequence identity thereto; and/or
   the scFv comprises an amino acid sequence of RASQDISKYLN (SEQ ID NO: 35), an amino acid sequence of SRLHSGV (SEQ ID NO: 36), and/or an amino acid sequence of GNTLPYTFG (SEQ ID NO: 37) and/or an amino acid sequence of DYGVS (SEQ ID NO: 38), an amino acid sequence of VIWGSETTYYNSALKS (SEQ ID NO: 39), and/or an amino acid sequence of YAMDYWG (SEQ ID NO: 40) or wherein the scFv comprises a variable heavy chain region of FMC63 and a variable light chain region of FMC63 and/or a CDRL1 sequence of FMC63, a CDRL2 sequence of FMC63, a CDRL3 sequence of FMC63, a CDRH1 sequence of FMC63, a CDRH2 sequence of FMC63, and a CDRH3 sequence of FMC63 or binds to the same epitope as or competes for binding with any of the foregoing, and optionally wherein the scFv comprises, in order, a V_{H}, a linker, optionally comprising SEQ ID NO: 24, and a V_{L}, and/or the scFv comprises a flexible linker and/or comprises the amino acid sequence set forth as SEQ ID NO: 43.
120. The method of any of embodiments 115-119, wherein:
   the spacer is a polypeptide spacer that comprises the sequence of SEQ ID NO: 1;
   the costimulatory domain comprises SEQ ID NO: 12;
   the primary signaling domain comprises SEQ ID NO: 13, 14 or 15;
   the antigen binding domain comprises an scFv that comprises a variable heavy chain region of FMC63 and a variable light chain region of FMC63.
121. The method of any of embodiments 1-120, wherein the dose of cells is administered parenterally, optionally intravenously.
122. The method of any of embodiments 1-121, wherein the subject is a human subject.
123. An article of manufacture comprising a composition comprising genetically engineered cells expressing a recombinant receptor; and optionally instructions for administering a dose of the cells in accord with the method of any of embodiments 1-122.
124. A method of assessing the risk of developing a toxicity after administration of a cell therapy, the method comprising:
   (a) assessing one or more parameters in a subject, wherein the one or more parameters is selected from the level, amount or concentration of LDH, ferritin or C-reactive protein (CRP) in a biological sample from the subject, or is a sum of product dimensions (SPD) of a tumor in the subject;
      wherein the subject is a candidate for treatment with the cell therapy, said cell therapy comprising a dose of genetically engineered cells expressing a recombinant receptor; and
      wherein the assessing is carried out prior to administering the cell therapy and/or said biological sample or tumor does not comprise the recombinant receptor and/or said engineered cells; and
   (b) identifying if the subject is as at risk of toxicity wherein the subject is at risk of toxicity if the one or more parameters is above a threshold level and the subject is not at risk of toxicity if the one or more parameters is at or below a threshold level, wherein:
      (i) the parameter is ferritin, and the threshold level is above at or about 1000 nanograms per milliliter, 2000 nanograms per milliliter, 3000 nanograms per milliliter, 4000 nanograms per milliliter, 5000 nanograms per milliliter, 6000 nanograms per milliliter, 7000 nanograms per milliliter or 8000 nanograms per milliliter; and/or
      (ii) the parameter is CRP, and the threshold level is above at or about 5 milligrams per liter, 10 milligrams per liter, 15 milligrams per liter, 20 milligrams per liter, 25 milligrams per liter, 30 milligrams per liter, 40 milligrams per liter or 50 milligrams per liter.
125. A method of identifying a subject, the method comprising:
   (a) assessing one or more parameters in a subject, wherein the one or more parameters is selected from the level, amount or concentration of LDH, ferritin or C-reactive protein (CRP) in a biological sample from the subject, or is a sum of product dimensions (SPD) of a tumor in the subject;
      wherein the subject is a candidate for treatment with a cell therapy, said cell therapy comprising a dose of genetically engineered cells expressing a recombinant receptor; and
      wherein the assessing is carried out prior to administering the cell therapy and/or said biological sample or tumor does not comprise the recombinant receptor and/or said engineered cells; and
   (b) identifying a subject who has a risk of developing a toxicity after administration of a cell therapy, wherein the subject is at risk of toxicity if the one or more parameters is above a threshold level and the subject is not at risk of toxicity if the one or more parameters is at or below a threshold level, wherein:
      (i) the parameter is ferritin, and the threshold level is above at or about 1000 nanograms per milliliter, 2000 nanograms per milliliter, 3000 nanograms per milliliter, 4000 nanograms per milliliter, 5000 nanograms per milliliter, 6000 nanograms per milliliter, 7000 nanograms per milliliter or 8000 nanograms per milliliter; and/or
      (ii) the parameter is CRP, and the threshold level is above at or about 5 milligrams per liter, 10 milligrams per liter, 15 milligrams per liter, 20 milligrams per liter, 25 milligrams per liter, 30 milligrams per liter, 40 milligrams per liter or 50 milligrams per liter.
126. A method of treatment, comprising:
   (a) assessing one or more parameters in a subject, wherein the one or more parameters is selected from the level, amount or concentration of LDH, ferritin or C-reactive protein (CRP) in a biological sample from the subject, or is a sum of product dimensions (SPD) of a tumor in the subject;
      wherein the subject is a candidate for treatment with a cell therapy, said cell therapy comprising a dose of genetically engineered cells expressing a recombinant receptor; and
      wherein the assessing is carried out prior to administering the cell therapy and/or said biological sample or tumor does not comprise the recombinant receptor and/or said engineered cells; and
   (b) identifying if the subject is as at risk of toxicity wherein the subject is at risk of toxicity if the one or more parameters is above a threshold level and the subject is not at risk of toxicity if the one or more parameters is at or below a threshold level, wherein:
      (i) the parameter is ferritin, and the threshold level is above at or about 1000 nanograms per milliliter, 2000 nanograms per milliliter, 3000 nanograms per milliliter, 4000 nanograms per milliliter, 5000 nanograms per milliliter, 6000 nanograms per milliliter, 7000 nanograms per milliliter or 8000 nanograms per milliliter; and/or
      (ii) the parameter is CRP, and the threshold level is above at or about 5 milligrams per liter, 10 milligrams per liter, 15 milligrams per liter, 20 milligrams per liter, 25 milligrams per liter, 30 milligrams per liter, 40 milligrams per liter or 50 milligrams per liter; and
   (c) following or based on the results of the assessment, administering to the subject the cell therapy, and, optionally, an agent or other treatment capable of treating, preventing, delaying, reducing or attenuating the development or risk of development of a toxicity.
127. The method of any of embodiments 124-126, wherein the parameter is ferritin, and the threshold level is 5000 nanograms per milliliter.
128. The method of any of embodiments 124-127, wherein the parameter is CRP, and the threshold level is 10 milligrams per liter.
129. The method of any of embodiments 124-128, wherein the one or more parameter is ferritin and CRP, and the threshold level for ferritin is 5000 nanograms per milliliter and the threshold level for CRP is 10 milligrams per liter.
130. The method of any of embodiments 124-129, wherein the biological sample is a blood or plasma sample.
131. The method of any of embodiments 124-130, wherein the level, amount or concentration of ferritin or CRP is measured prior to treatment, prior to apheresis, or prior to cell product manufacturing.
132. A method of assessing the risk of developing a toxicity after administration of a cell therapy, the method comprising:
   (a) assessing the peak concentration of genetically engineered cells expressing a recombinant receptor in a biological sample from a subject that has been previously administered a cell therapy comprising the genetically engineered cells; and
   (b) identifying if the subject is as at risk of toxicity wherein the subject is at risk of toxicity if the peak concentration of genetically engineered cells is above a threshold level and the subject is not at risk of toxicity if the peak concentration of genetically engineered cells is at or below a threshold level, wherein: the threshold level is above at or about 300 cells per microliter, 400 cells per microliter, 500 cells per microliter, 600 cells per microliter, 700 cells per microliter, 800 cells per microliter, 900 cells per microliter or 1000 cells per microliter.
133. A method of identifying a subject, the method comprising:
   (a) assessing the peak concentration of genetically engineered cells expressing a recombinant receptor in a biological sample from a subject that has been previously administered a cell therapy comprising the genetically engineered cells; and
   (b) identifying a subject who has a risk of developing a toxicity after administration of a cell therapy, wherein the subject is at risk of toxicity if the peak concentration of genetically engineered cells is above a threshold level and the subject is not at risk of toxicity if the peak concentration of genetically engineered cells is at or below a threshold level, wherein: the threshold level is above at or about 300 cells per microliter, 400 cells per microliter, 500 cells per microliter, 600 cells per microliter, 700 cells per microliter, 800 cells per microliter, 900 cells per microliter or 1000 cells per microliter.
134. A method of treatment, comprising:
   (a) assessing the peak concentration of genetically engineered cells expressing a recombinant receptor in a biological sample from a subject that has been previously administered a cell therapy comprising the genetically engineered cells; and
   (b) identifying if the subject is as at risk of toxicity wherein the subject is at risk of toxicity if the peak concentration of genetically engineered cells is above a threshold level and the subject is not at risk of toxicity if the peak concentration of genetically engineered cells is at or below a threshold level, wherein: the threshold level is above at or about 300 cells per microliter, 400 cells per microliter, 500 cells per microliter, 600 cells per microliter, 700 cells per microliter, 800 cells per microliter, 900 cells per microliter or 1000 cells per microliter; and
   (c) following or based on the results of the assessment, administering to the subject the cell therapy, and, optionally, an agent or other treatment capable of treating, preventing, delaying, reducing or attenuating the development or risk of development of a toxicity.
135. The method of any of embodiments 132-134, wherein the threshold level is 500 cells per microliter.
136. The method of any of embodiments 124-135, further comprising monitoring the subject for symptoms of toxicity if the subject is administered a cell therapy and is identified as having a risk of developing a toxicity.
137. The method of any of embodiments 124-136, wherein the toxicity is neurotoxicity or cytokine release syndrome (CRS).
138. The method of any of embodiments 124-137, wherein the toxicity is a grade 1 or higher neurotoxicity or CRS.
139. The method of any of embodiments 124-138, wherein the toxicity is a grade 1 or higher neurotoxicity.
140. The method of any of embodiments 124-139, wherein the toxicity is a severe neurotoxicity or is a grade 2 or higher neurotoxicity, a grade 3 or higher neurotoxicity, or a grade 4 or higher neurotoxicity
141. The method of any of embodiments 124-140, wherein the toxicity is a severe neurotoxicity or a grade 3 or higher neurotoxicity.
142. The method of any of embodiments 124-138, wherein the toxicity is a grade 1 or higher CRS.
143. The method of any of embodiments 124-138 and 142, wherein the toxicity is a severe CRS or is a grade 2 or higher CRS, a grade 3 or higher CRS, or a grade 4 or higher CRS.
144. The method of any of embodiments 124-138, 142 and 143, wherein the toxicity is a severe CRS or a grade 3 or higher CRS.
145. The method of any of embodiments 124-144, wherein if the subject is identified as having a risk of developing a toxicity, administering to the subject:
   (a) (1) an agent or other treatment capable of treating, preventing, delaying, reducing or attenuating the development or risk of development of a toxicity and (2) the cell therapy, wherein administration of the agent is to be administered (i) prior to, (ii) within one, two, or three days of, (iii) concurrently with and/or (iv) at first fever following, the initiation of administration of the cell therapy to the subject; and/or
   (b) a cell therapy at a reduced dose or at a dose that is not associated with risk of developing toxicity or severe toxicity, or is not associated with a risk of developing a toxicity or severe toxicity in a majority of subjects, and/or a majority of subjects having a disease or condition that the subject has or is suspected of having, following administration of the cell therapy; and/or
   (c) administering to the subject a cell therapy in an inpatient setting and/or with admission to the hospital for one or more days, optionally wherein the cell therapy is otherwise to be administered to subjects on an outpatient basis or without admission to the hospital for one or more days.
146. The method of embodiment 145, wherein the agent or other treatment is an anti-IL-6 antibody or an anti-IL-6 receptor antibody.
147. The method of embodiment 146, wherein the agent or other treatment is or comprises an agent selected from among tocilizumab, siltuximab, clazakizumab, sarilumab, olokizumab (CDP6038), elsilimomab, ALD518/BMS-945429, sirukumab (CNTO 136), CPSI-2634, ARGX-109, FE301 and FM101.
148. The method of embodiment 146 or embodiment 147, wherein the agent or other treatment is tocilizumab.
149. The method of embodiment 148, wherein the agent or other treatment is or comprises one or more steroids.
150. The method of embodiment 149, wherein the steroid is dexamethasone or methylprednisolone.
151. The method of embodiment 149 or embodiment 150, wherein the steroid is dexamethasone.
152. The method of any of embodiments 124-151, wherein the recombinant receptor specifically binds to an antigen associated with the disease or condition or expressed in cells of the environment of a lesion associated with the disease or condition.
153. The method of any of embodiments 124-152, wherein the disease or condition is a cancer.
154. The method of any of embodiments 124-153, wherein the disease or condition is a myeloma, a leukemia or a lymphoma.
155. The method of any of embodiments 124-54, wherein the disease or condition is a B cell malignancy and/or is acute lymphoblastic leukemia (ALL), adult ALL, chronic lymphoblastic leukemia (CLL), non-Hodgkin lymphoma (NHL), or a large B cell lymphoma.
156. The method of any of embodiments 124-155, wherein the disease or condition is a large B cell lymphoma.
157. The method of embodiment 156, wherein the large B cell lymphoma is selected from an aggressive non-Hodgkin lymphoma (NHL), diffuse large B cell lymphoma (DLBCL), optionally DLBCL NOS (de novo or transformed from indolent), high-grade B-cell lymphoma (HGBCL), double/triple hit lymphoma, primary mediastinal large B cell lymphoma (PMBCL), mantle cell lymphoma (MCL), transformed follicular lymphoma (tFL), and/or follicular lymphoma (FL), optionally follicular lymphoma Grade 3B (FL3B).
158. The method of embodiment 156 or embodiment 157, wherein the large B cell lymphoma is a Diffuse Large B-Cell Lymphoma (DLBCL).
159. The method of embodiment 156 or embodiment 157, wherein the large B cell lymphoma is a follicular lymphoma (FL).
160. The method of embodiment 159, wherein the FL is associated with co-expression of CD10, BCL6 and BCL2 within the follicles, and/or t(14;18)/(q32;q21) (IGH-BCL2) and/or BCL6 rearrangements.
161. The method of embodiment 156 or embodiment 157, wherein the large B cell lymphoma is mantle cell lymphoma (MCL).
162. The method of any of embodiments 36-49, wherein the recombinant receptor binds to a target antigen, wherein the target antigen is a B cell antigen, optionally CD19.
163. The method of any of embodiments 124-162, wherein the recombinant receptor is a chimeric antigen receptor (CAR).
164. The method of any of embodiments 124-163, wherein the genetically engineered cells comprise T cells, optionally CD4⁺T cells and/or CD8⁺ T cells.
165. The method of any of embodiments 124-163, wherein administration of the cell therapy comprises administering a dose of CD4⁺ and CD8⁺ T cells to a subject, wherein T cells of each dose comprise a recombinant receptor that specifically binds to an antigen expressed by the disease or condition or a cell or tissue thereof and/or that is associated with the disease or condition, wherein the administration comprises administering a plurality of separate compositions, wherein the plurality of separate compositions comprises a first composition comprising CD8⁺ T cells and a second composition comprising CD4⁺ T cells.
166. The method of embodiment 165, wherein the initiation of the administration of the first composition is carried out prior to the initiation of the administration of the second composition.
167. The method of embodiment 165 or embodiment 166, wherein the administration of the first composition and the administration of the second composition are carried out no more than 48 hours apart.
168. The method of any of embodiments 165-167, wherein the administration of the first composition and the administration of the second composition are carried out no more than 36 hours apart, no more than 24 hours apart, no more than 12 hours apart, no more than 6 hours apart, no more than 4 hours apart, no more than 2 hours apart, no more than 1 hour apart or no more than 30 minutes apart.
169. The method of any of embodiments 124-168, wherein the recombinant receptor comprised by the CD4⁺ T cells and/or the recombinant receptor comprised by the CD8⁺ T cells is or comprises a recombinant receptor that is the same and/or wherein the CD4⁺ T cells and/or the CD8⁺ T cells are genetically engineered to express the recombinant receptor that is the same.
170. The method of any of embodiments 124-169, wherein:
   the dose of CD4⁺ and CD8⁺ T cells comprises a defined ratio of CD4⁺ T cells expressing the receptor to CD8⁺ T cells expressing the recombinant receptor and/or of CD4⁺ T cells to CD8⁺ T cells, that is or is approximately 1:1 or is between approximately 1:3 and approximately 3:1; and/or
   the CD4⁺ T cells comprising the recombinant receptor in the one of the first and second compositions and the CD8⁺ T cells comprising the recombinant receptor in the other of the first and second compositions are present at a defined ratio that is or is approximately 1:1 or is between approximately 1:3 and approximately 3:1; and/or
   the CD4⁺ T cells comprising the receptor and the CD8⁺ T cells comprising the recombinant receptor administered in the first and second compositions are present at a defined ratio, which ratio is or is approximately 1:1 or is between approximately 1:3 and approximately 3:1.
171. The method of any of embodiments 124-170, wherein the defined ratio is or is approximately 1:1.
172. The method of any of embodiments 124-171, wherein the dose of CD4⁺ and CD8⁺ T cells comprises:
   between at or about 1 x 10⁷ and at or about 2 x 10⁸ total recombinant receptor-expressing T cells, inclusive;
   between at or about 2.5 x 10⁷ and at or about 1.5 x 10⁸ total recombinant receptor-expressing T cells, inclusive;
   between at or about 5 x 10⁷ and at or about 1 x 10⁸ total recombinant receptor-expressing T cells, inclusive;
   at or about 5 x 10⁷ total recombinant receptor-expressing T cells;
   at or about 1 x 10⁸ total recombinant receptor-expressing T cells; or
   at or about 1.5 x 10⁸ total recombinant receptor-expressing T cells.
173. The method of any of embodiments 124-172, wherein the dose of CD4⁺ and CD8⁺ T cells comprises:
   between at or about 1 x 10⁷ and at or about 1 x 10⁸ recombinant receptor-expressing CD8⁺ T cells, inclusive;
   between at or about 1.25 x 10⁷ and at or about 7.5 x 10⁷ recombinant receptor-expressing CD8⁺ T cells, inclusive;
   between at or about 2.5 x 10⁷ and at or about 5 x 10⁷ recombinant receptor-expressing CD8⁺ T cells, inclusive;
   at or about 2.5 x 10⁷ recombinant receptor-expressing CD8⁺ T cells;
   at or about 5 x 10⁷ recombinant receptor-expressing CD8⁺ T cells; or
   at or about 7.5 x 10⁷ recombinant receptor-expressing CD8⁺ T cells.
174. The method of any of embodiments 124-173, wherein the T cells are primary T cells obtained from a subject or are autologous to the subject.
175. The method of any of embodiments 124-174, wherein the subject is a human subject.
176. An article of manufacture comprising a composition comprising genetically engineered cells expressing a recombinant receptor and optionally an agent or other treatment capable of treating, preventing, delaying, reducing or attenuating the development or risk of development of a toxicity; and instructions for assessing the risk of developing a toxicity, identifying a subject or treating a subject in accord with the method of any of embodiments 124-175.

## Claims

1. Engineered T cells for use in a method of treating a disease or condition that is a primary central nervous system lymphoma (PCNSL), the method comprising administering to a subject having or suspected of having the disease or condition a dose of the engineered T cells, wherein the engineered T cells express a chimeric antigen receptor (CAR), wherein the CAR comprises an extracellular antigen-binding domain that specifically binds to CD19.

2. The engineered T cells for use according to claim 1, wherein:
(i) the subject has relapsed following remission after treatment with, or become refractory to, one or more prior therapies;
(ii) at or immediately prior to the time of the administration of the dose of engineered T cells, the subject has relapsed following remission after treatment with, or become refractory to, two, three, four or more prior therapies for the disease or condition other than another dose of cells expressing the CAR, and/or
(iii) the subject has relapsed within 1 year or less than 1 year after receiving HSCT.

3. The engineered T cells for use according to claim 1 or claim 2, wherein the CAR comprises the extracellular antigen-binding domain, a transmembrane domain, and a cytoplasmic signaling domain derived from a costimulatory molecule and a cytoplastic signaling domain derived from CD3zeta, optionally wherein:
(i) the extracellular antigen-binding domain and transmembrane domain are linked by a spacer, and/or
(ii) the costimulatory molecule is 4-1BB.

4. The engineered T cells for use according to any one of claims 1-3, wherein the extracellular antigen-binding domain is an scFv, optionally wherein the scFv:
(i) is derived from FMC63;
(ii) comprises a CDRL1 sequence of RASQDISKYLN (SEQ ID NO:35), a CDRL2 sequence of SRLHSGV (SEQ ID NO:36), a CDRL3 sequence of GNTLPYTFG (SEQ ID NO:37), a CDRH1 sequence of DYGVS (SEQ ID NO:38), a CDRH2 sequence of VIWGSETTYYNSALKS (SEQ ID NO:39), and a CDRH3 sequence of YAMDYWG (SEQ ID NO:40);
(iii) comprises a VH sequence set forth in SEQ ID NO:41 and a VL sequence set forth in SEQ ID NO:42, and/or
(iv) comprises the sequence set forth in SEQ ID NO:43.

5. The engineered T cells for use according to any one of claims 1-4, wherein the dose of engineered T cells comprises:
(i) between at or about 2.5 x 10⁷ total viable CD19 CAR-expressing T cells and at or about 2.5 x 10⁸ total viable CD19 CAR-expressing T cells, inclusive;
(ii) between at or about 5 x 10⁷ total viable CD19 CAR-expressing T cells and at or about 1 x 10⁸ total viable CD19 CAR-expressing T cells, inclusive; and/or
(iii) about 1 x 10⁸ total viable CD19 CAR-expressing T cells.

6. The engineered T cells for use according to any one of claims 1-4, wherein the dose of engineered T cells comprises:
(i) between at or about 2 x 10⁵ viable CD19 CAR-expressing T cells/kg body weight and at or about 2 x 10⁶ viable CD19 CAR-expressing T cells/kg body weight, inclusive, and/or
(ii) between at or about 4 x 10⁵ viable CD19 CAR-expressing T cells/kg body weight and at or about 1 x 10⁶ viable CD19 CAR-expressing T cells/kg body weight, inclusive.

7. The engineered T cells for use according to any one of claims 1-6, wherein the T cells of the dose comprise CD4⁺ T cells and CD8+ T cells.

8. The engineered T cells for use according to claim 7, wherein the method comprises administering the engineered T cells at or within a tolerated range of a desired ratio of CD19 CAR-expressing CD4+ T cells to CD19 CAR-expressing CD8+ T cells between at or about 5:1 and at or about 1:5, and wherein the tolerated range is within about 1% of the desired ratio.

9. The engineered T cells for use according to any one of claims 1-8, wherein:
the dose of engineered T cells comprises between at or about 5 x 10⁷ total viable CD19 CAR-expressing T cells and at or about 1 x 10⁸ total viable CD19 CAR-expressing T cells, inclusive; and
the CAR comprises the extracellular antigen-binding domain, a transmembrane domain, and an intracellular signaling domain comprising i) a costimulatory signaling region that is a signaling domain of 4-1BB and ii) a CD3zeta chain, optionally wherein the extracellular antigen-binding domain and transmembrane domain are linked by a spacer.

10. The engineered T cells for use according to any one of claims 1-9, wherein the administering the dose of engineered T cells comprises administering a plurality of separate compositions, wherein the plurality of separate compositions comprises a first composition comprising CD8+ T cells and a second composition comprising CD4+ T cells.

11. The engineered T cells for use according to any one of claims 1-10, wherein the PCNSL is an extranodal high-grade non-Hodgkin B-cell lymphoma.
